(19) Europäisches Patentamt
European Patent Office
Office européen des brevets

(11) EP 2 344 523 B1

(12) EUROPEAN PATENT SPECIFICATION

(45) Date of publication and mention of the grant of the patent:
**10.02.2016 Bulletin 2016/06**

(21) Application number: **09786278.3**

(22) Date of filing: **17.09.2009**

(51) Int Cl.:
***C07K 14/315*** (2006.01) ***C07K 16/12*** (2006.01)
***A61K 39/09*** (2006.01) ***A61P 31/04*** (2006.01)

(86) International application number:
**PCT/IB2009/006949**

(87) International publication number:
**WO 2010/076618 (08.07.2010 Gazette 2010/27)**

(54) **COMBINATION GAS VACCINES AND THERAPEUTICS**

GAS-KOMBINATIONSIMPFSTOFFE UND THERAPEUTIKA

VACCINS ET AGENTS THÉRAPEUTIQUES ANTI-SGA COMBINÉS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.09.2008 US 97551 P**

(43) Date of publication of application:
**20.07.2011 Bulletin 2011/29**

(73) Proprietor: **GlaxoSmithKline Biologicals SA**
**1330 Rixensart (BE)**

(72) Inventors:
- **MANETTI, Andrea**
  **53100 Siena (IT)**
- **CAPO, Sabrina**
  **53100 Siena (IT)**
- **BENSI, Giuliano**
  **53100 Siena (IT)**
- **GRANDI, Guido**
  **53100 Siena (IT)**
- **MARGARIT Y ROS, Imma**
  **53100 Siena (IT)**

(74) Representative: **Evans, Stephen John Eves**
**GlaxoSmithKline S.A.**
**Global Patents CN925.1**
**980 Great West Road**
**Brentford**
**Middlesex TW8 9GS (GB)**

(56) References cited:

| | |
|---|---|
| **EP-A2- 0 369 825** | **WO-A-2005/032582** |
| **WO-A-2006/042027** | **WO-A-2008/108830** |
| **WO-A2-2009/034473** | **WO-A2-2009/081274** |

- **SEVERIN ANATOLY ET AL: "Proteomic analysis and identification of Streptococcus pyogenes surface-associated proteins" JOURNAL OF BACTERIOLOGY, vol. 189, no. 5, March 2007 (2007-03), pages 1514-1522, XP002564465 ISSN: 0021-9193**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

FIELD OF THE INVENTION

The invention relates to the fields of immunology and vaccinology.

BACKGROUND OF THE INVENTION

Group A streptococcus ("GAS," *S. pyogenes*) is a frequent human pathogen, estimated to be present in between 5-15% of normal individuals without signs of disease. An acute infection occurs, however, when host defenses are compromised, when the organism is able to exert its virulence, or when the organism is introduced to vulnerable tissues or hosts. Related diseases include puerperal fever, scarlet fever, erysipelas, pharyngitis, impetigo, necrotizing fasciitis, myositis, and streptococcal toxic shock syndrome.

Efforts to develop a prophylactic vaccine for use against GAS have been ongoing for many decades. Currently, however, there are no GAS vaccines available to the public. There is a need in the art for such vaccines.

BRIEF DESCRIPTION OF THE DRAWINGS

**FIG. 1.** Graph showing protective capacity of GAS antigen combinations in a subcutaneous challenge mouse model. "GAS57," Spy0416; "GAS25," Spy0167; "GAS40," Spy0269 (SEQ ID NO:177); "dGAS57," Spy0167 mutant D151A/S617A (SEQ ID NO:198); "dGAS25," Spy0167 mutant P427L/W535F (SEQ ID NO:125).

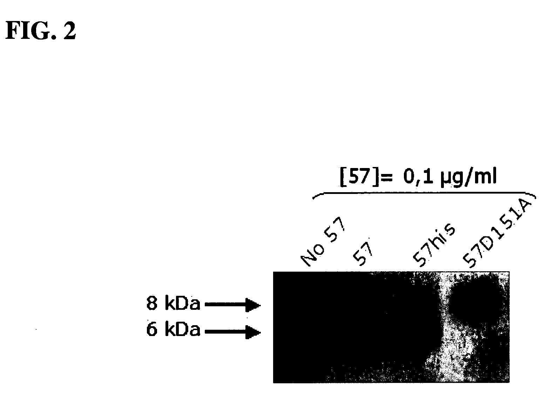

**FIG. 2.** Photomicrograph of SDS-polyacrylamide gels demonstrating that Spy0416 point mutant D151A has lost the ability to cleave I-8. "57," Spy0416 (GAS57).

**FIG. 3.** Graph showing the results of an ELISA assay demonstrating that Spy0416 point mutant D151A has lost the ability to cleave IL-8.

**FIGS. 4A-B.** Photomicrographs of SDS-polyacrylamide gels demonstrating that Spy0416 single mutants D 151 A and S617A and the mutant D151A + S617A have lost Spy0416 proteolytic activity.

**FIG. 5.** Graph showing the results of an ELISA assay demonstrating that single mutants D151A and S617A and Spy0416 mutant D151A + S617A have lost Spy0416 ("57") proteolytic activity.

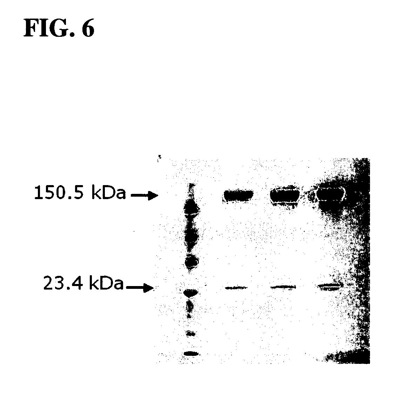

**FIG. 6.** Photomicrograph of an SDS-polyacrylamide gel demonstrating that wild-type Spy0416 is post-translationally modified into two polypeptide fragments of 150.5 kDa and 23.4 kDa.

**FIG. 7.** Photomicrograph of an SDS-polyacrylamide gel demonstrating that Spy0416 mutants D151A, S617A, and D151A + S617A are not post-translationally modified into two polypeptide fragments of 150.5 kDa and 23.4 kDa compared to wild-type (black arrows). A major band of 174 kDa corresponding to unprocessed protein is instead present in the lanes corresponding to inactive mutant strains (grey arrow). "57," Spy0416.

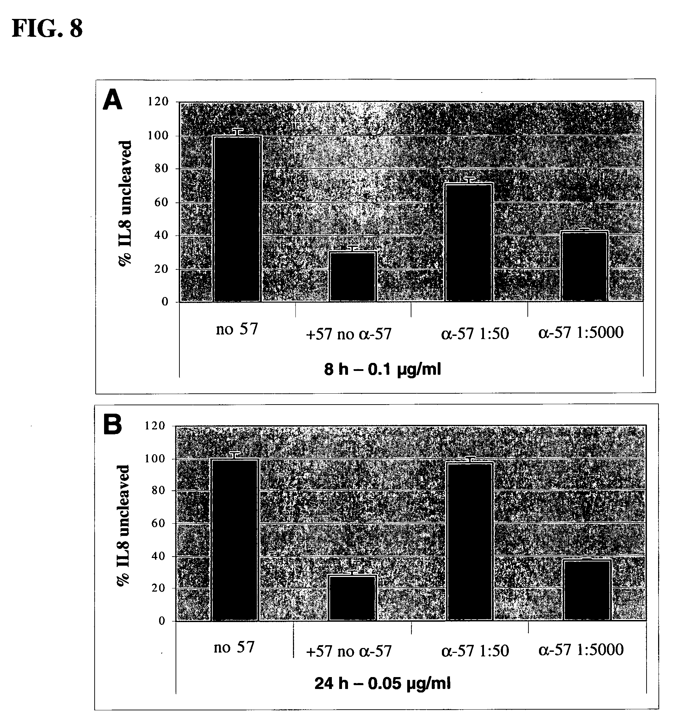

**FIGS. 8A-B.** ELISA assay results demonstrating dose-dependent inhibition of Spy0416-mediated IL-8 cleavage by polyclonal antisera against Spy0416 ("57") in two different experimental conditions. **FIG. 8A**, 8 hour incubation, 0.1 $\mu$g/ml of Spy0416. **FIG. 8B**, 24 hour incubation, 0.05 $\mu$g/ml of Spy0416.

**FIG. 9.** Graph showing results of hemolytic assay using *E. coli* extracts containing wild-type Spy0167 and Spy0167 mutant P427L.

**FIG. 10.** Photomicrograph of SDS-polyacrylamide gel showing purified Spy0167 mutant P427L.

**FIG. 11.** Graph showing results of hemolytic assay using purified wild-type Spy0167 and Spy0167 mutant P427L.

**FIG. 12.** Photomicrograph of SDS-polyacrylamide gel of *E. coli* lysate supernatants. Lane A, *E. coli* negative control; lane B, rSpy0167 wild-type, without tag; lane C, rSpy0167 P427L, without tag; and lane D, purified rSpy0167 wild-type, without tag (5mg).

**FIG. 13.** Graph demonstrating that under the same conditions, Spy0167 mutant P427L is 1000 times less hemolytic than wild-type Spy0167.

**FIG. 14.** Graph demonstrating effects of cholesterol on hemolysis by wild-type Spy0167 and Spy0167 mutant P427L.

**FIG. 15.** Photomicrographs of SDS-PAGE analysis of total *tag-less* proteins in cell extracts. **FIG. 15A**, expression of Spy0167 wild-type and P427L *tag-less* proteins; **FIG. 15B**, expression of Spy0167 P427L + W535, P427L + C530G, and P427L + C530G + W535F *tag-less* proteins.

**FIG. 16.** Photomicrograph of SDS-PAGE analysis of total His-tagged proteins in cell extracts.

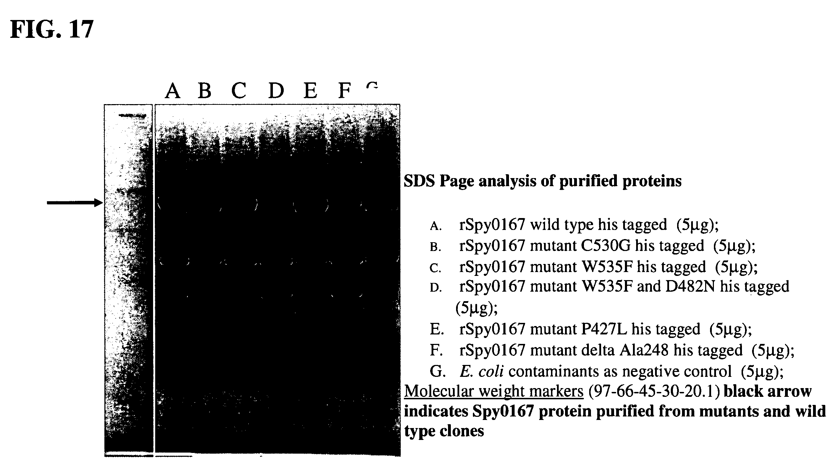

**FIG. 17.** Photomicrograph of SDS-PAGE analysis of purified His-tagged proteins.

**FIG. 18.** Photomicrographs of SDS-PAGE analysis of purified *tag-less* proteins. **FIG. 18A**, Lanes: A, Spy0167 wild-type *tag-less*; B, Spy0167 P427L *tag-less*; molecular weight markers (116-66.2-45-35-25-18.4-14.4); black arrow indicates Spy0167 protein purified from mutants and wild-type clones. **FIG. 18B**, lane A, Spy0167 Wild Type *tag-less* (3μg), lane B, Spy0167 P427L-W535F *tag-less* (3μg); molecular weight markers (116-66.2-45-35-25-18.4-14.4); black arrow indicates Spy0167 protein purified from mutants and wild-type clones.

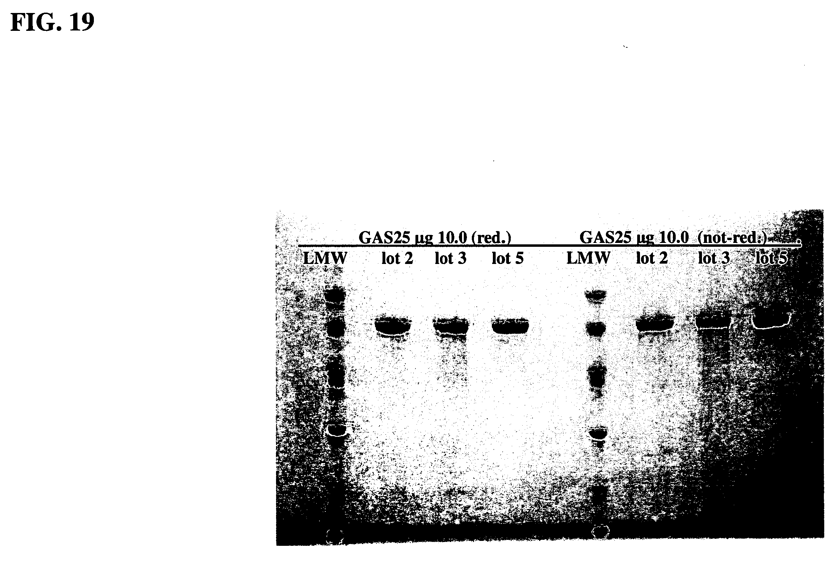

**FIG. 19.** Photomicrograph of SDS-PAGE analysis of purified *tag-less* Spy0167 ("GAS25") wild-type protein. Samples of different purification lots of wild-type Spy0167 were analyzed under reducing and non-reducing conditions.

**FIG. 20.** Graph showing results of hemolysis tests of His-tagged Spy0167 mutants.

**FIG. 21.** Graph showing inhibition of Spy0167-induced hemolytic activity by anti-Spy0167 antiserum.

**FIG. 22.** Graph showing titration of anti-Spy0167 antiserum inhibition of Spy0167 hemolysis.

**FIG. 23.** Graph showing Spy0167 hemolytic activity titration.

**FIG. 24.** Graph showing titration of hemolytic activity of wild-type Spy0167, chemically detoxified wild-type Spy0167, and Spy0167 mutants (P427L; P427L + W535F).

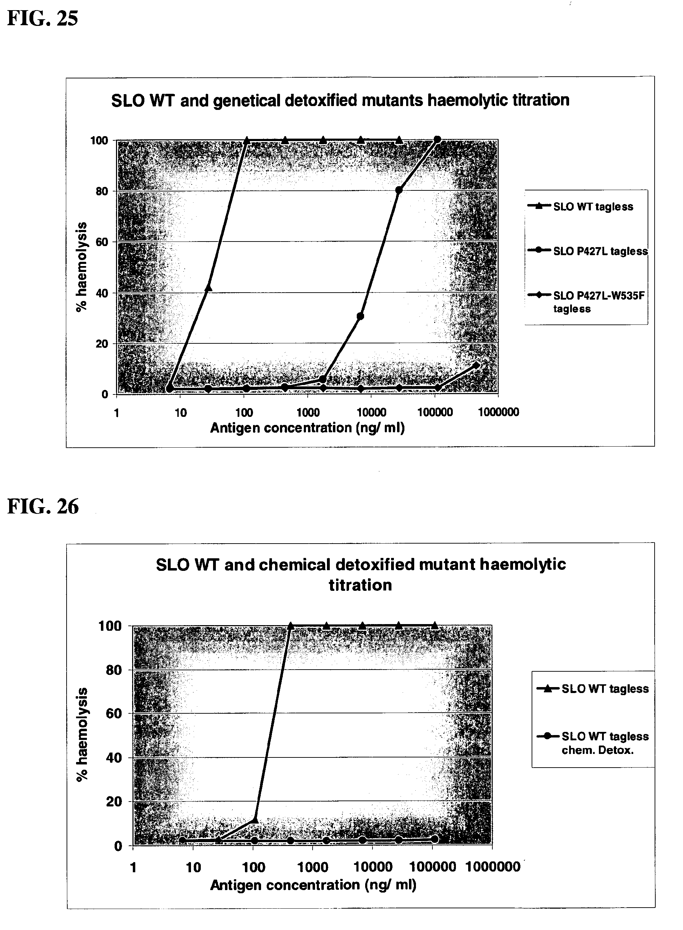

**FIG. 25.** Graph showing titration of hemolytic activity of wild-type Spy0167 and Spy0167 mutants (P427L; P427L + W535F).

**FIG. 26.** Graph showing titration of hemolytic activity of wild-type Spy0167 and chemically detoxified wild-type Spy0167.

**FIG. 27.** Graph showing dilution of antiserum against Spy0167 ("gas25") mutant P427L + W535F required to obtain 50% reduction of Spy0167 hemolytic activity (50 ng/ml Spy0167).

**FIG. 28.** Graph showing dilution of antiserum against Spy0167 ("gas25") mutant P427L + W535F required to obtain 50% reduction of Spy0167 hemolytic activity (100 ng/ml Spy0167).

**FIG. 29.** Titration curve showing that hemolysis inhibition assays were performed with toxin concentrations which allow 100% haemolysis.

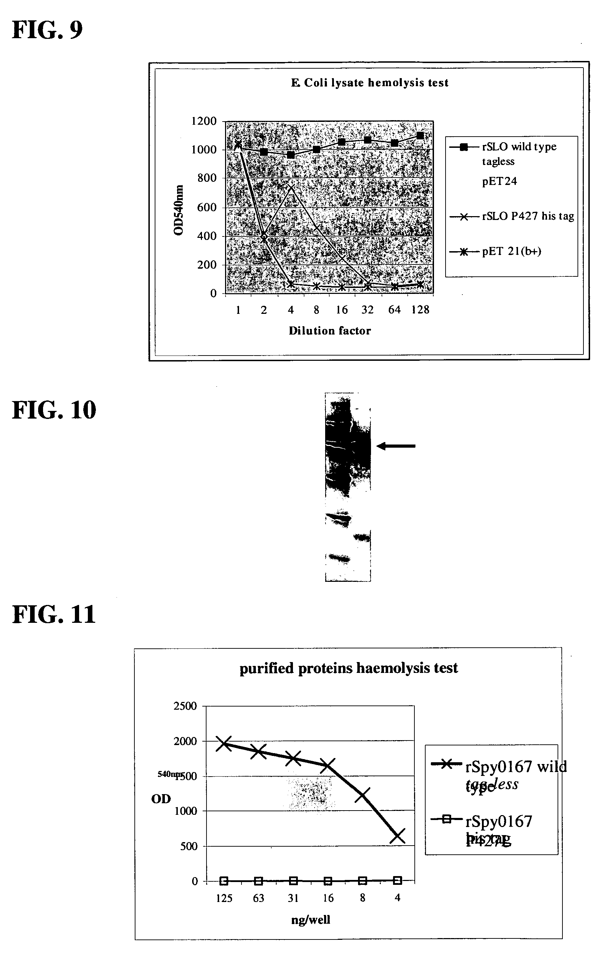

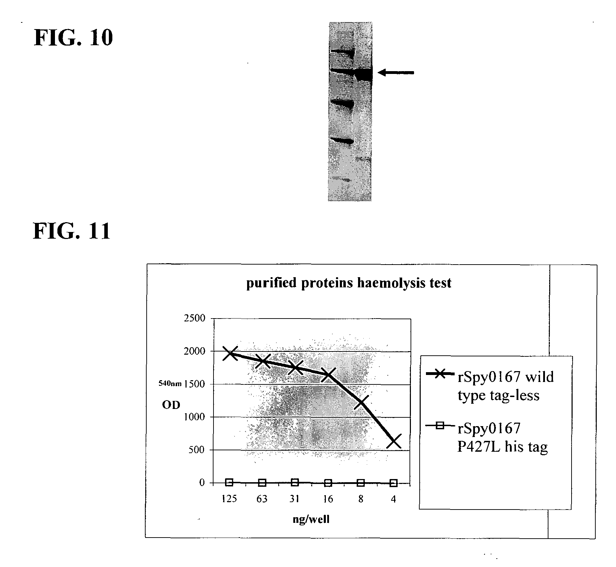

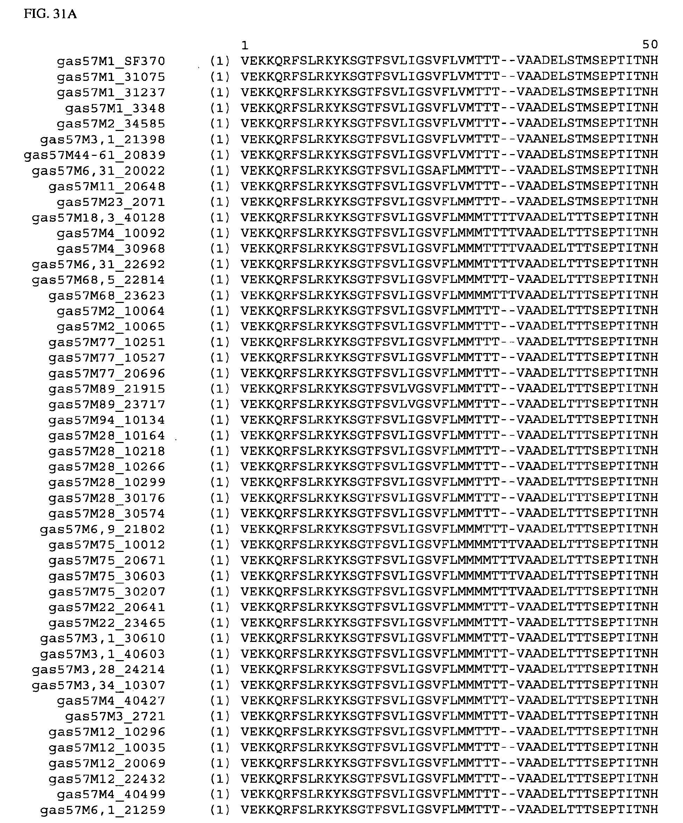

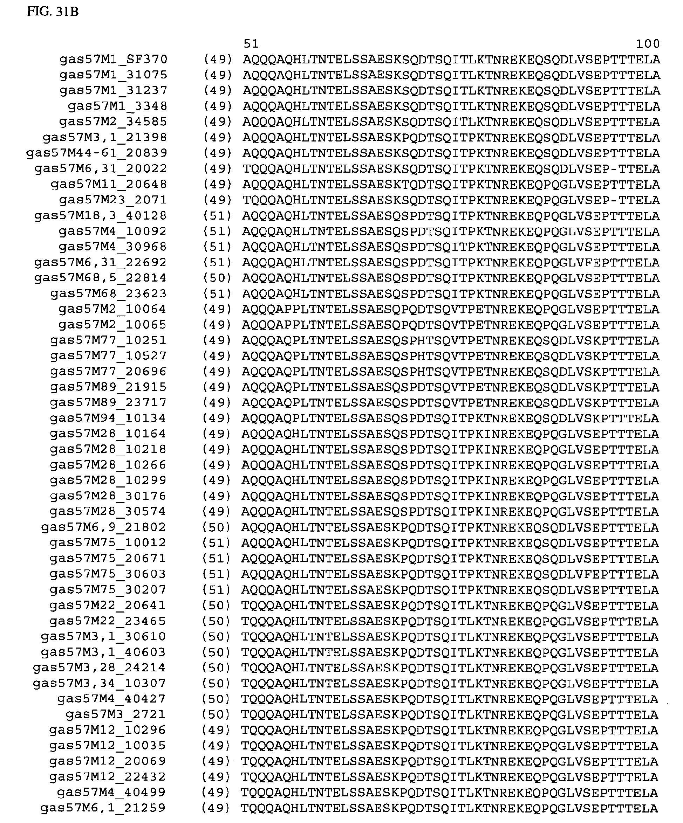

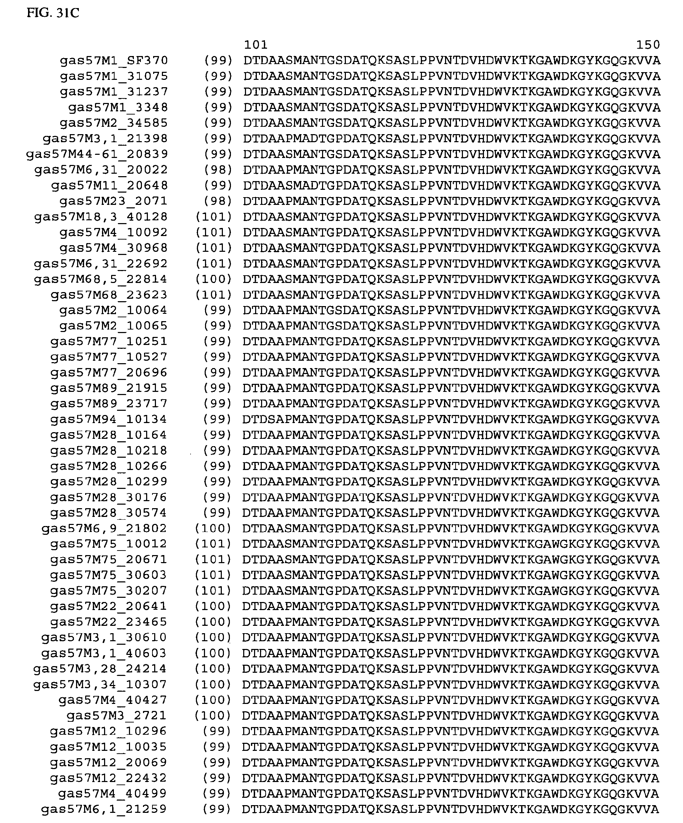

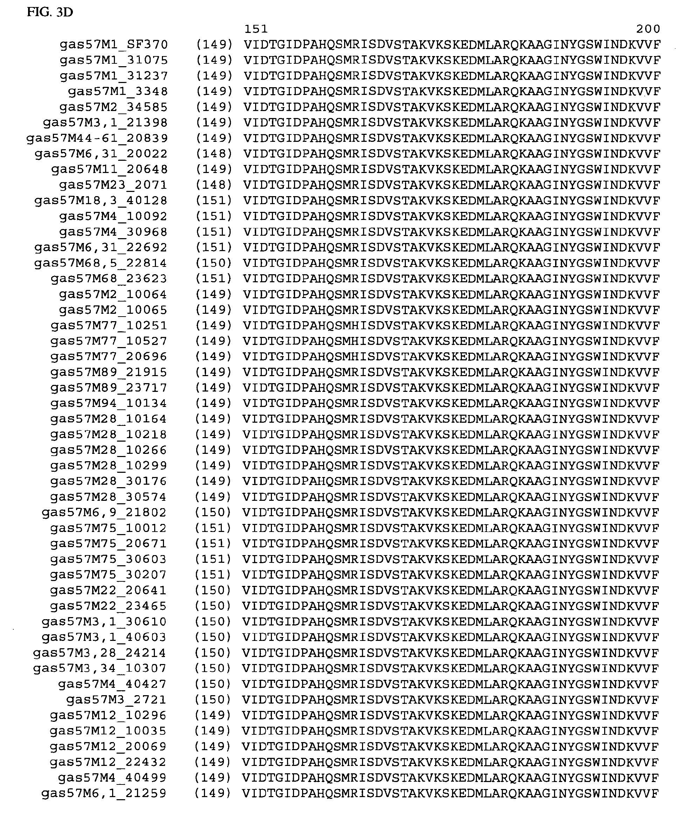

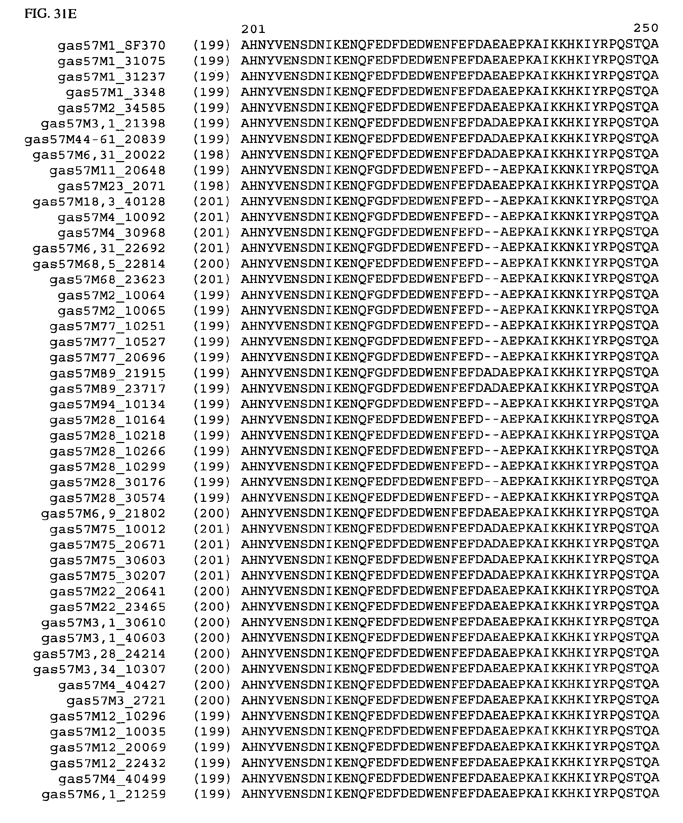

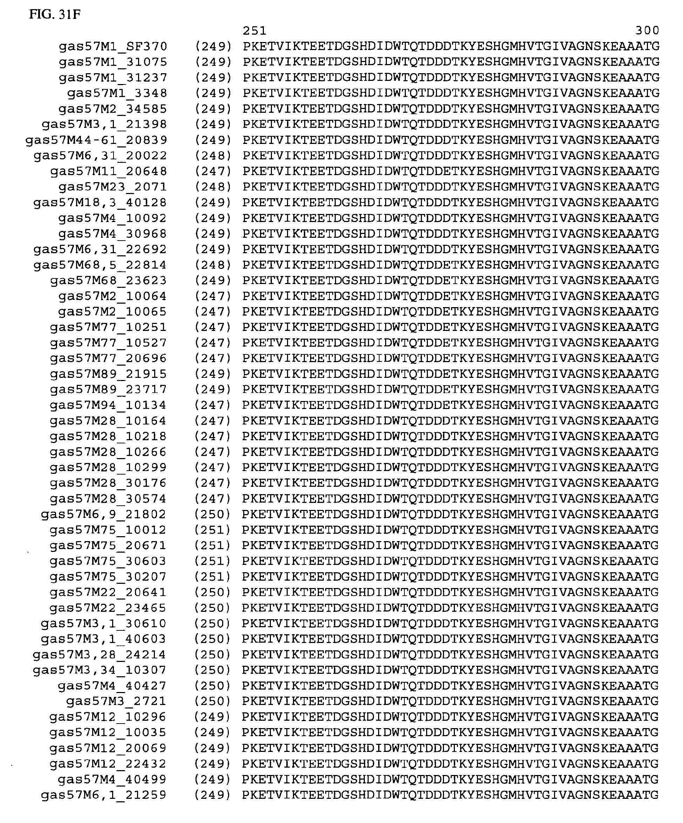

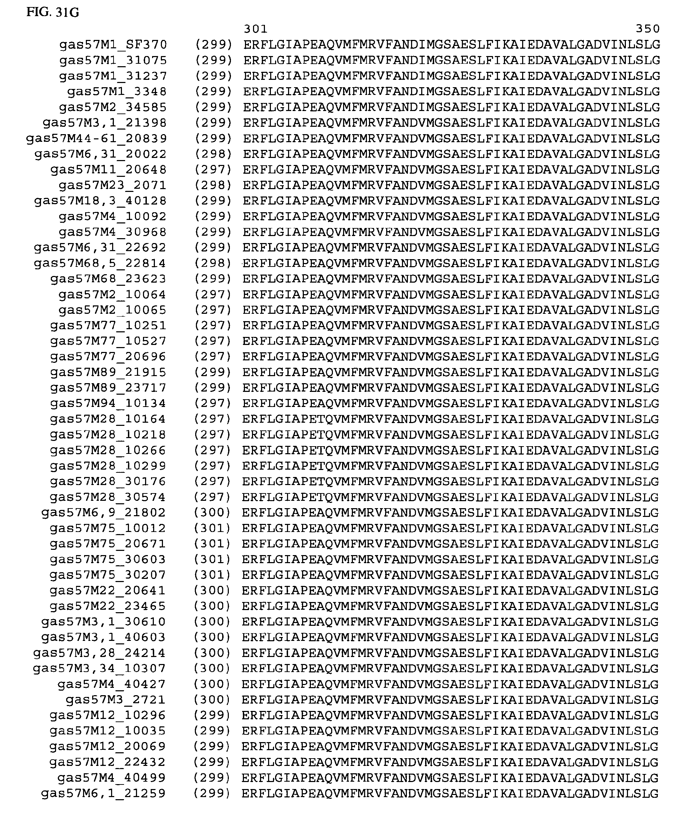

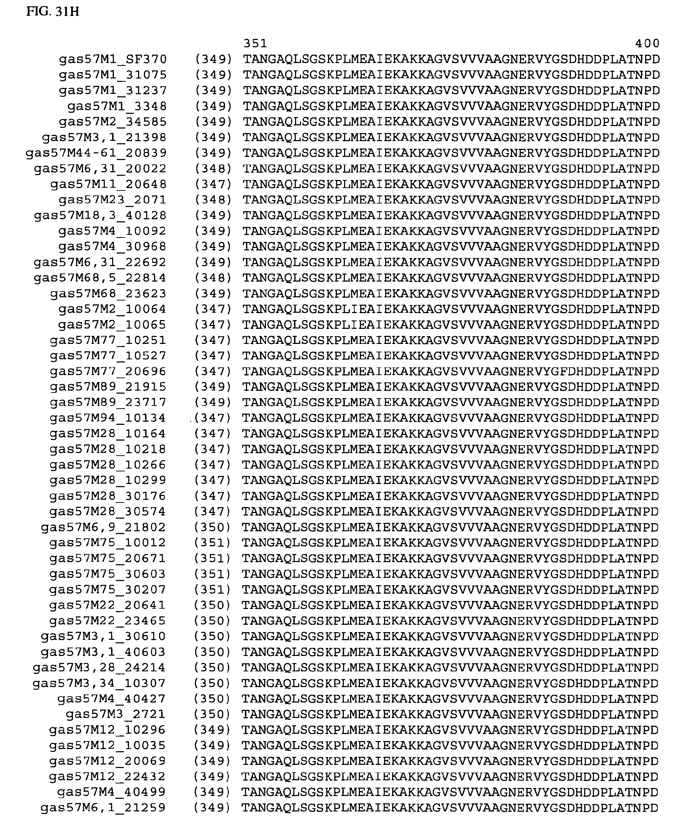

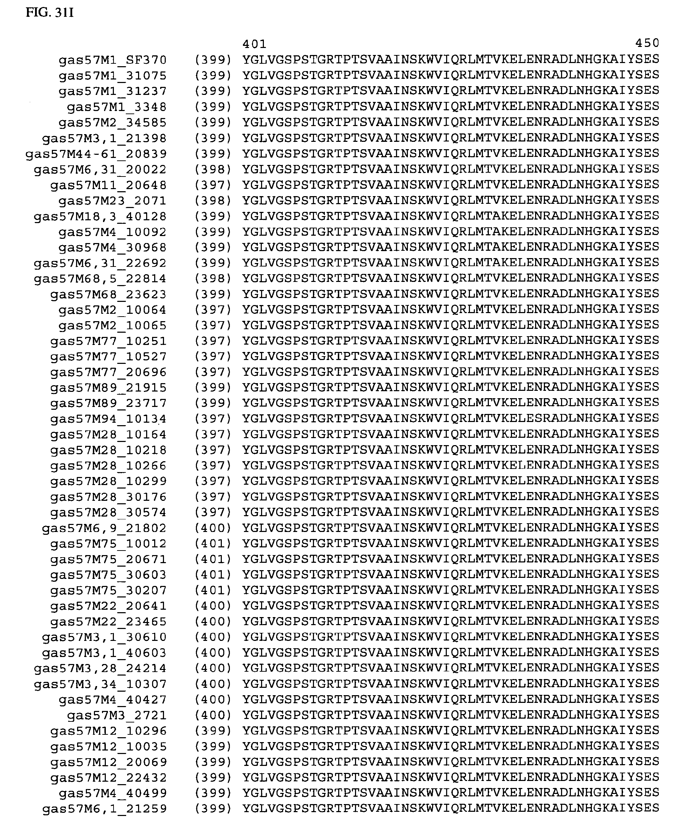

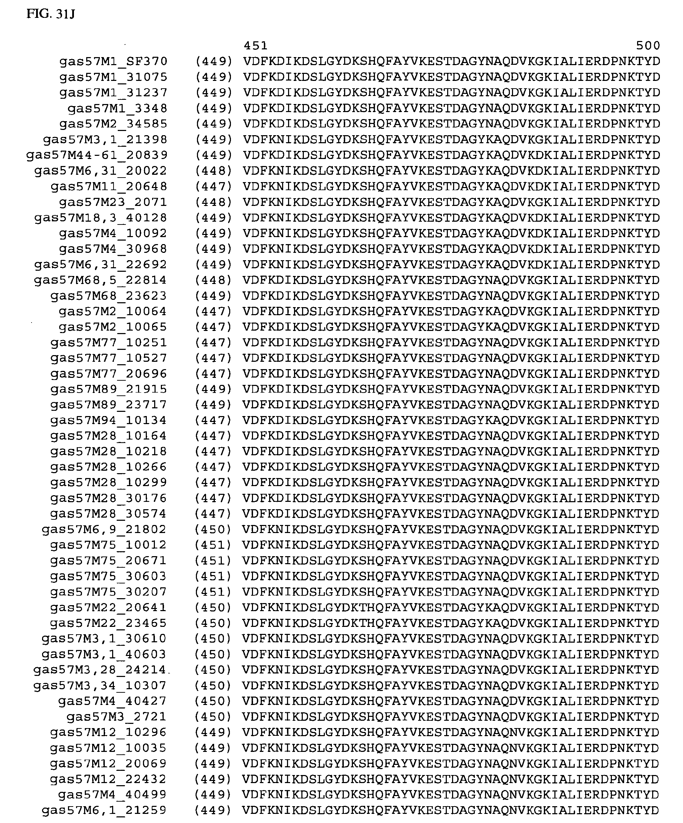

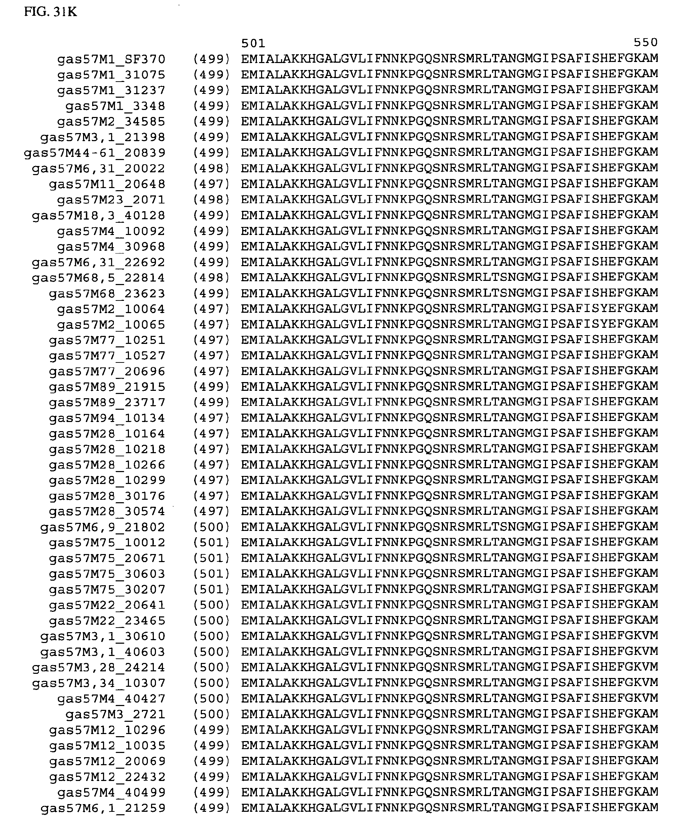

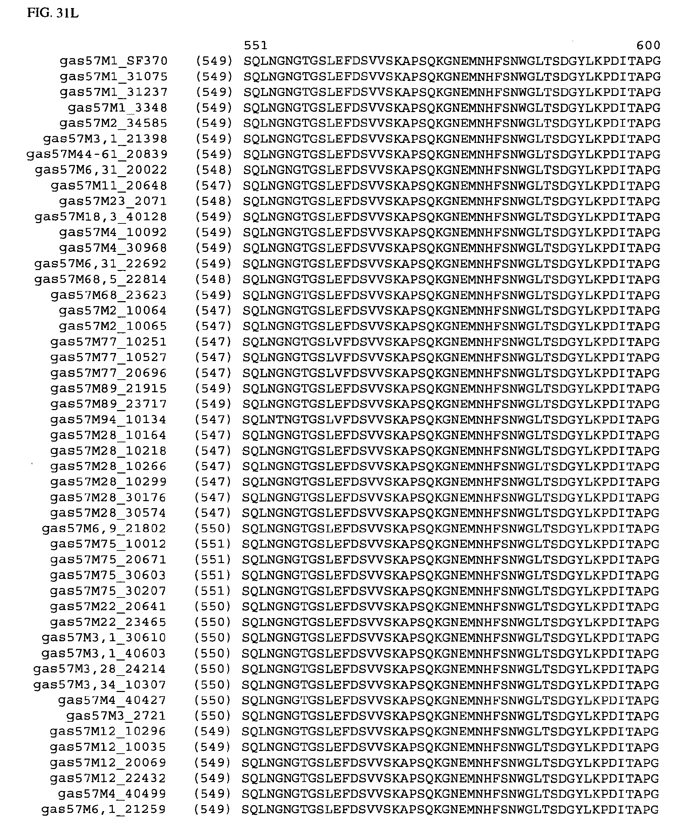

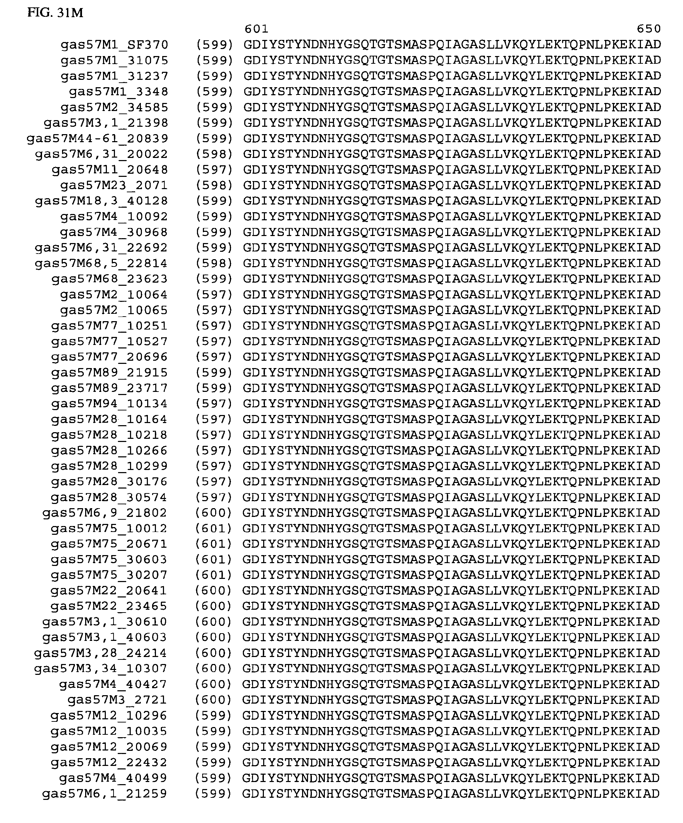

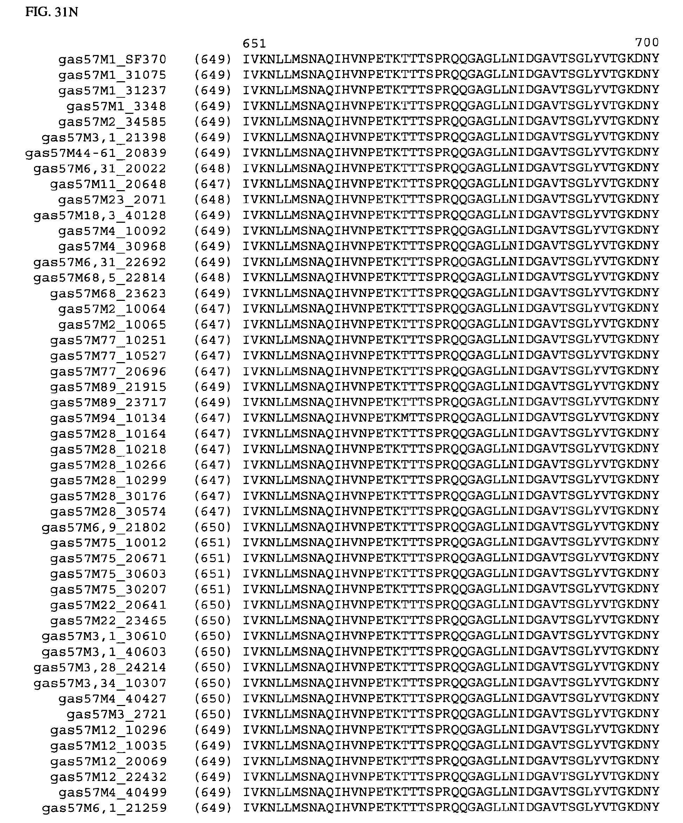

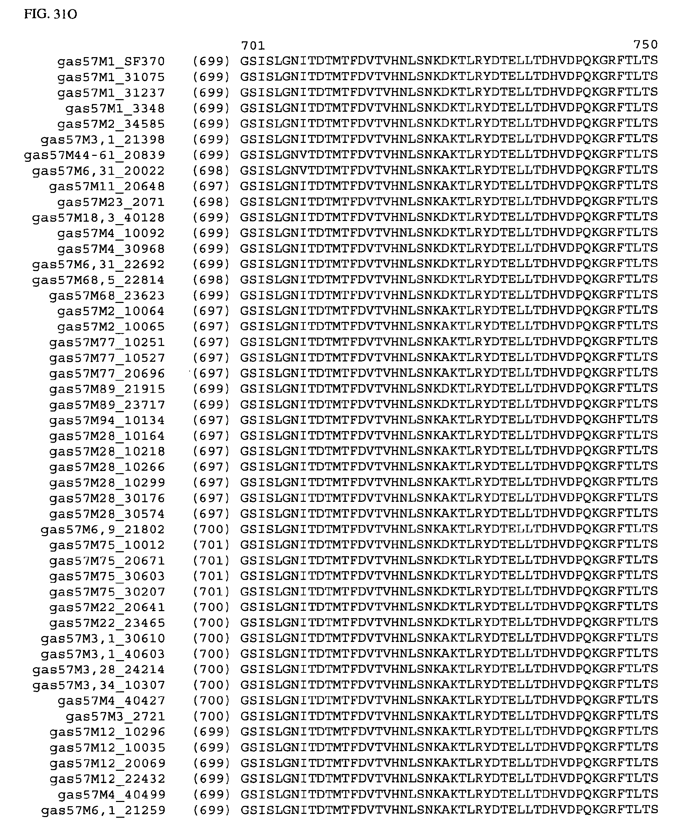

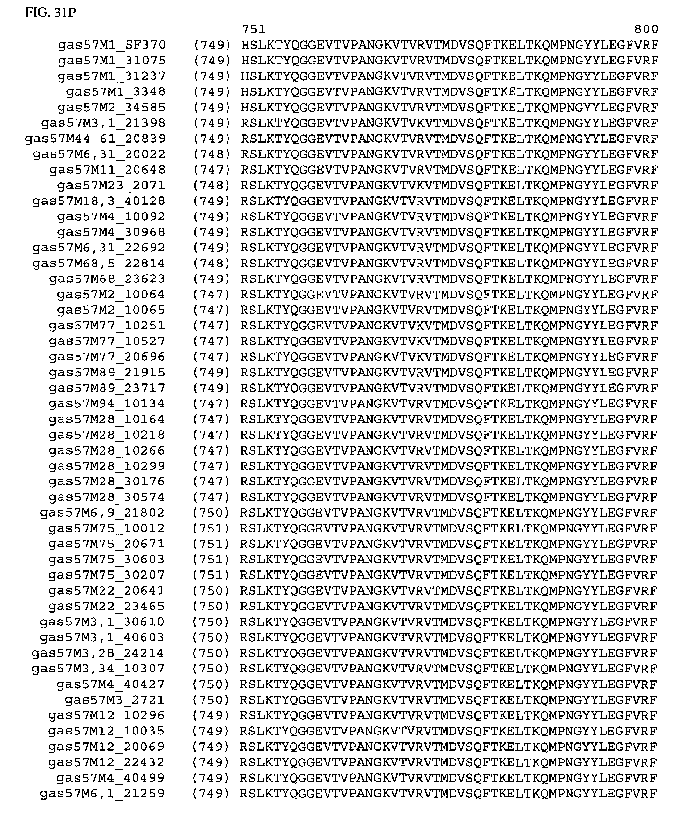

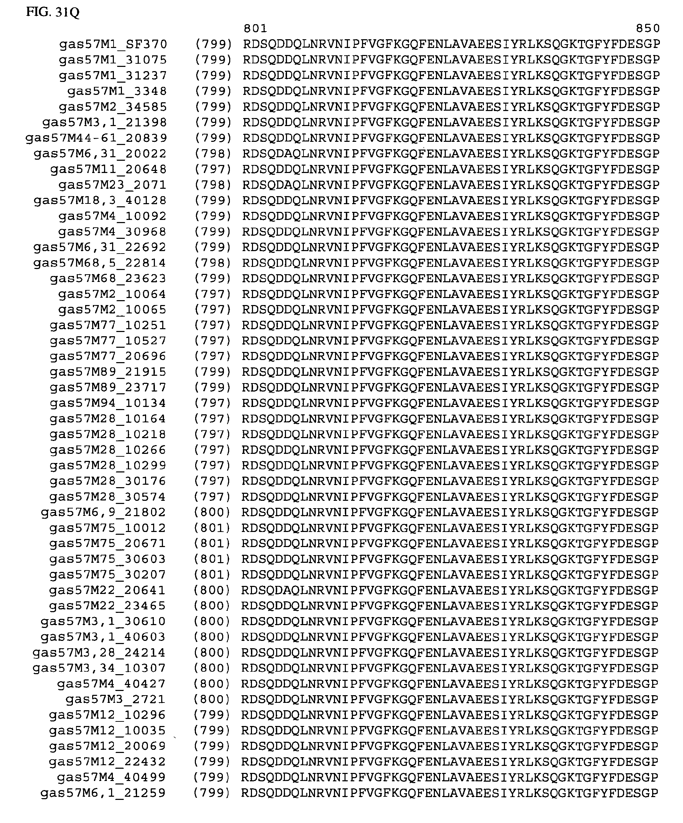

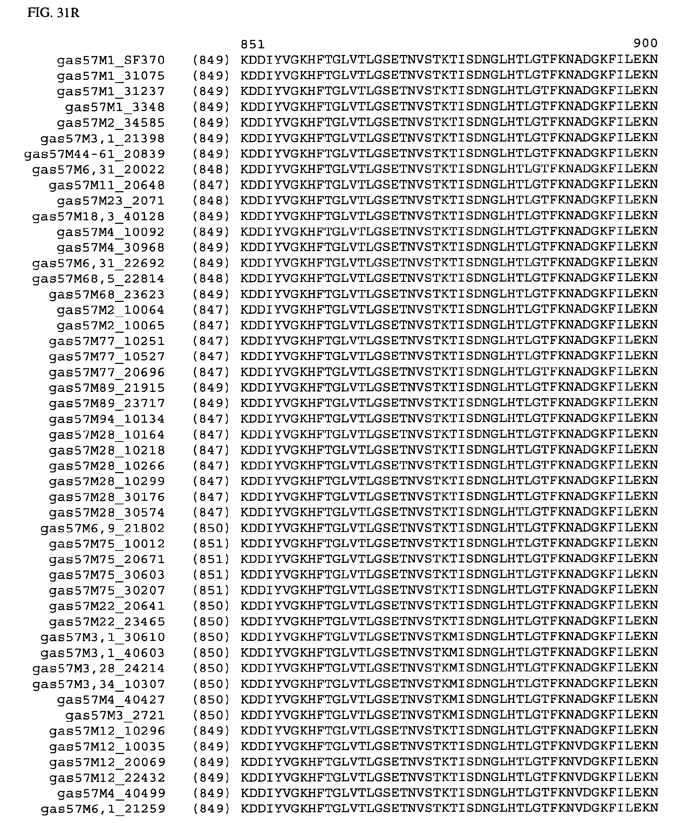

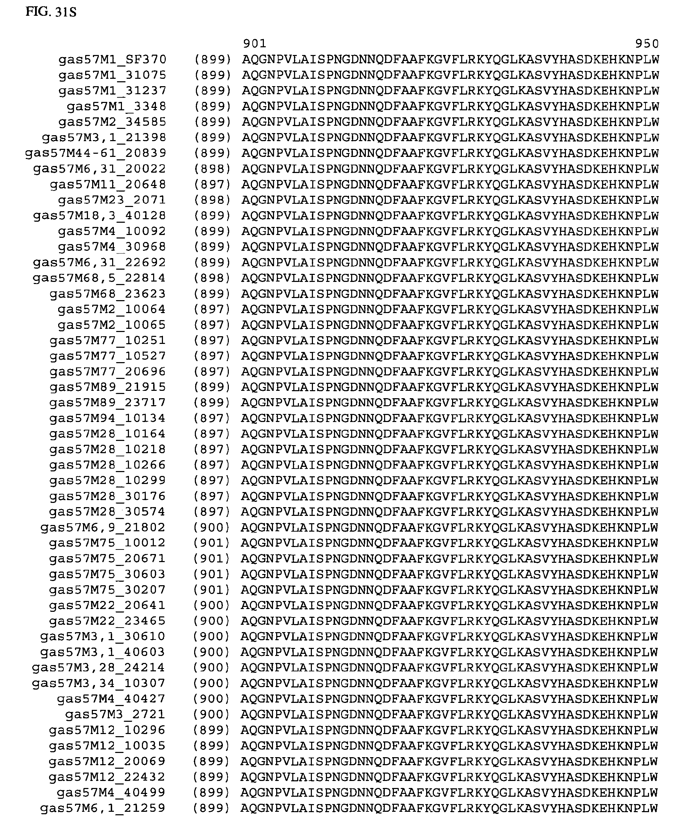

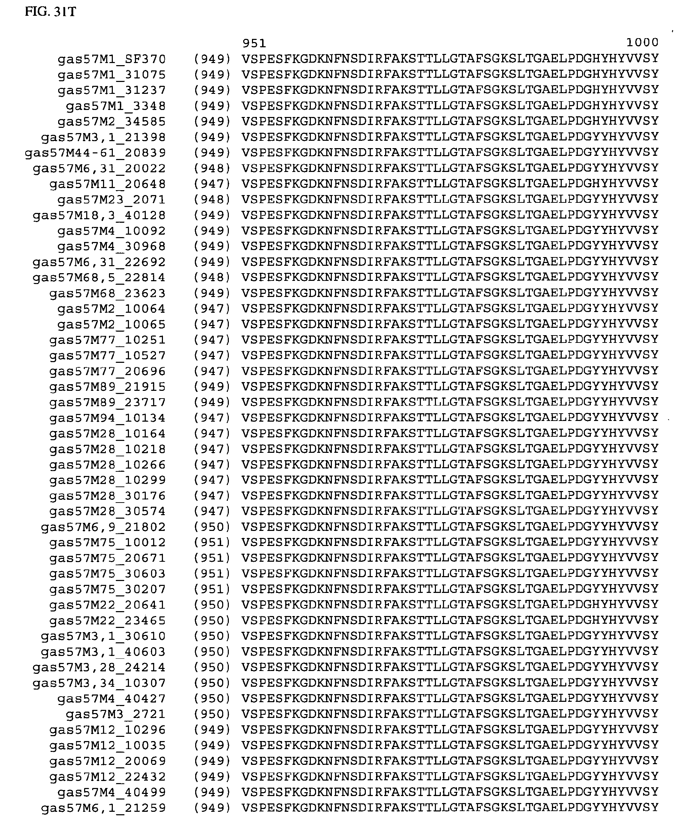

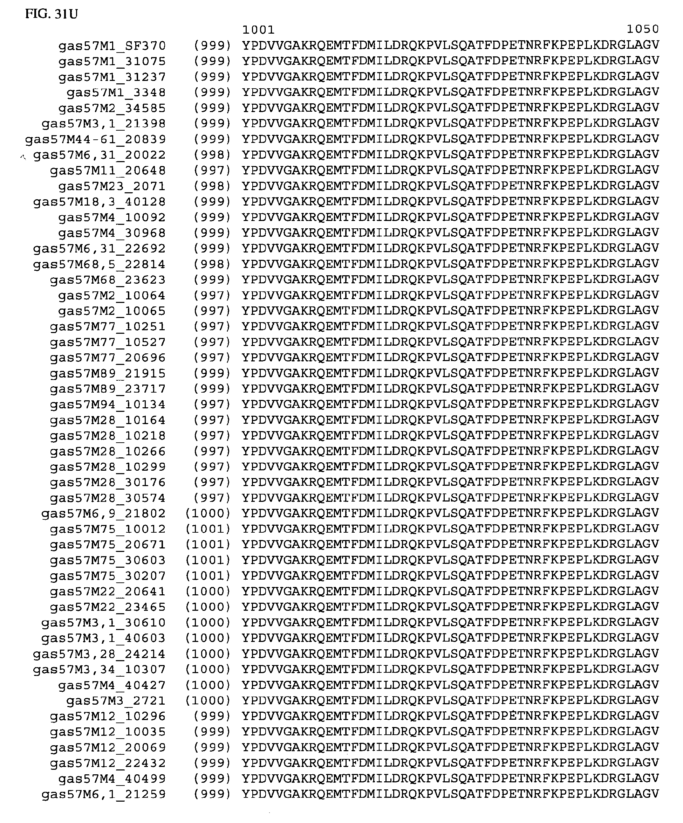

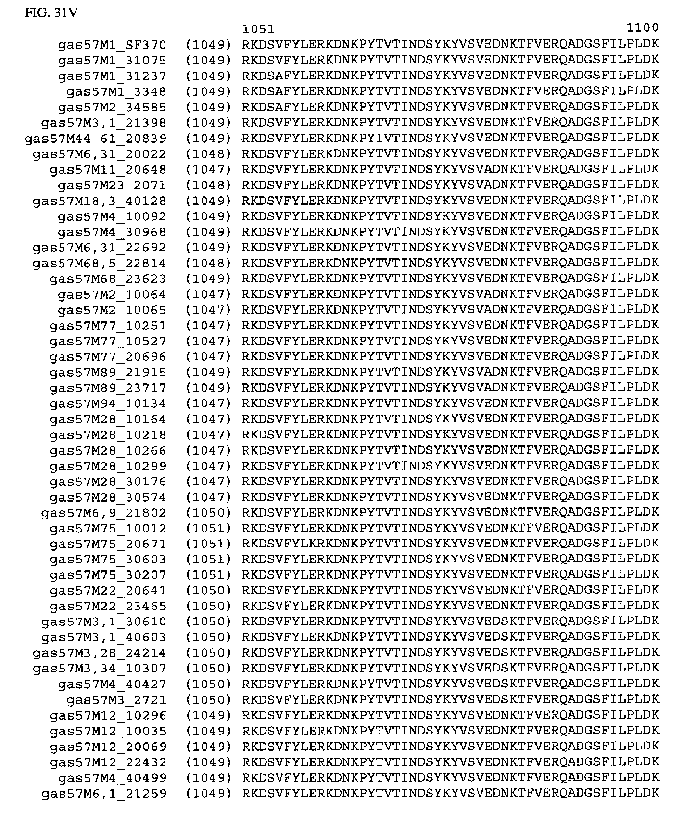

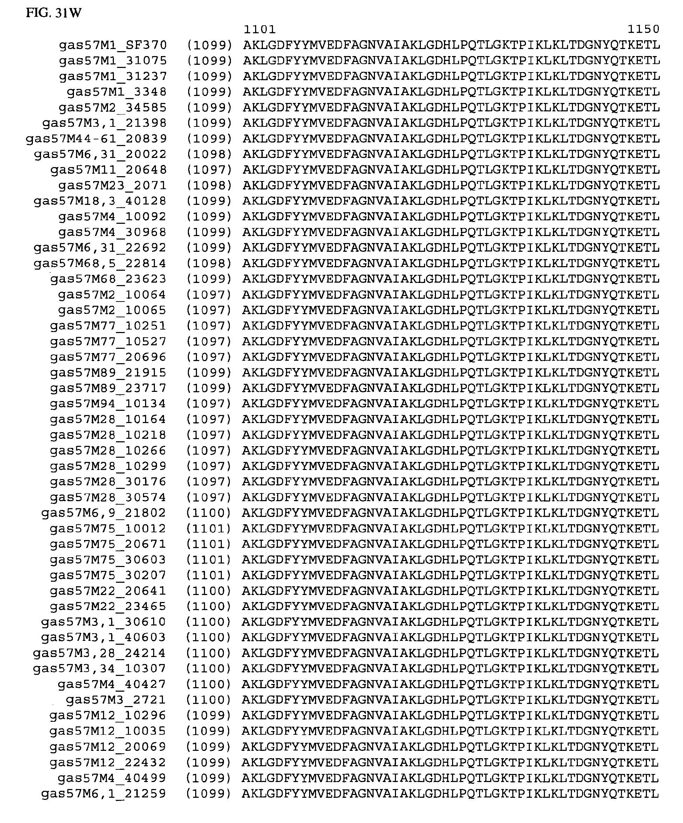

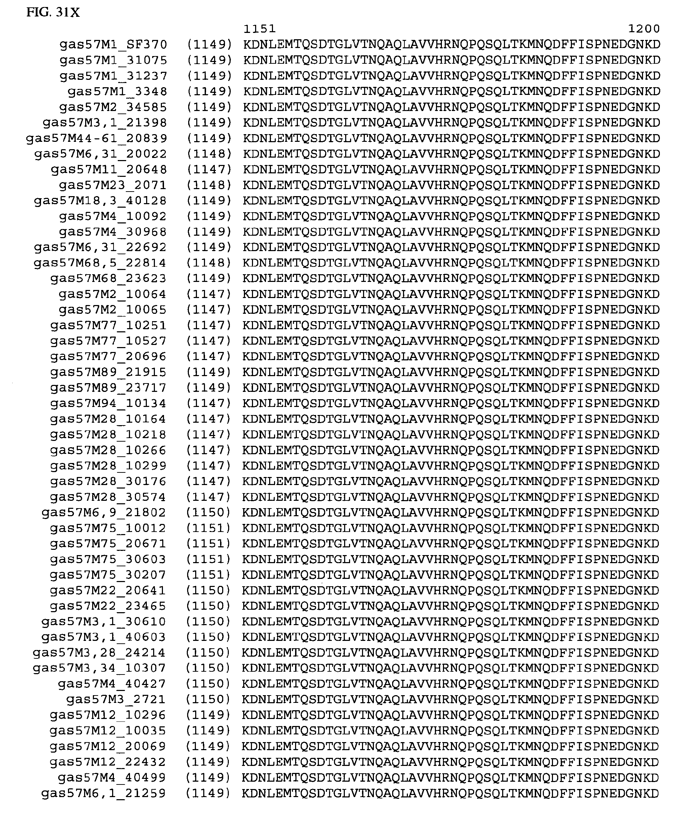

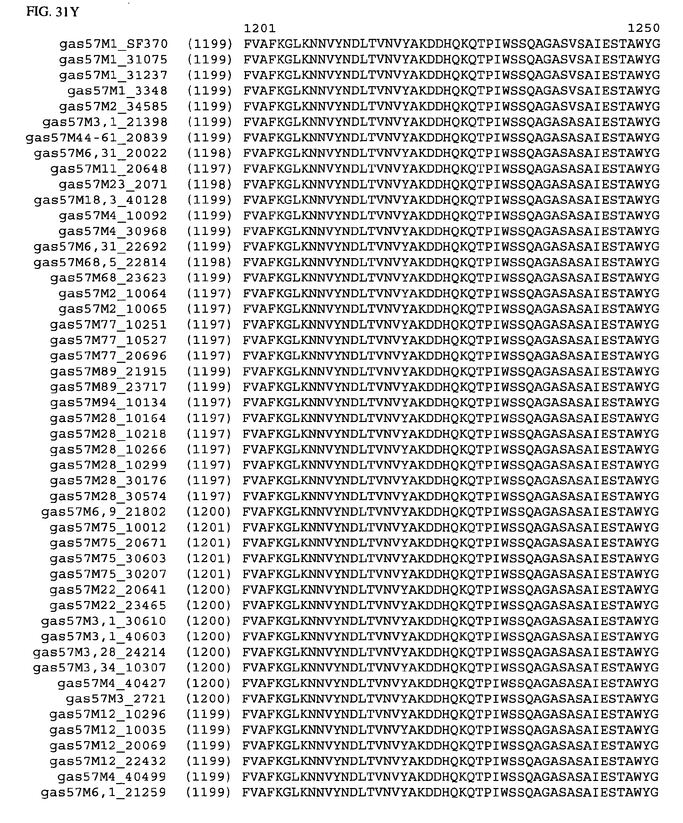

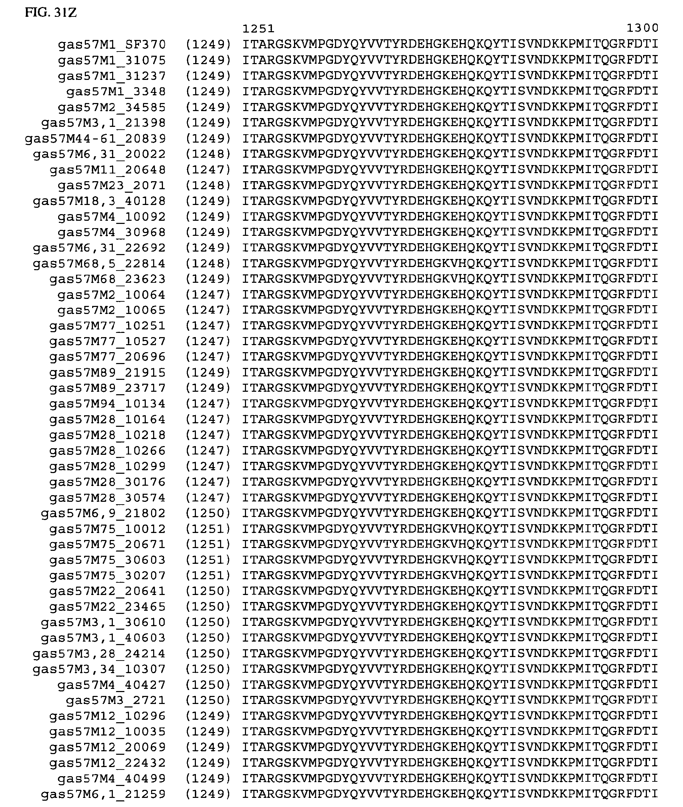

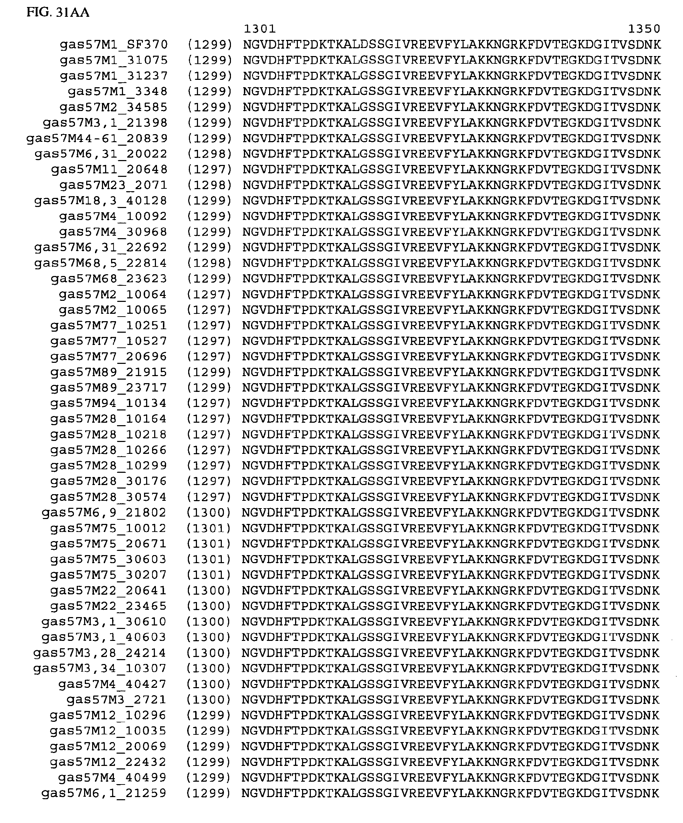

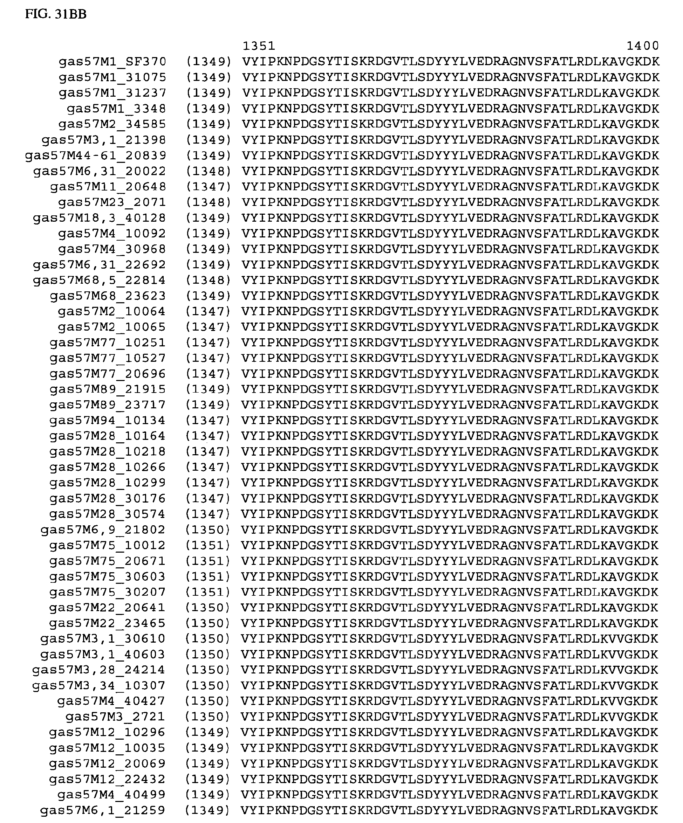

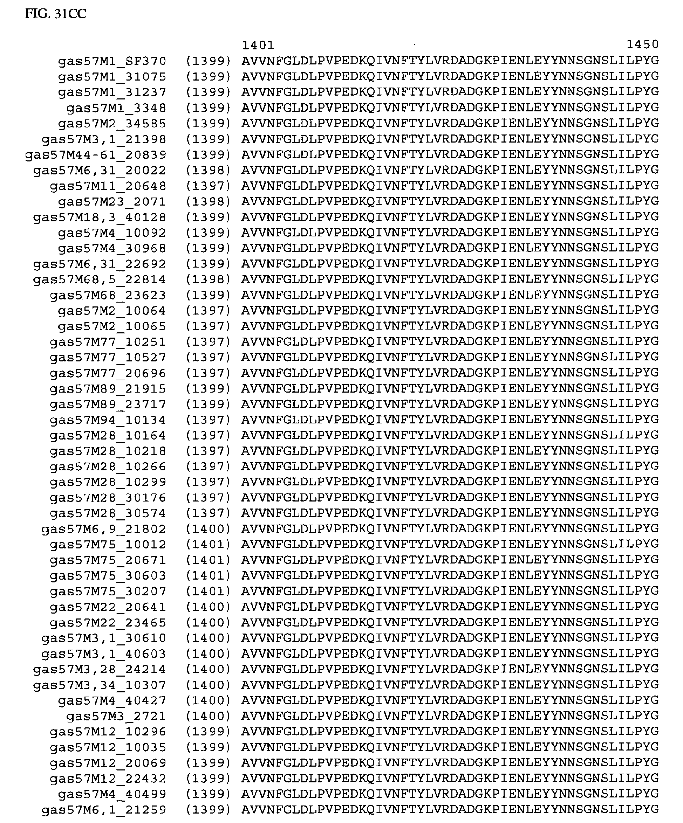

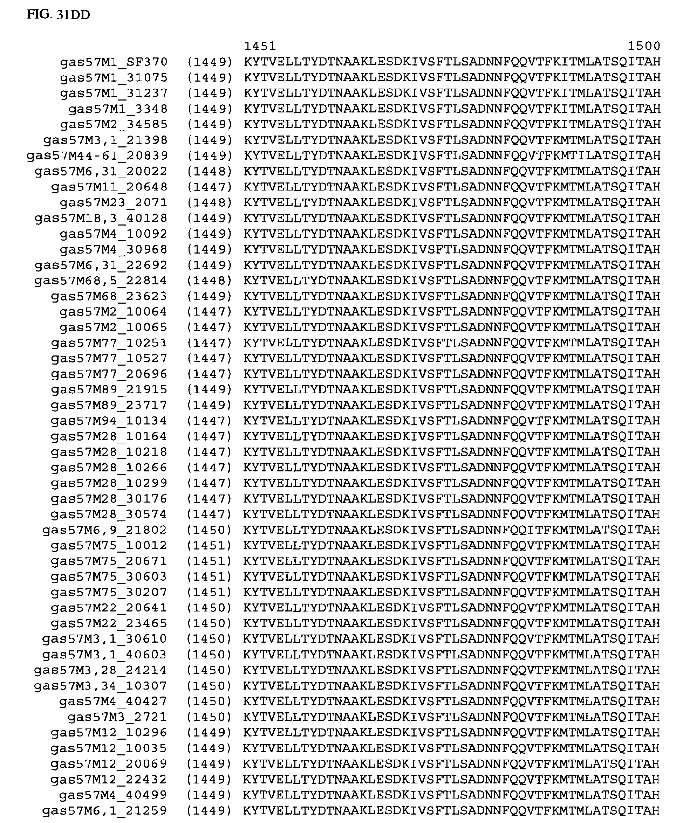

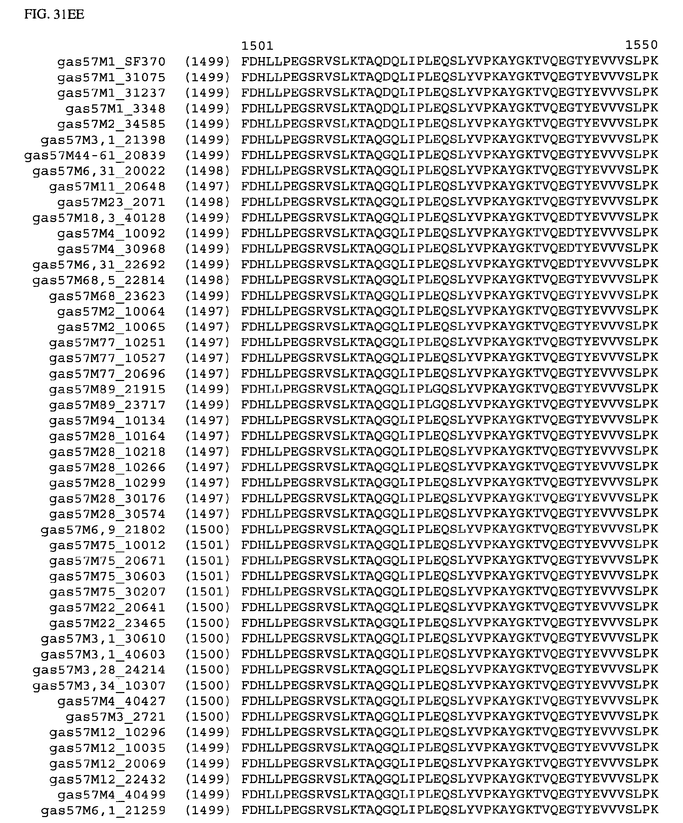

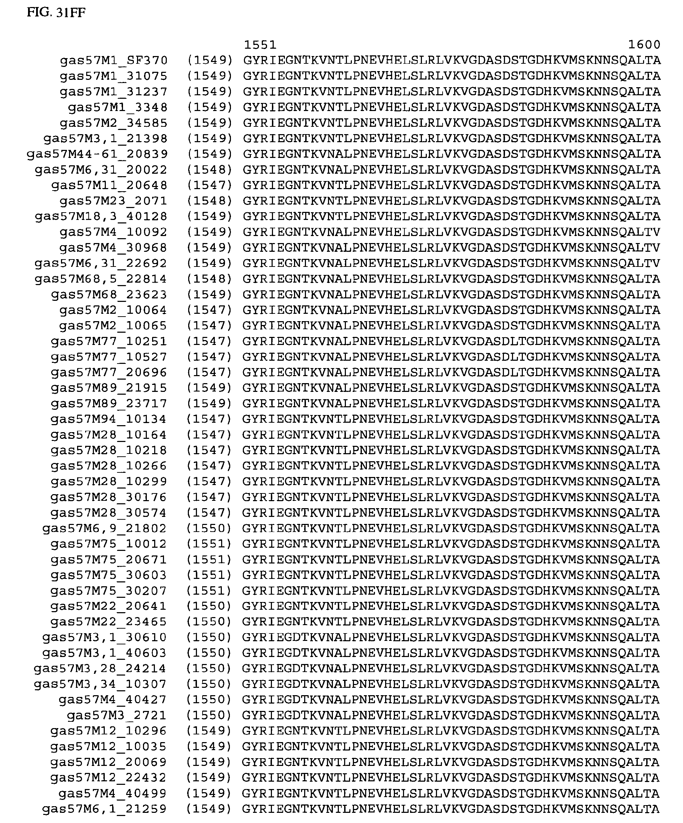

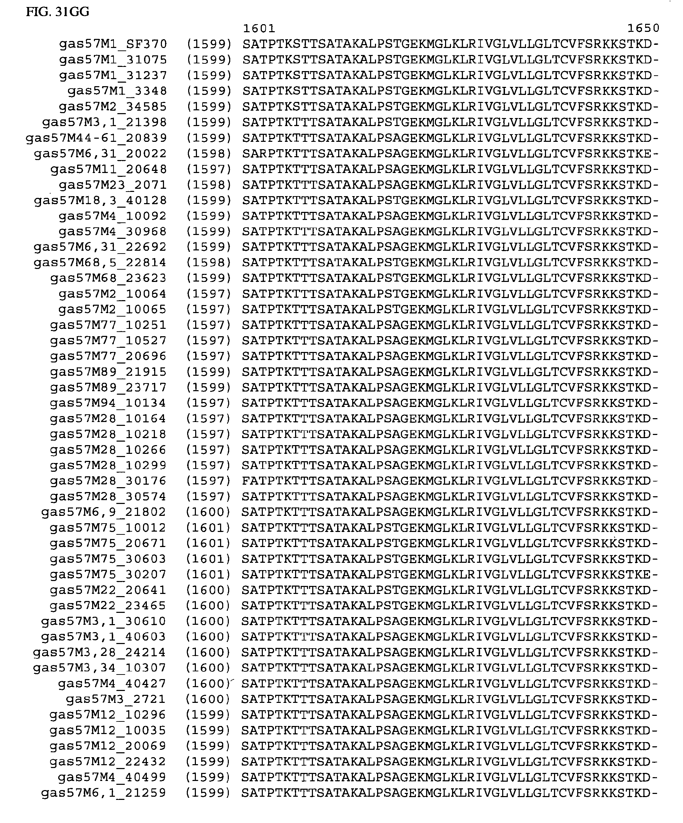

3**FIG. 31A-GG.** Alignments of Spy0416 ("gas57") antigens from different strains/M types. The catalytic triad (D, H, S) is in bold black characters. **FIG. 31A,** amino acids 1-50 (amino acid numbers at the top of each of FIGS. 10A-GG refers to the amino acid sequence of Spy0416M1_SF370, SEQ ID NO:1); **FIG. 31B**, amino acids 51-100; **FIG. 31C**, amino acids 101-150; **FIG. 31D**, amino acids 151-200; **FIG. 31E**, amino acids 201-250; **FIG. 31F**, amino acids 251-300; **FIG. 31G**, amino acids 301-350; **FIG. 31H**, amino acids 351-400, **FIG. 31I**, amino acids 401-450; **FIG. 31J**, amino acids 451-500; **FIG. 31K,** amino acids 501-550; **FIG. 31L**, amino acids 551-600; **FIG. 31M**, amino acids 601-650; **FIG. 31N**, amino acids 651-700; **FIG. 31O**, amino acids 701-750; **FIG. 31P**, amino acids 751-800; **FIG. 31Q**, amino acids 801-850; **FIG. 31R**, amino acids 851-900; **FIG. 31S**, amino acids 901-950; **FIG. 31T**, amino acids 951-1000; **FIG. 31U**, amino acids 1001-1050; **FIG. 31V**, amino acids 1051-1100; **FIG. 31W,** amino acids 1101-1150; **FIG. 31X**, amino acids 1151-1200; **FIG. 31Y**, amino acids 1201-1250; **FIG. 31Z**, amino acids 1251-1300; **FIG. 31AA**,

amino acids 1301-1350; **FIG. 31BB**, amino acids 1351-1400; **FIG. 31CC**, amino acids 1401-1450; **FIG. 31DD**, amino acids 1451-1500; **FIG. 31EE**, amino acids 1501-1550; **FIG. 31FF**, amino acids 1551-1600; **FIG. 31GG**, amino acids 1601-1650. M1_SF370, SEQ ID NO:1; M1_31075, SEQ ID NO:2; M1_31237, SEQ ID NO:3; M1_3348, SEQ ID NO:4; M2_34585, SEQ ID NO:5; M3,1_21398, SEQ ID NO:6; M44-61_20839, SEQ ID NO:7; M6,31_20022, SEQ ID NO:8; M11_20648, SEQ ID No:9; M23_2071, SEQ ID NO:10; M18,3_40128, SEQ ID NO:11; M4_10092, SEQ ID No:12; M4_30968, SEQ ID NO:13; M6,31_22692, SEQ ID NO:14; M68,5_22814, SEQ ID NO:15; M68_23623, SEQ ID NO:16; M2_10064, SEQ ID NO:17; M2_10065, SEQ ID NO:18; M77_10251, SEQ ID NO:19; M77_10527, SEQ ID NO:20; M77_20696, SEQ ID NO:21; M89_21915, SEQ ID NO:22; M89_23717, SEQ ID NO:23; M94_10134, SEQ ID NO:24; M28_10164, SEQ ID NO:25; M28_10218, SEQ ID NO:26; M29_10266, SEQ ID NO:27; M28_10299, SEQ ID NO:28; M28_30176, SEQ ID NO:29; M28_30574, SEQ ID NO:30; M6,9_21802, SEQ ID NO:31; M75_10012, SEQ ID NO:32; M75_20671, SEQ ID NO:33; M75_30603, SEQ ID NO:34; M75_30207, SEQ ID NO:35; M22_20641, SEQ ID NO:36; M22_23465, SEQ ID NO:37; M3,1_30610, SEQ ID NO:38; M3,1_40603, SEQ ID NO:39; M3,28_24214, SEQ ID N_:40; M3,34_10307, SEQ ID NO:41; M4_40427, SEQ ID NO:42; M3_2721, SEQ ID NO:43; M12_10296, SEQ ID No:44; M12_10035, SEQ ID NO:45; M12_20069, SEQ ID NO:46; M12_22432, SEQ ID NO:47; M4_40499, SEQ ID NO:48; and M6,1_21259, SEQ ID NO:49.

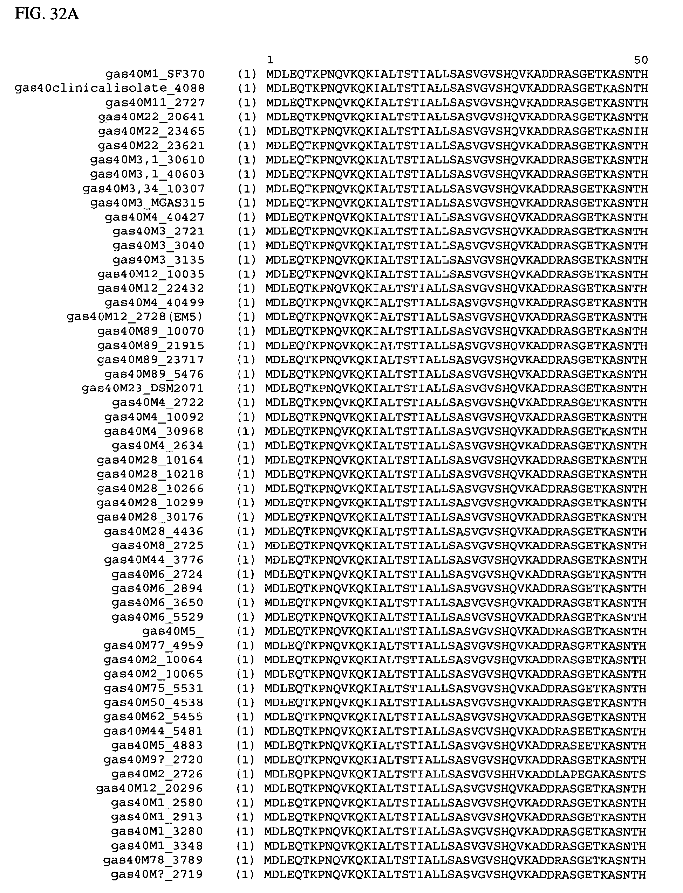

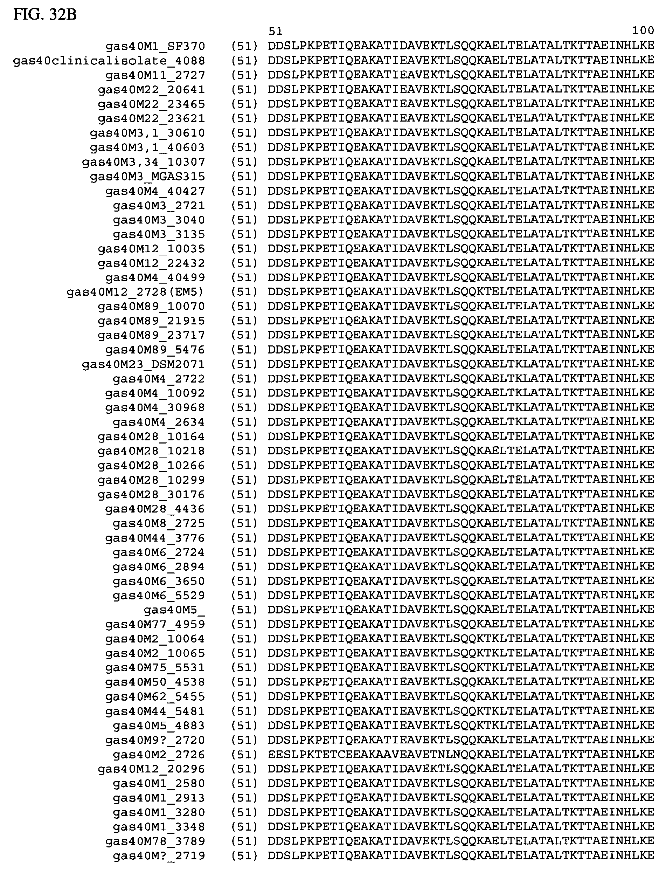

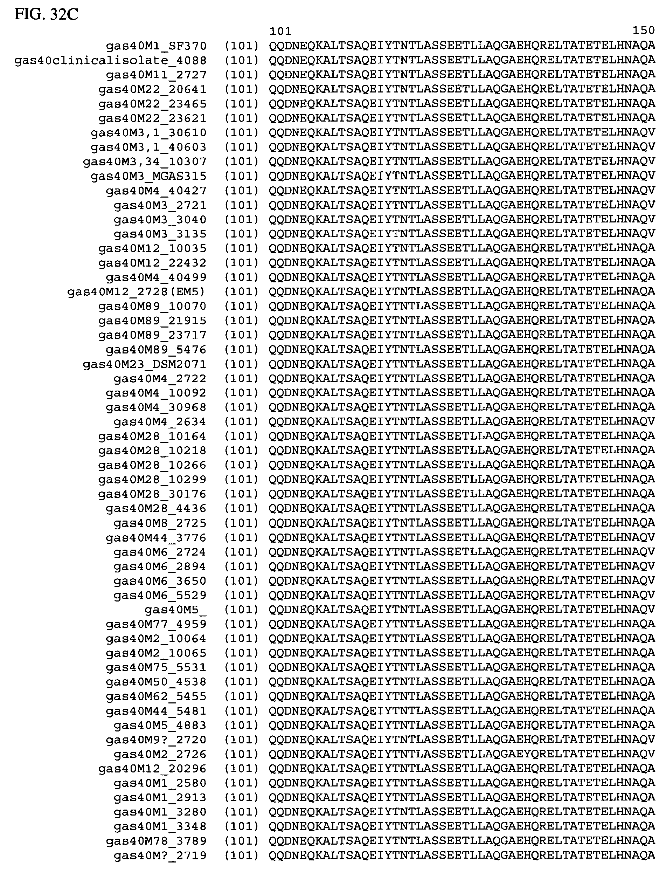

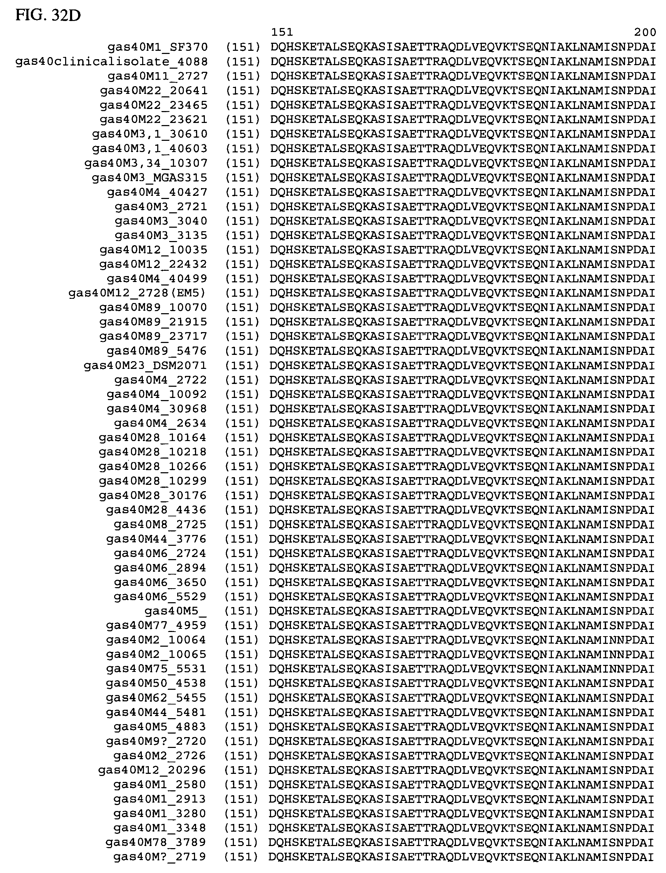

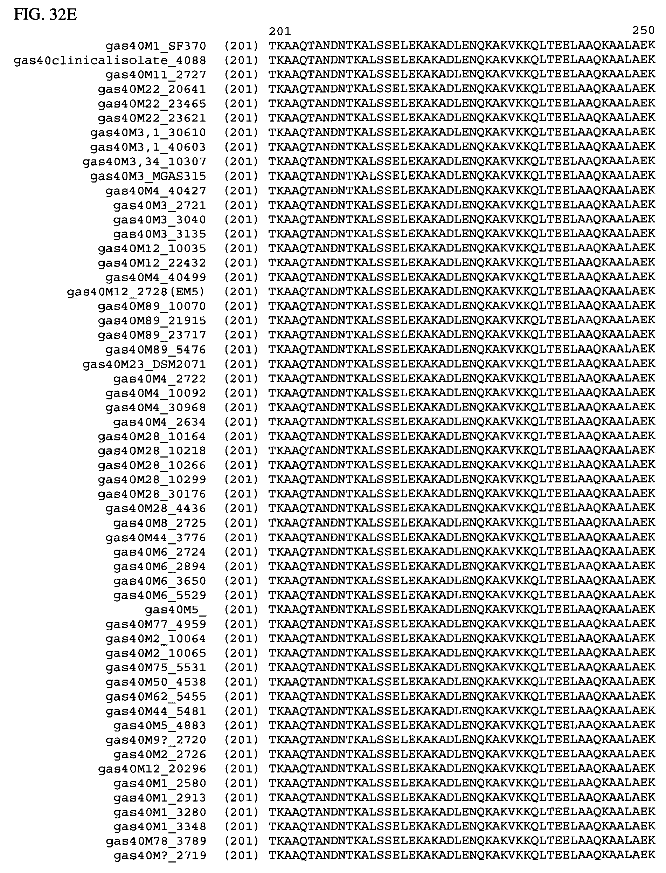

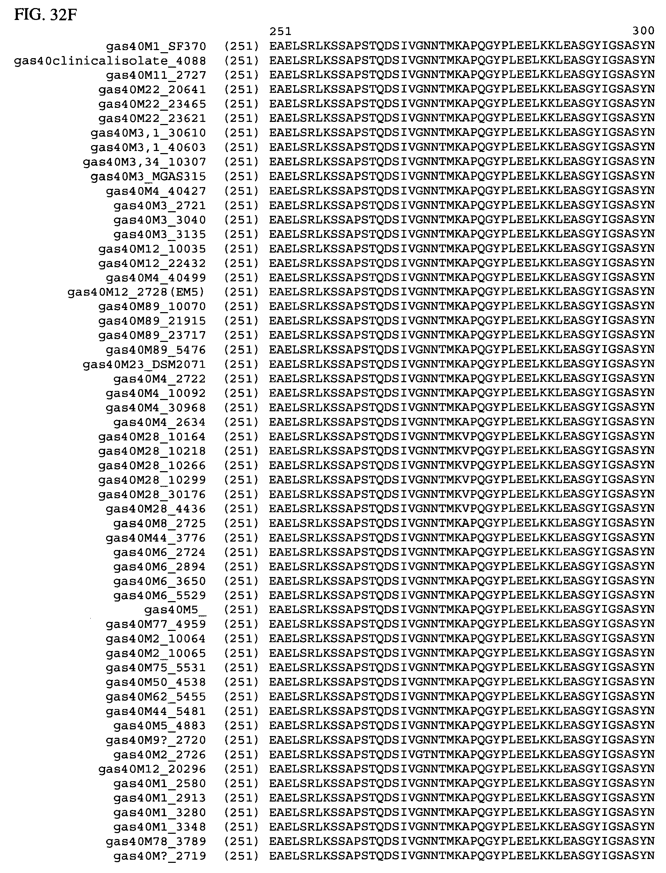

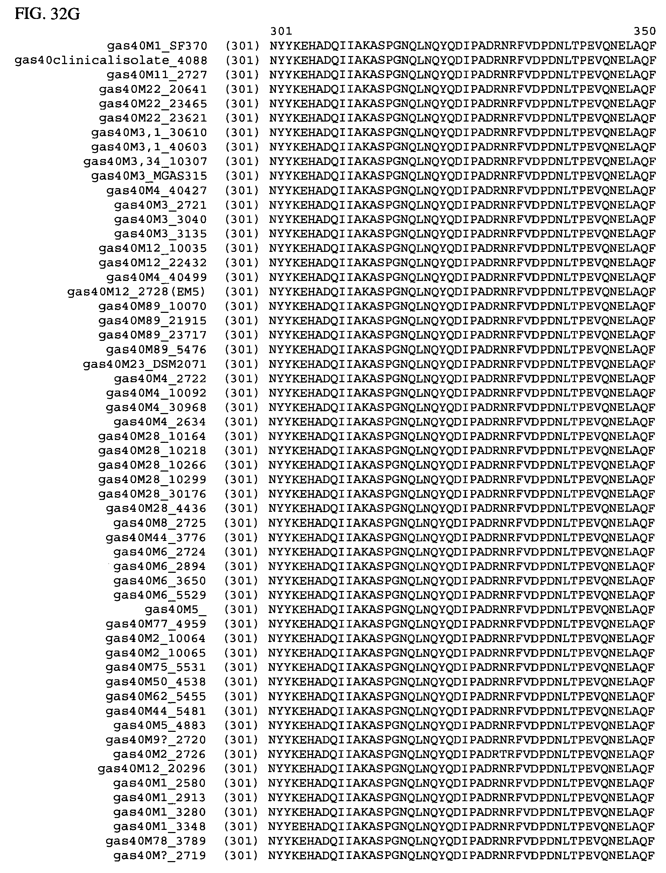

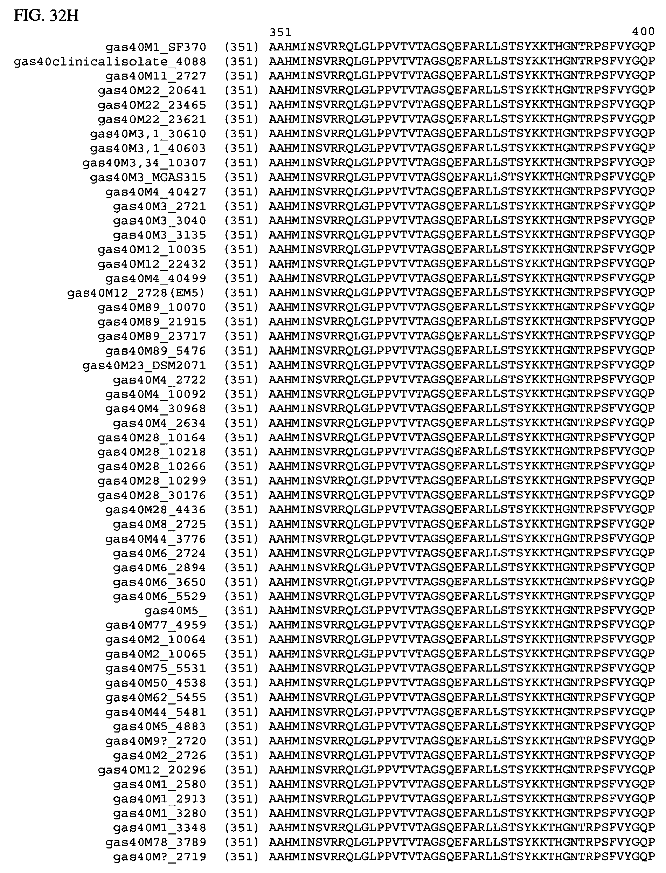

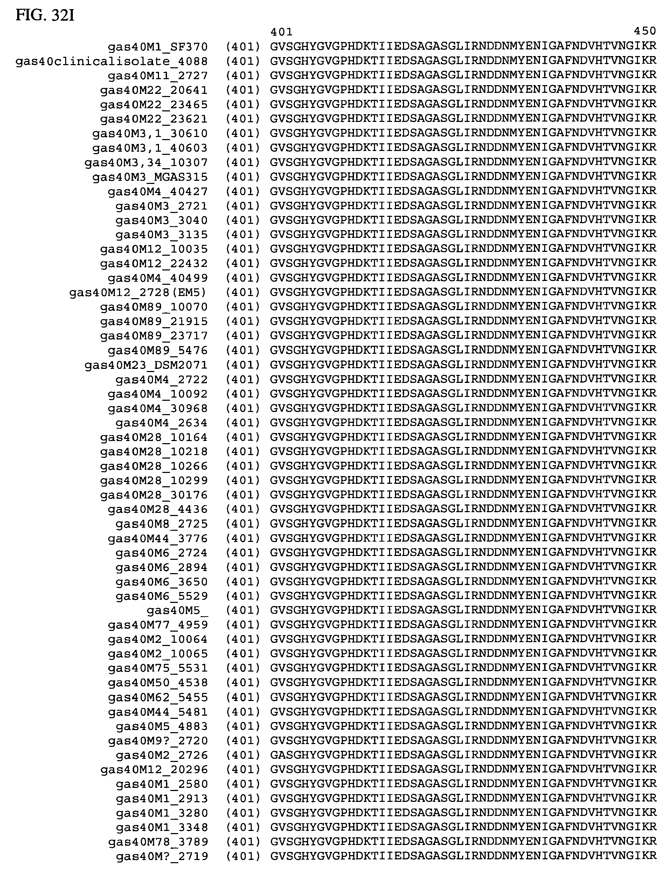

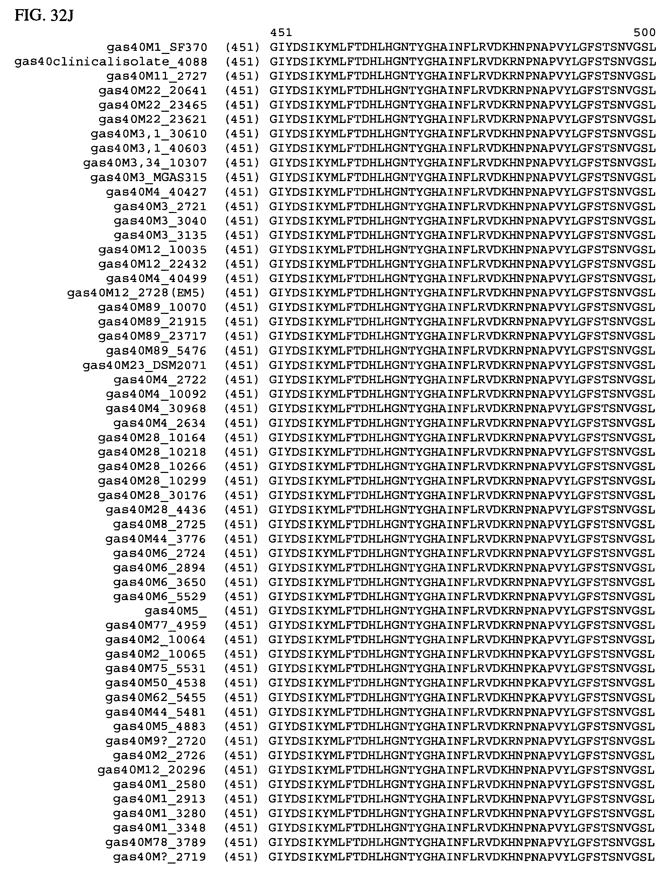

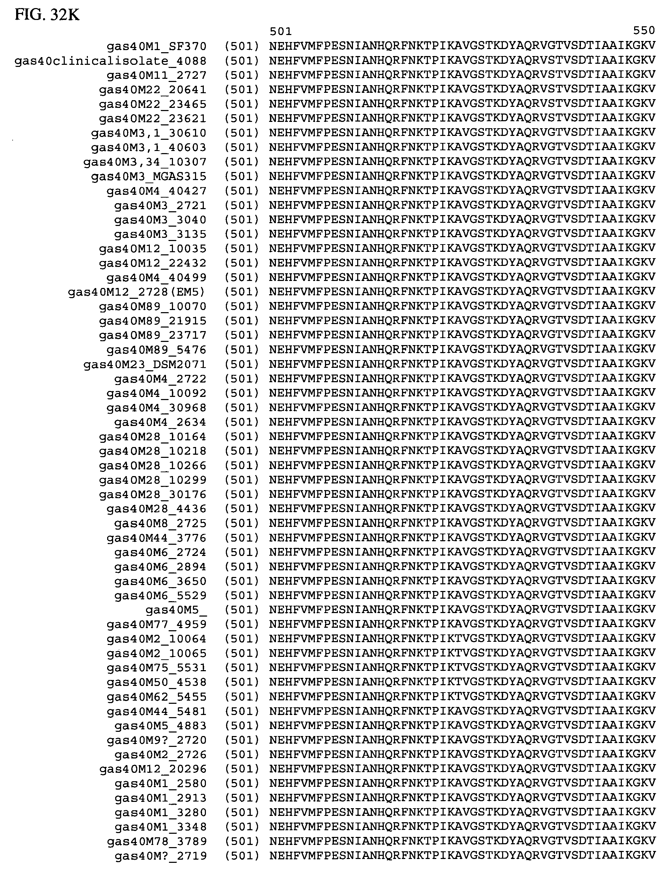

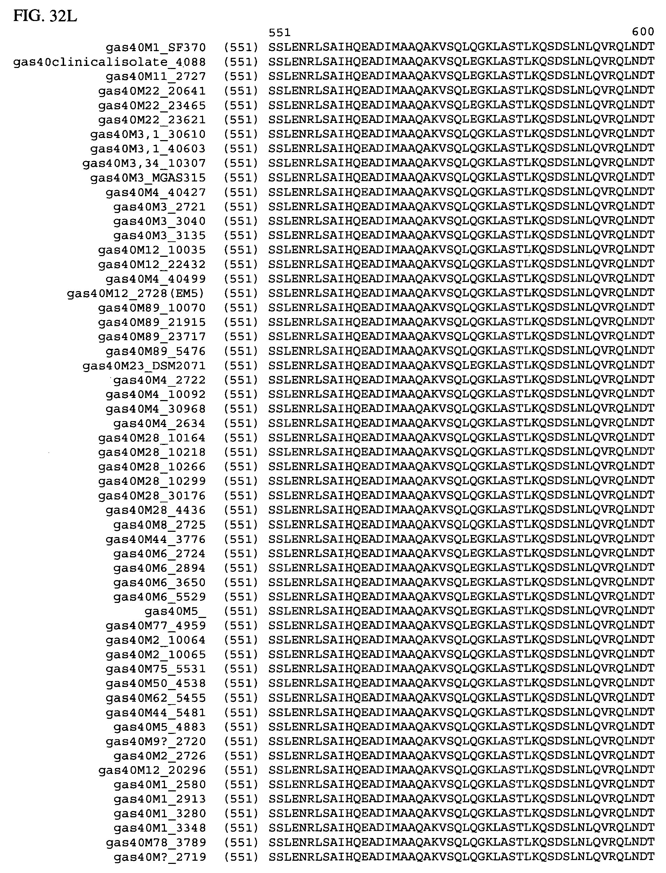

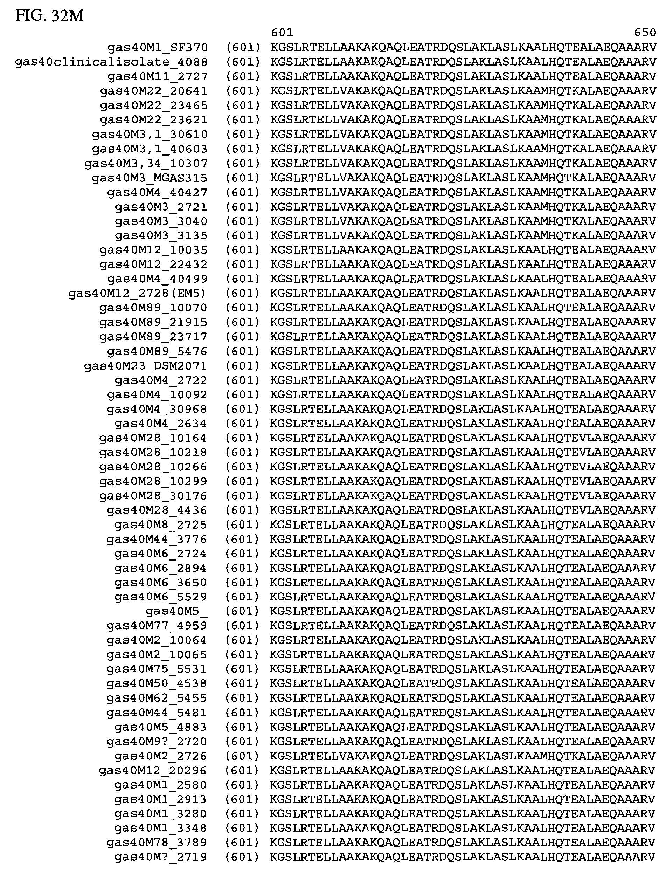

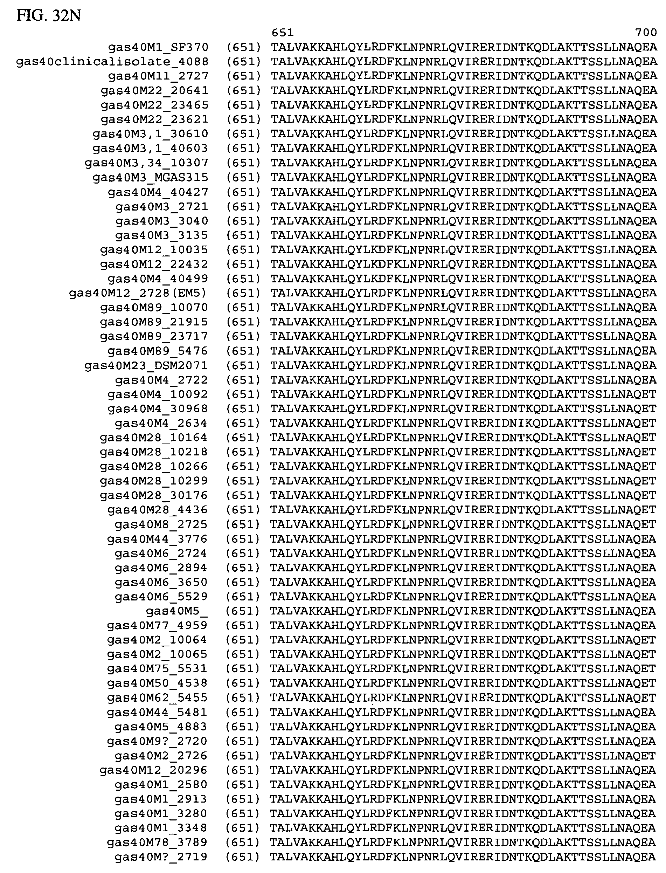

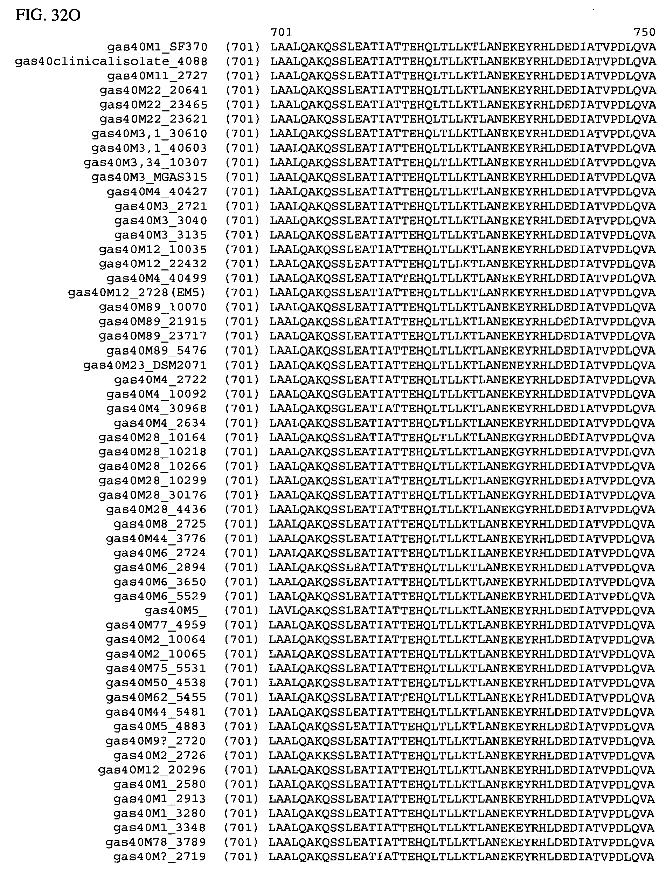

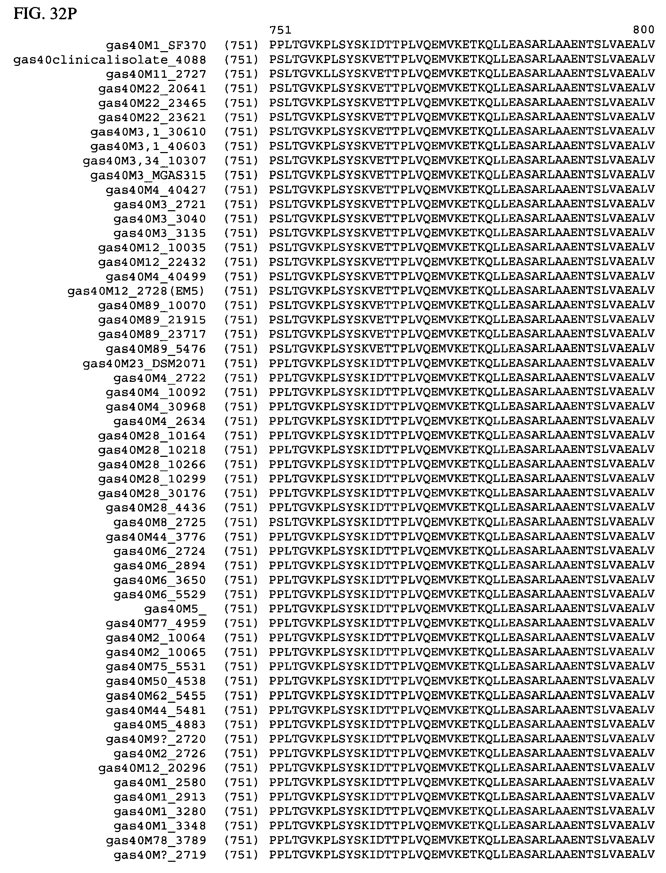

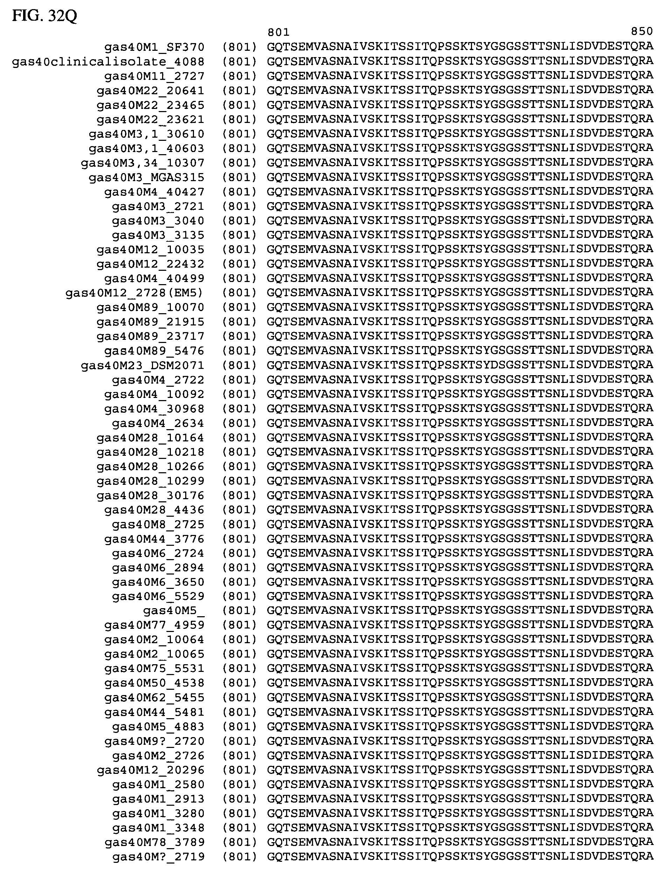

**FIG. 32A-GG.** Alignments of Spy0269 ("gas40") antigens from different strains/M types. **FIG. 32A**, amino acids 1-50 (amino acid numbers at the top of each of FIGS. 10A-GG refers to the amino acid sequence of Spy0269M1_SF370, SEQ ID NO:50); **FIG. 32B**, amino acids 51-100; **FIG. 32C**, amino acids 101-150; **FIG. 32D**, amino acids 151-200; **FIG. 32E**, amino acids 201-250; **FIG. 32F**, amino acids 251-300; **FIG. 32G**, amino acids 301-350; **FIG. 32H**, amino acids 351-400, **FIG. 32I**, amino acids 401-450; **FIG. 32J**, amino acids 451-500; **FIG. 32K**, amino acids 501-550; **FIG. 32L**, amino acids 551-600; **FIG. 32M**, amino acids 601-650; **FIG. 32N**, amino acids 651-700; **FIG. 32O**, amino acids 701-750; **FIG. 32P**, amino acids 751-800; **FIG. 32Q**, amino acids 801-850; **FIG**. **32R**, amino acids 851-874. M1_SF370, SEQ ID NO:50; clinicalisolate_40s88, SEQ ID NO:51; M11_2727, SEQ ID NO:52; M22_20641, SEQ ID NO:53; M22_23465, SEQ ID NO:54; M22_23621, SEQ ID NO:55; M3.1_30610, SEQ ID NO:56; M3.1_40603, SEQ ID NO:57; M3.34_10307, SEQ ID NO:58; M3_MGAS315, SEQ ID NO:59; M4_40427, SEQ ID NO:60; M3_2721, SEQ ID NO:61; M3_3040, SEQ ID NO:62; M3_3135, SEQ ID NO:63; M12_10035, SEQ ID NO:64; M12_22432, SEQ ID NO:65; M4_40499, SEQ ID NO:66; M78_3789, SEQ ID NO:67; M89_10070, SEQ ID NO:68; M89_21915, SEQ ID NO:69; M89_23717, SEQ ID NO:70; M89_5476, SEQ ID NO:71; M23_DSM2071, SEQ ID NO:72; M4_2722, SEQ ID NO:73; M4_10092, SEQ ID NO:74; M4_30968, SEQ ID NO:75; M4_2634, SEQ ID NO:76; M28_10164, SEQ ID NO:77; M28_10218, SEQ ID NO:78; M28_10266, SEQ ID NO:79; M28_10299, SEQ ID NO:80; M28_30176, SEQ ID NO:81; M28_4436, SEQ ID NO:82; M8-2725, SEQ ID NO:83; M44_3776, SEQ ID NO:84; M6_2724, SEQ ID NO:85; M6_2894, SEQ ID NO:86; M6_3650, SEQ ID NO:87; M6_5529, SEQ ID NO:88; M5, SEQ ID NO:89; M77_4959, SEQ ID NO:90; M2_10064, SEQ ID NO:91; M2_10065, SEQ ID NO:92; M75_5531, SEQ ID NO:93; M50_4538, SEQ ID NO:94; M62_5455, SEQ ID NO:95; M44_5481, SEQ ID NO:96; M5_4883, SEQ ID NO:97; M9?_2720, SEQ ID NO:98; M2_2726, SEQ ID NO:99; M12_20296, SEQ ID NO:100; M1_2580, SEQ ID NO:101; M1_2913, SEQ ID NO:102; M1_3280, SEQ ID NO:103; M1_3348, SEQ ID NO:104; M78_3789, SEQ ID NO:105; M?_2719, SEQ ID NO:106.

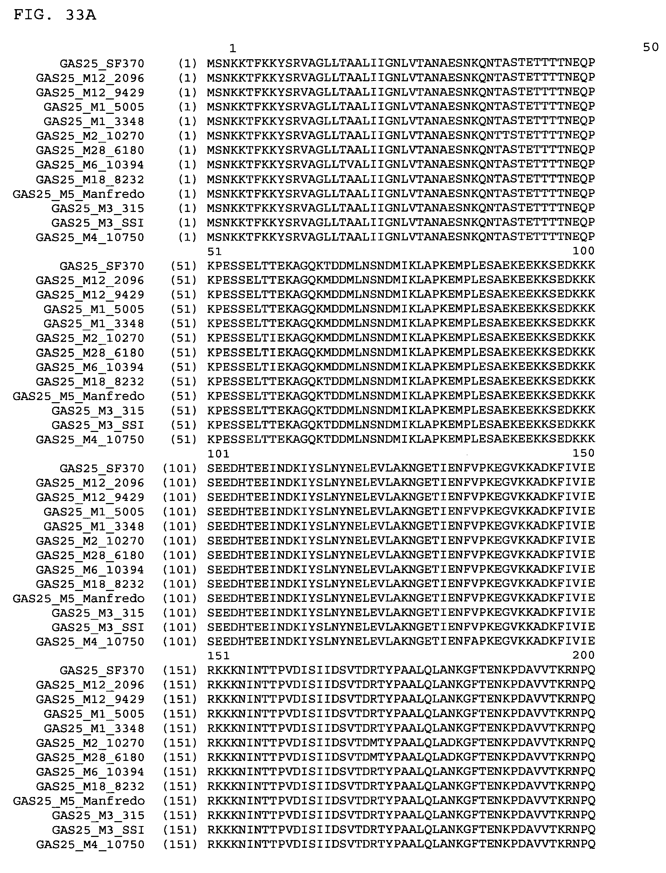

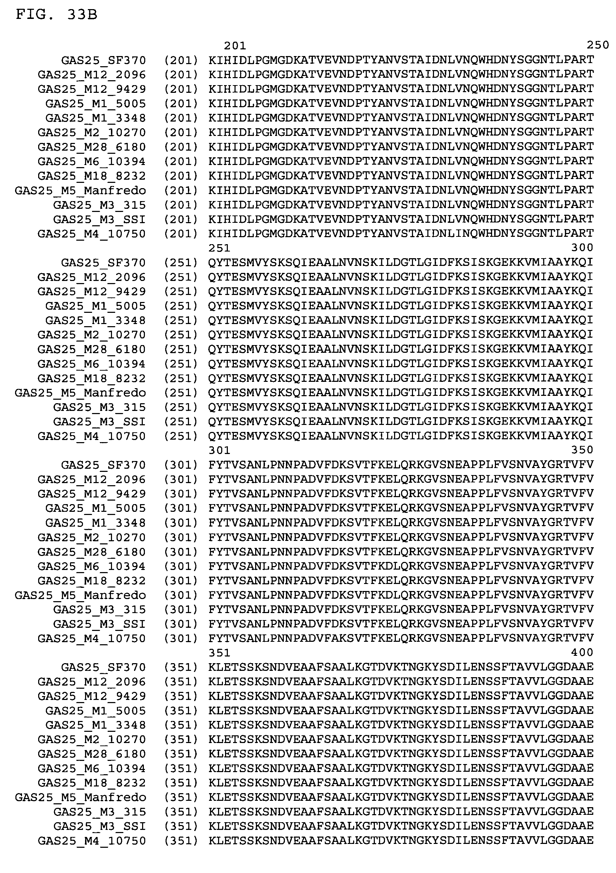

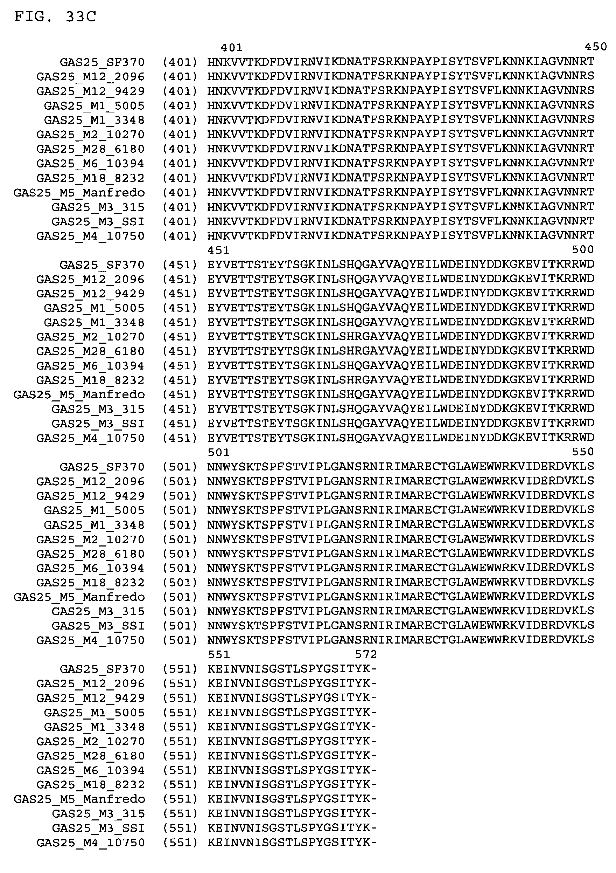

**FIG. 33A-C**. Alignments of Spy0167 ("gas25") antigens from different strains/M types. **FIG. 33A**, amino acids 1-150 (amino acid numbers at the top of each of FIGS. 10A-GG refers to the amino acid sequence of Spy0167M1-SF370, SEQ ID NO:107); **FIG. 33B**, amino acids 151-300; **FIG. 33C**, amino acids 301-500. M12_2096, SEQ ID NO:108; M12_9429, SEQ ID NO:109; M1_5005, SEQ ID NO:110; M1_3348, SEQ ID NO:111; M2_10270, SEQ ID NO:112; M28_6180, SEQ ID NO:13; M6_10394, SEQ ID NO:1 14; M18_8232, SEQ ID NO:115; M5_Manfredo, SEQ ID NO:116; M3_315, SEQ ID NO:117; M3_SSI, SEQ ID NO:119; M4_10750, SEQ ID NO:119.

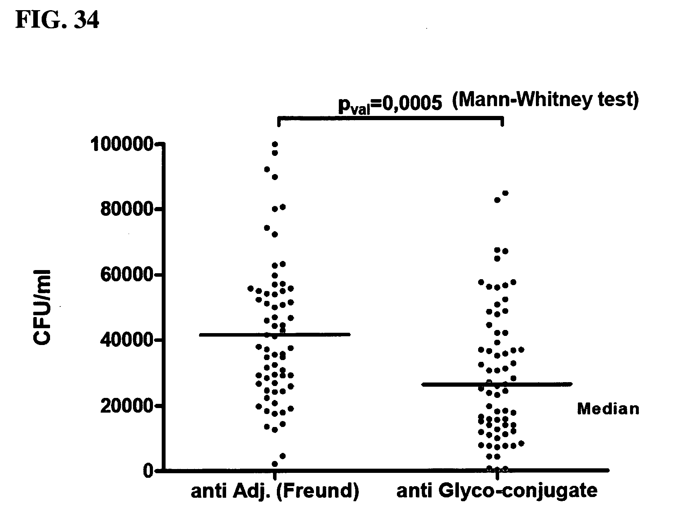

**FIG. 34**. Graph showing results of whole blood bactericidal assays demonstrating that anti-glycoconjugate (GC) antibodies mediate killing of *S. pyogenes*.

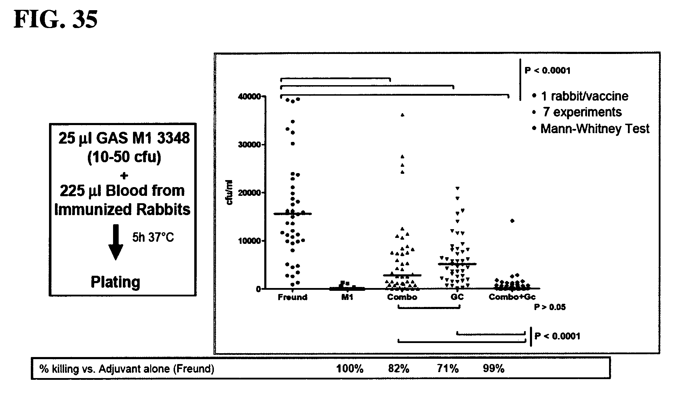

**FIG. 35**. Graph showing results of whole blood bactericidal assays demonstrating that the combination of anti-glycoconjugate (GC) antibodies and antibodies generated against GAS antigen combinations enhance killing of *S. pyogenes*. "Freund," Freund's adjuvant; "M1," *S. pyogenes* M1 protein; "COMBO," Spy0416 mutant D151A/S617A, Spy0167 mutant P427L/W535F5, and wild-type Spy0269; "GC," GAS polysaccharide antigen conjugated to CRM 197.

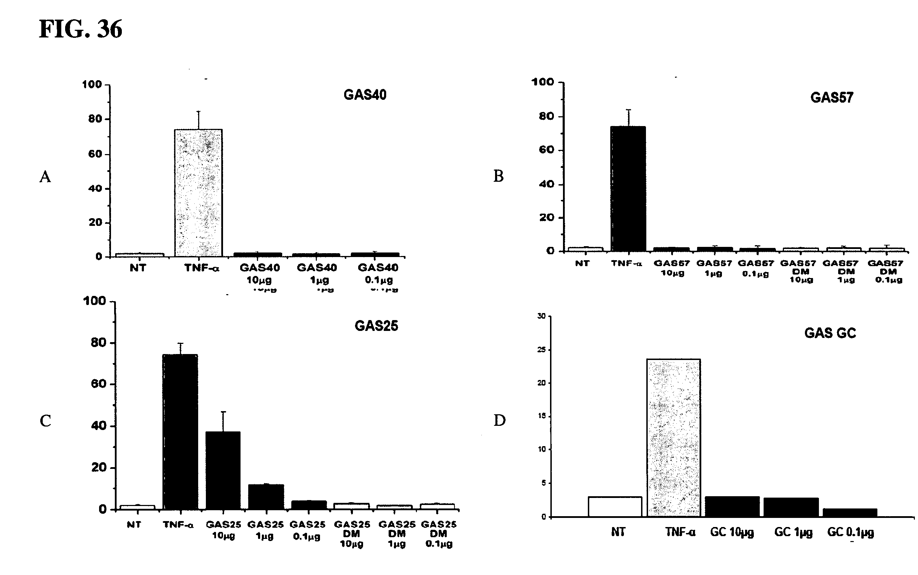

**FIGS. 36A-D**. Graphs showing results of cellular toxicity assays comparing various antigens with positive (tumor necrosis factor $\alpha$, TNF-$\alpha$) and negative (NT, not treated) controls. **FIG. 36A**, Spy0269 (GAS40, SEQ ID NO:177); **FIG. 36B**, Spy0416 (GAS57) and GAS57 mutant D151A/S617A (GAS57 DM, SEQ ID NO:198); **FIG. 36C**, Spy0167

(GAS25) and GAS25 mutant P427L/W535F (GAS25 DM, SEQ ID NO:125); **FIG. 36D**, glycoconjugate (GC).

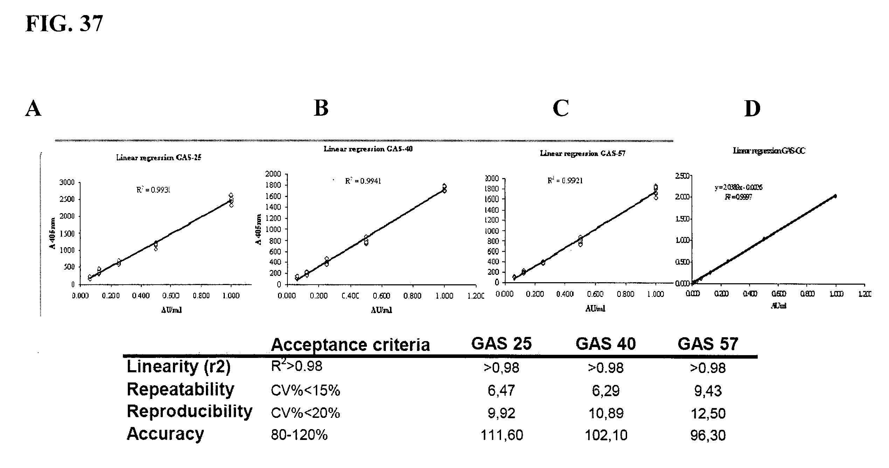

] **FIGS. 37A-D**. Graphs demonstrating validation of ELISA assay. **FIG. 37A**, Spy0167 (GAS25); **FIG. 37B**, Spy0269 (GAS40); **FIG. 37C**, Spy0416 (GAS57); **FIG. 37D**, glycoconjugate (GC).

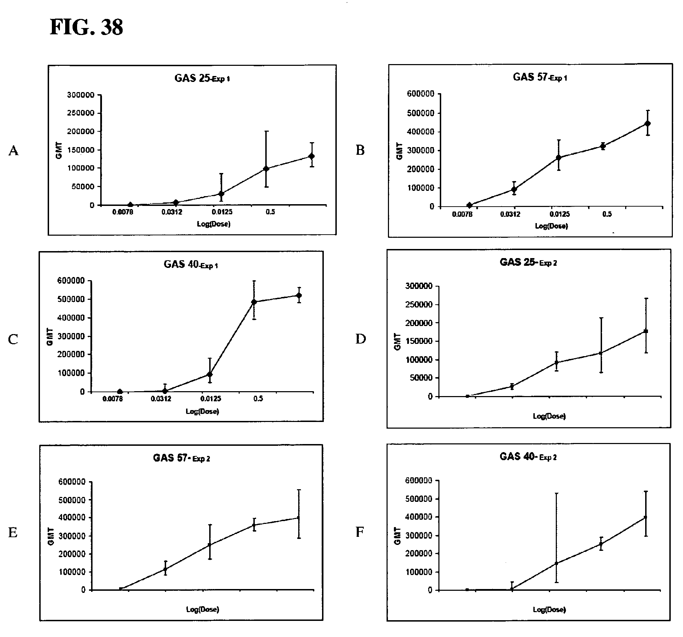

**FIGS. 38A-F**. Graphs showing results of ELISA assays testing doses of individual antigens. **FIGS. 38A**, **38D**, Spy0167 (GAS25) two experiments; **FIGS. 38B**, **38E**, Spy0416 (GAS57) (two experiments); **FIGS. 38C**, **38F**, Spy0269 (GAS40) (two experiments). "GMT," geometric mean titers.

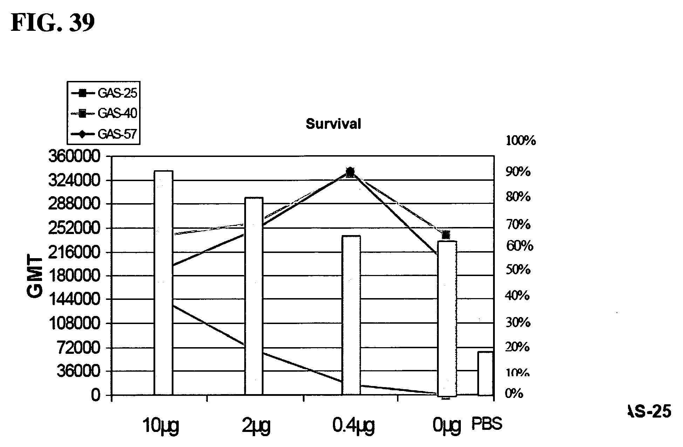

**FIG. 39**. Graph showing results of ELISA and challenge assays testing combinations of Spy0416 mutant D151A/S617A (GAS57), wild-type Spy0269 (GAS40), and dose ranges of Spy0167 mutant P427L/W535F (GAS25) in alum.

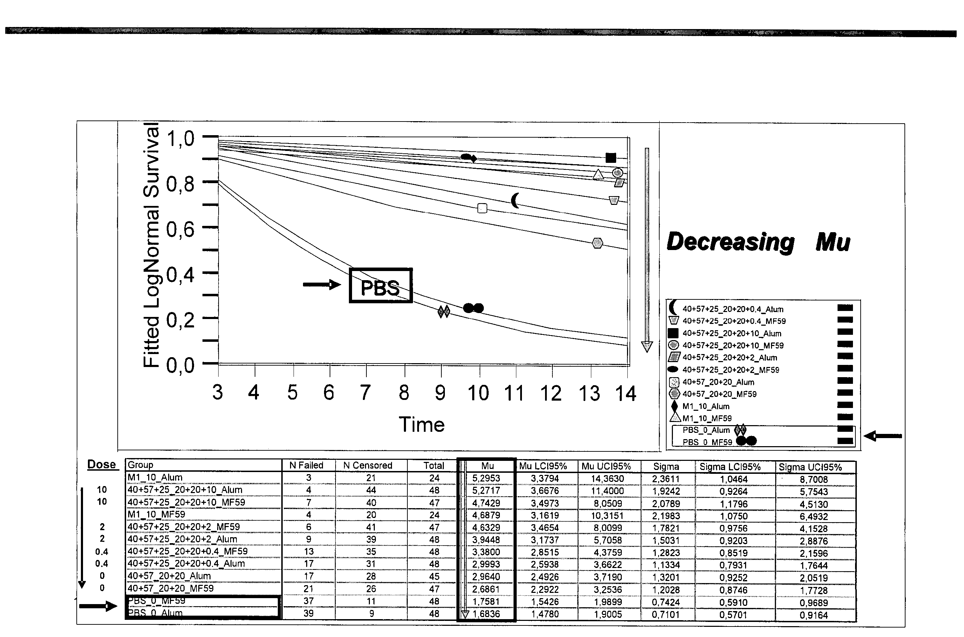

**FIG. 40**. Analysis of LogNormal model adopted as first approximation of mean survival time (MST; *Mu*) analysis, demonstrating that *Mu* decreases with decreasing doses of Spy0167 (GAS25).

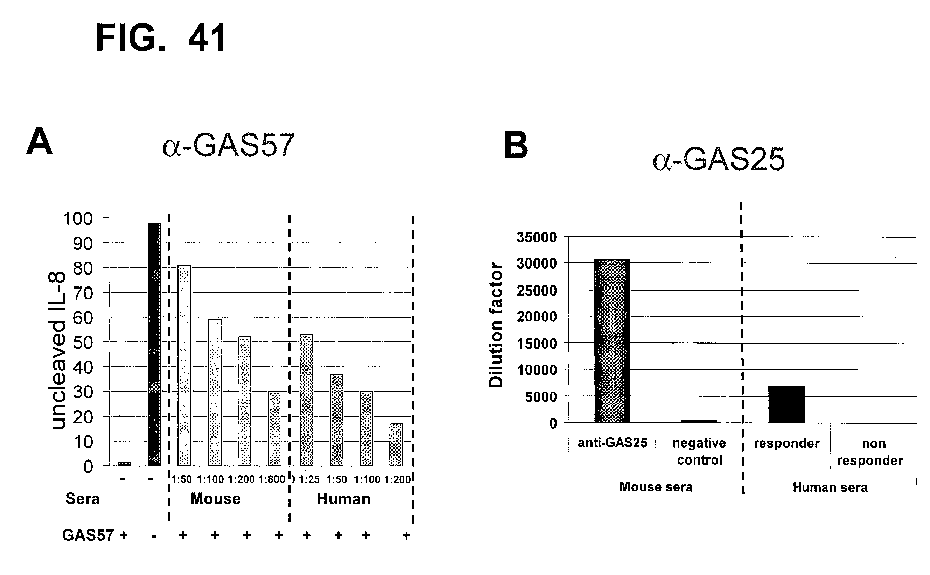

**FIGS. 41A-B.** Bar graphs demonstrating that antibodies to Spy0419 and Spy0167 block toxic activity. **FIG. 41A**, Spy0419 (GAS57). **FIG. 41B**, Spy0167 (GAS25). The titer is defined as the dilution factor required to neutralize 50% of maximum hemolysis.

WO 2006/042027, WO 2008/108830 and WO 2005/032582 describe antigens derived from *Streptococcus pyogenes* and their use in immunization.

DETAILED DESCRIPTION OF THE INVENTION

The invention provides a composition comprising a combination of three *S. pyogenes* (GAS) protein antigens, wherein the protein antigens are:

(1) Spy0167, wherein the Spy0167 protein antigen has an amino acid sequence that is at least 90% identical to SEQ ID NO:107;

(2) Spy0269, wherein the Spy0269 protein antigen has an amino acid sequence that is at least 90% identical to SEQ ID NO:50; and

(3) Spy0416, wherein the Spy0416 protein antigen has an amino acid sequence that is at least 90% identical to SEQ ID NO:1.

The invention provides multi-component compositions which are useful for treating, reducing the risk of, and/or preventing *S. pyogenes* infections. In some embodiments compositions of the invention are vaccine compositions which provide effective prophylaxis against pharyngitis caused by *S. pyogenes* infections in children.

Compositions of the invention are useful for preventing and/or treating S. pyogenes infections. Compositions of the invention comprise combinations of GAS antigens. "GAS protein antigen", unless otherwise defined encompasses a full-length GAS protein as well as fragments, fusions and mutants of the GAS protein as described below. Some compositions further comprise a Group A polysaccharide antigen as defined below. The invention also includes compositions comprising mixtures of combinations of GAS antigens.

Compositions of the invention preferably have one or more of the following properties:

- confer statistically significant protection against one or more *S. pyogenes* strains (*e.g.*, M1 3348, M12 EM5, M23 2071, M6 S43);
- elicit antibodies which mediate *in vitro* bacterial killing (opsonophagocytic killing);
- elicit antibodies which inactivate streptolysin O hemolytic activity;
- elicit antibodies which block Spy0416 protease activity; and/or
- elicit antibodies which prevent cell adhesion and/or cell division.

As described in the Examples below, compositions of the invention provide protection against different mouse-adapted *S. pyogenes* strains in lethal challenge models; elicit functional antibodies which neutralize potent toxins expressed by a majority of *S. pyogenes* strains; and mediate bacterial killing *in vitro.*

Compositions of the invention comprise a combination of three different GAS antigens wherein the GAS antigens are Spy0167 (also referred to as streptolysin O, Spy0167, or "GAS25"); Spy0269 (also referred to as "GAS40"); and Spy0416 (also referred to as "GAS57").

Other GAS antigens which can be included in compositions of the invention include Spy0019 (GAS5), Spy0163 (GAS23), Spy0385 (GAS56), Spy0714 (GAS67), Spy0737 (GAS68), Spy1274 (GAS84), Spy1361 (GAS88), Spy1390 (GAS89), Spy1733 (GAS95), Spy1882 (GAS98), Spy1979 (GAS99), Spy2000 (GAS100), Spy2016 (GAS102), Spy0591 (GAS130), Spy1105 (GAS159), Spy1718 (GAS179), Spy2025 (GAS193), Spy2043 (GAS195), Spy1939 (GAS277), Spy1625 (GAS372), and Spy1134 (GAS561).

Preferred combinations of GAS antigens include the following, each of which may also include a GAS polysaccharide antigen, as described below, and/or an adjuvant, such as alum:

i. Spy0167 and Spy0269;

ii. Spy0167, Spy0269, and Spy0416;

iii. Spy0167 and Spy0416;

iv. Spy0167 mutant P427L/W535F and Spy0269;

v. Spy0167 mutant P427L/W535F, Spy0269, and Spy0416;

vi. Spy0167 mutant P427L/W535F and Spy0416;

vii. Spy0269 and Spy0416 mutant D151A/S617A;

viii. Spy0167, Spy0269, and Spy0416 mutant D151A/S617A;

ix. Spy0167 and Spy0416 mutant D151A/S617A;

x. Spy0167 mutant P427L/W535F, Spy0269, and Spy0416 mutant D151A/S617A; and

xi. Spy0167 mutant P427L/W535F and Spy0416 mutant D151A/S617A.

The compositions can contain other components, such as pharmaceutically acceptable vehicles, antigens of other microorganisms, adjuvants, etc. In particular, any of the compositions described herein may further comprise a Group A polysaccharide antigen as described below and/or an adjuvant, such as alum.

As there is variance among wild-type GAS antigens between GAS M types and GAS strain isolates, references to the GAS amino acid or polynucleotide sequences herein include equivalent amino acid or polynucleotide sequences having some degree of sequence identity thereto, typically because of conservative amino acid substitutions (see Example 30 and **FIGS. 31-33**.

In some embodiments, variants of Spy0167, Spy0269, Spy0416, Spy0714, Spy1390, Spy2000, and disclosed mutants thereof have amino acid sequences which are at least 90%, 91%, 92%, 93%, 94%, 95%, 96%, 97%, 98%, or 99% identical to any of the Spy0167, Spy0269, Spy0416, Spy0714, Spy1390, Spy2000 amino acid sequences disclosed herein, respectively. Typically any difference between the amino acid sequence of a GAS antigen and the amino acid sequence of a variant of the GAS antigen is due to one or more conservative amino acid substitutions. As indicated in **FIG. 31** for example, one, two, or three amino acid deletions also are possible.

In some embodiments conservative amino acid substitutions are based on chemical properties and include substitution of a positively-charged amino acid for another positively charged amino acid (*e.g.,* H, K, R); a negatively-charged amino acid for another negatively charged amino acid (*e.g.,* D, E); a very hydrophobic amino acid for another very hydrophobic amino acid (*e.g.,* C, F, I, L, M, V, W); a less hydrophobic amino acid for another less hydrophobic amino acid (*e.g.,* A, G, H, P, S, T, Y); a partly hydrophobic amino acid for another partly hydrophobic amino acid (*e.g.,* K, R); an aliphatic amino acid for another aliphatic amino acid (*e.g.,* A, I, L, M, P, V); a polar amino acid for another polar amino acid (*e.g.*, A, D, E, G, H, K, N, P, Q, R, S, T, Y); an aromatic amino acid for another aromatic amino acid (*e.g.,* F, H, W, Y); and a small amino acid for another small amino acid (*e.g.,* D, N, T).

In some embodiments, conservative amino acid substitutions are determined using the BLOSUM62 table. The

BLOSUM62 table is an amino acid substitution matrix derived from about 2,000 local multiple alignments of protein sequence segments, representing highly conserved regions of more than 500 groups of related proteins (Henikoff & Henikoff, Proc. Natl. Acad. Sci. USA 89:10915, 1992). The BLOSUM62 substitution frequencies can be used to define conservative amino acid substitutions that may be introduced into amino acid sequences of Spy0167, Spy0269, Spy0416, Spy0714, Spy1390, and Spy2000 antigens. In these embodiments a conservative substitution preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (*e.g.,* 1, 2 or 3), while more preferred conservative amino acid substitutions are characterized by a BLOSUM62 value of at least 2 (*e.g.,* 2 or 3).

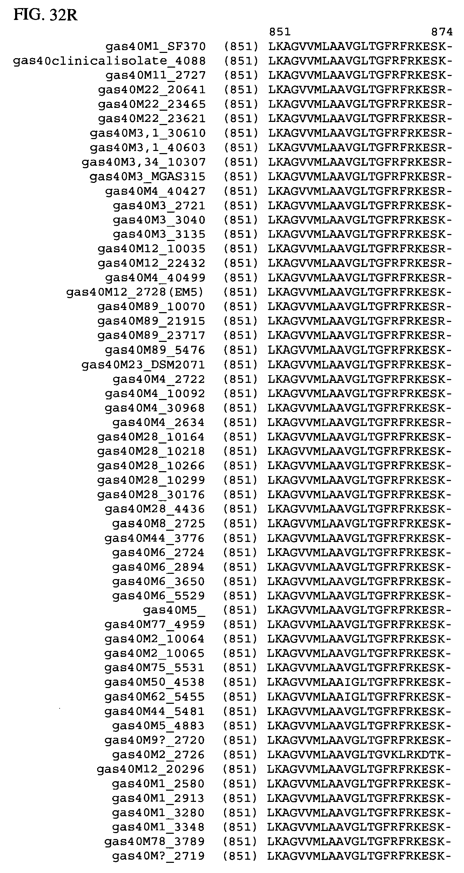

Particular amino acid substitutions or alterations can be identified by aligning the various Spy0167, Spy0269, Spy0416, Spy0714, Spy1390, and Spy2000 amino acid sequences as shown for wild-type Spy0416 in **FIG. 31**, wild-type Spy0269 in **FIG. 32**, and wild-type Spy0167 in **FIG. 33**. For example, based on the alignment in **FIG. 31**, Table 1 indicates some particular options at certain amino acid positions with respect to the Spy0416 amino acid sequence shown in SEQ NO:1. Similarly, options for amino acid variations in the amino acid sequences of Spy0269 and Spy0167 can be identified by inspection of **FIGS. 32** and **33**, respectively.

Table 1.

| position | options | position | options | position | options |
|---|---|---|---|---|---|
| 38 | S, T | 74 | I, V | 104 | S, P |
| 40 | M, S, T | 77 | E, K | 110 | S, P |
| 49 | A, T | 85 | S, P | 228 | A or missing |
| 55 | H, P | 87 | D, G | 229 | D, E, or missing |
| 67 | K, Q | 91 | E, K | 749 | H, R |
| 68 | S, P | 93 | T or missing | | |
| 69 | Q, P | 102 | A, S | | |

Variants of the GAS antigens described below preferably are immunogenic and confer protection against GAS lethal challenge in a mouse model (see the Examples, below). *Spy0167 and immunogenic mutants thereof*

Spy0167 (streptolysin, SLO, GAS25) is a potent pore-forming toxin which induces host cell lysis and is described, *inter alia*, in WO 02/34771. Amino acid sequences for wild-type Spy0167 are shown in SEQ ID NOS:107-119. Unless otherwise defined, a "Spy0167 antigen" includes full-length Spy0167 and Spy0167 mutants, fragments, and fusions, as described below.

In some embodiments a Spy0167 antigen consists essentially of the amino acid sequence SEQ ID NO:174 ("peptide 1 "), the amino acid sequence SEQ ID NO:175 ("peptide 2"), or the amino acid sequence SEQ ID NO:176 ("peptide 3"). In some embodiments a Spy0167 antigen consists essentially of, from N to C terminus, the amino acid sequence SEQ ID NO:175 ("peptide 2") and the amino acid sequence SEQ ID NO:176 ("peptide 3") covalently attached to the amino acid sequence SEQ ID NO:175. "covalently attached" as used herein includes direct covalent linkage as well as linkage via one or more additional amino acids. In other embodiments a Spy0167 antigen consists essentially of, from N to C terminus, the amino acid sequence SEQ ID NO:174; a glycine residue covalently attached to the amino acid sequence SEQ ID NO:174; the amino acid sequence SEQ ID NO:175 covalently attached to the glycine; and the amino acid sequence SEQ ID NO:176 covalently attached to the amino acid sequence SEQ ID NO:175.

Other Spy0167 antigens are fragments of Spy0167 which are less than full-length Spy0167 by at least one amino acid. Preferably the fragments retain an immunological property of the antigen, such as the ability to bind specific antibodies. Preferred amino acid fragments comprise 7 or more amino acids (*e.g.*, 8, 10, 12, 14, 16, 18, 20, 25, 30, 35, 40, 50 or more).

In some embodiments, a Spy0167 antigen is a monomer which comprises the amino acid sequence SEQ ID NO:174. In other embodiments a Spy0167 antigen is a monomer which comprises, from N to C terminus, the amino acid sequence SEQ ID NO:175 and the amino acid sequence SEQ ID NO:176 covalently attached to the amino acid sequence SEQ ID NO:175. In other embodiments a Spy0167 antigen is a monomer which comprises, from N to C terminus, the amino acid sequence SEQ ID NO:174; a glycine residue covalently attached to the amino acid sequence SEQ ID NO:174; the amino acid sequence SEQ ID NO:175 covalently attached to the glycine; and the amino acid sequence SEQ ID NO:176 covalently attached to the amino acid sequence SEQ ID NO:175.

*Fusion proteins*

As disclosed above, Spy0167 antigens used in the invention may be present in the composition as individual separate polypeptides (*e.g.*, "peptide 1," "peptide 2," "peptide 3," "peptide 1+2+3," "peptide 2+3"), but there also are embodiments in which at least two (*i.e*., 2, 3, 4, 5, 6, 7, 8, 9, 10, 11, 12, 13, 14, 15, 16, 17, 18, 19 or 20) antigens are expressed as a single polypeptide chain (a "fusion protein" or "hybrid polypeptide"). Hybrid polypeptides offer two principal advantages. First, a polypeptide that may be unstable or poorly expressed on its own can be assisted by adding a suitable hybrid partner that overcomes the problem. Second, commercial manufacture is simplified as only one expression and purification need be employed in order to produce two polypeptides which are both antigenically useful.

*Mutant forms of Spy0167*

Mutant forms of Spy0167 have at least 50% less hemolytic activity than wild-type Spy0167 (*e.g.,* 50, 55, 60, 65, 70, 75, 80, 85, 90, 95, 96, 97, 98, 99, or 100%) relative to wild-type Spy0167 as determined by a hemolysis assay (*e.g*., see Example 1) but are immunogenic and preferably confer protection against GAS lethal challenge in a mouse model (see Examples 4, 7, 8). Spy0167 mutants include those with an amino acid alteration (*i.e*., a substitution, deletion, or insertion) at one or more of amino acids P427, W535, C530, A248, and D482 numbered according to the wild-type Spy0167 sequence shown in SEQ ID NO:107. Examples of such mutants include P427L (SEQ ID NO:120), W535F (SEQ ID NO:121), C530G (SEQ ID NO:122), ∆A248 (SEQ ID NO:123), W535F/D482N (SEQ ID NO:124), P427L/W535F (SEQ ID NO:125), P427L/C530G (SEQ ID NO:126), and P427L/C530G/W535F (SEQ ID NO:127).

Spy0167 mutants for use in the invention include single, double, or triple amino acid alterations ("single mutants," "double mutants," "triple mutants") at positions P427, W535, C530, A248, and/or D482. Thus, Spy0167 mutants can comprise the following:

i. P427L (SEQ ID NO:120), P427R, P427N, P427C, P427Q, P427E, P427G, P427H, P427I, P427L, P427K, P427M, P427F, P427A, P427S, P427T, P427W, P427Y, or P427V;

ii. W535F (SEQ ID NO:121), W535R, W535N, W535D, W535C, W535Q, W535E, W535G, W535I, W535L, W535K, W535M, W535A, W535P, W535S, W535T, W535Y, or W535V;

iii. C530G (SEQ ID NO:122), C530R, C530N, C530D, C530S, C530Q, C530E, C530A, C530H, C530I, C530L, C530K, C530M, C530F, C530P, C530T, C530W, C530Y, or C530V;

iv. D482L, D482R, D482N, D482C, D482Q, D482E, D482G, D482H, D482I, D482L, D482K, D482M, D482F, D482A, D482S, D482T, D482W, D482Y, or D482V;

v. A248L, A248R, A248N, A248C, A248Q, A248E, A248G, A248H, A248I, A248L, A248K, A248M, A248F, A248S, A248T, A248W, A248Y, or A248V

vi. ∆P427; or ∆W535; or ∆C530; or ∆D482; or ∆A248 (SEQ ID NO:123); and

vii. combinations thereof, such as:

1. double mutants W535F/D482N (SEQ ID NO:124), P427L/W535F (SEQ ID NO:125), and P427L/C530G (SEQ ID NO:126), P427L/A248L, P427L/D482L, W535F/C530G, W535F/A248L, W535F/D482L, CS30G/A248L, and A248L/D482L; and
2. triple mutants P427L/C530G/A248L, P427L/C530G/D482L, P427L/A248L/D482L, P427L/C530G/W535F (SEQ ID NO:127), W535F/C530G/A248L, W535F/C530G/D482L, W535F/A248L/D482L, and C530G/A248L/D482L

Mutant Spy0167 proteins also include fusion polypeptides which comprise a mutant Spy0167 protein as disclosed above and another GAS antigen. GAS antigens are disclosed, *e.g.*, in WO 02/34771 and include, but are not limited to, GAS39 (Spy0266; gi13621542), GAS40 (Spy0269; discussed below), GAS42 (Spy0287; gi13621559), GAS45 (M5005_Spy0249; gi71910063), GAS57 (Spy0416; discussed below), GAS58 (Spy0430; gi13621663), GAS67 (Spy0714; gi3621898), GAS68 (Spy0163; gi13621456), GAS84 (SPy1274; gi13622398), GAS88 (Spy1361; gi13622470), GAS 89 (Spy1390; gi13622493), GAS95 (SPy1733; gi13622787), GAS98 (Spy1882; gi13622916), GAS99 (Spy1979; gi13622993), GAS 100 (Spy2000; gi13623012), GAS 102 (Spy2016, gi 13623025), GAS 117 (Spy0448; gi13621679), GAS130 (Spy0591; gi13621794), GAS137 (Spy0652; gil3621842), GAS146 (Spy0763; gi13621942),

GAS159 (Spy1105; gi13622244), GAS179 (Spy1718, gi13622773), GAS193 (Spy2025; gi3623029), GAS195 (Spy2043; gi13623043), GAS202 (Spy1309; gi13622431), GAS217 (Spy0925, gi1362208), GAS236 (Spy1126; gi13622264), GAS277 (Spy1939; gi13622962), GAS290 (SPy1959; gi13622978), GAS290 (SPy1959; gi13622978), GAS294 (Spy1173; gi13622306), GAS309 (Spy0124; gi13621426), GAS366 (Spy1525; gi13622612), GAS372 (spry1625; gi13622698), GAS384 (Spy1874; gi13622908), GAS389 (Spy1981; gi13622996), GAS504 (Spy1751; gi13622806), GAS509 (spry1618; gi13622692), GAS511 (Spy1743; gi13622798), GAS527 (Spy1204; gi3622332), GAS529 (Spy1280; gi3622403), GAS533 (Spy1877; gi13622912), GAS561 (Spy1134; gi13622269), GAS613 (Spy01673; gi13622736), and GAS681 (spy1152; gi1362228), as well as other antigens listed in Tables A-D, below. The gi numbers for these antigens are for the M1 strain, where available, but it will be appreciated that equivalent proteins from other M strains may also be used.

Preferred Spy0167 antigens according to the invention are immunogenic but not toxic. "Non-toxic" as used herein means that the Spy0167 antigen cannot bind to cholesterol or cannot form oligomers and, more in general, does not promote lysis of cholesterol-containing membranes. An Spy0167 protein can be rendered non-toxic, for example, by deleting at least the single cysteine residue, located in a highly conserved region in the C-terminal section of Spy0167 that can be used as a signature pattern for thiol-activated cytolysins.

Compositions of the invention also can comprise equivalents of Spy0167 mutants which are single polypeptides, which have at least 50% less hemolytic activity than wild-type Spy0167 (*e.g.*, 50, 55, 60, 65, 70, 75, 80, 85, 90, 95, 96, 97, 98, 99, or 100%) relative to wild-type Spy0167 as determined by a hemolytic assay, which are immunogenic, and which preferably confer protection against GAS lethal challenge in a mouse model. Such equivalents may include mutant Spy0167 antigens with amino acid deletions, insertions, and/or substitutions at positions other than P427, W535, C530, A248, and D482, including deletions of up to about 40 amino acids at the N or C terminus (*e.g.*, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 amino acids).

*Spy0269*

GAS40, also known as "spry0269" (M1), "SpyM3_0197" (M3), "SpyM18_0256" (M18) and "prgA," is described,, *e.g.*, in WO 02/34771 and in WO 2005/032582. Amino acid sequences of wild-type Spy0269 are provided in SEQ ID NOS:50-106 and 128-130. Spy0269 antigens are particularly useful in compositions of the invention because Spy0269 proteins are highly conserved both in many M types and in multiple strains of these M types (see WO 2006/042027). Spy0269 consistently provides protection in the animal model of systemic immunization and challenge and induction of bactericidal antibodies.

A Spy0269 protein typically contains a leader peptide sequence (*e.g.*, amino acids 1 - 26 of SEQ ID NO:50), a first coiled-coil region (*e.g.*, amino acids 58 - 261 of SEQ ID NO:50), a second coiled coil region (*e.g.*, amino acids 556 - 733 of SEQ ID NO:50), a leucine zipper region (*e.g.*, amino acids 673 - 701 of SEQ ID NO:50) and a transmembrane region (*e.g.*, amino acids 855 - 866 of SEQ ID NO:50). In some embodiments the leader sequence is removed (*e.g.*, SEQ ID NO:177).

Compositions of the invention also can comprise equivalents of Spy0269 which are single polypeptides, which are immunogenic, and which preferably confer protection against GAS lethal challenge in a mouse model (*e.g.*, Examples 4, 7, 8).

*Spry0416 and Immunogenic mutant thereof*

Spy0416 (M1) is also referred to as "GAS57," 'SpyM3_0298' (M3), 'SpyM18_0464' (M18), and 'prtS.' Spy0416 has been identified as a putative cell envelope proteinase. See WO 02/34771 and US 2006/0258849. There are 49 Spy0416 sequences from 17 different M types (1, 2, 3, 4, 6, 11, 12, 18, 22, 23, 28, 44/61, 68, 75, 77, 89, 94); according to the Centers for Disease Control, the 17 different M types account for over 95% of pharyngitis cases and about 68% of the invasive GAS isolates in the United States. Amino acid sequences of wild-type Spy0416 antigens from various M types are set forth in the sequence listing as SEQ ID NOS:1-49. Compositions of the invention also can comprise equivalents of Spy0416 which are single polypeptides, which are immunogenic, and which preferably confer protection against GAS lethal challenge in a mouse model.

Mutant Spy0416 antigens according to the invention have a proteolytic activity against interleukin 8 (IL-8) which is reduced by at least 50% (*e.g.,* 50, 55, 60, 65, 70, 75, 80, 85, 90, 95, 96, 97, 98, 99, or 100%) relative to wild-type Spy0416 as detected by either SDS-PAGE or ELISA (see Example 3), but are immunogenic, *e.g.*, they confer protection against GAS lethal challenge in a mouse model. Preferably, a mutant Spy0416 of the invention also does not cleave other human cytokines, such as CXCL1/GRO$\alpha$ (*e.g.*, SEQ ID NO:131), CXCL2/GRO$\beta$ (*e.g.,* SEQ ID NO:132), CXCL3/GRO$\gamma$ (*e.g.,* SEQ ID NO:133), CXCL4 (*e.g.,* SEQ ID NO:134), CXCL12/SDF-1a (*e.g.,* SEQ ID NO:135), CXCL12/SDF-1$\beta$ (*e.g.,* SEQ ID NO:136), CXCL12/SDF-1$\gamma$ (*e.g.,* SEQ ID NO:137), CXCL5/ENA78 (*e.g,* SEQ ID NO:138),

CXCL6/GCP-2 (*e.g.*, SEQ ID NO:139), CXCL7/NAP-2 (*e.g.,* SEQ ID NO:140), CXCL9/MIG (*e.g.,* SEQ ID NO:141), CXCL10/IP10 (*e.g.*, SEQ ID NO:142), CXCL11 (*e.g.*, SEQ ID NO:143), CXCL13 (*e.g.,* SEQ ID NO:144), CXCL14 (*e.g.,* SEQ ID NO:145), and CXCL16 (*e.g.,* SEQ ID NO:146).

Spy0416 mutants useful in the invention include those with at an amino acid alteration (*i.e.*, a substitution, deletion, or insertion) at one or more of amino acids D151, H279, or S617, numbered according to the wild-type Spy0416 sequence shown in SEQ ID NO:1, including single, double, or triple amino acid alterations ("single mutants," "double mutants," "triple mutants") at positions D151, H279, and/or S617. Thus, Spy0416 mutants can comprise the following:

i. D151A (SEQ ID NO:147), D151R, 151N, D151C, D151Q, D151E, D151G, D151H, D1511, D151L, D151K, D151M, D151F, D151P, D151S, D151T, D151W, D151Y, or D151V;

ii. H279A, H279R, H279N, H279D, H279C, H279Q, H279E, H279G, H279I, H279L, H279K, H279M, H279F, H279P, H279S, H279T, H279W, H279Y, or H279V;

iii. S617A (SEQ ID NO:148), S617R, S617N, S617D, S617C, S617Q, S617E, S617G, S617H, S617I, S617L, S617K, S617M, S617F, S617P, S617T, S617W, S617Y, or S617V;

iv. ∆D151; or ∆H279; or ∆S617; and

v. combinations thereof, such as D151A/S617A (SEQ ID NO:149, SEQ ID NO:198).

Spy0416 mutant antigens of the invention also include fusion polypeptides which comprise a Spy0416 mutant antigen as disclosed above and another GAS antigen. GAS antigens are disclosed, *e.g.*, in WO 02/34771 and include, but are not limited to, all or a portion of Spy0019 (GAS5; gi15675086), Spy0163 (GAS23; gi15675077), Spy0167 (GAS25, discussed above), Spy0266 (GAS39; gi13621542), Spy0269 (GAS40, discussed above), Spy0287 (GAS42; gi13621559), MS005_Spy0249 (GAS45; gi71910063), Spy0385 (GAS56; gi15675097), Spy0430 (GAS58; gi13621663), Spy0714 (GAS67; gi13621898), Spy0163 (GAS68; gi13621456), Spy1274 (GAS84; gi13622398), Spy1361 (GAS88; gi13622470), Spy1390 (GAS89; gi13622493), Spy1733 (GAS95; 13622787), Spy1882 (GAS98; gi136229!6), Spy1979 (GAS99; gi13622993), Spy2000 (GAS100; gi13623012), Spy2016 (GAS102; gi15675798), Spy0448 (GAS117; gi13621679), Spy0591 (GAS130; gi13621794), Spy0652 (GAS137; gi13621842), Spy0763 (GAS146; gi15674811), Spy1105 (GAS159; gi13622244), Spy1718 (GAS179, gi13622773), Spy2025 (GAS193; gi3623029), Spy2043 (GAS195; gi15675815), Spy1309 (GAS202; gi13622431), Spy0925 (GAS217; gi1362208), Spy1126 (GAS236; gi13622264), Spy1939 (GAS277; gi13622962), Spy1959 (GAS290; gi13622978), Spy1173 (GAS294; gi13622306), Spy0124 (GAS309; gi13621426), Spy1525 (GAS366; gi13622612), Spy1625 (GAS372; gi13622698), Spy1874 (GAS384; gi13622908), Spy1981 (GAS389; gi13622996), Spy1751 (GAS504; gi13622806), Spy1618 (GAS509; gi13622692), Spy1743 (GAS511; gi13622798), Spy1204 (GAS527; gi3622332), Spy1280 (GAS529; gi3622403), Spy1877 (GAS533; gi13622912), Spy1134 (GAS561; gi13622269), Spy01673 (GAS613; gi13622736), Spy1152 (GAS681; gi1362228), or other antigens disclosed in Tables A-D below. The gi numbers for these antigens are for the M1 strain, where available, but it will be appreciated that equivalent proteins from other M strains may also be used.

The invention also includes equivalents of Spy0416 mutants which are single polypeptides, which do not cleave IL-8 as determined by SDS-PAGE or ELISA, which are immunogenic, and which preferably confer protection against GAS lethal challenge in a mouse model (*e.g.,* Examples 4, 7, 8). Such equivalents may include mutant Spy0416 antigens with amino acid deletions, insertions, and/or substitutions at positions other than D151, H279, or S617, including deletions of up to about 40 amino acids at the N or C terminus (*e.g.*, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 amino acids).

*Other GAS antigens,*

One or more other GAS antigens can be included in compositions of the invention. GAS antigens are disclosed, for example, in WO 02/34771. Useful GAS antigens include all or portions of Spy0737, Spy0019, Spy0163, Spy0266, Spy0287 Spy0249, Spy0385, Spy0430, Spy0714, Spy0163, Spy1274, Spy1361, Spy1390, Spy1733, Spy1882, Spy1979, Spy2000, Spy2016, Spy0448, Spy0591, Spy0652, Spy0763, Spy1105, Spy1718, Spy2025, Spy2043, Spy1309, Spy0925, Spy1126, Spy1939, Spy1959, Spy1173, Spy0124, Spy1525, Spy1625, Spy1874, Spy1981, Spy1751, Spy1618, Spy1743, Spy1204, Spy1280, Spy1877, Spy1134, and Spy01673. Compositions of the invention also can comprise equivalents of these GAS antigens which are single polypeptides, which are immunogenic, and which preferably confer protection against GAS lethal challenge in a mouse model (*e.g.,* Examples 4, 7, 8). For example, each of Spy0763 (GAS146) and Spy1134 (GAS561) protects mice against challenge with *S. pyogenes* M1 3348 (70% survival compared with 20% survival of the negative controls; n = 20). See also Tables A-D, below.

Table A.

| ANTIGEN | SEQ ID NO: | Surfome | Secretome | FACS | in sequenced strains | | Serotype distribution: | |
|---|---|---|---|---|---|---|---|---|
| | | | | | genetic distribution | average % identity | present in | missing in |
| spy0019 | 178 | 2/4 | 4/4 | pos. in 2/4 | 12/12 | >90% | | |
| spy0163 | 179 | no | 2/4 | pos. in 1/4 | 12/12 | >90% | | |
| spy0385 | 180 | no | no | not tested | 12/12 | >90% | | |
| spy0714 | 181 | no | no | not tested | 12/12 | >90% | | |
| spy0737 | 182 | no | no | not tested | 6/12 | 70% | M1, M4, M12, M28, M49 | missing in M2, M3, M5, M6, M18 |
| spy1274 | 183 | no | no | not tested | 11/12 | >90% | M1, M2, M4, M5, M12, M28, M49 | missing in M6 |
| spy1361 | 184 | no | no | not tested | 12/12 | >90% | | |
| spy1390 | 185 | 1/4 | 2/4 | pos. in 2/4 | 12/12 | >90% | | |
| spy1733 | 186 | no | no | pos. in 2/4 | 12/12 | >90% | | |
| spy1882 | 187 | 4/4 | 2/4 | pos. in 2/4 | 12/12 | >90% | | |
| spy1979 | 188 | no | 1/4 | pos. in 2/4 | 12/12 | >90% | | |
| spy2000 | 189 | no | 1/4 | pos. in 1/4 | 12/12 | >90% | | |
| spy2016 | 190 | no | no | not tested | 4/12, variants M1 and M12 | >90% within variants | M1, M12 | missing in all the others |
| spy0591 | 191 | no | no | pos. in 2/4 | 12/12 | >90% | | |
| spy1105 | 192 | no | no | not tested | 12/12 | >90% | | |
| spy1718 | 193 | 2/4 | no | pos. in 2/4 | 12/12 | >90% within variants | Variant 1: M1, M2, M3, M5, M6, M12, M18, Variant 2: M4, M28, M49 | |
| spy2025 | 194 | no | no | not tested | 12/12 | >90% | | |

(continued)

| ANTIGEN | SEQ ID NO: | Surfome | Secretome | FACS | in sequenced strains | | Serotype distribution: | |
|---|---|---|---|---|---|---|---|---|
| | | | | | genetic distribution | average % identity | present in | missing in |
| spy2043 | 195 | no | 3/4 | pos. in 3/4 | 12/12 | >90% | | |
| spy1939 | 196 | 1/4 | 3/4 | pos. in 2/4 | 12/12 | >90% | | |
| spy1625 | 197 | no | no | not tested | 12/12 | >90% | | |

Table B.

| ANTIGEN | 3348 M1 | | | EM5 M12 | | | 2721 M3 | | | 2071 M23 | | | ALL STRAINS | ALL STRAINS |
|---|---|---|---|---|---|---|---|---|---|---|---|---|---|---|
| | FACS | SURF | SECR | FACS | SURF | SECR | FACS | SURF | SECR | FACS | SURF | SECR | SURF FREQ | SECR FREQ |
| | 322 | | Y | 268 | Y | Y | 13 | | Y | 31 | Y | Y | | ALL |
| spy0019 | 122 | | Y | 26 | | Y | 33 | | | 58 | | | | 2/4 |
| spy0163 | | | | | | | | | | | | | | |
| spy0385 | | | | | | | | | | | | | | |
| spy0714 | | | | | | | | | | | | | | |
| spy0737 | | | | | | | | | | | | | | |
| spy1274 | | | | | | | | | | | | | | |
| spy1361 | 120 | Y | Y | 254 | | | 7 | | Y | 92 | | | | 2/4 |
| spy1390 | 62 | | | 209 | | | 6 | | | 115 | | | | |
| spy1733 | 335 | Y | Y | 146 | Y | | 4 | Y | | 40 | Y | Y | 5/16 | 2/4 |
| spy1882 | 186 | | Y | 188 | | | 9 | | | 59 | | | | 1/4 |
| spy1979 | 163 | | Y | 80 | | | 15 | | | 22 | | | | 1/4 |
| spy2016 | 186 | | | 119 | | | 43 | | | 48 | | | | |
| spy0591 | | | | | | | | | | | | | | |
| spy1105 | 31 | Y | | 257 | | | 3 | Y | | 141 | | | | |
| spy1718 | | | | | | | | | | | | | | |
| spy2025 | 332 | | Y | 349 | | | 26 | | Y | 359 | | Y | | 3/4 |
| spy2043 | 225 | Y | Y | 203 | | | 37 | | Y | 71 | | Y | | 3/4 |
| spy1939 | | | | | | | | | | | | | | |
| spy1625 | | | | | | | | | | | | | | |

Table C.

| | | PROTECTION | | |
|---|---|---|---|---|
| SEQ ID NO: | ANTIGEN | M1 | M12 | M23 |
| | | + | + | - |
| 178 | spy0019 | + | nd | nd |
| 179 | spy0163 | | | |
| 180 | spy0385 | | | |
| 181 | spy0714 | | | |
| 182 | spy0737 | | | |
| 183 | spy1274 | | | |
| 184 | spy1361 | + | - | nd |
| 185 | spy1390 | nd | + | nd |
| 186 | spy1733 | + | - | - |
| 187 | spy1882 | + | - | + |
| 188 | spy1979 | - | + | - |
| 189 | spy2000 | | | |
| 190 | spy2016 | + | - | + |
| 191 | spy0591 | | | |
| 192 | spy1105 | nd | - | + |
| 193 | spy1718 | | | |
| 194 | spy2025 | + | + | - |
| 195 | spy2043 | - | - | nd |
| 196 | spy1939 | | | |
| 197 | spy1625 | | | |

Table D.

| | | FACS details | | | |
|---|---|---|---|---|---|
| ANTIGEN | SEQ ID NO: | 3348 M1 | EM5 M12 | 2721 M3 | 2071 M23 |
| | | 322 | 268 | 13 | 31 |
| spy0019 | 178 | 122 | 26 | 33 | 58 |
| spy0163 | 179 | | | | |
| spy0385 | 180 | | | | |
| spy0714 | 181 | | | | |
| spy0737 | 182 | | | | |
| spy1274 | 183 | | | | |
| spy1361 | 184 | 120 | 254 | 7 | 92 |
| spy1390 | 185 | 62 | 209 | 6 | 115 |
| spy1733 | 186 | 335 | 146 | 4 | 40 |
| spy1882 | 187 | 186 | 188 | 9 | 59 |

(continued)

| | | FACS details | | | |
|---|---|---|---|---|---|
| ANTIGEN | SEQ ID NO: | 3348 M1 | EM5 M12 | 2721 M3 | 2071 M23 |
| | | 322 | 268 | 13 | 31 |
| spy1979 | 188 | 163 | 80 | 15 | 22 |
| spy2000 | 189 | | | | |
| spy2016 | 190 | 186 | 119 | 43 | 48 |
| spy0591 | 191 | | | | |
| spy1105 | 192 | 31 | 257 | 3 | 141 |
| spy1718 | 193 | | | | |
| spy2025 | 194 | 332 | 349 | 26 | 359 |
| spy2043 | 195 | 225 | 203 | 37 | 71 |
| spy1939 | 196 | | | | |
| spy1625 | 197 | | | | |

*Fragments*

The length of fragments of the wild-type or mutant GAS proteins described above may vary depending on the amino acid sequence of the specific GAS antigen or mutant thereof, but the fragment is preferably at least seven consecutive amino acids, *e.g.,* 8, 10, 12, 14, 16, 18, 20, 25, 30, 35, 40, 50, 60, 70, 80, 90, 100, 150, 200 or more, up to one amino acid less than a full-length wild-type or mutant GAS protein. Preferably the fragment is immunogenic and comprises one or more epitopes from the sequence. Other preferred fragments include (1) the N-terminal signal peptides of each identified GAS protein, (2) the identified GAS protein without their N-terminal signal peptides, and (3) each identified GAS protein in which up to 10 amino acid residues (*e.g.,* 1, 2, 3, 4, 5, 6, 7, 8, 9, 10, 15, 20, 25 or more) are deleted from the N-terminus and/or the C-terminus (for example, the N-terminal amino acid residue may be deleted). Other fragments omit one or more domains of the protein (*e.g.,* omission of a signal peptide, a cytoplasmic domain, a transmembrane domain, or an extracellular domain). In some embodiments the fragment is amino acids 33-324 of Spy0269.

*GAS polysaccharide antigens*

GAS polysaccharide (PS) is a cell-wall polysaccharide present in all GAS strains. Antibody titers to PS correlate inversely with disease and colonization in children. In some embodiments compositions of the invention comprise a GAS polysaccharide antigen. *S. pyogenes* GAS carbohydrate typically features a branched structure with an L-rhamnopyranose (Rhap) backbone consisting of alternating alpha-(1→2) and alpha-(1→3) links and D-N-acetylglucosamine (GlcpNAc) residues beta-(1→3)-connected to alternating rhamnose rings (Kreis et al., Int. J. Biol. Macromol. 17, 117-30, 1995). GAS polysaccharide antigens useful in compositions of the invention have the formula:

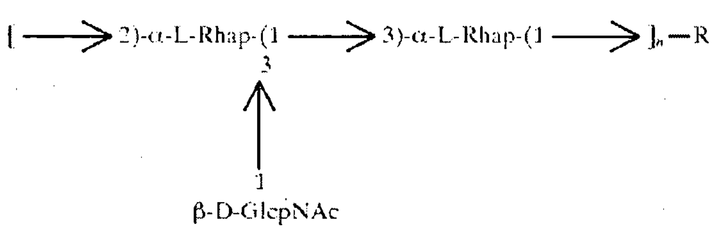

(I),

wherein R is a terminal reducing L-Rhamnose or D-GlcpNAc and n is a number from about 3 to about 30.

The GAS polysaccharide antigen used according to the invention may be a substantially full-length GAS carbohydrate, as found in nature, or it may be shorter than the natural length. Full-length polysaccharides may be depolymerized to give shorter fragments for use with the invention, *e.g.,* by hydrolysis in mild acid, by heating, by sizing

chromatography, etc. However, it is preferred to use saccharides of substantially full-length. In particular, it is preferred to use saccharides with a molecular weight of about 10 kDa. Molecular masses can be measured by gel filtration relative to dextran standards.

The saccharide may be chemically modified relative to the GAS carbohydrate as found in nature. For example, the saccharide may be de N acetylated (partially or fully), N propionated (partially or fully), etc. The effect of de-acetylation etc., for example on immunogenicity, can be assessed by routine assays.

In some embodiments the GAS polysaccharide antigen is conjugated to a carrier, such as the mutated diphtheria toxin CRM 197 (and other carriers described below. As described in the Examples, below, antibodies to PS conjugated with CRM197 ("GC") induce GAS opsonophagocytic killing.

*Production of GAS protein antigens*

*Recombinant production*

The redundancy of the genetic code is well-known. Thus, any nucleic acid molecule (polynucleotide) which encodes one of the GAS antigens described herein can be used to produce that protein recombinantly. Nucleic acid molecules encoding wild-type GAS antigens also can be isolated from the appropriate *S. pyogenes* bacterium using standard nucleic acid purification techniques or can be synthesized using an amplification technique, such as the polymerase chain reaction (PCR), or by using an automatic synthesizer. *See* Caruthers et al., Nucl. Acids Res. Symp. Ser. 215, 223, 1980; Horn et al., Nucl. Acids Res. Symp. Ser. 225, 232, 1980; Hunkapiller et al., Nature 310, 105-11, 1984; Grantham et al., Nucleic Acids Res. 9, r43-r74, 1981.

cDNA molecules can be made with standard molecular biology techniques, using mRNA as a template. cDNA molecules can thereafter be replicated using molecular biology techniques well known in the art. An amplification technique, such as PCR, can be used to obtain additional copies of polynucleotides of the invention, using either genomic DNA or cDNA as a template.

If desired, polynucleotides can be engineered using methods generally known in the art to alter antigen-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site directed mutagenesis can be used to insert new restriction sites, alter glycosylation patterns, change codon preference, produce splice variants, introduce mutations, and so forth.

Sequence modifications, such as the addition of a purification tag sequence or codon optimization, can be used to facilitate expression. For example, the N-terminal leader sequence may be replaced with a sequence encoding for a tag protein such as polyhistidine ("HIS") or glutathione S-transferase ("GST"). Such tag proteins may be used to facilitate purification, detection, and stability of the expressed protein. Codons preferred by a particular prokaryotic or eukaryotic host can be selected to increase the rate of protein expression or to produce an RNA transcript having desirable properties, such as a half life which is longer than that of a transcript generated from the naturally occurring sequence. These methods are well known in the art and are further described in WO05/032582.

*Expression vectors*

A nucleic acid molecule which encodes a GAS antigen for use in the invention can be inserted into an expression vector which contains the necessary elements for the transcription and translation of the inserted coding sequence. Methods which are well known to those skilled in the art can be used to construct expression vectors containing coding sequences and appropriate transcriptional and translational control elements. These methods include *in vitro* recombinant DNA techniques, synthetic techniques, and *in vivo* genetic recombination.

*Host cells*

Host cells for producing GAS antigens can be prokaryotic or eukaryotic. *E. coli* is a preferred host cell, but other suitable hosts include *Lactococcus lactis, Lactococcus cremoris, Bacillus subtilis*, *Vibrio cholerae*, *Salmonella typhi, Salmonella typhimurium, Neisseria lactamica*, *Neisseria cinerea, Mycobacteria (e.g., M. tuberculosis*), yeasts, baculovirus, mammalian cells, etc.

A host cell strain can be chosen for its ability to modulate the expression of the inserted sequences or to process the expressed polypeptide in the desired fashion. Such modifications of the polypeptide include, but are not limited to, acetylation, carboxylation, glycosylation, phosphorylation, lipidation, and acylation. Post translational processing which cleaves a "prepro" form of the polypeptide also can be used to facilitate correct insertion, folding and/or function. Different host cells which have specific cellular machinery and characteristic mechanisms for post translational activities are

available from the American Type Culture Collection (ATCC; 10801 University Boulevard, Manassas, VA 20110-2209) and can be chosen to ensure the correct modification and processing of a foreign protein. See WO 01/98340.

Expression constructs can be introduced into host cells using well-established techniques which include, but are not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun" methods, and DEAE- or calcium phosphate-mediated transfection.

Host cells transformed with expression vectors can be cultured under conditions suitable for the expression and recovery of the protein from cell culture. The protein produced by a transformed cell can be secreted or contained intracellularly depending on the nucleotide sequence and/or the expression vector used. Those of skill in the art understand that expression vectors can be designed to contain signal sequences which direct secretion of soluble antigens through a prokaryotic or eukaryotic cell membrane.

*Purification*

Signal export sequences can be included in a recombinantly produced GAS antigen so that the antigen can be purified from cell culture medium using known methods. Alternatively, recombinantly produced GAS antigens can be isolated from engineered host cells and separated from other components in the cell, such as proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis. A preparation of purified GAS antigens is at least 80% pure; preferably, the preparations are 90%, 95%, or 99% pure. Purity of the preparations can be assessed by any means known in the art, such as SDS-polyacrylamide gel electrophoresis or RP-HPLC analysis. Where appropriate, mutant Spy0167 proteins can be solubilized, for example, with urea.

*Chemical synthesis*

GAS antigens can be synthesized, for example, using solid phase techniques. See, *e.g.,* Merrifield, J. Am. Chem. Soc. 85, 2149 54, 1963; Roberge et al., Science 269, 202 04, 1995. Protein synthesis can be performed using manual techniques or by automation. Automated synthesis can be achieved, for example, using Applied Biosystems 431 A Peptide Synthesizer (Perkin Elmer). Optionally, fragments of GAS antigens can be separately synthesized and combined using chemical methods to produce a full-length molecule.

*Pharmaceutical Compositions*

The invention also provides compositions for use as medicaments (*e.g.,* as immunogenic compositions or vaccines). Compositions of the invention are useful for preventing *S. pyogenes* infection, reducing the risk of *S. pyogenes* infection, and/or treating disease caused as a result of *S. pyogenes* infection, such as bacteremia, meningitis, puerperal fever, scarlet fever, erysipelas, pharyngitis, impetigo, necrotizing fasciitis, myositis or toxic shock syndrome.

Compositions containing GAS antigens are preferably immunogenic compositions, and are more preferably vaccine compositions. The pH of such compositions preferably is between 6 and 8, preferably about 7. The pH can be maintained by the use of a buffer. The composition can be sterile and/or pyrogen free. The composition can be isotonic with respect to humans.

Vaccines according to the invention may be used either prophylactically or therapeutically, but will typically be prophylactic. Accordingly, the invention includes a method for the therapeutic or prophylactic treatment of a *Streptococcus pyogenes* infection. The animal is preferably a mammal, most preferably a human. The methods involve administering to the animal a therapeutic or prophylactic amount of the immunogenic compositions of the invention. The invention also provides the immunogenic compositions of the invention for treating, reducing the risk or, and/or preventing a *S. pyogenes* infection.

Some compositions disclosed herein comprise two different GAS antigens, as described above. Other compositions disclosed herein comprise at least one nucleic acid molecule which encodes the two different antigens. See, *e.g.,* Robinson & Torres (1997) Seminars in Immunology 9:271-283; Donnelly et al. (1997) Ann. Rev Immunol 15:617-648; Scott-Taylor & Dalgleish (2000) Expert Opin Investig Drugs 9:471-480; Apostolopoulos & Plebanski (2000) Curr Opin Mol Ther 2:441-447; Ilan (1999) Curr Opin Mol Ther 1:116-120; Dubensky et al. (2000) Mol Med 6:723-732; Robinson & Pertmer (2000) Adv Virus Res 55:1-74; Donnelly et al. (2000) Am J Respir Crit Care Med 162(4 Pt 2):S190-193; Davis (1999) Mt. Sinai J. Med. 66:84-90. Typically the nucleic acid molecule is a DNA molecule, *e.g.,* in the form of a plasmid.

In some embodiments, compositions of the invention can include one or more additional active agents. Such agents include, but are not limited to, (a) a polypeptide antigen which is useful in a pediatric vaccine, and (b) a polypeptide antigen which is useful in a vaccine for elderly or immunocompromised individuals.

*Additional antigens*

Compositions of the invention may be administered in conjunction with one or more additional antigens for use in therapeutic or prophylactic methods of the present invention. Suitable antigens include those listed below. Additionally, the compositions of the present invention may be used to treat, reduce the risk of, or prevent infections caused by any of the below-listed pathogens. In addition to combination with the antigens described below, the compositions of the invention may also be combined with an adjuvant as described herein.

Additional antigens for use with the invention include, but are not limited to, one or more of the following antigens set forth below, or antigens derived from one or more of the pathogens set forth below:

A. Bacterial Antigens

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below.

*Neisseria meningitides: Meningitides* antigens may include proteins (such as those identified in References 1 - 7), saccharides (including a polysaccharide, oligosaccharide or lipopolysaccharide), or outer-membrane vesicles (References 8, 9, 10, 11) purified or derived from *N. meningitides* serogroup such as A, C, W135, Y, and/or B. *Meningitides* protein antigens may be selected from adhesions, autotransporters, toxins, Fe acquisition proteins, and membrane associated proteins (preferably integral outer membrane protein).

*Streptococcus pneumoniae: Streptococcus pneumoniae* antigens may include a saccharide (including a polysaccharide or an oligosaccharide) and/or protein from *Streptococcus pneumoniae.* Saccharide antigens may be selected from serotypes 1, 2, 3, 4, 5, 6B, 7F, 8, 9N, 9V, 10A, 11A, 12F, 14, 15B, 17F, 18C, 19A, 19F, 20, 22F, 23F, and 33F. Protein antigens may be selected from a protein identified in WO 98/18931, WO 98/18930, US Patent No. 6,699,703, US Patent No. 6,800,744, WO 97/43303, and WO 97/37026. *Streptococcus pneumoniae* proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133.

*Streptococcus pyogenes* (Group A Streptococcus): Group A *Streptococcus antigens* may include a protein identified in WO 02/34771 or WO 2005/032582 (including, but not limited to, GAS39 (Spy0266), GAS40 (Spy0269, discussed above), GAS42 (Spy0287), GAS45(M5005_Spy0249), GAS57 (Spy0416), GAS58 (Spy0430), GAS67 (Spy0714), GAS68 (Spy0163), GAS84 (SPy1274), GAS88 (Spy1361), GAS 89 (Spy1390) GAS95 (SPy1733), GAS98 (Spy1882), GAS99 (Spy1979), GAS100 (Spy2000), GAS102 (Spy2016), GAS 117 (Spy0448), GAS130 (Spy0591), GAS137 (Spy0652), GAS 146 (Spy0763), GAS159 (Spy1105), GAS179 (Spy1718), GAS193 (Spy2025), GAS195 (Spy2043), GAS202 (Spy1309), GAS217 (Spy0925), GAS236 (Spy1126), GAS277 (Spy1939), GAS294 (Spy1173), GAS309 (Spy0124), GAS366 (Spy1525), GAS372 (Spy1625), GAS384 (Spy1874), GAS389 (Spy1981), GAS504 (Spy1751), GAS509 (Spy1618), GAS290 (SPy1959), GAS511(Spy1743), GAS527 (Spy1204), GAS529 (Spy1280), GAS533(Spy1877), GAS561 (Spy1134), GAS613 (Spy01673), and GAS681 (spy1152), other GAS antigens described above and in Tables A-D, fusions of fragments of GAS M proteins (including those described in WO 02/094851, and Dale, Vaccine (1999) 17:193-200, and Dale, Vaccine 14(10): 944-948), fibronectin binding protein (Sfb1), Streptococcal heme-associated protein (Shp), and Streptolysin S (SagA).

*Moraxella catarrhalis*: *Moraxella* antigens include antigens identified in WO 02/18595 and WO 99/58562, outer membrane protein antigens (HMW-OMP), C-antigen, and/or LPS.

*Bordetella pertussis*: Pertussis antigens include petussis holotoxin (PT) and filamentous haemagglutinin (FHA) from *B. pertussis*, optionally also combination with pertactin and/or agglutinogens 2 and 3 antigen.

*Staphylococcus aureus*: *Staphylococcus aureus* antigens include *S. aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin).

*Staphylococcus epidermis: S. epidermidis* antigens include slime-associated antigen (SAA).

*Clostridium tetani* (Tetanus): Tetanus antigens include tetanus toxoid (TT), preferably used as a carrier protein in conjunction/conjugated with the compositions of the present invention.

*Cornynebacterium diphtheriae* (Diphtheria): Diphtheria antigens include diphtheria toxin, preferably detoxified, such as CRM197. Additionally antigens capable of modulating, inhibiting or associated with ADP ribosylation are contemplated for combination/coadministration/conjugation with the compositions of the present invention. The diphtheria toxoids may be used as carrier proteins.

*Haemophilus influenzae B* (Hib): Hib antigens include a Hib saccharide antigen.

*Pseudomonas aeruginosa*: *Pseudomonas* antigens include endotoxin A, Wzz protein, P. aeruginosa LPS, more particularly LPS isolated from PAO1 (O5 serotype), and/or Outer Membrane Proteins, including Outer Membrane Proteins F (OprF) (Infect Immun. 2001 May; 69(5): 3510-3515).

*Legionella pneumophila.* Bacterial antigens may be derived from *Legionella pneumophila.*

*Streptococcus agalactiae* (Group B Streptococcus): Group B *Streptococcus* antigens include a protein or saccharide antigen identified in WO 02/34771, WO 03/093306, WO 04/041157, or WO 2005/002619 (including proteins GBS 80, GBS 104, GBS 276 and GBS 322, and including saccharide antigens derived from serotypes Ia, Ib, Ia/c, II, III, IV, V, VI, VII and VIII).

*Neiserria gonorrhoeae: Gonorrhoeae* antigens include Por (or porin) protein, such as PorB (see Zhu et al., Vaccine (2004) 22:660 - 669), a transferring binding protein, such as TbpA and TbpB (See Price et al., Infection and Immunity (2004) 71(1):277 - 283), a opacity protein (such as Opa), a reduction-modifiable protein (Rmp), and outer membrane vesicle (OMV) preparations (see Plante et al., J. Infections Disease 182, 848-55, 2000), also see e.g. WO99/24578, WO99/36544, WO99/57280, WO02/079243).

*Chlamydia trachomatis*: *Chlamydia trachomatis* antigens include antigens derived from serotypes A, B, Ba and C (agents of trachoma, a cause of blindness), serotypes L1, L2 & L3 (associated with *Lymphogranuloma venereum*), and serotypes, D-K. Chlamydia trachomas antigens may also include an antigen identified in WO 00/37494, WO 03/049762, WO 03/068811, or WO 05/0026f19, including PepA (CT045), LcrE (CT089), ArtJ (CT381), DnaK (CT396), CT398, OmpH-like (CT242), L7/L12 (CT316), OmcA (CT444), AtosS (CT467), CT547, Eno (CT587), HrtA (CT823), and MurG (CT761).

*Treponema pallidum* (Syphilis): Syphilis antigens include TmpA antigen.

*Haemophilus ducreyi* (causing chancroid): Ducreyi antigens include outer membrane protein (DsrA).

*Enterococcus faecalis* or *Enterococcus faecium*: Antigens include a trisaccharide repeat or other *Enterococcus* derived antigens provided in US Patent No. 6,756,361.

*Helicobacter pylori: H. pylori* antigens include Cag, Vac, Nap, HopX, HopY and/or urease antigen.

*Staphylococcus saprophyticus*: Antigens include the 160 kDa hemagglutinin of *S. saprophyticus* antigen.

*Yersinia enterocolitica* antigens include LPS (Infect Immun. 2002 August; 70(8): 4414).

*E. coli*: *E. coli* antigens may be derived from enterotoxigenic *E. coli* (ETEC), enteroaggregative *E. coli* (EAggEC), diffusely adhering *E. coli* (DAEC), enteropathogenic *E. coli* (EPEC), and/or enterohemorrhagic *E. coli* (EHEC).

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F1 capsular antigen (Infect Immun. 2003 Jan; 71(1)): 374-383, LPS (Infect Immun. 1999 Oct; 67(10): 5395), *Yersinia pestis* V antigen (Infect Immun. 1997 Nov; 65(11): 4476-4482).

*Mycobacterium tuberculosis*: Tuberculosis antigens include lipoproteins, LPS, BCG antigens, a fusion protein of antigen 85B (Ag85B) and/or ESAT-6 optionally formulated in cationic lipid vesicles (Infect Immun. 2004 October; 72(10): 6148), *Mycobacterium tuberculosis* (Mtb) isocitrate dehydrogenase associated antigens (Proc Natl Acad Sci U S A. 2004 Aug 24; 101(34): 12652), and/or MPT51 antigens (Infect Immun. 2004 July; 72(7): 3829).

*Rickettsia*: Antigens include outer membrane proteins, including the outer membrane protein A and/or B (OmpB) (Biochim Biophys Acta. 2004 Nov 1;1702(2):145), LPS, and surface protein antigen (SPA) (J Autoimmun. 1989 Jun;2 Suppl:81).

*Listeria monocytogenes.* Bacterial antigens may be derived from Listeria monocytogenes.

*Chlamydia pneumoniae*: Antigens include those identified in WO 02/02606.

*Vibrio cholerae*: Antigens include proteinase antigens, LPS, particularly lipopolysaccharides of *Vibrio cholerae* II, O1 Inaba O-specific polysaccharides, V. cholera 0139, antigens of IEM108 vaccine (Infect Immun. 2003 Oct;71(10):5498-504), and/or Zonula occludens toxin (Zot).

*Salmonella typhi* (typhoid fever): Antigens include capsular polysaccharides preferably conjugates (Vi, *i.e.* vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins. , such as antigens associated with P39 and P13 (an integral membrane protein, Infect Immun. 2001 May; 69(5): 3323-3334), VlsE Antigenic Variation Protein (J Clin Microbiol. 1999 Dec; 37(12): 3997).

*Porphyromonas gingivalis*: Antigens include *P. gingivalis* outer membrane protein (OMP).

*Klebsiella:* Antigens include an OMP, including OMP A, or a polysaccharide optionally conjugated to tetanus

toxoid.

Further bacterial antigens of the invention may be capsular antigens, polysaccharide antigens or protein antigens of any of the above. Further bacterial antigens may also include an outer membrane vesicle (OMV) preparation. Additionally, antigens include live, attenuated, and/or purified versions of any of the aforementioned bacteria. The antigens of the present invention may be derived from gram-negative or gram-positive bacteria. The antigens of the present invention may be derived from aerobic or anaerobic bacteria.

Additionally, any of the above bacterial-derived saccharides (polysaccharides, LPS, LOS or oligosaccharides) can be conjugated to another agent or antigen, such as a carrier protein (for example CRM197). Such conjugation may be direct conjugation effected by reductive amination of carbonyl moieties on the saccharide to amino groups on the protein, as provided in US Patent No. 5,360,897 and Can J Biochem Cell Biol. 1984 May;62(5):270-5. Alternatively, the saccharides can be conjugated through a linker, such as, with succinamide or other linkages provided in Bioconjugate Techniques, 1996 and CRC, Chemistry of Protein Conjugation and Cross-Linking, 1993.

B. Viral Antigens

Viral antigens suitable for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or other substrate. Alternatively, viral antigens may be expressed recombinantly. Viral antigens preferably include epitopes which are exposed on the surface of the virus during at least one stage of its life cycle. Viral antigens are preferably conserved across multiple serotypes or isolates. Viral antigens include antigens derived from one or more of the viruses set forth below as well as the specific antigens examples identified below.

*Orthomyxovirus*: Viral antigens may be derived from an *Orthomyxovirus,* such as Influenza A, B and C. *Orthomyxovirus* antigens may be selected from one or more of the viral proteins, including hemagglutinin (HA), neuraminidase (NA), nucleoprotein (NP), matrix protein (M1), membrane protein (M2), one or more of the transcriptase components (PB1, PB2 and PA). Preferred antigens include HA and NA.

Influenza antigens may be derived from interpandemic (annual) flu strains. Alternatively influenza antigens may be derived from strains with the potential to cause pandemic a pandemic outbreak (*i.e.,* influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

Paramyxoviridae viruses: Viral antigens may be derived from Paramyxoviridae viruses, such as Pneumoviruses (RSV), Paramyxoviruses (PIV) and Morbilliviruses (Measles).

Pneumovirus: Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See *e.g.,* J Gen Virol. 2004 Nov; 85(Pt 11):3229). Pneumovirus antigens may also be formulated in or derived from chimeric viruses. For example, chimeric RSV/PIV viruses may comprise components of both RSV and PIV.

Paramyxovirus: Viral antigens may be derived from a Paramyxovirus, such as Parainfluenza virus types 1 - 4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin -Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

Morbillivirus: Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

Picornavirus: Viral antigens may be derived from Picornaviruses, such as Enteroviruses, Rhinoviruses, Heparnavirus, Cardioviruses and Aphthoviruses. Antigens derived from Enteroviruses, such as Poliovirus are preferred.

Enterovirus: Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP1, VP2, VP3 and VP4. Commercially available polio vaccines include

Inactivated Polio Vaccine (IPV) and Oral poliovirus vaccine (OPV).

Heparnavirus: Viral antigens may be derived from an Heparnavirus, such as Hepatitis A virus (HAV). Commercially available HAV vaccines include inactivated HAV vaccine.

Togavirus: Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

Flavivirus: Viral antigens may be derived from a Flavivirus, such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus vaccines.

Pestivirus: Viral antigens may be derived from a Pestivirus, such as Bovine viral diarrhea (BVDV), Classical swine fever (CSFV) or Border disease (BDV).

Hepadnavirus: Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein.

Hepatitis C virus: Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions (Houghton et al., Hepatology (1991) 14:381).

Rhabdovirus: Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

Caliciviridae; Viral antigens may be derived from Calciviridae, such as Norwalk virus, and Norwalk-like Viruses, such as Hawaii Virus and Snow Mountain Virus.

Coronavirus: Viral antigens may be derived from a Coronavirus, SARS, Human respiratory Coronavirus, Avian infectious bronchitis (IBV), Mouse hepatitis virus (MHV), and Porcine transmissible gastroenteritis virus (TGEV). Coronavirus antigens may be selected from spike (S), envelope (E), matrix (M), nucleocapsid (N), and Hemagglutinin-esterase glycoprotein (HE). Preferably, the Coronavirus antigen is derived from a SARS virus. SARS viral antigens are described in WO 04/92360;

Retrovirus: Viral antigens may be derived from a Retrovirus, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, pol, env, tax, tat, rex, rev, nef, vif, vpu, and vpr. HIV antigens may be selected from gag (p24gag and p55gag), env (gp160 and gp41), pol, tat, nef, rev vpu, miniproteins, (preferably p55 gag and gp140v delete). HIV antigens may be derived from one or more of the following strains: HIVIIIb, HIVSF2, HIVLAV, HIVLAI, HIVMN, HIV-1CM235, HIV-1US4.

Reovirus: Viral antigens may be derived from a Reovirus, such as an Orthoreovirus, a Rotavirus, an Orbivirus, or a Coltivirus. Reovirus antigens may be selected from structural proteins λ1, λ2, λ3, μ1, μ2, σ1, σ2, or σ3, or nonstructural proteins σNS, μNS, or σ1s. Preferred Reovirus antigens may be derived from a Rotavirus. Rotavirus antigens may be selected from VP1, VP2, VP3, VP4 (or the cleaved product VP5 and VP8), NSP 1, VP6, NSP3, NSP2, VP7, NSP4, or NSP5. Preferred Rotavirus antigens include VP4 (or the cleaved product VP5 and VP8), and VP7.

Parvovirus: Viral antigens may be derived from a Parvovirus, such as Parvovirus B19. Parvovirus antigens may be selected from VP-1, VP-2, VP-3, NS-1 and NS-2. Preferably, the Parvovirus antigen is capsid protein VP-2.

Delta hepatitis virus (HDV): Viral antigens may be derived HDV, particularly δ-antigen from HDV (see, *e.g.,* U.S. Patent No. 5,378,814).

Hepatitis E virus (HEV): Viral antigens may be derived from HEV.

Hepatitis G virus (HGV): Viral antigens may be derived from HGV.

Human Herpesvirus: Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (α), early proteins (β), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is Commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins

Papovaviruses: Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

Further provided are antigens, compositions, methods, and microbes included in Vaccines, 4th Edition (Plotkin and Orenstein ed. 2004); Medical Microbiology 4th Edition (Murray et al. ed. 2002); Virology, 3rd Edition (W.K. Joklik ed. 1988); Fundamental Virology, 2nd Edition (B.N. Fields and D.M. Knipe, eds. 1991), which are contemplated in conjunction with the compositions of the present invention.

C. Fungal Antigens

Fungal antigens for use in the invention may be derived from one or more of the fungi set forth below.

Fungal antigens may be derived from Dermatophytres, including: Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum var. album, var. discoides, var. ochraceum, Trichophyton violaceum, and/or Trichophyton faviforme.

Fungal pathogens may be derived from Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neofonnans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing fungal antigens are well known in the art (see US Patent No. 6,333,164). In a preferred method a solubilized fraction extracted and separated from an insoluble fraction obtainable from fungal cells of which cell wall has been substantially removed or at least partially removed, characterized in that the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

D. STD Antigens

The compositions of the invention may include one or more antigens derived from a sexually transmitted disease (STD). Such antigens may provide for prophylactis or therapy for STD's such as chlamydia, genital herpes, hepatits (such as HCV), genital warts, gonorrhoea, syphilis and/or chancroid (See, WO00/15255). Antigens may be derived from one or more viral or bacterial STD's. Viral STD antigens for use in the invention may be derived from, for example, HIV, herpes simplex virus (HSV-1 and HSV-2), human papillomavirus (HPV), and hepatitis (HCV). Bacterial STD antigens for use in the invention may be derived from, for example, Neiserria gonorrhoeae, Chlamydia trachomatis, Treponema pallidum, Haemophilus ducreyi, E. coli, and Streptococcus agalactiae. Examples of specific antigens derived from these pathogens are described above.

E. Respiratory Antigens

The compositions of the invention may include one or more antigens derived from a pathogen which causes respiratory disease. For example, respiratory antigens may be derived from a respiratory virus such as Orthomyxoviruses (influenza), Pneumovirus (RSV), Paramyxovirus (PIV), Morbillivirus (measles), Togavirus (Rubella), VZV, and Coronavirus (SARS). Respiratory antigens may be derived from a bacteria which causes respiratory disease, such as Streptococcus pneumoniae, Pseudomonas aeruginosa, Bordetella pertussis, Mycobacterium tuberculosis, Mycoplasma pneumoniae, Chlamydia pneumoniae, Bacillus anthracis, and Moraxella catarrhalis. Examples of specific antigens derived

from these pathogens are described above.

F. Pediatric Vaccine Antigens

The compositions of the invention may include one or more antigens suitable for use in pediatric subjects. Pediatric subjects are typically less than about 3 years old, or less than about 2 years old, or less than about 1 years old. Pediatric antigens may be administered multiple times over the course of 6 months, 1, 2 or 3 years. Pediatric antigens may be derived from a virus which may target pediatric populations and/or a virus from which pediatric populations are susceptible to infection. Pediatric viral antigens include antigens derived from one or more of Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), and Varicella-zoster virus (VZV), Epstein Barr virus (EBV). Pediatric bacterial antigens include antigens derived from one or more of Streptococcus pneumoniae, Neisseria meningitides, Streptococcus pyogenes (Group A Streptococcus), Moraxella catarrhalis, Bordetella pertussis, Staphylococcus aureus, Clostridium tetani (Tetanus), Cornynebacterium diphtheriae (Diphtheria), Haemophilus influenzae B (Hib), Pseudomonas aeruginosa, Streptococcus agalactiae (Group B Streptococcus), and E. coli. Examples of specific antigens derived from these pathogens are described above.

G. Antigens suitable for use in Elderly or Immunocompromised Individuals

The compositions of the invention may include one or more antigens suitable for use in elderly or immunocompromised individuals. Such individuals may need to be vaccinated more frequently, with higher doses or with adjuvanted formulations to improve their immune response to the targeted antigens. Antigens which may be targeted for use in Elderly or Immunocompromised individuals include antigens derived from one or more of the following pathogens: Neisseria meningitides, Streptococcus pneumoniae, Streptococcus pyogenes (Group A Streptococcus), Moraxella catarrhalis, Bordetella pertussis, Staphylococcus aureus, Staphylococcus epidermis, Clostridium tetani (Tetanus), Cornynebacterium diphtheriae (Diphtheria), Haemophilus influenzae B (Hib), Pseudomonas aeruginosa, Legionella pneumophila, Streptococcus agalactiae (Group B Streptococcus), Enterococcus faecalis, Helicobacter pylori, Clamydia pneumoniae, Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), Varicella-zoster virus (VZV), Epstein Barr virus (EBV), Cytomegalovirus (CMV). Examples of specific antigens derived from these pathogens are described above.

H. Antigens suitable for use in Adolescent Vaccines

The compositions of the invention may include one or more antigens suitable for use in adolescent subjects. Adolescents may be in need of a boost of a previously administered pediatric antigen. Pediatric antigens which may be suitable for use in adolescents are described above. In addition, adolescents may be targeted to receive antigens derived from an STD pathogen in order to ensure protective or therapeutic immunity before the beginning of sexual activity. STD antigens which may be suitable for use in adolescents are described above.

I. Antigen Formulations

In other aspects of the invention, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly($\alpha$-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weight-to-weight detergent-to-polymer ratio of from about 0.00001:1 to about 0.1:1 (more typically about 0.0001:1 to about 0.1:1, about 0.001:1 to about 0.1:1, or about 0.005:1 to about 0.1:1); (b) removing the organic solvent from the emulsion; and (c) adsorbing an antigen on the surface of the microparticles. In certain embodiments, the biodegradable polymer is present at a concentration of about 3% to about 10% relative to the organic solvent.

Microparticles for use herein will be formed from materials that are sterilizable, non-toxic and biodegradable. Such materials include, without limitation, poly($\alpha$-hydroxy acid), polyhydroxybutyric acid, polycaprolactone, polyorthoester, polyanhydride, PACA, and polycyanoacrylate. Preferably, microparticles for use with the present invention are derived from a poly($\alpha$-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of

which will be largely a matter of choice, depending in part on the coadministered macromolecule. These parameters are discussed more fully below.

Further antigens may also include an outer membrane vesicle (OMV) preparation.

Additional formulation methods and antigens (especially tumor antigens) are provided in U.S. Patent Serial No. 09/581,772.

J. Antigen References

The following references include antigens useful in conjunction with the compositions of the present invention:


1 International patent application WO99/24578
2 International patent application WO99/36544.
3 International patent application WO99/57280.
4 International patent application WO00/22430.
5 Tettelin et al. (2000) Science 287:1809-1815.
6 International patent application WO96/29412.
7 Pizza et al. (2000) Science 287:1816-1820.
8 PCT WO 01/52885.
9 Bjune et al. (1991) Lancet 338(8775).
10 Fuskasawa et al. (1999) Vaccine 17:2951-2958.
11 Rosenqist et al. (1998) Dev. Biol. Strand 92:323-333.
12 Constantino et al. (1992) Vaccine 10:691-698.
13 Constantino et al. (1999) Vaccine 17:1251-1263.
14 Watson (2000) Pediatr Infect Dis J 19:331-332.
15 Rubin (20000) Pediatr Clin North Am 47:269-285,v.
16 Jedrzejas (2001) Microbiol Mol Biol Rev 65:187-207.
17 International patent application filed on 3rd July 2001 claiming priority from GB-0016363.4;WO 02/02606; PCT IB/01/00166.
18 Kalman et al. (1999) Nature Genetics 21:385-389.
19 Read et al. (2000) Nucleic Acids Res 28:1397-406.
20 Shirai et al. (2000) J. Infect. Dis 181(Suppl 3):S524-S527.
21 International patent application WO99/27105.
22 International patent application WO00/27994.
23 International patent application WO00/37494.
24 International patent application WO99/28475.
25 Bell (2000) Pediatr Infect Dis J 19:1187-1188.
26 Iwarson (1995) APMIS 103:321-326.
27 Gerlich et al. (1990) Vaccine 8 Suppl:S63-68 & 79-80.
28 Hsu et al. (1999) Clin Liver Dis 3:901-915.
29 Gastofsson et al. (1996) N. Engl. J. Med. 334-:349-355.
30 Rappuoli et al. (1991) TIBTECH 9:232-238.
31 Vaccines (1988) eds. Plotkin & Mortimer. ISBN 0-7216-1946-0.
32 Del Guidice et al. (1998) Molecular Aspects of Medicine 19:1-70.
33 International patent application WO93/018150.
34 International patent application WO99/53310.
35 International patent application WO98/04702.
36 Ross et al. (2001) Vaccine 19:135-142.
37 Sutter et al. (2000) Pediatr Clin North Am 47:287-308.
38 Zimmerman & Spann (1999) Am Fan Physician 59:113-118, 125-126.
39 Dreensen (1997) Vaccine 15 Suppl"S2-6.
40 MMWR Morb Mortal Wkly rep 1998 Jan 16:47(1):12, 9.
41 McMichael (2000) Vaccine 19 Suppl 1:S101-107.
42 Schuchat (1999) Lancer 353(9146):51-6.
43 GB patent applications 0026333.5, 0028727.6 & 0105640.7.
44 Dale (1999) Infect Disclin North Am 13:227-43, viii.
45 Ferretti et al. (2001) PNAS USA 98: 4658-4663.
46 Kuroda et al. (2001) Lancet 357(9264):1225-1240; see also pages 1218-1219.
47 Ramsay et al. (2001) Lancet 357(9251): 195-196.

48 Lindberg (1999) Vaccine 17 Suppl 2:S28-36.
49 Buttery & Moxon (2000) J R Coil Physicians Long 34:163-168.
50 Ahmad & Chapnick (1999) Infect Dis Clin North Am 13:113-133, vii.
51 Goldblatt (1998) J. Med. Microbiol. 47:663-567.
52 European patent 0 477 508.
53 U.S. Patent No. 5,306,492.
54 International patent application WO98/42721.
55 Conjugate Vaccines (eds. Cruse et al.) ISBN 3805549326, particularly vol. 10:48-114.
56 Hermanson (1996) Bioconjugate Techniques ISBN: 012323368 & 012342335X.
57 European patent application 0372501.
58 European patent application 0378881.
59 European patent application 0427347.
60 International patent application WO93/17712.
61 International patent application WO98/58668.
62 European patent application 0471177.
63 International patent application WO00/56360.
64 International patent application WO00/67161.

Where a saccharide or carbohydrate antigen is used, it is preferably conjugated to a carrier protein in order to enhance immunogenicity. See Ramsay et al. (2001) Lancet 357(9251):195-196; Lindberg (1999) Vaccine 17 Suppl 2:S28-36; Buttery & Moxon (2000) J R Coll Physicians Lond 34:163-168; Ahmad & Chapnick (1999) Infect Dis Clin North Am 13:113-133, vii; Goldblatt (1998) J. Med. Microbiol. 47:563-567; European patent 0 477 508; US Patent No. 5,306,492; WO98/42721; Conjugate Vaccines (eds. Cruse et al.) ISBN 3805549326, particularly vol. 10:48-114; Hermanson (1996) Bioconjugate Techniques ISBN: 0123423368 or 012342335X. Preferred carrier proteins are bacterial toxins or toxoids, such as diphtheria or tetanus toxoids. The CRM197 diphtheria toxoid is particularly preferred.

Other carrier polypeptides include the *N. meningitidis* outer membrane protein (EP-A-0372501), synthetic peptides (EP-A-0378881 and EP-A 0427347), heat shock proteins (WO 93/17712 and WO 94/03208), pertussis proteins (WO 98/58668 and EP A 0471177), protein D from H. influenzae (WO 00/56360), cytokines (WO 91/01146), lymphokines, hormones, growth factors, toxin A or B from C. difficile (WO 00/61761), iron-uptake proteins (WO 01/72337), etc. Where a mixture comprises capsular saccharide from both serigraphs A and C, it may be preferred that the ratio (w/w) of MenA saccharide:MenC saccharide is greater than 1 (*e.g.,* 2:1, 3:1, 4:1, 5:1, 10:1 or higher). Different saccharides can be conjugated to the same or different type of carrier protein. Any suitable conjugation reaction can be used, with any suitable linker where necessary.

Toxic protein antigens may be detoxified where necessary *e.g.*, detoxification of pertussis toxin by chemical and/or genetic means.

*Pharmaceutically acceptable carriers*

Compositions of the invention will typically, in addition to the components mentioned above, comprise one or more "pharmaceutically acceptable carriers." These include any carrier which does not itself induce the production of antibodies harmful to the individual receiving the composition. Suitable carriers typically are large, slowly metabolized macromolecules such as proteins, polysaccharides, polylactic acids, polyglycolic acids, polymeric amino acids, amino acid copolymers, and lipid aggregates (such as oil droplets or liposomes). Such carriers are well known to those of ordinary skill in the art. A composition may also contain a diluent, such as water, saline, glycerol, etc. Additionally, an auxiliary substance, such as a wetting or emulsifying agent, pH buffering substance, and the like, may be present. A thorough discussion of pharmaceutically acceptable components is available in Gennaro (2000) Remington: The Science and Practice of Pharmacy, 20th ed., ISBN: 0683306472.

*Immunoregulatory Agents*

*Adjuvants*

Vaccines of the invention may be administered in conjunction with other immunoregulatory agents. In particular, compositions will usually include an adjuvant. Adjuvants for use with the invention include, but are not limited to, one or more of the following set forth below:

A. Mineral Containing Compositions

Mineral containing compositions suitable for use as adjuvants in the invention include mineral salts, such as aluminum salts and calcium salts. The invention includes mineral salts such as hydroxides (*e.g.* oxyhydroxides), phosphates (*e.g.* hydroxyphosphates, orthophosphates), sulfates, etc. (*e.g.* see chapters 8 & 9 of Vaccine Design... (1995) eds. Powell & Newman. ISBN: 030644867X, Plenum Press), or mixtures of different mineral compounds (*e.g.* a mixture of a phosphate and a hydroxide adjuvant, optionally with an excess of the phosphate), with the compounds taking any suitable form (*e.g.* gel, crystalline, amorphous, etc.), and with adsorption to the salt(s) being preferred. The mineral containing compositions may also be formulated as a particle of metal salt (WO00/23105).

Aluminum salts may be included in vaccines of the invention such that the dose of $Al^{3+}$ is between 0.2 and 1.0 mg per dose.

In one embodiment the aluminum based adjuvant for use in the present invention is alum (aluminum potassium sulfate ($AlK(SO_4)_2$)), or an alum derivative, such as that formed in-situ by mixing an antigen in phosphate buffer with alum, followed by titration and precipitation with a base such as ammonium hydroxide or sodium hydroxide.

Another aluminum-based adjuvant for use in vaccine formulations of the present invention is aluminum hydroxide adjuvant ($Al(OH)_3$) or crystalline aluminum oxyhydroxide (AlOOH), which is an excellent adsorbant, having a surface area of approximately 500$m^2$/g. Alternatively, aluminum phosphate adjuvant ($AlPO_4$) or aluminum hydroxyphosphate, which contains phosphate groups in place of some or all of the hydroxyl groups of aluminum hydroxide adjuvant is provided. Preferred aluminum phosphate adjuvants provided herein are amorphous and soluble in acidic, basic and neutral media.

In another embodiment the adjuvant of the invention comprises both aluminum phosphate and aluminum hydroxide. In a more particular embodiment thereof, the adjuvant has a greater amount of aluminum phosphate than aluminum hydroxide, such as a ratio of 2:1, 3:1, 4:1, 5:1, 6:1, 7:1, 8:1, 9:1 or greater than 9:1, by weight aluminum phosphate to aluminum hydroxide. More particular still, aluminum salts in the vaccine are present at 0.4 to 1.0 mg per vaccine dose, or 0.4 to 0.8 mg per vaccine dose, or 0.5 to 0.7 mg per vaccine dose, or about 0.6 mg per vaccine dose.

Generally, the preferred aluminum-based adjuvant(s), or ratio of multiple aluminum-based adjuvants, such as aluminum phosphate to aluminum hydroxide is selected by optimization of electrostatic attraction between molecules such that the antigen carries an opposite charge as the adjuvant at the desired pH. For example, aluminum phosphate adjuvant (isoelectric point = 4) adsorbs lysozyme, but not albumin at pH 7.4. Should albumin be the target, aluminum hydroxide adjuvant would be selected (iep 11.4). Alternatively, pretreatment of aluminum hydroxide with phosphate lowers its isoelectric point, making it a preferred adjuvant for more basic antigens.

B. Oil-Emulsions

Oil-emulsion compositions suitable for use as adjuvants in the invention include squalene-water emulsions, such as MF59 (5% Squalene, 0.5% TWEEN™ 80, and 0.5% Span 85, formulated into submicron particles using a microfluidizer). See WO90/14837. See also, Podda, Vaccine (2001) 19: 2673-2680; Frey et al., Vaccine (2003) 21:4234-4237. MF59 is used as the adjuvant in the FLUAD™ influenza virus trivalent subunit vaccine.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v TWEEN™ 80□ (polyoxyelthylenesorbitan monooleate), and/or 0.25-1.0% SPAN 85™ (sorbitan trioleate), and, optionally, N-acetyl-muramyl-L-alanyl-D-isogluatminyl-L-alanine-2-(1'-2'-dipalmitoyl-sn-glycero-3-huydroxyphosphophoryloxy)-ethylamine (MTP-PE), for example, the submicron oil-in-water emulsion known as "MF59" (International Publication No. WO90/14837; US Patent Nos. 6,299,884 and 6,451,325, and Ott et al., in Vaccine Design: The Subunit and Adjuvant Approach (Powell, M.F. and Newman, M.J. eds.) Plenum Press, New York, 1995, pp. 277-296). MF59 contains 4-5% w/v Squalene (*e.g.* 4.3%), 0.25-0.5% w/v TWEEN™ 80, and 0.5% w/v SPAN 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 μg/dose, more preferably 0-250 μg/dose and most preferably, 0-100 μg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 μg MTP-PE per dose, and so on. MF69, another submicron oil-in-water emulsion for use herein, contains 4.3% w/v squalene, 0.25% w/v TWEEN™ 80, and 0.75% w/v SPAN 85™ and optionally MTP-PE. Yet another submicron oil-in-water emulsion is MF75, also known as SAF, containing 10% squalene, 0.4% TWEEN™ 80, 5% pluronic-blocked polymer L121, and thr-MDP, also microfluidized into a submicron emulsion. MF75-MTP denotes an MF75 formulation that includes MTP, such as from 100-400 μg MTP-PE per dose.

Submicron oil-in-water emulsions, methods of making the same and immunostimulating agents, such as muramyl peptides, for use in the compositions, are described in detail in WO90/14837 and U.S. Patents 6,299,884 and

6,45 1,325.

Complete Freund's adjuvant (CFA) and incomplete Freund's adjuvant (IFA) may also be used as adjuvants in the invention.

C. Saponin Formulations

Saponin formulations, may also be used as adjuvants in the invention. Saponins are a heterologous group of sterol glycosides and triterpenoid glycosides that are found in the bark, leaves, stems, roots and even flowers of a wide range of plant species. Saponins isolated from the bark of the Quillaia saponaria Molina tree have been widely studied as adjuvants. Saponins can also be commercially obtained from Smilax omata (sarsaprilla), Gypsophilla paniculata (brides veil), and Saponaria officianalis (soap root). Saponin adjuvant formulations include purified formulations, such as QS21, as well as lipid formulations, such as ISCOMs.

Saponin compositions have been purified using High Performance Thin Layer Chromatography (HP-TLC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in U.S. Patent 5,057,540. Saponin formulations may also comprise a sterol, such as cholesterol (see WO96/33739).

Combinations of saponins and cholesterols can be used to form unique particles called Immunostimulating Complexes (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP0109942, WO96/11711 and WO96/33739. Optionally, the ISCOMS may be devoid of (an) additional detergent(s). See WO00/07621.

A review of the development of saponin based adjuvants can be found in Barr, et al., Advanced Drug Delivery Reviews (1998) 32:247-271. *See also* Sjolander, et al., Advanced Drug Delivery Reviews (1998) 32:321-338.

D. Virosomes and Virus Like Particles (VLPs)

Virosomes and Virus Like Particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO03/024480, WO03/024481, and Niikura et al., Virology (2002) 293:273-280; Lenz et al., Journal of Immunology (2001) 5246-5355; Pinto, et al., Journal of Infectious Diseases (2003) 188:327-338; and Gerber et al., Journal of Virology (2001) 75(10):4752-4760. Virosomes are discussed further in, for example, Gluck et al., Vaccine (2002) 20:B10 -B16. Immunopotentiating reconstituted influenza virosomes (IRIV) are used as the subunit antigen delivery system in the intranasal trivalent INFLEXAL™ product {Mischler & Metcalfe (2002) Vaccine 20 Suppl 5:B17-23} and the INFLUVAC PLUS™ product.

E. Bacterial or Microbial Derivatives

Adjuvants suitable for use in the invention include bacterial or microbial derivatives such as:

(1) Non-toxic derivatives of enterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g.* RC 529. See Johnson et al. (1999) Bioorg Med Chem Lett 9:2273-2278.

(2) Lipid A Derivatives

Lipid A derivatives include derivatives of lipid A from Escherichia coli such as OM-174. OM-174 is described for example in Meraldi et al., Vaccine (2003) 21:2485-2491; and Pajak, et al., Vaccine (2003) 21:836-842.

(3) Immunostimulatory oligonucleotides

Immunostimulatory oligonucleotides suitable for use as adjuvants in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpGs can include nucleotide moditications/analogs such as phosphorothioate modifications and can be double-stranded or single-stranded. Optionally, the guanosine may be replaced with an analog such as 2'-deoxy-7-deazaguanosine. See Kandimalla, et al., Nucleic Acids Research (2003) 31(9): 2393-2400; WO02/26757 and WO99/62923 for examples of possible analog substitutions. The adjuvant effect of CpG oligonucleotides is further discussed in Krieg, Nature Medicine (2003) 9(7): 831-835; McCluskie, et al., FEMS Immunology and Medical Microbiology (2002) 32:179-185; WO98/40100; US Patent No. 6,207,646; US Patent No. 6,239,116 and US Patent No. 6,429,199.

The CpG sequence may be directed to TLR9, such as the motif GTCGTT or TTCGTT. See Kandimalla, et al., Biochemical Society Transactions (2003) 31 (part 3): 654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell, et al., J. Immunol. (2003) 170(8):4061-4068; Krieg, TRENDS in Immunology (2002) 23(2): 64-65 and WO01/95935. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, Kandimalla, et al., BBRC (2003) 306:948-953; Kandimalla, et al., Biochemical Society Transactions (2003) 31 (part 3):664-658; Bhagat et al., BBRC (2003) 300:853-861 and WO03/035836.

(4) ADP-ribosylating toxins and detoxified derivatives thereof.

Bacterial ADP-ribosylating toxins and detoxified derivatives thereof may be used as adjuvants in the invention. Preferably, the protein is derived from *E. coli* (*i.e.*, *E. coli* heat labile enterotoxin "LT), cholera ("CT"), or pertussis ("PT"). The use of detoxified ADP-ribosylating toxins as mucosal adjuvants is described in WO95/17211 and as parenteral adjuvants in WO98/42375. Preferably, the adjuvant is a detoxified LT mutant such as LT-K63, LT-R72, and LTR192G. The use of ADP-ribosylating toxins and detoxified derivatives thereof, particularly LT-K63 and LT-R72, as adjuvants can be found in the following references: Beignon, et al., Infection and Immunity (2002) 70(6):3012-3019; Pizza, et al., Vaccine (2001) 19:2534-2541; Pizza, et al., Int. J. Med. Microbiol (2000) 290(4-5):455-461; Scharton-Kersten et al., Infection and Immunity (2000) 68(9):5306-5313; Ryan et al., Infection and Immunity (1999) 67(12):6270-6280; Partidos et al., Immunol. Lett. (1999) 67(3):209-216; Peppoloni et al., Vaccines (2003) 2(2):285-293; and Pine et al., (2002) J. Control Release (2002) 85(1-3):263-270. Numerical reference for amino acid substitutions is preferably based on the alignments of the A and B subunits of ADP-ribosylating toxins set forth in Domenighini et al., Mol. Microbiol (1995) 15(6):1165-1167.

F. Bioadhesives and Mucoadhesives

Bioadhesives and mucoadhesives may also be used as adjuvants in the invention. Suitable bioadhesives include esterified hyaluronic acid microspheres (Singh et al. (2001) J. Cont. Rele. 70:267-276) or mucoadhesives such as cross-linked derivatives of polyacrylic acid, polyvinyl alcohol, polyvinyl pyrollidone, polysaccharides and carboxymethylcellulose. Chitosan and derivatives thereof may also be used as adjuvants in the invention. *See* WO99/27960.

G. Microparticles

Microparticles may also be used as adjuvants in the invention. Microparticles (*i.e.* a particle of ~100nm to ~150μm in diameter, more preferably ~200nm to ~30μm in diameter, and most preferably ~500nm to ~10μm in diameter) formed from materials that are biodegradable and non toxic (*e.g.* a poly(α-hydroxy acid), a polyhydroxybutyric acid, a polyorthoester, a polyanhydride, a polycaprolactone, etc.), with poly(lactide co glycolide) are preferred, optionally treated to have a negatively-charged surface (*e.g.* with SDS) or a positively-charged surface (*e.g.* with a cationic detergent, such as CTAB).

H. Liposomes

Examples of liposome formulations suitable for use as adjuvants are described in US Patent No. 6,090,406, US Patent No. 5,916,588, and EP 0 626 169.

I. Polyoxyethylene ether and Polyoxyethylene Ester Formulations

Adjuvants suitable for use in the invention include polyoxyethylene ethers and polyoxyethylene esters. WO99/52549. Such formulations further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO01/21152).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

J. Polyphosphazene (PCPP)

PCPP formulations are described, for example, in Andrianov et al., "Preparation of hydrogel microspheres by coacervation of aqueous polyphophazene solutions", Biomaterials (1998) 19(1-3):109-115 and Payne et al., "Protein Release from Polyphosphazene Matrices", Adv. Drug. Delivery Review (1998) 31(3): 185-196.

K. Muramyl peptides

Examples of muramyl peptides suitable for use as adjuvants in the invention include N-acetyl-muramyl-L-threonyl-D-isoglutamine (thr-MDP), N-acetyl-normuramyl-1-alanyl-d-isoglutamine (nor-MDP), and N acetylmuramyl-1-alanyl-d-isoglutaminyl-1-alanine-2-(1'-2'-dipalmitoyl-sn-glycero-3-hydroxyphosphoryloxy)-ethylamine MTP-PE).

L. Imidazoquinoline Compounds.

Examples of imidazoquinoline compounds suitable for use adjuvants in the invention include Imiquimod and its analogues, described further in Stanley, Clin Exp Dermatol (2002) 27(7):571-577; Jones, Curr Opin Investig Drugs (2003) 4(2):214-218; and U.S. Patents 4,689,338, 5,389,640, 5,268,376, 4,929,624, 5,266,575, 5,352,784, 5,494,916, 5,482,936, 5,346,905, 5,395,937, 5,238,944, and 5,525,612.

M. Thiosemicarbazone Compounds.

Examples of thiosemicarbazone compounds, as well as methods of formulating, manufacturing, and screening for compounds all suitable for use as adjuvants in the invention include those described in WO04/60308. The thiosemicarbazones are particularly effective in the stimulation of human peripheral blood mononuclear cells for the production of cytokines, such as TNF-$\alpha$.

N. Tryptanthrin Compounds.

Examples of tryptanthrin compounds, as well as methods of formulating, manufacturing, and screening for compounds all suitable for use as adjuvants in the invention include those described in WO04/64759. The tryptanthrin compounds are particularly effective in the stimulation of human peripheral blood mononuclear cells for the production of cytokines, such as TNF-$\alpha$.

The invention may also comprise combinations of aspects of one or more of the adjuvants identified above. For example, the following adjuvant compositions may be used in the invention:

(1) a saponin and an oil-in-water emulsion (WO099/11241);

(2) a saponin (*e.g.,* QS21) + a non-toxic LPS derivative (*e.g.* 3dMPL) (see WO94/00153);

(3) a saponin (*e.g.,* QS21) + a non-toxic LPS derivative (*e.g.* 3dMPL) + a cholesterol;

(4) a saponin (*e.g.*, QS21) + 3dMPL + IL 12 (optionally + a sterol) (WO98/57659);

(5) combinations of 3dMPL with, for example, QS21 and/or oil-in-water emulsions (See European patent applications 0835318, 0735898 and 0761231);

(6) SAF, containing 10% Squalane, 0.4% Tween 80, 5% pluronic-block polymer L121, and thr-MDP, either microfluidized into a submicron emulsion or vortexed to generate a larger particle size emulsion.

(7) RIBI™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (DETOX™); and

(8) one or more mineral salts (such as an aluminum salt) + a non-toxic derivative of LPS (such as 3dPML).

(9) one or more mineral salts (such as an aluminum salt) + an immunostimulatory oligonucleotide (such as a nucleotide sequence including a CpG motif).

O. Human Immunomodulators

Human immunomodulators suitable for use as adjuvants in the invention include cytokines, such as interleukins (*e.g.* IL-1, IL-2, IL-4, IL-5, IL-6, IL-7, IL-12, etc.), interferons (*e.g.* interferon-$\gamma$), macrophage colony stimulating factor, and tumor necrosis factor.

Aluminum salts and MF59 are preferred adjuvants for use with injectable influenza vaccines. Bacterial toxins and bioadhesives are preferred adjuvants for use with mucosally-delivered vaccines, such as nasal vaccines.

*Therapeutic methods*

The invention provides methods for inducing or increasing an immune response to *S. pyogenes* using the compositions described above. The immune response is preferably protective and can include antibodies and/or cell-mediated immunity (including systemic and mucosal immunity). Immune responses include booster responses.

The combinations of GAS antigens described above may be included in a single composition for simultaneous administration. Alternatively, the combinations of GAS antigens may be administered sequentially. For example, where the combination comprises Spy0167, Spy0269, and Spy0416 or mutants or fragments thereof, these 3 antigens may be administered simultaneously in a single composition or sequentially in separate compositions. In this situation, the invention provides: Spy0167 for administration to an animal that has already received Spy0269 and/or Spy416; Spy0269 for administration to an animal that has already received Spy0167 and/or Spy0416; and Spy0416 for administration to an animal that has already received Spy0167 and/or Spy0269.

Teenagers and children, including toddles and infants, can receive a vaccine for prophylactic use; therapeutic vaccines typically are administered to teenagers or adults. A vaccine intended for children may also be administered to adults *e.g.,* to assess safety, dosage, immunogenicity, etc.

Diseases caused by *Streptococcus pyogenes* which compositions of the invention can reduce the risk of, prevent, or treat include, but are not limited to, pharyngitis (such as streptococcal sore throat), scarlet fever, impetigo, erysipelas, cellulitis, septicemia, toxic shock syndrome, necrotizing fasciitis, and sequelae such as rheumatic fever and acute glomerulonephritis. The compositions may also be effective against other streptococcal bacteria, *e.g.*, GBS.

*Tests to determine the efficacy of the immune response*

One way of assessing efficacy of therapeutic treatment involves monitoring GAS infection after administration of the composition of the invention. One way of assessing efficacy of prophylactic treatment involves monitoring immune responses against the GAS antigens in the compositions of the invention after administration of the composition.

Another way of assessing the immunogenicity of the component proteins of the immunogenic compositions of the present invention is to express the GAS antigens recombinantly and to screen patient sera or mucosal secretions by immunoblot. A positive reaction between the protein and the patient serum indicates that the patient has previously mounted an immune response to the protein in question; *i.e.,* the protein is an immunogen. This method may also be used to identify immunodominant proteins and/or epitopes.

Another way of checking efficacy of therapeutic treatment involves monitoring GAS infection after administration of the compositions of the invention. One way of checking efficacy of prophylactic treatment involves monitoring immune responses both systemically (such as monitoring the level of IgG1 and IgG2a production) and mucosally (such as monitoring the level of IgA production) against GAS challenge after administration of the composition. Typically, serum specific antibody responses are determined post-immunization but pre-challenge whereas mucosal specific antibody body responses are determined post-immunization and post-challenge.

The vaccine compositions of the present invention can be evaluated in *in vitro* and *in vivo* animal models prior to host, *e.g.*, human, administration. Particularly useful mouse models include those in which intraperitoneal immunization is followed by either intraperitoneal challenge or intranasal challenge.

The efficacy of immunogenic compositions of the invention can also be determined *in vivo* by immunizing animal models, (*e.g.,* guinea pigs or mice) with the immunogenic compositions and ascertaining the level of protection obtained

after challenge with GAS.

*In vivo* efficacy models include but are not limited to: (i) a murine infection model using human GAS serotypes; (ii) a murine disease model which is a murine model using a mouse-adapted GAS strain, such as the M23 strain which is particularly virulent in mice, and (iii) a primate model using human GAS isolates.

The immune response may be one or both of a TH1 immune response and a TH2 response. The immune response may be an improved or an enhanced or an altered immune response. The immune response may be one or both of a systemic and a mucosal immune response. Preferably the immune response is an enhanced system and/or mucosal response.

An enhanced systemic and/or mucosal immunity is reflected in an enhanced TH1 and/or TH2 immune response. Preferably, the enhanced immune response includes an increase in the production of IgG1 and/or IgG2a and/or IgA.

Preferably the mucosal immune response is a TH2 immune response. Preferably, the mucosal immune response includes an increase in the production of IgA.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune response will include an increase in IgG1 production.

A TH1 immune response may include one or more of an increase in CTLs, an increase in one or more of the cytokines associated with a TH1 immune response (such as IL-2, IFN$\gamma$, and TNF$\beta$), an increase in activated macrophages, an increase in NK activity, or an increase in the production of IgG2a. Preferably, the enhanced TH1 immune response will include an increase in IgG2a production.

Immunogenic compositions of the invention may be used either alone or in combination with other GAS antigens optionally with an immunoregulatory agent capable of eliciting a Th1 and/or Th2 response.

The invention also comprises an immunogenic composition comprising one or more immunoregulatory agent, such as a mineral salt, such as an aluminium salt and an oligonucleotide containing a CpG motif. Most preferably, the immunogenic composition includes both an aluminium salt and an oligonucleotide containing a CpG motif. Alternatively, the immunogenic composition includes an ADP ribosylating toxin, such as a detoxified ADP ribosylating toxin and an oligonucleotide containing a CpG motif. Preferably, one or more of the immunoregulatory agents include an adjuvant. The adjuvant may be selected from one or more of the group consisting of a TH1 adjuvant and TH2 adjuvant.

The compositions of the invention will preferably elicit both a cell mediated immune response as well as a humoral immune response in order to effectively address a GAS infection. This immune response will preferably induce long lasting (*e.g.*, neutralizing) antibodies and a cell mediated immunity that can quickly respond upon exposure to one or more GAS antigens.

In one particularly preferred embodiment, the immunogenic composition comprises one or more GAS antigens which elicit(s) a neutralizing antibody response and one or more GAS antigens which elicit(s) a cell mediated immune response. In this way, the neutralizing antibody response prevents or inhibits an initial GAS infection while the cell-mediated immune response capable of eliciting an enhanced Th1 cellular response prevents further spreading of the GAS infection.

Compositions of the invention will generally be administered directly to a patient. The compositions of the present invention may be administered, either alone or as part of a composition, via a variety of different routes. Certain routes may be favored for certain compositions, as resulting in the generation of a more effective immune response, preferably a CMI response, or as being less likely to induce side effects, or as being easier for administration.

Delivery methods include parenteral injection (*e.g.*, subcutaneous, intraperitoneal, intravenous, intramuscular, or interstitial injection) and rectal, oral (*e.g.*, tablet, spray), vaginal, topical, transdermal (*e.g.,* see WO 99/27961), transcutaneous (*e.g.,* see WO02/074244 and WO02/064162), intranasal (*e.g.*, see WO03/028760), ocular, aural, and pulmonary or other mucosal administration.

By way of example, the compositions of the present invention may be administered via a systemic route or a mucosal route or a transdermal route or it may be administered directly into a specific tissue. As used herein, the term "systemic administration" includes but is not limited to any parenteral routes of administration. In particular, parenteral administration includes but is not limited to subcutaneous, intraperitoneal, intravenous, intraarterial, intramuscular, or intrasternal injection, intravenous, intraarterial, or kidney dialytic infusion techniques. Preferably, the systemic, parenteral administration is intramuscular injection. As used herein, the term "mucosal administration" includes but is not limited to oral, intranasal, intravaginal, intrarectal, intratracheal, intestinal and ophthalmic administration.

Dosage treatment can be a single dose schedule or a multiple dose schedule. Multiple doses may be used in a primary immunization schedule and/or in a booster immunization schedule. In a multiple dose schedule the various doses may be given by the same or different routes *e.g.*, a parenteral prime and mucosal boost, a mucosal prime and parenteral boost, etc.

The compositions of the invention may be prepared in various forms. For example, a composition can be prepared as an injectable, either as a liquid solution or a suspension. Solid forms suitable for solution in, or suspension in, liquid vehicles prior to injection can also be prepared (*e.g.*, a lyophilized composition). A composition can be prepared for oral administration, such as a tablet or capsule, as a spray, or as a syrup (optionally flavored). A composition can be prepared for pulmonary administration, *e.g.*, as an inhaler, using a fine powder or a spray. A composition can be prepared as a suppository or pessary. A composition can be prepared for nasal, aural or ocular administration *e.g.*, as drops. A composition can be in kit form, designed such that a combined composition is reconstituted just prior to administration to a patient. Such kits may comprise one or more mutant Spy0167 or other antigens in liquid form and one or more lyophilized antigens.

Immunogenic compositions used as vaccines comprise an immunologically effective amount of the GAS antigens or other antigens, as well as any other components, as needed, such as antibiotics. An "immunologically effective amount" is an amount which, when administered to an individual, either in a single dose or as part of a series, increases a measurable immune response or prevents or reduces a clinical symptom.

The immunogenic compositions of the present invention may be administered in combination with an antibiotic treatment regime. In one embodiment, the antibiotic is administered prior to administration of a composition of the invention. In another embodiment, the antibiotic is administered subsequent to the administration of a composition of the invention. Examples of antibiotics suitable for use in the treatment of a GAS infection include but are not limited to penicillin or a derivative thereof or clindamycin, cephalosporins, glycopeptides (*e.g.*, vancomycin), and cycloserine.

The amount of active agents in a composition varies depending upon the health and physical condition of the individual to be treated, age, the taxonomic group of individual to be treated (*e.g.*, non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range which can be determined through routine trials.

*Kits*

The invention also provides kits comprising one or more containers of compositions of the invention. Compositions can be in liquid form or can be lyophilized, as can individual antigens. Suitable containers for the compositions include, for example, bottles, vials, syringes, and test tubes. Containers can be formed from a variety of materials, including glass or plastic. A container may have a sterile access port (for example, the container may be an intravenous solution bag or a vial having a stopper pierceable by a hypodermic injection needle).

The kit can further comprise a second container comprising a pharmaceutically-acceptable buffer, such as phosphate-buffered saline, Ringer's solution, or dextrose solution. It can also contain other materials useful to the end-user, including other buffers, diluents, filters, needles, and syringes. The kit can also comprise a second or third container with another active agent, for example an antibiotic.

The kit can also comprise a package insert containing written instructions for methods of inducing immunity against *S. pyogenes* or for treating *S. pyogenes* infections. The package insert can be an unapproved draft package insert or can be a package insert approved by the Food and Drug Administration (FDA) or other regulatory body.

The above disclosure generally describes the present invention. A more complete understanding can be obtained by reference to the following specific examples, which are provided for purposes of illustration only.

EXAMPLE 1

*Hemolysis assays*

Serial dilutions of Spy0167 or a Spy0167 mutant are prepared in 96-well plates with U-shaped bottoms using PBS + 0.5% BSA. One ml of sheep blood is washed three times in PBS (with centrifugation at 3000 x g), and blood cells are suspended in 5 ml of PBS. An equal volume of suspension is added to 50 μl of each toxin dilution and incubated at 37°C for 30 min. Triton (2%) in water is used to give 100% hemolysis, and PBS + 0.5% BSA is used as negative control. Plates are then centrifuged for 5 min at 1,000 x g, and the supernatant is transferred carefully to 96-well flat-bottomed plates. The absorbance is read at 540 nm. One hemolytic unit (HU) is defined as the amount of Spy0167 or Spy0167 mutant required to obtained 50% of maximum lysis obtained treating the blood cells with 2% Triton.

EXAMPLE 2

*Assessment of in vivo toxicity of Spy0167 mutant antigens,*

*Intravenous injection of antigen.* A solution of either wild-type or mutant Spy0167 antigen in PBS is diluted in

a solution of PBS + 2 mM DTT, then 100 ml is injected into the tail vein of a mouse. Mice are observed for 2-3 days. Injection of wild-type Spy0167 typically results in death within a few minutes.

*In vivo lethality inhibition assay.* For lethality inhibition mediated by immune sera, 10 μg/mouse of wild-type Spy0167 (a solution of 100 μg/ml in PBS, 2 mM DTT) are incubated for 20 minutes with rotation "end over end" at room temperature with either anti-Spy0167 serum or control serum (obtained from mice immunized with adjuvant alone). After incubation, the samples are inoculated in the mice by intravenous injection into the tail vein. Mice are observed for 2-3 days.

*Acute in vivo toxicity.* Acute *in vivo* toxicity is assessed using a dose of 10 μg/mouse of wild-type Spy0167 as a positive control and injection of Freund's adjuvant alone as a negative control. Ten μg/mouse of wild-type Spy0167 are incubated with either wild-type Spy0167 antiserum or with control serum and inoculated into mice as described above.

EXAMPLE 3

*Inactivation of Spy0416 proteolytic activity*

*SDS-PAGE.* IL-8 is incubated with wild-type Spy0416 or a Spy0416 mutant. The incubation mixtures is loaded on SDS-PAGE and revealed by silver staining. Wild-type Spy0416 releases two bands: 8 kDa (active form) and 6 kDa (inactive cleaved IL-8). A Spy0416 mutant releases only one band, which corresponds to uncleaved IL-8, as in the control reaction (without enzyme).

*ELISA.* IL-8 is incubated with wild-type Spy0416 or a Spy0416 mutant at three different concentrations, and the incubation mixtures are tested for the presence of uncleaved IL-8 using an antibody which is specific for the cytokine but which is unable to recognize the cleaved inactive form. The results are expressed as percentage of uncleaved IL-8 after 0, 8 and 24 h reactions, and were calculated as follows:

$$\frac{[\text{IL-8 in the reaction mix}]}{[\text{IL-8 in the control mix}],} \times 100$$

where "control mix" is the reaction mix without the enzyme at time point 0.

EXAMPLE 4

*The protective capacity of GAS antigens*

A GAS antigen is used to immunize mice to test its capacity to confer protection against GAS lethal challenge. The antigen is administered intraperitoneally, optionally with an adjuvant, at days 0, 21, and 35. Blood samples are taken two weeks after the third immunization. The mice are then challenged intranasally with a GAS strain (*e.g.*, $10^8$ cfu of GAS strain 3348 M1 in 50 μl). Survival is monitored for a 10-14 day period.

EXAMPLE 5

*Dose-dependent inhibition of Spy0416-mediated IL-8 cleavage by Spy0416 antibodies*

Antisera specific for Spy0416, wild type and inactive mutants, are produced by immunizing CD1 mice with purified recombinant proteins. IL-8 (10 μg/ml) is incubated with wild-type Spy0416 with or without Spy0416 antiserum (1:50 and 1:5000), or with monoclonal antibodies raised against wild-type Spy0416, in two different conditions: (1) 8 hour incubation, 0.1 μg/ml of Spy0416 and (2) 24 hour incubation, 0.05 μg/ml of Spy0416. The incubation mixtures are then tested for the presence of uncleaved IL-8 by ELISA. The results demonstrate a dose-dependent inhibition of Spy0416-mediated IL-8 cleavage by the Spy0416 antiserum or monoclonal antibodies.

EXAMPLE 6

*Inhibition of Spy0167 hemolysis by antibodies against wild-type or mutant Spy0167 (Spy0167)*

Using 50 ng/ml (3.5 HU) of toxin, the antibody titer required to obtain 50% reduction of *Spy0167* hemolytic activity is tested using an adjuvant (*e.g.*, Freund's adjuvant, Alum, or MF59™). Adjuvant alone is used as a negative control.

EXAMPLE 7

*Protective capacity of the combination of GAS antigens in a subcutaneous challenge model*

Mice were immunized with single GAS antigens (Spy0167, Spy0416, or Spy0269) or with combinations of GAS antigens GAS (Spy0167 + Spy0416 + Spy0269; or Spy0416 + Spy0269). The mice were then infected subcutaneously with the SF370 M1 strain of GAS, which causes skin lesions. The protective effect of the GAS antigens or antigen combinations was determined by measuring lesion size.

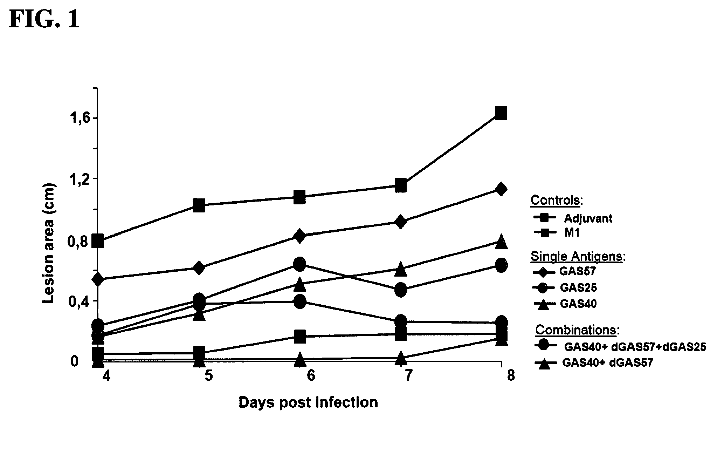

In this model, there is a synergistic protective effect obtained by using the combination of Spy0167 + Spy0416 + Spy0269 or the combination of Spy0416 + Spy0269 compared with the protective effect obtained by using any of these GAS antigens alone. In fact, the protective effect provided by the combinations tested is comparable to that provided using GAS M1 protein. See **FIG. 1.**

EXAMPLE 8

*Protective capacity of the combination of mutant GAS antigens*

The protective capacity of a combination of GAS mutant antigens (Spy0167 mutant antigen P427L/W535F and Spy0416 mutant antigen D151A/S617A) against intranasal challenge with various strains of GAS was tested essentially as described in Example 4. The results are shown in Table 2.

Table 2

| challenge strain | percent survival | | adjuvant | no. mice tested/vaccine |
|---|---|---|---|---|
| | negative control | combination | | |
| M1 | 19 | 85 | alum | 128 |
| M2 | 15 | 40 | alum | 32 |
| M6 | 25 | 58 | alum | 80 |
| M12 | 19 | 47 | alum | 144 |
| M23 | 19 | 54 | Freund's | 60 |

EXAMPLE 9

*Preparation of Spy0416 mutants*

By comparison with C5a protease, three amino acids in the Spy0416 were identified that putatively constitute the catalytic site of the protease: D151, H279 and S617. In order to obtain an inactive form of the enzyme, nucleotide substitutions resulting in amino acid changes D151A and/or S617A were introduced in the Spy0416 coding sequence by Splicing by Overlapping Extension PCR (SOE-PCR).

*Substitution D151A*

Three PCR reactions were carried out:

| PCR reaction | Template | Primers |
|---|---|---|
| PCR1 (360 bps) | genomic SF370 | 57F, GTGCGT***CATATG***GCAGATGAGCTAAGCA; SEQ ID NO:150<br>57mutDR1, CCCTGT**GGC**AATAACTGCGAC; SEQ ID NO:151 |
| PCR2 (910 bp) | genomic SF370 | 57mutDF1, cgCAGTTATT**GcC**ACAGGGAT, SEQ ID NO:152<br>57mutSalR, CTGACTGA***GTCGAC***AGACTCTGAATAGATG, SEQ ID NO:153 |
| PCR3 (1270 bps) | PCR1, PCR2 | 57F<br>57mutSalR |

PCR product 3 was then digested with Nde-Sal and introduced in pET21_57his digested with the same enzymes.

Clones containing the correct in-frame substitutions (pET21_57his_D151A) were selected by DNA sequencing.

*Substitution S617A*

Three PCR reactions were carried out:

| PCR reaction | Template | Primers |
|---|---|---|
| PCR4 (517 bp) | genomic SF370 | 57mutSalF, CTGACTGAG***TCGAC***TTTAAAGACATAAAAGATAG; SEQ ID NO:154<br>57mutSR1, GAGAGGCCAT**AGC**TGTTCCTG; SEQ ID NO:155 |
| PCR6 (4740 bp) | genomic SF370 | 57mutSF1, GGAACA**GCT**ATGGCCTCTCCT; SEQ ID NO:156<br>57R |
| PCR6 (5257 bp) | PCR4, PCR5 | 57FmutSalF<br>57R |

PCR product 6 was then digested with Sal-Xho and introduced in pET21_57his digested with the same enzymes. Clones containing the correct in-frame substitutions (pET21_57his_S617A) were selected by DNA sequencing.

*Substitution D151A+S617A*

PCR product 6 was digested Sal-Xho and introduced in pET21_57his_D151A digested with the same enzymes. Clones containing the correct in-frame substitutions (pET21_57his_D151A+S617A) were selected by DNA sequencing.
The single and double mutant proteins were expressed and purified using three chromatographic steps: ion exchange chromatography (Q Sepharose HP), hydroxylapatite chromatography and gel filtration chromatography.

EXAMPLE 10

*Point mutation D151A results in inactivation of Spy0416 proteolytic activity*

Spy0416 mutant D151A was expressed as a recombinant His-tagged protein. Two types of assays demonstrated that this mutant has lost the ability to cleave IL-8.

*SDS-PAGE*

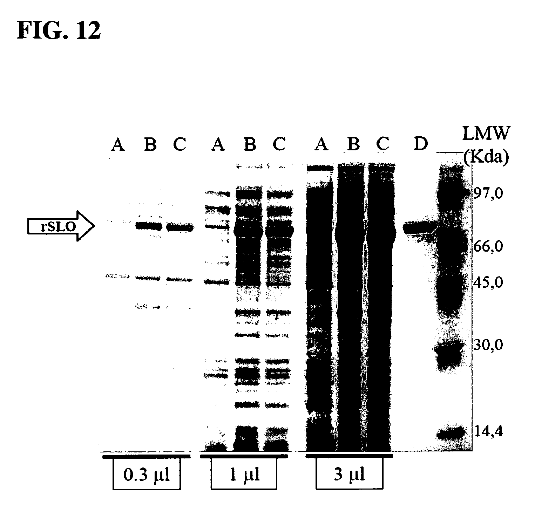

IL-8 was incubated with wild-type Spy0416 or the Spy0416 mutant D151A. The incubation mixtures were loaded on SDS-PAGE and revealed by silver staining. The results are shown in **FIG. 12.** Wild-type Spy0416 (lanes 2 and 3) released two bands: 8 kDa (active form) and 6 kDa (inactive cleaved IL-8). In contrast, the Spy0416 D151A mutant released only one band, which corresponded to uncleaved IL-8, as in the control reaction (without enzyme).

*ELISA*

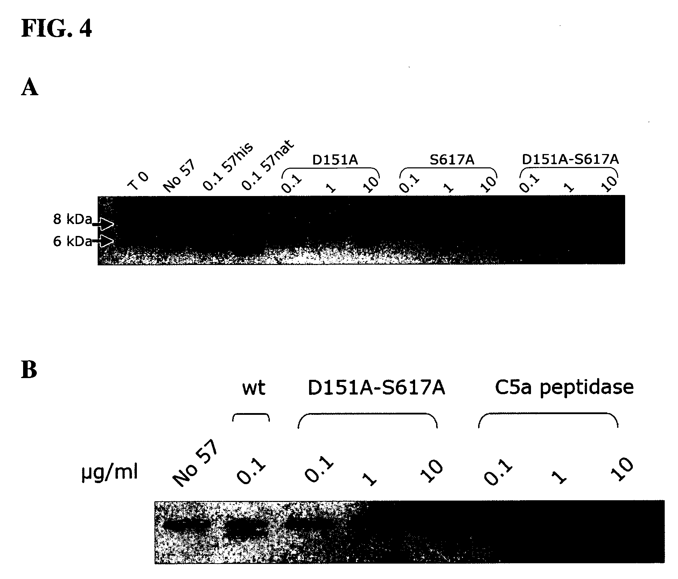

IL-8 was incubated with wild-type Spy0416 or the Spy0416 mutant D151A at three different concentrations, and the incubation mixtures were tested for the presence of uncleaved IL-8 using an antibody which is specific for the cytokine but which is unable to recognize the cleaved inactive form. The results are shown in **FIG. 4**, expressed as percentage of uncleaved IL-8 after 0, 8 and 24 h reactions, and were calculated as follows:

$$\frac{[\text{IL-8 in the reaction mix}]}{[\text{IL-8 in the control mix}]} \times 100,$$

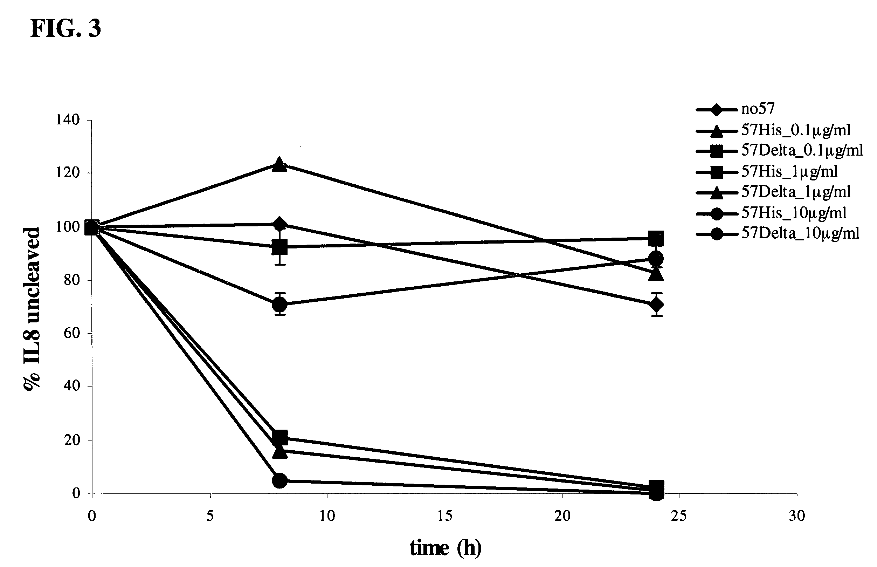

where "control mix" is the reaction mix without the enzyme at time point 0.
As shown in **FIG. 3**, wild-type Spy0416 almost completely inactivated IL-8 after 8 hours, even at the lower concentration, while no inactivation was observed for IL-8 treated with the mutant enzyme.

EXAMPLE 11

*Spy0416 mutant S617A and Spy0416 double mutant D151A + S617A do not cleave IL-8*

Spy0416 mutant S617A and Spy0416 double mutant D 151 A + S617A were expressed as His-tagged proteins and were tested in IL-8 inactivation experiments as described in Example 2.

*SDS-PAGE*

IL-8 was incubated with either wild-type Spy0416 (His-tagged or tag-less), or each of the Spy0416 mutants D151A, S617A and D151AS+S617A for 24 hours. The incubation mixtures were loaded on an SDS-polyacrylamide gel and revealed by silver staining. The results of two experiments are shown in **FIGS. 4A** and **4B.** Both the Spy0416 S617A mutant and the GAS D151 + S617A mutant are unable to cleave IL-8, even at a 100-fold higher concentration than wild-type Spy0416.

*ELISA*

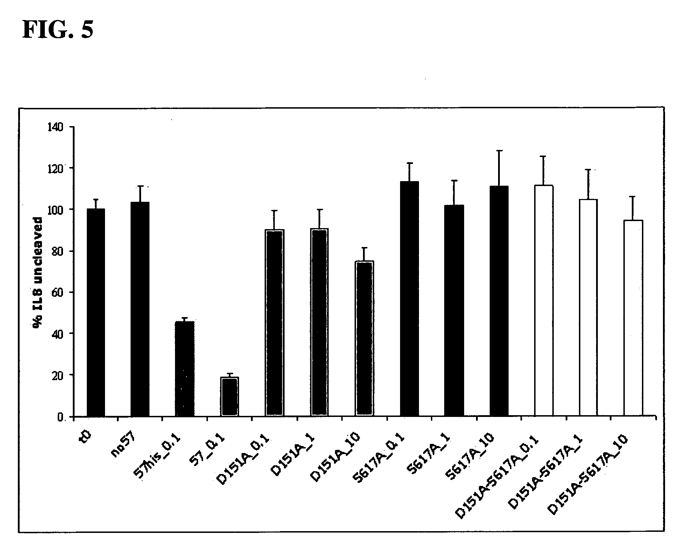

The same samples were used to perform an ELISA assay which confirmed that the single and double amino acid substitutions eliminate the ability of Spy0416 to cleave IL-8. The results, which are shown in **FIG. 5**, demonstrate that the mutants release 100% of uncleaved IL-8 after 24 h incubation, compared to 20-40% released by wild-type Spy0416.

EXAMPLE 12

*The protective capacity of Spy0416 mutants is similar to that obtained with wild-type Spy0416*

The Spy0416 mutants D151A and D151A + S617A were used to immunize mice to test their capacity to confer protection against GAS lethal challenge in comparison to wild-type Spy0416. The results of two experiments (20 mice each) are summarized below and expressed as average % survival.

Table 3.

| | NO. MICE | NO. DEAD | % SURVIVAL |
|---|---|---|---|
| PBS + Freund | 40 | 26 | 35 |
| 192 M1 + Freund | 20 | 0 | 100 |
| 57 WT + Freund | 40 | 12 | 70 |
| 57 D151A + Freund | 40 | 6 | 85 |
| 57 D151A-S617A + Freund | 40 | 9 | 78 |

EXAMPLE 13

*Purified inactive mutants appear as a single peptide compared to wild-type Spy0416, which exists only in the form of two non covalently associated protein fragments.*

Wild-type Spy0416 is obtained mainly in the form of two fragments, one of about 23 kDa and a one of 150 kDa. The two fragments are not separated in Ni-chelating affinity purification or by gel filtration, but appear as two different bands on SDS-PAGE (**FIG**. **6**). N-terminal sequencing confirmed that the 23 kDa fragment is the N-terminal portion of Spy0416 (amino acids 34-244 of SEQ ID NO:50) while the 150 kDa fragment is the C-terminal region (amino acids 245-1603 of SEQ ID NO:50).

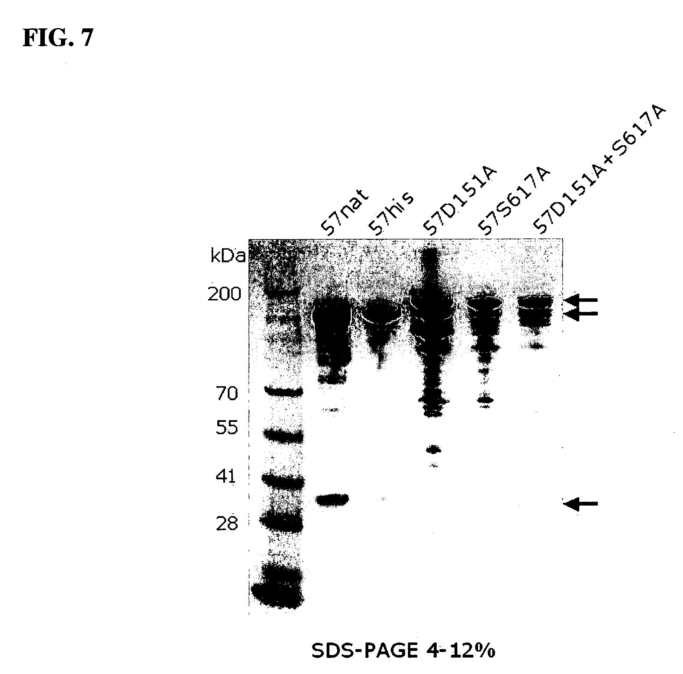

In contrast to wild-type Spy0416, Spy0416 mutants of the invention are obtained as proteins of higher molecular weight (174 kDa), and the 23 kDa band is absent (see **FIG. 7**, which shows the results of an experiment in which partially purified wild-type Spy0416 and Spy0416 mutants were loaded on SDS-polyacrylamide gels).

EXAMPLE 14

*Dose-dependent inhibition of Spy0416-mediated IL-8 cleavage by polyclonal antisera*

Mouse antisera specific for Spy0416, wild type and inactive mutants, were produced by immunizing CD1 mice with the purified recombinant proteins.

IL-8 (10 μg/ml) was incubated with wild-type Spy0416 with or without Spy0416 antiserum (1:50 and 1:5000) in two different conditions: (1) 8 hour incubation, 0.1 μg/ml of Spy0416 and (2) 24 hour incubation, 0.05 μg/ml of Spy0416. The incubation mixtures were then tested for the presence of uncleaved IL-8 by ELISA. The results shown in **FIGS**. **8A** and **8B** demonstrated a dose-dependent inhibition of Spy0416-mediated IL-8 cleavage by the mouse antiserum.

EXAMPLE 15

*Cloning of wild-type and mutant Spy0167 proteins*

Genes encoding wild-type and mutant Spy0167 proteins were amplified by PCR using the primers from the SF370 genome shown in Table 4.

The PCR products were digested with *Nhe*I-*Xho*I and ligated with pet24b+ (Novagen) vector cut with the same enzymes. *E. coli* DH5α electrocompetent cells were transformed with the ligation reactions. LBPTK medium was added and, after incubation for 1h at 37°C, with agitation at 250 rpm, bacteria were plated onto LBPTK plates containing 50 μg/ml kanamycin. Positive colonies were identified by colony PCR.

Plasmids from positive colonies were prepared from an overnight culture in LBPTK medium containing 50 μg/ml kanamycin and analyzed by DNA sequencing, which confirmed the expected insert gene under the T7 polymerase promoter. The final DNA and protein sequences of the cloned genes are shown in the sequence listing. See Table 5.

Table 4.

| gene | primers |
|---|---|
| *Spy0167* wild-type *tag-less* | 25F NheI, GTGCGTGCTAGCGAATCGAACAAACAAAACACTGC (SEQ ID NO:157)<br>25rev = GCATTCGATCCTCGAGCTACTTATAAGTAATCGAACCATATG (SEQ ID NO:158) |
| Spy0167 P427L *tag-less* | External primers:<br>25F NheI, GTGCGTGCTAGCGAATCGAACAAACAAAACACTGC (SEQ ID NO:157)<br>25rev, GCATTCGATCCTCGAGCTACTTATAAGTAATCGAACCATATG (SEQ ID NO:158)<br>Internal primers:<br>PL427_for, GCTACCTTCAGTAGAAAAAACCTAGCTTATCCTATTTCATACACC (SEQ ID NO:59)<br>PL427_rev, GGTGTATGAAATAGGATAAGCTAGGTTTTTTCTACTGAAGGTAGC (SEQ ID NO:160) |
| *Spy0167* Wild Type *His-tagged* | 25F NheI, GTGCGTGCTAGCGAATCGAACAAACAAAACACTGC (SEQ ID NO:157)<br>25revhis, GCATTCGATCCTCGAGCTTATAAGTAATCGAACCATATGGG (SEQ ID NO:161) |
| *Spy0167* W535F *His-tagged* | External primers: |
| | 25F NheI, GTGCGTGCTAGCGAATCGAACAAACAAAACACTGC (SEQ ID NO:157)<br>25revhis, GCATTCGATCCTCGAGCTTATAAGTAATCGAACCATATGGG (SEQ ID NO:161)<br>Internal primers:<br>WF535_for, GAGTGCACTGGCTTAGCTTTCGAATGGTGGCGAAAAGTGATC (SEQ ID NO:162)<br>WF535_rev, GATCACTTTTCGCCACCATTCGAAAGCTAAGCCAGTGCACTC (SEQ ID NO:163) |
| Spy0167 W535F-D482N *His-tagged* | External primers:<br>25F NheI, GTGCGTGCTAGCGAATCGAACAAACAAAACACTGC (SEQ ID NO:157)<br>25revhis, GCATTCGATCCTCGAGCTTATAAGTAATCGAACCATATGGG (SEQ ID NO:161)<br>Internal primers:<br>WF535_for, GAGTGCACTGGCTTAGCTTTCGAATGGTGGCGAAAAGTGATC (SEQ ID NO:162)<br>WF535_rev, GATCACTTTTCGCCACCATTCGAAAGCTAAGCCAGTGCACTC (SEQ ID NO:163)<br>and<br>DN482_for,<br>GTTGCTCAATATGAAATCCTTTGGAATGAAATCAATTATGATGACAAAGGAAAAG (SEQ ID NO:164)<br>DN482_rev,<br>CTTTTCCTTTGTCATCATAATTGATTTCATTCCAAAGGATTTCATATTGAGCAAC(SEQ ID NO:165) |
| Spy0167 C530G *His-tagged* | External primers:<br>25F NheI, GTGCGTGCTAGCGAATCGAACAAACAAAACACTGC (SEQ ID NO:157)<br>25revhis, GCATTCGATCCTCGAGCTTATAAGTAATCGAACCATATGGG (SEQ ID NO:161)<br>Internal primers: |

(continued)

| gene | primers |
|---|---|
| | CG530_for, CCGTATCATGGCTAGAGAGGGCACTGGCTTAGCTTGGGAATG (SEQ ID NO:166)<br>CG530_rev, CATTCCCAAGCTAAGCCAGTGCCCTCTCTAGCCATGATACGG (SEQ ID NO:167) |
| Spy0167 P427L *His-tagged* | External primers:<br>25F NheI, GTGCGTGCTAGCGAATCGAACAAACAAAACACTGC (SEQ ID NO:157)<br>25 revhis, GCATTCGATCCTCGAGCTTATAAGTAATCGAACCATATGGG (SEQ ID NO:161)<br>Internal primers:<br>PL427_for, GCTACCTTCAGTAGAAAAAACCTAGCTTATCCTATTTCATACACC (SEQ ID NO:149)<br>PL427_rev, GGTGTATGAAATAGGATAAGCTAGGTTTTTTCTACTGAAGGTAGC (SEQ ID NO:150) |
| Spy0167 P427L-W535F-C535G *tag-less* | External primers:<br>25_F, GTGCGTGCTAGCGAATCGAACAAACAAAAC (SEQ ID NO:168)<br>25_stopR, GCGTCTCGAGTCACTTATAAGTAATCGAACCATA (SEQ ID NO:17450)<br>Internal primers:<br>W-C_for, CCGTATCATGGCTAGAGAGGGCACTGGCTTAGCTTTCGAATG (SEQ ID NO:170)<br>W-C_rev, CATTCGAAAGCTAAGCCAGTGCCCTCTCTAGCCATGATACGG (SEQ ID NO:171) |
| Spy0167 P427L-W535F *tag-less* | External primers:<br>25_F, GTGCGTGCTAGCGAATCGAACAAACAAAAC (SEQ ID NO:168)<br>25_stopR, GCGTCTCGAGTCACTTATAAGTAATCGAACCATA (SEQ ID NO:169)<br>Internal primers:<br>WF535_for, GAGTGCACTGGCTTAGCTTTCGAATGGTGGCGAAAAGTGATC (SEQ ID NO:162)<br>WF535_rev, GATCACTTTTCGCCACCATTCGAAAGCTAAGCCAGTGCACTC (SEQ ID NO:163) |
| | |
| *Spy0167* P427L-C530G *tag-less* | External primers:<br>25_F, GTGCGTGCTAGCGAATCGAACAAACAAAAC (SEQ ID NO:168)<br>25_stopR, GCGTCTCGAGTCACTTATAAGTAATCGAACCATA(SEQ ID NO:169)<br>Internal primers:<br>CG530_for, CCGTATCATGGCTAGAGAGGGCACTGGCTTAGCTTGGGAATG (SEQ ID NO:166)<br>CG530_rev, CATTCCCAAGCTAAGCCAGTGCCCTCTCTAGCCATGATACGG (SEQ ID NO:167) |
| *Spy0167* ΔA248 *his-tagged* | External primers:<br>25F NheI, GTGCGTGCTAGCGAATCGAACAAACAAAACACTGC (SEQ ID NO:157)<br>25 revhis, GCATTCGATCCTCGAGCTTATAAGTAATCGAACCATATGGG (SEQ ID NO:161)<br>Internal primers: |

(continued)

| gene | primers |
|---|---|
| | Δ248for, CTGGTGGTAATACGCTTCCTAGAACACAATATACTGAATCAATGG (SEQ ID NO:172)<br>Δ248rev, CCATTGATTCAGTATATTGTGTTCTAGGAAGCGTATTACCACCAG (SEQ ID NO:173) |

Table 5.

| | sequence identifier | | | |
|---|---|---|---|---|
| Spy0167 gene | amino acid | | nucleotide | |
| | *tag-less* | *His-tagged* | *tag-less* | *His-tagged* |
| wild-type | 1-12 | 13 | 28 | 14 |
| P427L | 20 | 15 | 29 | 57 |
| C530G | 22 | 16 | 31 | 58 |
| W535F | 21 | 18 | 30 | 52 |
| ∆A248 | 23 | 17 | | 59 |
| W535F + D482N | 24 | 19 | | 53 |
| P427L + C530G | 26 | 54 | 33 | |
| P427L + W535F | 25 | 55 | 32 | |
| P427L + C530G + W535F | 27 | 56 | 34 | |

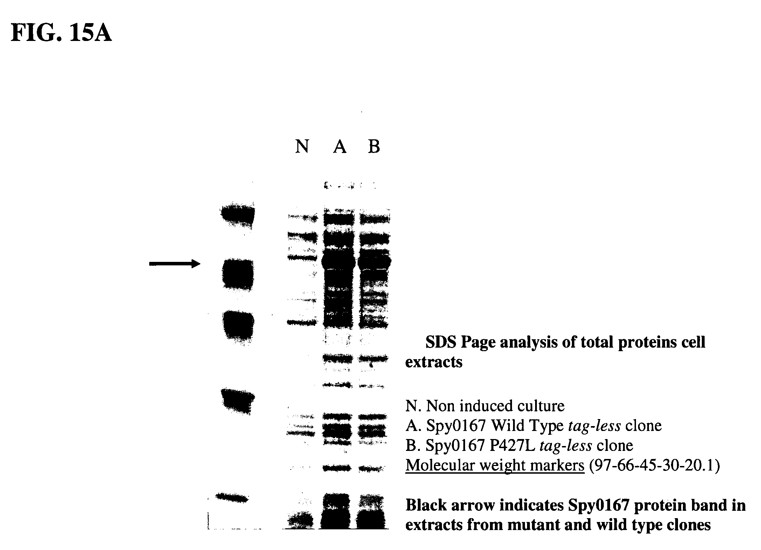

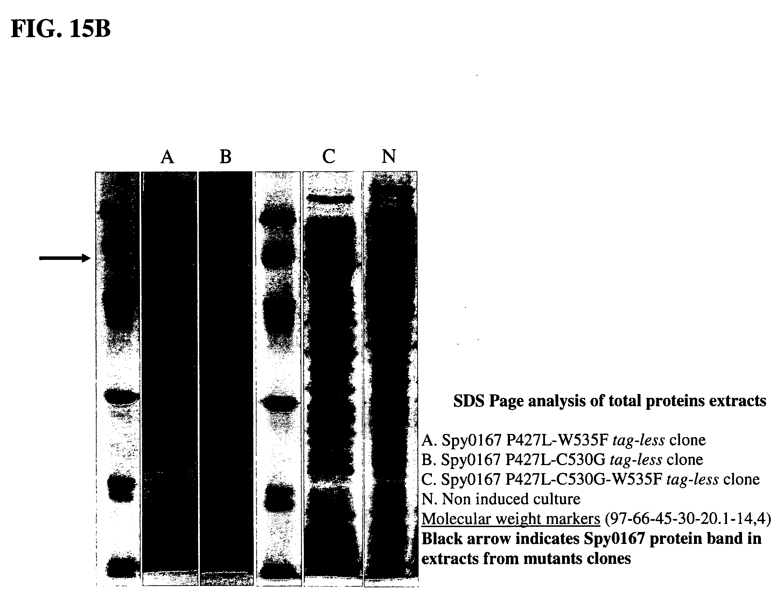

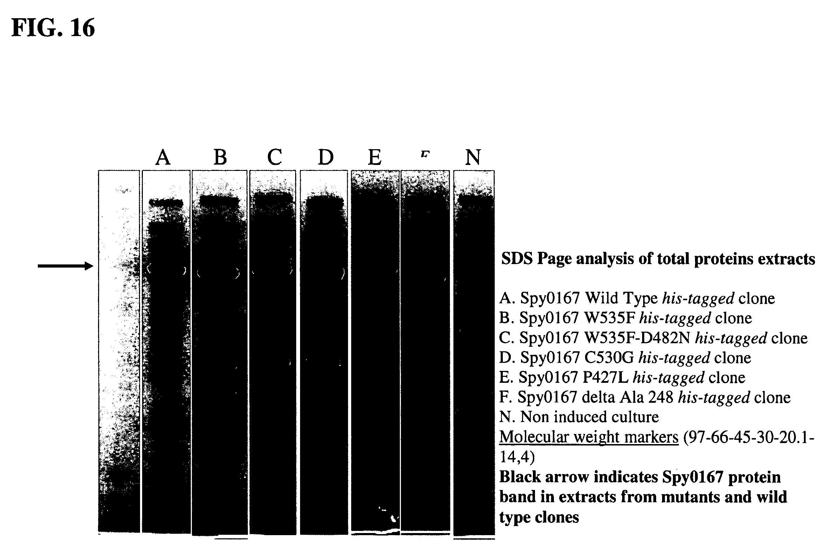

*E. coli* BL21(DE3) (Novagen) competent cells were transformed with the correct construct. LBPTK medium was added and, after incubation for 1h at 37°C, with agitation at 250 rpm, bacteria were plated onto LBPTK plates containing 50 µg/ml kanamycin. BL21(DE3) pet24b+ Spy0167 wild-type *tag-less* cells were grown at 25°C and induced with 1 mM IPTG. Clone expression was verified by SDS PAGE (*tag-less,* **FIGS. 15A** and **15B**; His-tagged, **FIG. 16**).

EXAMPLE 16

*Purification of His-tagged proteins*

*E. coli* pellets were suspended in lysis buffer and mixed for 30-40 minutes at room temperature. Lysates were centrifuged at 30-40000 x g for 20-25 minutes and supernatants were loaded onto wash buffer A equilibrated columns (Poly-Prep with 1 ml of Ni-Activated Chelating Sepharose Fast Flow resin). The loaded resin was washed three times with wash buffer A and three times with wash buffer B. Proteins were eluted with elution buffer in Eppendorf tubes containing 2mM final of DTT. Total elution proteins are quantified with Bradford reagent and then analyzed by SDS-polyacrylamide gel electrophoresis (**FIGS. 15** and **16**).

*Buffers*

lysis buffer:

10 ml B-PER™ (Bacterial-Protein Extraction Reagent, Pierce cat. 78266)
$MgCl_2$ final concentration of 0.1 mM
DNAsi I (Sigma cat. D-4263) 100 units
lysozyme (Sigma cat. L-7651) final concentration of 1 mg/ml

wash buffer A: 50 mM $NaH_2PO_4$, 300 mM NaCl, pH 8.0
wash buffer B: 20 mM imidazole, 50 mM $NaH_2PO_4$, 300 mM NaCl, pH 8.0
elution buffer: 250 mM imidazole, 50 mM $NaPO_2$, 300 mM NaCl, pH 8.0

EXAMPLE 17

*Purification of tag-less proteins*

*Lysate preparation*

About 80-110 g of bacterial culture pellet were suspended in 200-280 ml B-PER reagent (Pierce) supplemented with 6 tablets of COMPLETE® protease inhibitor, 10 ml 0,2M EDTA pH 7.5 (5 mM final concentration), 10 ml of a 100 mg/ml lysozyme solution, 8 ml of a 10000 K units/ml DNAse I solution and 1 ml of 50 mM $MgCl_2$ solution. Bacterial lysis was achieved by shaking the bacterial suspension for 60 minutes until a homogeneous suspension was obtained.

Following centrifugation for 60 minutes at 13000 rpm (25400 x g), the supernatant was filtered using a 0.22 μm filter and is diluted with $H_2O$ until a 1.8-1.9 mS conductivity was obtained. The pH was adjusted to 8.0. Protein concentration was determined by the Bradford method.

*Anionic exchange chromatography*

The supernatant derived from the lysate treated as described above was loaded on an HP 50/10 Q Sepharose column (~-200 ml), previously equilibrated with 30 mM TRIS, pH 8.0. The flow-through was collected. Fractions containing the Spy0167 protein were pooled and dialyzed against 10 mM Na phosphate, pH 6.8. Protein concentration was determined by the Bradford method.

Buffer A: 30mM TRIS, pH 8.0
Buffer B: 30mM TRIS, 1M NaCl, pH 8.0
Equilibrium and Loading: 0% B
Gradient: 0-25% B in 5 CV - 25% B 2 CV
Wash: 100%B 2 CV + 3 CV
Flux: 20 ml/min
Fraction volume: 14 ml

*Hydroxylapatite chromatography*

The previously obtained pool was loaded on a CHT20 column previously equilibrated with 10mM Na-phosphate, pH 6.8. The flow through was collected.

Buffer A: 10mM Na-phosphate, pH 6.8
Buffer B: 500mM Na phosphate, pH 6.8
Wash: 8 CV
Wash: 30%B 6 CV
Gradient: 30-100%B (10 CV)
Wash: 100%B
Flux: 5 ml/min.
Fraction volume: 5 ml

Fraction aliquots were loaded on 12% Criterion gels under reducing and non-reducing conditions. Fractions containing Spy0167 protein were pooled and protein concentration was determined by Bradford method.

*Gel filtration chromatography*

The collected pool was concentrated using an Amicon filter in order to get a volume < 10 ml. The concentrated material was loaded on a HiLoad Superdex 200 26/60 equilibrated with at least 3-4 column volumes of PBS.

Buffer: PBS
Elution: Isocratic
Flux: 2.5 ml/min.
Fraction volume: 5 ml

Fractions containing *Spy0167* protein were pooled and protein concentration was determined by Bradford. An additional estimation of protein concentration was performed by UV measurement considering Abs 0.1% (=1 g/l) 1.119.

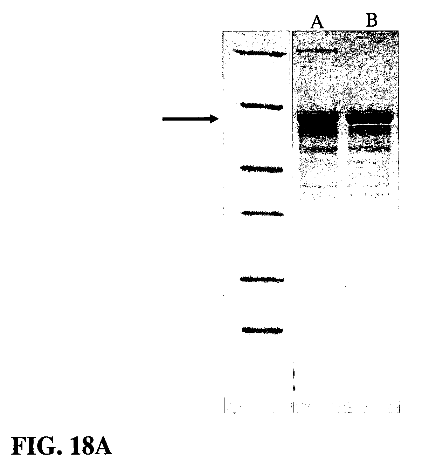

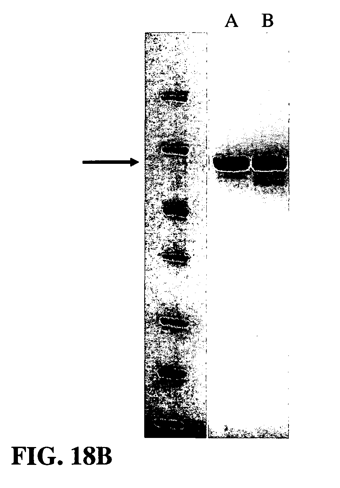

Protein purity is analyzed by polyacrylamide gel electrophoresis (**FIG. 18**).

EXAMPLE 18

*Hemolytic assays*

*Protocol for quantitative hemolytic assay*

Serial dilutions of toxin were prepared in 96-well plates with U-shaped bottoms using PBS + 0.5% BSA. One ml of sheep blood was washed three times in PBS (with centrifugation at 3000 x g), and blood cells were suspended in 5 ml of PBS. An equal volume of suspension was added to 50 μl of each toxin dilution and incubated at 37°C for 30 min. Triton (2%) in water was used to give 100% hemolysis, and PBS + 0.5% BSA was used as negative control. Plates were then centrifuged for 5 min at 1,000 x g, and the supernatant was transferred carefully to 96-well flat-bottomed plates. The absorbance was read at 540 nm.

*Comparison of E. coli extracts containing wild-type Spy0167 and Spy0167 mutant P427L*

The gene encoding Spy0167 P427L was amplified using PCR from the SF370 M1 genome and cloned into the vector pET21b+, which allowed expression in *E. coli* BL21DE3 of the His-tagged protein. Soluble extracts of *E. coli* expressing similar amounts of the wild-type and mutated streptolysin O proteins (see **FIG. 12**) were used to perform a hemolytic assay to compare the cytolytic properties of the two antigens. The result of the assay is shown in **FIG. 9**, which demonstrates that the mutated protein is at least 100 times less toxic than wild-type.

*Comparison of purified wild-type Spy0167 and Spy0167 mutant P427L*

The *Spy0167* P427L mutant was purified according to purification standard procedures for His-tagged recombinant proteins (**FIG. 10**). Different concentrations of the purified wt and mutated proteins were used to repeat the hemolytic assay, which confirmed the decreased cytolytic activity (**FIG. 11**).

*Hemolytic activity of E. coli extracts containing His-tagged and tag-less wild-type Spy0167 and Spy0167 mutant P427L*

We compared the hemolytic activity of *E. coli* lysates transformed with wild-type recombinant Spy0167 (rSpy0167) without a His tag (BL21 DE3, Novagen No. 71382-pET24) and P427L mutant rSpy0167 without a His tag (BL21 DE3, Novagen No. 71382-pET24). *E. coli* BL21 DE3 (Novagen, No. 71382) transformed with pET24 without insert was used as a negative control. The positive control was a hypotonic solution containing Triton 2% in water. The negative control was the protein dilution buffer (PBS containing 0.5% BSA, pH 7.4).

Hemolysis was determined by measuring absorbance at 540 nm ($A_{540nm}$) of the supernatants. The titer was calculated as the dilution with 50% of maximum $A_{540nm}$.

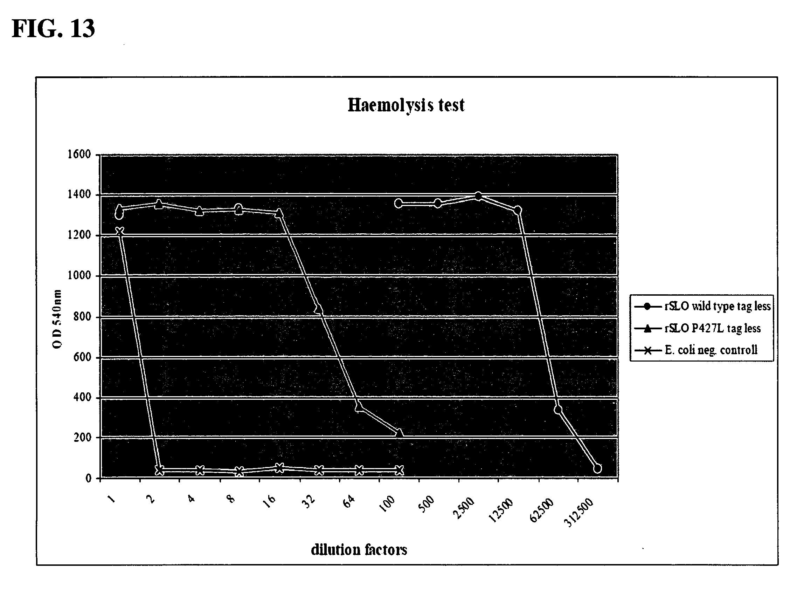

Results are shown in Tables 6 and 7 and in **FIG. 13.** These data demonstrate that, under the same conditions, mutant P427L is 1000 times less hemolytic than wild type Spy0167.

Table 6.

| ***E. coli*** | **CFU/ml** |
|---|---|
| negative control | $3.9 \times 10^8$ |
| Wild-type rSpy0167 (*tag-less*) | $1.2 \times 10^9$ |
| P427L rSpy0167 (*tag-less*) | $1.03 \times 10^9$ |

Table 7.

| | **rSpy0167 wild-type *tag-less*** | **rSpy0167P427L *tag-less*** |
|---|---|---|
| titer (OD=50% hemolysis) | 50,000 | 48 |
| titer Wt/P427L | 1042 | |

*Comparison of wild-type Spy0167 and various Spy0167 mutants*

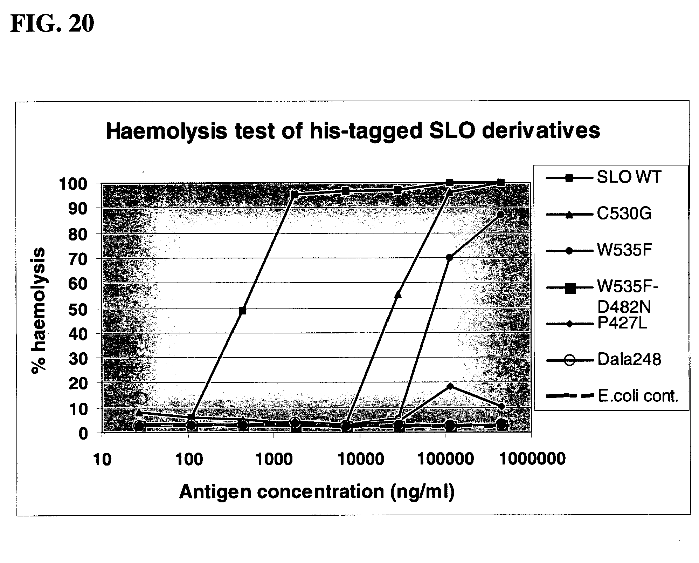

Hemolytic activity of wild-type *Spy0167* was compared with hemolytic activity of several different *Spy0167* mutants. The results are shown in **FIG. 20** and in Table 8, below. One hemolytic unit (HU) is defined as the amount of toxin required to obtained 50% of maximum lysis obtained treating the blood cells with 2% Triton.

Table 8.

| Protein | HU/mg | HU/mg-Spy0167/ mutants |
|---|---|---|
| rSpy0167 WT | 22760 | 1 |
| C530G | 620 | 37 |
| W535F | 160 | 146 |
| **W535F-D482N** | << **20** | >> **1000** |
| P427L | about 20 | about 1000 |
| **∆ala248** | << **20** | >> **1000** |
| Neg. Control | << 20 | >> 1000 |

Due to differences in protein purity, the hemolysis units/mg of mutants indicated in bold are overestimated; however, it is clear that (1) mutant W535F is less hemolytic than mutant C530G; (2) mutant P427L is about 1000 times less hemolytic than wild type and about 6-25 times less hemolytic than other two mutants W535F and C530G; and (3) mutant ∆248 is certainly less hemolytic than wild type).

*Effect of cholesterol*

Two-fivefold serial dilutions in PBS-BSA 0.5% of *E. coli* lysates or *E. coli* lysate with 200 mg/ml of cholesterol obtained after cells' growing at 30°C and induction with 1mM IPTG at 25°C and $OD_{600nm}$ about 0.4-0.6, were assayed for their haemolytic activity. Fifty microliters of a 2% sheep erythrocyte solution in PBS were treated with an equal volume of protein preparations obtained by lysing bacteria, 3 hours after induction, with lysis buffer (B-PER solution-PIERCE-1 mM $MgCl_2$, 100K units/ml DNAse (Sigma) and lysozyme (Sigma) for 30-40 minutes. The insoluble fraction was then centrifuged (15 minutes, 21000 x g, 4°C), and the supernatant (*E. coli* lysate) was transferred to a new Eppendorf tube containing DTT at final concentration of 5 mM.

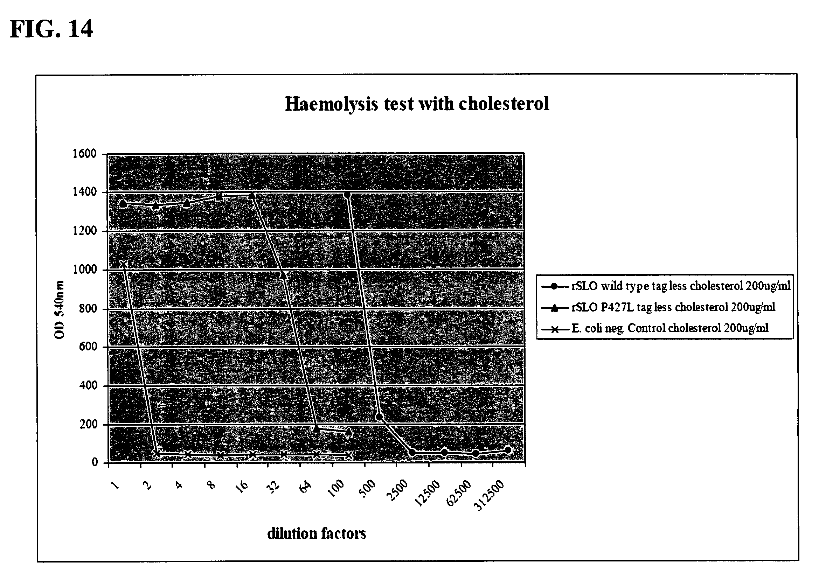

Under this condition, cholesterol did not inhibit either wild-type or mutant Spy0167 until a 100-fold dilution factor was used; thus, there was no effect on the mutant-induced lysis. In contrast, wild-type-induced lysis was greatly reduced. Lysis induced by the negative control was not influenced by cholesterol, which suggests that cholesterol-induced inhibition is specific. See Table 9 and **FIG. 14.**

Table 9

| | rSpy0167 wild-type *tag-less* | rSpy0167 P427L *tag-less* |
|---|---|---|
| titer (OD=50% hemolysis) | 400 | 40 |
| titre Wt/P427L | 10 | |

EXAMPLE 19

*Inhibition of hemolysis*

*Protocol*

Serial two-fold dilutions of sera from mice immunized with wild-type or mutant Spy0167 proteins (without adjuvants or with Alum or MF59™ as adjuvants) were prepared in 96-well plates with U-shaped bottoms using PBS + 0.5% BSA. Sera of mice immunized with PBS or with adjuvant alone, as appropriate, were used as negative controls. An equal volume of a 50-100 ng/ml (3.5-7 HU) toxin solution in PBS + 0.5% BSA was added, and the plates were incubated at room temperature for 20 minutes under agitation (800 rpm). After incubation, 50 ml of this solution were transferred to a new 96-well plate, and an equal volume of a sheep red blood cell suspension (washed 3 x in PBS) was added and

incubated at 37°C for 30 min. Plates were then centrifuged for 1 min at 1,000 x g, the supernatant was carefully transferred to 96-well flat-bottomed plates, and the absorbance was read at 540 nm. In the results described below, inhibition titer is expressed as the sera dilution that reduced Triton-induced hemolysis by 50%.

*Inhibition of Spy0167 hemolysis by wild-type Spy0167 antisera*

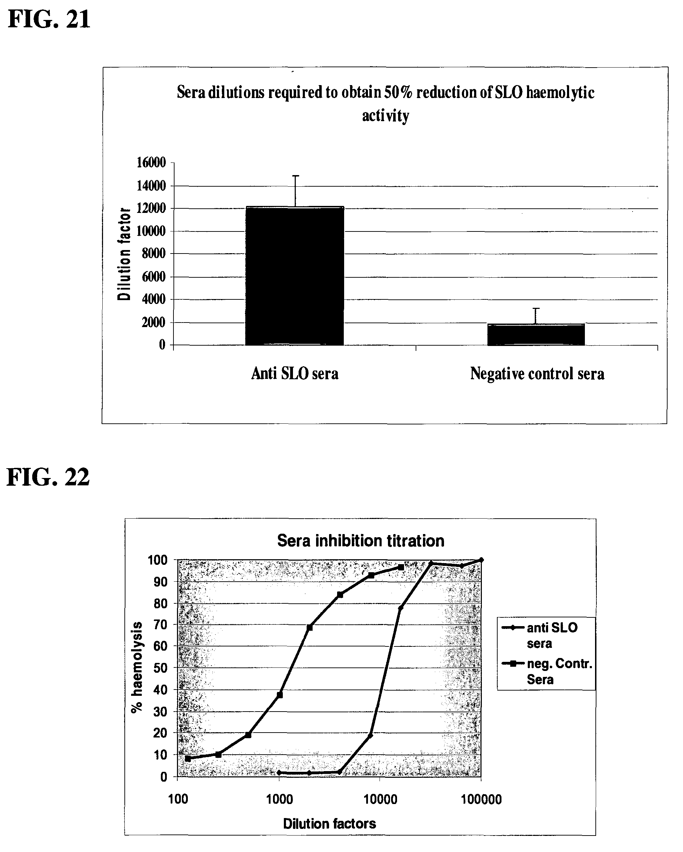

Inhibition of Spy0167 hemolysis by anti-wild-type Spy0167 antisera is shown in **FIGS. 21-23** and Tables 10-12. Anti-Spy0167 sera titers are included between 1/7,000 and 1/14,000 (arithmetic mean, 1/12,167 ± 2,714. Negative control sera (Freund's adjuvant) titers are included between 1/375 and 1/4,000 (arithmetic mean, 1/1,854 ± 1,384).

Table 10 (shown graphically in **FIG. 22**).

| arithmetic mean of tested sera - % hemolysis | | |
|---|---|---|
| dilution factor/sera | anti-Spy0167 sera | negative control sera |
| 125 | | 9 |
| 250 | | 10 |
| 500 | | 19 |
| 1,000 | 2 | 38 |
| 2,000 | 2 | 69 |
| 4,000 | 2 | 84 |
| 8,000 | 19 | 93 |
| 16,000 | 78 | 97 |
| 32,000 | 99 | |
| 64,000 | 97 | |
| 128,000 | 100 | |

Table 11

| anti-Spy0167 sera (Freund's adjuvant) | | negative control sera (Freund's adjuvant) | |
|---|---|---|---|
| serum | 50% hemolysis inhib. | serum | 50% hemolysis inhib. |
| A | 14,000 | 1 | 4,000 |
| B | 7,000 | 2 | 1,500 |
| C | 12,000 | 3 | 375 |
| D | 12,000 | 4 | 3,000 |
| E | 14,000 | 5 | 1,500 |
| F | 14,000 | 6 | 750 |

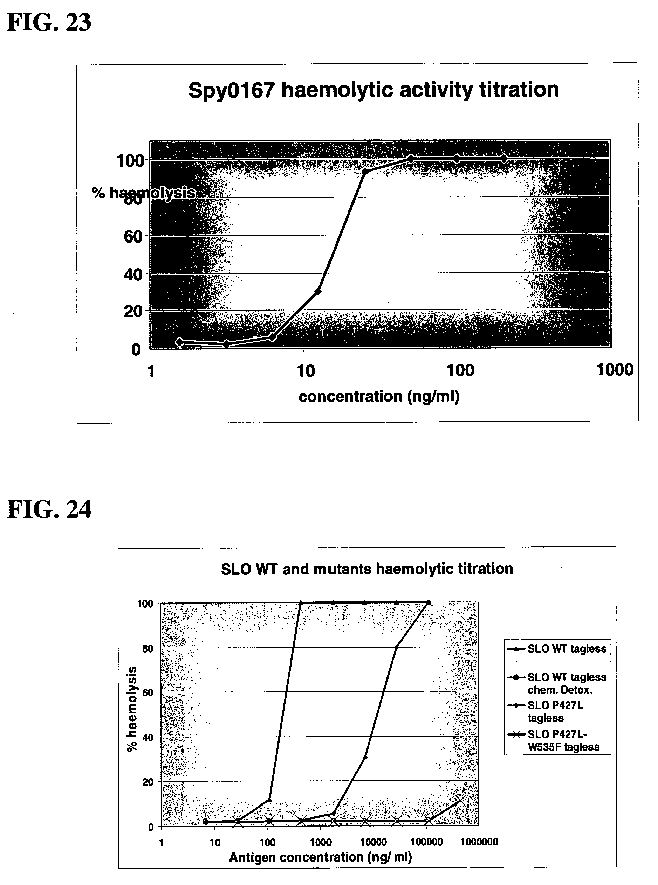

Table 12 (shown graphically in **FIG. 23**)

| ng/ml Spy0167 | % hemolysis |
|---|---|
| 1.6 | 4 |
| 3.1 | 3 |
| 6.3 | 6 |
| 12.5 | 30 |
| 25 | 94 |

(continued)

| ng/ml Spy0167 | % hemolysis |
|---|---|
| 50 | 100 |
| 100 | 100 |
| 200 | 100 |

*Titration of hemolytic activity of wild-type Spy0167, chemically detoxified wild-type Spy0167 and Spy0167 mutant*

Titration of hemolytic activity of wild-type Spy0167, chemically detoxified wild-type Spy0167, and Spy0167 mutants (P427L; P427L + W535F) is shown in Table 13.

Table 13

| protein | HU/mg | HU/mg-Spy0167/mutants |
|---|---|---|
| Spy0167 wild-type *tag-less* | 728,307 | 1 |
| Spy0167 P427L *tag-less* | 711 | 1,024 |
| Spy0167 P427L + W535F *tag-less* | <22 (stim. 10) | >33.000 |
| Spy0167 wild-type *tag-less* | 45,511 | |
| Spy0167 wild-type *tag-less,* detoxified | <<89 | >>511 |

*Inhibition of Spy0167 hemolysis by antiserum against mutant Spy0167 proteins*

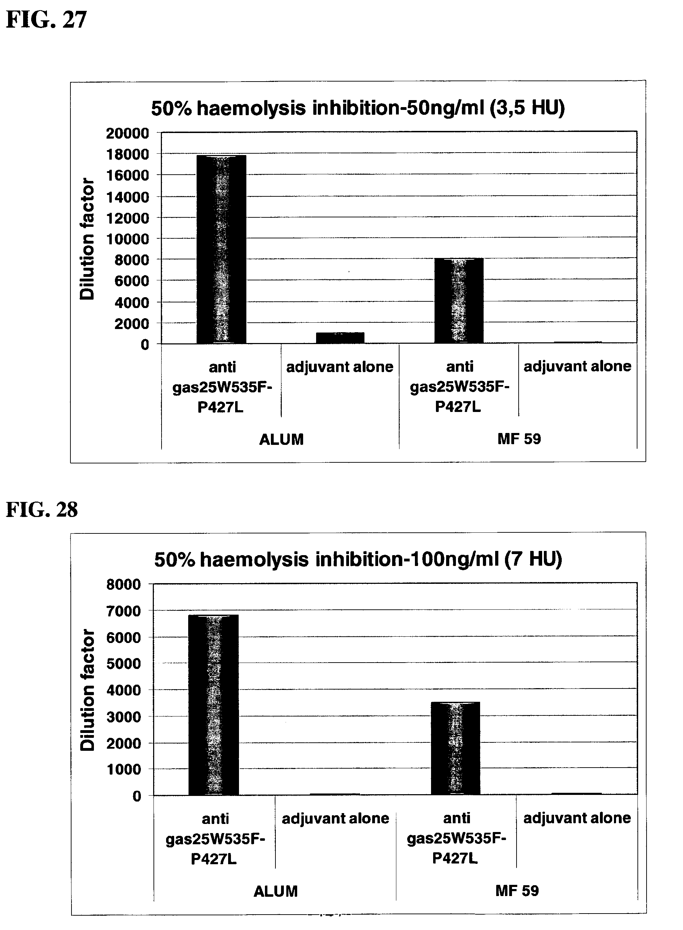

Inhibition of Spy0167 hemolysis by antisera against mutant Spy0167 proteins is shown in FIGS. 27-29 and Tables 14-16. Using 50 ng/ml (3.5 HU) of toxin, the sera dilution required to obtain 50% reduction of Spy0167 hemolytic activity for Spy0167 mutant W535-P427L is 1/17,860 using Alum adjuvant and 1/7991 using MF59™ adjuvant. Negative control (adjuvant alone) titers are 1/1,000 (Alum) and 1/125 (MF59™).

Table 14 (shown graphically in **FIG. 27**).

| 50 ng/ml (3.5 HU) of wild-type Spy0167 | |
|---|---|
| adjuvant | specific inhibition/non-specific inhibition |
| alum | 18 |
| MF™59 | 64 |

Table 15 (shown graphically in **FIG. 28**)

| 100 ng/ml (37 HU) of wild-type *Spy0167* | |
|---|---|
| adjuvant | specific inhibition/non-specific inhibition |
| alum | >227 |
| MF™59 | >117 |

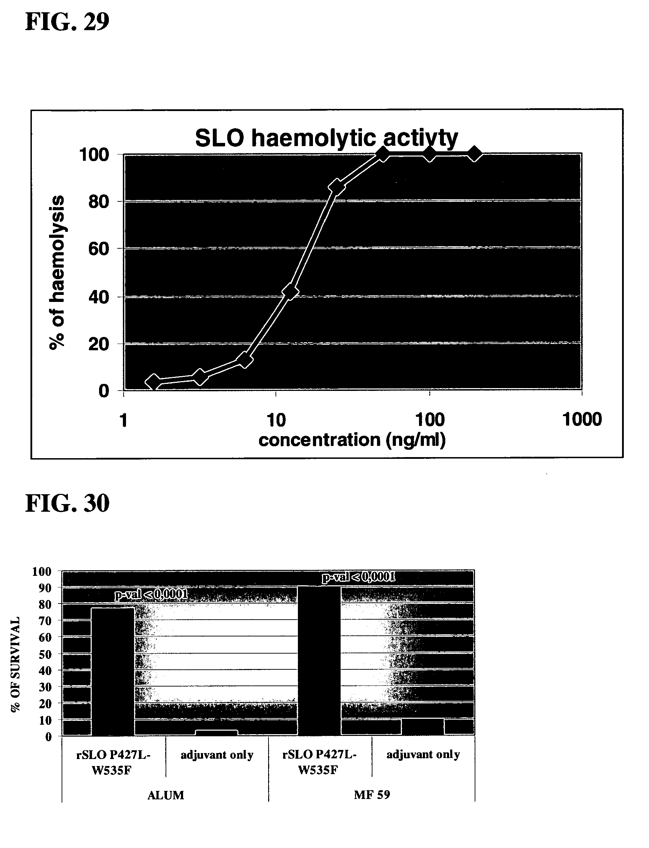

Table 16 (shown graphically in **FIG. 29**)

| ng/ml Spy0167 | % hemolysis |
|---|---|
| 1.6 | 3.5 |
| 3.1 | 5.8 |
| 6.3 | 13 |

(continued)

| ng/ml Spy0167 | % hemolysis |
|---|---|
| 12.5 | 42 |
| 25 | 86 |
| 50 | 100 |
| 100 | 100 |
| 200 | 100 |

EXAMPLE 20

*In vivo protection experiments*

The purified Spy0167 P427L protein, together with Freund's adjuvant, was administered intraperitoneally to 40 mice. The mice were then challenged intranasally with the 3348 M1 GAS strain. Table 17 reports the data obtained in 3 separate experiments, showing that 100% protection was consistently achieved in all experiments.

Table 17. Infection survival rate of mice

| | % surviving mice | | |
|---|---|---|---|
| antigen | Experiment 1 | Experiment 2 | Experiment 3 |
| Spy0167 Pro247Leu | 100 | 100 | 100 |
| *E. coli* contaminants (negative control) | 10 | 10 | 10 |
| homologous M1 protein (positive control) | 100 | 90 | 90 |

Groups of 10-20 mice were immunized with 20 μg of the recombinant protein at days 0, 21 and 35. Mice of negative control groups were immunized either with GST alone or with *E. coli* contaminants, depending on the version of the GAS recombinant protein used. Two weeks after the third immunization, blood samples were taken. A few days afterwards, immunized mice were challenged intranasally with $10^8$ cfu (50 μl) of the M1 3348 GAS strains. Survival of mice was monitored for a 10-14 day period. Immune sera obtained from the different groups were tested for immunogenicity on the entire *Spy0167* recombinant protein (western blot analysis). The results are shown in Tables 18 and 19.

Table 18

| Protein | # mice | % survival | % negative control survival |
|---|---|---|---|
| Spy0167_Pro247Leu His | 10 | 90 | 30 |
| Spy0167_Pro247Leu His | 10 | 100 | 20 |
| Spy0167_Pro247Leu His | 10 | 80 | 30 |
| Spy0167_WT | 20 | 95 | 15 |
| Spy0167_WT | 10 | 100 | 40 |

Table 19

| Protein | # mice | % survival | % negative control survival |
|---|---|---|---|
| rSpy0167 WT *his-tagged* | 20 | 100 | 45 |
| C530G *his-tagged* | 20 | 100 | 45 |
| W535F *his-tagged* | 20 | 100 | 45 |
| W535F-D482N *his-tagged* | 20 | 100 | 45 |
| P427L *his-tagged* | 20 | 95 | 45 |

(continued)

| Protein | # mice | % survival | % negative control survival |
|---|---|---|---|
| Δala248 *his-tagged* | 20 | 100 | 45 |

EXAMPLE 21

*In vivo toxicity experiments*

*Protocols*

*Intravenous injection of Spy0167.* A solution of either wild-type or mutant Spy0167 in PBS is diluted in a solution of PBS + 2 mM DTT, then 100 ml is injected into the tail vein of a mouse. Mice are observed for 2-3 days. Injection of wild-type Spy0167 typically results in death within a few minutes.

*In vivo lethality Inhibition assay.* For lethality inhibition mediated by immune sera, 10 μg/mouse of wild-type Spy0167 (a solution of 100 μg/ml in PBS, 2 mM DTT) are incubated for 20 minutes with rotation at room temperature with either anti-Spy0167 serum or control serum (obtained from mice immunized with adjuvant alone). After incubation, the samples are inoculated in the mice by intravenous injection into the tail vein. Mice are observed for 2-3 days.

The results for wild-type Spy0167 and mutant Spy0167 P427L-W535F are shown in Table 20.

Table 20.

| wild-type Spy0167 | | P427L-W535F | |
|---|---|---|---|
| μg/mouse | dead/treated | μg/mouse | dead/treated |
| | | 100 | 0/4 |
| 50 | 4/4 | 50 | 0/4 |
| 10 | 8/8 | 10 | 0/8 |
| 2 | 0/4 | | |
| 0.4 | 0/4 | | |
| 0.04 | 0/4 | | |

Acute *in vivo* acute toxicity was assessed using a dose of 10 μg/mouse of wild-type Spy0167 as a positive control and injection of Freund's adjuvant alone as a negative control. Ten μg/mouse of wild-type Spy0167 was incubated with either wild-type Spy0167 antiserum or with control serum and inoculated into mice as described above. The results are shown in Table 21.

Table 21.

| wild-type Spy0167 (10 μg/mouse) | | |
|---|---|---|
| sera | serum dilution | dead/treated |
| none | | 8/8 |
| wild-type Spy0167 | 1/5 | 0/4 |
| wild-type Spy0167 | 1/10 | 0/4 |
| wild-type Spy0167 | 1/20 | 4/4 |
| wild-type Spy0167 | 1/50 | 4/4 |
| wild-type Spy0167 | 1/100 | 4/4 |
| negative control | 1/5 | 4/4 |

The results of another set of experiments performed as described above are shown in Tables 22 and 23. In vivo acute toxicity was assessed using either 5 or 10 μg/mouse of wild-type Spy0167. In particular, 10 μg/mouse of wild

type Spy0167 were preincubated either with sera from mice immunized with Spy0167 P427L-W535F or only PBS (no serum). In addition, 5 μg/mouse of wild type Spy0167 were preincubated either with sera from mice immunized with Spy0167 P427L-W535F or sera from mice immunized with PBS plus adjuvant (Alum), as negative control serum.

The results demonstrate that lethal doses of wild-type Spy0167 are neutralized by anti-Spy0167 P427L-W535F sera but not by negative control sera at the same dilution.

Table 22.

| wild-type Spy0167 (10 μg/mouse) | | |
|---|---|---|
| Sera | serum dilution | dead/treated |
| none | --- | 4/4 |
| anti-Spy0167 P427L-W535F, alum adjuvant | 1/5 | 0/4 |

Table 23.

| wild-type Spy0167 (5 μg/mouse) | | |
|---|---|---|
| Sera | serum dilution | dead/treated |
| anti-Spy0167 P427L-W535F, alum adjuvant | 1/5 | 0/4 |
| negative control (alum alone) | 1/5 | 4/4 |

EXAMPLE 22

*Immunization with Spy0167 P427L- W535F protects mice against intravenous injection of wild-type Spy0167*

Mice were immunized intraperitoneally three times (day 0, day 21, and day 35) with either wild-type Spy0167 or with the Spy0167 mutant P427L-W535F using alum as an adjuvant (20 μg protein in 2 mg/ml aluminium hydroxide). Mice immunized with adjuvant alone were used as a negative control. On day 55 mice were injected intravenously with different concentrations of a solution of wild-type Spy0167 in PBS, 2 mM DTT and monitored for at least 72 hours. The results are shown in Table 24.

Table 24

| | Dose of wild-type tagless Spy0167 injected into mouse tail vein | | | |
|---|---|---|---|---|
| | 2.5 μg/mouse | 5 μg/mouse | 10 μg/mouse | 20 μg/mouse |
| | survival (no. of mice treated) | survival (no. of mice treated) | survival (no. of mice treated) | survival (no. of mice treated) |
| adjuvant (alum) | 100% (4) | 0% (12) | not tested | not tested |
| wild-type Spy0167 tagless | not tested | 100% (8) | 100% (4) | 100% (4) |
| Spy0167 P427L-W535F tagless | not tested | 100% (8) | 100% (4) | 100% (4) |

Five μg/mouse of wild-type Spy0167 is lethal for mice immunized with adjuvant alone; these mice died within a few minutes after Spy0167 injection. However, even 20 μg/mouse of the same wild-type Spy0167 preparation did not kill mice immunized with either wild-type Spy0167 or with the P427L-W535F Spy0167 mutant.

EXAMPLE 23

*Protection against intranasal challenge with GAS M1 strain by Spy0167 mutant P427L-W535F*

Thirty mice were immunized intraperitoneally with the Spy0167 mutant P427L-W535F, with either Alum or MF59 as adjuvants, and challenged intranasally with a GAS M1 strain. The results are shown in **FIG. 30**. Seventy-seven

percent of the mice immunized with the Spy0167 mutant P427L-W535F and Alum were protected against intranasal challenge with a GAS M1 strain, as compared with 3% of the negative control mice (immunized with adjuvant only). Ninety percent of the mice immunized with the Spy0167 mutant P427L-W535F and MF59 were protected against intranasal challenge with a GAS M1 strain, as compared with 10% of the negative control mice (immunized with adjuvant only). These protection levels are comparable with those obtained by immunizing mice with wild-type Spy0167.

EXAMPLE 24

*In vivo protection studies of mice immunized with GAS antigens*

This example provides the results of immunogenicity / protection tests carried out with various combinations of GAS antigens and/or GAS-specific polysaccharide conjugated with CRM197 (GC) following challenge with GAS strains of different M types. GAS proteins and GC were formulated either with Freund's adjuvant, aluminium hydroxide, or MF59. Protein antigen doses were 20 $\mu$g when used alone; protein combination formulations contained 20 $\mu$g each of wild-type Spy0269 (SEQ ID NO:177) and Spy0416 D151A/S617A (SEQ ID NO:198) and 10 $\mu$g of Spy0617 P427L/W535F (SEQ ID NO:125). GC doses are indicated in the tables.

The immunization schedule involved three doses at days 0, 21, and 35. Bleedings were done before first immunization and two weeks after the third immunization. Negative control groups were immunized with adjuvant only. Positive control groups were immunized with M protein homologous to the challenge strain.

Two weeks after the third immunization, mice were infected with lethal doses ranging from 2.5 x $10^6$ to 2.5 x $10^8$ (intranasal infection) or 20 to 2.5 x $10^6$ (intraperitoneal infection), depending on the challenge strain used. Survival rates were determined and are reported in Tables 25 and 26. The p-value was calculated with Fisher's test.

Immunogenicity was tested by ELISA.

*Protection by single antigens and their combination in Freund's adjuvant against intranasal infection with M1, M12, and M23*

Table 25 reports the results of experiments in which mice were immunized with Spy0269 (SEQ ID NO:177), Spy0416 D151A/S617A (SEQ ID NO:198), or Spy0617 P427L/W535F (SEQ ID NO:125), or a combination of these antigens ("combo") formulated with Freund's adjuvant and then challenged intranasally with M1, M12 and M23 strains. The results indicate that:

a. Spy0269 confers statistically significant protection against M1, M12 and M23 infection;

b. Spy0416 D151A/S617A and Spy0617 P427L/W535F confer, significant protection against intranasal infection with M1 serotype; and

c. the combination of Spy0269, Spy0416 D151A/S617A and Spy0617 P427L/W535F confers >40% protection against M1, M12 and M23 GAS serotypes.

Table 25.

| | M1 3348 | | | M12 EM5 | | | M23 2071 | | |
|---|---|---|---|---|---|---|---|---|---|
| antigen | Live/Total | % surv | Pval | Live/Total | % surv | Pval | Live/Total | % surv | Pval |
| Spy0269 | 82/145 | 57 | 0.0001 | 73/165 | 44 | 0.0001 | 23/35 | 66 | 0.0001 |
| Spy0416 D151 A/S617A | 114/168 | 68 | 0.0001 | 22/80 | 28 | 0.1855 | 27/60 | 45 | 0.1855 |
| Spy0167 P427L/W534F | 105/114 | 92 | 0.0001 | 23/80 | 29 | 0.4842 | 7/20 | 35 | 0.2733 |
| combo | 145/152 | 95 | 0.0001 | 33/80 | 41 | 0.1354 | 39/80 | 49 | 0.0002 |
| M protein | 176/184 | 96 | | 136/180 | 76 | | 78/79 | 99 | |
| negative | 67/241 | 28 | | 64/258 | 25 | | 33/134 | 25 | |

*Protection by the combination of GAS25, GAS40, and GAS57 antigens plus GC, formulated with Alum against intraperitoneal infection with M1*

Table 26 reports the results of experiments in which mice were immunized with the combination of Spy0167 mutant P427L/W535F, wild-type Spy0269, and Spy0416 mutant D151A/S617A ("combo") with or without GC formulated with Alum and then challenged intraperitoneally with M1. The results indicate that statistically significant protection was obtained both with the protein combination alone and with the protein combination plus GC. Thus, even in combination, the exceptional immunogenicity of these GAS antigens is maintained.

Table 26

| | M1 3348 | | |
|---|---|---|---|
| antigen | Live/Total | % Surv | Pval |
| combo | 45/92 | 49 | 0,0001 |
| GC | 82/168 | 49 | 0,0001 |
| combo+GC | 36/72 | 50 | 0,0001 |
| M protein | 51/58 | 88 | 0,0001 |
| negative | 36/252 | 14 | |

EXAMPLE 25

*Cell binding assays*

Human (A549, HeLa, 293, Detroit, ME180) or monkey (LLCMK2) epithelial cell lines are non-enzymatically detached from their support using a cell dissociation solution (Sigma), harvested, and suspended in Dulbecco's modified Eagle medium (DMEM). Approximately 2 x $10^5$ cells are mixed with either medium alone or with different Spy0269 recombinant protein concentrations (μg/ml) in a total volume of 200 ml in 96-well plates with U-shaped bottoms. Incubation at 4°C is carried out for 1 hour. After two washes with PBS, cells are incubated with Spy0269 antibodies or antiserum (*e.g.*, for antiserum, 1:200 in PBS/BSA 1%) for 1 hour at 4°C. After two washes, the samples are incubated at 4°C for 30 minutes with a secondary antibody (*e.g.*, for a mouse Spy0269 antiserum, the secondary antibody can be a R-phycoerythrin-conjugated goat $F(ab)_2$ antibody specific for mouse immunoglobulin diluted 1:100 in PBS/BSA 1%). Binding reactions are analyzed by flow cytometry. The mean fluorescence intensity for each population is calculated.

EXAMPLE 26

*Opsonophagocytosis assay*

This example describes the opsonophagocytosis assay used in the Examples below. Briefly, bacteria (10-50 colony forming units, CFUs, 25 μl in PBS) are incubated with 225 μl of whole blood from rabbits immunized either with adjuvant alone or with the tested antigen(s). The samples are incubated 5 hr at 37° with end-over-end rotation. Following dilution, samples are plated on blood agar plates and the number of CFUs is estimated.

In this assay, background killing by sera from animals immunized with adjuvant alone ranges from 7-36%. Killing activity by antigens varies but is consistently positive (*e.g.*, 72-97% for M1 antibodies, 47-64% for GC antibodies, and 76-85% for antibodies raised against the combination of wild-type Spy0269 (SEQ ID NO:177), Spy0167 double mutant P427L/W535F (SEQ ID NO:125), and Spy0416 double mutant D151A/S617A (SEQ ID NO:198).

EXAMPLE 27

*Whole blood bactericidal assay demonstrating that anti-glycoconjugate (GC) antibodies meditate killing of S. pyogenes*

The assay described in Example 26 was carried out using whole blood obtained from rabbits immunized with 100 μg GC). The results, shown in **FIG. 34**, demonstrate that anti-GC antibodies mediate killing of *S. pyogenes.*

EXAMPLE 28

*Whole blood bactericidal assays remonstrating that the combination of anti-glycoconjugate (GC) antibodies and antibodies generated against GAS antigen combinations enhanced killing of S. pyogenes*

The assay described in Example 26 was carried out with whole blood obtained from rabbits immunized with (a) Freund's adjuvant, (b) M1 protein, (c) a combination of wild-type Spy0269 (SEQ ID NO:177), Spy0167 double mutant P427L/W535F (SEQ ID NO:125), and Spy0416 double mutant D151A/S617A (SEQ ID NO:198) (100 μg each), (d) GC, and (e) the combination of wild-type Spy0269 (SEQ ID NO:177), Spy0167 double mutant P427L/W535F (SEQ ID NO:125), Spy0416 double mutant D151A/S617A (SEQ ID NO:198), and GC. The results are shown in **FIG. 35.**

It is desirable for a GAS vaccine to be bactericidal as well as immunogenic. These results demonstrat that, even in combination, these GAS antigens have bactericidal activity. The results also demonstrate a higher bactericidal effect of the combination of GAS antigens and GC antigen compared with that of either the GAS antigen combination or GC antigen alone.

EXAMPLE 29

*Experiments demonstrating lack of cellular toxicity of GAS antigens*

Endothelial cells human brain microvascular endothelial cells (HBMECs) were treated *in vitro* for 24 hours with various concentrations of recombinant GAS antigens in RPMI 1640 medium. Negative controls were not treated ("NT"), and cells treated with TNFα 1 μg/ml were used as positive controls. Annexin V and propidium iodide staining were used to measure the percentage of apoptotic cells by flow cytometry. The results indicate no significant toxicity at the concentrations of wild-type Spy0269 (SEQ ID NO:177), Spy0167 double mutant P427L/W535F (SEQ ID NO:125), Spy0416 double mutant D151A/S617A (SEQ ID NO:198), and glycoconjugate, ("GAS GC") used in these Examples. See **FIGS. 36A-D.**

EXAMPLE 30

*Protein antigen conservation and expression.*

The table below shows the average percent identity for each of Spy0269, Spy0416, and Spy0167 among 57, 49, and 13 *S. pyogenes* strains, respectively.

| antigen | % identity (no. strains analyzed) | FACS positive |
|---|---|---|
| Spy0269 | 93% (57 strains) | 119/188 (63.3%) |
| Spy0416 | 95% (49 strains) | 98/174 (56.3%) |
| Spy0167 | 97% (13 strains) | 32/60 (53.3%) |

EXAMPLE 31

*ELISA assays*

Briefly, plates are coated with antigen (0.1-0.3 μg/well) and blocked with 2% bovine serum albumin (BSA) in phosphate-buffered saline (PBS). After incubation with two-fold serial dilutions of the tested sera, plates are washed with 2% bovine serum albumin (BSA) in phosphate-buffered saline (PBS), and 0.05% TWEEN20® and incubated with secondary antibody (anti-total IgG, 1:2000) conjugated with alkaline phosphatase. After incubation with the substrate p-nitrophenyl phosphate (pNPP, 3 μg/ml), absorbance is measured at 405 nm. Serum titers are calculated by interpolating ODs from a standard curve. This assay is linear and reproducible, as shown in **FIGS. 37A-D.**

EXAMPLE 32

*In vivo challenge experiments*

CD1 5-6 week old female mice were immunized intraperitoneally 3 times on days 0, 21 and 35 with various

doses of GAS antigens adjuvanted with alum in PBS and challenged either intranasally (50 -ml Todd Hewitt containing an LD90 bacterial dose) or intraperitoneally (200 μl Todd Hewitt containing an LD90 bacterial dose) with various strains of *S. pyogenes.* Results are shown in Tables 27 and 28. In Tables 27 and 28, "40," "25," and "57," respectively, are wild-type Spy0269 (SEQ ID NO:177), Spy0167 double mutant P427L/W535F (SEQ ID NO:125), and Spy0416 double mutant D151A/S617A (SEQ ID NO:198).

Table 27. Protection conferred by GAS antigens and combinations of GAS antigens in an intraperitoneal challenge model.

| | | Challenge serotypes/strains | | | | | |
|---|---|---|---|---|---|---|---|
| | | M1 3348 | | M23 2071 | | M6 S43 | |
| adjuvant | antigen | % survival (no. mice) | P | % survival (no. mice) | P | % survival (no. mice) | P |
| | GC | 47 (176) | <0.001 | 30 (80) | <0.001 | 33 (104) | <0.001 |
| | 25+40+57 | 79 (156) | <0.001 | 36 (64) | <0.001 | 36 (88) | <0.001 |
| | 25+40+57 +GC | 74 (80) | <0.001 | 48 (64) | <0.001 | 48 (88) | <0.001 |
| alum | adjuvant alone | 14 (>100) | | 10 (>100) | | 11 (>100) | |

Table 28. Protection conferred by GAS antigens and combinations of GAS antigens in an intranasal challenge model.

| | | challenge serotypes/strains | | | | | | | |
|---|---|---|---|---|---|---|---|---|---|
| | | M1 3348 | | M12 EM5 | | M23 2071 | | M6 S43 | |
| adjuvant | GAS antigen | % survival (no. mice) | P | % survival (no. mice) | P | % survival (no. mice) | P | % survival (no. mice) | P |
| Freund's | 25 | 95 (100) | <0.001 | 38 (50) | | 30 (60) | | | |
| | 40 | 45 (82) | <0.001 | 42 (150) | <0.001 | 66 (35) | <0.001 | | |
| | 57 | 72 (143) | <0.001 | 33 (189) | <0.05 | 39 (80) | | | |
| | 25+40+57 | 96 (56) | <0.001 | 40 (100) | <0.05 | 49 (80) | <0.001 | | |
| | (adjuvant alone) | 23 (>100) | | 21 (>100) | | 21 (>100) | | | |
| alum | 25 | 88 (48) | <0.001 | | | | | | |
| | 40 | 27 (130) | <0.05 | | | | | | |
| | 57 | 38 (157) | <0.001 | | | | | | |
| | 40+57 | 67 (46) | <0.001 | | | | | | |
| | 25+40+57 | 87 (280) | <0.001 | 33 (128) | <0.001 | 54 (48) | <0.001 | 53 (64) | <0.001 |
| | (adjuvant alone) | 15 (>100) | | 9 (128) | | 10 (48) | | 22 (64) | |

EXAMPLE 33

*Inclusion of alum provides protection against strain M1 3348*

This example demonstrates that inclusion of Alum in both Spy0167 and in combination formulations provides protection against *S. pyogenes* strain M1 3348.

CD1 5-6 week female mice were immunized with Spy0167 (GAS25) 10 μg or with a combination of Spy0167 (10 μg) together with Spy0269 (GAS40, 20 μg) and Spy0416 (GAS57, 20 μg) ("combination"), with or without alum. Animals were immunized intraperitoneally with 3 doses at days 0, 21 and 35. Intranasal challenge with M1 3348 was carried out essentially as described in Example 4. The results are shown in Table 29.

Table 29. Effect of including alum on survival after intranasal challenge with M1 3348.

| antigen | adjuvant | % survival after M1 3348 challenge (no. animals) |
|---|---|---|
| Spy0167 | alum | 84 (32) |
| Spy0167 | -- | 29 (32) |
| -- | alum | 14 (31) |
| combination | alum | 66 (32) |
| combination | | 23 (32) |
| -- | alum | 14 (29) |

EXAMPLE 34

*Stability of GAS antigen formulations*

Stability and *in vivo* potency of a combination GAS antigen formulation containing 100 μg/ml Spy0269 (1 mg/ml solution in PBS), 100 μg Spy0416 double mutant D151A/S617A (1 mg/ml solution in PBS), 50 μg Spy0167 double mutant P427L/W535F (1 mg/ml solution in PBS), 2 mg/ml aluminum hydroxide, 10 mM histidine buffer (pH 7.0), 9 g/l sodium chloride, with a pH of 7.0 +/- 0.3, with an osmolality of 300 +/- 20 mOsm/kg was tested by SDS-PAGE analysis for antigen integrity. The formulation is stable up to 1 year at 4°C. Antigen stability was evaluated by incubating at 2-8°C over a one year period. All three protein components appeared quite stable after one year as assessed by SDS-PAGE. The protein antigens remain adsorbed to alum (>97.5 %) for at least 36 weeks 2-8°C.

EXAMPLE 35

*Effect of Spy0416 and Spy0167 antibodies*

This example demonstrates that antibodies to Spy0416 and Spy0167 block toxic activity.

**Spy0416:** Spy0416 was pre-incubated with pools of mouse specific sera or with a human serum with high ELISA titres to Spy0416. The mix was then incubated with IL-8 (10 μg/ml), and then tested for the presence of uncleaved IL-8 using an antibody which is specific for the cytokine but which is unable to recognize the cleaved inactive form. The results are expressed as percentage of uncleaved IL-8 calculated as follows:

$$\frac{[\text{IL-8 in the reaction mix}]}{[\text{IL-8 in the control mix}]} \times 100,$$

where "control mix" is the reaction mix without the enzyme at time point 0.

**Spy0167:** Wildtype Spy0167 was pre-incubated with a pool of sera from mice immunized either with 20 μg of Spy0167P427L/W535F or with adjuvant alone, and with human sera from responders and non responders. The mix was added to a sheep blood cell suspension and the OD540 nm decrease of the reaction supernatant was determined. Inhibition titer is expressed as the serum dilution required to reduce Spy0167-induced hemolysis by 50%.

The results are shown in **FIGS. 41A-B.**

EXAMPLE 36

*Dose-range experiments*

Five-week old female CD1 mice were immunized with varying doses of wild-type Spy0269 (SEQ ID NO:177), Spy0416 mutant D151A/S617A (SEQ ID NO:198), and Spy0167 mutant P427L/W535F (SEQ ID NO:125) on days 0,

21, and 35. Dose-dependent IgG responses in the mice were measured by ELISA as described in Example 31. The results are shown in **FIGS. 38A-C.**

Mice were immunized with individual GAS protein antigens at various concentrations and challenged intranasally with *S. pyogenes* M1. The results are shown in Table 30.

Table 30.

| protein | dose (μg) | mice | dead | % survival |
|---|---|---|---|---|
| GAS40 | 2 | 32 | 20 | 38 |
| | 20 | 32 | 18 | 44 |
| Spy0416 mutant D151A/S617A | 2 | 32 | 16 | 53 |
| | 20 | 32 | 19 | 43 |
| Spy0167 mutant P427L/W535F (SEQ ID NO:125) | 2 | 32 | 24 | 75 |
| | 0.5 | 32 | 28 | 87 |
| | 0.125 | 32 | 20 | 62 |
| (PBS) | | 32 | 27 | 16 |

As shown in Table 30, there is no clear dose-dependent protection, indicating that a variety of concentrations of these antigens are useful for achieving protection against *S. pyogenes* challenge.

Mice were immunized with the combination of 20 μg wild-type Spy0269 (SEQ ID NO:177), 10 μg Spy0167 double mutant P427L/W535F (SEQ ID NO.:125), and 20 μg Spy0416 double mutant D151A/S617A (SEQ ID NO:198) at various concentrations and challenged intranasally with *S. pyogenes* M1. The results are shown in Table 31.

Table 31.

| protein | dose (μg) | mice | dead | % survival |
|---|---|---|---|---|
| combination | 20+20+10 | 16 | 11 | 31 |
| | 20+20+2 | 16 | 10 | 38 |
| | 20+2+10 | 16 | 6 | 63 |
| | 20+2+2 | 16 | 7 | 50 |
| | 2+20+10 | 16 | 2 | 88 |
| | 2+20+2 | 16 | 9 | 44 |
| | 2+2+10 | 16 | 16 | 0 |
| | 2+2+2 | 16 | 14 | 13 |
| none | 0+0+0 | 16 | 15 | 6 |

As with the single antigen dose experiments described above, there is no clear dose-dependent protection, indicating that, even in combination, a variety of concentrations of these antigens are useful for achieving protection against *S. pyogenes* challenge.

The results are summarized in **FIG. 39. FIG. 40** shows an analysis of a LogNormal model adopted as a first approximation of mean survival time (MST; *Mu*).

## Claims

1. A composition comprising a combination of three *S. pyogenes* (GAS) protein antigens, wherein the protein antigens are:

   (1) Spy0167, wherein the Spy0167 protein antigen has an amino acid sequence that is at least 90% identical to SEQ ID NO:107;
   (2) Spy0269, wherein the Spy0269 protein antigen has an amino acid sequence that is at least 90% identical to SEQ ID NO:50; and
   (3) Spy0416, wherein the Spy0416 protein antigen has an amino acid sequence that is at least 90% identical to SEQ ID NO:1.

2. The composition of claim 1, wherein the Spy0167 protein antigen is a mutant Spy0167 protein comprising an amino acid alteration at one or more amino acid positions selected from the group consisting of amino acids P427, W535, C530, A248, and D482, wherein the amino acid positions are numbered according to SEQ ID NO:107 and wherein the hemolytic activity of the mutant Spy0167 protein is reduced by at least 50% relative to wild-type Spy0167.

3. The composition of claim 1 or claim 2, wherein the Spy0416 protein antigen is a mutant Spy0416 protein comprising an amino acid alteration at one or more amino acid positions selected from the group consisting of amino acids D151, H279, and S617, wherein the amino acid positions are numbered according to SEQ ID NO:1 and wherein proteolytic activity of the purified mutant Spy0416 antigen against interleukin 8 (IL-8) is reduced by at least 50% relative to wild-type Spy0416 as detected by SDS-polyacrylamide gel electrophoresis or by an ELISA assay.

4. The composition of claim 2 or claim 3 wherein the three GAS protein antigens are:

   1. Spy0167 mutant P427L/W535F, Spy0269, and Spy0416;
   2. Spy0167, Spy0269, and Spy0416 mutant D151A/S617A; or
   3. Spy0167 mutant P427L/W535F, Spy0269, and Spy0416 mutant D151A/S617A.

5. The composition of claim 3, wherein the mutant Spy0416 antigen comprises at least one amino acid substitution selected from the group consisting of D151A and S617A.

6. The composition of claim 5, wherein the mutant Spy0416 antigen comprises SEQ ID NO:147, SEQ ID NO:148, SEQ ID NO:149, or SEQ ID NO:198.

7. The composition of claim 6, wherein the Spy0269 antigen comprises SEQ ID NO:177.

8. The composition of claim 3, wherein the mutant Spy0416 antigen is a fusion protein comprising a second GAS antigen.

9. The composition of claim 2, wherein the mutant Spy0167 antigen comprises an amino acid sequence selected from the group consisting of SEQ ID NO:120, SEQ ID NO:121, SEQ ID NO:122, SEQ ID NO:123, SEQ ID NO:124, SEQ ID NO:125, SEQ ID NO:126, and SEQ ID NO:127.

10. The composition of any of claims 1-9, wherein at least one of the GAS antigens is coupled to a carrier protein, such as a carrier protein selected from the group consisting of a bacterial toxin, a bacterial toxoid, a *N. meningitidis* outer membrane protein, a heat shock protein, a pertussis protein, *H. influenzae* protein D, a cytokine, a lymphokine, a hormone, a growth factor, *C. difficile* toxin A, *C. difficile* toxin B, and an iron-uptake protein.

11. The composition of any of claims 1-10 which further comprises a pharmaceutically acceptable carrier.

12. The composition of any of claims 1-11 further comprising an active agent which is useful in a pediatric vaccine, such as an active agent selected from the group consisting of:

(a) a polypeptide antigen selected from the group consisting of *N. meningitidis, S. pneumoniae, Bordetella pertussis, Moraxella catarrhalis, Clostridium tetani, Chorinebacterim diphtheriae*, respiratory syncytial virus, polio virus, measles virus, mumps virus, rubella virus, and rotavirus polypeptide antigens; and
(b) a nucleic acid molecule which encodes the polypeptide antigen.

**13.** The composition of any of claims 1-11 further comprising a second active agent which is useful in a vaccine for elderly or immunocompromised individuals, such as a second active agent selected from the group consisting of:

(a) a polypeptide antigen selected from the group consisting of *Enterococcus faecalis, Staphylococcus aureaus, Staphylococcus epidermis, Pseudomonas aeruginosa, Legionella pneumophila*, *Listeria monocytogenes*, influenza virus, and parainfluenza virus polypeptide antigens; and
(b) a nucleic acid molecule which encodes the polypeptide antigen.

**14.** The composition of any of claims 1-13 further comprising a group A polysaccharide of formula

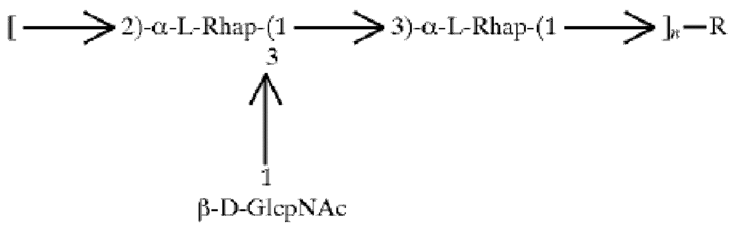

(I),

wherein R is a terminal reducing L-rhamnose or D-GlcpNAc and n is a number from about 3 to about 30.

**15.** The composition of claim 14, wherein said composition comprises Spy0167 mutant P427L/W535F, Spy0269, Spy0416 mutant D151A/S617A and a group A polysaccharide of formula

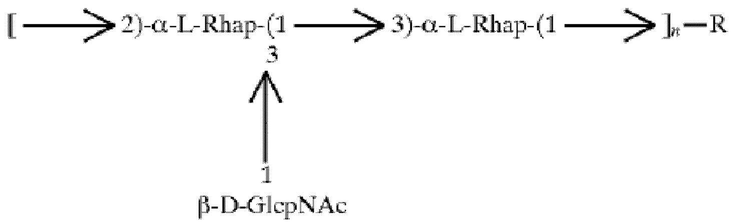

(I),

wherein R is a terminal reducing L-rhamnose or D-GlcpNAc and n is a number from about 3 to about 30, and wherein the group A polysaccharide antigen is conjugated to the mutated diphtheria toxin CRM 197.

**16.** The composition of any of claims 1-14 further comprising an adjuvant, such as alum.

**17.** The composition of any of claims 1-16 for use as a vaccine.

**18.** The composition of any of claims 1-16 for use in treating a *Streptococcus pyogenes* infection.

**Patentansprüche**

**1.** Zusammensetzung, umfassend eine Kombination dreier S. pyogenes (GAS)-Proteinantigene, wobei die Proteinantigene sind:

(1) Spy0167, wobei das Spy0167-Proteinantigen eine Aminosäuresequenz aufweist, die zu mindestens 90 % identisch zu SEQ ID NO: 107 ist;
(2) Spy0269, wobei das Spy0269-Proteinantigen eine Aminosäuresequenz aufweist, die zu mindestens 90 %

identisch zu SEQ ID NO: 50 ist; und
(3) Spy0416, wobei das Spy0416-Proteinantigen eine Aminosäuresequenz aufweist, die zu mindestens 90 % identisch zu SEQ ID NO: 1 ist.

**2.** Zusammensetzung gemäß Anspruch 1, wobei das Spy0167-Proteinantigen eine Spy0167-Proteinmutante ist, umfassend eine Aminosäureänderung an einer oder mehreren Aminosäurepositionen, ausgewählt aus der Gruppe, bestehend aus den Aminosäuren P427, W535, C530, A248 und D482, wobei die Aminosäurepositionen gemäß SEQ ID NO: 107 nummeriert sind und wobei die hämolytische Aktivität der Spy0167-Proteinmutante um mindestens 50 % relativ zum Wildtyp Spy0167 verringert ist.

**3.** Zusammensetzung gemäß Anspruch 1 oder Anspruch 2, wobei das Spy0416-Proteinantigen eine Spy0416-Proteinmutante ist, umfassend eine Aminosäureänderung an einer oder mehreren Aminosäurepositionen, ausgewählt aus der Gruppe, bestehend aus den Aminosäuren D151, H279 und S617, wobei die Aminosäurepositionen gemäß SEQ ID NO: 1 nummeriert sind und wobei die proteolytische Aktivität der aufgereinigten Spy0416-Antigenmutante gegenüber Interleukin 8 (IL-8) um mindestens 50 % relativ zum Wildtyp Spy0416 verringert ist, wie durch SDS-Polyacrylamid-Gelelektrophorese oder durch einen ELISA-Assay nachgewiesen.

**4.** Zusammensetzung gemäß Anspruch 2 oder Anspruch 3, wobei die drei GAS-Proteinantigene sind:

1. Spy0167-Mutante P427L/W535F, Spy0269 und Spy0416,
2. Spy0167, Spy0269 und Spy0416-Mutante D151A/S617A; oder
3. Spy0167-Mutante P427L/W535F, Spy0269 und Spy0416-Mutante D151A/S617A.

**5.** Zusammensetzung gemäß Anspruch 3, wobei die Spy0416-Antigenmutante mindestens eine Aminosäuresubstitution, ausgewählt aus der Gruppe, bestehend aus D151A und S617A, umfasst.

**6.** Zusammensetzung gemäß Anspruch 5, wobei die Spy0416-Antigenmutante SEQ ID NO: 147, SEQ ID NO: 148, SEQ ID NO: 149 oder SEQ ID NO: 198 umfasst.

**7.** Zusammensetzung gemäß Anspruch 6, wobei das Spy0269-Antigen SEQ ID NO: 177 umfasst.

**8.** Zusammensetzung gemäß Anspruch 3, wobei die Spy0416-Antigenmutante ein Fusionsprotein, umfassend ein zweites GAS-Antigen, ist.

**9.** Zusammensetzung gemäß Anspruch 2, wobei die Spy0167-Antigenmutante eine Aminosäuresequenz, ausgewählt aus der Gruppe, bestehend aus SEQ ID NO: 120, SEQ ID NO: 121, SEQ ID NO: 122, SEQ ID NO: 123, SEQ ID NO: 124, SEQ ID NO: 125, SEQ ID NO: 126 und SEQ ID NO: 127 umfasst.

**10.** Zusammensetzung gemäß einem der Ansprüche 1 bis 9, wobei mindestens eines der GAS-Antigene an ein Trägerprotein, wie z.B. ein Trägerprotein, ausgewählt aus der Gruppe, bestehend aus einem bakteriellen Toxin, einem bakteriellen Toxoid, einem N. meningitidis-Außenmembranprotein, einem Hitzeschockprotein, einem Pertussis-Protein, H. influenzae-Protein D, einem Cytokin, einem Lymphokin, einem Hormon, einem Wachstumsfaktor, C. difficile-Toxin A, C. difficile-Toxin B und einem Eisenaufnahmeprotein, gekoppelt ist.

**11.** Zusammensetzung gemäß einem der Ansprüche 1 bis 10, die ferner einen pharmazeutisch akzeptablen Träger umfasst.

**12.** Zusammensetzung gemäß einem der Ansprüche 1 bis 11, ferner umfassend einen Wirkstoff, der in einem pädiatrischen Impfstoff nützlich ist, wie z.B. ein Wirkstoff, ausgewählt aus der Gruppe, bestehend aus:

(a) einem Polypeptidantigen, ausgewählt aus der Gruppe, bestehend aus N. meningitidis-, S. pneumoniae-, Bordetella pertussis-, Moraxella catarrhalis-, Clostridium tetani-, Chorinebacterim diphteriae-, respiratorischen Syncytialvirus-, Poliovirus-, Masernvirus-, Mumpsvirus-, Rubellavirus- und Rotavirus-Polypeptidantigenen; und
(b) einem Nukleinsäuremolekül, das das Polypeptidantigen kodiert.

**13.** Zusammensetzung gemäß einem der Ansprüche 1 bis 11, ferner umfassend einen zweiten Wirkstoff, der in einem Impfstoff für ältere oder immungeschwächten Individuen nützlich ist, wie z.B. ein zweiter Wirkstoff, ausgewählt aus der Gruppe, bestehend aus:

(a) einem Polypeptidantigen, ausgewählt aus der Gruppe, bestehend aus Enterococcus faecalis-, Staphylococcus aureaus-, Staphylococcus epidermis-, Pseudomonas aeruginosa-, Legionella pneumophila-, Listeria monocytogenes-, Influenzavirus- und Parainfluenzavirus-Polypeptidantigenen; und
(b) einem Nukleinsäuremolekül, das das Polypeptidantigen kodiert.

**14.** Zusammensetzung gemäß einem der Ansprüche 1 bis 13, ferner umfassend ein Gruppe A-Polysaccharid der Formel

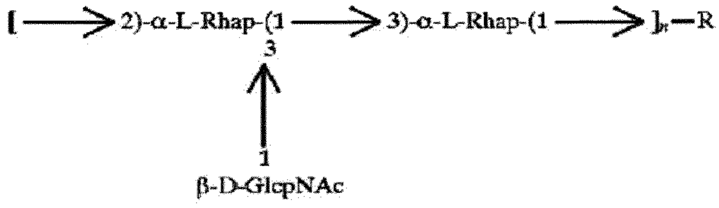

(I)

wobei R eine terminal reduzierende L-Rhamnose oder D-GlcpNAc ist und n eine Zahl von etwa 3 bis etwa 30 ist.

**15.** Zusammensetzung gemäß Anspruch 14, wobei die genannte Zusammensetzung die Spy0167-Mutante P427L/W535F, Spy0269, Spy0416-Mutante D151A/s617A und ein Gruppe A-Polysaccharid der Formel

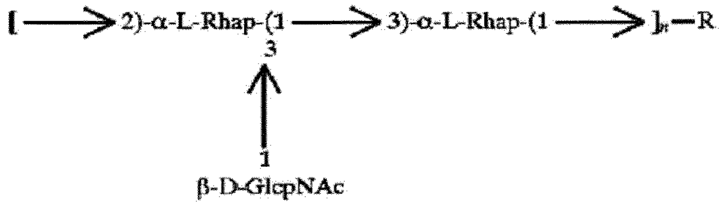

(I)

umfasst,
wobei R eine terminal reduzierende L-Rhamnose oder D-GlcpNAc ist und n eine Zahl von etwa 3 bis etwa 30 ist und wobei das Gruppe A-Polysaccharidantigen an das mutierte Diphtherie-Toxin CRM197 konjugiert ist.

**16.** Zusammensetzung gemäß einem der Ansprüche 1 bis 14, ferner umfassend ein Adjuvans, wie z.B. Alum.

**17.** Zusammensetzung gemäß einem der Ansprüche 1 bis 16 zur Anwendung als ein Impfstoff.

**18.** Zusammensetzung gemäß einem der Ansprüche 1 bis 16 zur Anwendung bei der Behandlung einer Streptococcus pyogenes-Infektion.

## Revendications

**1.** Composition comprenant une combinaison de trois antigènes protéiques contre *S. pyogenes* (GAS), dans laquelle les antigènes protéiques sont :

(1) Spy0167, où l'antigène protéique Spy0167 a une séquence d'acides aminés qui est au moins 90 % identiques à la SEQ ID NO : 107 ;
(2) Spy0269, où l'antigène protéique Spy0269 a une séquence d'acides aminés qui est au moins 90 % identiques à la SEQ ID NO : 50 ; et
(3) Spy0416, où l'antigène protéique Spy0416 a une séquence d'acides aminés qui est au moins 90 % identiques à la SEQ ID NO : 1.

**2.** Composition selon la revendication 1, dans laquelle l'antigène protéique Spy0167 est une protéine Spy0167 mutante

comprenant une modification d'acide aminé sur une ou plusieurs positions d'acides aminés choisies dans le groupe constitué par les acides aminés P427, W535, C530, A248 et D482, dans laquelle les positions des acides aminés sont numérotées selon la SED ID NO : 107 et dans laquelle l'activité hémolytique de la protéine Spy0167 mutante est réduite par au moins 50 % par rapport à la protéine SPy0167 de type sauvage.

**3.** Composition selon la revendication 1 ou la revendication 2, dans laquelle l'antigène protéique Spy0416 est une protéine Spy0416 mutante comprenant une modification d'acide aminé sur une ou plusieurs positions d'acides aminés choisies dans le groupe constitué par les acides aminés D151, H279 et S617, dans laquelle les positions des acides aminés sont numérotées selon la SED ID NO : 1 et dans laquelle l'activité protéolytique de l'antigène Spy0416 mutant purifié contre l'interleukine 8 (IL-8) est réduite par au moins 50 % par rapport à l'antigène Spy00416 de type sauvage comme détecté par électrophorèse sur gel de SDS-polyacrylamide ou par un essai ELISA.

**4.** Composition selon la revendication 2 ou la revendication 3, dans laquelle les trois antigènes protéiques contre GAS sont :

1. Spy0167 mutant P427L/W535F, Spy0269 et Spy0416 ;
2. Spy0167, Spy0269 et Spy0416 mutant D151A/S617A ; ou
3. Spy0167 mutant P427L/W535F, Spy0269 et Spy0416 mutant D151A/S617A.

**5.** Composition selon la revendication 3, dans laquelle l'antigène Spy0416 mutant comprend au moins une substitution d'acide aminé choisie dans le groupe constitué par D151A et S617A.

**6.** Composition selon la revendication 5, dans laquelle l'antigène Spy0416 mutant comprend SEQ ID NO : 147, SEQ ID NO : 148, SEQ ID NO : 149 ou SEQ ID NO : 198.

**7.** Composition selon la revendication 6, dans laquelle l'antigène Spy0269 comprend SEQ ID NO : 177.

**8.** Composition selon la revendication 3, dans laquelle l'antigène Spy0416 mutant est une protéine de fusion comprenant un second antigène contre GAS.

**9.** Composition selon la revendication 2, dans laquelle l'antigène Spy0167 mutant comprend une séquence d'acides aminés choisie dans le groupe constitué par SEQ ID NO : 120, SEQ ID NO : 121, SEQ ID NO : 122, SEQ ID NO : 123, SEQ ID NO : 124, SEQ ID NO : 125, SEQ ID NO : 126 et SEQ ID NO : 127.

**10.** Composition selon l'une quelconque des revendications 1 à 9, dans laquelle au moins un des antigènes contre GAS est couplé à une protéine support, telle qu'une protéine support choisie dans le groupe constitué par une toxine bactérienne, un toxoïde bactérien, une protéine de membrane externe de *N. meningitidis,* une protéine de choc thermique, une protéine de la coqueluche, la protéine D de *H. influenza,* une cytokine, une lymphokine, une hormone, un facteur de croissance, la toxine A de *C. difficile,* la toxine B de *C. difficile* et une protéine d'absorption de fer.

**11.** Composition selon l'une quelconque des revendications 1 à 10, qui comprend en outre un support pharmaceutiquement acceptable.

**12.** Composition selon l'une quelconque des revendications 1 à 11, comprenant en outre un agent actif qui est utile dans un vaccin pédiatrique, tel qu'un agent actif choisi dans le groupe constitué par :

(a) un antigène polypeptidique choisi dans le groupe constitué par *N. meningitidis, S. pneumoniae, Bordetella pertussis, Moraxella catarrhalis, Clostridium tetani, Chorinebacterim diphteriae,* le virus respiratoire syncytial, le virus de la polio, le virus de la rougeole, le virus des oreillons, le virus de la rubéole, et des antigènes polypeptidiques de rotavirus ; et
(b) une molécule d'acide nucléique qui code pour l'antigène polypeptidique.

**13.** Composition selon l'une quelconque des revendications 1 à 11, comprenant en outre un second agent actif qui est utile dans un vaccin pour des personnes âgées ou des individus immunocompromis, tel qu'un second agent actif choisi dans le groupe constitué par :

(a) un antigène polypeptidique choisi dans le groupe constitué par *Enterococcus faecalis, Staphylococcus aureus, Staphylococcus epidermis, Pseudomonas aeruginosa, Legionella pneumophila, Listeria monocytoge-*

*nes,* le virus de la grippe et des antigènes polypeptidiques d'un virus parainfluenza ; et
(b) une molécule d'acide nucléique qui code pour l'antigène polypeptidique.

**14.** Composition selon l'une quelconque des revendications 1 à 13, comprenant en outre un polysaccharide du groupe A de formule

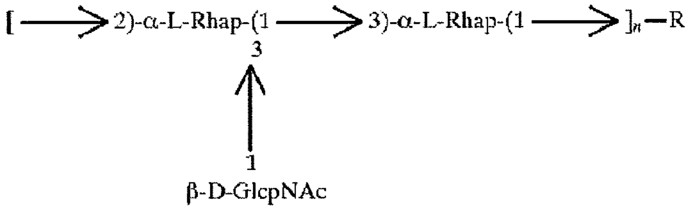

(I),

dans laquelle R est une L-rhamnose réductrice d'extrémité terminale ou D-GlcpNAc et n est un nombre d'environ 3 à environ 30.

**15.** Composition selon la revendication 14, dans laquelle ladite composition comprend Spy0167 mutant P427L/W535F, Spy0269, Spy0416 mutant D151A/S617A et un polysaccharide du groupe A de formule

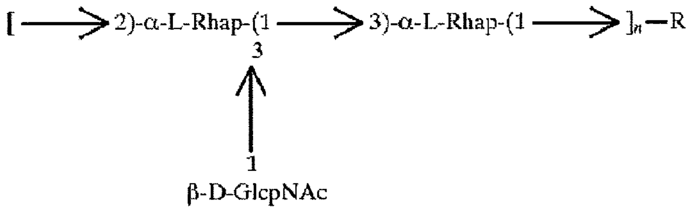

(I),

dans laquelle R est une L-rhamnose réductrice d'extrémité terminale ou D-GlcpNAc et n est un nombre d'environ 3 à environ 30, et dans laquelle l'antigène polysaccharidique du groupe A est conjugué à la toxine diphtérique mutée CRM197.

**16.** Composition selon l'une quelconque des revendications 1 à 14, comprenant en outre un adjuvant, tel que l'alun.

**17.** Composition selon l'une quelconque des revendications 1 à 16, pour une utilisation en tant que vaccin.

**18.** Composition selon l'une quelconque des revendications 1 à 16, pour une utilisation dans le traitement d'une infection à *Streptococcus pyogenes.*

FIG. 1

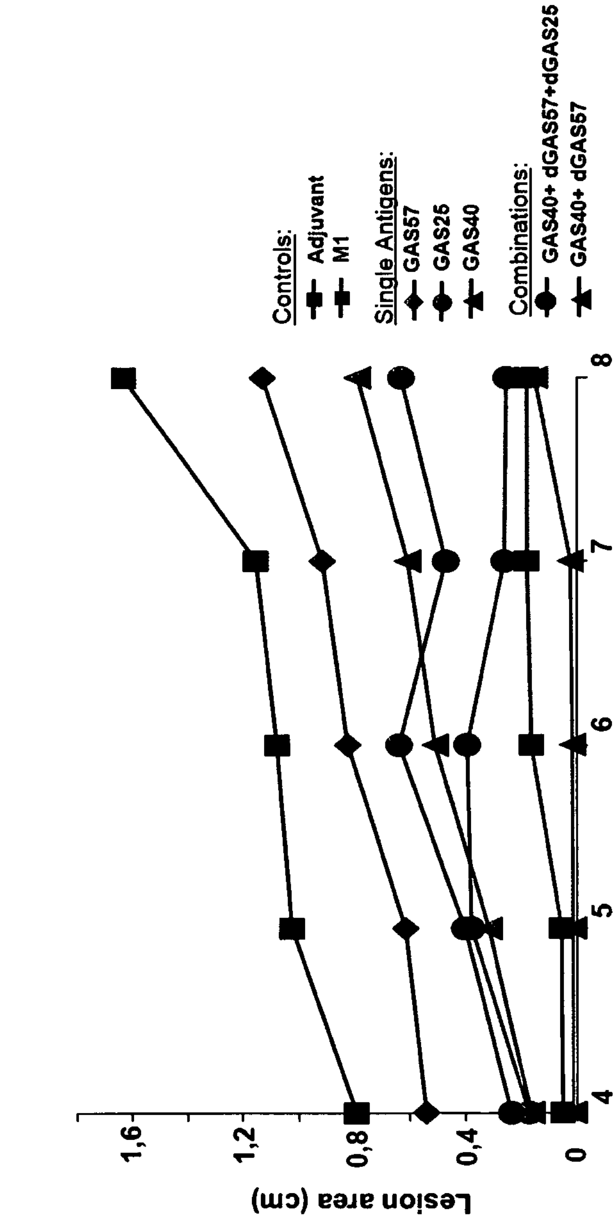

Lesion area (cm)
1,6
1,2
0,8
0,4
0
4
5
6
7
8
Days post infection
Controls:
Adjuvant
M1
Single Antigens:
GAS57
GAS25
GAS40
Combinations:
GAS40+ dGAS57+dGAS25
GAS40+ dGAS57

FIG. 2

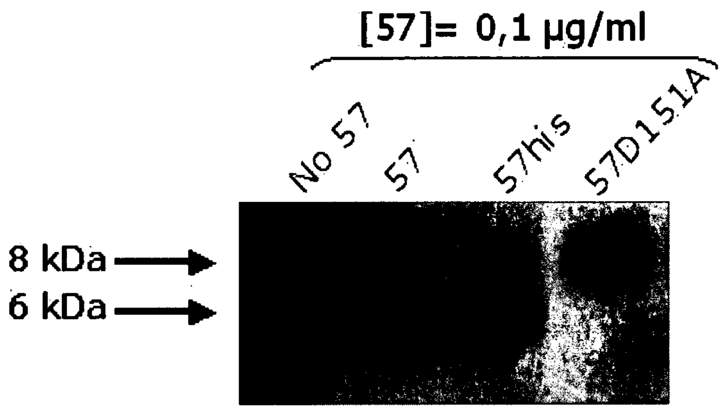

FIG. 3

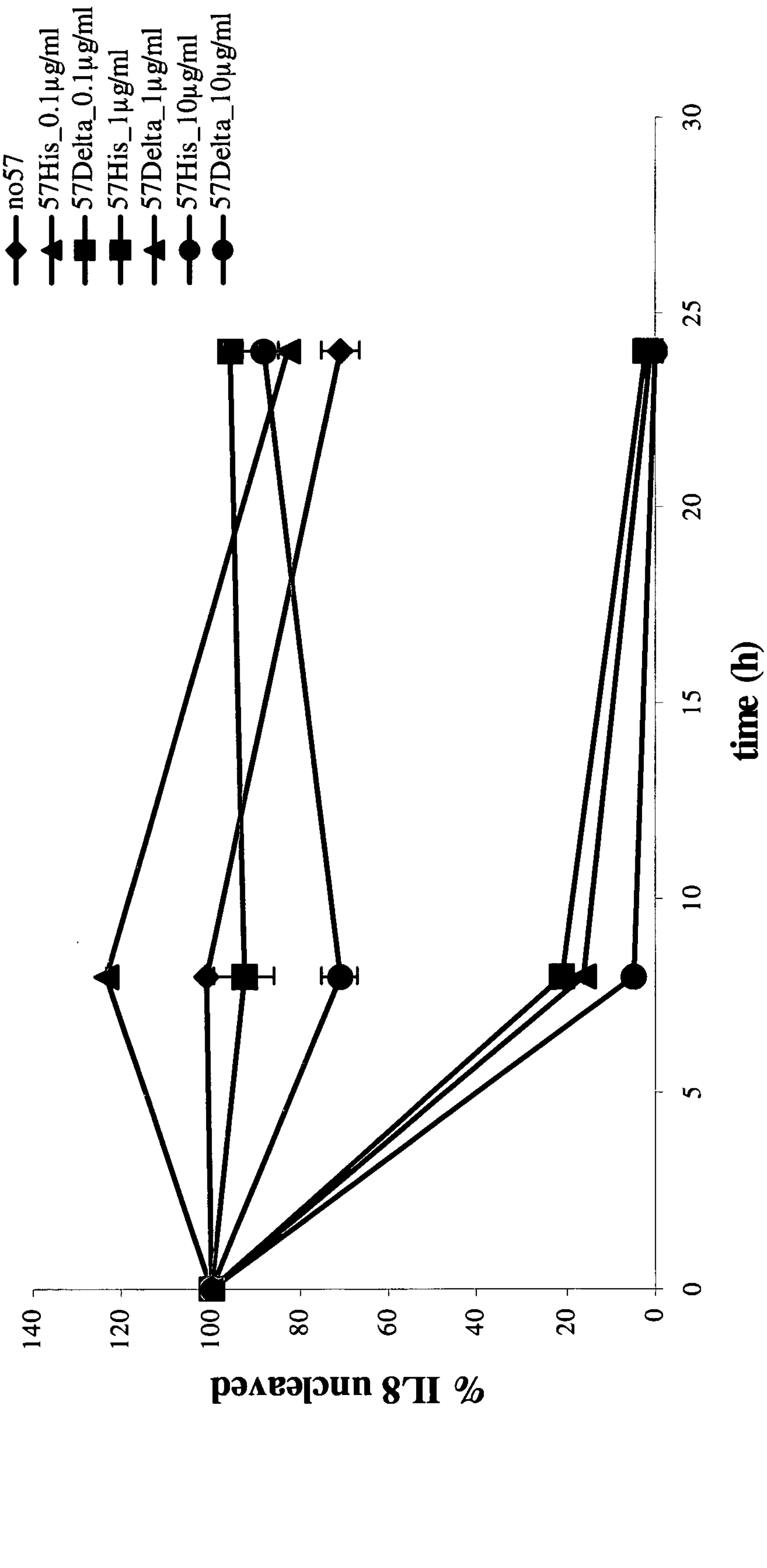

% IL8 uncleaved
140
120
100
80
60
40
20
0
0
5
10
15
20
25
30
time (h)
no57
57His_0.1µg/ml
57Delta_0.1µg/ml
57His_1µg/ml
57Delta_1µg/ml
57His_10µg/ml
57Delta_10µg/ml

# FIG. 4

## A

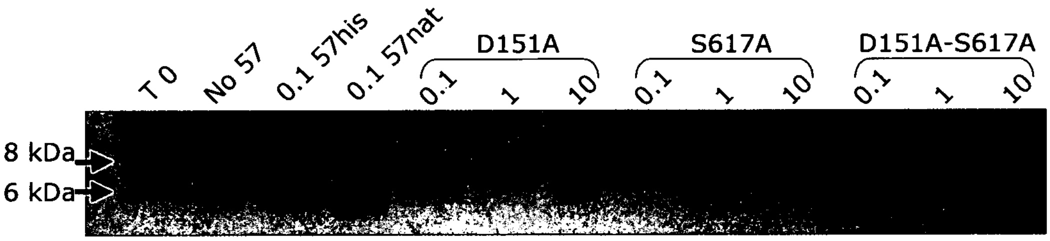
T 0
No 57
0.1 57his
0.1 57nat
D151A
S617A
D151A-S617A
0.1
1
10
0.1
1
10
0.1
1
10
8 kDa
6 kDa

## B

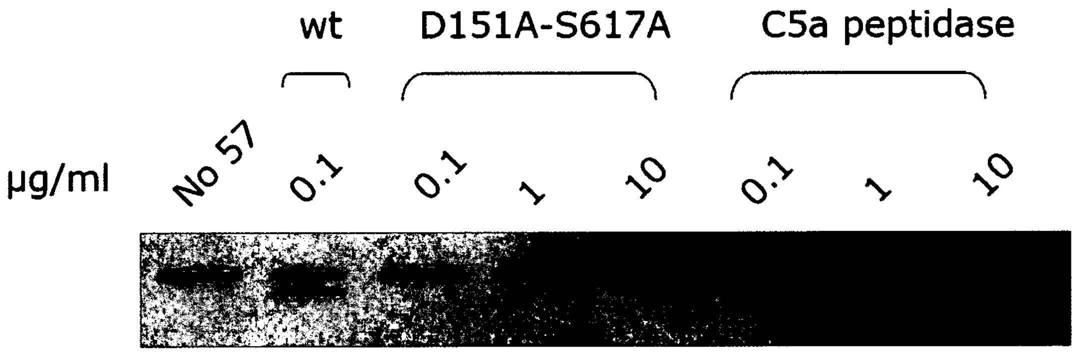
wt
D151A-S617A
C5a peptidase
μg/ml
No 57
0.1
0.1
1
10
0.1
1
10

# FIG. 5

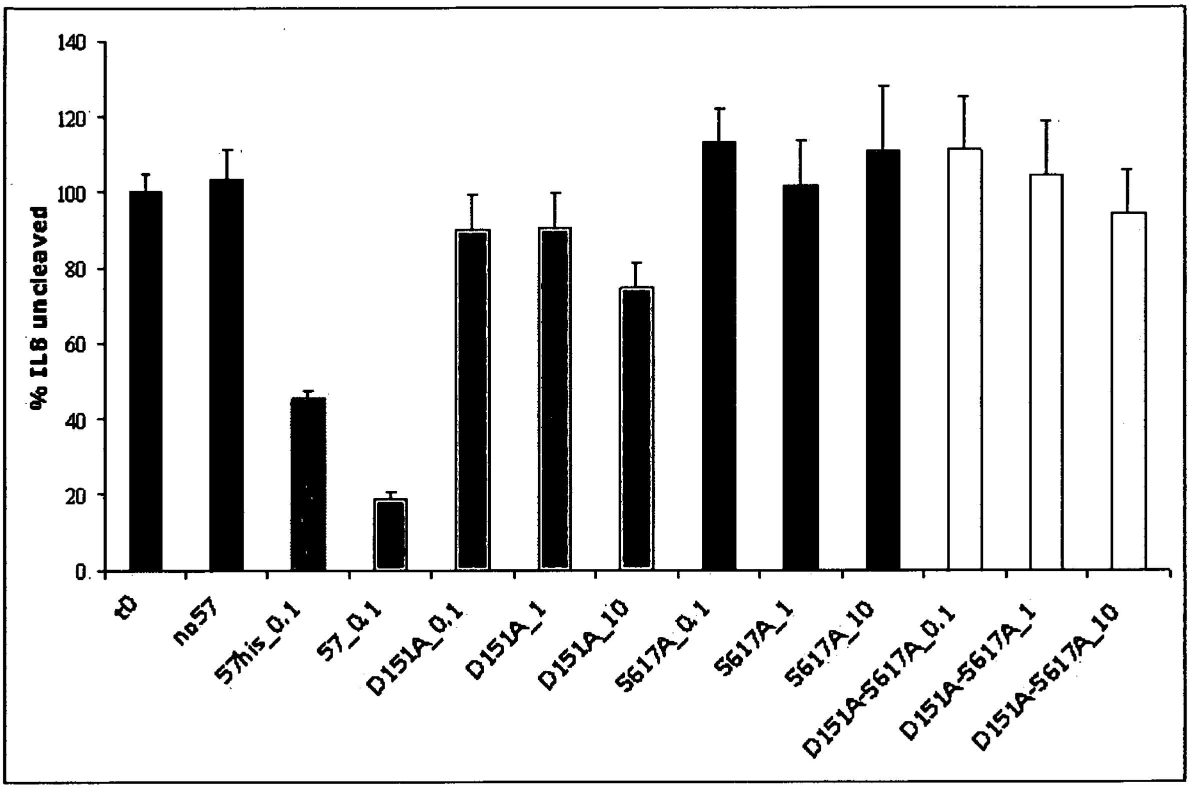
% IL8 uncleaved
0
20
40
60
80
100
120
140
t0
no57
57his_0.1
57_0.1
D151A_0.1
D151A_1
D151A_10
S617A_0.1
S617A_1
S617A_10
D151A-S617A_0.1
D151A-S617A_1
D151A-S617A_10

**FIG. 6**

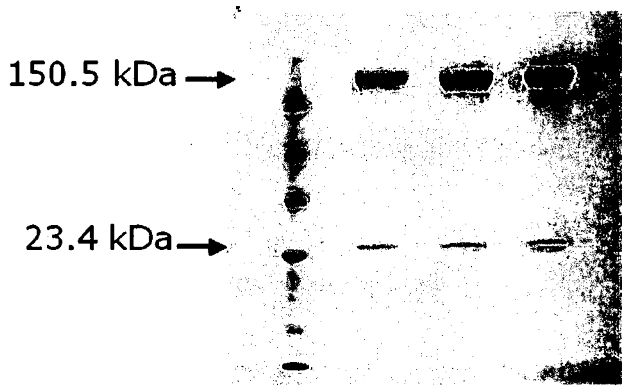

FIG. 7

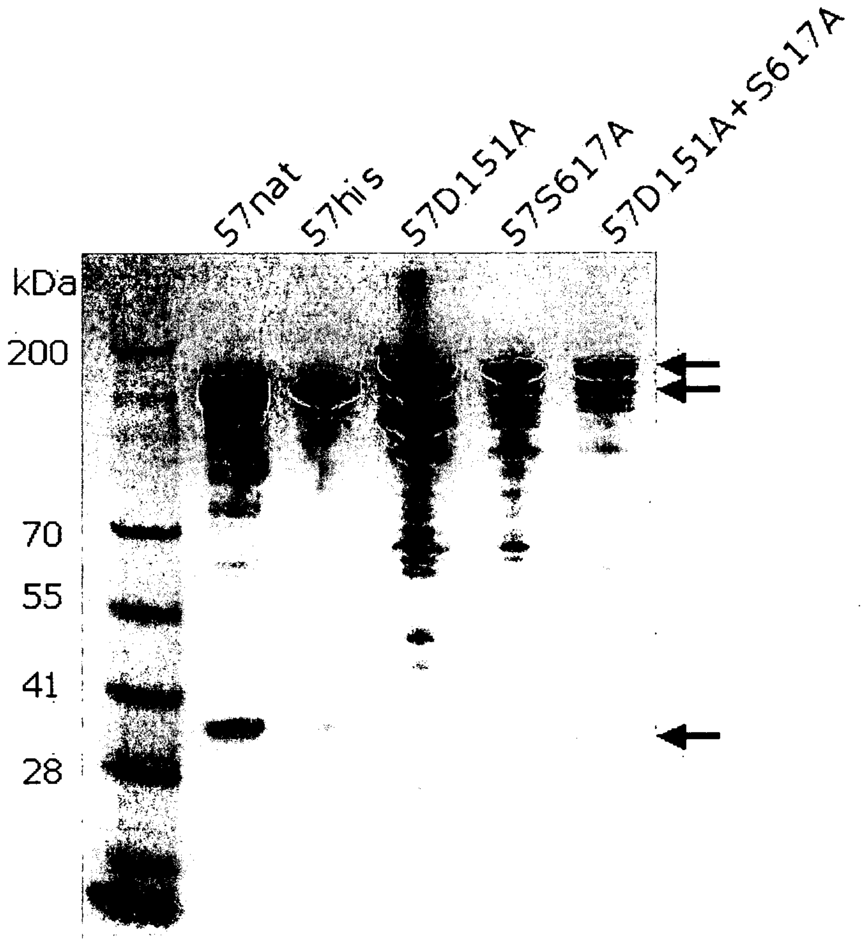


SDS-PAGE 4-12%

FIG. 8

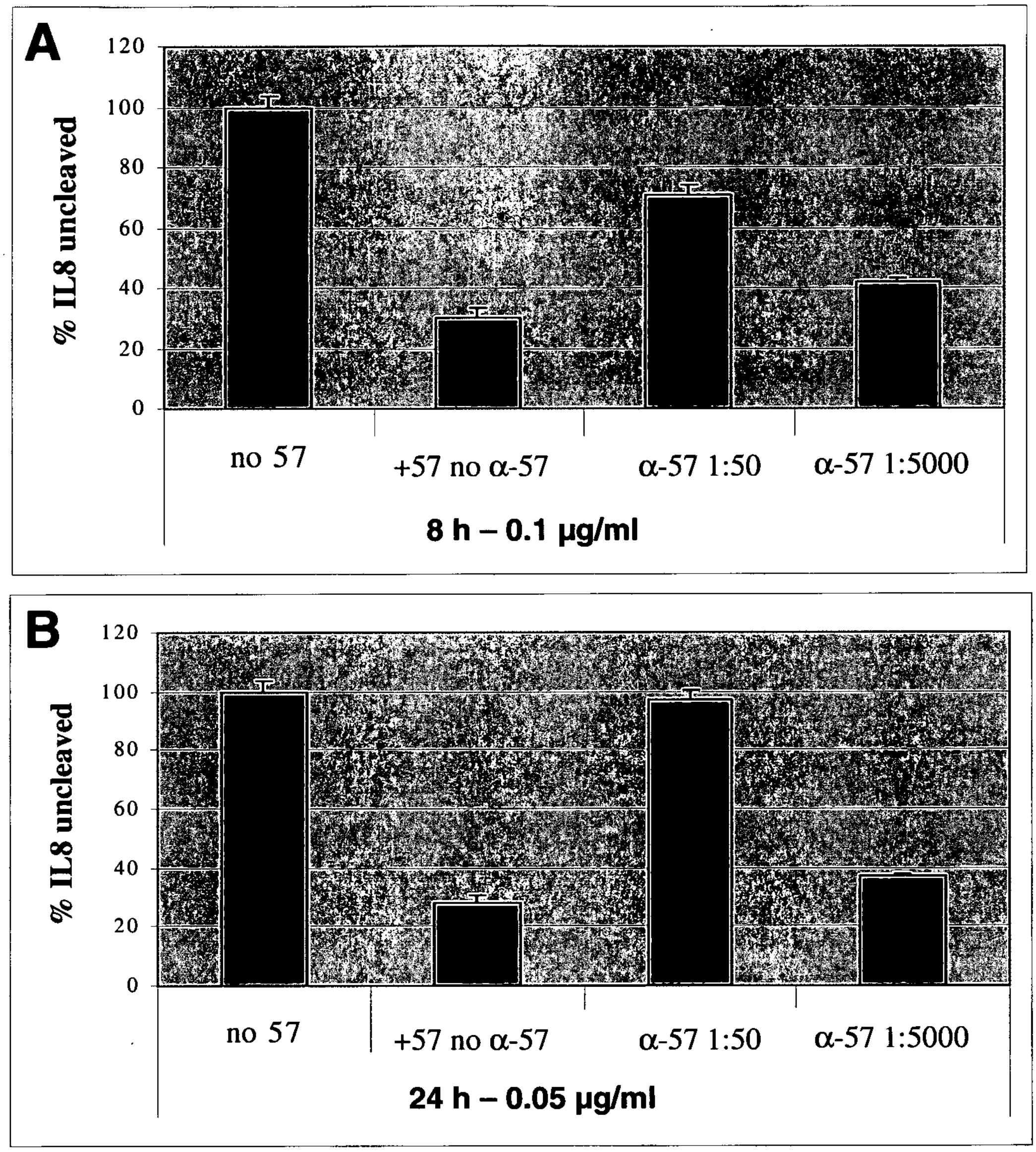
A
120
100
80
60
40
20
0
% IL8 uncleaved
no 57
+57 no α-57
α-57 1:50
α-57 1:5000
8 h – 0.1 μg/ml
B
120
100
80
60
40
20
0
% IL8 uncleaved
no 57
+57 no α-57
α-57 1:50
α-57 1:5000
24 h – 0.05 μg/ml

**FIG. 9**

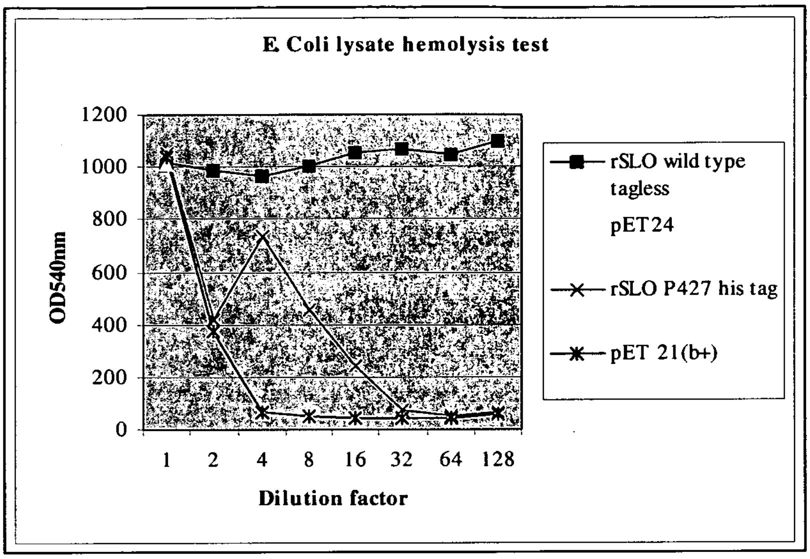


**FIG. 10**

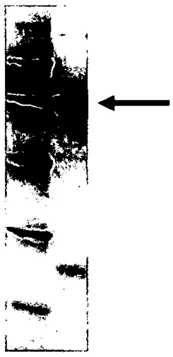

**FIG. 11**

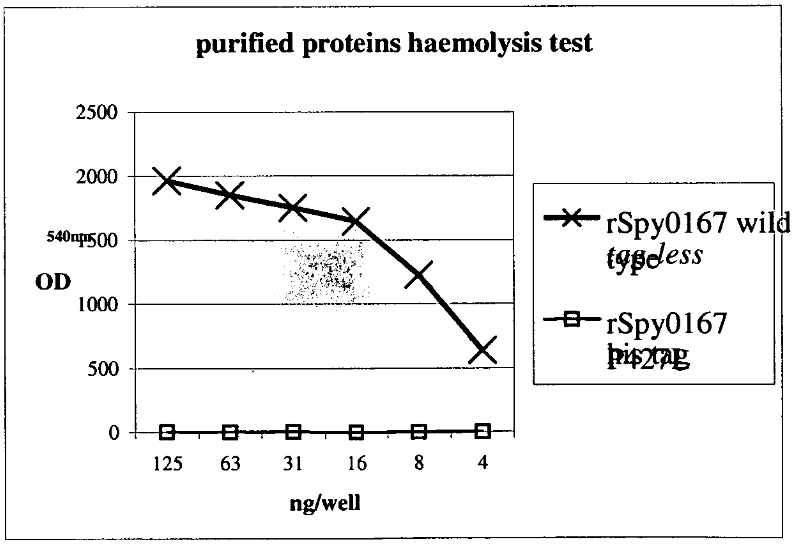

FIG. 10

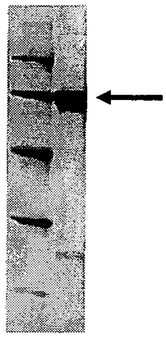

FIG. 11

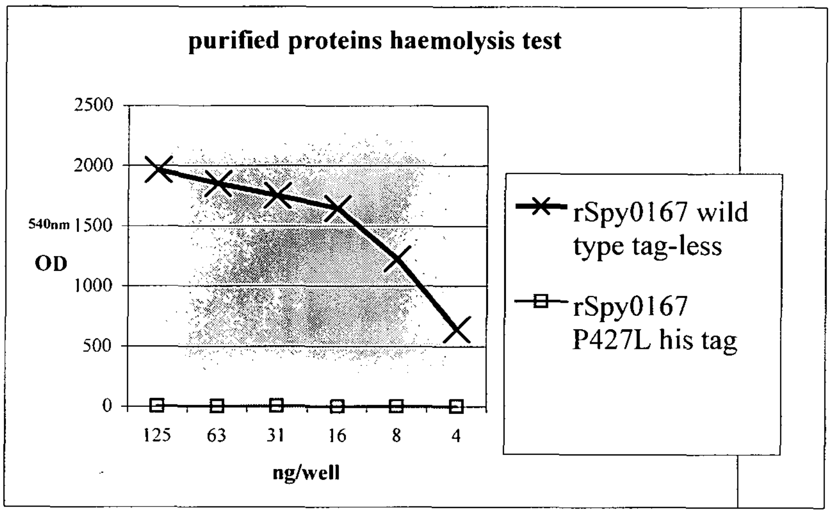
purified proteins haemolysis test
2500
2000
540nm 1500
OD
1000
500
0
125
63
31
16
8
4
ng/well
rSpy0167 wild type tag-less
rSpy0167 P427L his tag

FIG. 12

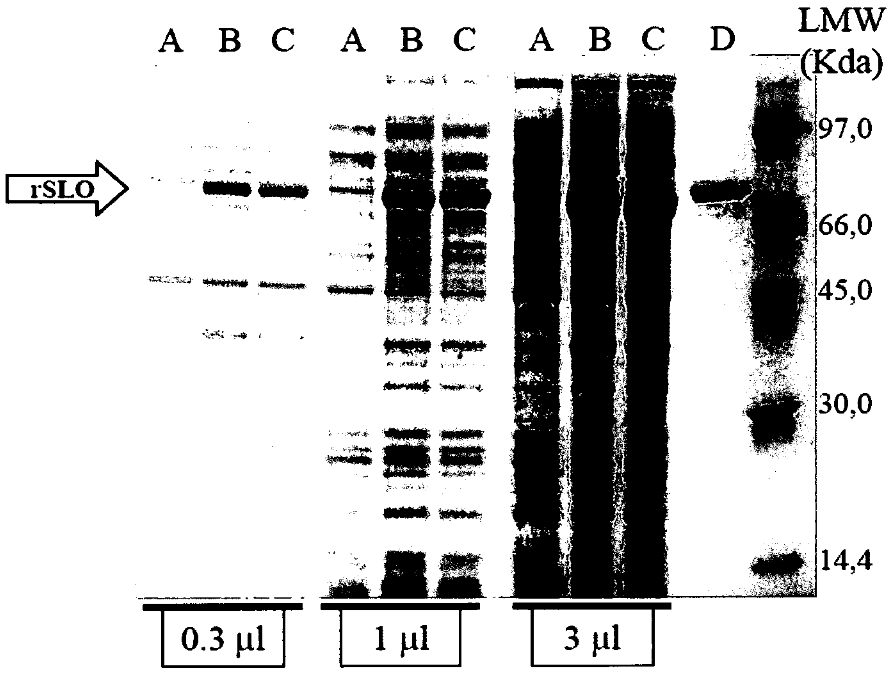

FIG. 13

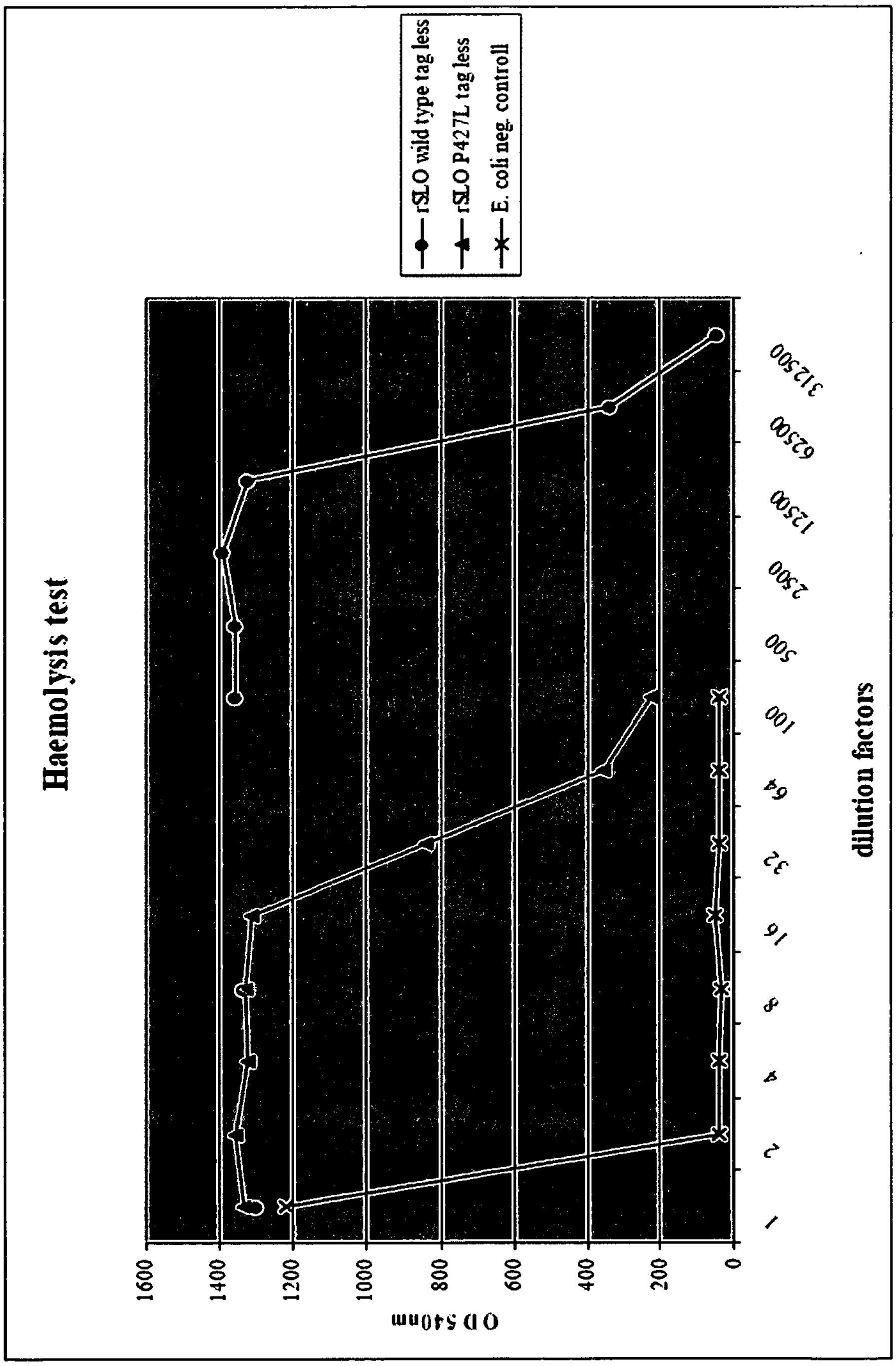
Haemolysis test
rSLO wild type tag less
rSLO P427L tag less
E. coli neg. controll
O D 540nm
0
200
400
600
800
1000
1200
1400
1600
1
2
4
8
16
32
64
100
500
2500
12500
62500
312500
dilution factors

FIG. 14

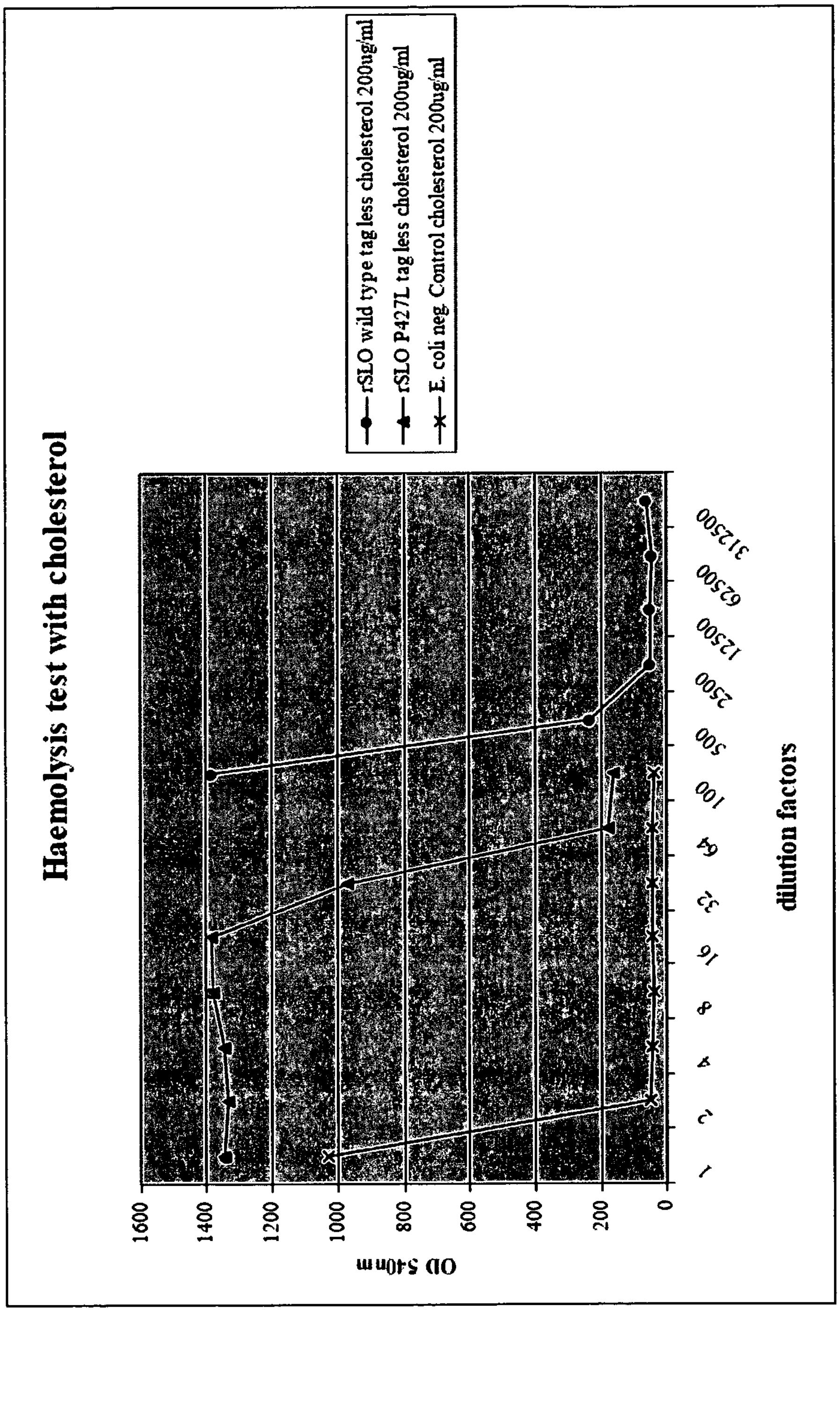
Haemolysis test with cholesterol
OD 540nm
1600
1400
1200
1000
800
600
400
200
0
1
2
4
8
16
32
64
100
500
2500
12500
62500
312500
dilution factors
rSLO wild type tag less cholesterol 200ug/ml
rSLO P427L tag less cholesterol 200ug/ml
E. coli neg. Control cholesterol 200ug/ml

FIG. 15A

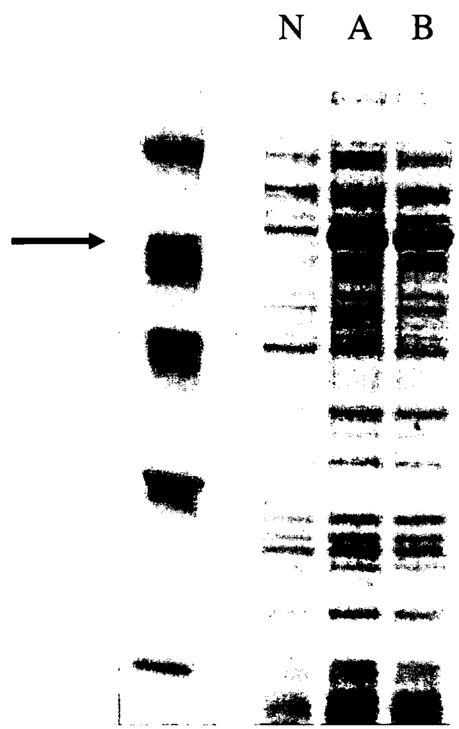


**SDS Page analysis of total proteins cell extracts**

N. Non induced culture
A. Spy0167 Wild Type *tag-less* clone
B. Spy0167 P427L *tag-less* clone
Molecular weight markers (97-66-45-30-20.1)

**Black arrow indicates Spy0167 protein band in extracts from mutant and wild type clones**

# FIG. 15B

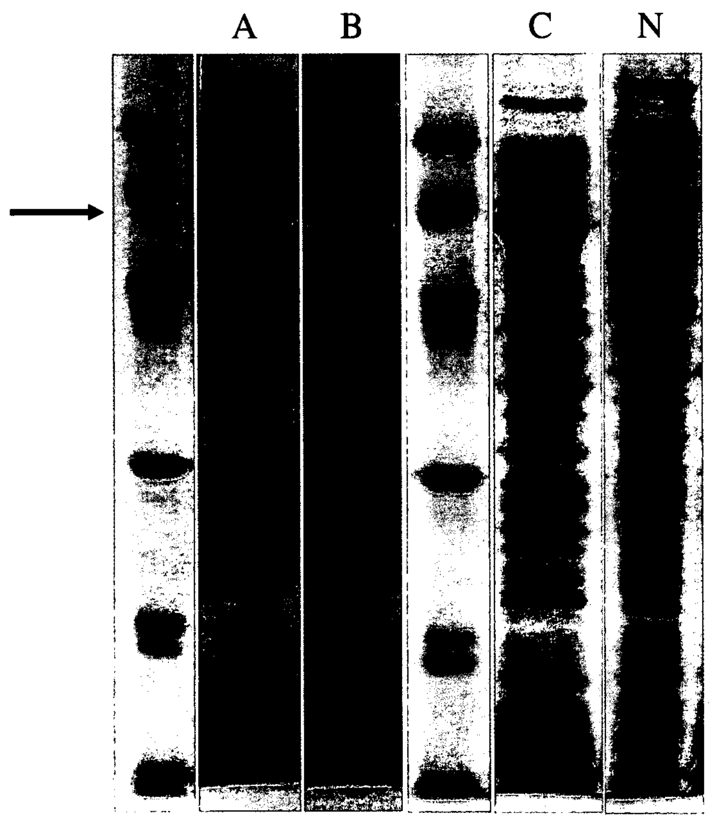


**SDS Page analysis of total proteins extracts**

A. Spy0167 P427L-W535F *tag-less* clone
B. Spy0167 P427L-C530G *tag-less* clone
C. Spy0167 P427L-C530G-W535F *tag-less* clone
N. Non induced culture
Molecular weight markers (97-66-45-30-20.1-14,4)
**Black arrow indicates Spy0167 protein band in extracts from mutants clones**

FIG. 16

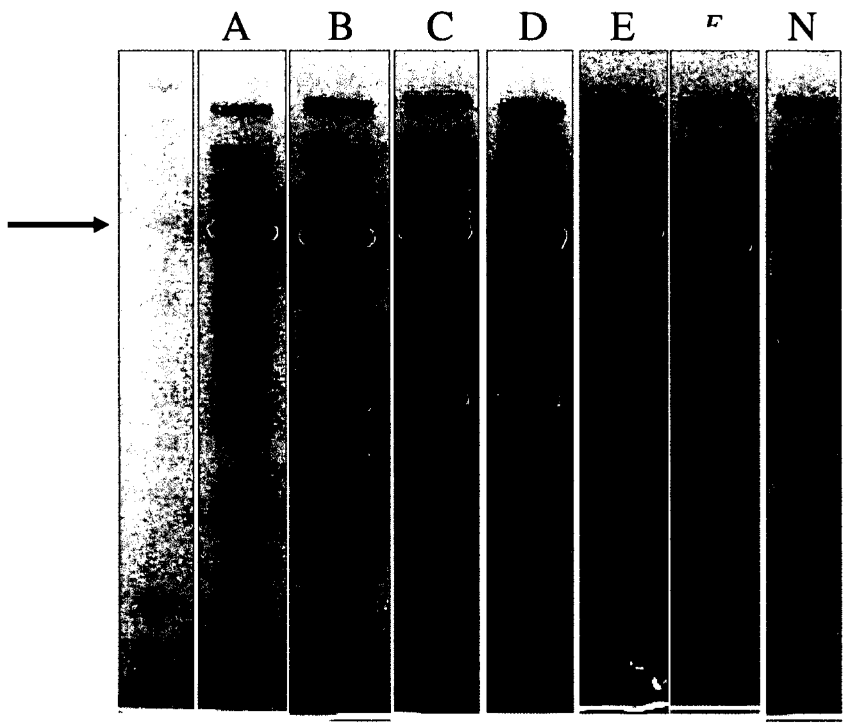


**SDS Page analysis of total proteins extracts**

A. Spy0167 Wild Type *his-tagged* clone
B. Spy0167 W535F *his-tagged* clone
C. Spy0167 W535F-D482N *his-tagged* clone
D. Spy0167 C530G *his-tagged* clone
E. Spy0167 P427L *his-tagged* clone
F. Spy0167 delta Ala 248 *his-tagged* clone
N. Non induced culture
Molecular weight markers (97-66-45-30-20.1-14,4)
**Black arrow indicates Spy0167 protein band in extracts from mutants and wild type clones**

FIG. 17

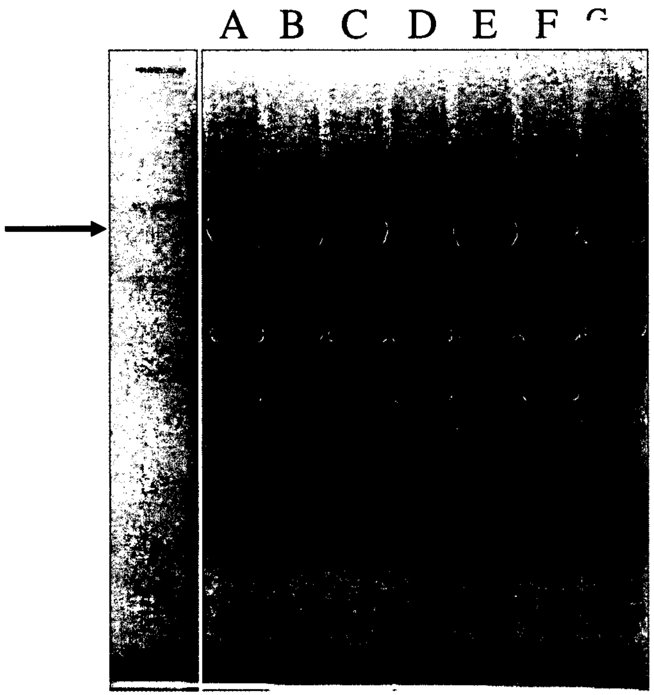


**SDS Page analysis of purified proteins**

A. rSpy0167 wild type his tagged (5μg);
B. rSpy0167 mutant C530G his tagged (5μg);
C. rSpy0167 mutant W535F his tagged (5μg);
D. rSpy0167 mutant W535F and D482N his tagged (5μg);
E. rSpy0167 mutant P427L his tagged (5μg);
F. rSpy0167 mutant delta Ala248 his tagged (5μg);
G. *E. coli* contaminants as negative control (5μg);

Molecular weight markers (97-66-45-30-20.1) **black arrow indicates Spy0167 protein purified from mutants and wild type clones**

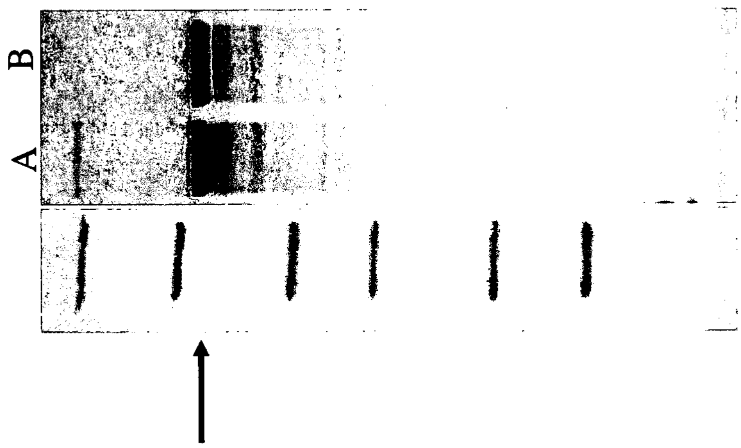


FIG. 18A

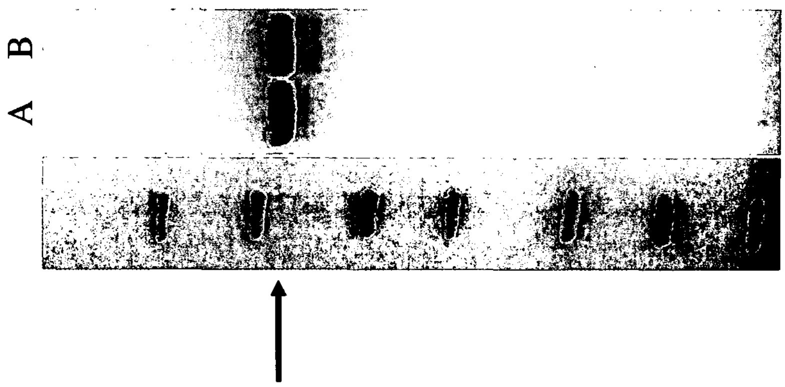


FIG. 18B

FIG. 19

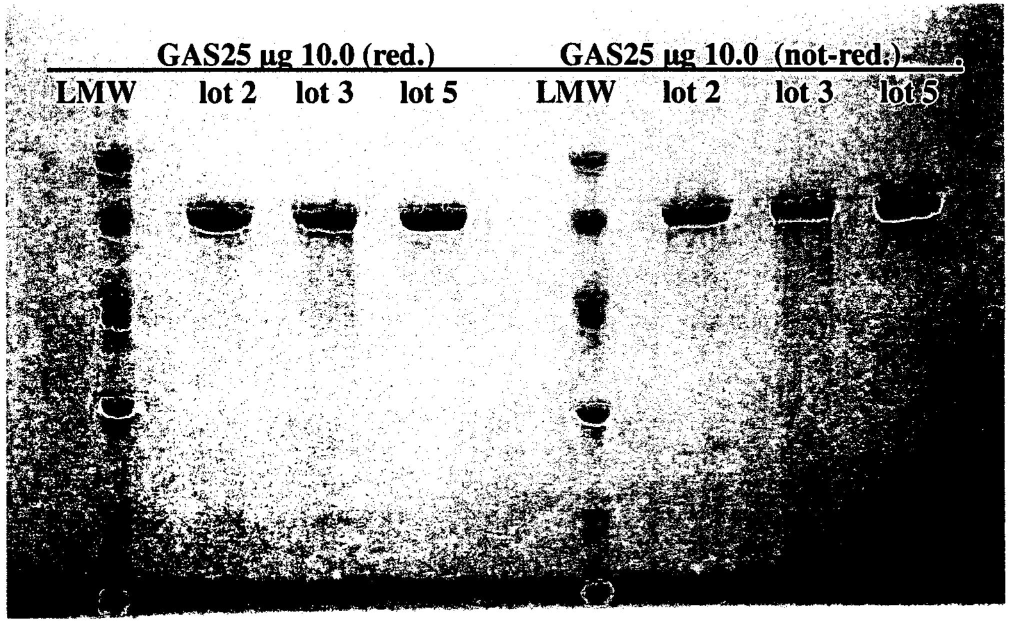
GAS25 µg 10.0 (red.)
GAS25 µg 10.0 (not-red.)
LMW
lot 2
lot 3
lot 5
LMW
lot 2
lot 3
lot 5

FIG. 20

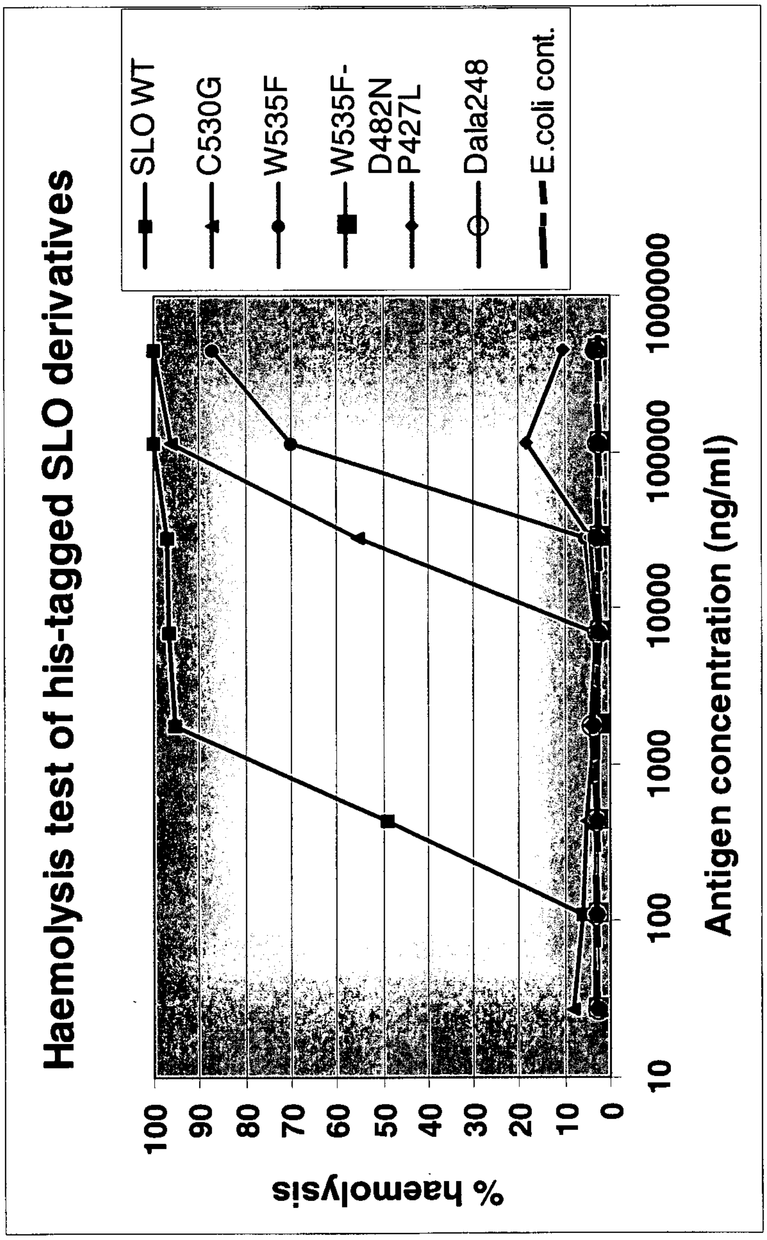
Haemolysis test of his-tagged SLO derivatives
% haemolysis
100
90
80
70
60
50
40
30
20
10
0
10
100
1000
10000
100000
1000000
Antigen concentration (ng/ml)
SLO WT
C530G
W535F
W535F-D482N
P427L
Dala248
E.coli cont.

FIG. 21

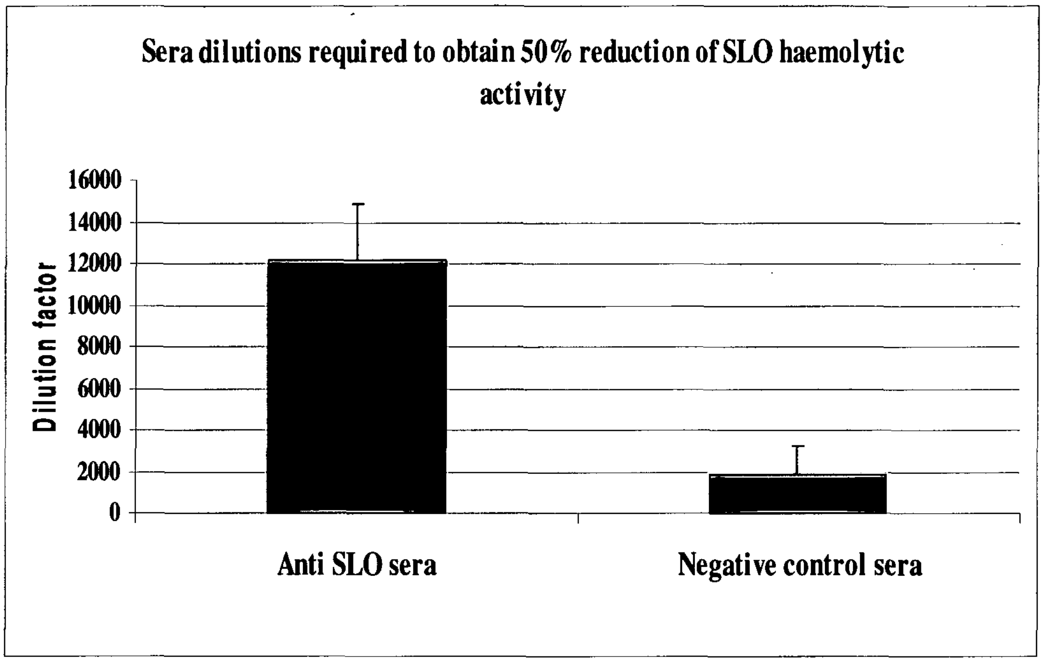
Sera dilutions required to obtain 50% reduction of SLO haemolytic activity
Dilution factor
16000
14000
12000
10000
8000
6000
4000
2000
0
Anti SLO sera
Negative control sera

FIG. 22

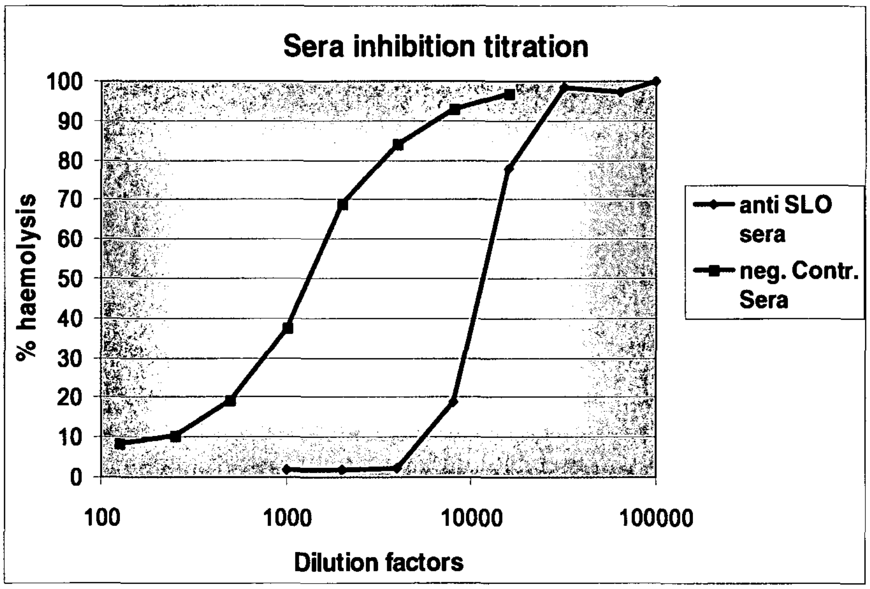
Sera inhibition titration
% haemolysis
100
90
80
70
60
50
40
30
20
10
0
100
1000
10000
100000
Dilution factors
anti SLO sera
neg. Contr. Sera

# FIG. 23

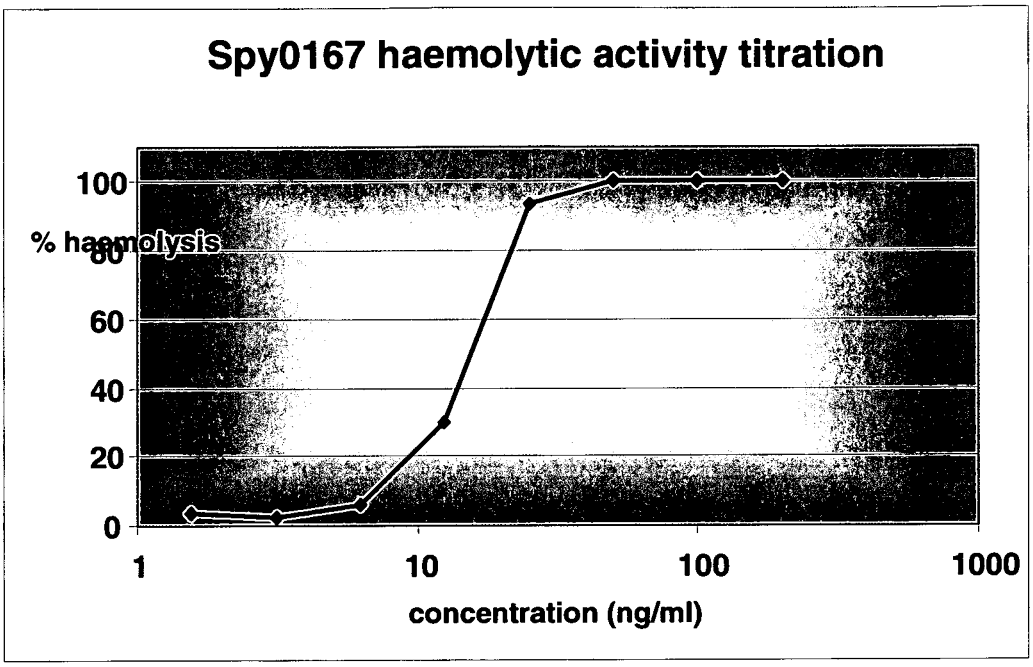
Spy0167 haemolytic activity titration
% haemolysis
100
80
60
40
20
0
1
10
100
1000
concentration (ng/ml)

# FIG. 24

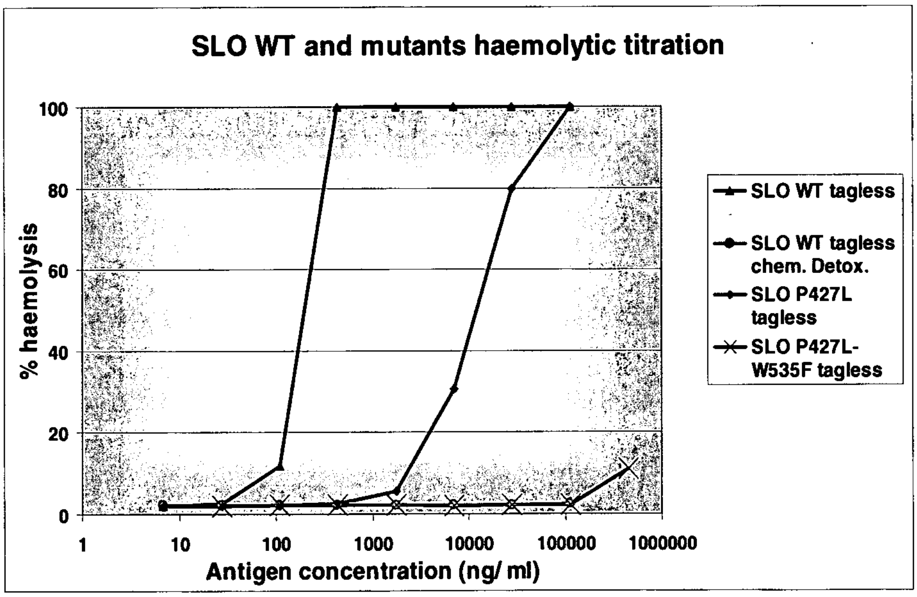
SLO WT and mutants haemolytic titration
% haemolysis
100
80
60
40
20
0
1
10
100
1000
10000
100000
1000000
Antigen concentration (ng/ ml)
SLO WT tagless
SLO WT tagless chem. Detox.
SLO P427L tagless
SLO P427L-W535F tagless

FIG. 25

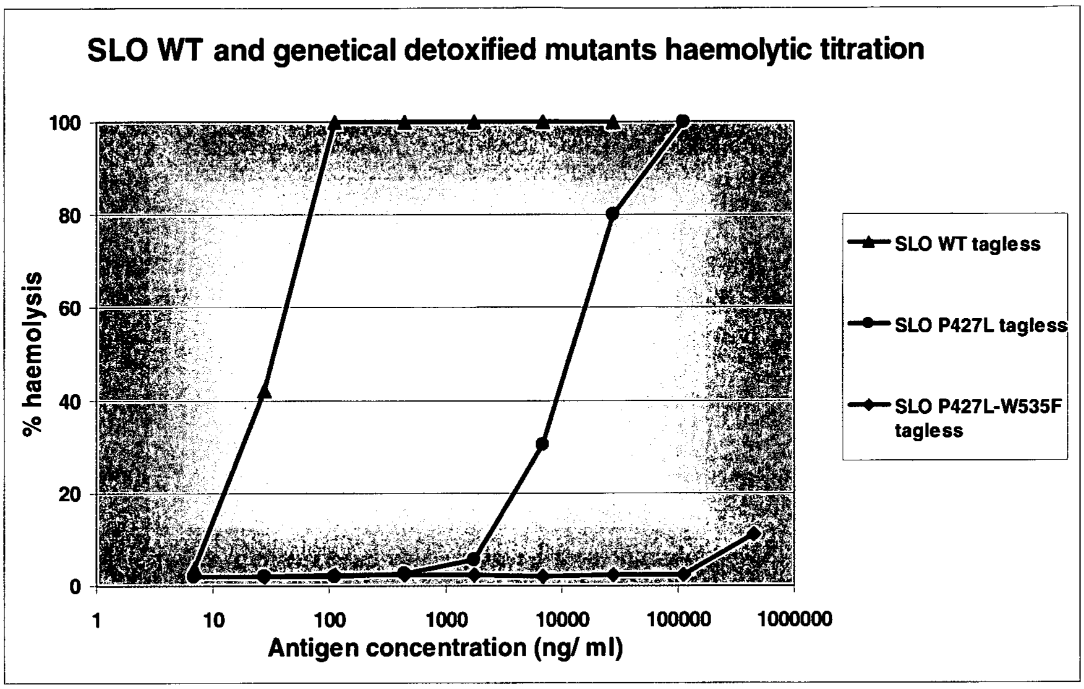
SLO WT and genetical detoxified mutants haemolytic titration
100
80
60
40
20
0
% haemolysis
1
10
100
1000
10000
100000
1000000
Antigen concentration (ng/ ml)
SLO WT tagless
SLO P427L tagless
SLO P427L-W535F tagless

FIG. 26

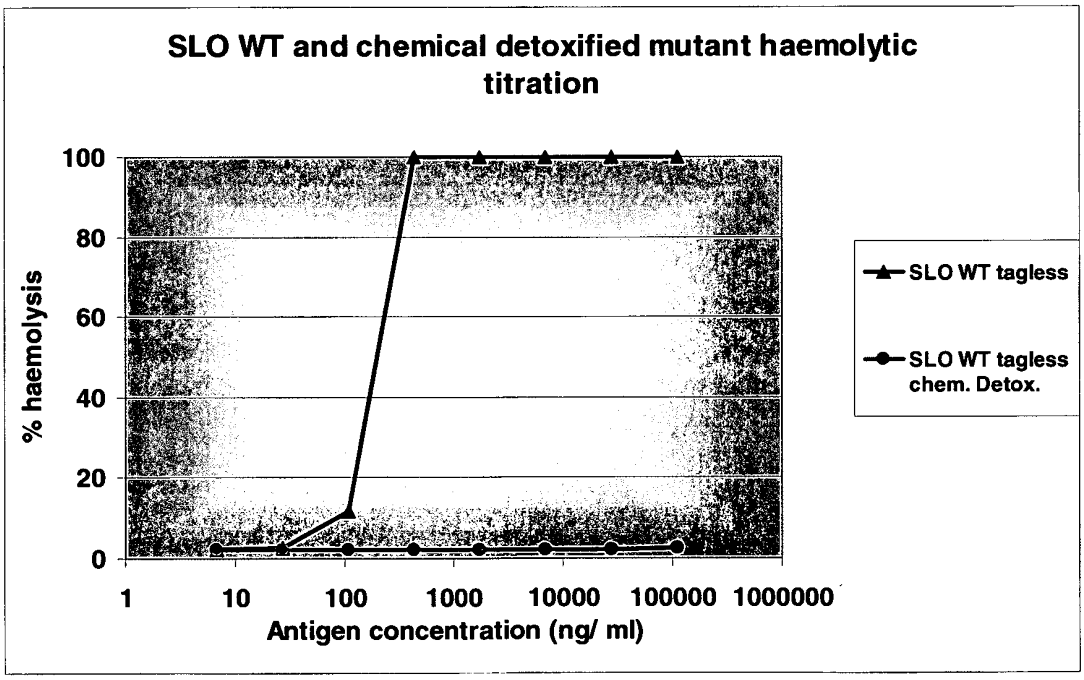
SLO WT and chemical detoxified mutant haemolytic titration
100
80
60
40
20
0
% haemolysis
1
10
100
1000
10000
100000
1000000
Antigen concentration (ng/ ml)
SLO WT tagless
SLO WT tagless chem. Detox.

FIG. 27

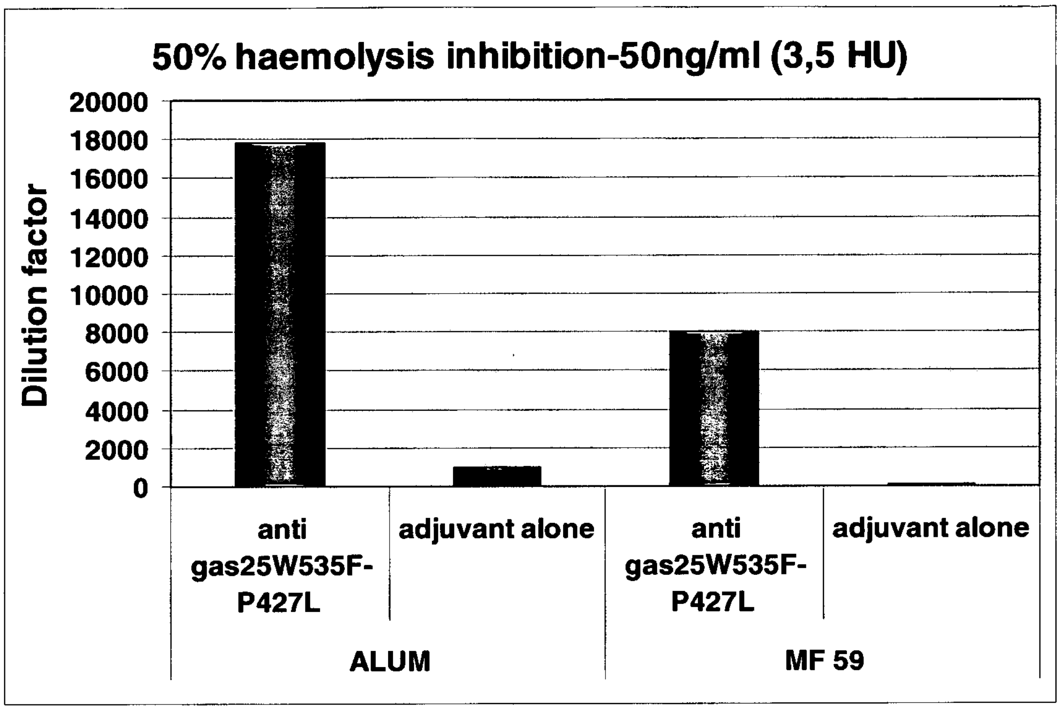
50% haemolysis inhibition-50ng/ml (3,5 HU)
Dilution factor
20000
18000
16000
14000
12000
10000
8000
6000
4000
2000
0
anti gas25W535F-P427L
adjuvant alone
anti gas25W535F-P427L
adjuvant alone
ALUM
MF 59

FIG. 28

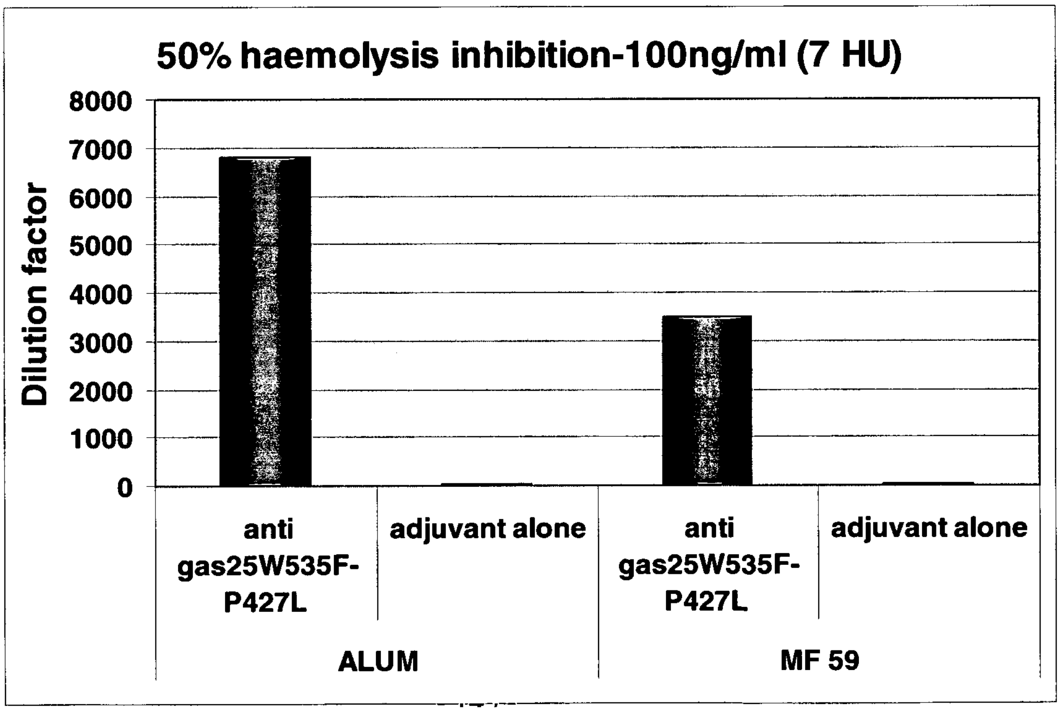
50% haemolysis inhibition-100ng/ml (7 HU)
Dilution factor
8000
7000
6000
5000
4000
3000
2000
1000
0
anti gas25W535F-P427L
adjuvant alone
anti gas25W535F-P427L
adjuvant alone
ALUM
MF 59

FIG. 29

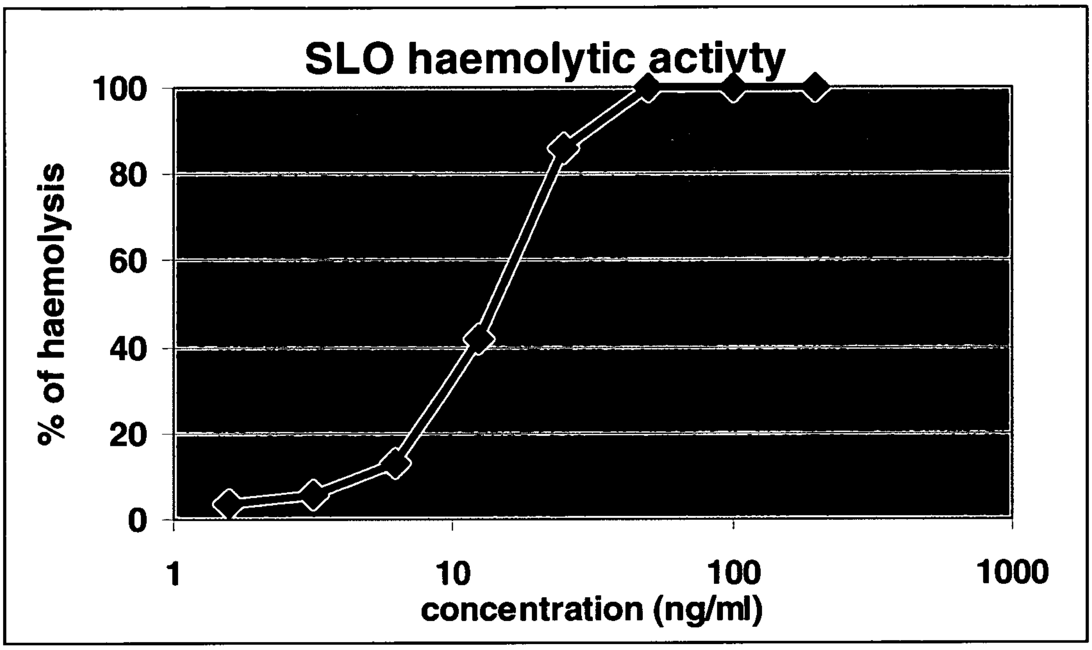
SLO haemolytic activty
% of haemolysis
100
80
60
40
20
0
1
10
100
1000
concentration (ng/ml)

FIG. 30

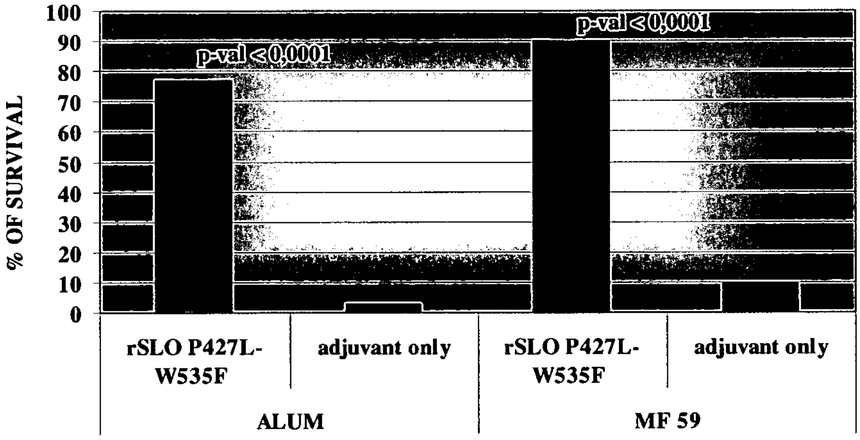
% OF SURVIVAL
100
90
80
70
60
50
40
30
20
10
0
p-val < 0,0001
p-val < 0,0001
rSLO P427L-W535F
adjuvant only
rSLO P427L-W535F
adjuvant only
ALUM
MF 59

FIG. 31A

```
                          1                                                50
gas57M1_SF370       (1) VEKKQRFSLRKYKSGTFSVLIGSVFLVMTTT--VAADELSTMSEPTITNH
gas57M1_31075       (1) VEKKQRFSLRKYKSGTFSVLIGSVFLVMTTT--VAADELSTMSEPTITNH
gas57M1_31237       (1) VEKKQRFSLRKYKSGTFSVLIGSVFLVMTTT--VAADELSTMSEPTITNH
gas57M1_3348        (1) VEKKQRFSLRKYKSGTFSVLIGSVFLVMTTT--VAADELSTMSEPTITNH
gas57M2_34585       (1) VEKKQRFSLRKYKSGTFSVLIGSVFLVMTTT--VAADELSTMSEPTITNH
gas57M3,1_21398     (1) VEKKQRFSLRKYKSGTFSVLIGSVFLVMTTT--VAANELSTMSEPTITNH
gas57M44-61_20839   (1) VEKKQRFSLRKYKSGTFSVLIGSVFLVMTTT--VAADELSTMSEPTITNH
gas57M6,31_20022    (1) VEKKQRFSLRKYKSGTFSVLIGSAFLMMTTT--VAADELSTMSEPTITNH
gas57M11_20648      (1) VEKKQRFSLRKYKSGTFSVLIGSVFLVMTTT--VAADELSTMSEPTITNH
gas57M23_2071       (1) VEKKQRFSLRKYKSGTFSVLIGSVFLMMTTT--VAADELSTMSEPTITNH
gas57M18,3_40128    (1) VEKKQRFSLRKYKSGTFSVLIGSVFLMMMTTTTVAADELTTTSEPTITNH
gas57M4_10092       (1) VEKKQRFSLRKYKSGTFSVLIGSVFLMMMTTTTVAADELTTTSEPTITNH
gas57M4_30968       (1) VEKKQRFSLRKYKSGTFSVLIGSVFLMMMTTTTVAADELTTTSEPTITNH
gas57M6,31_22692    (1) VEKKQRFSLRKYKSGTFSVLIGSVFLMMMTTTTVAADELTTTSEPTITNH
gas57M68,5_22814    (1) VEKKQRFSLRKYKSGTFSVLIGSVFLMMMTTT-VAADELTTTSEPTITNH
gas57M68_23623      (1) VEKKQRFSLRKYKSGTFSVLIGSVFLMMMMTTTVAADELTTTSEPTITNH
gas57M2_10064       (1) VEKKQRFSLRKYKSGTFSVLIGSVFLMMTTT--VAADELTTTSEPTITNH
gas57M2_10065       (1) VEKKQRFSLRKYKSGTFSVLIGSVFLMMTTT--VAADELTTTSEPTITNH
gas57M77_10251      (1) VEKKQRFSLRKYKSGTFSVLIGSVFLMMTTT--VAADELTTTSEPTITNH
gas57M77_10527      (1) VEKKQRFSLRKYKSGTFSVLIGSVFLMMTTT--VAADELTTTSEPTITNH
gas57M77_20696      (1) VEKKQRFSLRKYKSGTFSVLIGSVFLMMTTT--VAADELTTTSEPTITNH
gas57M89_21915      (1) VEKKQRFSLRKYKSGTFSVLVGSVFLMMTTT--VAADELTTTSEPTITNH
gas57M89_23717      (1) VEKKQRFSLRKYKSGTFSVLVGSVFLMMTTT--VAADELTTTSEPTITNH
gas57M94_10134      (1) VEKKQRFSLRKYKSGTFSVLIGSVFLMMTTT--VAADELTTTSEPTITNH
gas57M28_10164      (1) VEKKQRFSLRKYKSGTFSVLIGSVFLMMTTT--VAADELTTTSEPTITNH
gas57M28_10218      (1) VEKKQRFSLRKYKSGTFSVLIGSVFLMMTTT--VAADELTTTSEPTITNH
gas57M28_10266      (1) VEKKQRFSLRKYKSGTFSVLIGSVFLMMTTT--VAADELTTTSEPTITNH
gas57M28_10299      (1) VEKKQRFSLRKYKSGTFSVLIGSVFLMMTTT--VAADELTTTSEPTITNH
gas57M28_30176      (1) VEKKQRFSLRKYKSGTFSVLIGSVFLMMTTT--VAADELTTTSEPTITNH
gas57M28_30574      (1) VEKKQRFSLRKYKSGTFSVLIGSVFLMMTTT--VAADELTTTSEPTITNH
gas57M6,9_21802     (1) VEKKQRFSLRKYKSGTFSVLIGSVFLMMMTTT-VAADELTTTSEPTITNH
gas57M75_10012      (1) VEKKQRFSLRKYKSGTFSVLIGSVFLMMMMTTTVAADELTTTSEPTITNH
gas57M75_20671      (1) VEKKQRFSLRKYKSGTFSVLIGSVFLMMMMTTTVAADELTTTSEPTITNH
gas57M75_30603      (1) VEKKQRFSLRKYKSGTFSVLIGSVFLMMMMTTTVAADELTTTSEPTITNH
gas57M75_30207      (1) VEKKQRFSLRKYKSGTFSVLIGSVFLMMMMTTTVAADELTTTSEPTITNH
gas57M22_20641      (1) VEKKQRFSLRKYKSGTFSVLIGSVFLMMMTTT-VAADELTTTSEPTITNH
gas57M22_23465      (1) VEKKQRFSLRKYKSGTFSVLIGSVFLMMMTTT-VAADELTTTSEPTITNH
gas57M3,1_30610     (1) VEKKQRFSLRKYKSGTFSVLIGSVFLMMMTTT-VAADELTTTSEPTITNH
gas57M3,1_40603     (1) VEKKQRFSLRKYKSGTFSVLIGSVFLMMMTTT-VAADELTTTSEPTITNH
gas57M3,28_24214    (1) VEKKQRFSLRKYKSGTFSVLIGSVFLMMMTTT-VAADELTTTSEPTITNH
gas57M3,34_10307    (1) VEKKQRFSLRKYKSGTFSVLIGSVFLMMMTTT-VAADELTTTSEPTITNH
gas57M4_40427       (1) VEKKQRFSLRKYKSGTFSVLIGSVFLMMMTTT-VAADELTTTSEPTITNH
gas57M3_2721        (1) VEKKQRFSLRKYKSGTFSVLIGSVFLMMMTTT-VAADELTTTSEPTITNH
gas57M12_10296      (1) VEKKQRFSLRKYKSGTFSVLIGSVFLMMTTT--VAADELTTTSEPTITNH
gas57M12_10035      (1) VEKKQRFSLRKYKSGTFSVLIGSVFLMMTTT--VAADELTTTSEPTITNH
gas57M12_20069      (1) VEKKQRFSLRKYKSGTFSVLIGSVFLMMTTT--VAADELTTTSEPTITNH
gas57M12_22432      (1) VEKKQRFSLRKYKSGTFSVLIGSVFLMMTTT--VAADELTTTSEPTITNH
gas57M4_40499       (1) VEKKQRFSLRKYKSGTFSVLIGSVFLMMTTT--VAADELTTTSEPTITNH
gas57M6,1_21259     (1) VEKKQRFSLRKYKSGTFSVLIGSVFLMMTTT--VAADELTTTSEPTITNH
```

FIG. 31B

```
                        51                                               100
     gas57M1_SF370  (49) AQQQAQHLTNTELSSAESKSQDTSQITLKTNREKEQSQDLVSEPTTTELA
     gas57M1_31075  (49) AQQQAQHLTNTELSSAESKSQDTSQITLKTNREKEQSQDLVSEPTTTELA
     gas57M1_31237  (49) AQQQAQHLTNTELSSAESKSQDTSQITLKTNREKEQSQDLVSEPTTTELA
      gas57M1_3348  (49) AQQQAQHLTNTELSSAESKSQDTSQITLKTNREKEQSQDLVSEPTTTELA
     gas57M2_34585  (49) AQQQAQHLTNTELSSAESKSQDTSQITLKTNREKEQSQDLVSEPTTTELA
   gas57M3,1_21398  (49) AQQQAQHLTNTELSSAESKPQDTSQITPKTNREKEQSQDLVSEPTTTELA
 gas57M44-61_20839  (49) AQQQAQHLTNTELSSAESKSQDTSQITPKTNREKEQSQDLVSEPTTTELA
  gas57M6,31_20022  (49) TQQQAQHLTNTELSSAESKSQDTSQITPKTNREKEQSQDLVSEP-TTELA
    gas57M11_20648  (49) AQQQAQHLTNTELSSAESKTQDTSQITPKTNREKEQPQGLVSEPTTTELA
     gas57M23_2071  (49) TQQQAQHLTNTELSSAESKSQDTSQITPKTNREKEQPQGLVSEP-TTELA
  gas57M18,3_40128  (51) AQQQAQHLTNTELSSAESQSPDTSQITPKTNREKEQPQGLVSEPTTTELA
     gas57M4_10092  (51) AQQQAQHLTNTELSSAESQSPDTSQITPKTNREKEQPQGLVSEPTTTELA
     gas57M4_30968  (51) AQQQAQHLTNTELSSAESQSPDTSQITPKTNREKEQPQGLVSEPTTTELA
  gas57M6,31_22692  (51) AQQQAQHLTNTELSSAESQSPDTSQITPKTNREKEQPQGLVFEPTTTELA
  gas57M68,5_22814  (50) AQQQAQHLTNTELSSAESQSPDTSQITPKTNREKEQPQGLVSEPTTTELA
    gas57M68_23623  (51) AQQQAQHLTNTELSSAESQSPDTSQITPKTNREKEQPQGLVSEPTTTELA
     gas57M2_10064  (49) AQQQAPPLTNTELSSAESQPQDTSQVTPETNREKEQPQGLVSEPTTTELA
     gas57M2_10065  (49) AQQQAPPLTNTELSSAESQPQDTSQVTPETNREKEQPQGLVSEPTTTELA
    gas57M77_10251  (49) AQQQAQPLTNTELSSAESQSPHTSQVTPETNREKEQSQDLVSKPTTTELA
    gas57M77_10527  (49) AQQQAQPLTNTELSSAESQSPHTSQVTPETNREKEQSQDLVSKPTTTELA
    gas57M77_20696  (49) AQQQAQPLTNTELSSAESQSPHTSQVTPETNREKEQSQDLVSKPTTTELA
    gas57M89_21915  (49) AQQQAQPLTNTELSSAESQSPDTSQVTPETNREKEQSQDLVSKPTTTELA
    gas57M89_23717  (49) AQQQAQPLTNTELSSAESQSPDTSQVTPETNREKEQSQDLVSKPTTTELA
    gas57M94_10134  (49) AQQQAQPLTNTELSSAESQSPDTSQITPKTNREKEQSQDLVSKPTTTELA
    gas57M28_10164  (49) AQQQAQHLTNTELSSAESQSPDTSQITPKINREKEQPQGLVSEPTTTELA
    gas57M28_10218  (49) AQQQAQHLTNTELSSAESQSPDTSQITPKINREKEQPQGLVSEPTTTELA
    gas57M28_10266  (49) AQQQAQHLTNTELSSAESQSPDTSQITPKINREKEQPQGLVSEPTTTELA
    gas57M28_10299  (49) AQQQAQHLTNTELSSAESQSPDTSQITPKINREKEQPQGLVSEPTTTELA
    gas57M28_30176  (49) AQQQAQHLTNTELSSAESQSPDTSQITPKINREKEQPQGLVSEPTTTELA
    gas57M28_30574  (49) AQQQAQHLTNTELSSAESQSPDTSQITPKINREKEQPQGLVSEPTTTELA
   gas57M6,9_21802  (50) AQQQAQHLTNTELSSAESKPQDTSQITPKTNREKEQSQDLVSEPTTTELA
    gas57M75_10012  (51) AQQQAQHLTNTELSSAESKPQDTSQITPKTNREKEQSQDLVSEPTTTELA
    gas57M75_20671  (51) AQQQAQHLTNTELSSAESKPQDTSQITPKTNREKEQSQDLVSEPTTTELA
    gas57M75_30603  (51) AQQQAQHLTNTELSSAESKPQDTSQITPKTNREKEQSQDLVFEPTTTELA
    gas57M75_30207  (51) AQQQAQHLTNTELSSAESKPQDTSQITPKTNREKEQSQDLVSEPTTTELA
    gas57M22_20641  (50) TQQQAQHLTNTELSSAESKPQDTSQITLKTNREKEQPQGLVSEPTTTELA
    gas57M22_23465  (50) TQQQAQHLTNTELSSAESKPQDTSQITLKTNREKEQPQGLVSEPTTTELA
   gas57M3,1_30610  (50) TQQQAQHLTNTELSSAESKPQDTSQITLKTNREKEQPQGLVSEPTTTELA
   gas57M3,1_40603  (50) TQQQAQHLTNTELSSAESKPQDTSQITLKTNREKEQPQGLVSEPTTTELA
  gas57M3,28_24214  (50) TQQQAQHLTNTELSSAESKPQDTSQITLKTNREKEQPQGLVSEPTTTELA
  gas57M3,34_10307  (50) TQQQAQHLTNTELSSAESKPQDTSQITLKTNREKEQPQGLVSEPTTTELA
     gas57M4_40427  (50) TQQQAQHLTNTELSSAESKPQDTSQITLKTNREKEQPQGLVSEPTTTELA
      gas57M3_2721  (50) TQQQAQHLTNTELSSAESKPQDTSQITLKTNREKEQPQGLVSEPTTTELA
    gas57M12_10296  (49) TQQQAQHLTNTELSSAESKPQDTSQITLKTNREKEQPQGLVSEPTTTELA
    gas57M12_10035  (49) TQQQAQHLTNTELSSAESKPQDTSQITLKTNREKEQPQGLVSEPTTTELA
    gas57M12_20069  (49) TQQQAQHLTNTELSSAESKPQDTSQITLKTNREKEQPQGLVSEPTTTELA
    gas57M12_22432  (49) TQQQAQHLTNTELSSAESKPQDTSQITLKTNREKEQPQGLVSEPTTTELA
     gas57M4_40499  (49) TQQQAQHLTNTELSSAESKPQDTSQITLKTNREKEQPQGLVSEPTTTELA
    gas57M6,1_21259  (49) TQQQAQHLTNTELSSAESKPQDTSQITLKTNREKEQPQGLVSEPTTTELA
```

FIG. 31C

| | | |
|---|---|---|
| | | 101 150 |
| gas57M1_SF370 | (99) | DTDAASMANTGSDATQKSASLPPVNTDVHDWVKTKGAWDKGYKGQGKVVA |
| gas57M1_31075 | (99) | DTDAASMANTGSDATQKSASLPPVNTDVHDWVKTKGAWDKGYKGQGKVVA |
| gas57M1_31237 | (99) | DTDAASMANTGSDATQKSASLPPVNTDVHDWVKTKGAWDKGYKGQGKVVA |
| gas57M1_3348 | (99) | DTDAASMANTGSDATQKSASLPPVNTDVHDWVKTKGAWDKGYKGQGKVVA |
| gas57M2_34585 | (99) | DTDAASMANTGSDATQKSASLPPVNTDVHDWVKTKGAWDKGYKGQGKVVA |
| gas57M3,1_21398 | (99) | DTDAAPMADTGPDATQKSASLPPVNTDVHDWVKTKGAWDKGYKGQGKVVA |
| gas57M44-61_20839 | (99) | DTDAASMANTGSDATQKSASLPPVNTDVHDWVKTKGAWDKGYKGQGKVVA |
| gas57M6,31_20022 | (98) | DTDAAPMANTGPDATQKSASLPPVNTDVHDWVKTKGAWDKGYKGQGKVVA |
| gas57M11_20648 | (99) | DTDAASMADTGPDATQKSASLPPVNTDVHDWVKTKGAWDKGYKGQGKVVA |
| gas57M23_2071 | (98) | DTDAAPMANTGPDATQKSASLPPVNTDVHDWVKTKGAWDKGYKGQGKVVA |
| gas57M18,3_40128 | (101) | DTDAASMANTGPDATQKSASLPPVNTDVHDWVKTKGAWDKGYKGQGKVVA |
| gas57M4_10092 | (101) | DTDAASMANTGPDATQKSASLPPVNTDVHDWVKTKGAWDKGYKGQGKVVA |
| gas57M4_30968 | (101) | DTDAASMANTGPDATQKSASLPPVNTDVHDWVKTKGAWDKGYKGQGKVVA |
| gas57M6,31_22692 | (101) | DTDAASMANTGPDATQKSASLPPVNTDVHDWVKTKGAWDKGYKGQGKVVA |
| gas57M68,5_22814 | (100) | DTDAASMANTGPDATQKSASLPPVNTDVHDWVKTKGAWDKGYKGQGKVVA |
| gas57M68_23623 | (101) | DTDAASMANTGPDATQKSASLPPVNTDVHDWVKTKGAWDKGYKGQGKVVA |
| gas57M2_10064 | (99) | DTDAAPMANTGSDATQKSASLPPVNTDVHDWVKTKGAWDKGYKGQGKVVA |
| gas57M2_10065 | (99) | DTDAAPMANTGSDATQKSASLPPVNTDVHDWVKTKGAWDKGYKGQGKVVA |
| gas57M77_10251 | (99) | DTDAAPMANTGPDATQKSASLPPVNTDVHDWVKTKGAWDKGYKGQGKVVA |
| gas57M77_10527 | (99) | DTDAAPMANTGPDATQKSASLPPVNTDVHDWVKTKGAWDKGYKGQGKVVA |
| gas57M77_20696 | (99) | DTDAAPMANTGPDATQKSASLPPVNTDVHDWVKTKGAWDKGYKGQGKVVA |
| gas57M89_21915 | (99) | DTDAAPMANTGPDATQKSASLPPVNTDVHDWVKTKGAWDKGYKGQGKVVA |
| gas57M89_23717 | (99) | DTDAAPMANTGPDATQKSASLPPVNTDVHDWVKTKGAWDKGYKGQGKVVA |
| gas57M94_10134 | (99) | DTDSAPMANTGPDATQKSASLPPVNTDVHDWVKTKGAWDKGYKGQGKVVA |
| gas57M28_10164 | (99) | DTDAAPMANTGPDATQKSASLPPVNTDVHDWVKTKGAWDKGYKGQGKVVA |
| gas57M28_10218 | (99) | DTDAAPMANTGPDATQKSASLPPVNTDVHDWVKTKGAWDKGYKGQGKVVA |
| gas57M28_10266 | (99) | DTDAAPMANTGPDATQKSASLPPVNTDVHDWVKTKGAWDKGYKGQGKVVA |
| gas57M28_10299 | (99) | DTDAAPMANTGPDATQKSASLPPVNTDVHDWVKTKGAWDKGYKGQGKVVA |
| gas57M28_30176 | (99) | DTDAAPMANTGPDATQKSASLPPVNTDVHDWVKTKGAWDKGYKGQGKVVA |
| gas57M28_30574 | (99) | DTDAAPMANTGPDATQKSASLPPVNTDVHDWVKTKGAWDKGYKGQGKVVA |
| gas57M6,9_21802 | (100) | DTDAASMANTGPDATQKSASLPPVNTDVHDWVKTKGAWDKGYKGQGKVVA |
| gas57M75_10012 | (101) | DTDAASMANTGPDATQKSASLPPVNTDVHDWVKTKGAWGKGYKGQGKVVA |
| gas57M75_20671 | (101) | DTDAASMANTGPDATQKSASLPPVNTDVHDWVKTKGAWGKGYKGQGKVVA |
| gas57M75_30603 | (101) | DTDAASMANTGPDATQKSASLPPVNTDVHDWVKTKGAWGKGYKGQGKVVA |
| gas57M75_30207 | (101) | DTDAASMANTGPDATQKSASLPPVNTDVHDWVKTKGAWGKGYKGQGKVVA |
| gas57M22_20641 | (100) | DTDAAPMANTGPDATQKSASLPPVNTDVHDWVKTKGAWDKGYKGQGKVVA |
| gas57M22_23465 | (100) | DTDAAPMANTGPDATQKSASLPPVNTDVHDWVKTKGAWDKGYKGQGKVVA |
| gas57M3,1_30610 | (100) | DTDAAPMANTGPDATQKSASLPPVNTDVHDWVKTKGAWDKGYKGQGKVVA |
| gas57M3,1_40603 | (100) | DTDAAPMANTGPDATQKSASLPPVNTDVHDWVKTKGAWDKGYKGQGKVVA |
| gas57M3,28_24214 | (100) | DTDAAPMANTGPDATQKSASLPPVNTDVHDWVKTKGAWDKGYKGQGKVVA |
| gas57M3,34_10307 | (100) | DTDAAPMANTGPDATQKSASLPPVNTDVHDWVKTKGAWDKGYKGQGKVVA |
| gas57M4_40427 | (100) | DTDAAPMANTGPDATQKSASLPPVNTDVHDWVKTKGAWDKGYKGQGKVVA |
| gas57M3_2721 | (100) | DTDAAPMANTGPDATQKSASLPPVNTDVHDWVKTKGAWDKGYKGQGKVVA |
| gas57M12_10296 | (99) | DTDAAPMANTGPDATQKSASLPPVNTDVHDWVKTKGAWDKGYKGQGKVVA |
| gas57M12_10035 | (99) | DTDAAPMANTGPDATQKSASLPPVNTDVHDWVKTKGAWDKGYKGQGKVVA |
| gas57M12_20069 | (99) | DTDAAPMANTGPDATQKSASLPPVNTDVHDWVKTKGAWDKGYKGQGKVVA |
| gas57M12_22432 | (99) | DTDAAPMANTGPDATQKSASLPPVNTDVHDWVKTKGAWDKGYKGQGKVVA |
| gas57M4_40499 | (99) | DTDAAPMANTGPDATQKSASLPPVNTDVHDWVKTKGAWDKGYKGQGKVVA |
| gas57M6,1_21259 | (99) | DTDAAPMANTGPDATQKSASLPPVNTDVHDWVKTKGAWDKGYKGQGKVVA |

FIG. 3D

| | | 151 200 |
|---|---|---|
| gas57M1_SF370 | (149) | VIDTGIDPAHQSMRISDVSTAKVKSKEDMLARQKAAGINYGSWINDKVVF |
| gas57M1_31075 | (149) | VIDTGIDPAHQSMRISDVSTAKVKSKEDMLARQKAAGINYGSWINDKVVF |
| gas57M1_31237 | (149) | VIDTGIDPAHQSMRISDVSTAKVKSKEDMLARQKAAGINYGSWINDKVVF |
| gas57M1_3348 | (149) | VIDTGIDPAHQSMRISDVSTAKVKSKEDMLARQKAAGINYGSWINDKVVF |
| gas57M2_34585 | (149) | VIDTGIDPAHQSMRISDVSTAKVKSKEDMLARQKAAGINYGSWINDKVVF |
| gas57M3,1_21398 | (149) | VIDTGIDPAHQSMRISDVSTAKVKSKEDMLARQKAAGINYGSWINDKVVF |
| gas57M44-61_20839 | (149) | VIDTGIDPAHQSMRISDVSTAKVKSKEDMLARQKAAGINYGSWINDKVVF |
| gas57M6,31_20022 | (148) | VIDTGIDPAHQSMRISDVSTAKVKSKEDMLARQKAAGINYGSWINDKVVF |
| gas57M11_20648 | (149) | VIDTGIDPAHQSMRISDVSTAKVKSKEDMLARQKAAGINYGSWINDKVVF |
| gas57M23_2071 | (148) | VIDTGIDPAHQSMRISDVSTAKVKSKEDMLARQKAAGINYGSWINDKVVF |
| gas57M18,3_40128 | (151) | VIDTGIDPAHQSMRISDVSTAKVKSKEDMLARQKAAGINYGSWINDKVVF |
| gas57M4_10092 | (151) | VIDTGIDPAHQSMRISDVSTAKVKSKEDMLARQKAAGINYGSWINDKVVF |
| gas57M4_30968 | (151) | VIDTGIDPAHQSMRISDVSTAKVKSKEDMLARQKAAGINYGSWINDKVVF |
| gas57M6,31_22692 | (151) | VIDTGIDPAHQSMRISDVSTAKVKSKEDMLARQKAAGINYGSWINDKVVF |
| gas57M68,5_22814 | (150) | VIDTGIDPAHQSMRISDVSTAKVKSKEDMLARQKAAGINYGSWINDKVVF |
| gas57M68_23623 | (151) | VIDTGIDPAHQSMRISDVSTAKVKSKEDMLARQKAAGINYGSWINDKVVF |
| gas57M2_10064 | (149) | VIDTGIDPAHQSMRISDVSTAKVKSKEDMLARQKAAGINYGSWINDKVVF |
| gas57M2_10065 | (149) | VIDTGIDPAHQSMRISDVSTAKVKSKEDMLARQKAAGINYGSWINDKVVF |
| gas57M77_10251 | (149) | VIDTGIDPAHQSMHISDVSTAKVKSKEDMLARQKAAGINYGSWINDKVVF |
| gas57M77_10527 | (149) | VIDTGIDPAHQSMHISDVSTAKVKSKEDMLARQKAAGINYGSWINDKVVF |
| gas57M77_20696 | (149) | VIDTGIDPAHQSMHISDVSTAKVKSKEDMLARQKAAGINYGSWINDKVVF |
| gas57M89_21915 | (149) | VIDTGIDPAHQSMRISDVSTAKVKSKEDMLARQKAAGINYGSWINDKVVF |
| gas57M89_23717 | (149) | VIDTGIDPAHQSMRISDVSTAKVKSKEDMLARQKAAGINYGSWINDKVVF |
| gas57M94_10134 | (149) | VIDTGIDPAHQSMRISDVSTAKVKSKEDMLARQKAAGINYGSWINDKVVF |
| gas57M28_10164 | (149) | VIDTGIDPAHQSMRISDVSTAKVKSKEDMLARQKAAGINYGSWINDKVVF |
| gas57M28_10218 | (149) | VIDTGIDPAHQSMRISDVSTAKVKSKEDMLARQKAAGINYGSWINDKVVF |
| gas57M28_10266 | (149) | VIDTGIDPAHQSMRISDVSTAKVKSKEDMLARQKAAGINYGSWINDKVVF |
| gas57M28_10299 | (149) | VIDTGIDPAHQSMRISDVSTAKVKSKEDMLARQKAAGINYGSWINDKVVF |
| gas57M28_30176 | (149) | VIDTGIDPAHQSMRISDVSTAKVKSKEDMLARQKAAGINYGSWINDKVVF |
| gas57M28_30574 | (149) | VIDTGIDPAHQSMRISDVSTAKVKSKEDMLARQKAAGINYGSWINDKVVF |
| gas57M6,9_21802 | (150) | VIDTGIDPAHQSMRISDVSTAKVKSKEDMLARQKAAGINYGSWINDKVVF |
| gas57M75_10012 | (151) | VIDTGIDPAHQSMRISDVSTAKVKSKEDMLARQKAAGINYGSWINDKVVF |
| gas57M75_20671 | (151) | VIDTGIDPAHQSMRISDVSTAKVKSKEDMLARQKAAGINYGSWINDKVVF |
| gas57M75_30603 | (151) | VIDTGIDPAHQSMRISDVSTAKVKSKEDMLARQKAAGINYGSWINDKVVF |
| gas57M75_30207 | (151) | VIDTGIDPAHQSMRISDVSTAKVKSKEDMLARQKAAGINYGSWINDKVVF |
| gas57M22_20641 | (150) | VIDTGIDPAHQSMRISDVSTAKVKSKEDMLARQKAAGINYGSWINDKVVF |
| gas57M22_23465 | (150) | VIDTGIDPAHQSMRISDVSTAKVKSKEDMLARQKAAGINYGSWINDKVVF |
| gas57M3,1_30610 | (150) | VIDTGIDPAHQSMRISDVSTAKVKSKEDMLARQKAAGINYGSWINDKVVF |
| gas57M3,1_40603 | (150) | VIDTGIDPAHQSMRISDVSTAKVKSKEDMLARQKAAGINYGSWINDKVVF |
| gas57M3,28_24214 | (150) | VIDTGIDPAHQSMRISDVSTAKVKSKEDMLARQKAAGINYGSWINDKVVF |
| gas57M3,34_10307 | (150) | VIDTGIDPAHQSMRISDVSTAKVKSKEDMLARQKAAGINYGSWINDKVVF |
| gas57M4_40427 | (150) | VIDTGIDPAHQSMRISDVSTAKVKSKEDMLARQKAAGINYGSWINDKVVF |
| gas57M3_2721 | (150) | VIDTGIDPAHQSMRISDVSTAKVKSKEDMLARQKAAGINYGSWINDKVVF |
| gas57M12_10296 | (149) | VIDTGIDPAHQSMRISDVSTAKVKSKEDMLARQKAAGINYGSWINDKVVF |
| gas57M12_10035 | (149) | VIDTGIDPAHQSMRISDVSTAKVKSKEDMLARQKAAGINYGSWINDKVVF |
| gas57M12_20069 | (149) | VIDTGIDPAHQSMRISDVSTAKVKSKEDMLARQKAAGINYGSWINDKVVF |
| gas57M12_22432 | (149) | VIDTGIDPAHQSMRISDVSTAKVKSKEDMLARQKAAGINYGSWINDKVVF |
| gas57M4_40499 | (149) | VIDTGIDPAHQSMRISDVSTAKVKSKEDMLARQKAAGINYGSWINDKVVF |
| gas57M6,1_21259 | (149) | VIDTGIDPAHQSMRISDVSTAKVKSKEDMLARQKAAGINYGSWINDKVVF |

FIG. 31E

```
                          201                                                250
      gas57M1_SF370  (199) AHNYVENSDNIKENQFEDFDEDWENFEFDAEAEPKAIKKHKIYRPQSTQA
      gas57M1_31075  (199) AHNYVENSDNIKENQFEDFDEDWENFEFDAEAEPKAIKKHKIYRPQSTQA
      gas57M1_31237  (199) AHNYVENSDNIKENQFEDFDEDWENFEFDAEAEPKAIKKHKIYRPQSTQA
       gas57M1_3348  (199) AHNYVENSDNIKENQFEDFDEDWENFEFDAEAEPKAIKKHKIYRPQSTQA
      gas57M2_34585  (199) AHNYVENSDNIKENQFEDFDEDWENFEFDAEAEPKAIKKHKIYRPQSTQA
    gas57M3,1_21398  (199) AHNYVENSDNIKENQFEDFDEDWENFEFDADAEPKAIKKHKIYRPQSTQA
  gas57M44-61_20839  (199) AHNYVENSDNIKENQFEDFDEDWENFEFDADAEPKAIKKHKIYRPQSTQA
   gas57M6,31_20022  (198) AHNYVENSDNIKENQFEDFDEDWENFEFDADAEPKAIKKHKIYRPQSTQA
     gas57M11_20648  (199) AHNYVENSDNIKENQFGDFDEDWENFEFD--AEPKAIKKNKIYRPQSTQA
      gas57M23_2071  (198) AHNYVENSDNIKENQFGDFDEDWENFEFDAEAEPKAIKKHKIYRPQSTQA
   gas57M18,3_40128  (201) AHNYVENSDNIKENQFGDFDEDWENFEFD--AEPKAIKKNKIYRPQSTQA
      gas57M4_10092  (201) AHNYVENSDNIKENQFGDFDEDWENFEFD--AEPKAIKKNKIYRPQSTQA
      gas57M4_30968  (201) AHNYVENSDNIKENQFGDFDEDWENFEFD--AEPKAIKKNKIYRPQSTQA
   gas57M6,31_22692  (201) AHNYVENSDNIKENQFGDFDEDWENFEFD--AEPKAIKKNKIYRPQSTQA
   gas57M68,5_22814  (200) AHNYVENSDNIKENQFEDFDEDWENFEFD--AEPKAIKKNKIYRPQSTQA
     gas57M68_23623  (201) AHNYVENSDNIKENQFEDFDEDWENFEFD--AEPKAIKKNKIYRPQSTQA
      gas57M2_10064  (199) AHNYVENSDNIKENQFGDFDEDWENFEFD--AEPKAIKKNKIYRPQSTQA
      gas57M2_10065  (199) AHNYVENSDNIKENQFGDFDEDWENFEFD--AEPKAIKKNKIYRPQSTQA
     gas57M77_10251  (199) AHNYVENSDNIKENQFGDFDEDWENFEFD--AEPKAIKKHKIYRPQSTQA
     gas57M77_10527  (199) AHNYVENSDNIKENQFGDFDEDWENFEFD--AEPKAIKKHKIYRPQSTQA
     gas57M77_20696  (199) AHNYVENSDNIKENQFGDFDEDWENFEFD--AEPKAIKKHKIYRPQSTQA
     gas57M89_21915  (199) AHNYVENSDNIKENQFGDFDEDWENFEFDADAEPKAIKKHKIYRPQSTQA
     gas57M89_23717  (199) AHNYVENSDNIKENQFGDFDEDWENFEFDADAEPKAIKKHKIYRPQSTQA
     gas57M94_10134  (199) AHNYVENSDNIKENQFGDFDEDWENFEFD--AEPKAIKKHKIYRPQSTQA
     gas57M28_10164  (199) AHNYVENSDNIKENQFEDFDEDWENFEFD--AEPKAIKKHKIYRPQSTQA
     gas57M28_10218  (199) AHNYVENSDNIKENQFEDFDEDWENFEFD--AEPKAIKKHKIYRPQSTQA
     gas57M28_10266  (199) AHNYVENSDNIKENQFEDFDEDWENFEFD--AEPKAIKKHKIYRPQSTQA
     gas57M28_10299  (199) AHNYVENSDNIKENQFEDFDEDWENFEFD--AEPKAIKKHKIYRPQSTQA
     gas57M28_30176  (199) AHNYVENSDNIKENQFEDFDEDWENFEFD--AEPKAIKKHKIYRPQSTQA
     gas57M28_30574  (199) AHNYVENSDNIKENQFEDFDEDWENFEFD--AEPKAIKKHKIYRPQSTQA
    gas57M6,9_21802  (200) AHNYVENSDNIKENQFEDFDEDWENFEFDAEAEPKAIKKHKIYRPQSTQA
     gas57M75_10012  (201) AHNYVENSDNIKENQFEDFDEDWENFEFDADAEPKAIKKHKIYRPQSTQA
     gas57M75_20671  (201) AHNYVENSDNIKENQFEDFDEDWENFEFDADAEPKAIKKHKIYRPQSTQA
     gas57M75_30603  (201) AHNYVENSDNIKENQFEDFDEDWENFEFDADAEPKAIKKHKIYRPQSTQA
     gas57M75_30207  (201) AHNYVENSDNIKENQFEDFDEDWENFEFDADAEPKAIKKHKIYRPQSTQA
     gas57M22_20641  (200) AHNYVENSDNIKENQFEDFDEDWENFEFDAEAEPKAIKKHKIYRPQSTQA
     gas57M22_23465  (200) AHNYVENSDNIKENQFEDFDEDWENFEFDAEAEPKAIKKHKIYRPQSTQA
    gas57M3,1_30610  (200) AHNYVENSDNIKENQFEDFDEDWENFEFDAEAEPKAIKKHKIYRPQSTQA
    gas57M3,1_40603  (200) AHNYVENSDNIKENQFEDFDEDWENFEFDAEAEPKAIKKHKIYRPQSTQA
   gas57M3,28_24214  (200) AHNYVENSDNIKENQFEDFDEDWENFEFDAEAEPKAIKKHKIYRPQSTQA
   gas57M3,34_10307  (200) AHNYVENSDNIKENQFEDFDEDWENFEFDAEAEPKAIKKHKIYRPQSTQA
      gas57M4_40427  (200) AHNYVENSDNIKENQFEDFDEDWENFEFDAEAEPKAIKKHKIYRPQSTQA
       gas57M3_2721  (200) AHNYVENSDNIKENQFEDFDEDWENFEFDAEAEPKAIKKHKIYRPQSTQA
     gas57M12_10296  (199) AHNYVENSDNIKENQFEDFDEDWENFEFDAEAEPKAIKKHKIYRPQSTQA
     gas57M12_10035  (199) AHNYVENSDNIKENQFEDFDEDWENFEFDAEAEPKAIKKHKIYRPQSTQA
     gas57M12_20069  (199) AHNYVENSDNIKENQFEDFDEDWENFEFDAEAEPKAIKKHKIYRPQSTQA
     gas57M12_22432  (199) AHNYVENSDNIKENQFEDFDEDWENFEFDAEAEPKAIKKHKIYRPQSTQA
      gas57M4_40499  (199) AHNYVENSDNIKENQFEDFDEDWENFEFDAEAEPKAIKKHKIYRPQSTQA
    gas57M6,1_21259  (199) AHNYVENSDNIKENQFEDFDEDWENFEFDAEAEPKAIKKHKIYRPQSTQA
```

FIG. 31F

| | | 251 300 |
|---|---|---|
| gas57M1_SF370 | (249) | PKETVIKTEETDGSHDIDWTQTDDDTKYESHGMHVTGIVAGNSKEAAATG |
| gas57M1_31075 | (249) | PKETVIKTEETDGSHDIDWTQTDDDTKYESHGMHVTGIVAGNSKEAAATG |
| gas57M1_31237 | (249) | PKETVIKTEETDGSHDIDWTQTDDDTKYESHGMHVTGIVAGNSKEAAATG |
| gas57M1_3348 | (249) | PKETVIKTEETDGSHDIDWTQTDDDTKYESHGMHVTGIVAGNSKEAAATG |
| gas57M2_34585 | (249) | PKETVIKTEETDGSHDIDWTQTDDDTKYESHGMHVTGIVAGNSKEAAATG |
| gas57M3,1_21398 | (249) | PKETVIKTEETDGSHDIDWTQTDDDTKYESHGMHVTGIVAGNSKEAAATG |
| gas57M44-61_20839 | (249) | PKETVIKTEETDGSHDIDWTQTDDDTKYESHGMHVTGIVAGNSKEAAATG |
| gas57M6,31_20022 | (248) | PKETVIKTEETDGSHDIDWTQTDDDTKYESHGMHVTGIVAGNSKEAAATG |
| gas57M11_20648 | (247) | PKETVIKTEETDGSHDIDWTQTDDETKYESHGMHVTGIVAGNSKEAAATG |
| gas57M23_2071 | (248) | PKETVIKTEETDGSHDIDWTQTDDDTKYESHGMHVTGIVAGNSKEAAATG |
| gas57M18,3_40128 | (249) | PKETVIKTEETDGSHDIDWTQTDDDTKYESHGMHVTGIVAGNSKEAAATG |
| gas57M4_10092 | (249) | PKETVIKTEETDGSHDIDWTQTDDDTKYESHGMHVTGIVAGNSKEAAATG |
| gas57M4_30968 | (249) | PKETVIKTEETDGSHDIDWTQTDDDTKYESHGMHVTGIVAGNSKEAAATG |
| gas57M6,31_22692 | (249) | PKETVIKTEETDGSHDIDWTQTDDDTKYESHGMHVTGIVAGNSKEAAATG |
| gas57M68,5_22814 | (248) | PKETVIKTEETDGSHDIDWTQTDDETKYESHGMHVTGIVAGNSKEAAATG |
| gas57M68_23623 | (249) | PKETVIKTEETDGSHDIDWTQTDDETKYESHGMHVTGIVAGNSKEAAATG |
| gas57M2_10064 | (247) | PKETVIKTEETDGSHDIDWTQTDDETKYESHGMHVTGIVAGNSKEAAATG |
| gas57M2_10065 | (247) | PKETVIKTEETDGSHDIDWTQTDDETKYESHGMHVTGIVAGNSKEAAATG |
| gas57M77_10251 | (247) | PKETVIKTEETDGSHDIDWTQTDDETKYESHGMHVTGIVAGNSKEAAATG |
| gas57M77_10527 | (247) | PKETVIKTEETDGSHDIDWTQTDDETKYESHGMHVTGIVAGNSKEAAATG |
| gas57M77_20696 | (247) | PKETVIKTEETDGSHDIDWTQTDDETKYESHGMHVTGIVAGNSKEAAATG |
| gas57M89_21915 | (249) | PKETVIKTEETDGSHDIDWTQTDDETKYESHGMHVTGIVAGNSKEAAATG |
| gas57M89_23717 | (249) | PKETVIKTEETDGSHDIDWTQTDDETKYESHGMHVTGIVAGNSKEAAATG |
| gas57M94_10134 | (247) | PKETVIKTEETDGSHDIDWTQTDDETKYESHGMHVTGIVAGNSKEAAATG |
| gas57M28_10164 | (247) | PKETVIKTEETDGSHDIDWTQTDDDTKYESHGMHVTGIVAGNSKEAAATG |
| gas57M28_10218 | (247) | PKETVIKTEETDGSHDIDWTQTDDDTKYESHGMHVTGIVAGNSKEAAATG |
| gas57M28_10266 | (247) | PKETVIKTEETDGSHDIDWTQTDDDTKYESHGMHVTGIVAGNSKEAAATG |
| gas57M28_10299 | (247) | PKETVIKTEETDGSHDIDWTQTDDDTKYESHGMHVTGIVAGNSKEAAATG |
| gas57M28_30176 | (247) | PKETVIKTEETDGSHDIDWTQTDDDTKYESHGMHVTGIVAGNSKEAAATG |
| gas57M28_30574 | (247) | PKETVIKTEETDGSHDIDWTQTDDDTKYESHGMHVTGIVAGNSKEAAATG |
| gas57M6,9_21802 | (250) | PKETVIKTEETDGSHDIDWTQTDDDTKYESHGMHVTGIVAGNSKEAAATG |
| gas57M75_10012 | (251) | PKETVIKTEETDGSHDIDWTQTDDDTKYESHGMHVTGIVAGNSKEAAATG |
| gas57M75_20671 | (251) | PKETVIKTEETDGSHDIDWTQTDDDTKYESHGMHVTGIVAGNSKEAAATG |
| gas57M75_30603 | (251) | PKETVIKTEETDGSHDIDWTQTDDDTKYESHGMHVTGIVAGNSKEAAATG |
| gas57M75_30207 | (251) | PKETVIKTEETDGSHDIDWTQTDDDTKYESHGMHVTGIVAGNSKEAAATG |
| gas57M22_20641 | (250) | PKETVIKTEETDGSHDIDWTQTDDDTKYESHGMHVTGIVAGNSKEAAATG |
| gas57M22_23465 | (250) | PKETVIKTEETDGSHDIDWTQTDDDTKYESHGMHVTGIVAGNSKEAAATG |
| gas57M3,1_30610 | (250) | PKETVIKTEETDGSHDIDWTQTDDDTKYESHGMHVTGIVAGNSKEAAATG |
| gas57M3,1_40603 | (250) | PKETVIKTEETDGSHDIDWTQTDDDTKYESHGMHVTGIVAGNSKEAAATG |
| gas57M3,28_24214 | (250) | PKETVIKTEETDGSHDIDWTQTDDDTKYESHGMHVTGIVAGNSKEAAATG |
| gas57M3,34_10307 | (250) | PKETVIKTEETDGSHDIDWTQTDDDTKYESHGMHVTGIVAGNSKEAAATG |
| gas57M4_40427 | (250) | PKETVIKTEETDGSHDIDWTQTDDDTKYESHGMHVTGIVAGNSKEAAATG |
| gas57M3_2721 | (250) | PKETVIKTEETDGSHDIDWTQTDDDTKYESHGMHVTGIVAGNSKEAAATG |
| gas57M12_10296 | (249) | PKETVIKTEETDGSHDIDWTQTDDDTKYESHGMHVTGIVAGNSKEAAATG |
| gas57M12_10035 | (249) | PKETVIKTEETDGSHDIDWTQTDDDTKYESHGMHVTGIVAGNSKEAAATG |
| gas57M12_20069 | (249) | PKETVIKTEETDGSHDIDWTQTDDDTKYESHGMHVTGIVAGNSKEAAATG |
| gas57M12_22432 | (249) | PKETVIKTEETDGSHDIDWTQTDDDTKYESHGMHVTGIVAGNSKEAAATG |
| gas57M4_40499 | (249) | PKETVIKTEETDGSHDIDWTQTDDDTKYESHGMHVTGIVAGNSKEAAATG |
| gas57M6,1_21259 | (249) | PKETVIKTEETDGSHDIDWTQTDDDTKYESHGMHVTGIVAGNSKEAAATG |

FIG. 31G

| | | |
|---|---|---|
| | | 301 350 |
| gas57M1_SF370 | (299) | ERFLGIAPEAQVMFMRVFANDIMGSAESLFIKAIEDAVALGADVINLSLG |
| gas57M1_31075 | (299) | ERFLGIAPEAQVMFMRVFANDIMGSAESLFIKAIEDAVALGADVINLSLG |
| gas57M1_31237 | (299) | ERFLGIAPEAQVMFMRVFANDIMGSAESLFIKAIEDAVALGADVINLSLG |
| gas57M1_3348 | (299) | ERFLGIAPEAQVMFMRVFANDIMGSAESLFIKAIEDAVALGADVINLSLG |
| gas57M2_34585 | (299) | ERFLGIAPEAQVMFMRVFANDIMGSAESLFIKAIEDAVALGADVINLSLG |
| gas57M3,1_21398 | (299) | ERFLGIAPEAQVMFMRVFANDVMGSAESLFIKAIEDAVALGADVINLSLG |
| gas57M44-61_20839 | (299) | ERFLGIAPEAQVMFMRVFANDVMGSAESLFIKAIEDAVALGADVINLSLG |
| gas57M6,31_20022 | (298) | ERFLGIAPEAQVMFMRVFANDVMGSAESLFIKAIEDAVALGADVINLSLG |
| gas57M11_20648 | (297) | ERFLGIAPEAQVMFMRVFANDVMGSAESLFIKAIEDAVALGADVINLSLG |
| gas57M23_2071 | (298) | ERFLGIAPEAQVMFMRVFANDVMGSAESLFIKAIEDAVALGADVINLSLG |
| gas57M18,3_40128 | (299) | ERFLGIAPEAQVMFMRVFANDVMGSAESLFIKAIEDAVALGADVINLSLG |
| gas57M4_10092 | (299) | ERFLGIAPEAQVMFMRVFANDVMGSAESLFIKAIEDAVALGADVINLSLG |
| gas57M4_30968 | (299) | ERFLGIAPEAQVMFMRVFANDVMGSAESLFIKAIEDAVALGADVINLSLG |
| gas57M6,31_22692 | (299) | ERFLGIAPEAQVMFMRVFANDVMGSAESLFIKAIEDAVALGADVINLSLG |
| gas57M68,5_22814 | (298) | ERFLGIAPEAQVMFMRVFANDVMGSAESLFIKAIEDAVALGADVINLSLG |
| gas57M68_23623 | (299) | ERFLGIAPEAQVMFMRVFANDVMGSAESLFIKAIEDAVALGADVINLSLG |
| gas57M2_10064 | (297) | ERFLGIAPEAQVMFMRVFANDVMGSAESLFIKAIEDAVALGADVINLSLG |
| gas57M2_10065 | (297) | ERFLGIAPEAQVMFMRVFANDVMGSAESLFIKAIEDAVALGADVINLSLG |
| gas57M77_10251 | (297) | ERFLGIAPEAQVMFMRVFANDVMGSAESLFIKAIEDAVALGADVINLSLG |
| gas57M77_10527 | (297) | ERFLGIAPEAQVMFMRVFANDVMGSAESLFIKAIEDAVALGADVINLSLG |
| gas57M77_20696 | (297) | ERFLGIAPEAQVMFMRVFANDVMGSAESLFIKAIEDAVALGADVINLSLG |
| gas57M89_21915 | (299) | ERFLGIAPEAQVMFMRVFANDVMGSAESLFIKAIEDAVALGADVINLSLG |
| gas57M89_23717 | (299) | ERFLGIAPEAQVMFMRVFANDVMGSAESLFIKAIEDAVALGADVINLSLG |
| gas57M94_10134 | (297) | ERFLGIAPEAQVMFMRVFANDVMGSAESLFIKAIEDAVALGADVINLSLG |
| gas57M28_10164 | (297) | ERFLGIAPETQVMFMRVFANDVMGSAESLFIKAIEDAVALGADVINLSLG |
| gas57M28_10218 | (297) | ERFLGIAPETQVMFMRVFANDVMGSAESLFIKAIEDAVALGADVINLSLG |
| gas57M28_10266 | (297) | ERFLGIAPETQVMFMRVFANDVMGSAESLFIKAIEDAVALGADVINLSLG |
| gas57M28_10299 | (297) | ERFLGIAPETQVMFMRVFANDVMGSAESLFIKAIEDAVALGADVINLSLG |
| gas57M28_30176 | (297) | ERFLGIAPETQVMFMRVFANDVMGSAESLFIKAIEDAVALGADVINLSLG |
| gas57M28_30574 | (297) | ERFLGIAPETQVMFMRVFANDVMGSAESLFIKAIEDAVALGADVINLSLG |
| gas57M6,9_21802 | (300) | ERFLGIAPEAQVMFMRVFANDVMGSAESLFIKAIEDAVALGADVINLSLG |
| gas57M75_10012 | (301) | ERFLGIAPEAQVMFMRVFANDVMGSAESLFIKAIEDAVALGADVINLSLG |
| gas57M75_20671 | (301) | ERFLGIAPEAQVMFMRVFANDVMGSAESLFIKAIEDAVALGADVINLSLG |
| gas57M75_30603 | (301) | ERFLGIAPEAQVMFMRVFANDVMGSAESLFIKAIEDAVALGADVINLSLG |
| gas57M75_30207 | (301) | ERFLGIAPEAQVMFMRVFANDVMGSAESLFIKAIEDAVALGADVINLSLG |
| gas57M22_20641 | (300) | ERFLGIAPEAQVMFMRVFANDVMGSAESLFIKAIEDAVALGADVINLSLG |
| gas57M22_23465 | (300) | ERFLGIAPEAQVMFMRVFANDVMGSAESLFIKAIEDAVALGADVINLSLG |
| gas57M3,1_30610 | (300) | ERFLGIAPEAQVMFMRVFANDVMGSAESLFIKAIEDAVALGADVINLSLG |
| gas57M3,1_40603 | (300) | ERFLGIAPEAQVMFMRVFANDVMGSAESLFIKAIEDAVALGADVINLSLG |
| gas57M3,28_24214 | (300) | ERFLGIAPEAQVMFMRVFANDVMGSAESLFIKAIEDAVALGADVINLSLG |
| gas57M3,34_10307 | (300) | ERFLGIAPEAQVMFMRVFANDVMGSAESLFIKAIEDAVALGADVINLSLG |
| gas57M4_40427 | (300) | ERFLGIAPEAQVMFMRVFANDVMGSAESLFIKAIEDAVALGADVINLSLG |
| gas57M3_2721 | (300) | ERFLGIAPEAQVMFMRVFANDVMGSAESLFIKAIEDAVALGADVINLSLG |
| gas57M12_10296 | (299) | ERFLGIAPEAQVMFMRVFANDVMGSAESLFIKAIEDAVALGADVINLSLG |
| gas57M12_10035 | (299) | ERFLGIAPEAQVMFMRVFANDVMGSAESLFIKAIEDAVALGADVINLSLG |
| gas57M12_20069 | (299) | ERFLGIAPEAQVMFMRVFANDVMGSAESLFIKAIEDAVALGADVINLSLG |
| gas57M12_22432 | (299) | ERFLGIAPEAQVMFMRVFANDVMGSAESLFIKAIEDAVALGADVINLSLG |
| gas57M4_40499 | (299) | ERFLGIAPEAQVMFMRVFANDVMGSAESLFIKAIEDAVALGADVINLSLG |
| gas57M6,1_21259 | (299) | ERFLGIAPEAQVMFMRVFANDVMGSAESLFIKAIEDAVALGADVINLSLG |

FIG. 31H

| | | 351 400 |
|---|---|---|
| gas57M1_SF370 | (349) | TANGAQLSGSKPLMEAIEKAKKAGVSVVVAAGNERVYGSDHDDPLATNPD |
| gas57M1_31075 | (349) | TANGAQLSGSKPLMEAIEKAKKAGVSVVVAAGNERVYGSDHDDPLATNPD |
| gas57M1_31237 | (349) | TANGAQLSGSKPLMEAIEKAKKAGVSVVVAAGNERVYGSDHDDPLATNPD |
| gas57M1_3348 | (349) | TANGAQLSGSKPLMEAIEKAKKAGVSVVVAAGNERVYGSDHDDPLATNPD |
| gas57M2_34585 | (349) | TANGAQLSGSKPLMEAIEKAKKAGVSVVVAAGNERVYGSDHDDPLATNPD |
| gas57M3,1_21398 | (349) | TANGAQLSGSKPLMEAIEKAKKAGVSVVVAAGNERVYGSDHDDPLATNPD |
| gas57M44-61_20839 | (349) | TANGAQLSGSKPLMEAIEKAKKAGVSVVVAAGNERVYGSDHDDPLATNPD |
| gas57M6,31_20022 | (348) | TANGAQLSGSKPLMEAIEKAKKAGVSVVVAAGNERVYGSDHDDPLATNPD |
| gas57M11_20648 | (347) | TANGAQLSGSKPLMEAIEKAKKAGVSVVVAAGNERVYGSDHDDPLATNPD |
| gas57M23_2071 | (348) | TANGAQLSGSKPLMEAIEKAKKAGVSVVVAAGNERVYGSDHDDPLATNPD |
| gas57M18,3_40128 | (349) | TANGAQLSGSKPLMEAIEKAKKAGVSVVVAAGNERVYGSDHDDPLATNPD |
| gas57M4_10092 | (349) | TANGAQLSGSKPLMEAIEKAKKAGVSVVVAAGNERVYGSDHDDPLATNPD |
| gas57M4_30968 | (349) | TANGAQLSGSKPLMEAIEKAKKAGVSVVVAAGNERVYGSDHDDPLATNPD |
| gas57M6,31_22692 | (349) | TANGAQLSGSKPLMEAIEKAKKAGVSVVVAAGNERVYGSDHDDPLATNPD |
| gas57M68,5_22814 | (348) | TANGAQLSGSKPLMEAIEKAKKAGVSVVVAAGNERVYGSDHDDPLATNPD |
| gas57M68_23623 | (349) | TANGAQLSGSKPLMEAIEKAKKAGVSVVVAAGNERVYGSDHDDPLATNPD |
| gas57M2_10064 | (347) | TANGAQLSGSKPLIEAIEKAKKAGVSVVVAAGNERVYGSDHDDPLATNPD |
| gas57M2_10065 | (347) | TANGAQLSGSKPLIEAIEKAKKAGVSVVVAAGNERVYGSDHDDPLATNPD |
| gas57M77_10251 | (347) | TANGAQLSGSKPLMEAIEKAKKAGVSVVVAAGNERVYGSDHDDPLATNPD |
| gas57M77_10527 | (347) | TANGAQLSGSKPLMEAIEKAKKAGVSVVVAAGNERVYGSDHDDPLATNPD |
| gas57M77_20696 | (347) | TANGAQLSGSKPLMEAIEKAKKAGVSVVVAAGNERVYGFDHDDPLATNPD |
| gas57M89_21915 | (349) | TANGAQLSGSKPLMEAIEKAKKAGVSVVVAAGNERVYGSDHDDPLATNPD |
| gas57M89_23717 | (349) | TANGAQLSGSKPLMEAIEKAKKAGVSVVVAAGNERVYGSDHDDPLATNPD |
| gas57M94_10134 | (347) | TANGAQLSGSKPLMEAIEKAKKAGVSVVVAAGNERVYGSDHDDPLATNPD |
| gas57M28_10164 | (347) | TANGAQLSGSKPLMEAIEKAKKAGVSVVVAAGNERVYGSDHDDPLATNPD |
| gas57M28_10218 | (347) | TANGAQLSGSKPLMEAIEKAKKAGVSVVVAAGNERVYGSDHDDPLATNPD |
| gas57M28_10266 | (347) | TANGAQLSGSKPLMEAIEKAKKAGVSVVVAAGNERVYGSDHDDPLATNPD |
| gas57M28_10299 | (347) | TANGAQLSGSKPLMEAIEKAKKAGVSVVVAAGNERVYGSDHDDPLATNPD |
| gas57M28_30176 | (347) | TANGAQLSGSKPLMEAIEKAKKAGVSVVVAAGNERVYGSDHDDPLATNPD |
| gas57M28_30574 | (347) | TANGAQLSGSKPLMEAIEKAKKAGVSVVVAAGNERVYGSDHDDPLATNPD |
| gas57M6,9_21802 | (350) | TANGAQLSGSKPLMEAIEKAKKAGVSVVVAAGNERVYGSDHDDPLATNPD |
| gas57M75_10012 | (351) | TANGAQLSGSKPLMEAIEKAKKAGVSVVVAAGNERVYGSDHDDPLATNPD |
| gas57M75_20671 | (351) | TANGAQLSGSKPLMEAIEKAKKAGVSVVVAAGNERVYGSDHDDPLATNPD |
| gas57M75_30603 | (351) | TANGAQLSGSKPLMEAIEKAKKAGVSVVVAAGNERVYGSDHDDPLATNPD |
| gas57M75_30207 | (351) | TANGAQLSGSKPLMEAIEKAKKAGVSVVVAAGNERVYGSDHDDPLATNPD |
| gas57M22_20641 | (350) | TANGAQLSGSKPLMEAIEKAKKAGVSVVVAAGNERVYGSDHDDPLATNPD |
| gas57M22_23465 | (350) | TANGAQLSGSKPLMEAIEKAKKAGVSVVVAAGNERVYGSDHDDPLATNPD |
| gas57M3,1_30610 | (350) | TANGAQLSGSKPLMEAIEKAKKAGVSVVVAAGNERVYGSDHDDPLATNPD |
| gas57M3,1_40603 | (350) | TANGAQLSGSKPLMEAIEKAKKAGVSVVVAAGNERVYGSDHDDPLATNPD |
| gas57M3,28_24214 | (350) | TANGAQLSGSKPLMEAIEKAKKAGVSVVVAAGNERVYGSDHDDPLATNPD |
| gas57M3,34_10307 | (350) | TANGAQLSGSKPLMEAIEKAKKAGVSVVVAAGNERVYGSDHDDPLATNPD |
| gas57M4_40427 | (350) | TANGAQLSGSKPLMEAIEKAKKAGVSVVVAAGNERVYGSDHDDPLATNPD |
| gas57M3_2721 | (350) | TANGAQLSGSKPLMEAIEKAKKAGVSVVVAAGNERVYGSDHDDPLATNPD |
| gas57M12_10296 | (349) | TANGAQLSGSKPLMEAIEKAKKAGVSVVVAAGNERVYGSDHDDPLATNPD |
| gas57M12_10035 | (349) | TANGAQLSGSKPLMEAIEKAKKAGVSVVVAAGNERVYGSDHDDPLATNPD |
| gas57M12_20069 | (349) | TANGAQLSGSKPLMEAIEKAKKAGVSVVVAAGNERVYGSDHDDPLATNPD |
| gas57M12_22432 | (349) | TANGAQLSGSKPLMEAIEKAKKAGVSVVVAAGNERVYGSDHDDPLATNPD |
| gas57M4_40499 | (349) | TANGAQLSGSKPLMEAIEKAKKAGVSVVVAAGNERVYGSDHDDPLATNPD |
| gas57M6,1_21259 | (349) | TANGAQLSGSKPLMEAIEKAKKAGVSVVVAAGNERVYGSDHDDPLATNPD |

FIG. 31I

| | | 401 450 |
|---|---|---|
| gas57M1_SF370 | (399) | YGLVGSPSTGRTPTSVAAINSKWVIQRLMTVKELENRADLNHGKAIYSES |
| gas57M1_31075 | (399) | YGLVGSPSTGRTPTSVAAINSKWVIQRLMTVKELENRADLNHGKAIYSES |
| gas57M1_31237 | (399) | YGLVGSPSTGRTPTSVAAINSKWVIQRLMTVKELENRADLNHGKAIYSES |
| gas57M1_3348 | (399) | YGLVGSPSTGRTPTSVAAINSKWVIQRLMTVKELENRADLNHGKAIYSES |
| gas57M2_34585 | (399) | YGLVGSPSTGRTPTSVAAINSKWVIQRLMTVKELENRADLNHGKAIYSES |
| gas57M3,1_21398 | (399) | YGLVGSPSTGRTPTSVAAINSKWVIQRLMTVKELENRADLNHGKAIYSES |
| gas57M44-61_20839 | (399) | YGLVGSPSTGRTPTSVAAINSKWVIQRLMTVKELENRADLNHGKAIYSES |
| gas57M6,31_20022 | (398) | YGLVGSPSTGRTPTSVAAINSKWVIQRLMTVKELENRADLNHGKAIYSES |
| gas57M11_20648 | (397) | YGLVGSPSTGRTPTSVAAINSKWVIQRLMTVKELENRADLNHGKAIYSES |
| gas57M23_2071 | (398) | YGLVGSPSTGRTPTSVAAINSKWVIQRLMTVKELENRADLNHGKAIYSES |
| gas57M18,3_40128 | (399) | YGLVGSPSTGRTPTSVAAINSKWVIQRLMTAKELENRADLNHGKAIYSES |
| gas57M4_10092 | (399) | YGLVGSPSTGRTPTSVAAINSKWVIQRLMTAKELENRADLNHGKAIYSES |
| gas57M4_30968 | (399) | YGLVGSPSTGRTPTSVAAINSKWVIQRLMTAKELENRADLNHGKAIYSES |
| gas57M6,31_22692 | (399) | YGLVGSPSTGRTPTSVAAINSKWVIQRLMTAKELENRADLNHGKAIYSES |
| gas57M68,5_22814 | (398) | YGLVGSPSTGRTPTSVAAINSKWVIQRLMTVKELENRADLNHGKAIYSES |
| gas57M68_23623 | (399) | YGLVGSPSTGRTPTSVAAINSKWVIQRLMTVKELENRADLNHGKAIYSES |
| gas57M2_10064 | (397) | YGLVGSPSTGRTPTSVAAINSKWVIQRLMTVKELENRADLNHGKAIYSES |
| gas57M2_10065 | (397) | YGLVGSPSTGRTPTSVAAINSKWVIQRLMTVKELENRADLNHGKAIYSES |
| gas57M77_10251 | (397) | YGLVGSPSTGRTPTSVAAINSKWVIQRLMTVKELENRADLNHGKAIYSES |
| gas57M77_10527 | (397) | YGLVGSPSTGRTPTSVAAINSKWVIQRLMTVKELENRADLNHGKAIYSES |
| gas57M77_20696 | (397) | YGLVGSPSTGRTPTSVAAINSKWVIQRLMTVKELENRADLNHGKAIYSES |
| gas57M89_21915 | (399) | YGLVGSPSTGRTPTSVAAINSKWVIQRLMTVKELENRADLNHGKAIYSES |
| gas57M89_23717 | (399) | YGLVGSPSTGRTPTSVAAINSKWVIQRLMTVKELENRADLNHGKAIYSES |
| gas57M94_10134 | (397) | YGLVGSPSTGRTPTSVAAINSKWVIQRLMTVKELESRADLNHGKAIYSES |
| gas57M28_10164 | (397) | YGLVGSPSTGRTPTSVAAINSKWVIQRLMTVKELENRADLNHGKAIYSES |
| gas57M28_10218 | (397) | YGLVGSPSTGRTPTSVAAINSKWVIQRLMTVKELENRADLNHGKAIYSES |
| gas57M28_10266 | (397) | YGLVGSPSTGRTPTSVAAINSKWVIQRLMTVKELENRADLNHGKAIYSES |
| gas57M28_10299 | (397) | YGLVGSPSTGRTPTSVAAINSKWVIQRLMTVKELENRADLNHGKAIYSES |
| gas57M28_30176 | (397) | YGLVGSPSTGRTPTSVAAINSKWVIQRLMTVKELENRADLNHGKAIYSES |
| gas57M28_30574 | (397) | YGLVGSPSTGRTPTSVAAINSKWVIQRLMTVKELENRADLNHGKAIYSES |
| gas57M6,9_21802 | (400) | YGLVGSPSTGRTPTSVAAINSKWVIQRLMTVKELENRADLNHGKAIYSES |
| gas57M75_10012 | (401) | YGLVGSPSTGRTPTSVAAINSKWVIQRLMTVKELENRADLNHGKAIYSES |
| gas57M75_20671 | (401) | YGLVGSPSTGRTPTSVAAINSKWVIQRLMTVKELENRADLNHGKAIYSES |
| gas57M75_30603 | (401) | YGLVGSPSTGRTPTSVAAINSKWVIQRLMTVKELENRADLNHGKAIYSES |
| gas57M75_30207 | (401) | YGLVGSPSTGRTPTSVAAINSKWVIQRLMTVKELENRADLNHGKAIYSES |
| gas57M22_20641 | (400) | YGLVGSPSTGRTPTSVAAINSKWVIQRLMTVKELENRADLNHGKAIYSES |
| gas57M22_23465 | (400) | YGLVGSPSTGRTPTSVAAINSKWVIQRLMTVKELENRADLNHGKAIYSES |
| gas57M3,1_30610 | (400) | YGLVGSPSTGRTPTSVAAINSKWVIQRLMTVKELENRADLNHGKAIYSES |
| gas57M3,1_40603 | (400) | YGLVGSPSTGRTPTSVAAINSKWVIQRLMTVKELENRADLNHGKAIYSES |
| gas57M3,28_24214 | (400) | YGLVGSPSTGRTPTSVAAINSKWVIQRLMTVKELENRADLNHGKAIYSES |
| gas57M3,34_10307 | (400) | YGLVGSPSTGRTPTSVAAINSKWVIQRLMTVKELENRADLNHGKAIYSES |
| gas57M4_40427 | (400) | YGLVGSPSTGRTPTSVAAINSKWVIQRLMTVKELENRADLNHGKAIYSES |
| gas57M3_2721 | (400) | YGLVGSPSTGRTPTSVAAINSKWVIQRLMTVKELENRADLNHGKAIYSES |
| gas57M12_10296 | (399) | YGLVGSPSTGRTPTSVAAINSKWVIQRLMTVKELENRADLNHGKAIYSES |
| gas57M12_10035 | (399) | YGLVGSPSTGRTPTSVAAINSKWVIQRLMTVKELENRADLNHGKAIYSES |
| gas57M12_20069 | (399) | YGLVGSPSTGRTPTSVAAINSKWVIQRLMTVKELENRADLNHGKAIYSES |
| gas57M12_22432 | (399) | YGLVGSPSTGRTPTSVAAINSKWVIQRLMTVKELENRADLNHGKAIYSES |
| gas57M4_40499 | (399) | YGLVGSPSTGRTPTSVAAINSKWVIQRLMTVKELENRADLNHGKAIYSES |
| gas57M6,1_21259 | (399) | YGLVGSPSTGRTPTSVAAINSKWVIQRLMTVKELENRADLNHGKAIYSES |

FIG. 31J

| | | 451 500 |
|---|---|---|
| gas57M1_SF370 | (449) | VDFKDIKDSLGYDKSHQFAYVKESTDAGYNAQDVKGKIALIERDPNKTYD |
| gas57M1_31075 | (449) | VDFKDIKDSLGYDKSHQFAYVKESTDAGYNAQDVKGKIALIERDPNKTYD |
| gas57M1_31237 | (449) | VDFKDIKDSLGYDKSHQFAYVKESTDAGYNAQDVKGKIALIERDPNKTYD |
| gas57M1_3348 | (449) | VDFKDIKDSLGYDKSHQFAYVKESTDAGYNAQDVKGKIALIERDPNKTYD |
| gas57M2_34585 | (449) | VDFKDIKDSLGYDKSHQFAYVKESTDAGYNAQDVKGKIALIERDPNKTYD |
| gas57M3,1_21398 | (449) | VDFKNIKDSLGYDKSHQFAYVKESTDAGYKAQDVKGKIALIERDPNKTYD |
| gas57M44-61_20839 | (449) | VDFKDIKDSLGYDKSHQFAYVKESTDAGYKAQDVKDKIALIERDPNKTYD |
| gas57M6,31_20022 | (448) | VDFKNIKDSLGYDKSHQFAYVKESTDAGYKAQDVKDKIALIERDPNKTYD |
| gas57M11_20648 | (447) | VDFKNIKDSLGYDKSHQFAYVKESTDAGYKAQDVKDKIALIERDPNKTYD |
| gas57M23_2071 | (448) | VDFKNIKDSLGYDKSHQFAYVKESTDAGYKAQDVKGKIALIERDPNKTYD |
| gas57M18,3_40128 | (449) | VDFKNIKDSLGYDKSHQFAYVKESTDAGYKAQDVKDKIALIERDPNKTYD |
| gas57M4_10092 | (449) | VDFKNIKDSLGYDKSHQFAYVKESTDAGYKAQDVKDKIALIERDPNKTYD |
| gas57M4_30968 | (449) | VDFKNIKDSLGYDKSHQFAYVKESTDAGYKAQDVKDKIALIERDPNKTYD |
| gas57M6,31_22692 | (449) | VDFKNIKDSLGYDKSHQFAYVKESTDAGYKAQDVKDKIALIERDPNKTYD |
| gas57M68,5_22814 | (448) | VDFKDIKDSLGYDKSHQFAYVKESTDAGYNAQDVKGKIALIERDPNKTYD |
| gas57M68_23623 | (449) | VDFKDIKDSLGYDKSHQFAYVKESTDAGYNAQDVKGKIALIERDPNKTYD |
| gas57M2_10064 | (447) | VDFKDIKDSLGYDKSHQFAYVKESTDAGYKAQDVKGKIALIERDPNKTYD |
| gas57M2_10065 | (447) | VDFKDIKDSLGYDKSHQFAYVKESTDAGYKAQDVKGKIALIERDPNKTYD |
| gas57M77_10251 | (447) | VDFKDIKDSLGYDKSHQFAYVKESTDAGYNAQDVKGKIALIERDPNKTYD |
| gas57M77_10527 | (447) | VDFKDIKDSLGYDKSHQFAYVKESTDAGYNAQDVKGKIALIERDPNKTYD |
| gas57M77_20696 | (447) | VDFKDIKDSLGYDKSHQFAYVKESTDAGYNAQDVKGKIALIERDPNKTYD |
| gas57M89_21915 | (449) | VDFKDIKDSLGYDKSHQFAYVKESTDAGYNAQDVKGKIALIERDPNKTYD |
| gas57M89_23717 | (449) | VDFKDIKDSLGYDKSHQFAYVKESTDAGYNAQDVKGKIALIERDPNKTYD |
| gas57M94_10134 | (447) | VDFKDIKDSLGYDKSHQFAYVKESTDAGYKAQDVKGKIALIERDPNKTYD |
| gas57M28_10164 | (447) | VDFKDIKDSLGYDKSHQFAYVKESTDAGYNAQDVKGKIALIERDPNKTYD |
| gas57M28_10218 | (447) | VDFKDIKDSLGYDKSHQFAYVKESTDAGYNAQDVKGKIALIERDPNKTYD |
| gas57M28_10266 | (447) | VDFKDIKDSLGYDKSHQFAYVKESTDAGYNAQDVKGKIALIERDPNKTYD |
| gas57M28_10299 | (447) | VDFKDIKDSLGYDKSHQFAYVKESTDAGYNAQDVKGKIALIERDPNKTYD |
| gas57M28_30176 | (447) | VDFKDIKDSLGYDKSHQFAYVKESTDAGYNAQDVKGKIALIERDPNKTYD |
| gas57M28_30574 | (447) | VDFKDIKDSLGYDKSHQFAYVKESTDAGYNAQDVKGKIALIERDPNKTYD |
| gas57M6,9_21802 | (450) | VDFKNIKDSLGYDKSHQFAYVKESTDAGYNAQDVKGKIALIERDPNKTYD |
| gas57M75_10012 | (451) | VDFKNIKDSLGYDKSHQFAYVKESTDAGYNAQDVKGKIALIERDPNKTYD |
| gas57M75_20671 | (451) | VDFKNIKDSLGYDKSHQFAYVKESTDAGYNAQDVKGKIALIERDPNKTYD |
| gas57M75_30603 | (451) | VDFKNIKDSLGYDKSHQFAYVKESTDAGYNAQDVKGKIALIERDPNKTYD |
| gas57M75_30207 | (451) | VDFKNIKDSLGYDKSHQFAYVKESTDAGYNAQDVKGKIALIERDPNKTYD |
| gas57M22_20641 | (450) | VDFKNIKDSLGYDKTHQFAYVKESTDAGYKAQDVKGKIALIERDPNKTYD |
| gas57M22_23465 | (450) | VDFKNIKDSLGYDKTHQFAYVKESTDAGYKAQDVKGKIALIERDPNKTYD |
| gas57M3,1_30610 | (450) | VDFKNIKDSLGYDKSHQFAYVKESTDAGYNAQDVKGKIALIERDPNKTYD |
| gas57M3,1_40603 | (450) | VDFKNIKDSLGYDKSHQFAYVKESTDAGYNAQDVKGKIALIERDPNKTYD |
| gas57M3,28_24214 | (450) | VDFKNIKDSLGYDKSHQFAYVKESTDAGYNAQDVKGKIALIERDPNKTYD |
| gas57M3,34_10307 | (450) | VDFKNIKDSLGYDKSHQFAYVKESTDAGYNAQDVKGKIALIERDPNKTYD |
| gas57M4_40427 | (450) | VDFKNIKDSLGYDKSHQFAYVKESTDAGYNAQDVKGKIALIERDPNKTYD |
| gas57M3_2721 | (450) | VDFKNIKDSLGYDKSHQFAYVKESTDAGYNAQDVKGKIALIERDPNKTYD |
| gas57M12_10296 | (449) | VDFKNIKDSLGYDKSHQFAYVKESTDAGYNAQNVKGKIALIERDPNKTYD |
| gas57M12_10035 | (449) | VDFKNIKDSLGYDKSHQFAYVKESTDAGYNAQNVKGKIALIERDPNKTYD |
| gas57M12_20069 | (449) | VDFKNIKDSLGYDKSHQFAYVKESTDAGYNAQNVKGKIALIERDPNKTYD |
| gas57M12_22432 | (449) | VDFKNIKDSLGYDKSHQFAYVKESTDAGYNAQNVKGKIALIERDPNKTYD |
| gas57M4_40499 | (449) | VDFKNIKDSLGYDKSHQFAYVKESTDAGYNAQNVKGKIALIERDPNKTYD |
| gas57M6,1_21259 | (449) | VDFKNIKDSLGYDKSHQFAYVKESTDAGYNAQNVKGKIALIERDPNKTYD |

FIG. 31K

| | | 501 550 |
|---|---|---|
| gas57M1_SF370 | (499) | EMIALAKKHGALGVLIFNNKPGQSNRSMRLTANGMGIPSAFISHEFGKAM |
| gas57M1_31075 | (499) | EMIALAKKHGALGVLIFNNKPGQSNRSMRLTANGMGIPSAFISHEFGKAM |
| gas57M1_31237 | (499) | EMIALAKKHGALGVLIFNNKPGQSNRSMRLTANGMGIPSAFISHEFGKAM |
| gas57M1_3348 | (499) | EMIALAKKHGALGVLIFNNKPGQSNRSMRLTANGMGIPSAFISHEFGKAM |
| gas57M2_34585 | (499) | EMIALAKKHGALGVLIFNNKPGQSNRSMRLTANGMGIPSAFISHEFGKAM |
| gas57M3,1_21398 | (499) | EMIALAKKHGALGVLIFNNKPGQSNRSMRLTANGMGIPSAFISHEFGKAM |
| gas57M44-61_20839 | (499) | EMIALAKKHGALGVLIFNNKPGQSNRSMRLTANGMGIPSAFISHEFGKAM |
| gas57M6,31_20022 | (498) | EMIALAKKHGALGVLIFNNKPGQSNRSMRLTANGMGIPSAFISHEFGKAM |
| gas57M11_20648 | (497) | EMIALAKKHGALGVLIFNNKPGQSNRSMRLTANGMGIPSAFISHEFGKAM |
| gas57M23_2071 | (498) | EMIALAKKHGALGVLIFNNKPGQSNRSMRLTANGMGIPSAFISHEFGKAM |
| gas57M18,3_40128 | (499) | EMIALAKKHGALGVLIFNNKPGQSNRSMRLTANGMGIPSAFISHEFGKAM |
| gas57M4_10092 | (499) | EMIALAKKHGALGVLIFNNKPGQSNRSMRLTANGMGIPSAFISHEFGKAM |
| gas57M4_30968 | (499) | EMIALAKKHGALGVLIFNNKPGQSNRSMRLTANGMGIPSAFISHEFGKAM |
| gas57M6,31_22692 | (499) | EMIALAKKHGALGVLIFNNKPGQSNRSMRLTANGMGIPSAFISHEFGKAM |
| gas57M68,5_22814 | (498) | EMIALAKKHGALGVLIFNNKPGQSNRSMRLTSNGMGIPSAFISHEFGKAM |
| gas57M68_23623 | (499) | EMIALAKKHGALGVLIFNNKPGQSNRSMRLTSNGMGIPSAFISHEFGKAM |
| gas57M2_10064 | (497) | EMIALAKKHGALGVLIFNNKPGQSNRSMRLTANGMGIPSAFISYEFGKAM |
| gas57M2_10065 | (497) | EMIALAKKHGALGVLIFNNKPGQSNRSMRLTANGMGIPSAFISYEFGKAM |
| gas57M77_10251 | (497) | EMIALAKKHGALGVLIFNNKPGQSNRSMRLTANGMGIPSAFISHEFGKAM |
| gas57M77_10527 | (497) | EMIALAKKHGALGVLIFNNKPGQSNRSMRLTANGMGIPSAFISHEFGKAM |
| gas57M77_20696 | (497) | EMIALAKKHGALGVLIFNNKPGQSNRSMRLTANGMGIPSAFISHEFGKAM |
| gas57M89_21915 | (499) | EMIALAKKHGALGVLIFNNKPGQSNRSMRLTANGMGIPSAFISHEFGKAM |
| gas57M89_23717 | (499) | EMIALAKKHGALGVLIFNNKPGQSNRSMRLTANGMGIPSAFISHEFGKAM |
| gas57M94_10134 | (497) | EMIALAKKHGALGVLIFNNKPGQSNRSMRLTANGMGIPSAFISHEFGKAM |
| gas57M28_10164 | (497) | EMIALAKKHGALGVLIFNNKPGQSNRSMRLTANGMGIPSAFISHEFGKAM |
| gas57M28_10218 | (497) | EMIALAKKHGALGVLIFNNKPGQSNRSMRLTANGMGIPSAFISHEFGKAM |
| gas57M28_10266 | (497) | EMIALAKKHGALGVLIFNNKPGQSNRSMRLTANGMGIPSAFISHEFGKAM |
| gas57M28_10299 | (497) | EMIALAKKHGALGVLIFNNKPGQSNRSMRLTANGMGIPSAFISHEFGKAM |
| gas57M28_30176 | (497) | EMIALAKKHGALGVLIFNNKPGQSNRSMRLTANGMGIPSAFISHEFGKAM |
| gas57M28_30574 | (497) | EMIALAKKHGALGVLIFNNKPGQSNRSMRLTANGMGIPSAFISHEFGKAM |
| gas57M6,9_21802 | (500) | EMIALAKKHGALGVLIFNNKPGQSNRSMRLTSNGMGIPSAFISHEFGKAM |
| gas57M75_10012 | (501) | EMIALAKKHGALGVLIFNNKPGQSNRSMRLTANGMGIPSAFISHEFGKAM |
| gas57M75_20671 | (501) | EMIALAKKHGALGVLIFNNKPGQSNRSMRLTANGMGIPSAFISHEFGKAM |
| gas57M75_30603 | (501) | EMIALAKKHGALGVLIFNNKPGQSNRSMRLTANGMGIPSAFISHEFGKAM |
| gas57M75_30207 | (501) | EMIALAKKHGALGVLIFNNKPGQSNRSMRLTANGMGIPSAFISHEFGKAM |
| gas57M22_20641 | (500) | EMIALAKKHGALGVLIFNNKPGQSNRSMRLTANGMGIPSAFISHEFGKAM |
| gas57M22_23465 | (500) | EMIALAKKHGALGVLIFNNKPGQSNRSMRLTANGMGIPSAFISHEFGKAM |
| gas57M3,1_30610 | (500) | EMIALAKKHGALGVLIFNNKPGQSNRSMRLTANGMGIPSAFISHEFGKVM |
| gas57M3,1_40603 | (500) | EMIALAKKHGALGVLIFNNKPGQSNRSMRLTANGMGIPSAFISHEFGKVM |
| gas57M3,28_24214 | (500) | EMIALAKKHGALGVLIFNNKPGQSNRSMRLTANGMGIPSAFISHEFGKVM |
| gas57M3,34_10307 | (500) | EMIALAKKHGALGVLIFNNKPGQSNRSMRLTANGMGIPSAFISHEFGKVM |
| gas57M4_40427 | (500) | EMIALAKKHGALGVLIFNNKPGQSNRSMRLTANGMGIPSAFISHEFGKVM |
| gas57M3_2721 | (500) | EMIALAKKHGALGVLIFNNKPGQSNRSMRLTANGMGIPSAFISHEFGKAM |
| gas57M12_10296 | (499) | EMIALAKKHGALGVLIFNNKPGQSNRSMRLTANGMGIPSAFISHEFGKAM |
| gas57M12_10035 | (499) | EMIALAKKHGALGVLIFNNKPGQSNRSMRLTANGMGIPSAFISHEFGKAM |
| gas57M12_20069 | (499) | EMIALAKKHGALGVLIFNNKPGQSNRSMRLTANGMGIPSAFISHEFGKAM |
| gas57M12_22432 | (499) | EMIALAKKHGALGVLIFNNKPGQSNRSMRLTANGMGIPSAFISHEFGKAM |
| gas57M4_40499 | (499) | EMIALAKKHGALGVLIFNNKPGQSNRSMRLTANGMGIPSAFISHEFGKAM |
| gas57M6,1_21259 | (499) | EMIALAKKHGALGVLIFNNKPGQSNRSMRLTANGMGIPSAFISHEFGKAM |

FIG. 31L

| | | |
|---|---|---|
| | | 551 600 |
| gas57M1_SF370 | (549) | SQLNGNGTGSLEFDSVVSKAPSQKGNEMNHFSNWGLTSDGYLKPDITAPG |
| gas57M1_31075 | (549) | SQLNGNGTGSLEFDSVVSKAPSQKGNEMNHFSNWGLTSDGYLKPDITAPG |
| gas57M1_31237 | (549) | SQLNGNGTGSLEFDSVVSKAPSQKGNEMNHFSNWGLTSDGYLKPDITAPG |
| gas57M1_3348 | (549) | SQLNGNGTGSLEFDSVVSKAPSQKGNEMNHFSNWGLTSDGYLKPDITAPG |
| gas57M2_34585 | (549) | SQLNGNGTGSLEFDSVVSKAPSQKGNEMNHFSNWGLTSDGYLKPDITAPG |
| gas57M3,1_21398 | (549) | SQLNGNGTGSLEFDSVVSKAPSQKGNEMNHFSNWGLTSDGYLKPDITAPG |
| gas57M44-61_20839 | (549) | SQLNGNGTGSLEFDSVVSKAPSQKGNEMNHFSNWGLTSDGYLKPDITAPG |
| gas57M6,31_20022 | (548) | SQLNGNGTGSLEFDSVVSKAPSQKGNEMNHFSNWGLTSDGYLKPDITAPG |
| gas57M11_20648 | (547) | SQLNGNGTGSLEFDSVVSKAPSQKGNEMNHFSNWGLTSDGYLKPDITAPG |
| gas57M23_2071 | (548) | SQLNGNGTGSLEFDSVVSKAPSQKGNEMNHFSNWGLTSDGYLKPDITAPG |
| gas57M18,3_40128 | (549) | SQLNGNGTGSLEFDSVVSKAPSQKGNEMNHFSNWGLTSDGYLKPDITAPG |
| gas57M4_10092 | (549) | SQLNGNGTGSLEFDSVVSKAPSQKGNEMNHFSNWGLTSDGYLKPDITAPG |
| gas57M4_30968 | (549) | SQLNGNGTGSLEFDSVVSKAPSQKGNEMNHFSNWGLTSDGYLKPDITAPG |
| gas57M6,31_22692 | (549) | SQLNGNGTGSLEFDSVVSKAPSQKGNEMNHFSNWGLTSDGYLKPDITAPG |
| gas57M68,5_22814 | (548) | SQLNGNGTGSLEFDSVVSKAPSQKGNEMNHFSNWGLTSDGYLKPDITAPG |
| gas57M68_23623 | (549) | SQLNGNGTGSLEFDSVVSKAPSQKGNEMNHFSNWGLTSDGYLKPDITAPG |
| gas57M2_10064 | (547) | SQLNGNGTGSLEFDSVVSKAPSQKGNEMNHFSNWGLTSDGYLKPDITAPG |
| gas57M2_10065 | (547) | SQLNGNGTGSLEFDSVVSKAPSQKGNEMNHFSNWGLTSDGYLKPDITAPG |
| gas57M77_10251 | (547) | SQLNGNGTGSLVFDSVVSKAPSQKGNEMNHFSNWGLTSDGYLKPDITAPG |
| gas57M77_10527 | (547) | SQLNGNGTGSLVFDSVVSKAPSQKGNEMNHFSNWGLTSDGYLKPDITAPG |
| gas57M77_20696 | (547) | SQLNGNGTGSLVFDSVVSKAPSQKGNEMNHFSNWGLTSDGYLKPDITAPG |
| gas57M89_21915 | (549) | SQLNGNGTGSLEFDSVVSKAPSQKGNEMNHFSNWGLTSDGYLKPDITAPG |
| gas57M89_23717 | (549) | SQLNGNGTGSLEFDSVVSKAPSQKGNEMNHFSNWGLTSDGYLKPDITAPG |
| gas57M94_10134 | (547) | SQLNTNGTGSLVFDSVVSKAPSQKGNEMNHFSNWGLTSDGYLKPDITAPG |
| gas57M28_10164 | (547) | SQLNGNGTGSLEFDSVVSKAPSQKGNEMNHFSNWGLTSDGYLKPDITAPG |
| gas57M28_10218 | (547) | SQLNGNGTGSLEFDSVVSKAPSQKGNEMNHFSNWGLTSDGYLKPDITAPG |
| gas57M28_10266 | (547) | SQLNGNGTGSLEFDSVVSKAPSQKGNEMNHFSNWGLTSDGYLKPDITAPG |
| gas57M28_10299 | (547) | SQLNGNGTGSLEFDSVVSKAPSQKGNEMNHFSNWGLTSDGYLKPDITAPG |
| gas57M28_30176 | (547) | SQLNGNGTGSLEFDSVVSKAPSQKGNEMNHFSNWGLTSDGYLKPDITAPG |
| gas57M28_30574 | (547) | SQLNGNGTGSLEFDSVVSKAPSQKGNEMNHFSNWGLTSDGYLKPDITAPG |
| gas57M6,9_21802 | (550) | SQLNGNGTGSLEFDSVVSKAPSQKGNEMNHFSNWGLTSDGYLKPDITAPG |
| gas57M75_10012 | (551) | SQLNGNGTGSLEFDSVVSKAPSQKGNEMNHFSNWGLTSDGYLKPDITAPG |
| gas57M75_20671 | (551) | SQLNGNGTGSLEFDSVVSKAPSQKGNEMNHFSNWGLTSDGYLKPDITAPG |
| gas57M75_30603 | (551) | SQLNGNGTGSLEFDSVVSKAPSQKGNEMNHFSNWGLTSDGYLKPDITAPG |
| gas57M75_30207 | (551) | SQLNGNGTGSLEFDSVVSKAPSQKGNEMNHFSNWGLTSDGYLKPDITAPG |
| gas57M22_20641 | (550) | SQLNGNGTGSLEFDSVVSKAPSQKGNEMNHFSNWGLTSDGYLKPDITAPG |
| gas57M22_23465 | (550) | SQLNGNGTGSLEFDSVVSKAPSQKGNEMNHFSNWGLTSDGYLKPDITAPG |
| gas57M3,1_30610 | (550) | SQLNGNGTGSLEFDSVVSKAPSQKGNEMNHFSNWGLTSDGYLKPDITAPG |
| gas57M3,1_40603 | (550) | SQLNGNGTGSLEFDSVVSKAPSQKGNEMNHFSNWGLTSDGYLKPDITAPG |
| gas57M3,28_24214 | (550) | SQLNGNGTGSLEFDSVVSKAPSQKGNEMNHFSNWGLTSDGYLKPDITAPG |
| gas57M3,34_10307 | (550) | SQLNGNGTGSLEFDSVVSKAPSQKGNEMNHFSNWGLTSDGYLKPDITAPG |
| gas57M4_40427 | (550) | SQLNGNGTGSLEFDSVVSKAPSQKGNEMNHFSNWGLTSDGYLKPDITAPG |
| gas57M3_2721 | (550) | SQLNGNGTGSLEFDSVVSKAPSQKGNEMNHFSNWGLTSDGYLKPDITAPG |
| gas57M12_10296 | (549) | SQLNGNGTGSLEFDSVVSKAPSQKGNEMNHFSNWGLTSDGYLKPDITAPG |
| gas57M12_10035 | (549) | SQLNGNGTGSLEFDSVVSKAPSQKGNEMNHFSNWGLTSDGYLKPDITAPG |
| gas57M12_20069 | (549) | SQLNGNGTGSLEFDSVVSKAPSQKGNEMNHFSNWGLTSDGYLKPDITAPG |
| gas57M12_22432 | (549) | SQLNGNGTGSLEFDSVVSKAPSQKGNEMNHFSNWGLTSDGYLKPDITAPG |
| gas57M4_40499 | (549) | SQLNGNGTGSLEFDSVVSKAPSQKGNEMNHFSNWGLTSDGYLKPDITAPG |
| gas57M6,1_21259 | (549) | SQLNGNGTGSLEFDSVVSKAPSQKGNEMNHFSNWGLTSDGYLKPDITAPG |

FIG. 31M

| | | 601 650 |
|---|---|---|
| gas57M1_SF370 | (599) | GDIYSTYNDNHYGSQTGTSMASPQIAGASLLVKQYLEKTQPNLPKEKIAD |
| gas57M1_31075 | (599) | GDIYSTYNDNHYGSQTGTSMASPQIAGASLLVKQYLEKTQPNLPKEKIAD |
| gas57M1_31237 | (599) | GDIYSTYNDNHYGSQTGTSMASPQIAGASLLVKQYLEKTQPNLPKEKIAD |
| gas57M1_3348 | (599) | GDIYSTYNDNHYGSQTGTSMASPQIAGASLLVKQYLEKTQPNLPKEKIAD |
| gas57M2_34585 | (599) | GDIYSTYNDNHYGSQTGTSMASPQIAGASLLVKQYLEKTQPNLPKEKIAD |
| gas57M3,1_21398 | (599) | GDIYSTYNDNHYGSQTGTSMASPQIAGASLLVKQYLEKTQPNLPKEKIAD |
| gas57M44-61_20839 | (599) | GDIYSTYNDNHYGSQTGTSMASPQIAGASLLVKQYLEKTQPNLPKEKIAD |
| gas57M6,31_20022 | (598) | GDIYSTYNDNHYGSQTGTSMASPQIAGASLLVKQYLEKTQPNLPKEKIAD |
| gas57M11_20648 | (597) | GDIYSTYNDNHYGSQTGTSMASPQIAGASLLVKQYLEKTQPNLPKEKIAD |
| gas57M23_2071 | (598) | GDIYSTYNDNHYGSQTGTSMASPQIAGASLLVKQYLEKTQPNLPKEKIAD |
| gas57M18,3_40128 | (599) | GDIYSTYNDNHYGSQTGTSMASPQIAGASLLVKQYLEKTQPNLPKEKIAD |
| gas57M4_10092 | (599) | GDIYSTYNDNHYGSQTGTSMASPQIAGASLLVKQYLEKTQPNLPKEKIAD |
| gas57M4_30968 | (599) | GDIYSTYNDNHYGSQTGTSMASPQIAGASLLVKQYLEKTQPNLPKEKIAD |
| gas57M6,31_22692 | (599) | GDIYSTYNDNHYGSQTGTSMASPQIAGASLLVKQYLEKTQPNLPKEKIAD |
| gas57M68,5_22814 | (598) | GDIYSTYNDNHYGSQTGTSMASPQIAGASLLVKQYLEKTQPNLPKEKIAD |
| gas57M68_23623 | (599) | GDIYSTYNDNHYGSQTGTSMASPQIAGASLLVKQYLEKTQPNLPKEKIAD |
| gas57M2_10064 | (597) | GDIYSTYNDNHYGSQTGTSMASPQIAGASLLVKQYLEKTQPNLPKEKIAD |
| gas57M2_10065 | (597) | GDIYSTYNDNHYGSQTGTSMASPQIAGASLLVKQYLEKTQPNLPKEKIAD |
| gas57M77_10251 | (597) | GDIYSTYNDNHYGSQTGTSMASPQIAGASLLVKQYLEKTQPNLPKEKIAD |
| gas57M77_10527 | (597) | GDIYSTYNDNHYGSQTGTSMASPQIAGASLLVKQYLEKTQPNLPKEKIAD |
| gas57M77_20696 | (597) | GDIYSTYNDNHYGSQTGTSMASPQIAGASLLVKQYLEKTQPNLPKEKIAD |
| gas57M89_21915 | (599) | GDIYSTYNDNHYGSQTGTSMASPQIAGASLLVKQYLEKTQPNLPKEKIAD |
| gas57M89_23717 | (599) | GDIYSTYNDNHYGSQTGTSMASPQIAGASLLVKQYLEKTQPNLPKEKIAD |
| gas57M94_10134 | (597) | GDIYSTYNDNHYGSQTGTSMASPQIAGASLLVKQYLEKTQPNLPKEKIAD |
| gas57M28_10164 | (597) | GDIYSTYNDNHYGSQTGTSMASPQIAGASLLVKQYLEKTQPNLPKEKIAD |
| gas57M28_10218 | (597) | GDIYSTYNDNHYGSQTGTSMASPQIAGASLLVKQYLEKTQPNLPKEKIAD |
| gas57M28_10266 | (597) | GDIYSTYNDNHYGSQTGTSMASPQIAGASLLVKQYLEKTQPNLPKEKIAD |
| gas57M28_10299 | (597) | GDIYSTYNDNHYGSQTGTSMASPQIAGASLLVKQYLEKTQPNLPKEKIAD |
| gas57M28_30176 | (597) | GDIYSTYNDNHYGSQTGTSMASPQIAGASLLVKQYLEKTQPNLPKEKIAD |
| gas57M28_30574 | (597) | GDIYSTYNDNHYGSQTGTSMASPQIAGASLLVKQYLEKTQPNLPKEKIAD |
| gas57M6,9_21802 | (600) | GDIYSTYNDNHYGSQTGTSMASPQIAGASLLVKQYLEKTQPNLPKEKIAD |
| gas57M75_10012 | (601) | GDIYSTYNDNHYGSQTGTSMASPQIAGASLLVKQYLEKTQPNLPKEKIAD |
| gas57M75_20671 | (601) | GDIYSTYNDNHYGSQTGTSMASPQIAGASLLVKQYLEKTQPNLPKEKIAD |
| gas57M75_30603 | (601) | GDIYSTYNDNHYGSQTGTSMASPQIAGASLLVKQYLEKTQPNLPKEKIAD |
| gas57M75_30207 | (601) | GDIYSTYNDNHYGSQTGTSMASPQIAGASLLVKQYLEKTQPNLPKEKIAD |
| gas57M22_20641 | (600) | GDIYSTYNDNHYGSQTGTSMASPQIAGASLLVKQYLEKTQPNLPKEKIAD |
| gas57M22_23465 | (600) | GDIYSTYNDNHYGSQTGTSMASPQIAGASLLVKQYLEKTQPNLPKEKIAD |
| gas57M3,1_30610 | (600) | GDIYSTYNDNHYGSQTGTSMASPQIAGASLLVKQYLEKTQPNLPKEKIAD |
| gas57M3,1_40603 | (600) | GDIYSTYNDNHYGSQTGTSMASPQIAGASLLVKQYLEKTQPNLPKEKIAD |
| gas57M3,28_24214 | (600) | GDIYSTYNDNHYGSQTGTSMASPQIAGASLLVKQYLEKTQPNLPKEKIAD |
| gas57M3,34_10307 | (600) | GDIYSTYNDNHYGSQTGTSMASPQIAGASLLVKQYLEKTQPNLPKEKIAD |
| gas57M4_40427 | (600) | GDIYSTYNDNHYGSQTGTSMASPQIAGASLLVKQYLEKTQPNLPKEKIAD |
| gas57M3_2721 | (600) | GDIYSTYNDNHYGSQTGTSMASPQIAGASLLVKQYLEKTQPNLPKEKIAD |
| gas57M12_10296 | (599) | GDIYSTYNDNHYGSQTGTSMASPQIAGASLLVKQYLEKTQPNLPKEKIAD |
| gas57M12_10035 | (599) | GDIYSTYNDNHYGSQTGTSMASPQIAGASLLVKQYLEKTQPNLPKEKIAD |
| gas57M12_20069 | (599) | GDIYSTYNDNHYGSQTGTSMASPQIAGASLLVKQYLEKTQPNLPKEKIAD |
| gas57M12_22432 | (599) | GDIYSTYNDNHYGSQTGTSMASPQIAGASLLVKQYLEKTQPNLPKEKIAD |
| gas57M4_40499 | (599) | GDIYSTYNDNHYGSQTGTSMASPQIAGASLLVKQYLEKTQPNLPKEKIAD |
| gas57M6,1_21259 | (599) | GDIYSTYNDNHYGSQTGTSMASPQIAGASLLVKQYLEKTQPNLPKEKIAD |

FIG. 31N

| | | 651 700 |
|---|---|---|
| gas57M1_SF370 | (649) | IVKNLLMSNAQIHVNPETKTTTSPRQQGAGLLNIDGAVTSGLYVTGKDNY |
| gas57M1_31075 | (649) | IVKNLLMSNAQIHVNPETKTTTSPRQQGAGLLNIDGAVTSGLYVTGKDNY |
| gas57M1_31237 | (649) | IVKNLLMSNAQIHVNPETKTTTSPRQQGAGLLNIDGAVTSGLYVTGKDNY |
| gas57M1_3348 | (649) | IVKNLLMSNAQIHVNPETKTTTSPRQQGAGLLNIDGAVTSGLYVTGKDNY |
| gas57M2_34585 | (649) | IVKNLLMSNAQIHVNPETKTTTSPRQQGAGLLNIDGAVTSGLYVTGKDNY |
| gas57M3,1_21398 | (649) | IVKNLLMSNAQIHVNPETKTTTSPRQQGAGLLNIDGAVTSGLYVTGKDNY |
| gas57M44-61_20839 | (649) | IVKNLLMSNAQIHVNPETKTTTSPRQQGAGLLNIDGAVTSGLYVTGKDNY |
| gas57M6,31_20022 | (648) | IVKNLLMSNAQIHVNPETKTTTSPRQQGAGLLNIDGAVTSGLYVTGKDNY |
| gas57M11_20648 | (647) | IVKNLLMSNAQIHVNPETKTTTSPRQQGAGLLNIDGAVTSGLYVTGKDNY |
| gas57M23_2071 | (648) | IVKNLLMSNAQIHVNPETKTTTSPRQQGAGLLNIDGAVTSGLYVTGKDNY |
| gas57M18,3_40128 | (649) | IVKNLLMSNAQIHVNPETKTTTSPRQQGAGLLNIDGAVTSGLYVTGKDNY |
| gas57M4_10092 | (649) | IVKNLLMSNAQIHVNPETKTTTSPRQQGAGLLNIDGAVTSGLYVTGKDNY |
| gas57M4_30968 | (649) | IVKNLLMSNAQIHVNPETKTTTSPRQQGAGLLNIDGAVTSGLYVTGKDNY |
| gas57M6,31_22692 | (649) | IVKNLLMSNAQIHVNPETKTTTSPRQQGAGLLNIDGAVTSGLYVTGKDNY |
| gas57M68,5_22814 | (648) | IVKNLLMSNAQIHVNPETKTTTSPRQQGAGLLNIDGAVTSGLYVTGKDNY |
| gas57M68_23623 | (649) | IVKNLLMSNAQIHVNPETKTTTSPRQQGAGLLNIDGAVTSGLYVTGKDNY |
| gas57M2_10064 | (647) | IVKNLLMSNAQIHVNPETKTTTSPRQQGAGLLNIDGAVTSGLYVTGKDNY |
| gas57M2_10065 | (647) | IVKNLLMSNAQIHVNPETKTTTSPRQQGAGLLNIDGAVTSGLYVTGKDNY |
| gas57M77_10251 | (647) | IVKNLLMSNAQIHVNPETKTTTSPRQQGAGLLNIDGAVTSGLYVTGKDNY |
| gas57M77_10527 | (647) | IVKNLLMSNAQIHVNPETKTTTSPRQQGAGLLNIDGAVTSGLYVTGKDNY |
| gas57M77_20696 | (647) | IVKNLLMSNAQIHVNPETKTTTSPRQQGAGLLNIDGAVTSGLYVTGKDNY |
| gas57M89_21915 | (649) | IVKNLLMSNAQIHVNPETKTTTSPRQQGAGLLNIDGAVTSGLYVTGKDNY |
| gas57M89_23717 | (649) | IVKNLLMSNAQIHVNPETKTTTSPRQQGAGLLNIDGAVTSGLYVTGKDNY |
| gas57M94_10134 | (647) | IVKNLLMSNAQIHVNPETKMTTSPRQQGAGLLNIDGAVTSGLYVTGKDNY |
| gas57M28_10164 | (647) | IVKNLLMSNAQIHVNPETKTTTSPRQQGAGLLNIDGAVTSGLYVTGKDNY |
| gas57M28_10218 | (647) | IVKNLLMSNAQIHVNPETKTTTSPRQQGAGLLNIDGAVTSGLYVTGKDNY |
| gas57M28_10266 | (647) | IVKNLLMSNAQIHVNPETKTTTSPRQQGAGLLNIDGAVTSGLYVTGKDNY |
| gas57M28_10299 | (647) | IVKNLLMSNAQIHVNPETKTTTSPRQQGAGLLNIDGAVTSGLYVTGKDNY |
| gas57M28_30176 | (647) | IVKNLLMSNAQIHVNPETKTTTSPRQQGAGLLNIDGAVTSGLYVTGKDNY |
| gas57M28_30574 | (647) | IVKNLLMSNAQIHVNPETKTTTSPRQQGAGLLNIDGAVTSGLYVTGKDNY |
| gas57M6,9_21802 | (650) | IVKNLLMSNAQIHVNPETKTTTSPRQQGAGLLNIDGAVTSGLYVTGKDNY |
| gas57M75_10012 | (651) | IVKNLLMSNAQIHVNPETKTTTSPRQQGAGLLNIDGAVTSGLYVTGKDNY |
| gas57M75_20671 | (651) | IVKNLLMSNAQIHVNPETKTTTSPRQQGAGLLNIDGAVTSGLYVTGKDNY |
| gas57M75_30603 | (651) | IVKNLLMSNAQIHVNPETKTTTSPRQQGAGLLNIDGAVTSGLYVTGKDNY |
| gas57M75_30207 | (651) | IVKNLLMSNAQIHVNPETKTTTSPRQQGAGLLNIDGAVTSGLYVTGKDNY |
| gas57M22_20641 | (650) | IVKNLLMSNAQIHVNPETKTTTSPRQQGAGLLNIDGAVTSGLYVTGKDNY |
| gas57M22_23465 | (650) | IVKNLLMSNAQIHVNPETKTTTSPRQQGAGLLNIDGAVTSGLYVTGKDNY |
| gas57M3,1_30610 | (650) | IVKNLLMSNAQIHVNPETKTTTSPRQQGAGLLNIDGAVTSGLYVTGKDNY |
| gas57M3,1_40603 | (650) | IVKNLLMSNAQIHVNPETKTTTSPRQQGAGLLNIDGAVTSGLYVTGKDNY |
| gas57M3,28_24214 | (650) | IVKNLLMSNAQIHVNPETKTTTSPRQQGAGLLNIDGAVTSGLYVTGKDNY |
| gas57M3,34_10307 | (650) | IVKNLLMSNAQIHVNPETKTTTSPRQQGAGLLNIDGAVTSGLYVTGKDNY |
| gas57M4_40427 | (650) | IVKNLLMSNAQIHVNPETKTTTSPRQQGAGLLNIDGAVTSGLYVTGKDNY |
| gas57M3_2721 | (650) | IVKNLLMSNAQIHVNPETKTTTSPRQQGAGLLNIDGAVTSGLYVTGKDNY |
| gas57M12_10296 | (649) | IVKNLLMSNAQIHVNPETKTTTSPRQQGAGLLNIDGAVTSGLYVTGKDNY |
| gas57M12_10035 | (649) | IVKNLLMSNAQIHVNPETKTTTSPRQQGAGLLNIDGAVTSGLYVTGKDNY |
| gas57M12_20069 | (649) | IVKNLLMSNAQIHVNPETKTTTSPRQQGAGLLNIDGAVTSGLYVTGKDNY |
| gas57M12_22432 | (649) | IVKNLLMSNAQIHVNPETKTTTSPRQQGAGLLNIDGAVTSGLYVTGKDNY |
| gas57M4_40499 | (649) | IVKNLLMSNAQIHVNPETKTTTSPRQQGAGLLNIDGAVTSGLYVTGKDNY |
| gas57M6,1_21259 | (649) | IVKNLLMSNAQIHVNPETKTTTSPRQQGAGLLNIDGAVTSGLYVTGKDNY |

FIG. 31O

| | | 701 750 |
|---|---|---|
| gas57M1_SF370 | (699) | GSISLGNITDTMTFDVTVHNLSNKDKTLRYDTELLTDHVDPQKGRFTLTS |
| gas57M1_31075 | (699) | GSISLGNITDTMTFDVTVHNLSNKDKTLRYDTELLTDHVDPQKGRFTLTS |
| gas57M1_31237 | (699) | GSISLGNITDTMTFDVTVHNLSNKDKTLRYDTELLTDHVDPQKGRFTLTS |
| gas57M1_3348 | (699) | GSISLGNITDTMTFDVTVHNLSNKDKTLRYDTELLTDHVDPQKGRFTLTS |
| gas57M2_34585 | (699) | GSISLGNITDTMTFDVTVHNLSNKDKTLRYDTELLTDHVDPQKGRFTLTS |
| gas57M3,1_21398 | (699) | GSISLGNITDTMTFDVTVHNLSNKAKTLRYDTELLTDHVDPQKGRFTLTS |
| gas57M44-61_20839 | (699) | GSISLGNVTDTMTFDVTVHNLSNKAKTLRYDTELLTDHVDPQKGRFTLTS |
| gas57M6,31_20022 | (698) | GSISLGNVTDTMTFDVTVHNLSNKAKTLRYDTELLTDHVDPQKGRFTLTS |
| gas57M11_20648 | (697) | GSISLGNITDTMTFDVTVHNLSNKAKTLRYDTELLTDHVDPQKGRFTLTS |
| gas57M23_2071 | (698) | GSISLGNITDTMTFDVTVHNLSNKAKTLRYDTELLTDHVDPQKGRFTLTS |
| gas57M18,3_40128 | (699) | GSISLGNITDTMTFDVTVHNLSNKDKTLRYDTELLTDHVDPQKGRFTLTS |
| gas57M4_10092 | (699) | GSISLGNITDTMTFDVTVHNLSNKDKTLRYDTELLTDHVDPQKGRFTLTS |
| gas57M4_30968 | (699) | GSISLGNITDTMTFDVTVHNLSNKDKTLRYDTELLTDHVDPQKGRFTLTS |
| gas57M6,31_22692 | (699) | GSISLGNITDTMTFDVTVHNLSNKDKTLRYDTELLTDHVDPQKGRFTLTS |
| gas57M68,5_22814 | (698) | GSISLGNITDTMTFDVTVHNLSNKDKTLRYDTELLTDHVDPQKGRFTLTS |
| gas57M68_23623 | (699) | GSISLGNITDTMTFDVTVHNLSNKDKTLRYDTELLTDHVDPQKGRFTLTS |
| gas57M2_10064 | (697) | GSISLGNITDTMTFDVTVHNLSNKAKTLRYDTELLTDHVDPQKGRFTLTS |
| gas57M2_10065 | (697) | GSISLGNITDTMTFDVTVHNLSNKAKTLRYDTELLTDHVDPQKGRFTLTS |
| gas57M77_10251 | (697) | GSISLGNITDTMTFDVTVHNLSNKAKTLRYDTELLTDHVDPQKGRFTLTS |
| gas57M77_10527 | (697) | GSISLGNITDTMTFDVTVHNLSNKAKTLRYDTELLTDHVDPQKGRFTLTS |
| gas57M77_20696 | (697) | GSISLGNITDTMTFDVTVHNLSNKAKTLRYDTELLTDHVDPQKGRFTLTS |
| gas57M89_21915 | (699) | GSISLGNITDTMTFDVTVHNLSNKDKTLRYDTELLTDHVDPQKGRFTLTS |
| gas57M89_23717 | (699) | GSISLGNITDTMTFDVTVHNLSNKDKTLRYDTELLTDHVDPQKGRFTLTS |
| gas57M94_10134 | (697) | GSISLGNITDTMTFDVTVHNLSNKAKTLRYDTELLTDHVDPQKGHFTLTS |
| gas57M28_10164 | (697) | GSISLGNITDTMTFDVTVHNLSNKAKTLRYDTELLTDHVDPQKGRFTLTS |
| gas57M28_10218 | (697) | GSISLGNITDTMTFDVTVHNLSNKAKTLRYDTELLTDHVDPQKGRFTLTS |
| gas57M28_10266 | (697) | GSISLGNITDTMTFDVTVHNLSNKAKTLRYDTELLTDHVDPQKGRFTLTS |
| gas57M28_10299 | (697) | GSISLGNITDTMTFDVTVHNLSNKAKTLRYDTELLTDHVDPQKGRFTLTS |
| gas57M28_30176 | (697) | GSISLGNITDTMTFDVTVHNLSNKAKTLRYDTELLTDHVDPQKGRFTLTS |
| gas57M28_30574 | (697) | GSISLGNITDTMTFDVTVHNLSNKAKTLRYDTELLTDHVDPQKGRFTLTS |
| gas57M6,9_21802 | (700) | GSISLGNITDTMTFDVTVHNLSNKAKTLRYDTELLTDHVDPQKGRFTLTS |
| gas57M75_10012 | (701) | GSISLGNITDTMTFDVTVHNLSNKDKTLRYDTELLTDHVDPQKGRFTLTS |
| gas57M75_20671 | (701) | GSISLGNITDTMTFDVTVHNLSNKDKTLRYDTELLTDHVDPQKGRFTLTS |
| gas57M75_30603 | (701) | GSISLGNITDTMTFDVTVHNLSNKDKTLRYDTELLTDHVDPQKGRFTLTS |
| gas57M75_30207 | (701) | GSISLGNITDTMTFDVTVHNLSNKDKTLRYDTELLTDHVDPQKGRFTLTS |
| gas57M22_20641 | (700) | GSISLGNITDTMTFDVTVHNLSNKDKTLRYDTELLTDHVDPQKGRFTLTS |
| gas57M22_23465 | (700) | GSISLGNITDTMTFDVTVHNLSNKDKTLRYDTELLTDHVDPQKGRFTLTS |
| gas57M3,1_30610 | (700) | GSISLGNITDTMTFDVTVHNLSNKAKTLRYDTELLTDHVDPQKGRFTLTS |
| gas57M3,1_40603 | (700) | GSISLGNITDTMTFDVTVHNLSNKAKTLRYDTELLTDHVDPQKGRFTLTS |
| gas57M3,28_24214 | (700) | GSISLGNITDTMTFDVTVHNLSNKAKTLRYDTELLTDHVDPQKGRFTLTS |
| gas57M3,34_10307 | (700) | GSISLGNITDTMTFDVTVHNLSNKAKTLRYDTELLTDHVDPQKGRFTLTS |
| gas57M4_40427 | (700) | GSISLGNITDTMTFDVTVHNLSNKAKTLRYDTELLTDHVDPQKGRFTLTS |
| gas57M3_2721 | (700) | GSISLGNITDTMTFDVTVHNLSNKAKTLRYDTELLTDHVDPQKGRFTLTS |
| gas57M12_10296 | (699) | GSISLGNITDTMTFDVTVHNLSNKAKTLRYDTELLTDHVDPQKGRFTLTS |
| gas57M12_10035 | (699) | GSISLGNITDTMTFDVTVHNLSNKAKTLRYDTELLTDHVDPQKGRFTLTS |
| gas57M12_20069 | (699) | GSISLGNITDTMTFDVTVHNLSNKAKTLRYDTELLTDHVDPQKGRFTLTS |
| gas57M12_22432 | (699) | GSISLGNITDTMTFDVTVHNLSNKAKTLRYDTELLTDHVDPQKGRFTLTS |
| gas57M4_40499 | (699) | GSISLGNITDTMTFDVTVHNLSNKAKTLRYDTELLTDHVDPQKGRFTLTS |
| gas57M6,1_21259 | (699) | GSISLGNITDTMTFDVTVHNLSNKAKTLRYDTELLTDHVDPQKGRFTLTS |

FIG. 31P

```
                        751                                              800
     gas57M1_SF370   (749) HSLKTYQGGEVTVPANGKVTVRVTMDVSQFTKELTKQMPNGYYLEGFVRF
     gas57M1_31075   (749) HSLKTYQGGEVTVPANGKVTVRVTMDVSQFTKELTKQMPNGYYLEGFVRF
     gas57M1_31237   (749) HSLKTYQGGEVTVPANGKVTVRVTMDVSQFTKELTKQMPNGYYLEGFVRF
      gas57M1_3348   (749) HSLKTYQGGEVTVPANGKVTVRVTMDVSQFTKELTKQMPNGYYLEGFVRF
     gas57M2_34585   (749) HSLKTYQGGEVTVPANGKVTVRVTMDVSQFTKELTKQMPNGYYLEGFVRF
   gas57M3,1_21398   (749) RSLKTYQGGEVTVPANGKVTVKVTMDVSQFTKELTKQMPNGYYLEGFVRF
gas57M44-61_20839    (749) RSLKTYQGGEVTVPANGKVTVRVTMDVSQFTKELTKQMPNGYYLEGFVRF
  gas57M6,31_20022   (748) RSLKTYQGGEVTVPANGKVTVRVTMDVSQFTKELTKQMPNGYYLEGFVRF
    gas57M11_20648   (747) RSLKTYQGGEVTVPANGKVTVRVTMDVSQFTKELTKQMPNGYYLEGFVRF
     gas57M23_2071   (748) RSLKTYQGGEVTVPANGKVTVKVTMDVSQFTKELTKQMPNGYYLEGFVRF
  gas57M18,3_40128   (749) RSLKTYQGGEVTVPANGKVTVRVTMDVSQFTKELTKQMPNGYYLEGFVRF
     gas57M4_10092   (749) RSLKTYQGGEVTVPANGKVTVRVTMDVSQFTKELTKQMPNGYYLEGFVRF
     gas57M4_30968   (749) RSLKTYQGGEVTVPANGKVTVRVTMDVSQFTKELTKQMPNGYYLEGFVRF
  gas57M6,31_22692   (749) RSLKTYQGGEVTVPANGKVTVRVTMDVSQFTKELTKQMPNGYYLEGFVRF
  gas57M68,5_22814   (748) RSLKTYQGGEVTVPANGKVTVRVTMDVSQFTKELTKQMPNGYYLEGFVRF
    gas57M68_23623   (749) RSLKTYQGGEVTVPANGKVTVRVTMDVSQFTKELTKQMPNGYYLEGFVRF
     gas57M2_10064   (747) RSLKTYQGGEVTVPANGKVTVRVTMDVSQFTKELTKQMPNGYYLEGFVRF
     gas57M2_10065   (747) RSLKTYQGGEVTVPANGKVTVRVTMDVSQFTKELTKQMPNGYYLEGFVRF
    gas57M77_10251   (747) RSLKTYQGGEVTVPANGKVTVKVTMDVSQFTKELTKQMPNGYYLEGFVRF
    gas57M77_10527   (747) RSLKTYQGGEVTVPANGKVTVKVTMDVSQFTKELTKQMPNGYYLEGFVRF
    gas57M77_20696   (747) RSLKTYQGGEVTVPANGKVTVKVTMDVSQFTKELTKQMPNGYYLEGFVRF
    gas57M89_21915   (749) RSLKTYQGGEVTVPANGKVTVRVTMDVSQFTKELTKQMPNGYYLEGFVRF
    gas57M89_23717   (749) RSLKTYQGGEVTVPANGKVTVRVTMDVSQFTKELTKQMPNGYYLEGFVRF
    gas57M94_10134   (747) RSLKTYQGGEVTVPANGKVTVRVTMDVSQFTKELTKQMPNGYYLEGFVRF
    gas57M28_10164   (747) RSLKTYQGGEVTVPANGKVTVRVTMDVSQFTKELTKQMPNGYYLEGFVRF
    gas57M28_10218   (747) RSLKTYQGGEVTVPANGKVTVRVTMDVSQFTKELTKQMPNGYYLEGFVRF
    gas57M28_10266   (747) RSLKTYQGGEVTVPANGKVTVRVTMDVSQFTKELTKQMPNGYYLEGFVRF
    gas57M28_10299   (747) RSLKTYQGGEVTVPANGKVTVRVTMDVSQFTKELTKQMPNGYYLEGFVRF
    gas57M28_30176   (747) RSLKTYQGGEVTVPANGKVTVRVTMDVSQFTKELTKQMPNGYYLEGFVRF
    gas57M28_30574   (747) RSLKTYQGGEVTVPANGKVTVRVTMDVSQFTKELTKQMPNGYYLEGFVRF
   gas57M6,9_21802   (750) RSLKTYQGGEVTVPANGKVTVRVTMDVSQFTKELTKQMPNGYYLEGFVRF
    gas57M75_10012   (751) RSLKTYQGGEVTVPANGKVTVRVTMDVSQFTKELTKQMPNGYYLEGFVRF
    gas57M75_20671   (751) RSLKTYQGGEVTVPANGKVTVRVTMDVSQFTKELTKQMPNGYYLEGFVRF
    gas57M75_30603   (751) RSLKTYQGGEVTVPANGKVTVRVTMDVSQFTKELTKQMPNGYYLEGFVRF
    gas57M75_30207   (751) RSLKTYQGGEVTVPANGKVTVRVTMDVSQFTKELTKQMPNGYYLEGFVRF
    gas57M22_20641   (750) RSLKTYQGGEVTVPANGKVTVRVTMDVSQFTKELTKQMPNGYYLEGFVRF
    gas57M22_23465   (750) RSLKTYQGGEVTVPANGKVTVRVTMDVSQFTKELTKQMPNGYYLEGFVRF
   gas57M3,1_30610   (750) RSLKTYQGGEVTVPANGKVTVRVTMDVSQFTKELTKQMPNGYYLEGFVRF
   gas57M3,1_40603   (750) RSLKTYQGGEVTVPANGKVTVRVTMDVSQFTKELTKQMPNGYYLEGFVRF
  gas57M3,28_24214   (750) RSLKTYQGGEVTVPANGKVTVRVTMDVSQFTKELTKQMPNGYYLEGFVRF
  gas57M3,34_10307   (750) RSLKTYQGGEVTVPANGKVTVRVTMDVSQFTKELTKQMPNGYYLEGFVRF
     gas57M4_40427   (750) RSLKTYQGGEVTVPANGKVTVRVTMDVSQFTKELTKQMPNGYYLEGFVRF
      gas57M3_2721   (750) RSLKTYQGGEVTVPANGKVTVRVTMDVSQFTKELTKQMPNGYYLEGFVRF
    gas57M12_10296   (749) RSLKTYQGGEVTVPANGKVTVRVTMDVSQFTKELTKQMPNGYYLEGFVRF
    gas57M12_10035   (749) RSLKTYQGGEVTVPANGKVTVRVTMDVSQFTKELTKQMPNGYYLEGFVRF
    gas57M12_20069   (749) RSLKTYQGGEVTVPANGKVTVRVTMDVSQFTKELTKQMPNGYYLEGFVRF
    gas57M12_22432   (749) RSLKTYQGGEVTVPANGKVTVRVTMDVSQFTKELTKQMPNGYYLEGFVRF
     gas57M4_40499   (749) RSLKTYQGGEVTVPANGKVTVRVTMDVSQFTKELTKQMPNGYYLEGFVRF
   gas57M6,1_21259   (749) RSLKTYQGGEVTVPANGKVTVRVTMDVSQFTKELTKQMPNGYYLEGFVRF
```

FIG. 31Q

| | | 801 850 |
|---|---|---|
| gas57M1_SF370 | (799) | RDSQDDQLNRVNIPFVGFKGQFENLAVAEESIYRLKSQGKTGFYFDESGP |
| gas57M1_31075 | (799) | RDSQDDQLNRVNIPFVGFKGQFENLAVAEESIYRLKSQGKTGFYFDESGP |
| gas57M1_31237 | (799) | RDSQDDQLNRVNIPFVGFKGQFENLAVAEESIYRLKSQGKTGFYFDESGP |
| gas57M1_3348 | (799) | RDSQDDQLNRVNIPFVGFKGQFENLAVAEESIYRLKSQGKTGFYFDESGP |
| gas57M2_34585 | (799) | RDSQDDQLNRVNIPFVGFKGQFENLAVAEESIYRLKSQGKTGFYFDESGP |
| gas57M3,1_21398 | (799) | RDSQDDQLNRVNIPFVGFKGQFENLAVAEESIYRLKSQGKTGFYFDESGP |
| gas57M44-61_20839 | (799) | RDSQDDQLNRVNIPFVGFKGQFENLAVAEESIYRLKSQGKTGFYFDESGP |
| gas57M6,31_20022 | (798) | RDSQDAQLNRVNIPFVGFKGQFENLAVAEESIYRLKSQGKTGFYFDESGP |
| gas57M11_20648 | (797) | RDSQDDQLNRVNIPFVGFKGQFENLAVAEESIYRLKSQGKTGFYFDESGP |
| gas57M23_2071 | (798) | RDSQDAQLNRVNIPFVGFKGQFENLAVAEESIYRLKSQGKTGFYFDESGP |
| gas57M18,3_40128 | (799) | RDSQDDQLNRVNIPFVGFKGQFENLAVAEESIYRLKSQGKTGFYFDESGP |
| gas57M4_10092 | (799) | RDSQDDQLNRVNIPFVGFKGQFENLAVAEESIYRLKSQGKTGFYFDESGP |
| gas57M4_30968 | (799) | RDSQDDQLNRVNIPFVGFKGQFENLAVAEESIYRLKSQGKTGFYFDESGP |
| gas57M6,31_22692 | (799) | RDSQDDQLNRVNIPFVGFKGQFENLAVAEESIYRLKSQGKTGFYFDESGP |
| gas57M68,5_22814 | (798) | RDSQDDQLNRVNIPFVGFKGQFENLAVAEESIYRLKSQGKTGFYFDESGP |
| gas57M68_23623 | (799) | RDSQDDQLNRVNIPFVGFKGQFENLAVAEESIYRLKSQGKTGFYFDESGP |
| gas57M2_10064 | (797) | RDSQDDQLNRVNIPFVGFKGQFENLAVAEESIYRLKSQGKTGFYFDESGP |
| gas57M2_10065 | (797) | RDSQDDQLNRVNIPFVGFKGQFENLAVAEESIYRLKSQGKTGFYFDESGP |
| gas57M77_10251 | (797) | RDSQDDQLNRVNIPFVGFKGQFENLAVAEESIYRLKSQGKTGFYFDESGP |
| gas57M77_10527 | (797) | RDSQDDQLNRVNIPFVGFKGQFENLAVAEESIYRLKSQGKTGFYFDESGP |
| gas57M77_20696 | (797) | RDSQDDQLNRVNIPFVGFKGQFENLAVAEESIYRLKSQGKTGFYFDESGP |
| gas57M89_21915 | (799) | RDSQDDQLNRVNIPFVGFKGQFENLAVAEESIYRLKSQGKTGFYFDESGP |
| gas57M89_23717 | (799) | RDSQDDQLNRVNIPFVGFKGQFENLAVAEESIYRLKSQGKTGFYFDESGP |
| gas57M94_10134 | (797) | RDSQDDQLNRVNIPFVGFKGQFENLAVAEESIYRLKSQGKTGFYFDESGP |
| gas57M28_10164 | (797) | RDSQDDQLNRVNIPFVGFKGQFENLAVAEESIYRLKSQGKTGFYFDESGP |
| gas57M28_10218 | (797) | RDSQDDQLNRVNIPFVGFKGQFENLAVAEESIYRLKSQGKTGFYFDESGP |
| gas57M28_10266 | (797) | RDSQDDQLNRVNIPFVGFKGQFENLAVAEESIYRLKSQGKTGFYFDESGP |
| gas57M28_10299 | (797) | RDSQDDQLNRVNIPFVGFKGQFENLAVAEESIYRLKSQGKTGFYFDESGP |
| gas57M28_30176 | (797) | RDSQDDQLNRVNIPFVGFKGQFENLAVAEESIYRLKSQGKTGFYFDESGP |
| gas57M28_30574 | (797) | RDSQDDQLNRVNIPFVGFKGQFENLAVAEESIYRLKSQGKTGFYFDESGP |
| gas57M6,9_21802 | (800) | RDSQDDQLNRVNIPFVGFKGQFENLAVAEESIYRLKSQGKTGFYFDESGP |
| gas57M75_10012 | (801) | RDSQDDQLNRVNIPFVGFKGQFENLAVAEESIYRLKSQGKTGFYFDESGP |
| gas57M75_20671 | (801) | RDSQDDQLNRVNIPFVGFKGQFENLAVAEESIYRLKSQGKTGFYFDESGP |
| gas57M75_30603 | (801) | RDSQDDQLNRVNIPFVGFKGQFENLAVAEESIYRLKSQGKTGFYFDESGP |
| gas57M75_30207 | (801) | RDSQDDQLNRVNIPFVGFKGQFENLAVAEESIYRLKSQGKTGFYFDESGP |
| gas57M22_20641 | (800) | RDSQDAQLNRVNIPFVGFKGQFENLAVAEESIYRLKSQGKTGFYFDESGP |
| gas57M22_23465 | (800) | RDSQDDQLNRVNIPFVGFKGQFENLAVAEESIYRLKSQGKTGFYFDESGP |
| gas57M3,1_30610 | (800) | RDSQDDQLNRVNIPFVGFKGQFENLAVAEESIYRLKSQGKTGFYFDESGP |
| gas57M3,1_40603 | (800) | RDSQDDQLNRVNIPFVGFKGQFENLAVAEESIYRLKSQGKTGFYFDESGP |
| gas57M3,28_24214 | (800) | RDSQDDQLNRVNIPFVGFKGQFENLAVAEESIYRLKSQGKTGFYFDESGP |
| gas57M3,34_10307 | (800) | RDSQDDQLNRVNIPFVGFKGQFENLAVAEESIYRLKSQGKTGFYFDESGP |
| gas57M4_40427 | (800) | RDSQDDQLNRVNIPFVGFKGQFENLAVAEESIYRLKSQGKTGFYFDESGP |
| gas57M3_2721 | (800) | RDSQDDQLNRVNIPFVGFKGQFENLAVAEESIYRLKSQGKTGFYFDESGP |
| gas57M12_10296 | (799) | RDSQDDQLNRVNIPFVGFKGQFENLAVAEESIYRLKSQGKTGFYFDESGP |
| gas57M12_10035 | (799) | RDSQDDQLNRVNIPFVGFKGQFENLAVAEESIYRLKSQGKTGFYFDESGP |
| gas57M12_20069 | (799) | RDSQDDQLNRVNIPFVGFKGQFENLAVAEESIYRLKSQGKTGFYFDESGP |
| gas57M12_22432 | (799) | RDSQDDQLNRVNIPFVGFKGQFENLAVAEESIYRLKSQGKTGFYFDESGP |
| gas57M4_40499 | (799) | RDSQDDQLNRVNIPFVGFKGQFENLAVAEESIYRLKSQGKTGFYFDESGP |
| gas57M6,1_21259 | (799) | RDSQDDQLNRVNIPFVGFKGQFENLAVAEESIYRLKSQGKTGFYFDESGP |

FIG. 31R

| | | 851 900 |
|---|---|---|
| gas57M1_SF370 | (849) | KDDIYVGKHFTGLVTLGSETNVSTKTISDNGLHTLGTFKNADGKFILEKN |
| gas57M1_31075 | (849) | KDDIYVGKHFTGLVTLGSETNVSTKTISDNGLHTLGTFKNADGKFILEKN |
| gas57M1_31237 | (849) | KDDIYVGKHFTGLVTLGSETNVSTKTISDNGLHTLGTFKNADGKFILEKN |
| gas57M1_3348 | (849) | KDDIYVGKHFTGLVTLGSETNVSTKTISDNGLHTLGTFKNADGKFILEKN |
| gas57M2_34585 | (849) | KDDIYVGKHFTGLVTLGSETNVSTKTISDNGLHTLGTFKNADGKFILEKN |
| gas57M3,1_21398 | (849) | KDDIYVGKHFTGLVTLGSETNVSTKTISDNGLHTLGTFKNADGKFILEKN |
| gas57M44-61_20839 | (849) | KDDIYVGKHFTGLVTLGSETNVSTKTISDNGLHTLGTFKNADGKFILEKN |
| gas57M6,31_20022 | (848) | KDDIYVGKHFTGLVTLGSETNVSTKTISDNGLHTLGTFKNADGKFILEKN |
| gas57M11_20648 | (847) | KDDIYVGKHFTGLVTLGSETNVSTKTISDNGLHTLGTFKNADGKFILEKN |
| gas57M23_2071 | (848) | KDDIYVGKHFTGLVTLGSETNVSTKTISDNGLHTLGTFKNADGKFILEKN |
| gas57M18,3_40128 | (849) | KDDIYVGKHFTGLVTLGSETNVSTKTISDNGLHTLGTFKNADGKFILEKN |
| gas57M4_10092 | (849) | KDDIYVGKHFTGLVTLGSETNVSTKTISDNGLHTLGTFKNADGKFILEKN |
| gas57M4_30968 | (849) | KDDIYVGKHFTGLVTLGSETNVSTKTISDNGLHTLGTFKNADGKFILEKN |
| gas57M6,31_22692 | (849) | KDDIYVGKHFTGLVTLGSETNVSTKTISDNGLHTLGTFKNADGKFILEKN |
| gas57M68,5_22814 | (848) | KDDIYVGKHFTGLVTLGSETNVSTKTISDNGLHTLGTFKNADGKFILEKN |
| gas57M68_23623 | (849) | KDDIYVGKHFTGLVTLGSETNVSTKTISDNGLHTLGTFKNADGKFILEKN |
| gas57M2_10064 | (847) | KDDIYVGKHFTGLVTLGSETNVSTKTISDNGLHTLGTFKNADGKFILEKN |
| gas57M2_10065 | (847) | KDDIYVGKHFTGLVTLGSETNVSTKTISDNGLHTLGTFKNADGKFILEKN |
| gas57M77_10251 | (847) | KDDIYVGKHFTGLVTLGSETNVSTKTISDNGLHTLGTFKNADGKFILEKN |
| gas57M77_10527 | (847) | KDDIYVGKHFTGLVTLGSETNVSTKTISDNGLHTLGTFKNADGKFILEKN |
| gas57M77_20696 | (847) | KDDIYVGKHFTGLVTLGSETNVSTKTISDNGLHTLGTFKNADGKFILEKN |
| gas57M89_21915 | (849) | KDDIYVGKHFTGLVTLGSETNVSTKTISDNGLHTLGTFKNADGKFILEKN |
| gas57M89_23717 | (849) | KDDIYVGKHFTGLVTLGSETNVSTKTISDNGLHTLGTFKNADGKFILEKN |
| gas57M94_10134 | (847) | KDDIYVGKHFTGLVTLGSETNVSTKTISDNGLHTLGTFKNADGKFILEKN |
| gas57M28_10164 | (847) | KDDIYVGKHFTGLVTLGSETNVSTKTISDNGLHTLGTFKNADGKFILEKN |
| gas57M28_10218 | (847) | KDDIYVGKHFTGLVTLGSETNVSTKTISDNGLHTLGTFKNADGKFILEKN |
| gas57M28_10266 | (847) | KDDIYVGKHFTGLVTLGSETNVSTKTISDNGLHTLGTFKNADGKFILEKN |
| gas57M28_10299 | (847) | KDDIYVGKHFTGLVTLGSETNVSTKTISDNGLHTLGTFKNADGKFILEKN |
| gas57M28_30176 | (847) | KDDIYVGKHFTGLVTLGSETNVSTKTISDNGLHTLGTFKNADGKFILEKN |
| gas57M28_30574 | (847) | KDDIYVGKHFTGLVTLGSETNVSTKTISDNGLHTLGTFKNADGKFILEKN |
| gas57M6,9_21802 | (850) | KDDIYVGKHFTGLVTLGSETNVSTKTISDNGLHTLGTFKNADGKFILEKN |
| gas57M75_10012 | (851) | KDDIYVGKHFTGLVTLGSETNVSTKTISDNGLHTLGTFKNADGKFILEKN |
| gas57M75_20671 | (851) | KDDIYVGKHFTGLVTLGSETNVSTKTISDNGLHTLGTFKNADGKFILEKN |
| gas57M75_30603 | (851) | KDDIYVGKHFTGLVTLGSETNVSTKTISDNGLHTLGTFKNADGKFILEKN |
| gas57M75_30207 | (851) | KDDIYVGKHFTGLVTLGSETNVSTKTISDNGLHTLGTFKNADGKFILEKN |
| gas57M22_20641 | (850) | KDDIYVGKHFTGLVTLGSETNVSTKTISDNGLHTLGTFKNADGKFILEKN |
| gas57M22_23465 | (850) | KDDIYVGKHFTGLVTLGSETNVSTKTISDNGLHTLGTFKNADGKFILEKN |
| gas57M3,1_30610 | (850) | KDDIYVGKHFTGLVTLGSETNVSTKMISDNGLHTLGTFKNADGKFILEKN |
| gas57M3,1_40603 | (850) | KDDIYVGKHFTGLVTLGSETNVSTKMISDNGLHTLGTFKNADGKFILEKN |
| gas57M3,28_24214 | (850) | KDDIYVGKHFTGLVTLGSETNVSTKMISDNGLHTLGTFKNADGKFILEKN |
| gas57M3,34_10307 | (850) | KDDIYVGKHFTGLVTLGSETNVSTKMISDNGLHTLGTFKNADGKFILEKN |
| gas57M4_40427 | (850) | KDDIYVGKHFTGLVTLGSETNVSTKMISDNGLHTLGTFKNADGKFILEKN |
| gas57M3_2721 | (850) | KDDIYVGKHFTGLVTLGSETNVSTKMISDNGLHTLGTFKNADGKFILEKN |
| gas57M12_10296 | (849) | KDDIYVGKHFTGLVTLGSETNVSTKTISDNGLHTLGTFKNADGKFILEKN |
| gas57M12_10035 | (849) | KDDIYVGKHFTGLVTLGSETNVSTKTISDNGLHTLGTFKNVDGKFILEKN |
| gas57M12_20069 | (849) | KDDIYVGKHFTGLVTLGSETNVSTKTISDNGLHTLGTFKNVDGKFILEKN |
| gas57M12_22432 | (849) | KDDIYVGKHFTGLVTLGSETNVSTKTISDNGLHTLGTFKNVDGKFILEKN |
| gas57M4_40499 | (849) | KDDIYVGKHFTGLVTLGSETNVSTKTISDNGLHTLGTFKNVDGKFILEKN |
| gas57M6,1_21259 | (849) | KDDIYVGKHFTGLVTLGSETNVSTKTISDNGLHTLGTFKNVDGKFILEKN |

FIG. 31S

| | | 901 950 |
|---|---|---|
| gas57M1_SF370 | (899) | AQGNPVLAISPNGDNNQDFAAFKGVFLRKYQGLKASVYHASDKEHKNPLW |
| gas57M1_31075 | (899) | AQGNPVLAISPNGDNNQDFAAFKGVFLRKYQGLKASVYHASDKEHKNPLW |
| gas57M1_31237 | (899) | AQGNPVLAISPNGDNNQDFAAFKGVFLRKYQGLKASVYHASDKEHKNPLW |
| gas57M1_3348 | (899) | AQGNPVLAISPNGDNNQDFAAFKGVFLRKYQGLKASVYHASDKEHKNPLW |
| gas57M2_34585 | (899) | AQGNPVLAISPNGDNNQDFAAFKGVFLRKYQGLKASVYHASDKEHKNPLW |
| gas57M3,1_21398 | (899) | AQGNPVLAISPNGDNNQDFAAFKGVFLRKYQGLKASVYHASDKEHKNPLW |
| gas57M44-61_20839 | (899) | AQGNPVLAISPNGDNNQDFAAFKGVFLRKYQGLKASVYHASDKEHKNPLW |
| gas57M6,31_20022 | (898) | AQGNPVLAISPNGDNNQDFAAFKGVFLRKYQGLKASVYHASDKEHKNPLW |
| gas57M11_20648 | (897) | AQGNPVLAISPNGDNNQDFAAFKGVFLRKYQGLKASVYHASDKEHKNPLW |
| gas57M23_2071 | (898) | AQGNPVLAISPNGDNNQDFAAFKGVFLRKYQGLKASVYHASDKEHKNPLW |
| gas57M18,3_40128 | (899) | AQGNPVLAISPNGDNNQDFAAFKGVFLRKYQGLKASVYHASDKEHKNPLW |
| gas57M4_10092 | (899) | AQGNPVLAISPNGDNNQDFAAFKGVFLRKYQGLKASVYHASDKEHKNPLW |
| gas57M4_30968 | (899) | AQGNPVLAISPNGDNNQDFAAFKGVFLRKYQGLKASVYHASDKEHKNPLW |
| gas57M6,31_22692 | (899) | AQGNPVLAISPNGDNNQDFAAFKGVFLRKYQGLKASVYHASDKEHKNPLW |
| gas57M68,5_22814 | (898) | AQGNPVLAISPNGDNNQDFAAFKGVFLRKYQGLKASVYHASDKEHKNPLW |
| gas57M68_23623 | (899) | AQGNPVLAISPNGDNNQDFAAFKGVFLRKYQGLKASVYHASDKEHKNPLW |
| gas57M2_10064 | (897) | AQGNPVLAISPNGDNNQDFAAFKGVFLRKYQGLKASVYHASDKEHKNPLW |
| gas57M2_10065 | (897) | AQGNPVLAISPNGDNNQDFAAFKGVFLRKYQGLKASVYHASDKEHKNPLW |
| gas57M77_10251 | (897) | AQGNPVLAISPNGDNNQDFAAFKGVFLRKYQGLKASVYHASDKEHKNPLW |
| gas57M77_10527 | (897) | AQGNPVLAISPNGDNNQDFAAFKGVFLRKYQGLKASVYHASDKEHKNPLW |
| gas57M77_20696 | (897) | AQGNPVLAISPNGDNNQDFAAFKGVFLRKYQGLKASVYHASDKEHKNPLW |
| gas57M89_21915 | (899) | AQGNPVLAISPNGDNNQDFAAFKGVFLRKYQGLKASVYHASDKEHKNPLW |
| gas57M89_23717 | (899) | AQGNPVLAISPNGDNNQDFAAFKGVFLRKYQGLKASVYHASDKEHKNPLW |
| gas57M94_10134 | (897) | AQGNPVLAISPNGDNNQDFAAFKGVFLRKYQGLKASVYHASDKEHKNPLW |
| gas57M28_10164 | (897) | AQGNPVLAISPNGDNNQDFAAFKGVFLRKYQGLKASVYHASDKEHKNPLW |
| gas57M28_10218 | (897) | AQGNPVLAISPNGDNNQDFAAFKGVFLRKYQGLKASVYHASDKEHKNPLW |
| gas57M28_10266 | (897) | AQGNPVLAISPNGDNNQDFAAFKGVFLRKYQGLKASVYHASDKEHKNPLW |
| gas57M28_10299 | (897) | AQGNPVLAISPNGDNNQDFAAFKGVFLRKYQGLKASVYHASDKEHKNPLW |
| gas57M28_30176 | (897) | AQGNPVLAISPNGDNNQDFAAFKGVFLRKYQGLKASVYHASDKEHKNPLW |
| gas57M28_30574 | (897) | AQGNPVLAISPNGDNNQDFAAFKGVFLRKYQGLKASVYHASDKEHKNPLW |
| gas57M6,9_21802 | (900) | AQGNPVLAISPNGDNNQDFAAFKGVFLRKYQGLKASVYHASDKEHKNPLW |
| gas57M75_10012 | (901) | AQGNPVLAISPNGDNNQDFAAFKGVFLRKYQGLKASVYHASDKEHKNPLW |
| gas57M75_20671 | (901) | AQGNPVLAISPNGDNNQDFAAFKGVFLRKYQGLKASVYHASDKEHKNPLW |
| gas57M75_30603 | (901) | AQGNPVLAISPNGDNNQDFAAFKGVFLRKYQGLKASVYHASDKEHKNPLW |
| gas57M75_30207 | (901) | AQGNPVLAISPNGDNNQDFAAFKGVFLRKYQGLKASVYHASDKEHKNPLW |
| gas57M22_20641 | (900) | AQGNPVLAISPNGDNNQDFAAFKGVFLRKYQGLKASVYHASDKEHKNPLW |
| gas57M22_23465 | (900) | AQGNPVLAISPNGDNNQDFAAFKGVFLRKYQGLKASVYHASDKEHKNPLW |
| gas57M3,1_30610 | (900) | AQGNPVLAISPNGDNNQDFAAFKGVFLRKYQGLKASVYHASDKEHKNPLW |
| gas57M3,1_40603 | (900) | AQGNPVLAISPNGDNNQDFAAFKGVFLRKYQGLKASVYHASDKEHKNPLW |
| gas57M3,28_24214 | (900) | AQGNPVLAISPNGDNNQDFAAFKGVFLRKYQGLKASVYHASDKEHKNPLW |
| gas57M3,34_10307 | (900) | AQGNPVLAISPNGDNNQDFAAFKGVFLRKYQGLKASVYHASDKEHKNPLW |
| gas57M4_40427 | (900) | AQGNPVLAISPNGDNNQDFAAFKGVFLRKYQGLKASVYHASDKEHKNPLW |
| gas57M3_2721 | (900) | AQGNPVLAISPNGDNNQDFAAFKGVFLRKYQGLKASVYHASDKEHKNPLW |
| gas57M12_10296 | (899) | AQGNPVLAISPNGDNNQDFAAFKGVFLRKYQGLKASVYHASDKEHKNPLW |
| gas57M12_10035 | (899) | AQGNPVLAISPNGDNNQDFAAFKGVFLRKYQGLKASVYHASDKEHKNPLW |
| gas57M12_20069 | (899) | AQGNPVLAISPNGDNNQDFAAFKGVFLRKYQGLKASVYHASDKEHKNPLW |
| gas57M12_22432 | (899) | AQGNPVLAISPNGDNNQDFAAFKGVFLRKYQGLKASVYHASDKEHKNPLW |
| gas57M4_40499 | (899) | AQGNPVLAISPNGDNNQDFAAFKGVFLRKYQGLKASVYHASDKEHKNPLW |
| gas57M6,1_21259 | (899) | AQGNPVLAISPNGDNNQDFAAFKGVFLRKYQGLKASVYHASDKEHKNPLW |

FIG. 31T

| | | |
|---|---|---|
| | | 951 1000 |
| gas57M1_SF370 | (949) | VSPESFKGDKNFNSDIRFAKSTTLLGTAFSGKSLTGAELPDGHYHYVVSY |
| gas57M1_31075 | (949) | VSPESFKGDKNFNSDIRFAKSTTLLGTAFSGKSLTGAELPDGHYHYVVSY |
| gas57M1_31237 | (949) | VSPESFKGDKNFNSDIRFAKSTTLLGTAFSGKSLTGAELPDGHYHYVVSY |
| gas57M1_3348 | (949) | VSPESFKGDKNFNSDIRFAKSTTLLGTAFSGKSLTGAELPDGHYHYVVSY |
| gas57M2_34585 | (949) | VSPESFKGDKNFNSDIRFAKSTTLLGTAFSGKSLTGAELPDGHYHYVVSY |
| gas57M3,1_21398 | (949) | VSPESFKGDKNFNSDIRFAKSTTLLGTAFSGKSLTGAELPDGYYHYVVSY |
| gas57M44-61_20839 | (949) | VSPESFKGDKNFNSDIRFAKSTTLLGTAFSGKSLTGAELPDGYYHYVVSY |
| gas57M6,31_20022 | (948) | VSPESFKGDKNFNSDIRFAKSTTLLGTAFSGKSLTGAELPDGYYHYVVSY |
| gas57M11_20648 | (947) | VSPESFKGDKNFNSDIRFAKSTTLLGTAFSGKSLTGAELPDGHYHYVVSY |
| gas57M23_2071 | (948) | VSPESFKGDKNFNSDIRFAKSTTLLGTAFSGKSLTGAELPDGYYHYVVSY |
| gas57M18,3_40128 | (949) | VSPESFKGDKNFNSDIRFAKSTTLLGTAFSGKSLTGAELPDGYYHYVVSY |
| gas57M4_10092 | (949) | VSPESFKGDKNFNSDIRFAKSTTLLGTAFSGKSLTGAELPDGYYHYVVSY |
| gas57M4_30968 | (949) | VSPESFKGDKNFNSDIRFAKSTTLLGTAFSGKSLTGAELPDGYYHYVVSY |
| gas57M6,31_22692 | (949) | VSPESFKGDKNFNSDIRFAKSTTLLGTAFSGKSLTGAELPDGYYHYVVSY |
| gas57M68,5_22814 | (948) | VSPESFKGDKNFNSDIRFAKSTTLLGTAFSGKSLTGAELPDGYYHYVVSY |
| gas57M68_23623 | (949) | VSPESFKGDKNFNSDIRFAKSTTLLGTAFSGKSLTGAELPDGYYHYVVSY |
| gas57M2_10064 | (947) | VSPESFKGDKNFNSDIRFAKSTTLLGTAFSGKSLTGAELPDGYYHYVVSY |
| gas57M2_10065 | (947) | VSPESFKGDKNFNSDIRFAKSTTLLGTAFSGKSLTGAELPDGYYHYVVSY |
| gas57M77_10251 | (947) | VSPESFKGDKNFNSDIRFAKSTTLLGTAFSGKSLTGAELPDGYYHYVVSY |
| gas57M77_10527 | (947) | VSPESFKGDKNFNSDIRFAKSTTLLGTAFSGKSLTGAELPDGYYHYVVSY |
| gas57M77_20696 | (947) | VSPESFKGDKNFNSDIRFAKSTTLLGTAFSGKSLTGAELPDGYYHYVVSY |
| gas57M89_21915 | (949) | VSPESFKGDKNFNSDIRFAKSTTLLGTAFSGKSLTGAELPDGYYHYVVSY |
| gas57M89_23717 | (949) | VSPESFKGDKNFNSDIRFAKSTTLLGTAFSGKSLTGAELPDGYYHYVVSY |
| gas57M94_10134 | (947) | VSPESFKGDKNFNSDIRFAKSTTLLGTAFSGKSLTGAELPDGYYHYVVSY |
| gas57M28_10164 | (947) | VSPESFKGDKNFNSDIRFAKSTTLLGTAFSGKSLTGAELPDGYYHYVVSY |
| gas57M28_10218 | (947) | VSPESFKGDKNFNSDIRFAKSTTLLGTAFSGKSLTGAELPDGYYHYVVSY |
| gas57M28_10266 | (947) | VSPESFKGDKNFNSDIRFAKSTTLLGTAFSGKSLTGAELPDGYYHYVVSY |
| gas57M28_10299 | (947) | VSPESFKGDKNFNSDIRFAKSTTLLGTAFSGKSLTGAELPDGYYHYVVSY |
| gas57M28_30176 | (947) | VSPESFKGDKNFNSDIRFAKSTTLLGTAFSGKSLTGAELPDGYYHYVVSY |
| gas57M28_30574 | (947) | VSPESFKGDKNFNSDIRFAKSTTLLGTAFSGKSLTGAELPDGYYHYVVSY |
| gas57M6,9_21802 | (950) | VSPESFKGDKNFNSDIRFAKSTTLLGTAFSGKSLTGAELPDGYYHYVVSY |
| gas57M75_10012 | (951) | VSPESFKGDKNFNSDIRFAKSTTLLGTAFSGKSLTGAELPDGYYHYVVSY |
| gas57M75_20671 | (951) | VSPESFKGDKNFNSDIRFAKSTTLLGTAFSGKSLTGAELPDGYYHYVVSY |
| gas57M75_30603 | (951) | VSPESFKGDKNFNSDIRFAKSTTLLGTAFSGKSLTGAELPDGYYHYVVSY |
| gas57M75_30207 | (951) | VSPESFKGDKNFNSDIRFAKSTTLLGTAFSGKSLTGAELPDGYYHYVVSY |
| gas57M22_20641 | (950) | VSPESFKGDKNFNSDIRFAKSTTLLGTAFSGKSLTGAELPDGHYHYVVSY |
| gas57M22_23465 | (950) | VSPESFKGDKNFNSDIRFAKSTTLLGTAFSGKSLTGAELPDGHYHYVVSY |
| gas57M3,1_30610 | (950) | VSPESFKGDKNFNSDIRFAKSTTLLGTAFSGKSLTGAELPDGYYHYVVSY |
| gas57M3,1_40603 | (950) | VSPESFKGDKNFNSDIRFAKSTTLLGTAFSGKSLTGAELPDGYYHYVVSY |
| gas57M3,28_24214 | (950) | VSPESFKGDKNFNSDIRFAKSTTLLGTAFSGKSLTGAELPDGYYHYVVSY |
| gas57M3,34_10307 | (950) | VSPESFKGDKNFNSDIRFAKSTTLLGTAFSGKSLTGAELPDGYYHYVVSY |
| gas57M4_40427 | (950) | VSPESFKGDKNFNSDIRFAKSTTLLGTAFSGKSLTGAELPDGYYHYVVSY |
| gas57M3_2721 | (950) | VSPESFKGDKNFNSDIRFAKSTTLLGTAFSGKSLTGAELPDGYYHYVVSY |
| gas57M12_10296 | (949) | VSPESFKGDKNFNSDIRFAKSTTLLGTAFSGKSLTGAELPDGYYHYVVSY |
| gas57M12_10035 | (949) | VSPESFKGDKNFNSDIRFAKSTTLLGTAFSGKSLTGAELPDGYYHYVVSY |
| gas57M12_20069 | (949) | VSPESFKGDKNFNSDIRFAKSTTLLGTAFSGKSLTGAELPDGYYHYVVSY |
| gas57M12_22432 | (949) | VSPESFKGDKNFNSDIRFAKSTTLLGTAFSGKSLTGAELPDGYYHYVVSY |
| gas57M4_40499 | (949) | VSPESFKGDKNFNSDIRFAKSTTLLGTAFSGKSLTGAELPDGYYHYVVSY |
| gas57M6,1_21259 | (949) | VSPESFKGDKNFNSDIRFAKSTTLLGTAFSGKSLTGAELPDGYYHYVVSY |

FIG. 31U

```
                                1001                                              1050
     gas57M1_SF370     (999)   YPDVVGAKRQEMTFDMILDRQKPVLSQATFDPETNRFKPEPLKDRGLAGV
     gas57M1_31075     (999)   YPDVVGAKRQEMTFDMILDRQKPVLSQATFDPETNRFKPEPLKDRGLAGV
     gas57M1_31237     (999)   YPDVVGAKRQEMTFDMILDRQKPVLSQATFDPETNRFKPEPLKDRGLAGV
      gas57M1_3348     (999)   YPDVVGAKRQEMTFDMILDRQKPVLSQATFDPETNRFKPEPLKDRGLAGV
     gas57M2_34585     (999)   YPDVVGAKRQEMTFDMILDRQKPVLSQATFDPETNRFKPEPLKDRGLAGV
   gas57M3,1_21398     (999)   YPDVVGAKRQEMTFDMILDRQKPVLSQATFDPETNRFKPEPLKDRGLAGV
 gas57M44-61_20839     (999)   YPDVVGAKRQEMTFDMILDRQKPVLSQATFDPETNRFKPEPLKDRGLAGV
  gas57M6,31_20022     (998)   YPDVVGAKRQEMTFDMILDRQKPVLSQATFDPETNRFKPEPLKDRGLAGV
    gas57M11_20648     (997)   YPDVVGAKRQEMTFDMILDRQKPVLSQATFDPETNRFKPEPLKDRGLAGV
     gas57M23_2071     (998)   YPDVVGAKRQEMTFDMILDRQKPVLSQATFDPETNRFKPEPLKDRGLAGV
  gas57M18,3_40128     (999)   YPDVVGAKRQEMTFDMILDRQKPVLSQATFDPETNRFKPEPLKDRGLAGV
     gas57M4_10092     (999)   YPDVVGAKRQEMTFDMILDRQKPVLSQATFDPETNRFKPEPLKDRGLAGV
     gas57M4_30968     (999)   YPDVVGAKRQEMTFDMILDRQKPVLSQATFDPETNRFKPEPLKDRGLAGV
  gas57M6,31_22692     (999)   YPDVVGAKRQEMTFDMILDRQKPVLSQATFDPETNRFKPEPLKDRGLAGV
  gas57M68,5_22814     (998)   YPDVVGAKRQEMTFDMILDRQKPVLSQATFDPETNRFKPEPLKDRGLAGV
    gas57M68_23623     (999)   YPDVVGAKRQEMTFDMILDRQKPVLSQATFDPETNRFKPEPLKDRGLAGV
     gas57M2_10064     (997)   YPDVVGAKRQEMTFDMILDRQKPVLSQATFDPETNRFKPEPLKDRGLAGV
     gas57M2_10065     (997)   YPDVVGAKRQEMTFDMILDRQKPVLSQATFDPETNRFKPEPLKDRGLAGV
    gas57M77_10251     (997)   YPDVVGAKRQEMTFDMILDRQKPVLSQATFDPETNRFKPEPLKDRGLAGV
    gas57M77_10527     (997)   YPDVVGAKRQEMTFDMILDRQKPVLSQATFDPETNRFKPEPLKDRGLAGV
    gas57M77_20696     (997)   YPDVVGAKRQEMTFDMILDRQKPVLSQATFDPETNRFKPEPLKDRGLAGV
    gas57M89_21915     (999)   YPDVVGAKRQEMTFDMILDRQKPVLSQATFDPETNRFKPEPLKDRGLAGV
    gas57M89_23717     (999)   YPDVVGAKRQEMTFDMILDRQKPVLSQATFDPETNRFKPEPLKDRGLAGV
    gas57M94_10134     (997)   YPDVVGAKRQEMTFDMILDRQKPVLSQATFDPETNRFKPEPLKDRGLAGV
    gas57M28_10164     (997)   YPDVVGAKRQEMTFDMILDRQKPVLSQATFDPETNRFKPEPLKDRGLAGV
    gas57M28_10218     (997)   YPDVVGAKRQEMTFDMILDRQKPVLSQATFDPETNRFKPEPLKDRGLAGV
    gas57M28_10266     (997)   YPDVVGAKRQEMTFDMILDRQKPVLSQATFDPETNRFKPEPLKDRGLAGV
    gas57M28_10299     (997)   YPDVVGAKRQEMTFDMILDRQKPVLSQATFDPETNRFKPEPLKDRGLAGV
    gas57M28_30176     (997)   YPDVVGAKRQEMTFDMILDRQKPVLSQATFDPETNRFKPEPLKDRGLAGV
    gas57M28_30574     (997)   YPDVVGAKRQEMTFDMILDRQKPVLSQATFDPETNRFKPEPLKDRGLAGV
   gas57M6,9_21802    (1000)   YPDVVGAKRQEMTFDMILDRQKPVLSQATFDPETNRFKPEPLKDRGLAGV
    gas57M75_10012    (1001)   YPDVVGAKRQEMTFDMILDRQKPVLSQATFDPETNRFKPEPLKDRGLAGV
    gas57M75_20671    (1001)   YPDVVGAKRQEMTFDMILDRQKPVLSQATFDPETNRFKPEPLKDRGLAGV
    gas57M75_30603    (1001)   YPDVVGAKRQEMTFDMILDRQKPVLSQATFDPETNRFKPEPLKDRGLAGV
    gas57M75_30207    (1001)   YPDVVGAKRQEMTFDMILDRQKPVLSQATFDPETNRFKPEPLKDRGLAGV
    gas57M22_20641    (1000)   YPDVVGAKRQEMTFDMILDRQKPVLSQATFDPETNRFKPEPLKDRGLAGV
    gas57M22_23465    (1000)   YPDVVGAKRQEMTFDMILDRQKPVLSQATFDPETNRFKPEPLKDRGLAGV
   gas57M3,1_30610    (1000)   YPDVVGAKRQEMTFDMILDRQKPVLSQATFDPETNRFKPEPLKDRGLAGV
   gas57M3,1_40603    (1000)   YPDVVGAKRQEMTFDMILDRQKPVLSQATFDPETNRFKPEPLKDRGLAGV
  gas57M3,28_24214    (1000)   YPDVVGAKRQEMTFDMILDRQKPVLSQATFDPETNRFKPEPLKDRGLAGV
  gas57M3,34_10307    (1000)   YPDVVGAKRQEMTFDMILDRQKPVLSQATFDPETNRFKPEPLKDRGLAGV
     gas57M4_40427    (1000)   YPDVVGAKRQEMTFDMILDRQKPVLSQATFDPETNRFKPEPLKDRGLAGV
      gas57M3_2721    (1000)   YPDVVGAKRQEMTFDMILDRQKPVLSQATFDPETNRFKPEPLKDRGLAGV
    gas57M12_10296     (999)   YPDVVGAKRQEMTFDMILDRQKPVLSQATFDPETNRFKPEPLKDRGLAGV
    gas57M12_10035     (999)   YPDVVGAKRQEMTFDMILDRQKPVLSQATFDPETNRFKPEPLKDRGLAGV
    gas57M12_20069     (999)   YPDVVGAKRQEMTFDMILDRQKPVLSQATFDPETNRFKPEPLKDRGLAGV
    gas57M12_22432     (999)   YPDVVGAKRQEMTFDMILDRQKPVLSQATFDPETNRFKPEPLKDRGLAGV
     gas57M4_40499     (999)   YPDVVGAKRQEMTFDMILDRQKPVLSQATFDPETNRFKPEPLKDRGLAGV
   gas57M6,1_21259     (999)   YPDVVGAKRQEMTFDMILDRQKPVLSQATFDPETNRFKPEPLKDRGLAGV
```

FIG. 31V

| | | 1051 1100 |
|---|---|---|
| gas57M1_SF370 | (1049) | RKDSVFYLERKDNKPYTVTINDSYKYVSVEDNKTFVERQADGSFILPLDK |
| gas57M1_31075 | (1049) | RKDSVFYLERKDNKPYTVTINDSYKYVSVEDNKTFVERQADGSFILPLDK |
| gas57M1_31237 | (1049) | RKDSAFYLERKDNKPYTVTINDSYKYVSVEDNKTFVERQADGSFILPLDK |
| gas57M1_3348 | (1049) | RKDSAFYLERKDNKPYTVTINDSYKYVSVEDNKTFVERQADGSFILPLDK |
| gas57M2_34585 | (1049) | RKDSAFYLERKDNKPYTVTINDSYKYVSVEDNKTFVERQADGSFILPLDK |
| gas57M3,1_21398 | (1049) | RKDSVFYLERKDNKPYTVTINDSYKYVSVEDNKTFVERQADGSFILPLDK |
| gas57M44-61_20839 | (1049) | RKDSVFYLERKDNKPYIVTINDSYKYVSVEDNKTFVERQADGSFILPLDK |
| gas57M6,31_20022 | (1048) | RKDSVFYLERKDNKPYTVTINDSYKYVSVEDNKTFVERQADGSFILPLDK |
| gas57M11_20648 | (1047) | RKDSVFYLERKDNKPYTVTINDSYKYVSVADNKTFVERQADGSFILPLDK |
| gas57M23_2071 | (1048) | RKDSVFYLERKDNKPYTVTINDSYKYVSVADNKTFVERQADGSFILPLDK |
| gas57M18,3_40128 | (1049) | RKDSVFYLERKDNKPYTVTINDSYKYVSVEDNKTFVERQADGSFILPLDK |
| gas57M4_10092 | (1049) | RKDSVFYLERKDNKPYTVTINDSYKYVSVEDNKTFVERQADGSFILPLDK |
| gas57M4_30968 | (1049) | RKDSVFYLERKDNKPYTVTINDSYKYVSVEDNKTFVERQADGSFILPLDK |
| gas57M6,31_22692 | (1049) | RKDSVFYLERKDNKPYTVTINDSYKYVSVEDNKTFVERQADGSFILPLDK |
| gas57M68,5_22814 | (1048) | RKDSVFYLERKDNKPYTVTINDSYKYVSVEDNKTFVERQADGSFILPLDK |
| gas57M68_23623 | (1049) | RKDSVFYLERKDNKPYTVTINDSYKYVSVEDNKTFVERQADGSFILPLDK |
| gas57M2_10064 | (1047) | RKDSVFYLERKDNKPYTVTINDSYKYVSVADNKTFVERQADGSFILPLDK |
| gas57M2_10065 | (1047) | RKDSVFYLERKDNKPYTVTINDSYKYVSVADNKTFVERQADGSFILPLDK |
| gas57M77_10251 | (1047) | RKDSVFYLERKDNKPYTVTINDSYKYVSVEDNKTFVERQADGSFILPLDK |
| gas57M77_10527 | (1047) | RKDSVFYLERKDNKPYTVTINDSYKYVSVEDNKTFVERQADGSFILPLDK |
| gas57M77_20696 | (1047) | RKDSVFYLERKDNKPYTVTINDSYKYVSVEDNKTFVERQADGSFILPLDK |
| gas57M89_21915 | (1049) | RKDSVFYLERKDNKPYTVTINDSYKYVSVADNKTFVERQADGSFILPLDK |
| gas57M89_23717 | (1049) | RKDSVFYLERKDNKPYTVTINDSYKYVSVADNKTFVERQADGSFILPLDK |
| gas57M94_10134 | (1047) | RKDSVFYLERKDNKPYTVTINDSYKYVSVEDNKTFVERQADGSFILPLDK |
| gas57M28_10164 | (1047) | RKDSVFYLERKDNKPYTVTINDSYKYVSVEDNKTFVERQADGSFILPLDK |
| gas57M28_10218 | (1047) | RKDSVFYLERKDNKPYTVTINDSYKYVSVEDNKTFVERQADGSFILPLDK |
| gas57M28_10266 | (1047) | RKDSVFYLERKDNKPYTVTINDSYKYVSVEDNKTFVERQADGSFILPLDK |
| gas57M28_10299 | (1047) | RKDSVFYLERKDNKPYTVTINDSYKYVSVEDNKTFVERQADGSFILPLDK |
| gas57M28_30176 | (1047) | RKDSVFYLERKDNKPYTVTINDSYKYVSVEDNKTFVERQADGSFILPLDK |
| gas57M28_30574 | (1047) | RKDSVFYLERKDNKPYTVTINDSYKYVSVEDNKTFVERQADGSFILPLDK |
| gas57M6,9_21802 | (1050) | RKDSVFYLERKDNKPYTVTINDSYKYVSVEDNKTFVERQADGSFILPLDK |
| gas57M75_10012 | (1051) | RKDSVFYLERKDNKPYTVTINDSYKYVSVEDNKTFVERQADGSFILPLDK |
| gas57M75_20671 | (1051) | RKDSVFYLKRKDNKPYTVTINDSYKYVSVEDNKTFVERQADGSFILPLDK |
| gas57M75_30603 | (1051) | RKDSVFYLERKDNKPYTVTINDSYKYVSVEDNKTFVERQADGSFILPLDK |
| gas57M75_30207 | (1051) | RKDSVFYLERKDNKPYTVTINDSYKYVSVEDNKTFVERQADGSFILPLDK |
| gas57M22_20641 | (1050) | RKDSVFYLERKDNKPYTVTINDSYKYVSVEDNKTFVERQADGSFILPLDK |
| gas57M22_23465 | (1050) | RKDSVFYLERKDNKPYTVTINDSYKYVSVEDNKTFVERQADGSFILPLDK |
| gas57M3,1_30610 | (1050) | RKDSVFYLERKDNKPYTVTINDSYKYVSVEDSKTFVERQADGSFILPLDK |
| gas57M3,1_40603 | (1050) | RKDSVFYLERKDNKPYTVTINDSYKYVSVEDSKTFVERQADGSFILPLDK |
| gas57M3,28_24214 | (1050) | RKDSVFYLERKDNKPYTVTINDSYKYVSVEDSKTFVERQADGSFILPLDK |
| gas57M3,34_10307 | (1050) | RKDSVFYLERKDNKPYTVTINDSYKYVSVEDSKTFVERQADGSFILPLDK |
| gas57M4_40427 | (1050) | RKDSVFYLERKDNKPYTVTINDSYKYVSVEDSKTFVERQADGSFILPLDK |
| gas57M3_2721 | (1050) | RKDSVFYLERKDNKPYTVTINDSYKYVSVEDSKTFVERQADGSFILPLDK |
| gas57M12_10296 | (1049) | RKDSVFYLERKDNKPYTVTINDSYKYVSVEDNKTFVERQADGSFILPLDK |
| gas57M12_10035 | (1049) | RKDSVFYLERKDNKPYTVTINDSYKYVSVEDNKTFVERQADGSFILPLDK |
| gas57M12_20069 | (1049) | RKDSVFYLERKDNKPYTVTINDSYKYVSVEDNKTFVERQADGSFILPLDK |
| gas57M12_22432 | (1049) | RKDSVFYLERKDNKPYTVTINDSYKYVSVEDNKTFVERQADGSFILPLDK |
| gas57M4_40499 | (1049) | RKDSVFYLERKDNKPYTVTINDSYKYVSVEDNKTFVERQADGSFILPLDK |
| gas57M6,1_21259 | (1049) | RKDSVFYLERKDNKPYTVTINDSYKYVSVEDNKTFVERQADGSFILPLDK |

FIG. 31W

| | | 1101 1150 |
|---|---|---|
| gas57M1_SF370 | (1099) | AKLGDFYYMVEDFAGNVAIAKLGDHLPQTLGKTPIKLKLTDGNYQTKETL |
| gas57M1_31075 | (1099) | AKLGDFYYMVEDFAGNVAIAKLGDHLPQTLGKTPIKLKLTDGNYQTKETL |
| gas57M1_31237 | (1099) | AKLGDFYYMVEDFAGNVAIAKLGDHLPQTLGKTPIKLKLTDGNYQTKETL |
| gas57M1_3348 | (1099) | AKLGDFYYMVEDFAGNVAIAKLGDHLPQTLGKTPIKLKLTDGNYQTKETL |
| gas57M2_34585 | (1099) | AKLGDFYYMVEDFAGNVAIAKLGDHLPQTLGKTPIKLKLTDGNYQTKETL |
| gas57M3,1_21398 | (1099) | AKLGDFYYMVEDFAGNVAIAKLGDHLPQTLGKTPIKLKLTDGNYQTKETL |
| gas57M44-61_20839 | (1099) | AKLGDFYYMVEDFAGNVAIAKLGDHLPQTLGKTPIKLKLTDGNYQTKETL |
| gas57M6,31_20022 | (1098) | AKLGDFYYMVEDFAGNVAIAKLGDHLPQTLGKTPIKLKLTDGNYQTKETL |
| gas57M11_20648 | (1097) | AKLGDFYYMVEDFAGNVAIAKLGDHLPQTLGKTPIKLKLTDGNYQTKETL |
| gas57M23_2071 | (1098) | AKLGDFYYMVEDFAGNVAIAKLGDHLPQTLGKTPIKLKLTDGNYQTKETL |
| gas57M18,3_40128 | (1099) | AKLGDFYYMVEDFAGNVAIAKLGDHLPQTLGKTPIKLKLTDGNYQTKETL |
| gas57M4_10092 | (1099) | AKLGDFYYMVEDFAGNVAIAKLGDHLPQTLGKTPIKLKLTDGNYQTKETL |
| gas57M4_30968 | (1099) | AKLGDFYYMVEDFAGNVAIAKLGDHLPQTLGKTPIKLKLTDGNYQTKETL |
| gas57M6,31_22692 | (1099) | AKLGDFYYMVEDFAGNVAIAKLGDHLPQTLGKTPIKLKLTDGNYQTKETL |
| gas57M68,5_22814 | (1098) | AKLGDFYYMVEDFAGNVAIAKLGDHLPQTLGKTPIKLKLTDGNYQTKETL |
| gas57M68_23623 | (1099) | AKLGDFYYMVEDFAGNVAIAKLGDHLPQTLGKTPIKLKLTDGNYQTKETL |
| gas57M2_10064 | (1097) | AKLGDFYYMVEDFAGNVAIAKLGDHLPQTLGKTPIKLKLTDGNYQTKETL |
| gas57M2_10065 | (1097) | AKLGDFYYMVEDFAGNVAIAKLGDHLPQTLGKTPIKLKLTDGNYQTKETL |
| gas57M77_10251 | (1097) | AKLGDFYYMVEDFAGNVAIAKLGDHLPQTLGKTPIKLKLTDGNYQTKETL |
| gas57M77_10527 | (1097) | AKLGDFYYMVEDFAGNVAIAKLGDHLPQTLGKTPIKLKLTDGNYQTKETL |
| gas57M77_20696 | (1097) | AKLGDFYYMVEDFAGNVAIAKLGDHLPQTLGKTPIKLKLTDGNYQTKETL |
| gas57M89_21915 | (1099) | AKLGDFYYMVEDFAGNVAIAKLGDHLPQTLGKTPIKLKLTDGNYQTKETL |
| gas57M89_23717 | (1099) | AKLGDFYYMVEDFAGNVAIAKLGDHLPQTLGKTPIKLKLTDGNYQTKETL |
| gas57M94_10134 | (1097) | AKLGDFYYMVEDFAGNVAIAKLGDHLPQTLGKTPIKLKLTDGNYQTKETL |
| gas57M28_10164 | (1097) | AKLGDFYYMVEDFAGNVAIAKLGDHLPQTLGKTPIKLKLTDGNYQTKETL |
| gas57M28_10218 | (1097) | AKLGDFYYMVEDFAGNVAIAKLGDHLPQTLGKTPIKLKLTDGNYQTKETL |
| gas57M28_10266 | (1097) | AKLGDFYYMVEDFAGNVAIAKLGDHLPQTLGKTPIKLKLTDGNYQTKETL |
| gas57M28_10299 | (1097) | AKLGDFYYMVEDFAGNVAIAKLGDHLPQTLGKTPIKLKLTDGNYQTKETL |
| gas57M28_30176 | (1097) | AKLGDFYYMVEDFAGNVAIAKLGDHLPQTLGKTPIKLKLTDGNYQTKETL |
| gas57M28_30574 | (1097) | AKLGDFYYMVEDFAGNVAIAKLGDHLPQTLGKTPIKLKLTDGNYQTKETL |
| gas57M6,9_21802 | (1100) | AKLGDFYYMVEDFAGNVAIAKLGDHLPQTLGKTPIKLKLTDGNYQTKETL |
| gas57M75_10012 | (1101) | AKLGDFYYMVEDFAGNVAIAKLGDHLPQTLGKTPIKLKLTDGNYQTKETL |
| gas57M75_20671 | (1101) | AKLGDFYYMVEDFAGNVAIAKLGDHLPQTLGKTPIKLKLTDGNYQTKETL |
| gas57M75_30603 | (1101) | AKLGDFYYMVEDFAGNVAIAKLGDHLPQTLGKTPIKLKLTDGNYQTKETL |
| gas57M75_30207 | (1101) | AKLGDFYYMVEDFAGNVAIAKLGDHLPQTLGKTPIKLKLTDGNYQTKETL |
| gas57M22_20641 | (1100) | AKLGDFYYMVEDFAGNVAIAKLGDHLPQTLGKTPIKLKLTDGNYQTKETL |
| gas57M22_23465 | (1100) | AKLGDFYYMVEDFAGNVAIAKLGDHLPQTLGKTPIKLKLTDGNYQTKETL |
| gas57M3,1_30610 | (1100) | AKLGDFYYMVEDFAGNVAIAKLGDHLPQTLGKTPIKLKLTDGNYQTKETL |
| gas57M3,1_40603 | (1100) | AKLGDFYYMVEDFAGNVAIAKLGDHLPQTLGKTPIKLKLTDGNYQTKETL |
| gas57M3,28_24214 | (1100) | AKLGDFYYMVEDFAGNVAIAKLGDHLPQTLGKTPIKLKLTDGNYQTKETL |
| gas57M3,34_10307 | (1100) | AKLGDFYYMVEDFAGNVAIAKLGDHLPQTLGKTPIKLKLTDGNYQTKETL |
| gas57M4_40427 | (1100) | AKLGDFYYMVEDFAGNVAIAKLGDHLPQTLGKTPIKLKLTDGNYQTKETL |
| gas57M3_2721 | (1100) | AKLGDFYYMVEDFAGNVAIAKLGDHLPQTLGKTPIKLKLTDGNYQTKETL |
| gas57M12_10296 | (1099) | AKLGDFYYMVEDFAGNVAIAKLGDHLPQTLGKTPIKLKLTDGNYQTKETL |
| gas57M12_10035 | (1099) | AKLGDFYYMVEDFAGNVAIAKLGDHLPQTLGKTPIKLKLTDGNYQTKETL |
| gas57M12_20069 | (1099) | AKLGDFYYMVEDFAGNVAIAKLGDHLPQTLGKTPIKLKLTDGNYQTKETL |
| gas57M12_22432 | (1099) | AKLGDFYYMVEDFAGNVAIAKLGDHLPQTLGKTPIKLKLTDGNYQTKETL |
| gas57M4_40499 | (1099) | AKLGDFYYMVEDFAGNVAIAKLGDHLPQTLGKTPIKLKLTDGNYQTKETL |
| gas57M6,1_21259 | (1099) | AKLGDFYYMVEDFAGNVAIAKLGDHLPQTLGKTPIKLKLTDGNYQTKETL |

FIG. 31X

```
                                 1151                                              1200
     gas57M1_SF370     (1149)    KDNLEMTQSDTGLVTNQAQLAVVHRNQPQSQLTKMNQDFFISPNEDGNKD
      gas57M1_31075    (1149)    KDNLEMTQSDTGLVTNQAQLAVVHRNQPQSQLTKMNQDFFISPNEDGNKD
      gas57M1_31237    (1149)    KDNLEMTQSDTGLVTNQAQLAVVHRNQPQSQLTKMNQDFFISPNEDGNKD
       gas57M1_3348    (1149)    KDNLEMTQSDTGLVTNQAQLAVVHRNQPQSQLTKMNQDFFISPNEDGNKD
      gas57M2_34585    (1149)    KDNLEMTQSDTGLVTNQAQLAVVHRNQPQSQLTKMNQDFFISPNEDGNKD
    gas57M3,1_21398    (1149)    KDNLEMTQSDTGLVTNQAQLAVVHRNQPQSQLTKMNQDFFISPNEDGNKD
 gas57M44-61_20839    (1149)    KDNLEMTQSDTGLVTNQAQLAVVHRNQPQSQLTKMNQDFFISPNEDGNKD
   gas57M6,31_20022    (1148)    KDNLEMTQSDTGLVTNQAQLAVVHRNQPQSQLTKMNQDFFISPNEDGNKD
     gas57M11_20648    (1147)    KDNLEMTQSDTGLVTNQAQLAVVHRNQPQSQLTKMNQDFFISPNEDGNKD
      gas57M23_2071    (1148)    KDNLEMTQSDTGLVTNQAQLAVVHRNQPQSQLTKMNQDFFISPNEDGNKD
   gas57M18,3_40128    (1149)    KDNLEMTQSDTGLVTNQAQLAVVHRNQPQSQLTKMNQDFFISPNEDGNKD
      gas57M4_10092    (1149)    KDNLEMTQSDTGLVTNQAQLAVVHRNQPQSQLTKMNQDFFISPNEDGNKD
      gas57M4_30968    (1149)    KDNLEMTQSDTGLVTNQAQLAVVHRNQPQSQLTKMNQDFFISPNEDGNKD
   gas57M6,31_22692    (1149)    KDNLEMTQSDTGLVTNQAQLAVVHRNQPQSQLTKMNQDFFISPNEDGNKD
   gas57M68,5_22814    (1148)    KDNLEMTQSDTGLVTNQAQLAVVHRNQPQSQLTKMNQDFFISPNEDGNKD
     gas57M68_23623    (1149)    KDNLEMTQSDTGLVTNQAQLAVVHRNQPQSQLTKMNQDFFISPNEDGNKD
      gas57M2_10064    (1147)    KDNLEMTQSDTGLVTNQAQLAVVHRNQPQSQLTKMNQDFFISPNEDGNKD
      gas57M2_10065    (1147)    KDNLEMTQSDTGLVTNQAQLAVVHRNQPQSQLTKMNQDFFISPNEDGNKD
     gas57M77_10251    (1147)    KDNLEMTQSDTGLVTNQAQLAVVHRNQPQSQLTKMNQDFFISPNEDGNKD
     gas57M77_10527    (1147)    KDNLEMTQSDTGLVTNQAQLAVVHRNQPQSQLTKMNQDFFISPNEDGNKD
     gas57M77_20696    (1147)    KDNLEMTQSDTGLVTNQAQLAVVHRNQPQSQLTKMNQDFFISPNEDGNKD
     gas57M89_21915    (1149)    KDNLEMTQSDTGLVTNQAQLAVVHRNQPQSQLTKMNQDFFISPNEDGNKD
     gas57M89_23717    (1149)    KDNLEMTQSDTGLVTNQAQLAVVHRNQPQSQLTKMNQDFFISPNEDGNKD
     gas57M94_10134    (1147)    KDNLEMTQSDTGLVTNQAQLAVVHRNQPQSQLTKMNQDFFISPNEDGNKD
     gas57M28_10164    (1147)    KDNLEMTQSDTGLVTNQAQLAVVHRNQPQSQLTKMNQDFFISPNEDGNKD
     gas57M28_10218    (1147)    KDNLEMTQSDTGLVTNQAQLAVVHRNQPQSQLTKMNQDFFISPNEDGNKD
     gas57M28_10266    (1147)    KDNLEMTQSDTGLVTNQAQLAVVHRNQPQSQLTKMNQDFFISPNEDGNKD
     gas57M28_10299    (1147)    KDNLEMTQSDTGLVTNQAQLAVVHRNQPQSQLTKMNQDFFISPNEDGNKD
     gas57M28_30176    (1147)    KDNLEMTQSDTGLVTNQAQLAVVHRNQPQSQLTKMNQDFFISPNEDGNKD
     gas57M28_30574    (1147)    KDNLEMTQSDTGLVTNQAQLAVVHRNQPQSQLTKMNQDFFISPNEDGNKD
    gas57M6,9_21802    (1150)    KDNLEMTQSDTGLVTNQAQLAVVHRNQPQSQLTKMNQDFFISPNEDGNKD
     gas57M75_10012    (1151)    KDNLEMTQSDTGLVTNQAQLAVVHRNQPQSQLTKMNQDFFISPNEDGNKD
     gas57M75_20671    (1151)    KDNLEMTQSDTGLVTNQAQLAVVHRNQPQSQLTKMNQDFFISPNEDGNKD
     gas57M75_30603    (1151)    KDNLEMTQSDTGLVTNQAQLAVVHRNQPQSQLTKMNQDFFISPNEDGNKD
     gas57M75_30207    (1151)    KDNLEMTQSDTGLVTNQAQLAVVHRNQPQSQLTKMNQDFFISPNEDGNKD
     gas57M22_20641    (1150)    KDNLEMTQSDTGLVTNQAQLAVVHRNQPQSQLTKMNQDFFISPNEDGNKD
     gas57M22_23465    (1150)    KDNLEMTQSDTGLVTNQAQLAVVHRNQPQSQLTKMNQDFFISPNEDGNKD
    gas57M3,1_30610    (1150)    KDNLEMTQSDTGLVTNQAQLAVVHRNQPQSQLTKMNQDFFISPNEDGNKD
    gas57M3,1_40603    (1150)    KDNLEMTQSDTGLVTNQAQLAVVHRNQPQSQLTKMNQDFFISPNEDGNKD
   gas57M3,28_24214    (1150)    KDNLEMTQSDTGLVTNQAQLAVVHRNQPQSQLTKMNQDFFISPNEDGNKD
   gas57M3,34_10307    (1150)    KDNLEMTQSDTGLVTNQAQLAVVHRNQPQSQLTKMNQDFFISPNEDGNKD
      gas57M4_40427    (1150)    KDNLEMTQSDTGLVTNQAQLAVVHRNQPQSQLTKMNQDFFISPNEDGNKD
       gas57M3_2721    (1150)    KDNLEMTQSDTGLVTNQAQLAVVHRNQPQSQLTKMNQDFFISPNEDGNKD
     gas57M12_10296    (1149)    KDNLEMTQSDTGLVTNQAQLAVVHRNQPQSQLTKMNQDFFISPNEDGNKD
     gas57M12_10035    (1149)    KDNLEMTQSDTGLVTNQAQLAVVHRNQPQSQLTKMNQDFFISPNEDGNKD
     gas57M12_20069    (1149)    KDNLEMTQSDTGLVTNQAQLAVVHRNQPQSQLTKMNQDFFISPNEDGNKD
     gas57M12_22432    (1149)    KDNLEMTQSDTGLVTNQAQLAVVHRNQPQSQLTKMNQDFFISPNEDGNKD
      gas57M4_40499    (1149)    KDNLEMTQSDTGLVTNQAQLAVVHRNQPQSQLTKMNQDFFISPNEDGNKD
    gas57M6,1_21259    (1149)    KDNLEMTQSDTGLVTNQAQLAVVHRNQPQSQLTKMNQDFFISPNEDGNKD
```

FIG. 31Y

| | | 1201 1250 |
|---|---|---|
| gas57M1_SF370 | (1199) | FVAFKGLKNNVYNDLTVNVYAKDDHQKQTPIWSSQAGASVSAIESTAWYG |
| gas57M1_31075 | (1199) | FVAFKGLKNNVYNDLTVNVYAKDDHQKQTPIWSSQAGASVSAIESTAWYG |
| gas57M1_31237 | (1199) | FVAFKGLKNNVYNDLTVNVYAKDDHQKQTPIWSSQAGASVSAIESTAWYG |
| gas57M1_3348 | (1199) | FVAFKGLKNNVYNDLTVNVYAKDDHQKQTPIWSSQAGASVSAIESTAWYG |
| gas57M2_34585 | (1199) | FVAFKGLKNNVYNDLTVNVYAKDDHQKQTPIWSSQAGASVSAIESTAWYG |
| gas57M3,1_21398 | (1199) | FVAFKGLKNNVYNDLTVNVYAKDDHQKQTPIWSSQAGASASAIESTAWYG |
| gas57M44-61_20839 | (1199) | FVAFKGLKNNVYNDLTVNVYAKDDHQKQTPIWSSQAGASASAIESTAWYG |
| gas57M6,31_20022 | (1198) | FVAFKGLKNNVYNDLTVNVYAKDDHQKQTPIWSSQAGASASAIESTAWYG |
| gas57M11_20648 | (1197) | FVAFKGLKNNVYNDLTVNVYAKDDHQKQTPIWSSQAGASASAIESTAWYG |
| gas57M23_2071 | (1198) | FVAFKGLKNNVYNDLTVNVYAKDDHQKQTPIWSSQAGASASAIESTAWYG |
| gas57M18,3_40128 | (1199) | FVAFKGLKNNVYNDLTVNVYAKDDHQKQTPIWSSQAGASASAIESTAWYG |
| gas57M4_10092 | (1199) | FVAFKGLKNNVYNDLTVNVYAKDDHQKQTPIWSSQAGASASAIESTAWYG |
| gas57M4_30968 | (1199) | FVAFKGLKNNVYNDLTVNVYAKDDHQKQTPIWSSQAGASASAIESTAWYG |
| gas57M6,31_22692 | (1199) | FVAFKGLKNNVYNDLTVNVYAKDDHQKQTPIWSSQAGASASAIESTAWYG |
| gas57M68,5_22814 | (1198) | FVAFKGLKNNVYNDLTVNVYAKDDHQKQTPIWSSQAGASASAIESTAWYG |
| gas57M68_23623 | (1199) | FVAFKGLKNNVYNDLTVNVYAKDDHQKQTPIWSSQAGASASAIESTAWYG |
| gas57M2_10064 | (1197) | FVAFKGLKNNVYNDLTVNVYAKDDHQKQTPIWSSQAGASASAIESTAWYG |
| gas57M2_10065 | (1197) | FVAFKGLKNNVYNDLTVNVYAKDDHQKQTPIWSSQAGASASAIESTAWYG |
| gas57M77_10251 | (1197) | FVAFKGLKNNVYNDLTVNVYAKDDHQKQTPIWSSQAGASASAIESTAWYG |
| gas57M77_10527 | (1197) | FVAFKGLKNNVYNDLTVNVYAKDDHQKQTPIWSSQAGASASAIESTAWYG |
| gas57M77_20696 | (1197) | FVAFKGLKNNVYNDLTVNVYAKDDHQKQTPIWSSQAGASASAIESTAWYG |
| gas57M89_21915 | (1199) | FVAFKGLKNNVYNDLTVNVYAKDDHQKQTPIWSSQAGASASAIESTAWYG |
| gas57M89_23717 | (1199) | FVAFKGLKNNVYNDLTVNVYAKDDHQKQTPIWSSQAGASASAIESTAWYG |
| gas57M94_10134 | (1197) | FVAFKGLKNNVYNDLTVNVYAKDDHQKQTPIWSSQAGASASAIESTAWYG |
| gas57M28_10164 | (1197) | FVAFKGLKNNVYNDLTVNVYAKDDHQKQTPIWSSQAGASASAIESTAWYG |
| gas57M28_10218 | (1197) | FVAFKGLKNNVYNDLTVNVYAKDDHQKQTPIWSSQAGASASAIESTAWYG |
| gas57M28_10266 | (1197) | FVAFKGLKNNVYNDLTVNVYAKDDHQKQTPIWSSQAGASASAIESTAWYG |
| gas57M28_10299 | (1197) | FVAFKGLKNNVYNDLTVNVYAKDDHQKQTPIWSSQAGASASAIESTAWYG |
| gas57M28_30176 | (1197) | FVAFKGLKNNVYNDLTVNVYAKDDHQKQTPIWSSQAGASASAIESTAWYG |
| gas57M28_30574 | (1197) | FVAFKGLKNNVYNDLTVNVYAKDDHQKQTPIWSSQAGASASAIESTAWYG |
| gas57M6,9_21802 | (1200) | FVAFKGLKNNVYNDLTVNVYAKDDHQKQTPIWSSQAGASASAIESTAWYG |
| gas57M75_10012 | (1201) | FVAFKGLKNNVYNDLTVNVYAKDDHQKQTPIWSSQAGASASAIESTAWYG |
| gas57M75_20671 | (1201) | FVAFKGLKNNVYNDLTVNVYAKDDHQKQTPIWSSQAGASASAIESTAWYG |
| gas57M75_30603 | (1201) | FVAFKGLKNNVYNDLTVNVYAKDDHQKQTPIWSSQAGASASAIESTAWYG |
| gas57M75_30207 | (1201) | FVAFKGLKNNVYNDLTVNVYAKDDHQKQTPIWSSQAGASASAIESTAWYG |
| gas57M22_20641 | (1200) | FVAFKGLKNNVYNDLTVNVYAKDDHQKQTPIWSSQAGASASAIESTAWYG |
| gas57M22_23465 | (1200) | FVAFKGLKNNVYNDLTVNVYAKDDHQKQTPIWSSQAGASASAIESTAWYG |
| gas57M3,1_30610 | (1200) | FVAFKGLKNNVYNDLTVNVYAKDDHQKQTPIWSSQAGASASAIESTAWYG |
| gas57M3,1_40603 | (1200) | FVAFKGLKNNVYNDLTVNVYAKDDHQKQTPIWSSQAGASASAIESTAWYG |
| gas57M3,28_24214 | (1200) | FVAFKGLKNNVYNDLTVNVYAKDDHQKQTPIWSSQAGASASAIESTAWYG |
| gas57M3,34_10307 | (1200) | FVAFKGLKNNVYNDLTVNVYAKDDHQKQTPIWSSQAGASASAIESTAWYG |
| gas57M4_40427 | (1200) | FVAFKGLKNNVYNDLTVNVYAKDDHQKQTPIWSSQAGASASAIESTAWYG |
| gas57M3_2721 | (1200) | FVAFKGLKNNVYNDLTVNVYAKDDHQKQTPIWSSQAGASASAIESTAWYG |
| gas57M12_10296 | (1199) | FVAFKGLKNNVYNDLTVNVYAKDDHQKQTPIWSSQAGASASAIESTAWYG |
| gas57M12_10035 | (1199) | FVAFKGLKNNVYNDLTVNVYAKDDHQKQTPIWSSQAGASASAIESTAWYG |
| gas57M12_20069 | (1199) | FVAFKGLKNNVYNDLTVNVYAKDDHQKQTPIWSSQAGASASAIESTAWYG |
| gas57M12_22432 | (1199) | FVAFKGLKNNVYNDLTVNVYAKDDHQKQTPIWSSQAGASASAIESTAWYG |
| gas57M4_40499 | (1199) | FVAFKGLKNNVYNDLTVNVYAKDDHQKQTPIWSSQAGASASAIESTAWYG |
| gas57M6,1_21259 | (1199) | FVAFKGLKNNVYNDLTVNVYAKDDHQKQTPIWSSQAGASASAIESTAWYG |

FIG. 31Z

| | | 1251 1300 |
|---|---|---|
| gas57M1_SF370 | (1249) | ITARGSKVMPGDYQYVVTYRDEHGKEHQKQYTISVNDKKPMITQGRFDTI |
| gas57M1_31075 | (1249) | ITARGSKVMPGDYQYVVTYRDEHGKEHQKQYTISVNDKKPMITQGRFDTI |
| gas57M1_31237 | (1249) | ITARGSKVMPGDYQYVVTYRDEHGKEHQKQYTISVNDKKPMITQGRFDTI |
| gas57M1_3348 | (1249) | ITARGSKVMPGDYQYVVTYRDEHGKEHQKQYTISVNDKKPMITQGRFDTI |
| gas57M2_34585 | (1249) | ITARGSKVMPGDYQYVVTYRDEHGKEHQKQYTISVNDKKPMITQGRFDTI |
| gas57M3,1_21398 | (1249) | ITARGSKVMPGDYQYVVTYRDEHGKEHQKQYTISVNDKKPMITQGRFDTI |
| gas57M44-61_20839 | (1249) | ITARGSKVMPGDYQYVVTYRDEHGKEHQKQYTISVNDKKPMITQGRFDTI |
| gas57M6,31_20022 | (1248) | ITARGSKVMPGDYQYVVTYRDEHGKEHQKQYTISVNDKKPMITQGRFDTI |
| gas57M11_20648 | (1247) | ITARGSKVMPGDYQYVVTYRDEHGKEHQKQYTISVNDKKPMITQGRFDTI |
| gas57M23_2071 | (1248) | ITARGSKVMPGDYQYVVTYRDEHGKEHQKQYTISVNDKKPMITQGRFDTI |
| gas57M18,3_40128 | (1249) | ITARGSKVMPGDYQYVVTYRDEHGKEHQKQYTISVNDKKPMITQGRFDTI |
| gas57M4_10092 | (1249) | ITARGSKVMPGDYQYVVTYRDEHGKEHQKQYTISVNDKKPMITQGRFDTI |
| gas57M4_30968 | (1249) | ITARGSKVMPGDYQYVVTYRDEHGKEHQKQYTISVNDKKPMITQGRFDTI |
| gas57M6,31_22692 | (1249) | ITARGSKVMPGDYQYVVTYRDEHGKEHQKQYTISVNDKKPMITQGRFDTI |
| gas57M68,5_22814 | (1248) | ITARGSKVMPGDYQYVVTYRDEHGKVHQKQYTISVNDKKPMITQGRFDTI |
| gas57M68_23623 | (1249) | ITARGSKVMPGDYQYVVTYRDEHGKVHQKQYTISVNDKKPMITQGRFDTI |
| gas57M2_10064 | (1247) | ITARGSKVMPGDYQYVVTYRDEHGKEHQKQYTISVNDKKPMITQGRFDTI |
| gas57M2_10065 | (1247) | ITARGSKVMPGDYQYVVTYRDEHGKEHQKQYTISVNDKKPMITQGRFDTI |
| gas57M77_10251 | (1247) | ITARGSKVMPGDYQYVVTYRDEHGKEHQKQYTISVNDKKPMITQGRFDTI |
| gas57M77_10527 | (1247) | ITARGSKVMPGDYQYVVTYRDEHGKEHQKQYTISVNDKKPMITQGRFDTI |
| gas57M77_20696 | (1247) | ITARGSKVMPGDYQYVVTYRDEHGKEHQKQYTISVNDKKPMITQGRFDTI |
| gas57M89_21915 | (1249) | ITARGSKVMPGDYQYVVTYRDEHGKEHQKQYTISVNDKKPMITQGRFDTI |
| gas57M89_23717 | (1249) | ITARGSKVMPGDYQYVVTYRDEHGKEHQKQYTISVNDKKPMITQGRFDTI |
| gas57M94_10134 | (1247) | ITARGSKVMPGDYQYVVTYRDEHGKEHQKQYTISVNDKKPMITQGRFDTI |
| gas57M28_10164 | (1247) | ITARGSKVMPGDYQYVVTYRDEHGKEHQKQYTISVNDKKPMITQGRFDTI |
| gas57M28_10218 | (1247) | ITARGSKVMPGDYQYVVTYRDEHGKEHQKQYTISVNDKKPMITQGRFDTI |
| gas57M28_10266 | (1247) | ITARGSKVMPGDYQYVVTYRDEHGKEHQKQYTISVNDKKPMITQGRFDTI |
| gas57M28_10299 | (1247) | ITARGSKVMPGDYQYVVTYRDEHGKEHQKQYTISVNDKKPMITQGRFDTI |
| gas57M28_30176 | (1247) | ITARGSKVMPGDYQYVVTYRDEHGKEHQKQYTISVNDKKPMITQGRFDTI |
| gas57M28_30574 | (1247) | ITARGSKVMPGDYQYVVTYRDEHGKEHQKQYTISVNDKKPMITQGRFDTI |
| gas57M6,9_21802 | (1250) | ITARGSKVMPGDYQYVVTYRDEHGKEHQKQYTISVNDKKPMITQGRFDTI |
| gas57M75_10012 | (1251) | ITARGSKVMPGDYQYVVTYRDEHGKVHQKQYTISVNDKKPMITQGRFDTI |
| gas57M75_20671 | (1251) | ITARGSKVMPGDYQYVVTYRDEHGKVHQKQYTISVNDKKPMITQGRFDTI |
| gas57M75_30603 | (1251) | ITARGSKVMPGDYQYVVTYRDEHGKVHQKQYTISVNDKKPMITQGRFDTI |
| gas57M75_30207 | (1251) | ITARGSKVMPGDYQYVVTYRDEHGKVHQKQYTISVNDKKPMITQGRFDTI |
| gas57M22_20641 | (1250) | ITARGSKVMPGDYQYVVTYRDEHGKEHQKQYTISVNDKKPMITQGRFDTI |
| gas57M22_23465 | (1250) | ITARGSKVMPGDYQYVVTYRDEHGKEHQKQYTISVNDKKPMITQGRFDTI |
| gas57M3,1_30610 | (1250) | ITARGSKVMPGDYQYVVTYRDEHGKEHQKQYTISVNDKKPMITQGRFDTI |
| gas57M3,1_40603 | (1250) | ITARGSKVMPGDYQYVVTYRDEHGKEHQKQYTISVNDKKPMITQGRFDTI |
| gas57M3,28_24214 | (1250) | ITARGSKVMPGDYQYVVTYRDEHGKEHQKQYTISVNDKKPMITQGRFDTI |
| gas57M3,34_10307 | (1250) | ITARGSKVMPGDYQYVVTYRDEHGKEHQKQYTISVNDKKPMITQGRFDTI |
| gas57M4_40427 | (1250) | ITARGSKVMPGDYQYVVTYRDEHGKEHQKQYTISVNDKKPMITQGRFDTI |
| gas57M3_2721 | (1250) | ITARGSKVMPGDYQYVVTYRDEHGKEHQKQYTISVNDKKPMITQGRFDTI |
| gas57M12_10296 | (1249) | ITARGSKVMPGDYQYVVTYRDEHGKEHQKQYTISVNDKKPMITQGRFDTI |
| gas57M12_10035 | (1249) | ITARGSKVMPGDYQYVVTYRDEHGKEHQKQYTISVNDKKPMITQGRFDTI |
| gas57M12_20069 | (1249) | ITARGSKVMPGDYQYVVTYRDEHGKEHQKQYTISVNDKKPMITQGRFDTI |
| gas57M12_22432 | (1249) | ITARGSKVMPGDYQYVVTYRDEHGKEHQKQYTISVNDKKPMITQGRFDTI |
| gas57M4_40499 | (1249) | ITARGSKVMPGDYQYVVTYRDEHGKEHQKQYTISVNDKKPMITQGRFDTI |
| gas57M6,1_21259 | (1249) | ITARGSKVMPGDYQYVVTYRDEHGKEHQKQYTISVNDKKPMITQGRFDTI |

FIG. 31AA

| | | |
|---|---|---|
| | | 1301 1350 |
| gas57M1_SF370 | (1299) | NGVDHFTPDKTKALDSSGIVREEVFYLAKKNGRKFDVTEGKDGITVSDNK |
| gas57M1_31075 | (1299) | NGVDHFTPDKTKALGSSGIVREEVFYLAKKNGRKFDVTEGKDGITVSDNK |
| gas57M1_31237 | (1299) | NGVDHFTPDKTKALGSSGIVREEVFYLAKKNGRKFDVTEGKDGITVSDNK |
| gas57M1_3348 | (1299) | NGVDHFTPDKTKALGSSGIVREEVFYLAKKNGRKFDVTEGKDGITVSDNK |
| gas57M2_34585 | (1299) | NGVDHFTPDKTKALGSSGIVREEVFYLAKKNGRKFDVTEGKDGITVSDNK |
| gas57M3,1_21398 | (1299) | NGVDHFTPDKTKALGSSGIVREEVFYLAKKNGRKFDVTEGKDGITVSDNK |
| gas57M44-61_20839 | (1299) | NGVDHFTPDKTKALGSSGIVREEVFYLAKKNGRKFDVTEGKDGITVSDNK |
| gas57M6,31_20022 | (1298) | NGVDHFTPDKTKALGSSGIVREEVFYLAKKNGRKFDVTEGKDGITVSDNK |
| gas57M11_20648 | (1297) | NGVDHFTPDKTKALGSSGIVREEVFYLAKKNGRKFDVTEGKDGITVSDNK |
| gas57M23_2071 | (1298) | NGVDHFTPDKTKALGSSGIVREEVFYLAKKNGRKFDVTEGKDGITVSDNK |
| gas57M18,3_40128 | (1299) | NGVDHFTPDKTKALGSSGIVREEVFYLAKKNGRKFDVTEGKDGITVSDNK |
| gas57M4_10092 | (1299) | NGVDHFTPDKTKALGSSGIVREEVFYLAKKNGRKFDVTEGKDGITVSDNK |
| gas57M4_30968 | (1299) | NGVDHFTPDKTKALGSSGIVREEVFYLAKKNGRKFDVTEGKDGITVSDNK |
| gas57M6,31_22692 | (1299) | NGVDHFTPDKTKALGSSGIVREEVFYLAKKNGRKFDVTEGKDGITVSDNK |
| gas57M68,5_22814 | (1298) | NGVDHFTPDKTKALGSSGIVREEVFYLAKKNGRKFDVTEGKDGITVSDNK |
| gas57M68_23623 | (1299) | NGVDHFTPDKTKALGSSGIVREEVFYLAKKNGRKFDVTEGKDGITVSDNK |
| gas57M2_10064 | (1297) | NGVDHFTPDKTKALGSSGIVREEVFYLAKKNGRKFDVTEGKDGITVSDNK |
| gas57M2_10065 | (1297) | NGVDHFTPDKTKALGSSGIVREEVFYLAKKNGRKFDVTEGKDGITVSDNK |
| gas57M77_10251 | (1297) | NGVDHFTPDKTKALGSSGIVREEVFYLAKKNGRKFDVTEGKDGITVSDNK |
| gas57M77_10527 | (1297) | NGVDHFTPDKTKALGSSGIVREEVFYLAKKNGRKFDVTEGKDGITVSDNK |
| gas57M77_20696 | (1297) | NGVDHFTPDKTKALGSSGIVREEVFYLAKKNGRKFDVTEGKDGITVSDNK |
| gas57M89_21915 | (1299) | NGVDHFTPDKTKALGSSGIVREEVFYLAKKNGRKFDVTEGKDGITVSDNK |
| gas57M89_23717 | (1299) | NGVDHFTPDKTKALGSSGIVREEVFYLAKKNGRKFDVTEGKDGITVSDNK |
| gas57M94_10134 | (1297) | NGVDHFTPDKTKALGSSGIVREEVFYLAKKNGRKFDVTEGKDGITVSDNK |
| gas57M28_10164 | (1297) | NGVDHFTPDKTKALGSSGIVREEVFYLAKKNGRKFDVTEGKDGITVSDNK |
| gas57M28_10218 | (1297) | NGVDHFTPDKTKALGSSGIVREEVFYLAKKNGRKFDVTEGKDGITVSDNK |
| gas57M28_10266 | (1297) | NGVDHFTPDKTKALGSSGIVREEVFYLAKKNGRKFDVTEGKDGITVSDNK |
| gas57M28_10299 | (1297) | NGVDHFTPDKTKALGSSGIVREEVFYLAKKNGRKFDVTEGKDGITVSDNK |
| gas57M28_30176 | (1297) | NGVDHFTPDKTKALGSSGIVREEVFYLAKKNGRKFDVTEGKDGITVSDNK |
| gas57M28_30574 | (1297) | NGVDHFTPDKTKALGSSGIVREEVFYLAKKNGRKFDVTEGKDGITVSDNK |
| gas57M6,9_21802 | (1300) | NGVDHFTPDKTKALGSSGIVREEVFYLAKKNGRKFDVTEGKDGITVSDNK |
| gas57M75_10012 | (1301) | NGVDHFTPDKTKALGSSGIVREEVFYLAKKNGRKFDVTEGKDGITVSDNK |
| gas57M75_20671 | (1301) | NGVDHFTPDKTKALGSSGIVREEVFYLAKKNGRKFDVTEGKDGITVSDNK |
| gas57M75_30603 | (1301) | NGVDHFTPDKTKALGSSGIVREEVFYLAKKNGRKFDVTEGKDGITVSDNK |
| gas57M75_30207 | (1301) | NGVDHFTPDKTKALGSSGIVREEVFYLAKKNGRKFDVTEGKDGITVSDNK |
| gas57M22_20641 | (1300) | NGVDHFTPDKTKALGSSGIVREEVFYLAKKNGRKFDVTEGKDGITVSDNK |
| gas57M22_23465 | (1300) | NGVDHFTPDKTKALGSSGIVREEVFYLAKKNGRKFDVTEGKDGITVSDNK |
| gas57M3,1_30610 | (1300) | NGVDHFTPDKTKALGSSGIVREEVFYLAKKNGRKFDVTEGKDGITVSDNK |
| gas57M3,1_40603 | (1300) | NGVDHFTPDKTKALGSSGIVREEVFYLAKKNGRKFDVTEGKDGITVSDNK |
| gas57M3,28_24214 | (1300) | NGVDHFTPDKTKALGSSGIVREEVFYLAKKNGRKFDVTEGKDGITVSDNK |
| gas57M3,34_10307 | (1300) | NGVDHFTPDKTKALGSSGIVREEVFYLAKKNGRKFDVTEGKDGITVSDNK |
| gas57M4_40427 | (1300) | NGVDHFTPDKTKALGSSGIVREEVFYLAKKNGRKFDVTEGKDGITVSDNK |
| gas57M3_2721 | (1300) | NGVDHFTPDKTKALGSSGIVREEVFYLAKKNGRKFDVTEGKDGITVSDNK |
| gas57M12_10296 | (1299) | NGVDHFTPDKTKALGSSGIVREEVFYLAKKNGRKFDVTEGKDGITVSDNK |
| gas57M12_10035 | (1299) | NGVDHFTPDKTKALGSSGIVREEVFYLAKKNGRKFDVTEGKDGITVSDNK |
| gas57M12_20069 | (1299) | NGVDHFTPDKTKALGSSGIVREEVFYLAKKNGRKFDVTEGKDGITVSDNK |
| gas57M12_22432 | (1299) | NGVDHFTPDKTKALGSSGIVREEVFYLAKKNGRKFDVTEGKDGITVSDNK |
| gas57M4_40499 | (1299) | NGVDHFTPDKTKALGSSGIVREEVFYLAKKNGRKFDVTEGKDGITVSDNK |
| gas57M6,1_21259 | (1299) | NGVDHFTPDKTKALGSSGIVREEVFYLAKKNGRKFDVTEGKDGITVSDNK |

FIG. 31BB

| | | 1351 1400 |
|---|---|---|
| gas57M1_SF370 | (1349) | VYIPKNPDGSYTISKRDGVTLSDYYYLVEDRAGNVSFATLRDLKAVGKDK |
| gas57M1_31075 | (1349) | VYIPKNPDGSYTISKRDGVTLSDYYYLVEDRAGNVSFATLRDLKAVGKDK |
| gas57M1_31237 | (1349) | VYIPKNPDGSYTISKRDGVTLSDYYYLVEDRAGNVSFATLRDLKAVGKDK |
| gas57M1_3348 | (1349) | VYIPKNPDGSYTISKRDGVTLSDYYYLVEDRAGNVSFATLRDLKAVGKDK |
| gas57M2_34585 | (1349) | VYIPKNPDGSYTISKRDGVTLSDYYYLVEDRAGNVSFATLRDLKAVGKDK |
| gas57M3,1_21398 | (1349) | VYIPKNPDGSYTISKRDGVTLSDYYYLVEDRAGNVSFATLRDLKAVGKDK |
| gas57M44-61_20839 | (1349) | VYIPKNPDGSYTISKRDGVTLSDYYYLVEDRAGNVSFATLRDLKAVGKDK |
| gas57M6,31_20022 | (1348) | VYIPKNPDGSYTISKRDGVTLSDYYYLVEDRAGNVSFATLRDLKAVGKDK |
| gas57M11_20648 | (1347) | VYIPKNPDGSYTISKRDGVTLSDYYYLVEDRAGNVSFATLRDLKAVGKDK |
| gas57M23_2071 | (1348) | VYIPKNPDGSYTISKRDGVTLSDYYYLVEDRAGNVSFATLRDLKAVGKDK |
| gas57M18,3_40128 | (1349) | VYIPKNPDGSYTISKRDGVTLSDYYYLVEDRAGNVSFATLRDLKAVGKDK |
| gas57M4_10092 | (1349) | VYIPKNPDGSYTISKRDGVTLSDYYYLVEDRAGNVSFATLRDLKAVGKDK |
| gas57M4_30968 | (1349) | VYIPKNPDGSYTISKRDGVTLSDYYYLVEDRAGNVSFATLRDLKAVGKDK |
| gas57M6,31_22692 | (1349) | VYIPKNPDGSYTISKRDGVTLSDYYYLVEDRAGNVSFATLRDLKAVGKDK |
| gas57M68,5_22814 | (1348) | VYIPKNPDGSYTISKRDGVTLSDYYYLVEDRAGNVSFATLRDLKAVGKDK |
| gas57M68_23623 | (1349) | VYIPKNPDGSYTISKRDGVTLSDYYYLVEDRAGNVSFATLRDLKAVGKDK |
| gas57M2_10064 | (1347) | VYIPKNPDGSYTISKRDGVTLSDYYYLVEDRAGNVSFATLRDLKAVGKDK |
| gas57M2_10065 | (1347) | VYIPKNPDGSYTISKRDGVTLSDYYYLVEDRAGNVSFATLRDLKAVGKDK |
| gas57M77_10251 | (1347) | VYIPKNPDGSYTISKRDGVTLSDYYYLVEDRAGNVSFATLRDLKAVGKDK |
| gas57M77_10527 | (1347) | VYIPKNPDGSYTISKRDGVTLSDYYYLVEDRAGNVSFATLRDLKAVGKDK |
| gas57M77_20696 | (1347) | VYIPKNPDGSYTISKRDGVTLSDYYYLVEDRAGNVSFATLRDLKAVGKDK |
| gas57M89_21915 | (1349) | VYIPKNPDGSYTISKRDGVTLSDYYYLVEDRAGNVSFATLRDLKAVGKDK |
| gas57M89_23717 | (1349) | VYIPKNPDGSYTISKRDGVTLSDYYYLVEDRAGNVSFATLRDLKAVGKDK |
| gas57M94_10134 | (1347) | VYIPKNPDGSYTISKRDGVTLSDYYYLVEDRAGNVSFATLRDLKAVGKDK |
| gas57M28_10164 | (1347) | VYIPKNPDGSYTISKRDGVTLSDYYYLVEDRAGNVSFATLRDLKAVGKDK |
| gas57M28_10218 | (1347) | VYIPKNPDGSYTISKRDGVTLSDYYYLVEDRAGNVSFATLRDLKAVGKDK |
| gas57M28_10266 | (1347) | VYIPKNPDGSYTISKRDGVTLSDYYYLVEDRAGNVSFATLRDLKAVGKDK |
| gas57M28_10299 | (1347) | VYIPKNPDGSYTISKRDGVTLSDYYYLVEDRAGNVSFATLRDLKAVGKDK |
| gas57M28_30176 | (1347) | VYIPKNPDGSYTISKRDGVTLSDYYYLVEDRAGNVSFATLRDLKAVGKDK |
| gas57M28_30574 | (1347) | VYIPKNPDGSYTISKRDGVTLSDYYYLVEDRAGNVSFATLRDLKAVGKDK |
| gas57M6,9_21802 | (1350) | VYIPKNPDGSYTISKRDGVTLSDYYYLVEDRAGNVSFATLRDLKAVGKDK |
| gas57M75_10012 | (1351) | VYIPKNPDGSYTISKRDGVTLSDYYYLVEDRAGNVSFATLRDLKAVGKDK |
| gas57M75_20671 | (1351) | VYIPKNPDGSYTISKRDGVTLSDYYYLVEDRAGNVSFATLRDLKAVGKDK |
| gas57M75_30603 | (1351) | VYIPKNPDGSYTISKRDGVTLSDYYYLVEDRAGNVSFATLRDLKAVGKDK |
| gas57M75_30207 | (1351) | VYIPKNPDGSYTISKRDGVTLSDYYYLVEDRAGNVSFATLRDLKAVGKDK |
| gas57M22_20641 | (1350) | VYIPKNPDGSYTISKRDGVTLSDYYYLVEDRAGNVSFATLRDLKAVGKDK |
| gas57M22_23465 | (1350) | VYIPKNPDGSYTISKRDGVTLSDYYYLVEDRAGNVSFATLRDLKAVGKDK |
| gas57M3,1_30610 | (1350) | VYIPKNPDGSYTISKRDGVTLSDYYYLVEDRAGNVSFATLRDLKVVGKDK |
| gas57M3,1_40603 | (1350) | VYIPKNPDGSYTISKRDGVTLSDYYYLVEDRAGNVSFATLRDLKVVGKDK |
| gas57M3,28_24214 | (1350) | VYIPKNPDGSYTISKRDGVTLSDYYYLVEDRAGNVSFATLRDLKVVGKDK |
| gas57M3,34_10307 | (1350) | VYIPKNPDGSYTISKRDGVTLSDYYYLVEDRAGNVSFATLRDLKVVGKDK |
| gas57M4_40427 | (1350) | VYIPKNPDGSYTISKRDGVTLSDYYYLVEDRAGNVSFATLRDLKVVGKDK |
| gas57M3_2721 | (1350) | VYIPKNPDGSYTISKRDGVTLSDYYYLVEDRAGNVSFATLRDLKVVGKDK |
| gas57M12_10296 | (1349) | VYIPKNPDGSYTISKRDGVTLSDYYYLVEDRAGNVSFATLRDLKAVGKDK |
| gas57M12_10035 | (1349) | VYIPKNPDGSYTISKRDGVTLSDYYYLVEDRAGNVSFATLRDLKAVGKDK |
| gas57M12_20069 | (1349) | VYIPKNPDGSYTISKRDGVTLSDYYYLVEDRAGNVSFATLRDLKAVGKDK |
| gas57M12_22432 | (1349) | VYIPKNPDGSYTISKRDGVTLSDYYYLVEDRAGNVSFATLRDLKAVGKDK |
| gas57M4_40499 | (1349) | VYIPKNPDGSYTISKRDGVTLSDYYYLVEDRAGNVSFATLRDLKAVGKDK |
| gas57M6,1_21259 | (1349) | VYIPKNPDGSYTISKRDGVTLSDYYYLVEDRAGNVSFATLRDLKAVGKDK |

FIG. 31CC

```
                               1401                                              1450
     gas57M1_SF370  (1399) AVVNFGLDLPVPEDKQIVNFTYLVRDADGKPIENLEYYNNSGNSLILPYG
     gas57M1_31075  (1399) AVVNFGLDLPVPEDKQIVNFTYLVRDADGKPIENLEYYNNSGNSLILPYG
     gas57M1_31237  (1399) AVVNFGLDLPVPEDKQIVNFTYLVRDADGKPIENLEYYNNSGNSLILPYG
      gas57M1_3348  (1399) AVVNFGLDLPVPEDKQIVNFTYLVRDADGKPIENLEYYNNSGNSLILPYG
     gas57M2_34585  (1399) AVVNFGLDLPVPEDKQIVNFTYLVRDADGKPIENLEYYNNSGNSLILPYG
   gas57M3,1_21398  (1399) AVVNFGLDLPVPEDKQIVNFTYLVRDADGKPIENLEYYNNSGNSLILPYG
gas57M44-61_20839  (1399) AVVNFGLDLPVPEDKQIVNFTYLVRDADGKPIENLEYYNNSGNSLILPYG
  gas57M6,31_20022  (1398) AVVNFGLDLPVPEDKQIVNFTYLVRDADGKPIENLEYYNNSGNSLILPYG
    gas57M11_20648  (1397) AVVNFGLDLPVPEDKQIVNFTYLVRDADGKPIENLEYYNNSGNSLILPYG
     gas57M23_2071  (1398) AVVNFGLDLPVPEDKQIVNFTYLVRDADGKPIENLEYYNNSGNSLILPYG
  gas57M18,3_40128  (1399) AVVNFGLDLPVPEDKQIVNFTYLVRDADGKPIENLEYYNNSGNSLILPYG
     gas57M4_10092  (1399) AVVNFGLDLPVPEDKQIVNFTYLVRDADGKPIENLEYYNNSGNSLILPYG
     gas57M4_30968  (1399) AVVNFGLDLPVPEDKQIVNFTYLVRDADGKPIENLEYYNNSGNSLILPYG
  gas57M6,31_22692  (1399) AVVNFGLDLPVPEDKQIVNFTYLVRDADGKPIENLEYYNNSGNSLILPYG
  gas57M68,5_22814  (1398) AVVNFGLDLPVPEDKQIVNFTYLVRDADGKPIENLEYYNNSGNSLILPYG
    gas57M68_23623  (1399) AVVNFGLDLPVPEDKQIVNFTYLVRDADGKPIENLEYYNNSGNSLILPYG
     gas57M2_10064  (1397) AVVNFGLDLPVPEDKQIVNFTYLVRDADGKPIENLEYYNNSGNSLILPYG
     gas57M2_10065  (1397) AVVNFGLDLPVPEDKQIVNFTYLVRDADGKPIENLEYYNNSGNSLILPYG
    gas57M77_10251  (1397) AVVNFGLDLPVPEDKQIVNFTYLVRDADGKPIENLEYYNNSGNSLILPYG
    gas57M77_10527  (1397) AVVNFGLDLPVPEDKQIVNFTYLVRDADGKPIENLEYYNNSGNSLILPYG
    gas57M77_20696  (1397) AVVNFGLDLPVPEDKQIVNFTYLVRDADGKPIENLEYYNNSGNSLILPYG
    gas57M89_21915  (1399) AVVNFGLDLPVPEDKQIVNFTYLVRDADGKPIENLEYYNNSGNSLILPYG
    gas57M89_23717  (1399) AVVNFGLDLPVPEDKQIVNFTYLVRDADGKPIENLEYYNNSGNSLILPYG
    gas57M94_10134  (1397) AVVNFGLDLPVPEDKQIVNFTYLVRDADGKPIENLEYYNNSGNSLILPYG
    gas57M28_10164  (1397) AVVNFGLDLPVPEDKQIVNFTYLVRDADGKPIENLEYYNNSGNSLILPYG
    gas57M28_10218  (1397) AVVNFGLDLPVPEDKQIVNFTYLVRDADGKPIENLEYYNNSGNSLILPYG
    gas57M28_10266  (1397) AVVNFGLDLPVPEDKQIVNFTYLVRDADGKPIENLEYYNNSGNSLILPYG
    gas57M28_10299  (1397) AVVNFGLDLPVPEDKQIVNFTYLVRDADGKPIENLEYYNNSGNSLILPYG
    gas57M28_30176  (1397) AVVNFGLDLPVPEDKQIVNFTYLVRDADGKPIENLEYYNNSGNSLILPYG
    gas57M28_30574  (1397) AVVNFGLDLPVPEDKQIVNFTYLVRDADGKPIENLEYYNNSGNSLILPYG
   gas57M6,9_21802  (1400) AVVNFGLDLPVPEDKQIVNFTYLVRDADGKPIENLEYYNNSGNSLILPYG
    gas57M75_10012  (1401) AVVNFGLDLPVPEDKQIVNFTYLVRDADGKPIENLEYYNNSGNSLILPYG
    gas57M75_20671  (1401) AVVNFGLDLPVPEDKQIVNFTYLVRDADGKPIENLEYYNNSGNSLILPYG
    gas57M75_30603  (1401) AVVNFGLDLPVPEDKQIVNFTYLVRDADGKPIENLEYYNNSGNSLILPYG
    gas57M75_30207  (1401) AVVNFGLDLPVPEDKQIVNFTYLVRDADGKPIENLEYYNNSGNSLILPYG
    gas57M22_20641  (1400) AVVNFGLDLPVPEDKQIVNFTYLVRDADGKPIENLEYYNNSGNSLILPYG
    gas57M22_23465  (1400) AVVNFGLDLPVPEDKQIVNFTYLVRDADGKPIENLEYYNNSGNSLILPYG
   gas57M3,1_30610  (1400) AVVNFGLDLPVPEDKQIVNFTYLVRDADGKPIENLEYYNNSGNSLILPYG
   gas57M3,1_40603  (1400) AVVNFGLDLPVPEDKQIVNFTYLVRDADGKPIENLEYYNNSGNSLILPYG
  gas57M3,28_24214  (1400) AVVNFGLDLPVPEDKQIVNFTYLVRDADGKPIENLEYYNNSGNSLILPYG
  gas57M3,34_10307  (1400) AVVNFGLDLPVPEDKQIVNFTYLVRDADGKPIENLEYYNNSGNSLILPYG
     gas57M4_40427  (1400) AVVNFGLDLPVPEDKQIVNFTYLVRDADGKPIENLEYYNNSGNSLILPYG
      gas57M3_2721  (1400) AVVNFGLDLPVPEDKQIVNFTYLVRDADGKPIENLEYYNNSGNSLILPYG
    gas57M12_10296  (1399) AVVNFGLDLPVPEDKQIVNFTYLVRDADGKPIENLEYYNNSGNSLILPYG
    gas57M12_10035  (1399) AVVNFGLDLPVPEDKQIVNFTYLVRDADGKPIENLEYYNNSGNSLILPYG
    gas57M12_20069  (1399) AVVNFGLDLPVPEDKQIVNFTYLVRDADGKPIENLEYYNNSGNSLILPYG
    gas57M12_22432  (1399) AVVNFGLDLPVPEDKQIVNFTYLVRDADGKPIENLEYYNNSGNSLILPYG
     gas57M4_40499  (1399) AVVNFGLDLPVPEDKQIVNFTYLVRDADGKPIENLEYYNNSGNSLILPYG
   gas57M6,1_21259  (1399) AVVNFGLDLPVPEDKQIVNFTYLVRDADGKPIENLEYYNNSGNSLILPYG
```

FIG. 31DD

```
                          1451                                              1500
     gas57M1_SF370  (1449) KYTVELLTYDTNAAKLESDKIVSFTLSADNNFQQVTFKITMLATSQITAH
     gas57M1_31075  (1449) KYTVELLTYDTNAAKLESDKIVSFTLSADNNFQQVTFKITMLATSQITAH
     gas57M1_31237  (1449) KYTVELLTYDTNAAKLESDKIVSFTLSADNNFQQVTFKITMLATSQITAH
      gas57M1_3348  (1449) KYTVELLTYDTNAAKLESDKIVSFTLSADNNFQQVTFKITMLATSQITAH
     gas57M2_34585  (1449) KYTVELLTYDTNAAKLESDKIVSFTLSADNNFQQVTFKITMLATSQITAH
   gas57M3,1_21398  (1449) KYTVELLTYDTNAAKLESDKIVSFTLSADNNFQQVTFKMTMLATSQITAH
 gas57M44-61_20839  (1449) KYTVELLTYDTNAAKLESDKIVSFTLSADNNFQQVTFKMTILATSQITAH
  gas57M6,31_20022  (1448) KYTVELLTYDTNAAKLESDKIVSFTLSADNNFQQVTFKMTMLATSQITAH
    gas57M11_20648  (1447) KYTVELLTYDTNAAKLESDKIVSFTLSADNNFQQVTFKMTMLATSQITAH
     gas57M23_2071  (1448) KYTVELLTYDTNAAKLESDKIVSFTLSADNNFQQVTFKMTMLATSQITAH
  gas57M18,3_40128  (1449) KYTVELLTYDTNAAKLESDKIVSFTLSADNNFQQVTFKMTMLATSQITAH
     gas57M4_10092  (1449) KYTVELLTYDTNAAKLESDKIVSFTLSADNNFQQVTFKMTMLATSQITAH
     gas57M4_30968  (1449) KYTVELLTYDTNAAKLESDKIVSFTLSADNNFQQVTFKMTMLATSQITAH
  gas57M6,31_22692  (1449) KYTVELLTYDTNAAKLESDKIVSFTLSADNNFQQVTFKMTMLATSQITAH
  gas57M68,5_22814  (1448) KYTVELLTYDTNAAKLESDKIVSFTLSADNNFQQVTFKMTMLATSQITAH
    gas57M68_23623  (1449) KYTVELLTYDTNAAKLESDKIVSFTLSADNNFQQVTFKMTMLATSQITAH
     gas57M2_10064  (1447) KYTVELLTYDTNAAKLESDKIVSFTLSADNNFQQVTFKMTMLATSQITAH
     gas57M2_10065  (1447) KYTVELLTYDTNAAKLESDKIVSFTLSADNNFQQVTFKMTMLATSQITAH
    gas57M77_10251  (1447) KYTVELLTYDTNAAKLESDKIVSFTLSADNNFQQVTFKMTMLATSQITAH
    gas57M77_10527  (1447) KYTVELLTYDTNAAKLESDKIVSFTLSADNNFQQVTFKMTMLATSQITAH
    gas57M77_20696  (1447) KYTVELLTYDTNAAKLESDKIVSFTLSADNNFQQVTFKMTMLATSQITAH
    gas57M89_21915  (1449) KYTVELLTYDTNAAKLESDKIVSFTLSADNNFQQVTFKMTMLATSQITAH
    gas57M89_23717  (1449) KYTVELLTYDTNAAKLESDKIVSFTLSADNNFQQVTFKMTMLATSQITAH
    gas57M94_10134  (1447) KYTVELLTYDTNAAKLESDKIVSFTLSADNNFQQVTFKMTMLATSQITAH
    gas57M28_10164  (1447) KYTVELLTYDTNAAKLESDKIVSFTLSADNNFQQVTFKMTMLATSQITAH
    gas57M28_10218  (1447) KYTVELLTYDTNAAKLESDKIVSFTLSADNNFQQVTFKMTMLATSQITAH
    gas57M28_10266  (1447) KYTVELLTYDTNAAKLESDKIVSFTLSADNNFQQVTFKMTMLATSQITAH
    gas57M28_10299  (1447) KYTVELLTYDTNAAKLESDKIVSFTLSADNNFQQVTFKMTMLATSQITAH
    gas57M28_30176  (1447) KYTVELLTYDTNAAKLESDKIVSFTLSADNNFQQVTFKMTMLATSQITAH
    gas57M28_30574  (1447) KYTVELLTYDTNAAKLESDKIVSFTLSADNNFQQVTFKMTMLATSQITAH
   gas57M6,9_21802  (1450) KYTVELLTYDTNAAKLESDKIVSFTLSADNNFQQITFKMTMLATSQITAH
    gas57M75_10012  (1451) KYTVELLTYDTNAAKLESDKIVSFTLSADNNFQQVTFKMTMLATSQITAH
    gas57M75_20671  (1451) KYTVELLTYDTNAAKLESDKIVSFTLSADNNFQQVTFKMTMLATSQITAH
    gas57M75_30603  (1451) KYTVELLTYDTNAAKLESDKIVSFTLSADNNFQQVTFKMTMLATSQITAH
    gas57M75_30207  (1451) KYTVELLTYDTNAAKLESDKIVSFTLSADNNFQQVTFKMTMLATSQITAH
    gas57M22_20641  (1450) KYTVELLTYDTNAAKLESDKIVSFTLSADNNFQQVTFKMTMLATSQITAH
    gas57M22_23465  (1450) KYTVELLTYDTNAAKLESDKIVSFTLSADNNFQQVTFKMTMLATSQITAH
   gas57M3,1_30610  (1450) KYTVELLTYDTNAAKLESDKIVSFTLSADNNFQQVTFKMTMLATSQITAH
   gas57M3,1_40603  (1450) KYTVELLTYDTNAAKLESDKIVSFTLSADNNFQQVTFKMTMLATSQITAH
  gas57M3,28_24214  (1450) KYTVELLTYDTNAAKLESDKIVSFTLSADNNFQQVTFKMTMLATSQITAH
  gas57M3,34_10307  (1450) KYTVELLTYDTNAAKLESDKIVSFTLSADNNFQQVTFKMTMLATSQITAH
     gas57M4_40427  (1450) KYTVELLTYDTNAAKLESDKIVSFTLSADNNFQQVTFKMTMLATSQITAH
      gas57M3_2721  (1450) KYTVELLTYDTNAAKLESDKIVSFTLSADNNFQQVTFKMTMLATSQITAH
    gas57M12_10296  (1449) KYTVELLTYDTNAAKLESDKIVSFTLSADNNFQQVTFKMTMLATSQITAH
    gas57M12_10035  (1449) KYTVELLTYDTNAAKLESDKIVSFTLSADNNFQQVTFKMTMLATSQITAH
    gas57M12_20069  (1449) KYTVELLTYDTNAAKLESDKIVSFTLSADNNFQQVTFKMTMLATSQITAH
    gas57M12_22432  (1449) KYTVELLTYDTNAAKLESDKIVSFTLSADNNFQQVTFKMTMLATSQITAH
     gas57M4_40499  (1449) KYTVELLTYDTNAAKLESDKIVSFTLSADNNFQQVTFKMTMLATSQITAH
   gas57M6,1_21259  (1449) KYTVELLTYDTNAAKLESDKIVSFTLSADNNFQQVTFKMTMLATSQITAH
```

FIG. 31EE

| | | 1501 1550 |
|---|---|---|
| gas57M1_SF370 | (1499) | FDHLLPEGSRVSLKTAQDQLIPLEQSLYVPKAYGKTVQEGTYEVVVSLPK |
| gas57M1_31075 | (1499) | FDHLLPEGSRVSLKTAQDQLIPLEQSLYVPKAYGKTVQEGTYEVVVSLPK |
| gas57M1_31237 | (1499) | FDHLLPEGSRVSLKTAQDQLIPLEQSLYVPKAYGKTVQEGTYEVVVSLPK |
| gas57M1_3348 | (1499) | FDHLLPEGSRVSLKTAQDQLIPLEQSLYVPKAYGKTVQEGTYEVVVSLPK |
| gas57M2_34585 | (1499) | FDHLLPEGSRVSLKTAQDQLIPLEQSLYVPKAYGKTVQEGTYEVVVSLPK |
| gas57M3,1_21398 | (1499) | FDHLLPEGSRVSLKTAQGQLIPLEQSLYVPKAYGKTVQEGTYEVVVSLPK |
| gas57M44-61_20839 | (1499) | FDHLLPEGSRVSLKTAQGQLIPLEQSLYVPKAYGKTVQEGTYEVVVSLPK |
| gas57M6,31_20022 | (1498) | FDHLLPEGSRVSLKTAQGQLIPLEQSLYVPKAYGKTVQEGTYEVVVSLPK |
| gas57M11_20648 | (1497) | FDHLLPEGSRVSLKTAQGQLIPLEQSLYVPKAYGKTVQEGTYEVVVSLPK |
| gas57M23_2071 | (1498) | FDHLLPEGSRVSLKTAQGQLIPLEQSLYVPKAYGKTVQEGTYEVVVSLPK |
| gas57M18,3_40128 | (1499) | FDHLLPEGSRVSLKTAQGQLIPLEQSLYVPKAYGKTVQEDTYEVVVSLPK |
| gas57M4_10092 | (1499) | FDHLLPEGSRVSLKTAQGQLIPLEQSLYVPKAYGKTVQEDTYEVVVSLPK |
| gas57M4_30968 | (1499) | FDHLLPEGSRVSLKTAQGQLIPLEQSLYVPKAYGKTVQEDTYEVVVSLPK |
| gas57M6,31_22692 | (1499) | FDHLLPEGSRVSLKTAQGQLIPLEQSLYVPKAYGKTVQEDTYEVVVSLPK |
| gas57M68,5_22814 | (1498) | FDHLLPEGSRVSLKTAQGQLIPLEQSLYVPKAYGKTVQEGTYEVVVSLPK |
| gas57M68_23623 | (1499) | FDHLLPEGSRVSLKTAQGQLIPLEQSLYVPKAYGKTVQEGTYEVVVSLPK |
| gas57M2_10064 | (1497) | FDHLLPEGSRVSLKTAQGQLIPLEQSLYVPKAYGKTVQEGTYEVVVSLPK |
| gas57M2_10065 | (1497) | FDHLLPEGSRVSLKTAQGQLIPLEQSLYVPKAYGKTVQEGTYEVVVSLPK |
| gas57M77_10251 | (1497) | FDHLLPEGSRVSLKTAQGQLIPLEQSLYVPKAYGKTVQEGTYEVVVSLPK |
| gas57M77_10527 | (1497) | FDHLLPEGSRVSLKTAQGQLIPLEQSLYVPKAYGKTVQEGTYEVVVSLPK |
| gas57M77_20696 | (1497) | FDHLLPEGSRVSLKTAQGQLIPLEQSLYVPKAYGKTVQEGTYEVVVSLPK |
| gas57M89_21915 | (1499) | FDHLLPEGSRVSLKTAQGQLIPLGQSLYVPKAYGKTVQEGTYEVVVSLPK |
| gas57M89_23717 | (1499) | FDHLLPEGSRVSLKTAQGQLIPLGQSLYVPKAYGKTVQEGTYEVVVSLPK |
| gas57M94_10134 | (1497) | FDHLLPEGSRVSLKTAQGQLIPLEQSLYVPKAYGKTVQEGTYEVVVSLPK |
| gas57M28_10164 | (1497) | FDHLLPEGSRVSLKTAQGQLIPLEQSLYVPKAYGKTVQEGTYEVVVSLPK |
| gas57M28_10218 | (1497) | FDHLLPEGSRVSLKTAQGQLIPLEQSLYVPKAYGKTVQEGTYEVVVSLPK |
| gas57M28_10266 | (1497) | FDHLLPEGSRVSLKTAQGQLIPLEQSLYVPKAYGKTVQEGTYEVVVSLPK |
| gas57M28_10299 | (1497) | FDHLLPEGSRVSLKTAQGQLIPLEQSLYVPKAYGKTVQEGTYEVVVSLPK |
| gas57M28_30176 | (1497) | FDHLLPEGSRVSLKTAQGQLIPLEQSLYVPKAYGKTVQEGTYEVVVSLPK |
| gas57M28_30574 | (1497) | FDHLLPEGSRVSLKTAQGQLIPLEQSLYVPKAYGKTVQEGTYEVVVSLPK |
| gas57M6,9_21802 | (1500) | FDHLLPEGSRVSLKTAQGQLIPLEQSLYVPKAYGKTVQEGTYEVVVSLPK |
| gas57M75_10012 | (1501) | FDHLLPEGSRVSLKTAQGQLIPLEQSLYVPKAYGKTVQEGTYEVVVSLPK |
| gas57M75_20671 | (1501) | FDHLLPEGSRVSLKTAQGQLIPLEQSLYVPKAYGKTVQEGTYEVVVSLPK |
| gas57M75_30603 | (1501) | FDHLLPEGSRVSLKTAQGQLIPLEQSLYVPKAYGKTVQEGTYEVVVSLPK |
| gas57M75_30207 | (1501) | FDHLLPEGSRVSLKTAQGQLIPLEQSLYVPKAYGKTVQEGTYEVVVSLPK |
| gas57M22_20641 | (1500) | FDHLLPEGSRVSLKTAQGQLIPLEQSLYVPKAYGKTVQEGTYEVVVSLPK |
| gas57M22_23465 | (1500) | FDHLLPEGSRVSLKTAQGQLIPLEQSLYVPKAYGKTVQEGTYEVVVSLPK |
| gas57M3,1_30610 | (1500) | FDHLLPEGSRVSLKTAQGQLIPLEQSLYVPKAYGKTVQEGTYEVVVSLPK |
| gas57M3,1_40603 | (1500) | FDHLLPEGSRVSLKTAQGQLIPLEQSLYVPKAYGKTVQEGTYEVVVSLPK |
| gas57M3,28_24214 | (1500) | FDHLLPEGSRVSLKTAQGQLIPLEQSLYVPKAYGKTVQEGTYEVVVSLPK |
| gas57M3,34_10307 | (1500) | FDHLLPEGSRVSLKTAQGQLIPLEQSLYVPKAYGKTVQEGTYEVVVSLPK |
| gas57M4_40427 | (1500) | FDHLLPEGSRVSLKTAQGQLIPLEQSLYVPKAYGKTVQEGTYEVVVSLPK |
| gas57M3_2721 | (1500) | FDHLLPEGSRVSLKTAQGQLIPLEQSLYVPKAYGKTVQEGTYEVVVSLPK |
| gas57M12_10296 | (1499) | FDHLLPEGSRVSLKTAQGQLIPLEQSLYVPKAYGKTVQEGTYEVVVSLPK |
| gas57M12_10035 | (1499) | FDHLLPEGSRVSLKTAQGQLIPLEQSLYVPKAYGKTVQEGTYEVVVSLPK |
| gas57M12_20069 | (1499) | FDHLLPEGSRVSLKTAQGQLIPLEQSLYVPKAYGKTVQEGTYEVVVSLPK |
| gas57M12_22432 | (1499) | FDHLLPEGSRVSLKTAQGQLIPLEQSLYVPKAYGKTVQEGTYEVVVSLPK |
| gas57M4_40499 | (1499) | FDHLLPEGSRVSLKTAQGQLIPLEQSLYVPKAYGKTVQEGTYEVVVSLPK |
| gas57M6,1_21259 | (1499) | FDHLLPEGSRVSLKTAQGQLIPLEQSLYVPKAYGKTVQEGTYEVVVSLPK |

FIG. 31FF

| | | 1551 1600 |
|---|---|---|
| gas57M1_SF370 | (1549) | GYRIEGNTKVNTLPNEVHELSLRLVKVGDASDSTGDHKVMSKNNSQALTA |
| gas57M1_31075 | (1549) | GYRIEGNTKVNTLPNEVHELSLRLVKVGDASDSTGDHKVMSKNNSQALTA |
| gas57M1_31237 | (1549) | GYRIEGNTKVNTLPNEVHELSLRLVKVGDASDSTGDHKVMSKNNSQALTA |
| gas57M1_3348 | (1549) | GYRIEGNTKVNTLPNEVHELSLRLVKVGDASDSTGDHKVMSKNNSQALTA |
| gas57M2_34585 | (1549) | GYRIEGNTKVNTLPNEVHELSLRLVKVGDASDSTGDHKVMSKNNSQALTA |
| gas57M3,1_21398 | (1549) | GYRIEGNTKVNTLPNEVHELSLRLVKVGDASDSTGDHKVMSKNNSQALTA |
| gas57M44-61_20839 | (1549) | GYRIEGNTKVNALPNEVHELSLRLVKVGDASDSTGDHKVMSKNNSQALTA |
| gas57M6,31_20022 | (1548) | GYRIEGNTKVNALPNEVHELSLRLVKVGDASDSTGDHKVMSKNNSQALTA |
| gas57M11_20648 | (1547) | GYRIEGNTKVNTLPNEVHELSLRLVKVGDASDSTGDHKVMSKNNSQALTA |
| gas57M23_2071 | (1548) | GYRIEGNTKVNTLPNEVHELSLRLVKVGDASDSTGDHKVMSKNNSQALTA |
| gas57M18,3_40128 | (1549) | GYRIEGNTKVNTLPNEVHELSLRLVKVGDASDSTGDHKVMSKNNSQALTA |
| gas57M4_10092 | (1549) | GYRIEGNTKVNALPNEVHELSLRLVKVGDASDSTGDHKVMSKNNSQALTV |
| gas57M4_30968 | (1549) | GYRIEGNTKVNALPNEVHELSLRLVKVGDASDSTGDHKVMSKNNSQALTV |
| gas57M6,31_22692 | (1549) | GYRIEGNTKVNALPNEVHELSLRLVKVGDASDSTGDHKVMSKNNSQALTV |
| gas57M68,5_22814 | (1548) | GYRIEGNTKVNALPNEVHELSLRLVKVGDASDSTGDHKVMSKNNSQALTA |
| gas57M68_23623 | (1549) | GYRIEGNTKVNALPNEVHELSLRLVKVGDASDSTGDHKVMSKNNSQALTA |
| gas57M2_10064 | (1547) | GYRIEGNTKVNALPNEVHELSLRLVKVGDASDSTGDHKVMSKNNSQALTA |
| gas57M2_10065 | (1547) | GYRIEGNTKVNALPNEVHELSLRLVKVGDASDSTGDHKVMSKNNSQALTA |
| gas57M77_10251 | (1547) | GYRIEGNTKVNALPNEVHELSLRLVKVGDASDLTGDHKVMSKNNSQALTA |
| gas57M77_10527 | (1547) | GYRIEGNTKVNALPNEVHELSLRLVKVGDASDLTGDHKVMSKNNSQALTA |
| gas57M77_20696 | (1547) | GYRIEGNTKVNALPNEVHELSLRLVKVGDASDLTGDHKVMSKNNSQALTA |
| gas57M89_21915 | (1549) | GYRIEGNTKVNALPNEVHELSLRLVKVGDASDSTGDHKVMSKNNSQALTA |
| gas57M89_23717 | (1549) | GYRIEGNTKVNALPNEVHELSLRLVKVGDASDSTGDHKVMSKNNSQALTA |
| gas57M94_10134 | (1547) | GYRIEGNTKVNTLPNEVHELSLRLVKVGDASDSTGDHKVMSKNNSQALTA |
| gas57M28_10164 | (1547) | GYRIEGNTKVNTLPNEVHELSLRLVKVGDASDSTGDHKVMSKNNSQALTA |
| gas57M28_10218 | (1547) | GYRIEGNTKVNTLPNEVHELSLRLVKVGDASDSTGDHKVMSKNNSQALTA |
| gas57M28_10266 | (1547) | GYRIEGNTKVNTLPNEVHELSLRLVKVGDASDSTGDHKVMSKNNSQALTA |
| gas57M28_10299 | (1547) | GYRIEGNTKVNTLPNEVHELSLRLVKVGDASDSTGDHKVMSKNNSQALTA |
| gas57M28_30176 | (1547) | GYRIEGNTKVNTLPNEVHELSLRLVKVGDASDSTGDHKVMSKNNSQALTA |
| gas57M28_30574 | (1547) | GYRIEGNTKVNTLPNEVHELSLRLVKVGDASDSTGDHKVMSKNNSQALTA |
| gas57M6,9_21802 | (1550) | GYRIEGNTKVNTLPNEVHELSLRLVKVGDASDSTGDHKVMSKNNSQALTA |
| gas57M75_10012 | (1551) | GYRIEGNTKVNTLPNEVHELSLRLVKVGDASDSTGDHKVMSKNNSQALTA |
| gas57M75_20671 | (1551) | GYRIEGNTKVNTLPNEVHELSLRLVKVGDASDSTGDHKVMSKNNSQALTA |
| gas57M75_30603 | (1551) | GYRIEGNTKVNTLPNEVHELSLRLVKVGDASDSTGDHKVMSKNNSQALTA |
| gas57M75_30207 | (1551) | GYRIEGNTKVNTLPNEVHELSLRLVKVGDASDSTGDHKVMSKNNSQALTA |
| gas57M22_20641 | (1550) | GYRIEGNTKVNTLPNEVHELSLRLVKVGDASDSTGDHKVMSKNNSQALTA |
| gas57M22_23465 | (1550) | GYRIEGNTKVNTLPNEVHELSLRLVKVGDASDSTGDHKVMSKNNSQALTA |
| gas57M3,1_30610 | (1550) | GYRIEGDTKVNALPNEVHELSLRLVKVGDASDSTGDHKVMSKNNSQALTA |
| gas57M3,1_40603 | (1550) | GYRIEGDTKVNALPNEVHELSLRLVKVGDASDSTGDHKVMSKNNSQALTA |
| gas57M3,28_24214 | (1550) | GYRIEGDTKVNALPNEVHELSLRLVKVGDASDSTGDHKVMSKNNSQALTA |
| gas57M3,34_10307 | (1550) | GYRIEGDTKVNALPNEVHELSLRLVKVGDASDSTGDHKVMSKNNSQALTA |
| gas57M4_40427 | (1550) | GYRIEGDTKVNALPNEVHELSLRLVKVGDASDSTGDHKVMSKNNSQALTA |
| gas57M3_2721 | (1550) | GYRIEGDTKVNALPNEVHELSLRLVKVGDASDSTGDHKVMSKNNSQALTA |
| gas57M12_10296 | (1549) | GYRIEGNTKVNTLPNEVHELSLRLVKVGDASDSTGDHKVMSKNNSQALTA |
| gas57M12_10035 | (1549) | GYRIEGNTKVNTLPNEVHELSLRLVKVGDASDSTGDHKVMSKNNSQALTA |
| gas57M12_20069 | (1549) | GYRIEGNTKVNTLPNEVHELSLRLVKVGDASDSTGDHKVMSKNNSQALTA |
| gas57M12_22432 | (1549) | GYRIEGNTKVNTLPNEVHELSLRLVKVGDASDSTGDHKVMSKNNSQALTA |
| gas57M4_40499 | (1549) | GYRIEGNTKVNTLPNEVHELSLRLVKVGDASDSTGDHKVMSKNNSQALTA |
| gas57M6,1_21259 | (1549) | GYRIEGNTKVNTLPNEVHELSLRLVKVGDASDSTGDHKVMSKNNSQALTA |

FIG. 31GG

```
                              1601                                              1650
    gas57M1_SF370   (1599) SATPTKSTTSATAKALPSTGEKMGLKLRIVGLVLLGLTCVFSRKKSTKD-
    gas57M1_31075   (1599) SATPTKSTTSATAKALPSTGEKMGLKLRIVGLVLLGLTCVFSRKKSTKD-
    gas57M1_31237   (1599) SATPTKSTTSATAKALPSTGEKMGLKLRIVGLVLLGLTCVFSRKKSTKD-
     gas57M1_3348   (1599) SATPTKSTTSATAKALPSTGEKMGLKLRIVGLVLLGLTCVFSRKKSTKD-
    gas57M2_34585   (1599) SATPTKSTTSATAKALPSTGEKMGLKLRIVGLVLLGLTCVFSRKKSTKD-
  gas57M3,1_21398   (1599) SATPTKTTTSATAKALPSTGEKMGLKLRIVGLVLLGLTCVFSRKKSTKD-
gas57M44-61_20839   (1599) SATPTKTTTSATAKALPSAGEKMGLKLRIVGLVLLGLTCVFSRKKSTKD-
 gas57M6,31_20022   (1598) SARPTKTTTSATAKALPSAGEKMGLKLRIVGLVLLGLTCVFSRKKSTKE-
   gas57M11_20648   (1597) SATPTKTTTSATAKALPSAGEKMGLKLRIVGLVLLGLTCVFSRKKSTKD-
    gas57M23_2071   (1598) SATPTKTTTSATAKALPSAGEKMGLKLRIVGLVLLGLTCVFSRKKSTKD-
 gas57M18,3_40128   (1599) SATPTKTTTSATAKALPSTGEKMGLKLRIVGLVLLGLTCVFSRKKSTKD-
    gas57M4_10092   (1599) SATPTKTTTSATAKALPSAGEKMGLKLRIVGLVLLGLTCVFSRKKSTKD-
    gas57M4_30968   (1599) SATPTKTTTSATAKALPSAGEKMGLKLRIVGLVLLGLTCVFSRKKSTKD-
 gas57M6,31_22692   (1599) SATPTKTTTSATAKALPSAGEKMGLKLRIVGLVLLGLTCVFSRKKSTKD-
 gas57M68,5_22814   (1598) SATPTKTTTSATAKALPSTGEKMGLKLRIVGLVLLGLTCVFSRKKSTKD-
   gas57M68_23623   (1599) SATPTKTTTSATAKALPSTGEKMGLKLRIVGLVLLGLTCVFSRKKSTKD-
    gas57M2_10064   (1597) SATPTKTTTSATAKALPSTGEKMGLKLRIVGLVLLGLTCVFSRKKSTKD-
    gas57M2_10065   (1597) SATPTKTTTSATAKALPSTGEKMGLKLRIVGLVLLGLTCVFSRKKSTKD-
   gas57M77_10251   (1597) SATPTKTTTSATAKALPSAGEKMGLKLRIVGLVLLGLTCVFSRKKSTKD-
   gas57M77_10527   (1597) SATPTKTTTSATAKALPSAGEKMGLKLRIVGLVLLGLTCVFSRKKSTKD-
   gas57M77_20696   (1597) SATPTKTTTSATAKALPSAGEKMGLKLRIVGLVLLGLTCVFSRKKSTKD-
   gas57M89_21915   (1599) SATPTKTTTSATAKALPSAGEKMGLKLRIVGLVLLGLTCVFSRKKSTKD-
   gas57M89_23717   (1599) SATPTKTTTSATAKALPSAGEKMGLKLRIVGLVLLGLTCVFSRKKSTKD-
   gas57M94_10134   (1597) SATPTKTTTSATAKALPSAGEKMGLKLRIVGLVLLGLTCVFSRKKSTKD-
   gas57M28_10164   (1597) SATPTKTTTSATAKALPSAGEKMGLKLRIVGLVLLGLTCVFSRKKSTKD-
   gas57M28_10218   (1597) SATPTKTTTSATAKALPSAGEKMGLKLRIVGLVLLGLTCVFSRKKSTKD-
   gas57M28_10266   (1597) SATPTKTTTSATAKALPSAGEKMGLKLRIVGLVLLGLTCVFSRKKSTKD-
   gas57M28_10299   (1597) SATPTKTTTSATAKALPSAGEKMGLKLRIVGLVLLGLTCVFSRKKSTKD-
   gas57M28_30176   (1597) FATPTKTTTSATAKALPSAGEKMGLKLRIVGLVLLGLTCVFSRKKSTKD-
   gas57M28_30574   (1597) SATPTKTTTSATAKALPSAGEKMGLKLRIVGLVLLGLTCVFSRKKSTKD-
  gas57M6,9_21802   (1600) SATPTKTTTSATAKALPSAGEKMGLKLRIVGLVLLGLTCVFSRKKSTKD-
   gas57M75_10012   (1601) SATPTKTTTSATAKALPSTGEKMGLKLRIVGLVLLGLTCVFSRKKSTKD-
   gas57M75_20671   (1601) SATPTKTTTSATAKALPSTGEKMGLKLRIVGLVLLGLTCVFSRKKSTKD-
   gas57M75_30603   (1601) SATPTKTTTSATAKALPSTGEKMGLKLRIVGLVLLGLTCVFSRKKSTKD-
   gas57M75_30207   (1601) SATPTKTTTSATAKALPSTGEKMGLKLRIVGLVLLGLTCVFSRKKSTKE-
   gas57M22_20641   (1600) SATPTKTTTSATAKALPSTGEKMGLKLRIVGLVLLGLTCVFSRKKSTKD-
   gas57M22_23465   (1600) SATPTKTTTSATAKALPSTGEKMGLKLRIVGLVLLGLTCVFSRKKSTKD-
  gas57M3,1_30610   (1600) SATPTKTTTSATAKALPSAGEKMGLKLRIVGLVLLGLTCVFSRKKSTKD-
  gas57M3,1_40603   (1600) SATPTKTTTSATAKALPSAGEKMGLKLRIVGLVLLGLTCVFSRKKSTKD-
 gas57M3,28_24214   (1600) SATPTKTTTSATAKALPSAGEKMGLKLRIVGLVLLGLTCVFSRKKSTKD-
 gas57M3,34_10307   (1600) SATPTKTTTSATAKALPSAGEKMGLKLRIVGLVLLGLTCVFSRKKSTKD-
    gas57M4_40427   (1600) SATPTKTTTSATAKALPSAGEKMGLKLRIVGLVLLGLTCVFSRKKSTKD-
     gas57M3_2721   (1600) SATPTKTTTSATAKALPSAGEKMGLKLRIVGLVLLGLTCVFSRKKSTKD-
   gas57M12_10296   (1599) SATPTKTTTSATAKALPSAGEKMGLKLRIVGLVLLGLTCVFSRKKSTKD-
   gas57M12_10035   (1599) SATPTKTTTSATAKALPSAGEKMGLKLRIVGLVLLGLTCVFSRKKSTKD-
   gas57M12_20069   (1599) SATPTKTTTSATAKALPSAGEKMGLKLRIVGLVLLGLTCVFSRKKSTKD-
   gas57M12_22432   (1599) SATPTKTTTSATAKALPSAGEKMGLKLRIVGLVLLGLTCVFSRKKSTKD-
    gas57M4_40499   (1599) SATPTKTTTSATAKALPSAGEKMGLKLRIVGLVLLGLTCVFSRKKSTKD-
  gas57M6,1_21259   (1599) SATPTKTTTSATAKALPSAGEKMGLKLRIVGLVLLGLTCVFSRKKSTKD-
```

FIG. 32A

```
                                   1                                                50
gas40M1_SF370                (1) MDLEQTKPNQVKQKIALTSTIALLSASVGVSHQVKADDRASGETKASNTH
gas40clinicalisolate_4088    (1) MDLEQTKPNQVKQKIALTSTIALLSASVGVSHQVKADDRASGETKASNTH
gas40M11_2727                (1) MDLEQTKPNQVKQKIALTSTIALLSASVGVSHQVKADDRASGETKASNTH
gas40M22_20641               (1) MDLEQTKPNQVKQKIALTSTIALLSASVGVSHQVKADDRASGETKASNTH
gas40M22_23465               (1) MDLEQTKPNQVKQKIALTSTIALLSASVGVSHQVKADDRASGETKASNIH
gas40M22_23621               (1) MDLEQTKPNQVKQKIALTSTIALLSASVGVSHQVKADDRASGETKASNTH
gas40M3,1_30610              (1) MDLEQTKPNQVKQKIALTSTIALLSASVGVSHQVKADDRASGETKASNTH
gas40M3,1_40603              (1) MDLEQTKPNQVKQKIALTSTIALLSASVGVSHQVKADDRASGETKASNTH
gas40M3,34_10307             (1) MDLEQTKPNQVKQKIALTSTIALLSASVGVSHQVKADDRASGETKASNTH
gas40M3_MGAS315              (1) MDLEQTKPNQVKQKIALTSTIALLSASVGVSHQVKADDRASGETKASNTH
gas40M4_40427                (1) MDLEQTKPNQVKQKIALTSTIALLSASVGVSHQVKADDRASGETKASNTH
gas40M3_2721                 (1) MDLEQTKPNQVKQKIALTSTIALLSASVGVSHQVKADDRASGETKASNTH
gas40M3_3040                 (1) MDLEQTKPNQVKQKIALTSTIALLSASVGVSHQVKADDRASGETKASNTH
gas40M3_3135                 (1) MDLEQTKPNQVKQKIALTSTIALLSASVGVSHQVKADDRASGETKASNTH
gas40M12_10035               (1) MDLEQTKPNQVKQKIALTSTIALLSASVGVSHQVKADDRASGETKASNTH
gas40M12_22432               (1) MDLEQTKPNQVKQKIALTSTIALLSASVGVSHQVKADDRASGETKASNTH
gas40M4_40499                (1) MDLEQTKPNQVKQKIALTSTIALLSASVGVSHQVKADDRASGETKASNTH
gas40M12_2728(EM5)           (1) MDLEQTKPNQVKQKIALTSTIALLSASVGVSHQVKADDRASGETKASNTH
gas40M89_10070               (1) MDLEQTKPNQVKQKIALTSTIALLSASVGVSHQVKADDRASGETKASNTH
gas40M89_21915               (1) MDLEQTKPNQVKQKIALTSTIALLSASVGVSHQVKADDRASGETKASNTH
gas40M89_23717               (1) MDLEQTKPNQVKQKIALTSTIALLSASVGVSHQVKADDRASGETKASNTH
gas40M89_5476                (1) MDLEQTKPNQVKQKIALTSTIALLSASVGVSHQVKADDRASGETKASNTH
gas40M23_DSM2071             (1) MDLEQTKPNQVKQKIALTSTIALLSASVGVSHQVKADDRASGETKASNTH
gas40M4_2722                 (1) MDLEQTKPNQVKQKIALTSTIALLSASVGVSHQVKADDRASGETKASNTH
gas40M4_10092                (1) MDLEQTKPNQVKQKIALTSTIALLSASVGVSHQVKADDRASGETKASNTH
gas40M4_30968                (1) MDLEQTKPNQVKQKIALTSTIALLSASVGVSHQVKADDRASGETKASNTH
gas40M4_2634                 (1) MDLEQTKPNQVKQKIALTSTIALLSASVGVSHQVKADDRASGETKASNTH
gas40M28_10164               (1) MDLEQTKPNQVKQKIALTSTIALLSASVGVSHQVKADDRASGETKASNTH
gas40M28_10218               (1) MDLEQTKPNQVKQKIALTSTIALLSASVGVSHQVKADDRASGETKASNTH
gas40M28_10266               (1) MDLEQTKPNQVKQKIALTSTIALLSASVGVSHQVKADDRASGETKASNTH
gas40M28_10299               (1) MDLEQTKPNQVKQKIALTSTIALLSASVGVSHQVKADDRASGETKASNTH
gas40M28_30176               (1) MDLEQTKPNQVKQKIALTSTIALLSASVGVSHQVKADDRASGETKASNTH
gas40M28_4436                (1) MDLEQTKPNQVKQKIALTSTIALLSASVGVSHQVKADDRASGETKASNTH
gas40M8_2725                 (1) MDLEQTKPNQVKQKIALTSTIALLSASVGVSHQVKADDRASGETKASNTH
gas40M44_3776                (1) MDLEQTKPNQVKQKIALTSTIALLSASVGVSHQVKADDRASGETKASNTH
gas40M6_2724                 (1) MDLEQTKPNQVKQKIALTSTIALLSASVGVSHQVKADDRASGETKASNTH
gas40M6_2894                 (1) MDLEQTKPNQVKQKIALTSTIALLSASVGVSHQVKADDRASGETKASNTH
gas40M6_3650                 (1) MDLEQTKPNQVKQKIALTSTIALLSASVGVSHQVKADDRASGETKASNTH
gas40M6_5529                 (1) MDLEQTKPNQVKQKIALTSTIALLSASVGVSHQVKADDRASGETKASNTH
gas40M5_                     (1) MDLEQTKPNQVKQKIALTSTIALLSASVGVSHQVKADDRASGETKASNTH
gas40M77_4959                (1) MDLEQTKPNQVKQKIALTSTIALLSASVGVSHQVKADDRASGETKASNTH
gas40M2_10064                (1) MDLEQTKPNQVKQKIALTSTIALLSASVGVSHQVKADDRASGETKASNTH
gas40M2_10065                (1) MDLEQTKPNQVKQKIALTSTIALLSASVGVSHQVKADDRASGETKASNTH
gas40M75_5531                (1) MDLEQTKPNQVKQKIALTSTIALLSASVGVSHQVKADDRASGETKASNTH
gas40M50_4538                (1) MDLEQTKPNQVKQKIALTSTIALLSASVGVSHQVKADDRASGETKASNTH
gas40M62_5455                (1) MDLEQTKPNQVKQKIALTSTIALLSASVGVSHQVKADDRASGETKASNTH
gas40M44_5481                (1) MDLEQTKPNQVKQKIALTSTIALLSASVGVSHQVKADDRASEETKASNTH
gas40M5_4883                 (1) MDLEQTKPNQVKQKIALTSTIALLSASVGVSHQVKADDRASEETKASNTH
gas40M9?_2720                (1) MDLEQTKPNQVKQKIALTSTIALLSASVGVSHQVKADDRASGETKASNTH
gas40M2_2726                 (1) MDLEQPKPNQVKQKIALTSTIALLSASVGVSHHVKADDLAPEGAKASNTS
gas40M12_20296               (1) MDLEQTKPNQVKQKIALTSTIALLSASVGVSHQVKADDRASGETKASNTH
gas40M1_2580                 (1) MDLEQTKPNQVKQKIALTSTIALLSASVGVSHQVKADDRASGETKASNTH
gas40M1_2913                 (1) MDLEQTKPNQVKQKIALTSTIALLSASVGVSHQVKADDRASGETKASNTH
gas40M1_3280                 (1) MDLEQTKPNQVKQKIALTSTIALLSASVGVSHQVKADDRASGETKASNTH
gas40M1_3348                 (1) MDLEQTKPNQVKQKIALTSTIALLSASVGVSHQVKADDRASGETKASNTH
gas40M78_3789                (1) MDLEQTKPNQVKQKIALTSTIALLSASVGVSHQVKADDRASGETKASNTH
gas40M?_2719                 (1) MDLEQTKPNQVKQKIALTSTIALLSASVGVSHQVKADDRASGETKASNTH
```

FIG. 32B

| | | 51 100 |
|---|---|---|
| gas40M1_SF370 | (51) | DDSLPKPETIQEAKATIDAVEKTLSQQKAELTELATALTKTTAEINHLKE |
| gas40clinicalisolate_4088 | (51) | DDSLPKPETIQEAKATIEAVEKTLSQQKAELTELATALTKTTAEINHLKE |
| gas40M11_2727 | (51) | DDSLPKPETIQEAKATIEAVEKTLSQQKAELTELATALTKTTAEINHLKE |
| gas40M22_20641 | (51) | DDSLPKPETIQEAKATIEAVEKTLSQQKAELTELATALTKTTAEINHLKE |
| gas40M22_23465 | (51) | DDSLPKPETIQEAKATIEAVEKTLSQQKAELTELATALTKTTAEINHLKE |
| gas40M22_23621 | (51) | DDSLPKPETIQEAKATIEAVEKTLSQQKAELTELATALTKTTAEINHLKE |
| gas40M3,1_30610 | (51) | DDSLPKPETIQEAKATIDAVEKTLSQQKAELTELATALTKTTAEINHLKE |
| gas40M3,1_40603 | (51) | DDSLPKPETIQEAKATIDAVEKTLSQQKAELTELATALTKTTAEINHLKE |
| gas40M3,34_10307 | (51) | DDSLPKPETIQEAKATIDAVEKTLSQQKAELTELATALTKTTAEINHLKE |
| gas40M3_MGAS315 | (51) | DDSLPKPETIQEAKATIDAVEKTLSQQKAELTELATALTKTTAEINHLKE |
| gas40M4_40427 | (51) | DDSLPKPETIQEAKATIDAVEKTLSQQKAELTELATALTKTTAEINHLKE |
| gas40M3_2721 | (51) | DDSLPKPETIQEAKATIDAVEKTLSQQKAELTELATALTKTTAEINHLKE |
| gas40M3_3040 | (51) | DDSLPKPETIQEAKATIDAVEKTLSQQKAELTELATALTKTTAEINHLKE |
| gas40M3_3135 | (51) | DDSLPKPETIQEAKATIDAVEKTLSQQKAELTELATALTKTTAEINHLKE |
| gas40M12_10035 | (51) | DDSLPKPETIQEAKATIDAVEKTLSQQKAELTELATALTKTTAEINHLKE |
| gas40M12_22432 | (51) | DDSLPKPETIQEAKATIDAVEKTLSQQKAELTELATALTKTTAEINHLKE |
| gas40M4_40499 | (51) | DDSLPKPETIQEAKATIDAVEKTLSQQKAELTELATALTKTTAEINHLKE |
| gas40M12_2728(EM5) | (51) | DDSLPKPETIQEAKATIDAVEKTLSQQKTELTELATALTKTTAEINHLKE |
| gas40M89_10070 | (51) | DDSLPKPETIQEAKATIDAVEKTLSQQKAELTELATALTKTTAEINNLKE |
| gas40M89_21915 | (51) | DDSLPKPETIQEAKATIDAVEKTLSQQKAELTELATALTKTTAEINNLKE |
| gas40M89_23717 | (51) | DDSLPKPETIQEAKATIDAVEKTLSQQKAELTELATALTKTTAEINNLKE |
| gas40M89_5476 | (51) | DDSLPKPETIQEAKATIDAVEKTLSQQKAELTELATALTKTTAEINNLKE |
| gas40M23_DSM2071 | (51) | DDSLPKPETIQEAKATIDAVEKTLSQQKAELTKLATALTKTTAEINHLKE |
| gas40M4_2722 | (51) | DDSLPKPETIQEAKATIDAVEKTLSQQKAELTKLATALTKTTAEINHLKE |
| gas40M4_10092 | (51) | DDSLPKPETIQEAKATIDAVEKTLSQQKAELTKLATALTKTTAEINHLKE |
| gas40M4_30968 | (51) | DDSLPKPETIQEAKATIDAVEKTLSQQKAELTKLATALTKTTAEINHLKE |
| gas40M4_2634 | (51) | DDSLPKPETIQEAKATIDAVEKTLSQQKAELTKLATALTKTTAEINHLKE |
| gas40M28_10164 | (51) | DDSLPKPETIQEAKATIDAVEKTLSQQKAELTELATALTKTTAEINHLKE |
| gas40M28_10218 | (51) | DDSLPKPETIQEAKATIDAVEKTLSQQKAELTELATALTKTTAEINHLKE |
| gas40M28_10266 | (51) | DDSLPKPETIQEAKATIDAVEKTLSQQKAELTELATALTKTTAEINHLKE |
| gas40M28_10299 | (51) | DDSLPKPETIQEAKATIDAVEKTLSQQKAELTELATALTKTTAEINHLKE |
| gas40M28_30176 | (51) | DDSLPKPETIQEAKATIDAVEKTLSQQKAELTELATALTKTTAEINHLKE |
| gas40M28_4436 | (51) | DDSLPKPETIQEAKATIDAVEKTLSQQKAELTELATALTKTTAEINHLKE |
| gas40M8_2725 | (51) | DDSLPKPETIQEAKATIDAVEKTLSQQKAELTELATALTKTTAEINNLKE |
| gas40M44_3776 | (51) | DDSLPKPETIQEAKATIDAVEKTLSQQKAELTELATALTKTTAEINHLKE |
| gas40M6_2724 | (51) | DDSLPKPETIQEAKATIDAVEKTLSQQKAELTELATALTKTTAEINHLKE |
| gas40M6_2894 | (51) | DDSLPKPETIQEAKATIDAVEKTLSQQKAELTELATALTKTTAEINHLKE |
| gas40M6_3650 | (51) | DDSLPKPETIQEAKATIDAVEKTLSQQKAELTELATALTKTTAEINHLKE |
| gas40M6_5529 | (51) | DDSLPKPETIQEAKATIDAVEKTLSQQKAELTELATALTKTTAEINHLKE |
| gas40M5_ | (51) | DDSLPKPETIQEAKATIDAVEKTLSQQKAELTELATALTKTTAEINHLKE |
| gas40M77_4959 | (51) | DDSLPKPETIQEAKATIDAVEKTLSQQKAELTELATALTKTTAEINHLKE |
| gas40M2_10064 | (51) | DDSLPKPETIQEAKATIEAVEKTLSQQKTKLTELATALTKTTAEINHLKE |
| gas40M2_10065 | (51) | DDSLPKPETIQEAKATIEAVEKTLSQQKTKLTELATALTKTTAEINHLKE |
| gas40M75_5531 | (51) | DDSLPKPETIQEAKATIEAVEKTLSQQKTKLTELATALTKTTAEINHLKE |
| gas40M50_4538 | (51) | DDSLPKPETIQEAKATIEAVEKTLSQQKAKLTELATALTKTTAEINHLKE |
| gas40M62_5455 | (51) | DDSLPKPETIQEAKATIEAVEKTLSQQKAKLTELATALTKTTAEINHLKE |
| gas40M44_5481 | (51) | DDSLPKPETIQEAKATIEAVEKTLSQQKTKLTELATALTKTTAEINHLKE |
| gas40M5_4883 | (51) | DDSLPKPETIQEAKATIEAVEKTLSQQKTKLTELATALTKTTAEINHLKE |
| gas40M9?_2720 | (51) | DDSLPKPETIQEAKATIEAVEKTLSQQKAKLTELATALTKTTAEINHLKE |
| gas40M2_2726 | (51) | EESLPKTETCEEAKAAVEAVETNLNQQKAELTELATALTKTTAEINHLKE |
| gas40M12_20296 | (51) | DDSLPKPETIQEAKATIDAVEKTLSQQKAELTELATALTKTTAEINHLKE |
| gas40M1_2580 | (51) | DDSLPKPETIQEAKATIDAVEKTLSQQKAELTELATALTKTTAEINHLKE |
| gas40M1_2913 | (51) | DDSLPKPETIQEAKATIDAVEKTLSQQKAELTELATALTKTTAEINHLKE |
| gas40M1_3280 | (51) | DDSLPKPETIQEAKATIDAVEKTLSQQKAELTELATALTKTTAEINHLKE |
| gas40M1_3348 | (51) | DDSLPKPETIQEAKATIDAVEKTLSQQKAELTELATALTKTTAEINHLKE |
| gas40M78_3789 | (51) | DDSLPKPETIQEAKATIDAVEKTLSQQKAELTELATALTKTTAEINHLKE |
| gas40M?_2719 | (51) | DDSLPKPETIQEAKATIDAVEKTLSQQKAELTELATALTKTTAEINHLKE |

FIG. 32C

```
                                         101                                              150
            gas40M1_SF370        (101)   QQDNEQKALTSAQEIYTNTLASSEETLLAQGAEHQRELTATETELHNAQA
gas40clinicalisolate_4088        (101)   QQDNEQKALTSAQEIYTNTLASSEETLLAQGAEHQRELTATETELHNAQA
            gas40M11_2727        (101)   QQDNEQKALTSAQEIYTNTLASSEETLLAQGAEHQRELTATETELHNAQA
           gas40M22_20641        (101)   QQDNEQKALTSAQEIYTNTLASSEETLLAQGAEHQRELTATETELHNAQA
           gas40M22_23465        (101)   QQDNEQKALTSAQEIYTNTLASSEETLLAQGAEHQRELTATETELHNAQA
           gas40M22_23621        (101)   QQDNEQKALTSAQEIYTNTLASSEETLLAQGAEHQRELTATETELHNAQA
          gas40M3,1_30610        (101)   QQDNEQKALTSAQEIYTNTLASSEETLLAQGAEHQRELTATETELHNAQV
          gas40M3,1_40603        (101)   QQDNEQKALTSAQEIYTNTLASSEETLLAQGAEHQRELTATETELHNAQV
         gas40M3,34_10307        (101)   QQDNEQKALTSAQEIYTNTLASSEETLLAQGAEHQRELTATETELHNAQV
          gas40M3_MGAS315        (101)   QQDNEQKALTSAQEIYTNTLASSEETLLAQGAEHQRELTATETELHNAQV
            gas40M4_40427        (101)   QQDNEQKALTSAQEIYTNTLASSEETLLAQGAEHQRELTATETELHNAQV
             gas40M3_2721        (101)   QQDNEQKALTSAQEIYTNTLASSEETLLAQGAEHQRELTATETELHNAQV
             gas40M3_3040        (101)   QQDNEQKALTSAQEIYTNTLASSEETLLAQGAEHQRELTATETELHNAQV
             gas40M3_3135        (101)   QQDNEQKALTSAQEIYTNTLASSEETLLAQGAEHQRELTATETELHNAQV
           gas40M12_10035        (101)   QQDNEQKALTSAQEIYTNTLASSEETLLAQGAEHQRELTATETELHNAQA
           gas40M12_22432        (101)   QQDNEQKALTSAQEIYTNTLASSEETLLAQGAEHQRELTATETELHNAQA
            gas40M4_40499        (101)   QQDNEQKALTSAQEIYTNTLASSEETLLAQGAEHQRELTATETELHNAQA
      gas40M12_2728(EM5)         (101)   QQDNEQKALTSAQEIYTNTLASSEETLLAQGAEHQRELTATETELHNAQA
           gas40M89_10070        (101)   QQDNEQKALTSAQEIYTNTLASSEETLLAQGAEHQRELTATETELHNAQA
           gas40M89_21915        (101)   QQDNEQKALTSAQEIYTNTLASSEETLLAQGAEHQRELTATETELHNAQA
           gas40M89_23717        (101)   QQDNEQKALTSAQEIYTNTLASSEETLLAQGAEHQRELTATETELHNAQA
            gas40M89_5476        (101)   QQDNEQKALTSAQEIYTNTLASSEETLLAQGAEHQRELTATETELHNAQA
         gas40M23_DSM2071        (101)   QQDNEQKALTSAQEIYTNTLASSEETLLAQGAEHQRELTATETELHNAQA
             gas40M4_2722        (101)   QQDNEQKALTSAQEIYTNTLASSEETLLAQGAEHQRELTATETELHNAQA
            gas40M4_10092        (101)   QQDNEQKALTSAQEIYTNTLASSEETLLAQGAEHQRELTATETELHNAQA
            gas40M4_30968        (101)   QQDNEQKALTSAQEIYTNTLASSEETLLAQGAEHQRELTATETELHNAQA
             gas40M4_2634        (101)   QQDNEQKALTSAQEIYTNTLASSEETLLAQGAEHQRELTATETELHNAQV
           gas40M28_10164        (101)   QQDNEQKALTSAQEIYTNTLASSEETLLAQGAEHQRELTATETELHNAQA
           gas40M28_10218        (101)   QQDNEQKALTSAQEIYTNTLASSEETLLAQGAEHQRELTATETELHNAQA
           gas40M28_10266        (101)   QQDNEQKALTSAQEIYTNTLASSEETLLAQGAEHQRELTATETELHNAQA
           gas40M28_10299        (101)   QQDNEQKALTSAQEIYTNTLASSEETLLAQGAEHQRELTATETELHNAQA
           gas40M28_30176        (101)   QQDNEQKALTSAQEIYTNTLASSEETLLAQGAEHQRELTATETELHNAQA
            gas40M28_4436        (101)   QQDNEQKALTSAQEIYTNTLASSEETLLAQGAEHQRELTATETELHNAQA
             gas40M8_2725        (101)   QQDNEQKALTSAQEIYTNTLASSEETLLAQGAEHQRELTATETELHNAQA
            gas40M44_3776        (101)   QQDNEQKALTSAQEIYTNTLASSEETLLAQGAEHQRELTATETELHNAQV
             gas40M6_2724        (101)   QQDNEQKALTSAQEIYTNTLASSEETLLAQGAEHQRELTATETELHNAQV
             gas40M6_2894        (101)   QQDNEQKALTSAQEIYTNTLASSEETLLAQGAEHQRELTATETELHNAQV
             gas40M6_3650        (101)   QQDNEQKALTSAQEIYTNTLASSEETLLAQGAEHQRELTATETELHNAQV
             gas40M6_5529        (101)   QQDNEQKALTSAQEIYTNTLASSEETLLAQGAEHQRELTATETELHNAQV
                 gas40M5_        (101)   QQDNEQKALTSAQEIYTNTLASSEETLLAQGAEHQRELTATETELHNAQV
            gas40M77_4959        (101)   QQDNEQKALTSAQEIYTNTLASSEETLLAQGAEHQRELTATETELHNAQA
            gas40M2_10064        (101)   QQDNEQKALTSAQEIYTNTLASSEETLLAQGAEHQRELTATETELHNAQA
            gas40M2_10065        (101)   QQDNEQKALTSAQEIYTNTLASSEETLLAQGAEHQRELTATETELHNAQA
            gas40M75_5531        (101)   QQDNEQKALTSAQEIYTNTLASSEETLLAQGAEHQRELTATETELHNAQA
            gas40M50_4538        (101)   QQDNEQKALTSAQEIYTNTLASSEETLLAQGAEHQRELTATETELHNAQA
            gas40M62_5455        (101)   QQDNEQKALTSAQEIYTNTLASSEETLLAQGAEHQRELTATETELHNAQA
            gas40M44_5481        (101)   QQDNEQKALTSAQEIYTNTLASSEETLLAQGAEHQRELTATETELHNAQA
             gas40M5_4883        (101)   QQDNEQKALTSAQEIYTNTLASSEETLLAQGAEHQRELTATETELHNAQA
            gas40M9?_2720        (101)   QQDNEQKALTSAQEIYTNTLASSEETLLAQGAEHQRELTATETELHNAQV
             gas40M2_2726        (101)   QQDNEQKALTSAQEIYTNTLASSEETLLAQGAEYQRELTATETELHNAQV
           gas40M12_20296        (101)   QQDNEQKALTSAQEIYTNTLASSEETLLAQGAEHQRELTATETELHNAQA
             gas40M1_2580        (101)   QQDNEQKALTSAQEIYTNTLASSEETLLAQGAEHQRELTATETELHNAQA
             gas40M1_2913        (101)   QQDNEQKALTSAQEIYTNTLASSEETLLAQGAEHQRELTATETELHNAQA
             gas40M1_3280        (101)   QQDNEQKALTSAQEIYTNTLASSEETLLAQGAEHQRELTATETELHNAQA
             gas40M1_3348        (101)   QQDNEQKALTSAQEIYTNTLASSEETLLAQGAEHQRELTATETELHNAQA
            gas40M78_3789        (101)   QQDNEQKALTSAQEIYTNTLASSEETLLAQGAEHQRELTATETELHNAQA
             gas40M?_2719        (101)   QQDNEQKALTSAQEIYTNTLASSEETLLAQGAEHQRELTATETELHNAQA
```

FIG. 32D

| | | 151 200 |
|---|---|---|
| gas40M1_SF370 | (151) | DQHSKETALSEQKASISAETTRAQDLVEQVKTSEQNIAKLNAMISNPDAI |
| gas40clinicalisolate_4088 | (151) | DQHSKETALSEQKASISAETTRAQDLVEQVKTSEQNIAKLNAMISNPDAI |
| gas40M11_2727 | (151) | DQHSKETALSEQKASISAETTRAQDLVEQVKTSEQNIAKLNAMISNPDAI |
| gas40M22_20641 | (151) | DQHSKETALSEQKASISAETTRAQDLVEQVKTSEQNIAKLNAMISNPDAI |
| gas40M22_23465 | (151) | DQHSKETALSEQKASISAETTRAQDLVEQVKTSEQNIAKLNAMISNPDAI |
| gas40M22_23621 | (151) | DQHSKETALSEQKASISAETTRAQDLVEQVKTSEQNIAKLNAMISNPDAI |
| gas40M3,1_30610 | (151) | DQHSKETALSEQKASISAETTRAQDLVEQVKTSEQNIAKLNAMISNPDAI |
| gas40M3,1_40603 | (151) | DQHSKETALSEQKASISAETTRAQDLVEQVKTSEQNIAKLNAMISNPDAI |
| gas40M3,34_10307 | (151) | DQHSKETALSEQKASISAETTRAQDLVEQVKTSEQNIAKLNAMISNPDAI |
| gas40M3_MGAS315 | (151) | DQHSKETALSEQKASISAETTRAQDLVEQVKTSEQNIAKLNAMISNPDAI |
| gas40M4_40427 | (151) | DQHSKETALSEQKASISAETTRAQDLVEQVKTSEQNIAKLNAMISNPDAI |
| gas40M3_2721 | (151) | DQHSKETALSEQKASISAETTRAQDLVEQVKTSEQNIAKLNAMISNPDAI |
| gas40M3_3040 | (151) | DQHSKETALSEQKASISAETTRAQDLVEQVKTSEQNIAKLNAMISNPDAI |
| gas40M3_3135 | (151) | DQHSKETALSEQKASISAETTRAQDLVEQVKTSEQNIAKLNAMISNPDAI |
| gas40M12_10035 | (151) | DQHSKETALSEQKASISAETTRAQDLVEQVKTSEQNIAKLNAMISNPDAI |
| gas40M12_22432 | (151) | DQHSKETALSEQKASISAETTRAQDLVEQVKTSEQNIAKLNAMISNPDAI |
| gas40M4_40499 | (151) | DQHSKETALSEQKASISAETTRAQDLVEQVKTSEQNIAKLNAMISNPDAI |
| gas40M12_2728(EM5) | (151) | DQHSKETALSEQKASISAETTRAQDLVEQVKTSEQNIAKLNAMISNPDAI |
| gas40M89_10070 | (151) | DQHSKETALSEQKASISAETTRAQDLVEQVKTSEQNIAKLNAMISNPDAI |
| gas40M89_21915 | (151) | DQHSKETALSEQKASISAETTRAQDLVEQVKTSEQNIAKLNAMISNPDAI |
| gas40M89_23717 | (151) | DQHSKETALSEQKASISAETTRAQDLVEQVKTSEQNIAKLNAMISNPDAI |
| gas40M89_5476 | (151) | DQHSKETALSEQKASISAETTRAQDLVEQVKTSEQNIAKLNAMISNPDAI |
| gas40M23_DSM2071 | (151) | DQHSKETALSEQKASISAETTRAQDLVEQVKTSEQNIAKLNAMISNPDAI |
| gas40M4_2722 | (151) | DQHSKETALSEQKASISAETTRAQDLVEQVKTSEQNIAKLNAMISNPDAI |
| gas40M4_10092 | (151) | DQHSKETALSEQKASISAETTRAQDLVEQVKTSEQNIAKLNAMISNPDAI |
| gas40M4_30968 | (151) | DQHSKETALSEQKASISAETTRAQDLVEQVKTSEQNIAKLNAMISNPDAI |
| gas40M4_2634 | (151) | DQHSKETALSEQKASISAETTRAQDLVEQVKTSEQNIAKLNAMISNPDAI |
| gas40M28_10164 | (151) | DQHSKETALSEQKASISAETTRAQDLVEQVKTSEQNIAKLNAMISNPDAI |
| gas40M28_10218 | (151) | DQHSKETALSEQKASISAETTRAQDLVEQVKTSEQNIAKLNAMISNPDAI |
| gas40M28_10266 | (151) | DQHSKETALSEQKASISAETTRAQDLVEQVKTSEQNIAKLNAMISNPDAI |
| gas40M28_10299 | (151) | DQHSKETALSEQKASISAETTRAQDLVEQVKTSEQNIAKLNAMISNPDAI |
| gas40M28_30176 | (151) | DQHSKETALSEQKASISAETTRAQDLVEQVKTSEQNIAKLNAMISNPDAI |
| gas40M28_4436 | (151) | DQHSKETALSEQKASISAETTRAQDLVEQVKTSEQNIAKLNAMISNPDAI |
| gas40M8_2725 | (151) | DQHSKETALSEQKASISAETTRAQDLVEQVKTSEQNIAKLNAMISNPDAI |
| gas40M44_3776 | (151) | DQHSKETALSEQKASISAETTRAQDLVEQVKTSEQNIAKLNAMISNPDAI |
| gas40M6_2724 | (151) | DQHSKETALSEQKASISAETTRAQDLVEQVKTSEQNIAKLNAMISNPDAI |
| gas40M6_2894 | (151) | DQHSKETALSEQKASISAETTRAQDLVEQVKTSEQNIAKLNAMISNPDAI |
| gas40M6_3650 | (151) | DQHSKETALSEQKASISAETTRAQDLVEQVKTSEQNIAKLNAMISNPDAI |
| gas40M6_5529 | (151) | DQHSKETALSEQKASISAETTRAQDLVEQVKTSEQNIAKLNAMISNPDAI |
| gas40M5_ | (151) | DQHSKETALSEQKASISAETTRAQDLVEQVKTSEQNIAKLNAMISNPDAI |
| gas40M77_4959 | (151) | DQHSKETALSEQKASISAETTRAQDLVEQVKTSEQNIAKLNAMISNPDAI |
| gas40M2_10064 | (151) | DQHSKETALSEQKASISAETTRAQDLVEQVKTSEQNIAKLNAMINNPDAI |
| gas40M2_10065 | (151) | DQHSKETALSEQKASISAETTRAQDLVEQVKTSEQNIAKLNAMINNPDAI |
| gas40M75_5531 | (151) | DQHSKETALSEQKASISAETTRAQDLVEQVKTSEQNIAKLNAMINNPDAI |
| gas40M50_4538 | (151) | DQHSKETALSEQKASISAETTRAQDLVEQVKTSEQNIAKLNAMISNPDAI |
| gas40M62_5455 | (151) | DQHSKETALSEQKASISAETTRAQDLVEQVKTSEQNIAKLNAMISNPDAI |
| gas40M44_5481 | (151) | DQHSKETALSEQKASISAETTRAQDLVEQVKTSEQNIAKLNAMISNPDAI |
| gas40M5_4883 | (151) | DQHSKETALSEQKASISAETTRAQDLVEQVKTSEQNIAKLNAMISNPDAI |
| gas40M9?_2720 | (151) | DQHSKETALSEQKASISAETTRAQDLVEQVKTSEQNIAKLNAMISNPDAI |
| gas40M2_2726 | (151) | DQHSKETALSEQKASISAETTRAQDLVEQVKTSEQNIAKLNAMISNPDAI |
| gas40M12_20296 | (151) | DQHSKETALSEQKASISAETTRAQDLVEQVKTSEQNIAKLNAMISNPDAI |
| gas40M1_2580 | (151) | DQHSKETALSEQKASISAETTRAQDLVEQVKTSEQNIAKLNAMISNPDAI |
| gas40M1_2913 | (151) | DQHSKETALSEQKASISAETTRAQDLVEQVKTSEQNIAKLNAMISNPDAI |
| gas40M1_3280 | (151) | DQHSKETALSEQKASISAETTRAQDLVEQVKTSEQNIAKLNAMISNPDAI |
| gas40M1_3348 | (151) | DQHSKETALSEQKASISAETTRAQDLVEQVKTSEQNIAKLNAMISNPDAI |
| gas40M78_3789 | (151) | DQHSKETALSEQKASISAETTRAQDLVEQVKTSEQNIAKLNAMISNPDAI |
| gas40M?_2719 | (151) | DQHSKETALSEQKASISAETTRAQDLVEQVKTSEQNIAKLNAMISNPDAI |

FIG. 32E

| | | 201 250 |
|---|---|---|
| gas40M1_SF370 | (201) | TKAAQTANDNTKALSSELEKAKADLENQKAKVKKQLTEELAAQKAALAEK |
| gas40clinicalisolate_4088 | (201) | TKAAQTANDNTKALSSELEKAKADLENQKAKVKKQLTEELAAQKAALAEK |
| gas40M11_2727 | (201) | TKAAQTANDNTKALSSELEKAKADLENQKAKVKKQLTEELAAQKAALAEK |
| gas40M22_20641 | (201) | TKAAQTANDNTKALSSELEKAKADLENQKAKVKKQLTEELAAQKAALAEK |
| gas40M22_23465 | (201) | TKAAQTANDNTKALSSELEKAKADLENQKAKVKKQLTEELAAQKAALAEK |
| gas40M22_23621 | (201) | TKAAQTANDNTKALSSELEKAKADLENQKAKVKKQLTEELAAQKAALAEK |
| gas40M3,1_30610 | (201) | TKAAQTANDNTKALSSELEKAKADLENQKAKVKKQLTEELAAQKAALAEK |
| gas40M3,1_40603 | (201) | TKAAQTANDNTKALSSELEKAKADLENQKAKVKKQLTEELAAQKAALAEK |
| gas40M3,34_10307 | (201) | TKAAQTANDNTKALSSELEKAKADLENQKAKVKKQLTEELAAQKAALAEK |
| gas40M3_MGAS315 | (201) | TKAAQTANDNTKALSSELEKAKADLENQKAKVKKQLTEELAAQKAALAEK |
| gas40M4_40427 | (201) | TKAAQTANDNTKALSSELEKAKADLENQKAKVKKQLTEELAAQKAALAEK |
| gas40M3_2721 | (201) | TKAAQTANDNTKALSSELEKAKADLENQKAKVKKQLTEELAAQKAALAEK |
| gas40M3_3040 | (201) | TKAAQTANDNTKALSSELEKAKADLENQKAKVKKQLTEELAAQKAALAEK |
| gas40M3_3135 | (201) | TKAAQTANDNTKALSSELEKAKADLENQKAKVKKQLTEELAAQKAALAEK |
| gas40M12_10035 | (201) | TKAAQTANDNTKALSSELEKAKADLENQKAKVKKQLTEELAAQKAALAEK |
| gas40M12_22432 | (201) | TKAAQTANDNTKALSSELEKAKADLENQKAKVKKQLTEELAAQKAALAEK |
| gas40M4_40499 | (201) | TKAAQTANDNTKALSSELEKAKADLENQKAKVKKQLTEELAAQKAALAEK |
| gas40M12_2728(EM5) | (201) | TKAAQTANDNTKALSSELEKAKADLENQKAKVKKQLTEELAAQKAALAEK |
| gas40M89_10070 | (201) | TKAAQTANDNTKALSSELEKAKADLENQKAKVKKQLTEELAAQKAALAEK |
| gas40M89_21915 | (201) | TKAAQTANDNTKALSSELEKAKADLENQKAKVKKQLTEELAAQKAALAEK |
| gas40M89_23717 | (201) | TKAAQTANDNTKALSSELEKAKADLENQKAKVKKQLTEELAAQKAALAEK |
| gas40M89_5476 | (201) | TKAAQTANDNTKALSSELEKAKADLENQKAKVKKQLTEELAAQKAALAEK |
| gas40M23_DSM2071 | (201) | TKAAQTANDNTKALSSELEKAKADLENQKAKVKKQLTEELAAQKAALAEK |
| gas40M4_2722 | (201) | TKAAQTANDNTKALSSELEKAKADLENQKAKVKKQLTEELAAQKAALAEK |
| gas40M4_10092 | (201) | TKAAQTANDNTKALSSELEKAKADLENQKAKVKKQLTEELAAQKAALAEK |
| gas40M4_30968 | (201) | TKAAQTANDNTKALSSELEKAKADLENQKAKVKKQLTEELAAQKAALAEK |
| gas40M4_2634 | (201) | TKAAQTANDNTKALSSELEKAKADLENQKAKVKKQLTEELAAQKAALAEK |
| gas40M28_10164 | (201) | TKAAQTANDNTKALSSELEKAKADLENQKAKVKKQLTEELAAQKAALAEK |
| gas40M28_10218 | (201) | TKAAQTANDNTKALSSELEKAKADLENQKAKVKKQLTEELAAQKAALAEK |
| gas40M28_10266 | (201) | TKAAQTANDNTKALSSELEKAKADLENQKAKVKKQLTEELAAQKAALAEK |
| gas40M28_10299 | (201) | TKAAQTANDNTKALSSELEKAKADLENQKAKVKKQLTEELAAQKAALAEK |
| gas40M28_30176 | (201) | TKAAQTANDNTKALSSELEKAKADLENQKAKVKKQLTEELAAQKAALAEK |
| gas40M28_4436 | (201) | TKAAQTANDNTKALSSELEKAKADLENQKAKVKKQLTEELAAQKAALAEK |
| gas40M8_2725 | (201) | TKAAQTANDNTKALSSELEKAKADLENQKAKVKKQLTEELAAQKAALAEK |
| gas40M44_3776 | (201) | TKAAQTANDNTKALSSELEKAKADLENQKAKVKKQLTEELAAQKAALAEK |
| gas40M6_2724 | (201) | TKAAQTANDNTKALSSELEKAKADLENQKAKVKKQLTEELAAQKAALAEK |
| gas40M6_2894 | (201) | TKAAQTANDNTKALSSELEKAKADLENQKAKVKKQLTEELAAQKAALAEK |
| gas40M6_3650 | (201) | TKAAQTANDNTKALSSELEKAKADLENQKAKVKKQLTEELAAQKAALAEK |
| gas40M6_5529 | (201) | TKAAQTANDNTKALSSELEKAKADLENQKAKVKKQLTEELAAQKAALAEK |
| gas40M5_ | (201) | TKAAQTANDNTKALSSELEKAKADLENQKAKVKKQLTEELAAQKAALAEK |
| gas40M77_4959 | (201) | TKAAQTANDNTKALSSELEKAKADLENQKAKVKKQLTEELAAQKAALAEK |
| gas40M2_10064 | (201) | TKAAQTANDNTKALSSELEKAKADLENQKAKVKKQLTEELAAQKAALAEK |
| gas40M2_10065 | (201) | TKAAQTANDNTKALSSELEKAKADLENQKAKVKKQLTEELAAQKAALAEK |
| gas40M75_5531 | (201) | TKAAQTANDNTKALSSELEKAKADLENQKAKVKKQLTEELAAQKAALAEK |
| gas40M50_4538 | (201) | TKAAQTANDNTKALSSELEKAKADLENQKAKVKKQLTEELAAQKAALAEK |
| gas40M62_5455 | (201) | TKAAQTANDNTKALSSELEKAKADLENQKAKVKKQLTEELAAQKAALAEK |
| gas40M44_5481 | (201) | TKAAQTANDNTKALSSELEKAKADLENQKAKVKKQLTEELAAQKAALAEK |
| gas40M5_4883 | (201) | TKAAQTANDNTKALSSELEKAKADLENQKAKVKKQLTEELAAQKAALAEK |
| gas40M9?_2720 | (201) | TKAAQTANDNTKALSSELEKAKADLENQKAKVKKQLTEELAAQKAALAEK |
| gas40M2_2726 | (201) | TKAAQTANDNTKALSSELEKAKADLENQKAKVKKQLTEELAAQKAALAEK |
| gas40M12_20296 | (201) | TKAAQTANDNTKALSSELEKAKADLENQKAKVKKQLTEELAAQKAALAEK |
| gas40M1_2580 | (201) | TKAAQTANDNTKALSSELEKAKADLENQKAKVKKQLTEELAAQKAALAEK |
| gas40M1_2913 | (201) | TKAAQTANDNTKALSSELEKAKADLENQKAKVKKQLTEELAAQKAALAEK |
| gas40M1_3280 | (201) | TKAAQTANDNTKALSSELEKAKADLENQKAKVKKQLTEELAAQKAALAEK |
| gas40M1_3348 | (201) | TKAAQTANDNTKALSSELEKAKADLENQKAKVKKQLTEELAAQKAALAEK |
| gas40M78_3789 | (201) | TKAAQTANDNTKALSSELEKAKADLENQKAKVKKQLTEELAAQKAALAEK |
| gas40M?_2719 | (201) | TKAAQTANDNTKALSSELEKAKADLENQKAKVKKQLTEELAAQKAALAEK |

FIG. 32F

| | | 251 300 |
|---|---|---|
| gas40M1_SF370 | (251) | EAELSRLKSSAPSTQDSIVGNNTMKAPQGYPLEELKKLEASGYIGSASYN |
| gas40clinicalisolate_4088 | (251) | EAELSRLKSSAPSTQDSIVGNNTMKAPQGYPLEELKKLEASGYIGSASYN |
| gas40M11_2727 | (251) | EAELSRLKSSAPSTQDSIVGNNTMKAPQGYPLEELKKLEASGYIGSASYN |
| gas40M22_20641 | (251) | EAELSRLKSSAPSTQDSIVGNNTMKAPQGYPLEELKKLEASGYIGSASYN |
| gas40M22_23465 | (251) | EAELSRLKSSAPSTQDSIVGNNTMKAPQGYPLEELKKLEASGYIGSASYN |
| gas40M22_23621 | (251) | EAELSRLKSSAPSTQDSIVGNNTMKAPQGYPLEELKKLEASGYIGSASYN |
| gas40M3,1_30610 | (251) | EAELSRLKSSAPSTQDSIVGNNTMKAPQGYPLEELKKLEASGYIGSASYN |
| gas40M3,1_40603 | (251) | EAELSRLKSSAPSTQDSIVGNNTMKAPQGYPLEELKKLEASGYIGSASYN |
| gas40M3,34_10307 | (251) | EAELSRLKSSAPSTQDSIVGNNTMKAPQGYPLEELKKLEASGYIGSASYN |
| gas40M3_MGAS315 | (251) | EAELSRLKSSAPSTQDSIVGNNTMKAPQGYPLEELKKLEASGYIGSASYN |
| gas40M4_40427 | (251) | EAELSRLKSSAPSTQDSIVGNNTMKAPQGYPLEELKKLEASGYIGSASYN |
| gas40M3_2721 | (251) | EAELSRLKSSAPSTQDSIVGNNTMKAPQGYPLEELKKLEASGYIGSASYN |
| gas40M3_3040 | (251) | EAELSRLKSSAPSTQDSIVGNNTMKAPQGYPLEELKKLEASGYIGSASYN |
| gas40M3_3135 | (251) | EAELSRLKSSAPSTQDSIVGNNTMKAPQGYPLEELKKLEASGYIGSASYN |
| gas40M12_10035 | (251) | EAELSRLKSSAPSTQDSIVGNNTMKAPQGYPLEELKKLEASGYIGSASYN |
| gas40M12_22432 | (251) | EAELSRLKSSAPSTQDSIVGNNTMKAPQGYPLEELKKLEASGYIGSASYN |
| gas40M4_40499 | (251) | EAELSRLKSSAPSTQDSIVGNNTMKAPQGYPLEELKKLEASGYIGSASYN |
| gas40M12_2728(EM5) | (251) | EAELSRLKSSAPSTQDSIVGNNTMKAPQGYPLEELKKLEASGYIGSASYN |
| gas40M89_10070 | (251) | EAELSRLKSSAPSTQDSIVGNNTMKAPQGYPLEELKKLEASGYIGSASYN |
| gas40M89_21915 | (251) | EAELSRLKSSAPSTQDSIVGNNTMKAPQGYPLEELKKLEASGYIGSASYN |
| gas40M89_23717 | (251) | EAELSRLKSSAPSTQDSIVGNNTMKAPQGYPLEELKKLEASGYIGSASYN |
| gas40M89_5476 | (251) | EAELSRLKSSAPSTQDSIVGNNTMKAPQGYPLEELKKLEASGYIGSASYN |
| gas40M23_DSM2071 | (251) | EAELSRLKSSAPSTQDSIVGNNTMKAPQGYPLEELKKLEASGYIGSASYN |
| gas40M4_2722 | (251) | EAELSRLKSSAPSTQDSIVGNNTMKAPQGYPLEELKKLEASGYIGSASYN |
| gas40M4_10092 | (251) | EAELSRLKSSAPSTQDSIVGNNTMKAPQGYPLEELKKLEASGYIGSASYN |
| gas40M4_30968 | (251) | EAELSRLKSSAPSTQDSIVGNNTMKAPQGYPLEELKKLEASGYIGSASYN |
| gas40M4_2634 | (251) | EAELSRLKSSAPSTQDSIVGNNTMKAPQGYPLEELKKLEASGYIGSASYN |
| gas40M28_10164 | (251) | EAELSRLKSSAPSTQDSIVGNNTMKVPQGYPLEELKKLEASGYIGSASYN |
| gas40M28_10218 | (251) | EAELSRLKSSAPSTQDSIVGNNTMKVPQGYPLEELKKLEASGYIGSASYN |
| gas40M28_10266 | (251) | EAELSRLKSSAPSTQDSIVGNNTMKVPQGYPLEELKKLEASGYIGSASYN |
| gas40M28_10299 | (251) | EAELSRLKSSAPSTQDSIVGNNTMKVPQGYPLEELKKLEASGYIGSASYN |
| gas40M28_30176 | (251) | EAELSRLKSSAPSTQDSIVGNNTMKVPQGYPLEELKKLEASGYIGSASYN |
| gas40M28_4436 | (251) | EAELSRLKSSAPSTQDSIVGNNTMKVPQGYPLEELKKLEASGYIGSASYN |
| gas40M8_2725 | (251) | EAELSRLKSSAPSTQDSIVGNNTMKAPQGYPLEELKKLEASGYIGSASYN |
| gas40M44_3776 | (251) | EAELSRLKSSAPSTQDSIVGNNTMKAPQGYPLEELKKLEASGYIGSASYN |
| gas40M6_2724 | (251) | EAELSRLKSSAPSTQDSIVGNNTMKAPQGYPLEELKKLEASGYIGSASYN |
| gas40M6_2894 | (251) | EAELSRLKSSAPSTQDSIVGNNTMKAPQGYPLEELKKLEASGYIGSASYN |
| gas40M6_3650 | (251) | EAELSRLKSSAPSTQDSIVGNNTMKAPQGYPLEELKKLEASGYIGSASYN |
| gas40M6_5529 | (251) | EAELSRLKSSAPSTQDSIVGNNTMKAPQGYPLEELKKLEASGYIGSASYN |
| gas40M5_ | (251) | EAELSRLKSSAPSTQDSIVGNNTMKAPQGYPLEELKKLEASGYIGSASYN |
| gas40M77_4959 | (251) | EAELSRLKSSAPSTQDSIVGNNTMKAPQGYPLEELKKLEASGYIGSASYN |
| gas40M2_10064 | (251) | EAELSRLKSSAPSTQDSIVGNNTMKAPQGYPLEELKKLEASGYIGSASYN |
| gas40M2_10065 | (251) | EAELSRLKSSAPSTQDSIVGNNTMKAPQGYPLEELKKLEASGYIGSASYN |
| gas40M75_5531 | (251) | EAELSRLKSSAPSTQDSIVGNNTMKAPQGYPLEELKKLEASGYIGSASYN |
| gas40M50_4538 | (251) | EAELSRLKSSAPSTQDSIVGNNTMKAPQGYPLEELKKLEASGYIGSASYN |
| gas40M62_5455 | (251) | EAELSRLKSSAPSTQDSIVGNNTMKAPQGYPLEELKKLEASGYIGSASYN |
| gas40M44_5481 | (251) | EAELSRLKSSAPSTQDSIVGNNTMKAPQGYPLEELKKLEASGYIGSASYN |
| gas40M5_4883 | (251) | EAELSRLKSSAPSTQDSIVGNNTMKAPQGYPLEELKKLEASGYIGSASYN |
| gas40M9?_2720 | (251) | EAELSRLKSSAPSTQDSIVGNNTMKAPQGYPLEELKKLEASGYIGSASYN |
| gas40M2_2726 | (251) | EAELSRLKSSAPSTQDSIVGTNTMKAPQGYPLEELKKLEASGYIGSASYN |
| gas40M12_20296 | (251) | EAELSRLKSSAPSTQDSIVGNNTMKAPQGYPLEELKKLEASGYIGSASYN |
| gas40M1_2580 | (251) | EAELSRLKSSAPSTQDSIVGNNTMKAPQGYPLEELKKLEASGYIGSASYN |
| gas40M1_2913 | (251) | EAELSRLKSSAPSTQDSIVGNNTMKAPQGYPLEELKKLEASGYIGSASYN |
| gas40M1_3280 | (251) | EAELSRLKSSAPSTQDSIVGNNTMKAPQGYPLEELKKLEASGYIGSASYN |
| gas40M1_3348 | (251) | EAELSRLKSSAPSTQDSIVGNNTMKAPQGYPLEELKKLEASGYIGSASYN |
| gas40M78_3789 | (251) | EAELSRLKSSAPSTQDSIVGNNTMKAPQGYPLEELKKLEASGYIGSASYN |
| gas40M?_2719 | (251) | EAELSRLKSSAPSTQDSIVGNNTMKAPQGYPLEELKKLEASGYIGSASYN |

FIG. 32G

| | | 301 350 |
|---|---|---|
| gas40M1_SF370 | (301) | NYYKEHADQIIAKASPGNQLNQYQDIPADRNRFVDPDNLTPEVQNELAQF |
| gas40clinicalisolate_4088 | (301) | NYYKEHADQIIAKASPGNQLNQYQDIPADRNRFVDPDNLTPEVQNELAQF |
| gas40M11_2727 | (301) | NYYKEHADQIIAKASPGNQLNQYQDIPADRNRFVDPDNLTPEVQNELAQF |
| gas40M22_20641 | (301) | NYYKEHADQIIAKASPGNQLNQYQDIPADRNRFVDPDNLTPEVQNELAQF |
| gas40M22_23465 | (301) | NYYKEHADQIIAKASPGNQLNQYQDIPADRNRFVDPDNLTPEVQNELAQF |
| gas40M22_23621 | (301) | NYYKEHADQIIAKASPGNQLNQYQDIPADRNRFVDPDNLTPEVQNELAQF |
| gas40M3,1_30610 | (301) | NYYKEHADQIIAKASPGNQLNQYQDIPADRNRFVDPDNLTPEVQNELAQF |
| gas40M3,1_40603 | (301) | NYYKEHADQIIAKASPGNQLNQYQDIPADRNRFVDPDNLTPEVQNELAQF |
| gas40M3,34_10307 | (301) | NYYKEHADQIIAKASPGNQLNQYQDIPADRNRFVDPDNLTPEVQNELAQF |
| gas40M3_MGAS315 | (301) | NYYKEHADQIIAKASPGNQLNQYQDIPADRNRFVDPDNLTPEVQNELAQF |
| gas40M4_40427 | (301) | NYYKEHADQIIAKASPGNQLNQYQDIPADRNRFVDPDNLTPEVQNELAQF |
| gas40M3_2721 | (301) | NYYKEHADQIIAKASPGNQLNQYQDIPADRNRFVDPDNLTPEVQNELAQF |
| gas40M3_3040 | (301) | NYYKEHADQIIAKASPGNQLNQYQDIPADRNRFVDPDNLTPEVQNELAQF |
| gas40M3_3135 | (301) | NYYKEHADQIIAKASPGNQLNQYQDIPADRNRFVDPDNLTPEVQNELAQF |
| gas40M12_10035 | (301) | NYYKEHADQIIAKASPGNQLNQYQDIPADRNRFVDPDNLTPEVQNELAQF |
| gas40M12_22432 | (301) | NYYKEHADQIIAKASPGNQLNQYQDIPADRNRFVDPDNLTPEVQNELAQF |
| gas40M4_40499 | (301) | NYYKEHADQIIAKASPGNQLNQYQDIPADRNRFVDPDNLTPEVQNELAQF |
| gas40M12_2728(EM5) | (301) | NYYKEHADQIIAKASPGNQLNQYQDIPADRNRFVDPDNLTPEVQNELAQF |
| gas40M89_10070 | (301) | NYYKEHADQIIAKASPGNQLNQYQDIPADRNRFVDPDNLTPEVQNELAQF |
| gas40M89_21915 | (301) | NYYKEHADQIIAKASPGNQLNQYQDIPADRNRFVDPDNLTPEVQNELAQF |
| gas40M89_23717 | (301) | NYYKEHADQIIAKASPGNQLNQYQDIPADRNRFVDPDNLTPEVQNELAQF |
| gas40M89_5476 | (301) | NYYKEHADQIIAKASPGNQLNQYQDIPADRNRFVDPDNLTPEVQNELAQF |
| gas40M23_DSM2071 | (301) | NYYKEHADQIIAKASPGNQLNQYQDIPADRNRFVDPDNLTPEVQNELAQF |
| gas40M4_2722 | (301) | NYYKEHADQIIAKASPGNQLNQYQDIPADRNRFVDPDNLTPEVQNELAQF |
| gas40M4_10092 | (301) | NYYKEHADQIIAKASPGNQLNQYQDIPADRNRFVDPDNLTPEVQNELAQF |
| gas40M4_30968 | (301) | NYYKEHADQIIAKASPGNQLNQYQDIPADRNRFVDPDNLTPEVQNELAQF |
| gas40M4_2634 | (301) | NYYKEHADQIIAKASPGNQLNQYQDIPADRNRFVDPDNLTPEVQNELAQF |
| gas40M28_10164 | (301) | NYYKEHADQIIAKASPGNQLNQYQDIPADRNRFVDPDNLTPEVQNELAQF |
| gas40M28_10218 | (301) | NYYKEHADQIIAKASPGNQLNQYQDIPADRNRFVDPDNLTPEVQNELAQF |
| gas40M28_10266 | (301) | NYYKEHADQIIAKASPGNQLNQYQDIPADRNRFVDPDNLTPEVQNELAQF |
| gas40M28_10299 | (301) | NYYKEHADQIIAKASPGNQLNQYQDIPADRNRFVDPDNLTPEVQNELAQF |
| gas40M28_30176 | (301) | NYYKEHADQIIAKASPGNQLNQYQDIPADRNRFVDPDNLTPEVQNELAQF |
| gas40M28_4436 | (301) | NYYKEHADQIIAKASPGNQLNQYQDIPADRNRFVDPDNLTPEVQNELAQF |
| gas40M8_2725 | (301) | NYYKEHADQIIAKASPGNQLNQYQDIPADRNRFVDPDNLTPEVQNELAQF |
| gas40M44_3776 | (301) | NYYKEHADQIIAKASPGNQLNQYQDIPADRNRFVDPDNLTPEVQNELAQF |
| gas40M6_2724 | (301) | NYYKEHADQIIAKASPGNQLNQYQDIPADRNRFVDPDNLTPEVQNELAQF |
| gas40M6_2894 | (301) | NYYKEHADQIIAKASPGNQLNQYQDIPADRNRFVDPDNLTPEVQNELAQF |
| gas40M6_3650 | (301) | NYYKEHADQIIAKASPGNQLNQYQDIPADRNRFVDPDNLTPEVQNELAQF |
| gas40M6_5529 | (301) | NYYKEHADQIIAKASPGNQLNQYQDIPADRNRFVDPDNLTPEVQNELAQF |
| gas40M5_ | (301) | NYYKEHADQIIAKASPGNQLNQYQDIPADRNRFVDPDNLTPEVQNELAQF |
| gas40M77_4959 | (301) | NYYKEHADQIIAKASPGNQLNQYQDIPADRNRFVDPDNLTPEVQNELAQF |
| gas40M2_10064 | (301) | NYYKEHADQIIAKASPGNQLNQYQDIPADRNRFVDPDNLTPEVQNELAQF |
| gas40M2_10065 | (301) | NYYKEHADQIIAKASPGNQLNQYQDIPADRNRFVDPDNLTPEVQNELAQF |
| gas40M75_5531 | (301) | NYYKEHADQIIAKASPGNQLNQYQDIPADRNRFVDPDNLTPEVQNELAQF |
| gas40M50_4538 | (301) | NYYKEHADQIIAKASPGNQLNQYQDIPADRNRFVDPDNLTPEVQNELAQF |
| gas40M62_5455 | (301) | NYYKEHADQIIAKASPGNQLNQYQDIPADRNRFVDPDNLTPEVQNELAQF |
| gas40M44_5481 | (301) | NYYKEHADQIIAKASPGNQLNQYQDIPADRNRFVDPDNLTPEVQNELAQF |
| gas40M5_4883 | (301) | NYYKEHADQIIAKASPGNQLNQYQDIPADRNRFVDPDNLTPEVQNELAQF |
| gas40M9?_2720 | (301) | NYYKEHADQIIAKASPGNQLNQYQDIPADRNRFVDPDNLTPEVQNELAQF |
| gas40M2_2726 | (301) | NYYKEHADQIIAKASPGNQLNQYQDIPADRTRFVDPDNLTPEVQNELAQF |
| gas40M12_20296 | (301) | NYYKEHADQIIAKASPGNQLNQYQDIPADRNRFVDPDNLTPEVQNELAQF |
| gas40M1_2580 | (301) | NYYKEHADQIIAKASPGNQLNQYQDIPADRNRFVDPDNLTPEVQNELAQF |
| gas40M1_2913 | (301) | NYYKEHADQIIAKASPGNQLNQYQDIPADRNRFVDPDNLTPEVQNELAQF |
| gas40M1_3280 | (301) | NYYKEHADQIIAKASPGNQLNQYQDIPADRNRFVDPDNLTPEVQNELAQF |
| gas40M1_3348 | (301) | NYYEEHADQIIAKASPGNQLNQYQDIPADRNRFVDPDNLTPEVQNELAQF |
| gas40M78_3789 | (301) | NYYKEHADQIIAKASPGNQLNQYQDIPADRNRFVDPDNLTPEVQNELAQF |
| gas40M?_2719 | (301) | NYYKEHADQIIAKASPGNQLNQYQDIPADRNRFVDPDNLTPEVQNELAQF |

FIG. 32H

| | | |
|---|---|---|
| | | 351 400 |
| gas40M1_SF370 | (351) | AAHMINSVRRQLGLPPVTVTAGSQEFARLLSTSYKKTHGNTRPSFVYGQP |
| gas40clinicalisolate_4088 | (351) | AAHMINSVRRQLGLPPVTVTAGSQEFARLLSTSYKKTHGNTRPSFVYGQP |
| gas40M11_2727 | (351) | AAHMINSVRRQLGLPPVTVTAGSQEFARLLSTSYKKTHGNTRPSFVYGQP |
| gas40M22_20641 | (351) | AAHMINSVRRQLGLPPVTVTAGSQEFARLLSTSYKKTHGNTRPSFVYGQP |
| gas40M22_23465 | (351) | AAHMINSVRRQLGLPPVTVTAGSQEFARLLSTSYKKTHGNTRPSFVYGQP |
| gas40M22_23621 | (351) | AAHMINSVRRQLGLPPVTVTAGSQEFARLLSTSYKKTHGNTRPSFVYGQP |
| gas40M3,1_30610 | (351) | AAHMINSVRRQLGLPPVTVTAGSQEFARLLSTSYKKTHGNTRPSFVYGQP |
| gas40M3,1_40603 | (351) | AAHMINSVRRQLGLPPVTVTAGSQEFARLLSTSYKKTHGNTRPSFVYGQP |
| gas40M3,34_10307 | (351) | AAHMINSVRRQLGLPPVTVTAGSQEFARLLSTSYKKTHGNTRPSFVYGQP |
| gas40M3_MGAS315 | (351) | AAHMINSVRRQLGLPPVTVTAGSQEFARLLSTSYKKTHGNTRPSFVYGQP |
| gas40M4_40427 | (351) | AAHMINSVRRQLGLPPVTVTAGSQEFARLLSTSYKKTHGNTRPSFVYGQP |
| gas40M3_2721 | (351) | AAHMINSVRRQLGLPPVTVTAGSQEFARLLSTSYKKTHGNTRPSFVYGQP |
| gas40M3_3040 | (351) | AAHMINSVRRQLGLPPVTVTAGSQEFARLLSTSYKKTHGNTRPSFVYGQP |
| gas40M3_3135 | (351) | AAHMINSVRRQLGLPPVTVTAGSQEFARLLSTSYKKTHGNTRPSFVYGQP |
| gas40M12_10035 | (351) | AAHMINSVRRQLGLPPVTVTAGSQEFARLLSTSYKKTHGNTRPSFVYGQP |
| gas40M12_22432 | (351) | AAHMINSVRRQLGLPPVTVTAGSQEFARLLSTSYKKTHGNTRPSFVYGQP |
| gas40M4_40499 | (351) | AAHMINSVRRQLGLPPVTVTAGSQEFARLLSTSYKKTHGNTRPSFVYGQP |
| gas40M12_2728(EM5) | (351) | AAHMINSVRRQLGLPPVTVTAGSQEFARLLSTSYKKTHGNTRPSFVYGQP |
| gas40M89_10070 | (351) | AAHMINSVRRQLGLPPVTVTAGSQEFARLLSTSYKKTHGNTRPSFVYGQP |
| gas40M89_21915 | (351) | AAHMINSVRRQLGLPPVTVTAGSQEFARLLSTSYKKTHGNTRPSFVYGQP |
| gas40M89_23717 | (351) | AAHMINSVRRQLGLPPVTVTAGSQEFARLLSTSYKKTHGNTRPSFVYGQP |
| gas40M89_5476 | (351) | AAHMINSVRRQLGLPPVTVTAGSQEFARLLSTSYKKTHGNTRPSFVYGQP |
| gas40M23_DSM2071 | (351) | AAHMINSVRRQLGLPPVTVTAGSQEFARLLSTSYKKTHGNTRPSFVYGQP |
| gas40M4_2722 | (351) | AAHMINSVRRQLGLPPVTVTAGSQEFARLLSTSYKKTHGNTRPSFVYGQP |
| gas40M4_10092 | (351) | AAHMINSVRRQLGLPPVTVTAGSQEFARLLSTSYKKTHGNTRPSFVYGQP |
| gas40M4_30968 | (351) | AAHMINSVRRQLGLPPVTVTAGSQEFARLLSTSYKKTHGNTRPSFVYGQP |
| gas40M4_2634 | (351) | AAHMINSVRRQLGLPPVTVTAGSQEFARLLSTSYKKTHGNTRPSFVYGQP |
| gas40M28_10164 | (351) | AAHMINSVRRQLGLPPVTVTAGSQEFARLLSTSYKKTHGNTRPSFVYGQP |
| gas40M28_10218 | (351) | AAHMINSVRRQLGLPPVTVTAGSQEFARLLSTSYKKTHGNTRPSFVYGQP |
| gas40M28_10266 | (351) | AAHMINSVRRQLGLPPVTVTAGSQEFARLLSTSYKKTHGNTRPSFVYGQP |
| gas40M28_10299 | (351) | AAHMINSVRRQLGLPPVTVTAGSQEFARLLSTSYKKTHGNTRPSFVYGQP |
| gas40M28_30176 | (351) | AAHMINSVRRQLGLPPVTVTAGSQEFARLLSTSYKKTHGNTRPSFVYGQP |
| gas40M28_4436 | (351) | AAHMINSVRRQLGLPPVTVTAGSQEFARLLSTSYKKTHGNTRPSFVYGQP |
| gas40M8_2725 | (351) | AAHMINSVRRQLGLPPVTVTAGSQEFARLLSTSYKKTHGNTRPSFVYGQP |
| gas40M44_3776 | (351) | AAHMINSVRRQLGLPPVTVTAGSQEFARLLSTSYKKTHGNTRPSFVYGQP |
| gas40M6_2724 | (351) | AAHMINSVRRQLGLPPVTVTAGSQEFARLLSTSYKKTHGNTRPSFVYGQP |
| gas40M6_2894 | (351) | AAHMINSVRRQLGLPPVTVTAGSQEFARLLSTSYKKTHGNTRPSFVYGQP |
| gas40M6_3650 | (351) | AAHMINSVRRQLGLPPVTVTAGSQEFARLLSTSYKKTHGNTRPSFVYGQP |
| gas40M6_5529 | (351) | AAHMINSVRRQLGLPPVTVTAGSQEFARLLSTSYKKTHGNTRPSFVYGQP |
| gas40M5_ | (351) | AAHMINSVRRQLGLPPVTVTAGSQEFARLLSTSYKKTHGNTRPSFVYGQP |
| gas40M77_4959 | (351) | AAHMINSVRRQLGLPPVTVTAGSQEFARLLSTSYKKTHGNTRPSFVYGQP |
| gas40M2_10064 | (351) | AAHMINSVRRQLGLPPVTVTAGSQEFARLLSTSYKKTHGNTRPSFVYGQP |
| gas40M2_10065 | (351) | AAHMINSVRRQLGLPPVTVTAGSQEFARLLSTSYKKTHGNTRPSFVYGQP |
| gas40M75_5531 | (351) | AAHMINSVRRQLGLPPVTVTAGSQEFARLLSTSYKKTHGNTRPSFVYGQP |
| gas40M50_4538 | (351) | AAHMINSVRRQLGLPPVTVTAGSQEFARLLSTSYKKTHGNTRPSFVYGQP |
| gas40M62_5455 | (351) | AAHMINSVRRQLGLPPVTVTAGSQEFARLLSTSYKKTHGNTRPSFVYGQP |
| gas40M44_5481 | (351) | AAHMINSVRRQLGLPPVTVTAGSQEFARLLSTSYKKTHGNTRPSFVYGQP |
| gas40M5_4883 | (351) | AAHMINSVRRQLGLPPVTVTAGSQEFARLLSTSYKKTHGNTRPSFVYGQP |
| gas40M9?_2720 | (351) | AAHMINSVRRQLGLPPVTVTAGSQEFARLLSTSYKKTHGNTRPSFVYGQP |
| gas40M2_2726 | (351) | AAHMINSVRRQLGLPPVTVTAGSQEFARLLSTSYKKTHGNTRPSFVYGQP |
| gas40M12_20296 | (351) | AAHMINSVRRQLGLPPVTVTAGSQEFARLLSTSYKKTHGNTRPSFVYGQP |
| gas40M1_2580 | (351) | AAHMINSVRRQLGLPPVTVTAGSQEFARLLSTSYKKTHGNTRPSFVYGQP |
| gas40M1_2913 | (351) | AAHMINSVRRQLGLPPVTVTAGSQEFARLLSTSYKKTHGNTRPSFVYGQP |
| gas40M1_3280 | (351) | AAHMINSVRRQLGLPPVTVTAGSQEFARLLSTSYKKTHGNTRPSFVYGQP |
| gas40M1_3348 | (351) | AAHMINSVRRQLGLPPVTVTAGSQEFARLLSTSYKKTHGNTRPSFVYGQP |
| gas40M78_3789 | (351) | AAHMINSVRRQLGLPPVTVTAGSQEFARLLSTSYKKTHGNTRPSFVYGQP |
| gas40M?_2719 | (351) | AAHMINSVRRQLGLPPVTVTAGSQEFARLLSTSYKKTHGNTRPSFVYGQP |

FIG. 32I

```
                                    401                                               450
            gas40M1_SF370   (401)  GVSGHYGVGPHDKTIIEDSAGASGLIRNDDNMYENIGAFNDVHTVNGIKR
gas40clinicalisolate_4088   (401)  GVSGHYGVGPHDKTIIEDSAGASGLIRNDDNMYENIGAFNDVHTVNGIKR
           gas40M11_2727    (401)  GVSGHYGVGPHDKTIIEDSAGASGLIRNDDNMYENIGAFNDVHTVNGIKR
          gas40M22_20641    (401)  GVSGHYGVGPHDKTIIEDSAGASGLIRNDDNMYENIGAFNDVHTVNGIKR
          gas40M22_23465    (401)  GVSGHYGVGPHDKTIIEDSAGASGLIRNDDNMYENIGAFNDVHTVNGIKR
          gas40M22_23621    (401)  GVSGHYGVGPHDKTIIEDSAGASGLIRNDDNMYENIGAFNDVHTVNGIKR
         gas40M3,1_30610    (401)  GVSGHYGVGPHDKTIIEDSAGASGLIRNDDNMYENIGAFNDVHTVNGIKR
         gas40M3,1_40603    (401)  GVSGHYGVGPHDKTIIEDSAGASGLIRNDDNMYENIGAFNDVHTVNGIKR
        gas40M3,34_10307    (401)  GVSGHYGVGPHDKTIIEDSAGASGLIRNDDNMYENIGAFNDVHTVNGIKR
         gas40M3_MGAS315    (401)  GVSGHYGVGPHDKTIIEDSAGASGLIRNDDNMYENIGAFNDVHTVNGIKR
           gas40M4_40427    (401)  GVSGHYGVGPHDKTIIEDSAGASGLIRNDDNMYENIGAFNDVHTVNGIKR
            gas40M3_2721    (401)  GVSGHYGVGPHDKTIIEDSAGASGLIRNDDNMYENIGAFNDVHTVNGIKR
            gas40M3_3040    (401)  GVSGHYGVGPHDKTIIEDSAGASGLIRNDDNMYENIGAFNDVHTVNGIKR
            gas40M3_3135    (401)  GVSGHYGVGPHDKTIIEDSAGASGLIRNDDNMYENIGAFNDVHTVNGIKR
          gas40M12_10035    (401)  GVSGHYGVGPHDKTIIEDSAGASGLIRNDDNMYENIGAFNDVHTVNGIKR
          gas40M12_22432    (401)  GVSGHYGVGPHDKTIIEDSAGASGLIRNDDNMYENIGAFNDVHTVNGIKR
           gas40M4_40499    (401)  GVSGHYGVGPHDKTIIEDSAGASGLIRNDDNMYENIGAFNDVHTVNGIKR
      gas40M12_2728(EM5)    (401)  GVSGHYGVGPHDKTIIEDSAGASGLIRNDDNMYENIGAFNDVHTVNGIKR
          gas40M89_10070    (401)  GVSGHYGVGPHDKTIIEDSAGASGLIRNDDNMYENIGAFNDVHTVNGIKR
          gas40M89_21915    (401)  GVSGHYGVGPHDKTIIEDSAGASGLIRNDDNMYENIGAFNDVHTVNGIKR
          gas40M89_23717    (401)  GVSGHYGVGPHDKTIIEDSAGASGLIRNDDNMYENIGAFNDVHTVNGIKR
           gas40M89_5476    (401)  GVSGHYGVGPHDKTIIEDSAGASGLIRNDDNMYENIGAFNDVHTVNGIKR
        gas40M23_DSM2071    (401)  GVSGHYGVGPHDKTIIEDSAGASGLIRNDDNMYENIGAFNDVHTVNGIKR
            gas40M4_2722    (401)  GVSGHYGVGPHDKTIIEDSAGASGLIRNDDNMYENIGAFNDVHTVNGIKR
           gas40M4_10092    (401)  GVSGHYGVGPHDKTIIEDSAGASGLIRNDDNMYENIGAFNDVHTVNGIKR
           gas40M4_30968    (401)  GVSGHYGVGPHDKTIIEDSAGASGLIRNDDNMYENIGAFNDVHTVNGIKR
            gas40M4_2634    (401)  GVSGHYGVGPHDKTIIEDSAGASGLIRNDDNMYENIGAFNDVHTVNGIKR
          gas40M28_10164    (401)  GVSGHYGVGPHDKTIIEDSAGASGLIRNDDNMYENIGAFNDVHTVNGIKR
          gas40M28_10218    (401)  GVSGHYGVGPHDKTIIEDSAGASGLIRNDDNMYENIGAFNDVHTVNGIKR
          gas40M28_10266    (401)  GVSGHYGVGPHDKTIIEDSAGASGLIRNDDNMYENIGAFNDVHTVNGIKR
          gas40M28_10299    (401)  GVSGHYGVGPHDKTIIEDSAGASGLIRNDDNMYENIGAFNDVHTVNGIKR
          gas40M28_30176    (401)  GVSGHYGVGPHDKTIIEDSAGASGLIRNDDNMYENIGAFNDVHTVNGIKR
           gas40M28_4436    (401)  GVSGHYGVGPHDKTIIEDSAGASGLIRNDDNMYENIGAFNDVHTVNGIKR
            gas40M8_2725    (401)  GVSGHYGVGPHDKTIIEDSAGASGLIRNDDNMYENIGAFNDVHTVNGIKR
           gas40M44_3776    (401)  GVSGHYGVGPHDKTIIEDSAGASGLIRNDDNMYENIGAFNDVHTVNGIKR
            gas40M6_2724    (401)  GVSGHYGVGPHDKTIIEDSAGASGLIRNDDNMYENIGAFNDVHTVNGIKR
            gas40M6_2894    (401)  GVSGHYGVGPHDKTIIEDSAGASGLIRNDDNMYENIGAFNDVHTVNGIKR
            gas40M6_3650    (401)  GVSGHYGVGPHDKTIIEDSAGASGLIRNDDNMYENIGAFNDVHTVNGIKR
            gas40M6_5529    (401)  GVSGHYGVGPHDKTIIEDSAGASGLIRNDDNMYENIGAFNDVHTVNGIKR
                gas40M5_    (401)  GVSGHYGVGPHDKTIIEDSAGASGLIRNDDNMYENIGAFNDVHTVNGIKR
           gas40M77_4959    (401)  GVSGHYGVGPHDKTIIEDSAGASGLIRNDDNMYENIGAFNDVHTVNGIKR
           gas40M2_10064    (401)  GVSGHYGVGPHDKTIIEDSAGASGLIRNDDNMYENIGAFNDVHTVNGIKR
           gas40M2_10065    (401)  GVSGHYGVGPHDKTIIEDSAGASGLIRNDDNMYENIGAFNDVHTVNGIKR
           gas40M75_5531    (401)  GVSGHYGVGPHDKTIIEDSAGASGLIRNDDNMYENIGAFNDVHTVNGIKR
           gas40M50_4538    (401)  GVSGHYGVGPHDKTIIEDSAGASGLIRNDDNMYENIGAFNDVHTVNGIKR
           gas40M62_5455    (401)  GVSGHYGVGPHDKTIIEDSAGASGLIRNDDNMYENIGAFNDVHTVNGIKR
           gas40M44_5481    (401)  GVSGHYGVGPHDKTIIEDSAGASGLIRNDDNMYENIGAFNDVHTVNGIKR
            gas40M5_4883    (401)  GVSGHYGVGPHDKTIIEDSAGASGLIRNDDNMYENIGAFNDVHTVNGIKR
           gas40M9?_2720    (401)  GVSGHYGVGPHDKTIIEDSAGASGLIRNDDNMYENIGAFNDVHTVNGIKR
            gas40M2_2726    (401)  GASGHYGVGPHDKTIIEDSAGASGLIRNDDNMYENIGAFNDVHTVNGIKR
          gas40M12_20296    (401)  GVSGHYGVGPHDKTIIEDSAGASGLIRNDDNMYENIGAFNDVHTVNGIKR
            gas40M1_2580    (401)  GVSGHYGVGPHDKTIIEDSAGASGLIRNDDNMYENIGAFNDVHTVNGIKR
            gas40M1_2913    (401)  GVSGHYGVGPHDKTIIEDSAGASGLIRNDDNMYENIGAFNDVHTVNGIKR
            gas40M1_3280    (401)  GVSGHYGVGPHDKTIIEDSAGASGLIRNDDNMYENIGAFNDVHTVNGIKR
            gas40M1_3348    (401)  GVSGHYGVGPHDKTIIEDSAGASGLIRNDDNMYENIGAFNDVHTVNGIKR
           gas40M78_3789    (401)  GVSGHYGVGPHDKTIIEDSAGASGLIRNDDNMYENIGAFNDVHTVNGIKR
            gas40M?_2719    (401)  GVSGHYGVGPHDKTIIEDSAGASGLIRNDDNMYENIGAFNDVHTVNGIKR
```

FIG. 32J

| | | 451 500 |
|---|---|---|
| gas40M1_SF370 | (451) | GIYDSIKYMLFTDHLHGNTYGHAINFLRVDKHNPNAPVYLGFSTSNVGSL |
| gas40clinicalisolate_4088 | (451) | GIYDSIKYMLFTDHLHGNTYGHAINFLRVDKRNPNAPVYLGFSTSNVGSL |
| gas40M11_2727 | (451) | GIYDSIKYMLFTDHLHGNTYGHAINFLRVDKRNPNAPVYLGFSTSNVGSL |
| gas40M22_20641 | (451) | GIYDSIKYMLFTDHLHGNTYGHAINFLRVDKRNPNAPVYLGFSTSNVGSL |
| gas40M22_23465 | (451) | GIYDSIKYMLFTDHLHGNTYGHAINFLRVDKRNPNAPVYLGFSTSNVGSL |
| gas40M22_23621 | (451) | GIYDSIKYMLFTDHLHGNTYGHAINFLRVDKRNPNAPVYLGFSTSNVGSL |
| gas40M3,1_30610 | (451) | GIYDSIKYMLFTDHLHGNTYGHAINFLRVDKHNPNAPVYLGFSTSNVGSL |
| gas40M3,1_40603 | (451) | GIYDSIKYMLFTDHLHGNTYGHAINFLRVDKHNPNAPVYLGFSTSNVGSL |
| gas40M3,34_10307 | (451) | GIYDSIKYMLFTDHLHGNTYGHAINFLRVDKHNPNAPVYLGFSTSNVGSL |
| gas40M3_MGAS315 | (451) | GIYDSIKYMLFTDHLHGNTYGHAINFLRVDKHNPNAPVYLGFSTSNVGSL |
| gas40M4_40427 | (451) | GIYDSIKYMLFTDHLHGNTYGHAINFLRVDKHNPNAPVYLGFSTSNVGSL |
| gas40M3_2721 | (451) | GIYDSIKYMLFTDHLHGNTYGHAINFLRVDKHNPNAPVYLGFSTSNVGSL |
| gas40M3_3040 | (451) | GIYDSIKYMLFTDHLHGNTYGHAINFLRVDKHNPNAPVYLGFSTSNVGSL |
| gas40M3_3135 | (451) | GIYDSIKYMLFTDHLHGNTYGHAINFLRVDKHNPNAPVYLGFSTSNVGSL |
| gas40M12_10035 | (451) | GIYDSIKYMLFTDHLHGNTYGHAINFLRVDKHNPNAPVYLGFSTSNVGSL |
| gas40M12_22432 | (451) | GIYDSIKYMLFTDHLHGNTYGHAINFLRVDKHNPNAPVYLGFSTSNVGSL |
| gas40M4_40499 | (451) | GIYDSIKYMLFTDHLHGNTYGHAINFLRVDKHNPNAPVYLGFSTSNVGSL |
| gas40M12_2728(EM5) | (451) | GIYDSIKYMLFTDHLHGNTYGHAINFLRVDKHNPNAPVYLGFSTSNVGSL |
| gas40M89_10070 | (451) | GIYDSIKYMLFTDHLHGNTYGHAINFLRVDKRNPNAPVYLGFSTSNVGSL |
| gas40M89_21915 | (451) | GIYDSIKYMLFTDHLHGNTYGHAINFLRVDKRNPNAPVYLGFSTSNVGSL |
| gas40M89_23717 | (451) | GIYDSIKYMLFTDHLHGNTYGHAINFLRVDKRNPNAPVYLGFSTSNVGSL |
| gas40M89_5476 | (451) | GIYDSIKYMLFTDHLHGNTYGHAINFLRVDKRNPNAPVYLGFSTSNVGSL |
| gas40M23_DSM2071 | (451) | GIYDSIKYMLFTDHLHGNTYGHAINFLRVDKRNPNAPVYLGFSTSNVGSL |
| gas40M4_2722 | (451) | GIYDSIKYMLFTDHLHGNTYGHAINFLRVDKRNPNAPVYLGFSTSNVGSL |
| gas40M4_10092 | (451) | GIYDSIKYMLFTDHLHGNTYGHAINFLRVDKRNPNAPVYLGFSTSNVGSL |
| gas40M4_30968 | (451) | GIYDSIKYMLFTDHLHGNTYGHAINFLRVDKRNPNAPVYLGFSTSNVGSL |
| gas40M4_2634 | (451) | GIYDSIKYMLFTDHLHGNTYGHAINFLRVDKRNPNAPVYLGFSTSNVGSL |
| gas40M28_10164 | (451) | GIYDSIKYMLFTDHLHGNTYGHAINFLRVDKRNPNAPVYLGFSTSNVGSL |
| gas40M28_10218 | (451) | GIYDSIKYMLFTDHLHGNTYGHAINFLRVDKRNPNAPVYLGFSTSNVGSL |
| gas40M28_10266 | (451) | GIYDSIKYMLFTDHLHGNTYGHAINFLRVDKRNPNAPVYLGFSTSNVGSL |
| gas40M28_10299 | (451) | GIYDSIKYMLFTDHLHGNTYGHAINFLRVDKRNPNAPVYLGFSTSNVGSL |
| gas40M28_30176 | (451) | GIYDSIKYMLFTDHLHGNTYGHAINFLRVDKRNPNAPVYLGFSTSNVGSL |
| gas40M28_4436 | (451) | GIYDSIKYMLFTDHLHGNTYGHAINFLRVDKRNPNAPVYLGFSTSNVGSL |
| gas40M8_2725 | (451) | GIYDSIKYMLFTDHLHGNTYGHAINFLRVDKRNPNAPVYLGFSTSNVGSL |
| gas40M44_3776 | (451) | GIYDSIKYMLFTDHLHGNTYGHAINFLRVDKRNPNAPVYLGFSTSNVGSL |
| gas40M6_2724 | (451) | GIYDSIKYMLFTDHLHGNTYGHAINFLRVDKRNPNAPVYLGFSTSNVGSL |
| gas40M6_2894 | (451) | GIYDSIKYMLFTDHLHGNTYGHAINFLRVDKRNPNAPVYLGFSTSNVGSL |
| gas40M6_3650 | (451) | GIYDSIKYMLFTDHLHGNTYGHAINFLRVDKRNPNAPVYLGFSTSNVGSL |
| gas40M6_5529 | (451) | GIYDSIKYMLFTDHLHGNTYGHAINFLRVDKRNPNAPVYLGFSTSNVGSL |
| gas40M5_ | (451) | GIYDSIKYMLFTDHLHGNTYGHAINFLRVDKRNPNAPVYLGFSTSNVGSL |
| gas40M77_4959 | (451) | GIYDSIKYMLFTDHLHGNTYGHAINFLRVDKRNPNAPVYLGFSTSNVGSL |
| gas40M2_10064 | (451) | GIYDSIKYMLFTDHLHGNTYGHAINFLRVDKHNPKAPVYLGFSTSNVGSL |
| gas40M2_10065 | (451) | GIYDSIKYMLFTDHLHGNTYGHAINFLRVDKHNPKAPVYLGFSTSNVGSL |
| gas40M75_5531 | (451) | GIYDSIKYMLFTDHLHGNTYGHAINFLRVDKHNPKAPVYLGFSTSNVGSL |
| gas40M50_4538 | (451) | GIYDSIKYMLFTDHLHGNTYGHAINFLRVDKHNPKAPVYLGFSTSNVGSL |
| gas40M62_5455 | (451) | GIYDSIKYMLFTDHLHGNTYGHAINFLRVDKHNPKAPVYLGFSTSNVGSL |
| gas40M44_5481 | (451) | GIYDSIKYMLFTDHLHGNTYGHAINFLRVDKRNPNAPVYLGFSTSNVGSL |
| gas40M5_4883 | (451) | GIYDSIKYMLFTDHLHGNTYGHAINFLRVDKRNPNAPVYLGFSTSNVGSL |
| gas40M9?_2720 | (451) | GIYDSIKYMLFTDHLHGNTYGHAINFLRVDKRNPNAPVYLGFSTSNVGSL |
| gas40M2_2726 | (451) | GIYDSIKYMLFTDHLHGNTYGHAINFLRVDKHNPNAPVYLGFSTSNVGSL |
| gas40M12_20296 | (451) | GIYDSIKYMLFTDHLHGNTYGHAINFLRVDKHNPNAPVYLGFSTSNVGSL |
| gas40M1_2580 | (451) | GIYDSIKYMLFTDHLHGNTYGHAINFLRVDKHNPNAPVYLGFSTSNVGSL |
| gas40M1_2913 | (451) | GIYDSIKYMLFTDHLHGNTYGHAINFLRVDKHNPNAPVYLGFSTSNVGSL |
| gas40M1_3280 | (451) | GIYDSIKYMLFTDHLHGNTYGHAINFLRVDKHNPNAPVYLGFSTSNVGSL |
| gas40M1_3348 | (451) | GIYDSIKYMLFTDHLHGNTYGHAINFLRVDKHNPNAPVYLGFSTSNVGSL |
| gas40M78_3789 | (451) | GIYDSIKYMLFTDHLHGNTYGHAINFLRVDKHNPNAPVYLGFSTSNVGSL |
| gas40M?_2719 | (451) | GIYDSIKYMLFTDHLHGNTYGHAINFLRVDKHNPNAPVYLGFSTSNVGSL |

FIG. 32K

| | | 501 550 |
|---|---|---|
| gas40M1_SF370 | (501) | NEHFVMFPESNIANHQRFNKTPIKAVGSTKDYAQRVGTVSDTIAAIKGKV |
| gas40clinicalisolate_4088 | (501) | NEHFVMFPESNIANHQRFNKTPIKAVGSTKDYAQRVSTVSDTIAAIKGKV |
| gas40M11_2727 | (501) | NEHFVMFPESNIANHQRFNKTPIKAVGSTKDYAQRVSTVSDTIAAIKGKV |
| gas40M22_20641 | (501) | NEHFVMFPESNIANHQRFNKTPIKAVGSTKDYAQRVSTVSDTIAAIKGKV |
| gas40M22_23465 | (501) | NEHFVMFPESNIANHQRFNKTPIKAVGSTKDYAQRVSTVSDTIAAIKGKV |
| gas40M22_23621 | (501) | NEHFVMFPESNIANHQRFNKTPIKAVGSTKDYAQRVSTVSDTIAAIKGKV |
| gas40M3,1_30610 | (501) | NEHFVMFPESNIANHQRFNKTPIKAVGSTKDYAQRVGTVSDTIAAIKGKV |
| gas40M3,1_40603 | (501) | NEHFVMFPESNIANHQRFNKTPIKAVGSTKDYAQRVGTVSDTIAAIKGKV |
| gas40M3,34_10307 | (501) | NEHFVMFPESNIANHQRFNKTPIKAVGSTKDYAQRVGTVSDTIAAIKGKV |
| gas40M3_MGAS315 | (501) | NEHFVMFPESNIANHQRFNKTPIKAVGSTKDYAQRVGTVSDTIAAIKGKV |
| gas40M4_40427 | (501) | NEHFVMFPESNIANHQRFNKTPIKAVGSTKDYAQRVGTVSDTIAAIKGKV |
| gas40M3_2721 | (501) | NEHFVMFPESNIANHQRFNKTPIKAVGSTKDYAQRVGTVSDTIAAIKGKV |
| gas40M3_3040 | (501) | NEHFVMFPESNIANHQRFNKTPIKAVGSTKDYAQRVGTVSDTIAAIKGKV |
| gas40M3_3135 | (501) | NEHFVMFPESNIANHQRFNKTPIKAVGSTKDYAQRVGTVSDTIAAIKGKV |
| gas40M12_10035 | (501) | NEHFVMFPESNIANHQRFNKTPIKAVGSTKDYAQRVGTVSDTIAAIKGKV |
| gas40M12_22432 | (501) | NEHFVMFPESNIANHQRFNKTPIKAVGSTKDYAQRVGTVSDTIAAIKGKV |
| gas40M4_40499 | (501) | NEHFVMFPESNIANHQRFNKTPIKAVGSTKDYAQRVGTVSDTIAAIKGKV |
| gas40M12_2728(EM5) | (501) | NEHFVMFPESNIANHQRFNKTPIKAVGSTKDYAQRVGTVSDTIAAIKGKV |
| gas40M89_10070 | (501) | NEHFVMFPESNIANHQRFNKTPIKAVGSTKDYAQRVGTVSDTIAAIKGKV |
| gas40M89_21915 | (501) | NEHFVMFPESNIANHQRFNKTPIKAVGSTKDYAQRVGTVSDTIAAIKGKV |
| gas40M89_23717 | (501) | NEHFVMFPESNIANHQRFNKTPIKAVGSTKDYAQRVGTVSDTIAAIKGKV |
| gas40M89_5476 | (501) | NEHFVMFPESNIANHQRFNKTPIKAVGSTKDYAQRVGTVSDTIAAIKGKV |
| gas40M23_DSM2071 | (501) | NEHFVMFPESNIANHQRFNKTPIKAVGSTKDYAQRVGTVSDTIAAIKGKV |
| gas40M4_2722 | (501) | NEHFVMFPESNIANHQRFNKTPIKAVGSTKDYAQRVGTVSDTIAAIKGKV |
| gas40M4_10092 | (501) | NEHFVMFPESNIANHQRFNKTPIKAVGSTKDYAQRVGTVSDTIAAIKGKV |
| gas40M4_30968 | (501) | NEHFVMFPESNIANHQRFNKTPIKAVGSTKDYAQRVGTVSDTIAAIKGKV |
| gas40M4_2634 | (501) | NEHFVMFPESNIANHQRFNKTPIKAVGSTKDYAQRVGTVSDTIAAIKGKV |
| gas40M28_10164 | (501) | NEHFVMFPESNIANHQRFNKTPIKAVGSTKDYAQRVGTVSDTIAAIKGKV |
| gas40M28_10218 | (501) | NEHFVMFPESNIANHQRFNKTPIKAVGSTKDYAQRVGTVSDTIAAIKGKV |
| gas40M28_10266 | (501) | NEHFVMFPESNIANHQRFNKTPIKAVGSTKDYAQRVGTVSDTIAAIKGKV |
| gas40M28_10299 | (501) | NEHFVMFPESNIANHQRFNKTPIKAVGSTKDYAQRVGTVSDTIAAIKGKV |
| gas40M28_30176 | (501) | NEHFVMFPESNIANHQRFNKTPIKAVGSTKDYAQRVGTVSDTIAAIKGKV |
| gas40M28_4436 | (501) | NEHFVMFPESNIANHQRFNKTPIKAVGSTKDYAQRVGTVSDTIAAIKGKV |
| gas40M8_2725 | (501) | NEHFVMFPESNIANHQRFNKTPIKAVGSTKDYAQRVGTVSDTIAAIKGKV |
| gas40M44_3776 | (501) | NEHFVMFPESNIANHQRFNKTPIKAVGSTKDYAQRVGTVSDTIAAIKGKV |
| gas40M6_2724 | (501) | NEHFVMFPESNIANHQRFNKTPIKAVGSTKDYAQRVGTVSDTIAAIKGKV |
| gas40M6_2894 | (501) | NEHFVMFPESNIANHQRFNKTPIKAVGSTKDYAQRVGTVSDTIAAIKGKV |
| gas40M6_3650 | (501) | NEHFVMFPESNIANHQRFNKTPIKAVGSTKDYAQRVGTVSDTIAAIKGKV |
| gas40M6_5529 | (501) | NEHFVMFPESNIANHQRFNKTPIKAVGSTKDYAQRVGTVSDTIAAIKGKV |
| gas40M5_ | (501) | NEHFVMFPESNIANHQRFNKTPIKAVGSTKDYAQRVGTVSDTIAAIKGKV |
| gas40M77_4959 | (501) | NEHFVMFPESNIANHQRFNKTPIKAVGSTKDYAQRVGTVSDTIAAIKGKV |
| gas40M2_10064 | (501) | NEHFVMFPESNIANHQRFNKTPIKTVGSTKDYAQRVGTVSDTIAAIKGKV |
| gas40M2_10065 | (501) | NEHFVMFPESNIANHQRFNKTPIKTVGSTKDYAQRVGTVSDTIAAIKGKV |
| gas40M75_5531 | (501) | NEHFVMFPESNIANHQRFNKTPIKTVGSTKDYAQRVGTVSDTIAAIKGKV |
| gas40M50_4538 | (501) | NEHFVMFPESNIANHQRFNKTPIKTVGSTKDYAQRVGTVSDTIAAIKGKV |
| gas40M62_5455 | (501) | NEHFVMFPESNIANHQRFNKTPIKTVGSTKDYAQRVGTVSDTIAAIKGKV |
| gas40M44_5481 | (501) | NEHFVMFPESNIANHQRFNKTPIKAVGSTKDYAQRVGTVSDTIAAIKGKV |
| gas40M5_4883 | (501) | NEHFVMFPESNIANHQRFNKTPIKAVGSTKDYAQRVGTVSDTIAAIKGKV |
| gas40M9?_2720 | (501) | NEHFVMFPESNIANHQRFNKTPIKAVGSTKDYAQRVGTVSDTIAAIKGKV |
| gas40M2_2726 | (501) | NEHFVMFPESNIANHQRFNKTPIKAVGSTKDYAQRVGTVSDTIAAIKGKV |
| gas40M12_20296 | (501) | NEHFVMFPESNIANHQRFNKTPIKAVGSTKDYAQRVGTVSDTIAAIKGKV |
| gas40M1_2580 | (501) | NEHFVMFPESNIANHQRFNKTPIKAVGSTKDYAQRVGTVSDTIAAIKGKV |
| gas40M1_2913 | (501) | NEHFVMFPESNIANHQRFNKTPIKAVGSTKDYAQRVGTVSDTIAAIKGKV |
| gas40M1_3280 | (501) | NEHFVMFPESNIANHQRFNKTPIKAVGSTKDYAQRVGTVSDTIAAIKGKV |
| gas40M1_3348 | (501) | NEHFVMFPESNIANHQRFNKTPIKAVGSTKDYAQRVGTVSDTIAAIKGKV |
| gas40M78_3789 | (501) | NEHFVMFPESNIANHQRFNKTPIKAVGSTKDYAQRVGTVSDTIAAIKGKV |
| gas40M?_2719 | (501) | NEHFVMFPESNIANHQRFNKTPIKAVGSTKDYAQRVGTVSDTIAAIKGKV |

FIG. 32L

| | | 551 600 |
|---|---|---|
| gas40M1_SF370 | (551) | SSLENRLSAIHQEADIMAAQAKVSQLQGKLASTLKQSDSLNLQVRQLNDT |
| gas40clinicalisolate_4088 | (551) | SSLENRLSAIHQEADIMAAQAKVSQLEGKLASTLKQSDSLNLQVRQLNDT |
| gas40M11_2727 | (551) | SSLENRLSAIHQEADIMAAQAKVSQLEGKLASTLKQSDSLNLQVRQLNDT |
| gas40M22_20641 | (551) | SSLENRLSAIHQEADIMAAQAKVSQLEGKLASTLKQSDSLNLQVRQLNDT |
| gas40M22_23465 | (551) | SSLENRLSAIHQEADIMAAQAKVSQLEGKLASTLKQSDSLNLQVRQLNDT |
| gas40M22_23621 | (551) | SSLENRLSAIHQEADIMAAQAKVSQLEGKLASTLKQSDSLNLQVRQLNDT |
| gas40M3,1_30610 | (551) | SSLENRLSAIHQEADIMAAQAKVSQLQGKLASTLKQSDSLNLQVRQLNDT |
| gas40M3,1_40603 | (551) | SSLENRLSAIHQEADIMAAQAKVSQLQGKLASTLKQSDSLNLQVRQLNDT |
| gas40M3,34_10307 | (551) | SSLENRLSAIHQEADIMAAQAKVSQLQGKLASTLKQSDSLNLQVRQLNDT |
| gas40M3_MGAS315 | (551) | SSLENRLSAIHQEADIMAAQAKVSQLQGKLASTLKQSDSLNLQVRQLNDT |
| gas40M4_40427 | (551) | SSLENRLSAIHQEADIMAAQAKVSQLQGKLASTLKQSDSLNLQVRQLNDT |
| gas40M3_2721 | (551) | SSLENRLSAIHQEADIMAAQAKVSQLQGKLASTLKQSDSLNLQVRQLNDT |
| gas40M3_3040 | (551) | SSLENRLSAIHQEADIMAAQAKVSQLQGKLASTLKQSDSLNLQVRQLNDT |
| gas40M3_3135 | (551) | SSLENRLSAIHQEADIMAAQAKVSQLQGKLASTLKQSDSLNLQVRQLNDT |
| gas40M12_10035 | (551) | SSLENRLSAIHQEADIMAAQAKVSQLQGKLASTLKQSDSLNLQVRQLNDT |
| gas40M12_22432 | (551) | SSLENRLSAIHQEADIMAAQAKVSQLQGKLASTLKQSDSLNLQVRQLNDT |
| gas40M4_40499 | (551) | SSLENRLSAIHQEADIMAAQAKVSQLQGKLASTLKQSDSLNLQVRQLNDT |
| gas40M12_2728(EM5) | (551) | SSLENRLSAIHQEADIMAAQAKVSQLQGKLASTLKQSDSLNLQVRQLNDT |
| gas40M89_10070 | (551) | SSLENRLSAIHQEADIMAAQAKVSQLQGKLASTLKQSDSLNLQVRQLNDT |
| gas40M89_21915 | (551) | SSLENRLSAIHQEADIMAAQAKVSQLQGKLASTLKQSDSLNLQVRQLNDT |
| gas40M89_23717 | (551) | SSLENRLSAIHQEADIMAAQAKVSQLQGKLASTLKQSDSLNLQVRQLNDT |
| gas40M89_5476 | (551) | SSLENRLSAIHQEADIMAAQAKVSQLQGKLASTLKQSDSLNLQVRQLNDT |
| gas40M23_DSM2071 | (551) | SSLENRLSAIHQEADIMAAQAKVSQLEGKLASTLKQSDSLNLQVRQLNDT |
| gas40M4_2722 | (551) | SSLENRLSAIHQEADIMAAQAKVSQLQGKLASTLKQSDSLNLQVRQLNDT |
| gas40M4_10092 | (551) | SSLENRLSAIHQEADIMAAQAKVSQLQGKLASTLKQSDSLNLQVRQLNDT |
| gas40M4_30968 | (551) | SSLENRLSAIHQEADIMAAQAKVSQLQGKLASTLKQSDSLNLQVRQLNDT |
| gas40M4_2634 | (551) | SSLENRLSAIHQEADIMAAQAKVSQLQGKLASTLKQSDSLNLQVRQLNDT |
| gas40M28_10164 | (551) | SSLENRLSAIHQEADIMAAQAKVSQLQGKLASTLKQSDSLNLQVRQLNDT |
| gas40M28_10218 | (551) | SSLENRLSAIHQEADIMAAQAKVSQLQGKLASTLKQSDSLNLQVRQLNDT |
| gas40M28_10266 | (551) | SSLENRLSAIHQEADIMAAQAKVSQLQGKLASTLKQSDSLNLQVRQLNDT |
| gas40M28_10299 | (551) | SSLENRLSAIHQEADIMAAQAKVSQLQGKLASTLKQSDSLNLQVRQLNDT |
| gas40M28_30176 | (551) | SSLENRLSAIHQEADIMAAQAKVSQLQGKLASTLKQSDSLNLQVRQLNDT |
| gas40M28_4436 | (551) | SSLENRLSAIHQEADIMAAQAKVSQLQGKLASTLKQSDSLNLQVRQLNDT |
| gas40M8_2725 | (551) | SSLENRLSAIHQEADIMAAQAKVSQLQGKLASTLKQSDSLNLQVRQLNDT |
| gas40M44_3776 | (551) | SSLENRLSAIHQEADIMAAQAKVSQLEGKLASTLKQSDSLNLQVRQLNDT |
| gas40M6_2724 | (551) | SSLENRLSAIHQEADIMAAQAKVSQLEGKLASTLKQSDSLNLQVRQLNDT |
| gas40M6_2894 | (551) | SSLENRLSAIHQEADIMAAQAKVSQLEGKLASTLKQSDSLNLQVRQLNDT |
| gas40M6_3650 | (551) | SSLENRLSAIHQEADIMAAQAKVSQLEGKLASTLKQSDSLNLQVRQLNDT |
| gas40M6_5529 | (551) | SSLENRLSAIHQEADIMAAQAKVSQLEGKLASTLKQSDSLNLQVRQLNDT |
| gas40M5_ | (551) | SSLENRLSAIHQEADIMAAQAKVSQLEGKLASTLKQSDSLNLQVRQLNDT |
| gas40M77_4959 | (551) | SSLENRLSAIHQEADIMAAQAKVSQLEGKLASTLKQSDSLNLQVRQLNDT |
| gas40M2_10064 | (551) | SSLENRLSAIHQEADIMAAQAKVSQLQGKLASTLKQSDSLNLQVRQLNDT |
| gas40M2_10065 | (551) | SSLENRLSAIHQEADIMAAQAKVSQLQGKLASTLKQSDSLNLQVRQLNDT |
| gas40M75_5531 | (551) | SSLENRLSAIHQEADIMAAQAKVSQLQGKLASTLKQSDSLNLQVRQLNDT |
| gas40M50_4538 | (551) | SSLENRLSAIHQEADIMAAQAKVSQLQGKLASTLKQSDSLNLQVRQLNDT |
| gas40M62_5455 | (551) | SSLENRLSAIHQEADIMAAQAKVSQLQGKLASTLKQSDSLNLQVRQLNDT |
| gas40M44_5481 | (551) | SSLENRLSAIHQEADIMAAQAKVSQLQGKLASTLKQSDSLNLQVRQLNDT |
| gas40M5_4883 | (551) | SSLENRLSAIHQEADIMAAQAKVSQLQGKLASTLKQSDSLNLQVRQLNDT |
| gas40M9?_2720 | (551) | SSLENRLSAIHQEADIMAAQAKVSQLQGKLASTLKQSDSLNLQVRQLNDT |
| gas40M2_2726 | (551) | SSLENRLSAIHQEADIMAAQAKVSQLQGKLASTLKQSDSLNLQVRQLNDT |
| gas40M12_20296 | (551) | SSLENRLSAIHQEADIMAAQAKVSQLQGKLASTLKQSDSLNLQVRQLNDT |
| gas40M1_2580 | (551) | SSLENRLSAIHQEADIMAAQAKVSQLQGKLASTLKQSDSLNLQVRQLNDT |
| gas40M1_2913 | (551) | SSLENRLSAIHQEADIMAAQAKVSQLQGKLASTLKQSDSLNLQVRQLNDT |
| gas40M1_3280 | (551) | SSLENRLSAIHQEADIMAAQAKVSQLQGKLASTLKQSDSLNLQVRQLNDT |
| gas40M1_3348 | (551) | SSLENRLSAIHQEADIMAAQAKVSQLQGKLASTLKQSDSLNLQVRQLNDT |
| gas40M78_3789 | (551) | SSLENRLSAIHQEADIMAAQAKVSQLQGKLASTLKQSDSLNLQVRQLNDT |
| gas40M?_2719 | (551) | SSLENRLSAIHQEADIMAAQAKVSQLQGKLASTLKQSDSLNLQVRQLNDT |

FIG. 32M

| | | 601 650 |
|---|---|---|
| gas40M1_SF370 | (601) | KGSLRTELLAAKAKQAQLEATRDQSLAKLASLKAALHQTEALAEQAAARV |
| gas40clinicalisolate_4088 | (601) | KGSLRTELLAAKAKQAQLEATRDQSLAKLASLKAALHQTEALAEQAAARV |
| gas40M11_2727 | (601) | KGSLRTELLAAKAKQAQLEATRDQSLAKLASLKAALHQTEALAEQAAARV |
| gas40M22_20641 | (601) | KGSLRTELLVAKAKQAQLEATRDQSLAKLASLKAAMHQTKALAEQAAARV |
| gas40M22_23465 | (601) | KGSLRTELLVAKAKQAQLEATRDQSLAKLASLKAAMHQTKALAEQAAARV |
| gas40M22_23621 | (601) | KGSLRTELLVAKAKQAQLEATRDQSLAKLASLKAAMHQTKALAEQAAARV |
| gas40M3,1_30610 | (601) | KGSLRTELLVAKAKQAQLEATRDQSLAKLASLKAAMHQTKALAEQAAARV |
| gas40M3,1_40603 | (601) | KGSLRTELLVAKAKQAQLEATRDQSLAKLASLKAAMHQTKALAEQAAARV |
| gas40M3,34_10307 | (601) | KGSLRTELLVAKAKQAQLEATRDQSLAKLASLKAAMHQTKALAEQAAARV |
| gas40M3_MGAS315 | (601) | KGSLRTELLVAKAKQAQLEATRDQSLAKLASLKAAMHQTKALAEQAAARV |
| gas40M4_40427 | (601) | KGSLRTELLVAKAKQAQLEATRDQSLAKLASLKAAMHQTKALAEQAAARV |
| gas40M3_2721 | (601) | KGSLRTELLVAKAKQAQLEATRDQSLAKLASLKAAMHQTKALAEQAAARV |
| gas40M3_3040 | (601) | KGSLRTELLVAKAKQAQLEATRDQSLAKLASLKAAMHQTKALAEQAAARV |
| gas40M3_3135 | (601) | KGSLRTELLVAKAKQAQLEATRDQSLAKLASLKAAMHQTKALAEQAAARV |
| gas40M12_10035 | (601) | KGSLRTELLAAKAKQAQLEATRDQSLAKLASLKAALHQTEALAEQAAARV |
| gas40M12_22432 | (601) | KGSLRTELLAAKAKQAQLEATRDQSLAKLASLKAALHQTEALAEQAAARV |
| gas40M4_40499 | (601) | KGSLRTELLAAKAKQAQLEATRDQSLAKLASLKAALHQTEALAEQAAARV |
| gas40M12_2728(EM5) | (601) | KGSLRTELLAAKAKQAQLEATRDQSLAKLASLKAALHQTEALAEQAAARV |
| gas40M89_10070 | (601) | KGSLRTELLAAKAKQAQLEATRDQSLAKLASLKAALHQTEALAEQAAARV |
| gas40M89_21915 | (601) | KGSLRTELLAAKAKQAQLEATRDQSLAKLASLKAALHQTEALAEQAAARV |
| gas40M89_23717 | (601) | KGSLRTELLAAKAKQAQLEATRDQSLAKLASLKAALHQTEALAEQAAARV |
| gas40M89_5476 | (601) | KGSLRTELLAAKAKQAQLEATRDQSLAKLASLKAALHQTEALAEQAAARV |
| gas40M23_DSM2071 | (601) | KGSLRTELLAAKAKQAQLEATRDQSLAKLASLKAALHQTEALAEQAAARV |
| gas40M4_2722 | (601) | KGSLRTELLAAKAKQAQLEATRDQSLAKLASLKAALHQTEALAEQAAARV |
| gas40M4_10092 | (601) | KGSLRTELLAAKAKQAQLEATRDQSLAKLASLKAALHQTEALAEQAAARV |
| gas40M4_30968 | (601) | KGSLRTELLAAKAKQAQLEATRDQSLAKLASLKAALHQTEALAEQAAARV |
| gas40M4_2634 | (601) | KGSLRTELLAAKAKQAQLEATRDQSLAKLASLKAALHQTEALAEQAAARV |
| gas40M28_10164 | (601) | KGSLRTELLAAKAKQAQLEATRDQSLAKLASLKAALHQTEVLAEQAAARV |
| gas40M28_10218 | (601) | KGSLRTELLAAKAKQAQLEATRDQSLAKLASLKAALHQTEVLAEQAAARV |
| gas40M28_10266 | (601) | KGSLRTELLAAKAKQAQLEATRDQSLAKLASLKAALHQTEVLAEQAAARV |
| gas40M28_10299 | (601) | KGSLRTELLAAKAKQAQLEATRDQSLAKLASLKAALHQTEVLAEQAAARV |
| gas40M28_30176 | (601) | KGSLRTELLAAKAKQAQLEATRDQSLAKLASLKAALHQTEVLAEQAAARV |
| gas40M28_4436 | (601) | KGSLRTELLAAKAKQAQLEATRDQSLAKLASLKAALHQTEVLAEQAAARV |
| gas40M8_2725 | (601) | KGSLRTELLAAKAKQAQLEATRDQSLAKLASLKAALHQTEALAEQAAARV |
| gas40M44_3776 | (601) | KGSLRTELLAAKAKQAQLEATRDQSLAKLASLKAALHQTEALAEQAAARV |
| gas40M6_2724 | (601) | KGSLRTELLAAKAKQAQLEATRDQSLAKLASLKAALHQTEALAEQAAARV |
| gas40M6_2894 | (601) | KGSLRTELLAAKAKQAQLEATRDQSLAKLASLKAALHQTEALAEQAAARV |
| gas40M6_3650 | (601) | KGSLRTELLAAKAKQAQLEATRDQSLAKLASLKAALHQTEALAEQAAARV |
| gas40M6_5529 | (601) | KGSLRTELLAAKAKQAQLEATRDQSLAKLASLKAALHQTEALAEQAAARV |
| gas40M5_ | (601) | KGSLRTELLAAKAKQAQLEATRDQSLAKLASLKAALHQTEALAEQAAARV |
| gas40M77_4959 | (601) | KGSLRTELLAAKAKQAQLEATRDQSLAKLASLKAALHQTEALAEQAAARV |
| gas40M2_10064 | (601) | KGSLRTELLAAKAKQAQLEATRDQSLAKLASLKAALHQTEALAEQAAARV |
| gas40M2_10065 | (601) | KGSLRTELLAAKAKQAQLEATRDQSLAKLASLKAALHQTEALAEQAAARV |
| gas40M75_5531 | (601) | KGSLRTELLAAKAKQAQLEATRDQSLAKLASLKAALHQTEALAEQAAARV |
| gas40M50_4538 | (601) | KGSLRTELLAAKAKQAQLEATRDQSLAKLASLKAALHQTEALAEQAAARV |
| gas40M62_5455 | (601) | KGSLRTELLAAKAKQAQLEATRDQSLAKLASLKAALHQTEALAEQAAARV |
| gas40M44_5481 | (601) | KGSLRTELLAAKAKQAQLEATRDQSLAKLASLKAALHQTEALAEQAAARV |
| gas40M5_4883 | (601) | KGSLRTELLAAKAKQAQLEATRDQSLAKLASLKAALHQTEALAEQAAARV |
| gas40M9?_2720 | (601) | KGSLRTELLAAKAKQAQLEATRDQSLAKLASLKAALHQTEALAEQAAARV |
| gas40M2_2726 | (601) | KGSLRTELLVAKAKQAQLEATRDQSLAKLASLKAAMHQTKALAEQAAARV |
| gas40M12_20296 | (601) | KGSLRTELLAAKAKQAQLEATRDQSLAKLASLKAALHQTEALAEQAAARV |
| gas40M1_2580 | (601) | KGSLRTELLAAKAKQAQLEATRDQSLAKLASLKAALHQTEALAEQAAARV |
| gas40M1_2913 | (601) | KGSLRTELLAAKAKQAQLEATRDQSLAKLASLKAALHQTEALAEQAAARV |
| gas40M1_3280 | (601) | KGSLRTELLAAKAKQAQLEATRDQSLAKLASLKAALHQTEALAEQAAARV |
| gas40M1_3348 | (601) | KGSLRTELLAAKAKQAQLEATRDQSLAKLASLKAALHQTEALAEQAAARV |
| gas40M78_3789 | (601) | KGSLRTELLAAKAKQAQLEATRDQSLAKLASLKAALHQTEALAEQAAARV |
| gas40M?_2719 | (601) | KGSLRTELLAAKAKQAQLEATRDQSLAKLASLKAALHQTEALAEQAAARV |

FIG. 32N

| | | |
|---|---|---|
| | | 651 700 |
| gas40M1_SF370 | (651) | TALVAKKAHLQYLRDFKLNPNRLQVIRERIDNTKQDLAKTTSSLLNAQEA |
| gas40clinicalisolate_4088 | (651) | TALVAKKAHLQYLRDFKLNPNRLQVIRERIDNTKQDLAKTTSSLLNAQEA |
| gas40M11_2727 | (651) | TALVAKKAHLQYLRDFKLNPNRLQVIRERIDNTKQDLAKTTSSLLNAQEA |
| gas40M22_20641 | (651) | TALVAKKAHLQYLRDFKLNPNRLQVIRERIDNTKQDLAKTTSSLLNAQEA |
| gas40M22_23465 | (651) | TALVAKKAHLQYLRDFKLNPNRLQVIRERIDNTKQDLAKTTSSLLNAQEA |
| gas40M22_23621 | (651) | TALVAKKAHLQYLRDFKLNPNRLQVIRERIDNTKQDLAKTTSSLLNAQEA |
| gas40M3,1_30610 | (651) | TALVAKKAHLQYLRDFKLNPNRLQVIRERIDNTKQDLAKTTSSLLNAQEA |
| gas40M3,1_40603 | (651) | TALVAKKAHLQYLRDFKLNPNRLQVIRERIDNTKQDLAKTTSSLLNAQEA |
| gas40M3,34_10307 | (651) | TALVAKKAHLQYLRDFKLNPNRLQVIRERIDNTKQDLAKTTSSLLNAQEA |
| gas40M3_MGAS315 | (651) | TALVAKKAHLQYLRDFKLNPNRLQVIRERIDNTKQDLAKTTSSLLNAQEA |
| gas40M4_40427 | (651) | TALVAKKAHLQYLRDFKLNPNRLQVIRERIDNTKQDLAKTTSSLLNAQEA |
| gas40M3_2721 | (651) | TALVAKKAHLQYLRDFKLNPNRLQVIRERIDNTKQDLAKTTSSLLNAQEA |
| gas40M3_3040 | (651) | TALVAKKAHLQYLRDFKLNPNRLQVIRERIDNTKQDLAKTTSSLLNAQEA |
| gas40M3_3135 | (651) | TALVAKKAHLQYLRDFKLNPNRLQVIRERIDNTKQDLAKTTSSLLNAQEA |
| gas40M12_10035 | (651) | TALVAKKAHLQYLKDFKLNPNRLQVIRERIDNTKQDLAKTTSSLLNAQEA |
| gas40M12_22432 | (651) | TALVAKKAHLQYLKDFKLNPNRLQVIRERIDNTKQDLAKTTSSLLNAQEA |
| gas40M4_40499 | (651) | TALVAKKAHLQYLKDFKLNPNRLQVIRERIDNTKQDLAKTTSSLLNAQEA |
| gas40M12_2728(EM5) | (651) | TALVAKKAHLQYLRDFKLNPNRLQVIRERIDNTKQDLAKTTSSLLNAQEA |
| gas40M89_10070 | (651) | TALVAKKAHLQYLRDFKLNPNRLQVIRERIDNTKQDLAKTTSSLLNAQEA |
| gas40M89_21915 | (651) | TALVAKKAHLQYLRDFKLNPNRLQVIRERIDNTKQDLAKTTSSLLNAQEA |
| gas40M89_23717 | (651) | TALVAKKAHLQYLRDFKLNPNRLQVIRERIDNTKQDLAKTTSSLLNAQEA |
| gas40M89_5476 | (651) | TALVAKKAHLQYLRDFKLNPNRLQVIRERIDNTKQDLAKTTSSLLNAQEA |
| gas40M23_DSM2071 | (651) | TALVAKKAHLQYLRDFKLNPNRLQVIRERIDNTKQDLAKTTSSLLNAQEA |
| gas40M4_2722 | (651) | TALVAKKAHLQYLRDFKLNPNRLQVIRERIDNTKQDLAKTTSSLLNAQEA |
| gas40M4_10092 | (651) | TALVAKKAHLQYLRDFKLNPNRLQVIRERIDNTKQDLAKTTSSLLNAQET |
| gas40M4_30968 | (651) | TALVAKKAHLQYLRDFKLNPNRLQVIRERIDNTKQDLAKTTSSLLNAQET |
| gas40M4_2634 | (651) | TALVAKKAHLQYLRDFKLNPNRLQVIRERIDNIKQDLAKTTSSLLNAQET |
| gas40M28_10164 | (651) | TALVAKKAHLQYLRDFKLNPNRLQVIRERIDNTKQDLAKTTSSLLNAQET |
| gas40M28_10218 | (651) | TALVAKKAHLQYLRDFKLNPNRLQVIRERIDNTKQDLAKTTSSLLNAQET |
| gas40M28_10266 | (651) | TALVAKKAHLQYLRDFKLNPNRLQVIRERIDNTKQDLAKTTSSLLNAQET |
| gas40M28_10299 | (651) | TALVAKKAHLQYLRDFKLNPNRLQVIRERIDNTKQDLAKTTSSLLNAQET |
| gas40M28_30176 | (651) | TALVAKKAHLQYLRDFKLNPNRLQVIRERIDNTKQDLAKTTSSLLNAQET |
| gas40M28_4436 | (651) | TALVAKKAHLQYLRDFKLNPNRLQVIRERIDNTKQDLAKTTSSLLNAQET |
| gas40M8_2725 | (651) | TALVAKKAHLQYLRDFKLNPNRLQVIRERIDNTKQDLAKTTSSLLNAQET |
| gas40M44_3776 | (651) | TALVAKKAHLQYLRDFKLNPNRLQVIRERIDNTKQDLAKTTSSLLNAQEA |
| gas40M6_2724 | (651) | TALVAKKAHLQYLRDFKLNPNRLQVIRERIDNTKQDLAKTTSSLLNAQEA |
| gas40M6_2894 | (651) | TALVAKKAHLQYLRDFKLNPNRLQVIRERIDNTKQDLAKTTSSLLNAQEA |
| gas40M6_3650 | (651) | TALVAKKAHLQYLRDFKLNPNRLQVIRERIDNTKQDLAKTTSSLLNAQEA |
| gas40M6_5529 | (651) | TALVAKKAHLQYLRDFKLNPNRLQVIRERIDNTKQDLAKTTSSLLNAQEA |
| gas40M5_ | (651) | TALVAKKAHLQYLRDFKLNPNRLQVIRERIDNTKQDLAKTTSSLLNAQEA |
| gas40M77_4959 | (651) | TALVAKKAHLQYLRDFKLNPNRLQVIRERIDNTKQDLAKTTSSLLNAQEA |
| gas40M2_10064 | (651) | TALVAKKAHLQYLRDFKLNPNRLQVIRERIDNTKQDLAKTTSSLLNAQET |
| gas40M2_10065 | (651) | TALVAKKAHLQYLRDFKLNPNRLQVIRERIDNTKQDLAKTTSSLLNAQET |
| gas40M75_5531 | (651) | TALVAKKAHLQYLRDFKLNPNRLQVIRERIDNTKQDLAKTTSSLLNAQET |
| gas40M50_4538 | (651) | TALVAKKAHLQYLRDFKLNPNRLQVIRERIDNTKQDLAKTTSSLLNAQET |
| gas40M62_5455 | (651) | TALVAKKAHLQYLRDFKLNPNRLQVIRERIDNTKQDLAKTTSSLLNAQET |
| gas40M44_5481 | (651) | TALVAKKAHLQYLRDFKLNPNRLQVIRERIDNTKQDLAKTTSSLLNAQEA |
| gas40M5_4883 | (651) | TALVAKKAHLQYLRDFKLNPNRLQVIRERIDNTKQDLAKTTSSLLNAQEA |
| gas40M9?_2720 | (651) | TALVAKKAHLQYLRDFKLNPNRLQVIRERIDNTKQDLAKTTSSLLNAQEA |
| gas40M2_2726 | (651) | TALVAKKAHLQYLRDFKLNPNRLQVIRERIDNTKQDLAKTTSSLLNAQET |
| gas40M12_20296 | (651) | TALVAKKAHLQYLRDFKLNPNRLQVIRERIDNTKQDLAKTTSSLLNAQEA |
| gas40M1_2580 | (651) | TALVAKKAHLQYLRDFKLNPNRLQVIRERIDNTKQDLAKTTSSLLNAQEA |
| gas40M1_2913 | (651) | TALVAKKAHLQYLRDFKLNPNRLQVIRERIDNTKQDLAKTTSSLLNAQEA |
| gas40M1_3280 | (651) | TALVAKKAHLQYLRDFKLNPNRLQVIRERIDNTKQDLAKTTSSLLNAQEA |
| gas40M1_3348 | (651) | TALVAKKAHLQYLRDFKLNPNRLQVIRERIDNTKQDLAKTTSSLLNAQEA |
| gas40M78_3789 | (651) | TALVAKKAHLQYLRDFKLNPNRLQVIRERIDNTKQDLAKTTSSLLNAQEA |
| gas40M?_2719 | (651) | TALVAKKAHLQYLRDFKLNPNRLQVIRERIDNTKQDLAKTTSSLLNAQEA |

FIG. 32O

| | | |
|---|---|---|
| | | 701 750 |
| gas40M1_SF370 | (701) | LAALQAKQSSLEATIATTEHQLTLLKTLANEKEYRHLDEDIATVPDLQVA |
| gas40clinicalisolate_4088 | (701) | LAALQAKQSSLEATIATTEHQLTLLKTLANEKEYRHLDEDIATVPDLQVA |
| gas40M11_2727 | (701) | LAALQAKQSSLEATIATTEHQLTLLKTLANEKEYRHLDEDIATVPDLQVA |
| gas40M22_20641 | (701) | LAALQAKQSSLEATIATTEHQLTLLKTLANEKEYRHLDEDIATVPDLQVA |
| gas40M22_23465 | (701) | LAALQAKQSSLEATIATTEHQLTLLKTLANEKEYRHLDEDIATVPDLQVA |
| gas40M22_23621 | (701) | LAALQAKQSSLEATIATTEHQLTLLKTLANEKEYRHLDEDIATVPDLQVA |
| gas40M3,1_30610 | (701) | LAALQAKQSSLEATIATTEHQLTLLKTLANEKEYRHLDEDIATVPDLQVA |
| gas40M3,1_40603 | (701) | LAALQAKQSSLEATIATTEHQLTLLKTLANEKEYRHLDEDIATVPDLQVA |
| gas40M3,34_10307 | (701) | LAALQAKQSSLEATIATTEHQLTLLKTLANEKEYRHLDEDIATVPDLQVA |
| gas40M3_MGAS315 | (701) | LAALQAKQSSLEATIATTEHQLTLLKTLANEKEYRHLDEDIATVPDLQVA |
| gas40M4_40427 | (701) | LAALQAKQSSLEATIATTEHQLTLLKTLANEKEYRHLDEDIATVPDLQVA |
| gas40M3_2721 | (701) | LAALQAKQSSLEATIATTEHQLTLLKTLANEKEYRHLDEDIATVPDLQVA |
| gas40M3_3040 | (701) | LAALQAKQSSLEATIATTEHQLTLLKTLANEKEYRHLDEDIATVPDLQVA |
| gas40M3_3135 | (701) | LAALQAKQSSLEATIATTEHQLTLLKTLANEKEYRHLDEDIATVPDLQVA |
| gas40M12_10035 | (701) | LAALQAKQSSLEATIATTEHQLTLLKTLANEKEYRHLDEDIATVPDLQVA |
| gas40M12_22432 | (701) | LAALQAKQSSLEATIATTEHQLTLLKTLANEKEYRHLDEDIATVPDLQVA |
| gas40M4_40499 | (701) | LAALQAKQSSLEATIATTEHQLTLLKTLANEKEYRHLDEDIATVPDLQVA |
| gas40M12_2728(EM5) | (701) | LAALQAKQSSLEATIATTEHQLTLLKTLANEKEYRHLDEDIATVPDLQVA |
| gas40M89_10070 | (701) | LAALQAKQSSLEATIATTEHQLTLLKTLANEKEYRHLDEDIATVPDLQVA |
| gas40M89_21915 | (701) | LAALQAKQSSLEATIATTEHQLTLLKTLANEKEYRHLDEDIATVPDLQVA |
| gas40M89_23717 | (701) | LAALQAKQSSLEATIATTEHQLTLLKTLANEKEYRHLDEDIATVPDLQVA |
| gas40M89_5476 | (701) | LAALQAKQSSLEATIATTEHQLTLLKTLANEKEYRHLDEDIATVPDLQVA |
| gas40M23_DSM2071 | (701) | LAALQAKQSSLEATIATTEHQLTLLKTLANENEYRHLDEDIATVPDLQVA |
| gas40M4_2722 | (701) | LAALQAKQSSLEATIATTEHQLTLLKTLANEKEYRHLDEDIATVPDLQVA |
| gas40M4_10092 | (701) | LAALQAKQSGLEATIATTEHQLTLLKTLANEKEYRHLDEDIATVPDLQVA |
| gas40M4_30968 | (701) | LAALQAKQSGLEATIATTEHQLTLLKTLANEKEYRHLDEDIATVPDLQVA |
| gas40M4_2634 | (701) | LAALQAKQSSLEATIATTEHQLTLLKTLANEKEYRHLDEDIATVPDLQVA |
| gas40M28_10164 | (701) | LAALQAKQSSLEATIATTEHQLTLLKTLANEKGYRHLDEDIATVPDLQVA |
| gas40M28_10218 | (701) | LAALQAKQSSLEATIATTEHQLTLLKTLANEKGYRHLDEDIATVPDLQVA |
| gas40M28_10266 | (701) | LAALQAKQSSLEATIATTEHQLTLLKTLANEKGYRHLDEDIATVPDLQVA |
| gas40M28_10299 | (701) | LAALQAKQSSLEATIATTEHQLTLLKTLANEKGYRHLDEDIATVPDLQVA |
| gas40M28_30176 | (701) | LAALQAKQSSLEATIATTEHQLTLLKTLANEKGYRHLDEDIATVPDLQVA |
| gas40M28_4436 | (701) | LAALQAKQSSLEATIATTEHQLTLLKTLANEKGYRHLDEDIATVPDLQVA |
| gas40M8_2725 | (701) | LAALQAKQSSLEATIATTEHQLTLLKTLANEKEYRHLDEDIATVPDLQVA |
| gas40M44_3776 | (701) | LAALQAKQSSLEATIATTEHQLTLLKTLANEKEYRHLDEDIATVPDLQVA |
| gas40M6_2724 | (701) | LAALQAKQSSLEATIATTEHQLTLLKILANEKEYRHLDEDIATVPDLQVA |
| gas40M6_2894 | (701) | LAALQAKQSSLEATIATTEHQLTLLKTLANEKEYRHLDEDIATVPDLQVA |
| gas40M6_3650 | (701) | LAALQAKQSSLEATIATTEHQLTLLKTLANEKEYRHLDEDIATVPDLQVA |
| gas40M6_5529 | (701) | LAALQAKQSSLEATIATTEHQLTLLKTLANEKEYRHLDEDIATVPDLQVA |
| gas40M5_ | (701) | LAVLQAKQSSLEATIATTEHQLTLLKTLANEKEYRHLDEDIATVPDLQVA |
| gas40M77_4959 | (701) | LAALQAKQSSLEATIATTEHQLTLLKTLANEKEYRHLDEDIATVPDLQVA |
| gas40M2_10064 | (701) | LAALQAKQSSLEATIATTEHQLTLLKTLANEKEYRHLDEDIATVPDLQVA |
| gas40M2_10065 | (701) | LAALQAKQSSLEATIATTEHQLTLLKTLANEKEYRHLDEDIATVPDLQVA |
| gas40M75_5531 | (701) | LAALQAKQSSLEATIATTEHQLTLLKTLANEKEYRHLDEDIATVPDLQVA |
| gas40M50_4538 | (701) | LAALQAKQSSLEATIATTEHQLTLLKTLANEKEYRHLDEDIATVPDLQVA |
| gas40M62_5455 | (701) | LAALQAKQSSLEATIATTEHQLTLLKTLANEKEYRHLDEDIATVPDLQVA |
| gas40M44_5481 | (701) | LAALQAKQSSLEATIATTEHQLTLLKTLANEKEYRHLDEDIATVPDLQVA |
| gas40M5_4883 | (701) | LAALQAKQSSLEATIATTEHQLTLLKTLANEKEYRHLDEDIATVPDLQVA |
| gas40M9?_2720 | (701) | LAALQAKQSSLEATIATTEHQLTLLKTLANEKEYRHLDEDIATVPDLQVA |
| gas40M2_2726 | (701) | LAALQAKKSSLEATIATTEHQLTLLKTLANEKEYRHLDEDIATVPDLQVA |
| gas40M12_20296 | (701) | LAALQAKQSSLEATIATTEHQLTLLKTLANEKEYRHLDEDIATVPDLQVA |
| gas40M1_2580 | (701) | LAALQAKQSSLEATIATTEHQLTLLKTLANEKEYRHLDEDIATVPDLQVA |
| gas40M1_2913 | (701) | LAALQAKQSSLEATIATTEHQLTLLKTLANEKEYRHLDEDIATVPDLQVA |
| gas40M1_3280 | (701) | LAALQAKQSSLEATIATTEHQLTLLKTLANEKEYRHLDEDIATVPDLQVA |
| gas40M1_3348 | (701) | LAALQAKQSSLEATIATTEHQLTLLKTLANEKEYRHLDEDIATVPDLQVA |
| gas40M78_3789 | (701) | LAALQAKQSSLEATIATTEHQLTLLKTLANEKEYRHLDEDIATVPDLQVA |
| gas40M?_2719 | (701) | LAALQAKQSSLEATIATTEHQLTLLKTLANEKEYRHLDEDIATVPDLQVA |

FIG. 32P

| | | 751 800 |
|---|---|---|
| gas40M1_SF370 | (751) | PPLTGVKPLSYSKIDTTPLVQEMVKETKQLLEASARLAAENTSLVAEALV |
| gas40clinicalisolate_4088 | (751) | PSLTGVKPLSYSKVETTPLVQEMVKETKQLLEASARLAAENTSLVAEALV |
| gas40M11_2727 | (751) | PSLTGVKLLSYSKVETTPLVQEMVKETKQLLEASARLAAENTSLVAEALV |
| gas40M22_20641 | (751) | PSLTGVKPLSYSKVETTPLVQEMVKETKQLLEASARLAAENTSLVAEALV |
| gas40M22_23465 | (751) | PSLTGVKPLSYSKVETTPLVQEMVKETKQLLEASARLAAENTSLVAEALV |
| gas40M22_23621 | (751) | PSLTGVKPLSYSKVETTPLVQEMVKETKQLLEASARLAAENTSLVAEALV |
| gas40M3,1_30610 | (751) | PSLTGVKPLSYSKVETTPLVQEMVKETKQLLEASARLAAENTSLVAEALV |
| gas40M3,1_40603 | (751) | PSLTGVKPLSYSKVETTPLVQEMVKETKQLLEASARLAAENTSLVAEALV |
| gas40M3,34_10307 | (751) | PSLTGVKPLSYSKVETTPLVQEMVKETKQLLEASARLAAENTSLVAEALV |
| gas40M3_MGAS315 | (751) | PSLTGVKPLSYSKVETTPLVQEMVKETKQLLEASARLAAENTSLVAEALV |
| gas40M4_40427 | (751) | PSLTGVKPLSYSKVETTPLVQEMVKETKQLLEASARLAAENTSLVAEALV |
| gas40M3_2721 | (751) | PSLTGVKPLSYSKVETTPLVQEMVKETKQLLEASARLAAENTSLVAEALV |
| gas40M3_3040 | (751) | PSLTGVKPLSYSKVETTPLVQEMVKETKQLLEASARLAAENTSLVAEALV |
| gas40M3_3135 | (751) | PSLTGVKPLSYSKVETTPLVQEMVKETKQLLEASARLAAENTSLVAEALV |
| gas40M12_10035 | (751) | PSLTGVKPLSYSKVETTPLVQEMVKETKQLLEASARLAAENTSLVAEALV |
| gas40M12_22432 | (751) | PSLTGVKPLSYSKVETTPLVQEMVKETKQLLEASARLAAENTSLVAEALV |
| gas40M4_40499 | (751) | PSLTGVKPLSYSKVETTPLVQEMVKETKQLLEASARLAAENTSLVAEALV |
| gas40M12_2728(EM5) | (751) | PSLTGVKPLSYSKVETTPLVQEMVKETKQLLEASARLAAENTSLVAEALV |
| gas40M89_10070 | (751) | PSLTGVKPLSYSKVETTPLVQEMVKETKQLLEASARLAAENTSLVAEALV |
| gas40M89_21915 | (751) | PSLTGVKPLSYSKVETTPLVQEMVKETKQLLEASARLAAENTSLVAEALV |
| gas40M89_23717 | (751) | PSLTGVKPLSYSKVETTPLVQEMVKETKQLLEASARLAAENTSLVAEALV |
| gas40M89_5476 | (751) | PSLTGVKPLSYSKVETTPLVQEMVKETKQLLEASARLAAENTSLVAEALV |
| gas40M23_DSM2071 | (751) | PPLTGVKPLSYSKIDTTPLVQEMVKETKQLLEASARLAAENTSLVAEALV |
| gas40M4_2722 | (751) | PPLTGVKPLSYSKIDTTPLVQEMVKETKQLLEASARLAAENTSLVAEALV |
| gas40M4_10092 | (751) | PPLTGVKPLSYSKIDTTPLVQEMVKETKQLLEASARLAAENTSLVAEALV |
| gas40M4_30968 | (751) | PPLTGVKPLSYSKIDTTPLVQEMVKETKQLLEASARLAAENTSLVAEALV |
| gas40M4_2634 | (751) | PPLTGVKPLSYSKIDTTPLVQEMVKETKQLLEASARLAAENTSLVAEALV |
| gas40M28_10164 | (751) | PPLTGVKPLSYSKIDTTPLVQEMVKETKQLLEASARLAAENTSLVAEALV |
| gas40M28_10218 | (751) | PPLTGVKPLSYSKIDTTPLVQEMVKETKQLLEASARLAAENTSLVAEALV |
| gas40M28_10266 | (751) | PPLTGVKPLSYSKIDTTPLVQEMVKETKQLLEASARLAAENTSLVAEALV |
| gas40M28_10299 | (751) | PPLTGVKPLSYSKIDTTPLVQEMVKETKQLLEASARLAAENTSLVAEALV |
| gas40M28_30176 | (751) | PPLTGVKPLSYSKIDTTPLVQEMVKETKQLLEASARLAAENTSLVAEALV |
| gas40M28_4436 | (751) | PPLTGVKPLSYSKIDTTPLVQEMVKETKQLLEASARLAAENTSLVAEALV |
| gas40M8_2725 | (751) | PSLTGVKPLSYSKIDTTPLVQEMVKETKQLLEASARLAAENTSLVAEALV |
| gas40M44_3776 | (751) | PPLTGVKPLSYSKIDTTPLVQEMVKETKQLLEASARLAAENTSLVAEALV |
| gas40M6_2724 | (751) | PPLTGVKPLSYSKIDTTPLVQEMVKETKQLLEASARLAAENTSLVAEALV |
| gas40M6_2894 | (751) | PPLTGVKPLSYSKIDTTPLVQEMVKETKQLLEASARLAAENTSLVAEALV |
| gas40M6_3650 | (751) | PPLTGVKPLSYSKIDTTPLVQEMVKETKQLLEASARLAAENTSLVAEALV |
| gas40M6_5529 | (751) | PPLTGVKPLSYSKIDTTPLVQEMVKETKQLLEASARLAAENTSLVAEALV |
| gas40M5_ | (751) | PPLTGVKPLSYSKIDTTPLVQEMVKETKQLLEASARLAAENTSLVAEALV |
| gas40M77_4959 | (751) | PPLTGVKPLSYSKIDTTPLVQEMVKETKQLLEASARLAAENTSLVAEALV |
| gas40M2_10064 | (751) | PPLTGVKPLSYSKIDTTPLVQEMVKETKQLLEASARLAAENTSLVAEALV |
| gas40M2_10065 | (751) | PPLTGVKPLSYSKIDTTPLVQEMVKETKQLLEASARLAAENTSLVAEALV |
| gas40M75_5531 | (751) | PPLTGVKPLSYSKIDTTPLVQEMVKETKQLLEASARLAAENTSLVAEALV |
| gas40M50_4538 | (751) | PPLTGVKPLSYSKIDTTPLVQEMVKETKQLLEASARLAAENTSLVAEALV |
| gas40M62_5455 | (751) | PPLTGVKPLSYSKIDTTPLVQEMVKETKQLLEASARLAAENTSLVAEALV |
| gas40M44_5481 | (751) | PPLTGVKPLSYSKIDTTPLVQEMVKETKQLLEASARLAAENTSLVAEALV |
| gas40M5_4883 | (751) | PPLTGVKPLSYSKIDTTPLVQEMVKETKQLLEASARLAAENTSLVAEALV |
| gas40M9?_2720 | (751) | PPLTGVKPLSYSKIDTTPLVQEMVKETKQLLEASARLAAENTSLVAEALV |
| gas40M2_2726 | (751) | PPLTGVKPLSYSKIDTTPLVQEMVKETKQLLEASARLAAENTSLVAEALV |
| gas40M12_20296 | (751) | PPLTGVKPLSYSKIDTTPLVQEMVKETKQLLEASARLAAENTSLVAEALV |
| gas40M1_2580 | (751) | PPLTGVKPLSYSKIDTTPLVQEMVKETKQLLEASARLAAENTSLVAEALV |
| gas40M1_2913 | (751) | PPLTGVKPLSYSKIDTTPLVQEMVKETKQLLEASARLAAENTSLVAEALV |
| gas40M1_3280 | (751) | PPLTGVKPLSYSKIDTTPLVQEMVKETKQLLEASARLAAENTSLVAEALV |
| gas40M1_3348 | (751) | PPLTGVKPLSYSKIDTTPLVQEMVKETKQLLEASARLAAENTSLVAEALV |
| gas40M78_3789 | (751) | PPLTGVKPLSYSKIDTTPLVQEMVKETKQLLEASARLAAENTSLVAEALV |
| gas40M?_2719 | (751) | PPLTGVKPLSYSKIDTTPLVQEMVKETKQLLEASARLAAENTSLVAEALV |

FIG. 32Q

| | | 801 850 |
|---|---|---|
| gas40M1_SF370 | (801) | GQTSEMVASNAIVSKITSSITQPSSKTSYGSGSSTTSNLISDVDESTQRA |
| gas40clinicalisolate_4088 | (801) | GQTSEMVASNAIVSKITSSITQPSSKTSYGSGSSTTSNLISDVDESTQRA |
| gas40M11_2727 | (801) | GQTSEMVASNAIVSKITSSITQPSSKTSYGSGSSTTSNLISDVDESTQRA |
| gas40M22_20641 | (801) | GQTSEMVASNAIVSKITSSITQPSSKTSYGSGSSTTSNLISDVDESTQRA |
| gas40M22_23465 | (801) | GQTSEMVASNAIVSKITSSITQPSSKTSYGSGSSTTSNLISDVDESTQRA |
| gas40M22_23621 | (801) | GQTSEMVASNAIVSKITSSITQPSSKTSYGSGSSTTSNLISDVDESTQRA |
| gas40M3,1_30610 | (801) | GQTSEMVASNAIVSKITSSITQPSSKTSYGSGSSTTSNLISDVDESTQRA |
| gas40M3,1_40603 | (801) | GQTSEMVASNAIVSKITSSITQPSSKTSYGSGSSTTSNLISDVDESTQRA |
| gas40M3,34_10307 | (801) | GQTSEMVASNAIVSKITSSITQPSSKTSYGSGSSTTSNLISDVDESTQRA |
| gas40M3_MGAS315 | (801) | GQTSEMVASNAIVSKITSSITQPSSKTSYGSGSSTTSNLISDVDESTQRA |
| gas40M4_40427 | (801) | GQTSEMVASNAIVSKITSSITQPSSKTSYGSGSSTTSNLISDVDESTQRA |
| gas40M3_2721 | (801) | GQTSEMVASNAIVSKITSSITQPSSKTSYGSGSSTTSNLISDVDESTQRA |
| gas40M3_3040 | (801) | GQTSEMVASNAIVSKITSSITQPSSKTSYGSGSSTTSNLISDVDESTQRA |
| gas40M3_3135 | (801) | GQTSEMVASNAIVSKITSSITQPSSKTSYGSGSSTTSNLISDVDESTQRA |
| gas40M12_10035 | (801) | GQTSEMVASNAIVSKITSSITQPSSKTSYGSGSSTTSNLISDVDESTQRA |
| gas40M12_22432 | (801) | GQTSEMVASNAIVSKITSSITQPSSKTSYGSGSSTTSNLISDVDESTQRA |
| gas40M4_40499 | (801) | GQTSEMVASNAIVSKITSSITQPSSKTSYGSGSSTTSNLISDVDESTQRA |
| gas40M12_2728(EM5) | (801) | GQTSEMVASNAIVSKITSSITQPSSKTSYGSGSSTTSNLISDVDESTQRA |
| gas40M89_10070 | (801) | GQTSEMVASNAIVSKITSSITQPSSKTSYGSGSSTTSNLISDVDESTQRA |
| gas40M89_21915 | (801) | GQTSEMVASNAIVSKITSSITQPSSKTSYGSGSSTTSNLISDVDESTQRA |
| gas40M89_23717 | (801) | GQTSEMVASNAIVSKITSSITQPSSKTSYGSGSSTTSNLISDVDESTQRA |
| gas40M89_5476 | (801) | GQTSEMVASNAIVSKITSSITQPSSKTSYGSGSSTTSNLISDVDESTQRA |
| gas40M23_DSM2071 | (801) | GQTSEMVASNAIVSKITSSITQPSSKTSYDSGSSTTSNLISDVDESTQRA |
| gas40M4_2722 | (801) | GQTSEMVASNAIVSKITSSITQPSSKTSYGSGSSTTSNLISDVDESTQRA |
| gas40M4_10092 | (801) | GQTSEMVASNAIVSKITSSITQPSSKTSYGSGSSTTSNLISDVDESTQRA |
| gas40M4_30968 | (801) | GQTSEMVASNAIVSKITSSITQPSSKTSYGSGSSTTSNLISDVDESTQRA |
| gas40M4_2634 | (801) | GQTSEMVASNAIVSKITSSITQPSSKTSYGSGSSTTSNLISDVDESTQRA |
| gas40M28_10164 | (801) | GQTSEMVASNAIVSKITSSITQPSSKTSYGSGSSTTSNLISDVDESTQRA |
| gas40M28_10218 | (801) | GQTSEMVASNAIVSKITSSITQPSSKTSYGSGSSTTSNLISDVDESTQRA |
| gas40M28_10266 | (801) | GQTSEMVASNAIVSKITSSITQPSSKTSYGSGSSTTSNLISDVDESTQRA |
| gas40M28_10299 | (801) | GQTSEMVASNAIVSKITSSITQPSSKTSYGSGSSTTSNLISDVDESTQRA |
| gas40M28_30176 | (801) | GQTSEMVASNAIVSKITSSITQPSSKTSYGSGSSTTSNLISDVDESTQRA |
| gas40M28_4436 | (801) | GQTSEMVASNAIVSKITSSITQPSSKTSYGSGSSTTSNLISDVDESTQRA |
| gas40M8_2725 | (801) | GQTSEMVASNAIVSKITSSITQPSSKTSYGSGSSTTSNLISDVDESTQRA |
| gas40M44_3776 | (801) | GQTSEMVASNAIVSKITSSITQPSSKTSYGSGSSTTSNLISDVDESTQRA |
| gas40M6_2724 | (801) | GQTSEMVASNAIVSKITSSITQPSSKTSYGSGSSTTSNLISDVDESTQRA |
| gas40M6_2894 | (801) | GQTSEMVASNAIVSKITSSITQPSSKTSYGSGSSTTSNLISDVDESTQRA |
| gas40M6_3650 | (801) | GQTSEMVASNAIVSKITSSITQPSSKTSYGSGSSTTSNLISDVDESTQRA |
| gas40M6_5529 | (801) | GQTSEMVASNAIVSKITSSITQPSSKTSYGSGSSTTSNLISDVDESTQRA |
| gas40M5_ | (801) | GQTSEMVASNAIVSKITSSITQPSSKTSYGSGSSTTSNLISDVDESTQRA |
| gas40M77_4959 | (801) | GQTSEMVASNAIVSKITSSITQPSSKTSYGSGSSTTSNLISDVDESTQRA |
| gas40M2_10064 | (801) | GQTSEMVASNAIVSKITSSITQPSSKTSYGSGSSTTSNLISDVDESTQRA |
| gas40M2_10065 | (801) | GQTSEMVASNAIVSKITSSITQPSSKTSYGSGSSTTSNLISDVDESTQRA |
| gas40M75_5531 | (801) | GQTSEMVASNAIVSKITSSITQPSSKTSYGSGSSTTSNLISDVDESTQRA |
| gas40M50_4538 | (801) | GQTSEMVASNAIVSKITSSITQPSSKTSYGSGSSTTSNLISDVDESTQRA |
| gas40M62_5455 | (801) | GQTSEMVASNAIVSKITSSITQPSSKTSYGSGSSTTSNLISDVDESTQRA |
| gas40M44_5481 | (801) | GQTSEMVASNAIVSKITSSITQPSSKTSYGSGSSTTSNLISDVDESTQRA |
| gas40M5_4883 | (801) | GQTSEMVASNAIVSKITSSITQPSSKTSYGSGSSTTSNLISDVDESTQRA |
| gas40M9?_2720 | (801) | GQTSEMVASNAIVSKITSSITQPSSKTSYGSGSSTTSNLISDVDESTQRA |
| gas40M2_2726 | (801) | GQTSEMVASNAIVSKITSSITQPSSKTSYGSGSSTTSNLISDIDESTQRA |
| gas40M12_20296 | (801) | GQTSEMVASNAIVSKITSSITQPSSKTSYGSGSSTTSNLISDVDESTQRA |
| gas40M1_2580 | (801) | GQTSEMVASNAIVSKITSSITQPSSKTSYGSGSSTTSNLISDVDESTQRA |
| gas40M1_2913 | (801) | GQTSEMVASNAIVSKITSSITQPSSKTSYGSGSSTTSNLISDVDESTQRA |
| gas40M1_3280 | (801) | GQTSEMVASNAIVSKITSSITQPSSKTSYGSGSSTTSNLISDVDESTQRA |
| gas40M1_3348 | (801) | GQTSEMVASNAIVSKITSSITQPSSKTSYGSGSSTTSNLISDVDESTQRA |
| gas40M78_3789 | (801) | GQTSEMVASNAIVSKITSSITQPSSKTSYGSGSSTTSNLISDVDESTQRA |
| gas40M?_2719 | (801) | GQTSEMVASNAIVSKITSSITQPSSKTSYGSGSSTTSNLISDVDESTQRA |

FIG. 32R

| | | 851 874 |
|---|---|---|
| gas40M1_SF370 | (851) | LKAGVVMLAAVGLTGFRFRKESK- |
| gas40clinicalisolate_4088 | (851) | LKAGVVMLAAVGLTGFRFRKESK- |
| gas40M11_2727 | (851) | LKAGVVMLAAVGLTGFRFRKESK- |
| gas40M22_20641 | (851) | LKAGVVMLAAVGLTGFRFRKESR- |
| gas40M22_23465 | (851) | LKAGVVMLAAVGLTGFRFRKESR- |
| gas40M22_23621 | (851) | LKAGVVMLAAVGLTGFRFRKESR- |
| gas40M3,1_30610 | (851) | LKAGVVMLAAVGLTGFRFRKESR- |
| gas40M3,1_40603 | (851) | LKAGVVMLAAVGLTGFRFRKESR- |
| gas40M3,34_10307 | (851) | LKAGVVMLAAVGLTGFRFRKESR- |
| gas40M3_MGAS315 | (851) | LKAGVVMLAAVGLTGFRFRKESR- |
| gas40M4_40427 | (851) | LKAGVVMLAAVGLTGFRFRKESR- |
| gas40M3_2721 | (851) | LKAGVVMLAAVGLTGFRFRKESK- |
| gas40M3_3040 | (851) | LKAGVVMLAAVGLTGFRFRKESK- |
| gas40M3_3135 | (851) | LKAGVVMLAAVGLTGFRFRKESK- |
| gas40M12_10035 | (851) | LKAGVVMLAAVGLTGFRFRKESR- |
| gas40M12_22432 | (851) | LKAGVVMLAAVGLTGFRFRKESR- |
| gas40M4_40499 | (851) | LKAGVVMLAAVGLTGFRFRKESR- |
| gas40M12_2728(EM5) | (851) | LKAGVVMLAAVGLTGFRFRKESK- |
| gas40M89_10070 | (851) | LKAGVVMLAAVGLTGFRFRKESR- |
| gas40M89_21915 | (851) | LKAGVVMLAAVGLTGFRFRKESR- |
| gas40M89_23717 | (851) | LKAGVVMLAAVGLTGFRFRKESR- |
| gas40M89_5476 | (851) | LKAGVVMLAAVGLTGFRFRKESK- |
| gas40M23_DSM2071 | (851) | LKAGVVMLAAVGLTGFRFRKESK- |
| gas40M4_2722 | (851) | LKAGVVMLAAVGLTGFRFRKESK- |
| gas40M4_10092 | (851) | LKAGVVMLAAVGLTGFRFRKESK- |
| gas40M4_30968 | (851) | LKAGVVMLAAVGLTGFRFRKESK- |
| gas40M4_2634 | (851) | LKAGVVMLAAVGLTGFRFRKESR- |
| gas40M28_10164 | (851) | LKAGVVMLAAVGLTGFRFRKESK- |
| gas40M28_10218 | (851) | LKAGVVMLAAVGLTGFRFRKESK- |
| gas40M28_10266 | (851) | LKAGVVMLAAVGLTGFRFRKESK- |
| gas40M28_10299 | (851) | LKAGVVMLAAVGLTGFRFRKESK- |
| gas40M28_30176 | (851) | LKAGVVMLAAVGLTGFRFRKESK- |
| gas40M28_4436 | (851) | LKAGVVMLAAVGLTGFRFRKESK- |
| gas40M8_2725 | (851) | LKAGVVMLAAVGLTGFRFRKESK- |
| gas40M44_3776 | (851) | LKAGVVMLAAVGLTGFRFRKESK- |
| gas40M6_2724 | (851) | LKAGVVMLAAVGLTGFRFRKESK- |
| gas40M6_2894 | (851) | LKAGVVMLAAVGLTGFRFRKESK- |
| gas40M6_3650 | (851) | LKAGVVMLAAVGLTGFRFRKESK- |
| gas40M6_5529 | (851) | LKAGVVMLAAVGLTGFRFRKESK- |
| gas40M5_ | (851) | LKAGVVMLAAVGLTGFRFRKESR- |
| gas40M77_4959 | (851) | LKAGVVMLAAVGLTGFRFRKESK- |
| gas40M2_10064 | (851) | LKAGVVMLAAVGLTGFRFRKESK- |
| gas40M2_10065 | (851) | LKAGVVMLAAVGLTGFRFRKESK- |
| gas40M75_5531 | (851) | LKAGVVMLAAVGLTGFRFRKESK- |
| gas40M50_4538 | (851) | LKAGVVMLAAIGLTGFRFRKESK- |
| gas40M62_5455 | (851) | LKAGVVMLAAIGLTGFRFRKESK- |
| gas40M44_5481 | (851) | LKAGVVMLAAVGLTGFRFRKESK- |
| gas40M5_4883 | (851) | LKAGVVMLAAVGLTGFRFRKESK- |
| gas40M9?_2720 | (851) | LKAGVVMLAAVGLTGFRFRKESK- |
| gas40M2_2726 | (851) | LKAGVVMLAAVGLTGVKLRKDTK- |
| gas40M12_20296 | (851) | LKAGVVMLAAVGLTGFRFRKESK- |
| gas40M1_2580 | (851) | LKAGVVMLAAVGLTGFRFRKESK- |
| gas40M1_2913 | (851) | LKAGVVMLAAVGLTGFRFRKESK- |
| gas40M1_3280 | (851) | LKAGVVMLAAVGLTGFRFRKESK- |
| gas40M1_3348 | (851) | LKAGVVMLAAVGLTGFRFRKESK- |
| gas40M78_3789 | (851) | LKAGVVMLAAVGLTGFRFRKESK- |
| gas40M?_2719 | (851) | LKAGVVMLAAVGLTGFRFRKESK- |

FIG. 33A

```
                               1                                                50
       GAS25_SF370     (1)  MSNKKTFKKYSRVAGLLTAALIIGNLVTANAESNKQNTASTETTTTNEQP
    GAS25_M12_2096     (1)  MSNKKTFKKYSRVAGLLTAALIIGNLVTANAESNKQNTASTETTTTNEQP
    GAS25_M12_9429     (1)  MSNKKTFKKYSRVAGLLTAALIIGNLVTANAESNKQNTASTETTTTNEQP
     GAS25_M1_5005     (1)  MSNKKTFKKYSRVAGLLTAALIIGNLVTANAESNKQNTASTETTTTNEQP
     GAS25_M1_3348     (1)  MSNKKTFKKYSRVAGLLTAALIIGNLVTANAESNKQNTASTETTTTNEQP
    GAS25_M2_10270     (1)  MSNKKTFKKYSRVAGLLTAALIIGNLVTANAESNKQNTTSTETTTTNEQP
    GAS25_M28_6180     (1)  MSNKKTFKKYSRVAGLLTAALIIGNLVTANAESNKQNTASTETTTTNEQP
    GAS25_M6_10394     (1)  MSNKKTFKKYSRVAGLLTVALIIGNLVTANAESNKQNTASTETTTTNEQP
    GAS25_M18_8232     (1)  MSNKKTFKKYSRVAGLLTAALIIGNLVTANAESNKQNTASTETTTTNEQP
GAS25_M5_Manfredo      (1)  MSNKKTFKKYSRVAGLLTAALIIGNLVTANAESNKQNTASTETTTTNEQP
      GAS25_M3_315     (1)  MSNKKTFKKYSRVAGLLTAALIIGNLVTANAESNKQNTASTETTTTNEQP
      GAS25_M3_SSI     (1)  MSNKKTFKKYSRVAGLLTAALIIGNLVTANAESNKQNTASTETTTTNEQP
    GAS25_M4_10750     (1)  MSNKKTFKKYSRVAGLLTAALIIGNLVTANAESNKQNTASTETTTTNEQP
                            51                                              100
       GAS25_SF370    (51)  KPESSELTTEKAGQKTDDMLNSNDMIKLAPKEMPLESAEKEEKKSEDKKK
    GAS25_M12_2096    (51)  KPESSELTTEKAGQKMDDMLNSNDMIKLAPKEMPLESAEKEEKKSEDKKK
    GAS25_M12_9429    (51)  KPESSELTTEKAGQKMDDMLNSNDMIKLAPKEMPLESAEKEEKKSEDKKK
     GAS25_M1_5005    (51)  KPESSELTTEKAGQKMDDMLNSNDMIKLAPKEMPLESAEKEEKKSEDKKK
     GAS25_M1_3348    (51)  KPESSELTTEKAGQKMDDMLNSNDMIKLAPKEMPLESAEKEEKKSEDKKK
    GAS25_M2_10270    (51)  KPESSELTIEKAGQKMDDMLNSNDMIKLAPKEMPLESAEKEEKKSEDKKK
    GAS25_M28_6180    (51)  KPESSELTIEKAGQKMDDMLNSNDMIKLAPKEMPLESAEKEEKKSEDKKK
    GAS25_M6_10394    (51)  KPESSELTIEKAGQKMDDMLNSNDMIKLAPKEMPLESAEKEEKKSEDKKK
    GAS25_M18_8232    (51)  KPESSELTTEKAGQKTDDMLNSNDMIKLAPKEMPLESAEKEEKKSEDKKK
GAS25_M5_Manfredo     (51)  KPESSELTTEKAGQKTDDMLNSNDMIKLAPKEMPLESAEKEEKKSEDKKK
      GAS25_M3_315    (51)  KPESSELTTEKAGQKTDDMLNSNDMIKLAPKEMPLESAEKEEKKSEDKKK
      GAS25_M3_SSI    (51)  KPESSELTTEKAGQKTDDMLNSNDMIKLAPKEMPLESAEKEEKKSEDKKK
    GAS25_M4_10750    (51)  KPESSELTTEKAGQKTDDMLNSNDMIKLAPKEMPLESAEKEEKKSEDKKK
                            101                                             150
       GAS25_SF370   (101)  SEEDHTEEINDKIYSLNYNELEVLAKNGETIENFVPKEGVKKADKFIVIE
    GAS25_M12_2096   (101)  SEEDHTEEINDKIYSLNYNELEVLAKNGETIENFVPKEGVKKADKFIVIE
    GAS25_M12_9429   (101)  SEEDHTEEINDKIYSLNYNELEVLAKNGETIENFVPKEGVKKADKFIVIE
     GAS25_M1_5005   (101)  SEEDHTEEINDKIYSLNYNELEVLAKNGETIENFVPKEGVKKADKFIVIE
     GAS25_M1_3348   (101)  SEEDHTEEINDKIYSLNYNELEVLAKNGETIENFVPKEGVKKADKFIVIE
    GAS25_M2_10270   (101)  SEEDHTEEINDKIYSLNYNELEVLAKNGETIENFVPKEGVKKADKFIVIE
    GAS25_M28_6180   (101)  SEEDHTEEINDKIYSLNYNELEVLAKNGETIENFVPKEGVKKADKFIVIE
    GAS25_M6_10394   (101)  SEEDHTEEINDKIYSLNYNELEVLAKNGETIENFVPKEGVKKADKFIVIE
    GAS25_M18_8232   (101)  SEEDHTEEINDKIYSLNYNELEVLAKNGETIENFVPKEGVKKADKFIVIE
GAS25_M5_Manfredo    (101)  SEEDHTEEINDKIYSLNYNELEVLAKNGETIENFVPKEGVKKADKFIVIE
      GAS25_M3_315   (101)  SEEDHTEEINDKIYSLNYNELEVLAKNGETIENFVPKEGVKKADKFIVIE
      GAS25_M3_SSI   (101)  SEEDHTEEINDKIYSLNYNELEVLAKNGETIENFVPKEGVKKADKFIVIE
    GAS25_M4_10750   (101)  SEEDHTEEINDKIYSLNYNELEVLAKNGETIENFAPKEGVKKADKFIVIE
                            151                                             200
       GAS25_SF370   (151)  RKKKNINTTPVDISIIDSVTDRTYPAALQLANKGFTENKPDAVVTKRNPQ
    GAS25_M12_2096   (151)  RKKKNINTTPVDISIIDSVTDRTYPAALQLANKGFTENKPDAVVTKRNPQ
    GAS25_M12_9429   (151)  RKKKNINTTPVDISIIDSVTDRTYPAALQLANKGFTENKPDAVVTKRNPQ
     GAS25_M1_5005   (151)  RKKKNINTTPVDISIIDSVTDRTYPAALQLANKGFTENKPDAVVTKRNPQ
     GAS25_M1_3348   (151)  RKKKNINTTPVDISIIDSVTDRTYPAALQLANKGFTENKPDAVVTKRNPQ
    GAS25_M2_10270   (151)  RKKKNINTTPVDISIIDSVTDMTYPAALQLADKGFTENKPDAVVTKRNPQ
    GAS25_M28_6180   (151)  RKKKNINTTPVDISIIDSVTDMTYPAALQLADKGFTENKPDAVVTKRNPQ
    GAS25_M6_10394   (151)  RKKKNINTTPVDISIIDSVTDRTYPAALQLANKGFTENKPDAVVTKRNPQ
    GAS25_M18_8232   (151)  RKKKNINTTPVDISIIDSVTDRTYPAALQLANKGFTENKPDAVVTKRNPQ
GAS25_M5_Manfredo    (151)  RKKKNINTTPVDISIIDSVTDRTYPAALQLANKGFTENKPDAVVTKRNPQ
      GAS25_M3_315   (151)  RKKKNINTTPVDISIIDSVTDRTYPAALQLANKGFTENKPDAVVTKRNPQ
      GAS25_M3_SSI   (151)  RKKKNINTTPVDISIIDSVTDRTYPAALQLANKGFTENKPDAVVTKRNPQ
    GAS25_M4_10750   (151)  RKKKNINTTPVDISIIDSVTDRTYPAALQLANKGFTENKPDAVVTKRNPQ
```

FIG. 33B

```
                                201                                               250
       GAS25_SF370  (201) KIHIDLPGMGDKATVEVNDPTYANVSTAIDNLVNQWHDNYSGGNTLPART
    GAS25_M12_2096  (201) KIHIDLPGMGDKATVEVNDPTYANVSTAIDNLVNQWHDNYSGGNTLPART
    GAS25_M12_9429  (201) KIHIDLPGMGDKATVEVNDPTYANVSTAIDNLVNQWHDNYSGGNTLPART
     GAS25_M1_5005  (201) KIHIDLPGMGDKATVEVNDPTYANVSTAIDNLVNQWHDNYSGGNTLPART
     GAS25_M1_3348  (201) KIHIDLPGMGDKATVEVNDPTYANVSTAIDNLVNQWHDNYSGGNTLPART
    GAS25_M2_10270  (201) KIHIDLPGMGDKATVEVNDPTYANVSTAIDNLVNQWHDNYSGGNTLPART
    GAS25_M28_6180  (201) KIHIDLPGMGDKATVEVNDPTYANVSTAIDNLVNQWHDNYSGGNTLPART
    GAS25_M6_10394  (201) KIHIDLPGMGDKATVEVNDPTYANVSTAIDNLVNQWHDNYSGGNTLPART
    GAS25_M18_8232  (201) KIHIDLPGMGDKATVEVNDPTYANVSTAIDNLVNQWHDNYSGGNTLPART
GAS25_M5_Manfredo  (201) KIHIDLPGMGDKATVEVNDPTYANVSTAIDNLVNQWHDNYSGGNTLPART
      GAS25_M3_315  (201) KIHIDLPGMGDKATVEVNDPTYANVSTAIDNLVNQWHDNYSGGNTLPART
      GAS25_M3_SSI  (201) KIHIDLPGMGDKATVEVNDPTYANVSTAIDNLVNQWHDNYSGGNTLPART
    GAS25_M4_10750  (201) KIHIDLPGMGDKATVEVNDPTYANVSTAIDNLINQWHDNYSGGNTLPART
                          251                                               300
       GAS25_SF370  (251) QYTESMVYSKSQIEAALNVNSKILDGTLGIDFKSISKGEKKVMIAAYKQI
    GAS25_M12_2096  (251) QYTESMVYSKSQIEAALNVNSKILDGTLGIDFKSISKGEKKVMIAAYKQI
    GAS25_M12_9429  (251) QYTESMVYSKSQIEAALNVNSKILDGTLGIDFKSISKGEKKVMIAAYKQI
     GAS25_M1_5005  (251) QYTESMVYSKSQIEAALNVNSKILDGTLGIDFKSISKGEKKVMIAAYKQI
     GAS25_M1_3348  (251) QYTESMVYSKSQIEAALNVNSKILDGTLGIDFKSISKGEKKVMIAAYKQI
    GAS25_M2_10270  (251) QYTESMVYSKSQIEAALNVNSKILDGTLGIDFKSISKGEKKVMIAAYKQI
    GAS25_M28_6180  (251) QYTESMVYSKSQIEAALNVNSKILDGTLGIDFKSISKGEKKVMIAAYKQI
    GAS25_M6_10394  (251) QYTESMVYSKSQIEAALNVNSKILDGTLGIDFKSISKGEKKVMIAAYKQI
    GAS25_M18_8232  (251) QYTESMVYSKSQIEAALNVNSKILDGTLGIDFKSISKGEKKVMIAAYKQI
GAS25_M5_Manfredo  (251) QYTESMVYSKSQIEAALNVNSKILDGTLGIDFKSISKGEKKVMIAAYKQI
      GAS25_M3_315  (251) QYTESMVYSKSQIEAALNVNSKILDGTLGIDFKSISKGEKKVMIAAYKQI
      GAS25_M3_SSI  (251) QYTESMVYSKSQIEAALNVNSKILDGTLGIDFKSISKGEKKVMIAAYKQI
    GAS25_M4_10750  (251) QYTESMVYSKSQIEAALNVNSKILDGTLGIDFKSISKGEKKVMIAAYKQI
                          301                                               350
       GAS25_SF370  (301) FYTVSANLPNNPADVFDKSVTFKELQRKGVSNEAPPLFVSNVAYGRTVFV
    GAS25_M12_2096  (301) FYTVSANLPNNPADVFDKSVTFKELQRKGVSNEAPPLFVSNVAYGRTVFV
    GAS25_M12_9429  (301) FYTVSANLPNNPADVFDKSVTFKELQRKGVSNEAPPLFVSNVAYGRTVFV
     GAS25_M1_5005  (301) FYTVSANLPNNPADVFDKSVTFKELQRKGVSNEAPPLFVSNVAYGRTVFV
     GAS25_M1_3348  (301) FYTVSANLPNNPADVFDKSVTFKELQRKGVSNEAPPLFVSNVAYGRTVFV
    GAS25_M2_10270  (301) FYTVSANLPNNPADVFDKSVTFKELQRKGVSNEAPPLFVSNVAYGRTVFV
    GAS25_M28_6180  (301) FYTVSANLPNNPADVFDKSVTFKELQRKGVSNEAPPLFVSNVAYGRTVFV
    GAS25_M6_10394  (301) FYTVSANLPNNPADVFDKSVTFKDLQRKGVSNEAPPLFVSNVAYGRTVFV
    GAS25_M18_8232  (301) FYTVSANLPNNPADVFDKSVTFKELQRKGVSNEAPPLFVSNVAYGRTVFV
GAS25_M5_Manfredo  (301) FYTVSANLPNNPADVFDKSVTFKDLQRKGVSNEAPPLFVSNVAYGRTVFV
      GAS25_M3_315  (301) FYTVSANLPNNPADVFDKSVTFKELQRKGVSNEAPPLFVSNVAYGRTVFV
      GAS25_M3_SSI  (301) FYTVSANLPNNPADVFDKSVTFKELQRKGVSNEAPPLFVSNVAYGRTVFV
    GAS25_M4_10750  (301) FYTVSANLPNNPADVFAKSVTFKELQRKGVSNEAPPLFVSNVAYGRTVFV
                          351                                               400
       GAS25_SF370  (351) KLETSSKSNDVEAAFSAALKGTDVKTNGKYSDILENSSFTAVVLGGDAAE
    GAS25_M12_2096  (351) KLETSSKSNDVEAAFSAALKGTDVKTNGKYSDILENSSFTAVVLGGDAAE
    GAS25_M12_9429  (351) KLETSSKSNDVEAAFSAALKGTDVKTNGKYSDILENSSFTAVVLGGDAAE
     GAS25_M1_5005  (351) KLETSSKSNDVEAAFSAALKGTDVKTNGKYSDILENSSFTAVVLGGDAAE
     GAS25_M1_3348  (351) KLETSSKSNDVEAAFSAALKGTDVKTNGKYSDILENSSFTAVVLGGDAAE
    GAS25_M2_10270  (351) KLETSSKSNDVEAAFSAALKGTDVKTNGKYSDILENSSFTAVVLGGDAAE
    GAS25_M28_6180  (351) KLETSSKSNDVEAAFSAALKGTDVKTNGKYSDILENSSFTAVVLGGDAAE
    GAS25_M6_10394  (351) KLETSSKSNDVEAAFSAALKGTDVKTNGKYSDILENSSFTAVVLGGDAAE
    GAS25_M18_8232  (351) KLETSSKSNDVEAAFSAALKGTDVKTNGKYSDILENSSFTAVVLGGDAAE
GAS25_M5_Manfredo  (351) KLETSSKSNDVEAAFSAALKGTDVKTNGKYSDILENSSFTAVVLGGDAAE
      GAS25_M3_315  (351) KLETSSKSNDVEAAFSAALKGTDVKTNGKYSDILENSSFTAVVLGGDAAE
      GAS25_M3_SSI  (351) KLETSSKSNDVEAAFSAALKGTDVKTNGKYSDILENSSFTAVVLGGDAAE
    GAS25_M4_10750  (351) KLETSSKSNDVEAAFSAALKGTDVKTNGKYSDILENSSFTAVVLGGDAAE
```

FIG. 33C

```
                         401                                               450
     GAS25_SF370  (401) HNKVVTKDFDVIRNVIKDNATFSRKNPAYPISYTSVFLKNNKIAGVNNRT
   GAS25_M12_2096 (401) HNKVVTKDFDVIRNVIKDNATFSRKNPAYPISYTSVFLKNNKIAGVNNRS
   GAS25_M12_9429 (401) HNKVVTKDFDVIRNVIKDNATFSRKNPAYPISYTSVFLKNNKIAGVNNRS
    GAS25_M1_5005 (401) HNKVVTKDFDVIRNVIKDNATFSRKNPAYPISYTSVFLKNNKIAGVNNRS
    GAS25_M1_3348 (401) HNKVVTKDFDVIRNVIKDNATFSRKNPAYPISYTSVFLKNNKIAGVNNRS
   GAS25_M2_10270 (401) HNKVVTKDFDVIRNVIKDNATFSRKNPAYPISYTSVFLKNNKIAGVNNRT
   GAS25_M28_6180 (401) HNKVVTKDFDVIRNVIKDNATFSRKNPAYPISYTSVFLKNNKIAGVNNRT
   GAS25_M6_10394 (401) HNKVVTKDFDVIRNVIKDNATFSRKNPAYPISYTSVFLKNNKIAGVNNRT
   GAS25_M18_8232 (401) HNKVVTKDFDVIRNVIKDNATFSRKNPAYPISYTSVFLKNNKIAGVNNRT
GAS25_M5_Manfredo (401) HNKVVTKDFDVIRNVIKDNATFSRKNPAYPISYTSVFLKNNKIAGVNNRT
     GAS25_M3_315 (401) HNKVVTKDFDVIRNVIKDNATFSRKNPAYPISYTSVFLKNNKIAGVNNRT
     GAS25_M3_SSI (401) HNKVVTKDFDVIRNVIKDNATFSRKNPAYPISYTSVFLKNNKIAGVNNRT
   GAS25_M4_10750 (401) HNKVVTKDFDVIRNVIKDNATFSRKNPAYPISYTSVFLKNNKIAGVNNRT
                        451                                                500
     GAS25_SF370  (451) EYVETTSTEYTSGKINLSHQGAYVAQYEILWDEINYDDKGKEVITKRRWD
   GAS25_M12_2096 (451) EYVETTSTEYTSGKINLSHQGAYVAQYEILWDEINYDDKGKEVITKRRWD
   GAS25_M12_9429 (451) EYVETTSTEYTSGKINLSHQGAYVAQYEILWDEINYDDKGKEVITKRRWD
    GAS25_M1_5005 (451) EYVETTSTEYTSGKINLSHQGAYVAQYEILWDEINYDDKGKEVITKRRWD
    GAS25_M1_3348 (451) EYVETTSTEYTSGKINLSHQGAYVAQYEILWDEINYDDKGKEVITKRRWD
   GAS25_M2_10270 (451) EYVETTSTEYTSGKINLSHRGAYVAQYEILWDEINYDDKGKEVITKRRWD
   GAS25_M28_6180 (451) EYVETTSTEYTSGKINLSHRGAYVAQYEILWDEINYDDKGKEVITKRRWD
   GAS25_M6_10394 (451) EYVETTSTEYTSGKINLSHQGAYVAQYEILWDEINYDDKGKEVITKRRWD
   GAS25_M18_8232 (451) EYVETTSTEYTSGKINLSHRGAYVAQYEILWDEINYDDKGKEVITKRRWD
GAS25_M5_Manfredo (451) EYVETTSTEYTSGKINLSHQGAYVAQYEILWDEINYDDKGKEVITKRRWD
     GAS25_M3_315 (451) EYVETTSTEYTSGKINLSHQGAYVAQYEILWDEINYDDKGKEVITKRRWD
     GAS25_M3_SSI (451) EYVETTSTEYTSGKINLSHQGAYVAQYEILWDEINYDDKGKEVITKRRWD
   GAS25_M4_10750 (451) EYVETTSTEYTSGKINLSHQGAYVAQYEILWDEINYDDKGKEVITKRRWD
                        501                                                550
     GAS25_SF370  (501) NNWYSKTSPFSTVIPLGANSRNIRIMARECTGLAWEWWRKVIDERDVKLS
   GAS25_M12_2096 (501) NNWYSKTSPFSTVIPLGANSRNIRIMARECTGLAWEWWRKVIDERDVKLS
   GAS25_M12_9429 (501) NNWYSKTSPFSTVIPLGANSRNIRIMARECTGLAWEWWRKVIDERDVKLS
    GAS25_M1_5005 (501) NNWYSKTSPFSTVIPLGANSRNIRIMARECTGLAWEWWRKVIDERDVKLS
    GAS25_M1_3348 (501) NNWYSKTSPFSTVIPLGANSRNIRIMARECTGLAWEWWRKVIDERDVKLS
   GAS25_M2_10270 (501) NNWYSKTSPFSTVIPLGANSRNIRIMARECTGLAWEWWRKVIDERDVKLS
   GAS25_M28_6180 (501) NNWYSKTSPFSTVIPLGANSRNIRIMARECTGLAWEWWRKVIDERDVKLS
   GAS25_M6_10394 (501) NNWYSKTSPFSTVIPLGANSRNIRIMARECTGLAWEWWRKVIDERDVKLS
   GAS25_M18_8232 (501) NNWYSKTSPFSTVIPLGANSRNIRIMARECTGLAWEWWRKVIDERDVKLS
GAS25_M5_Manfredo (501) NNWYSKTSPFSTVIPLGANSRNIRIMARECTGLAWEWWRKVIDERDVKLS
     GAS25_M3_315 (501) NNWYSKTSPFSTVIPLGANSRNIRIMARECTGLAWEWWRKVIDERDVKLS
     GAS25_M3_SSI (501) NNWYSKTSPFSTVIPLGANSRNIRIMARECTGLAWEWWRKVIDERDVKLS
   GAS25_M4_10750 (501) NNWYSKTSPFSTVIPLGANSRNIRIMARECTGLAWEWWRKVIDERDVKLS
                        551                  572
     GAS25_SF370  (551) KEINVNISGSTLSPYGSITYK-
   GAS25_M12_2096 (551) KEINVNISGSTLSPYGSITYK-
   GAS25_M12_9429 (551) KEINVNISGSTLSPYGSITYK-
    GAS25_M1_5005 (551) KEINVNISGSTLSPYGSITYK-
    GAS25_M1_3348 (551) KEINVNISGSTLSPYGSITYK-
   GAS25_M2_10270 (551) KEINVNISGSTLSPYGSITYK-
   GAS25_M28_6180 (551) KEINVNISGSTLSPYGSITYK-
   GAS25_M6_10394 (551) KEINVNISGSTLSPYGSITYK-
   GAS25_M18_8232 (551) KEINVNISGSTLSPYGSITYK-
GAS25_M5_Manfredo (551) KEINVNISGSTLSPYGSITYK-
     GAS25_M3_315 (551) KEINVNISGSTLSPYGSITYK-
     GAS25_M3_SSI (551) KEINVNISGSTLSPYGSITYK-
   GAS25_M4_10750 (551) KEINVNISGSTLSPYGSITYK-
```

FIG. 34

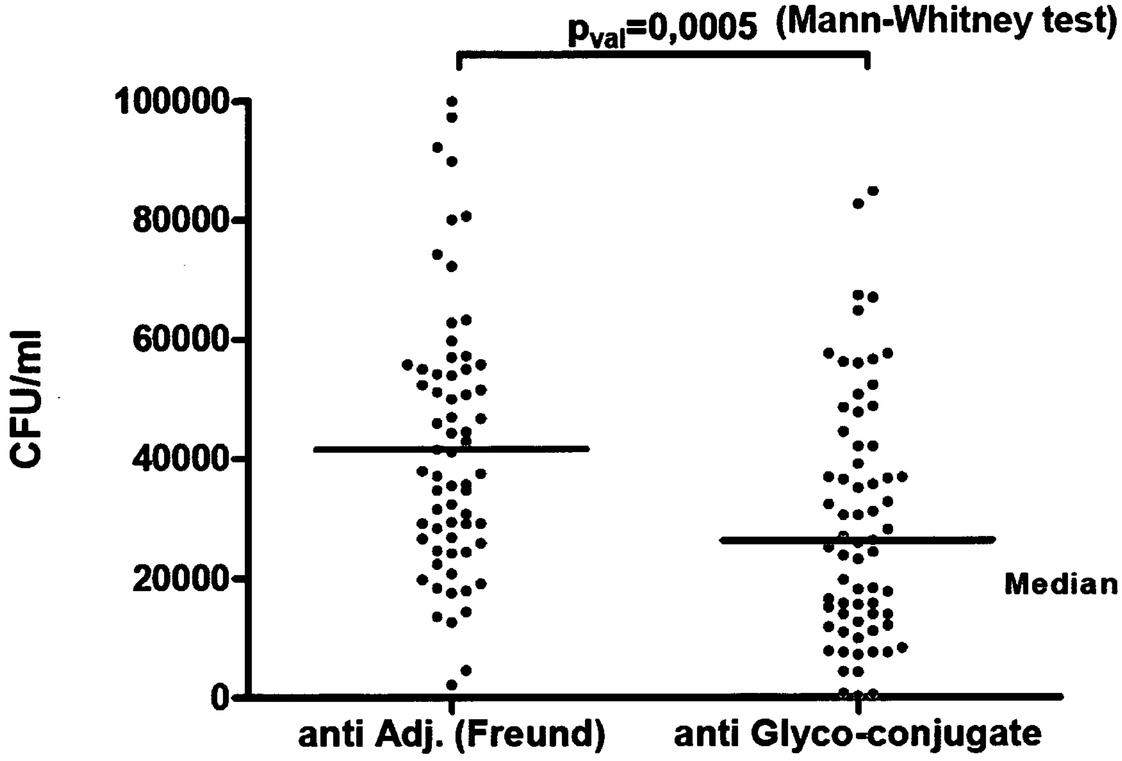

$p_{val}$=0,0005 (Mann-Whitney test)
100000
80000
60000
40000
20000
0
CFU/ml
Median
anti Adj. (Freund)
anti Glyco-conjugate

FIG. 35

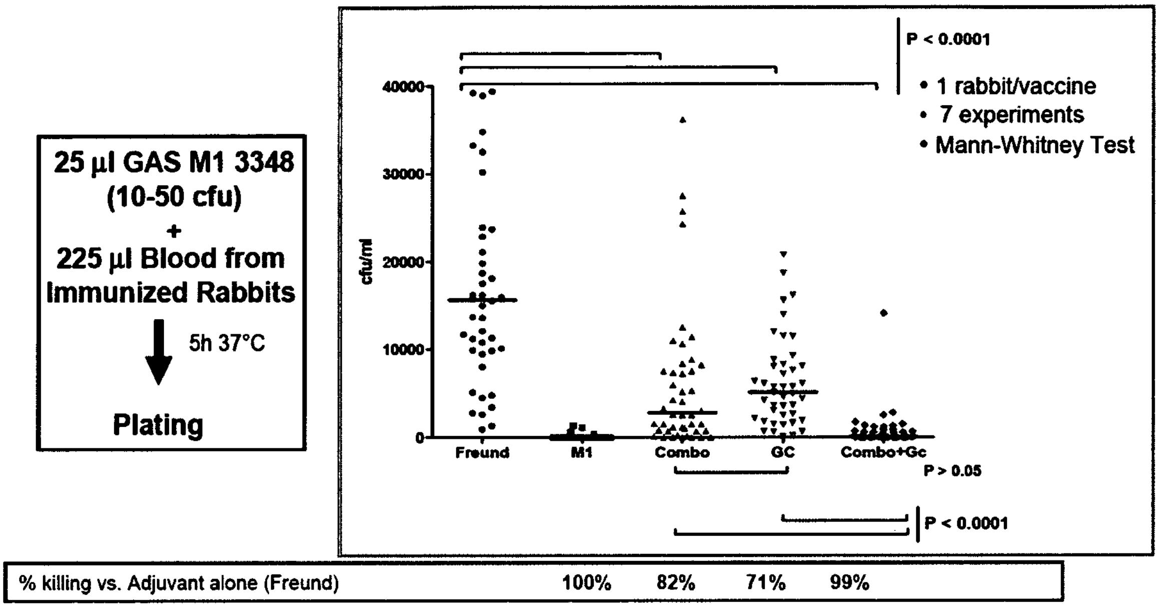
25 µl GAS M1 3348
(10-50 cfu)
+
225 µl Blood from
Immunized Rabbits
5h 37°C
Plating
P < 0.0001
• 1 rabbit/vaccine
• 7 experiments
• Mann-Whitney Test
cfu/ml
40000
30000
20000
10000
0
Freund
M1
Combo
GC
Combo+Gc
P > 0.05
P < 0.0001
% killing vs. Adjuvant alone (Freund)
100%
82%
71%
99%

FIG. 36

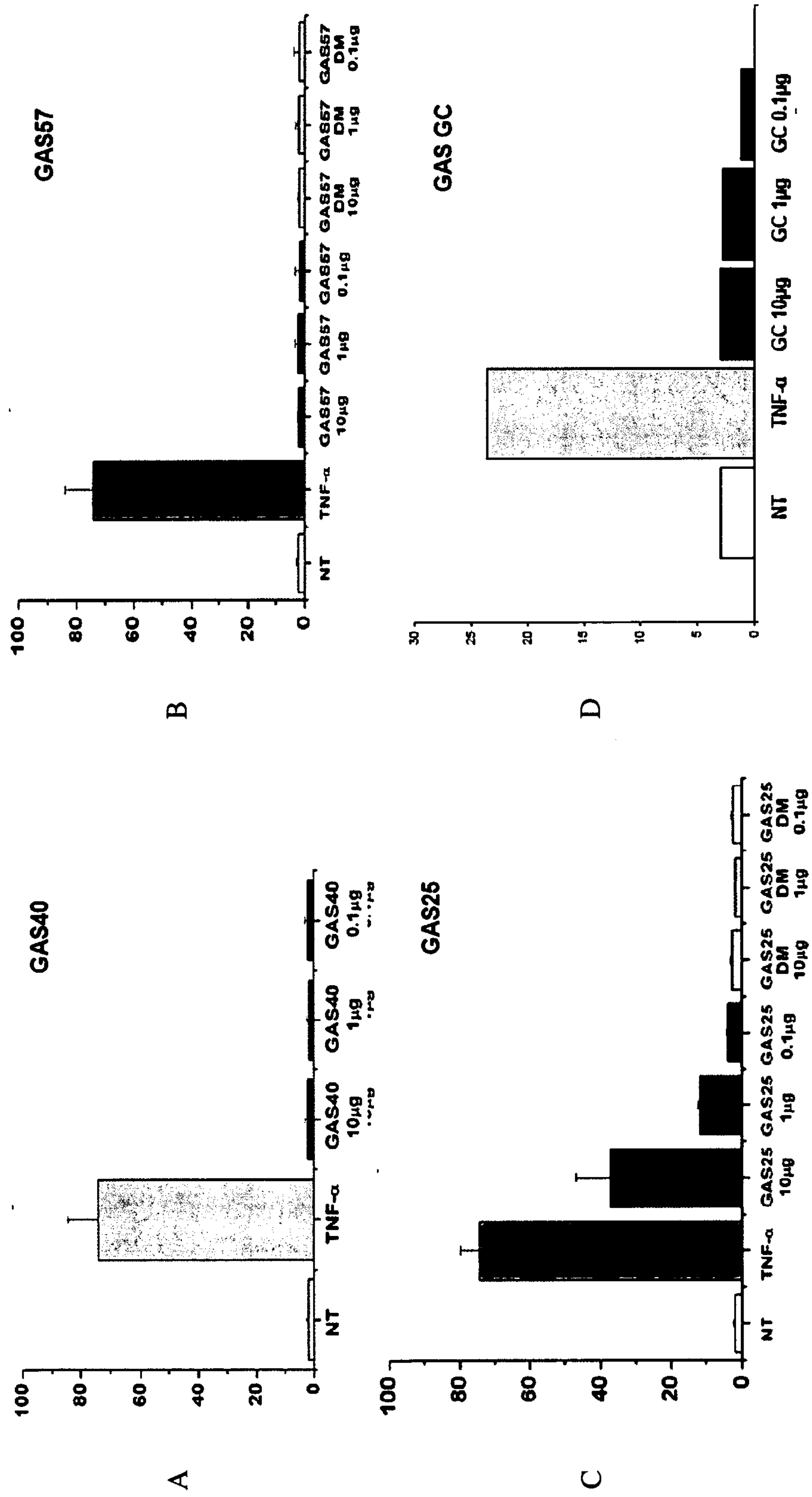
A
GAS40
100
80
60
40
20
0
NT
TNF-α
GAS40 10μg
GAS40 1μg
GAS40 0.1μg
B
GAS57
100
80
60
40
20
0
NT
TNF-α
GAS57 10μg
GAS57 1μg
GAS57 0.1μg
GAS57 DM 10μg
GAS57 DM 1μg
GAS57 DM 0.1μg
C
GAS25
100
80
60
40
20
0
NT
TNF-α
GAS25 10μg
GAS25 1μg
GAS25 0.1μg
GAS25 DM 10μg
GAS25 DM 1μg
GAS25 DM 0.1μg
D
GAS GC
30
25
20
15
10
5
0
NT
TNF-α
GC 10μg
GC 1μg
GC 0.1μg

# FIG. 37

A B C D

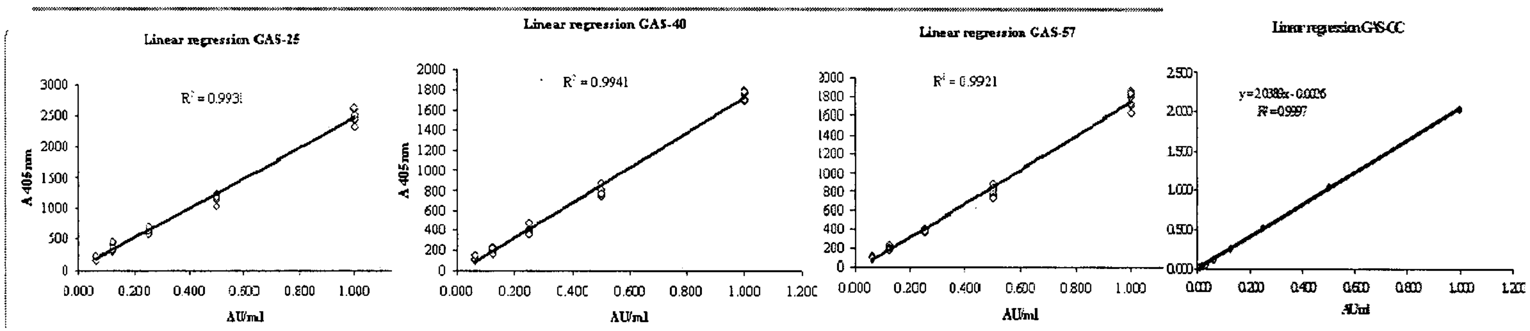


| | Acceptance criteria | GAS 25 | GAS 40 | GAS 57 |
|---|---|---|---|---|
| Linearity (r2) | $R^2$>0.98 | >0,98 | >0.98 | >0.98 |
| Repeatability | CV%<15% | 6,47 | 6,29 | 9,43 |
| Reproducibility | CV%<20% | 9,92 | 10,89 | 12,50 |
| Accuracy | 80-120% | 111,60 | 102,10 | 96,30 |

# FIG. 38

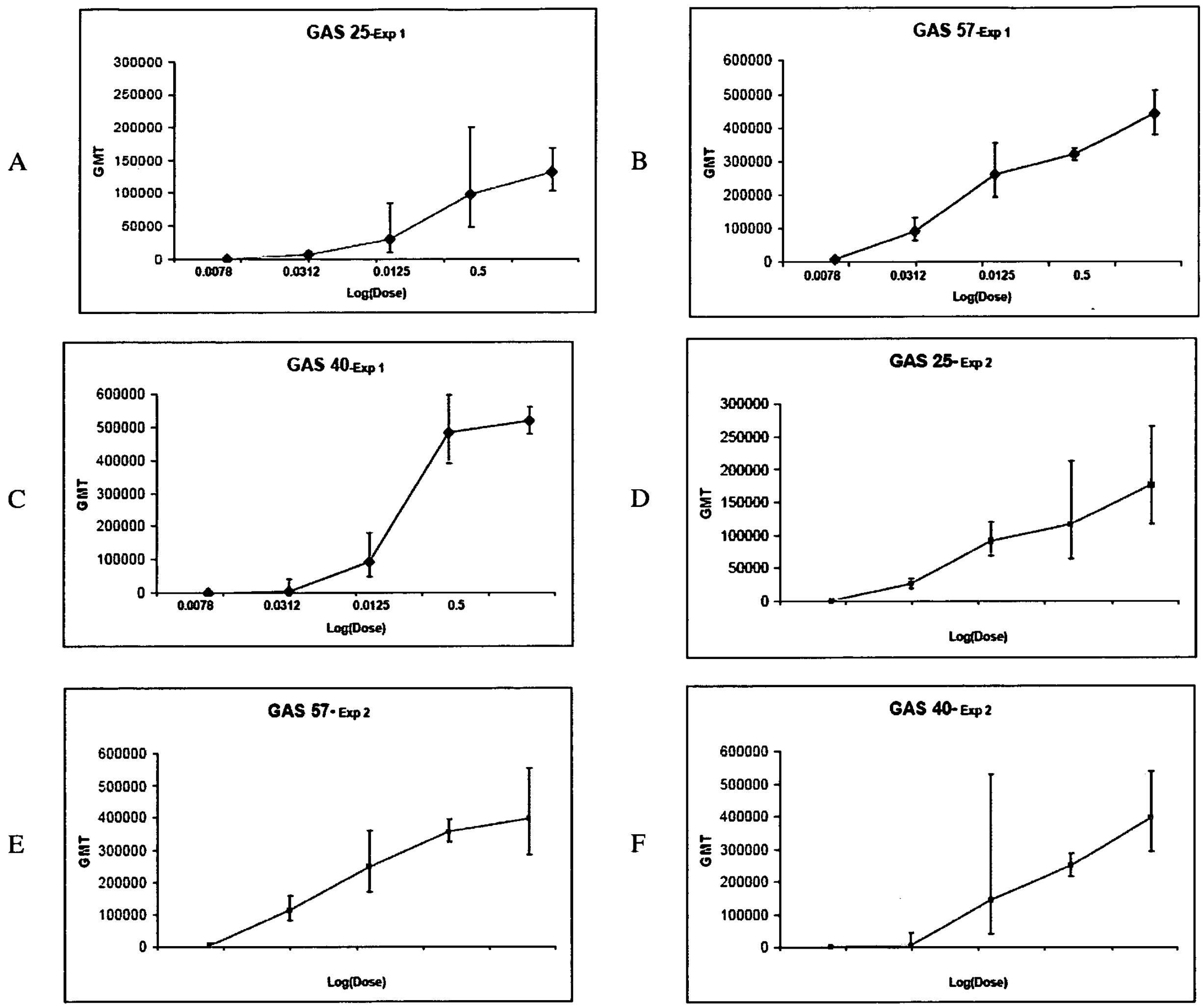
A
GAS 25-Exp 1
GMT
300000
250000
200000
150000
100000
50000
0
0.0078
0.0312
0.0125
0.5
Log(Dose)
B
GAS 57-Exp 1
GMT
600000
500000
400000
300000
200000
100000
0
0.0078
0.0312
0.0125
0.5
Log(Dose)
C
GAS 40-Exp 1
GMT
600000
500000
400000
300000
200000
100000
0
0.0078
0.0312
0.0125
0.5
Log(Dose)
D
GAS 25-Exp 2
GMT
300000
250000
200000
150000
100000
50000
0
Log(Dose)
E
GAS 57-Exp 2
GMT
600000
500000
400000
300000
200000
100000
0
Log(Dose)
F
GAS 40-Exp 2
GMT
600000
500000
400000
300000
200000
100000
0
Log(Dose)

# FIG. 39

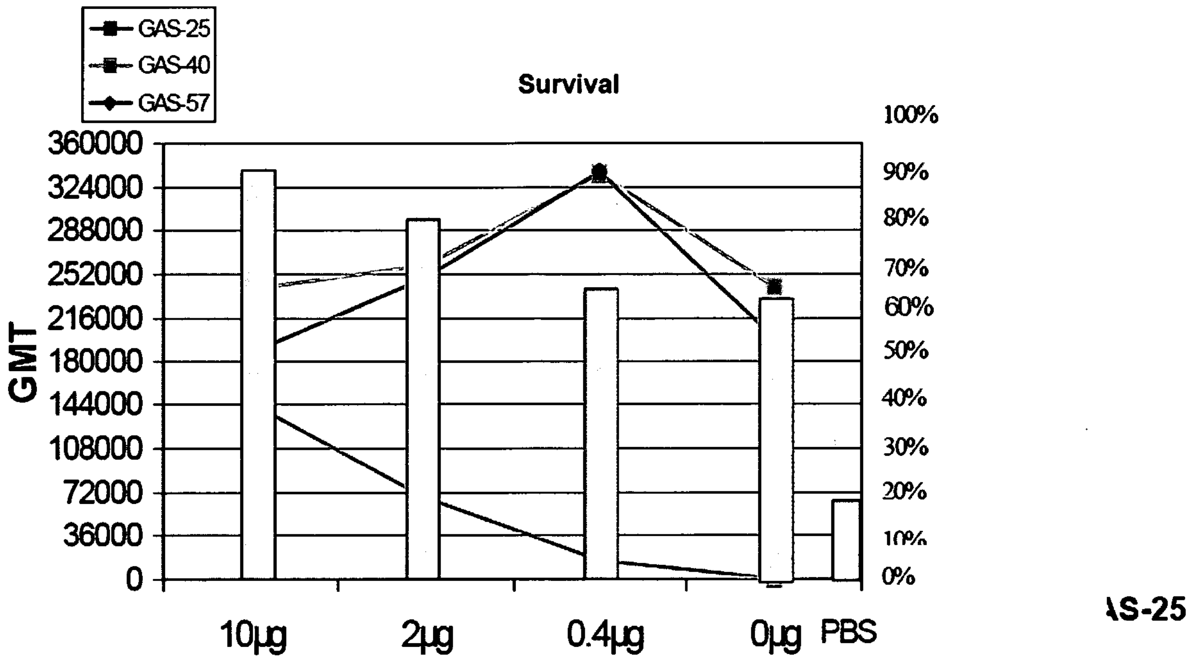
GAS-25
GAS-40
GAS-57
Survival
GMT
360000
324000
288000
252000
216000
180000
144000
108000
72000
36000
0
100%
90%
80%
70%
60%
50%
40%
30%
20%
10%
0%
10µg
2µg
0.4µg
0µg
PBS
AS-25

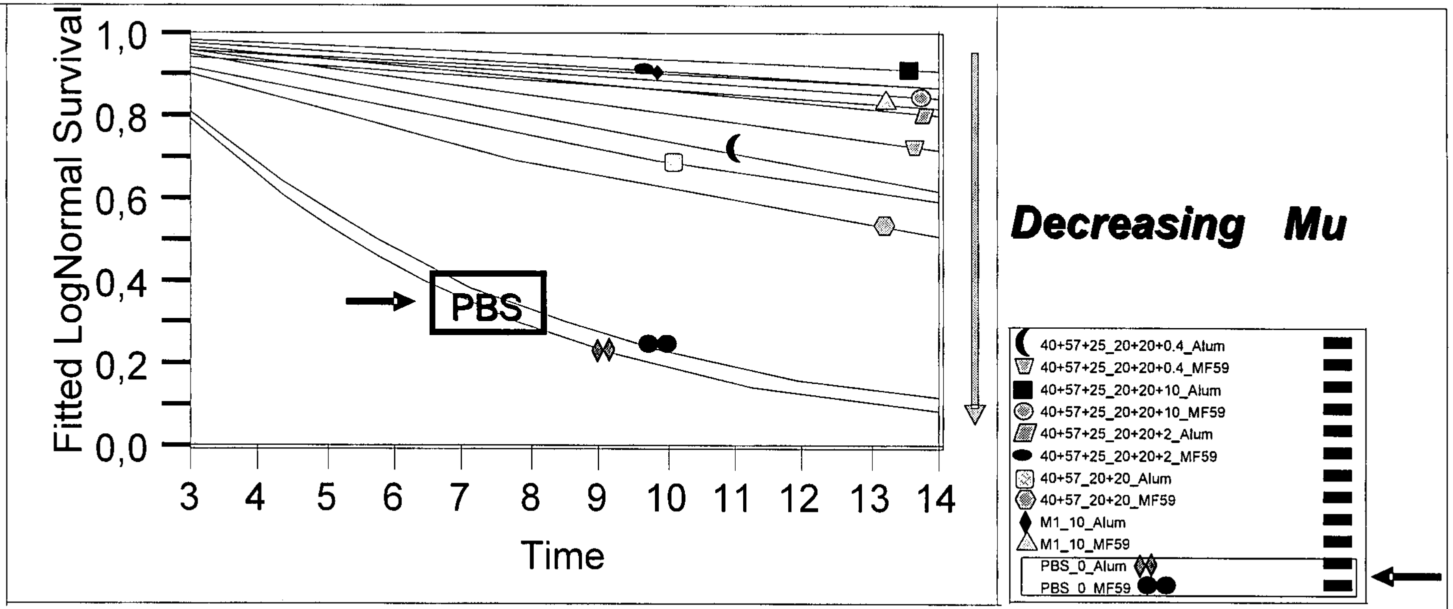


| Dose | Group | N Failed | N Censored | Total | Mu | Mu LCI95% | Mu UCI95% | Sigma | Sigma LCI95% | Sigma UCI95% |
|---|---|---|---|---|---|---|---|---|---|---|
| | M1_10_Alum | 3 | 21 | 24 | 5,2953 | 3,3794 | 14,3630 | 2,3611 | 1,0464 | 8,7008 |
| 10 | 40+57+25_20+20+10_Alum | 4 | 44 | 48 | 5,2717 | 3,6676 | 11,4000 | 1,9242 | 0,9264 | 5,7543 |
| 10 | 40+57+25_20+20+10_MF59 | 7 | 40 | 47 | 4,7429 | 3,4973 | 8,0509 | 2,0789 | 1,1796 | 4,5130 |
| | M1_10_MF59 | 4 | 20 | 24 | 4,6879 | 3,1619 | 10,3151 | 2,1983 | 1,0750 | 6,4932 |
| 2 | 40+57+25_20+20+2_MF59 | 6 | 41 | 47 | 4,6329 | 3,4654 | 8,0099 | 1,7821 | 0,9756 | 4,1528 |
| 2 | 40+57+25_20+20+2_Alum | 9 | 39 | 48 | 3,9448 | 3,1737 | 5,7058 | 1,5031 | 0,9203 | 2,8876 |
| 0.4 | 40+57+25_20+20+0.4_MF59 | 13 | 35 | 48 | 3,3800 | 2,8515 | 4,3759 | 1,2823 | 0,8519 | 2,1596 |
| 0.4 | 40+57+25_20+20+0.4_Alum | 17 | 31 | 48 | 2,9993 | 2,5938 | 3,6622 | 1,1334 | 0,7931 | 1,7644 |
| 0 | 40+57_20+20_Alum | 17 | 28 | 45 | 2,9640 | 2,4926 | 3,7190 | 1,3201 | 0,9252 | 2,0519 |
| 0 | 40+57_20+20_MF59 | 21 | 26 | 47 | 2,6861 | 2,2922 | 3,2536 | 1,2028 | 0,8746 | 1,7728 |
| | PBS_0_MF59 | 37 | 11 | 48 | 1,7581 | 1,5426 | 1,9899 | 0,7424 | 0,5910 | 0,9689 |
| | PBS_0_Alum | 39 | 9 | 48 | 1,6836 | 1,4780 | 1,9005 | 0,7101 | 0,5701 | 0,9164 |

FIG. 41

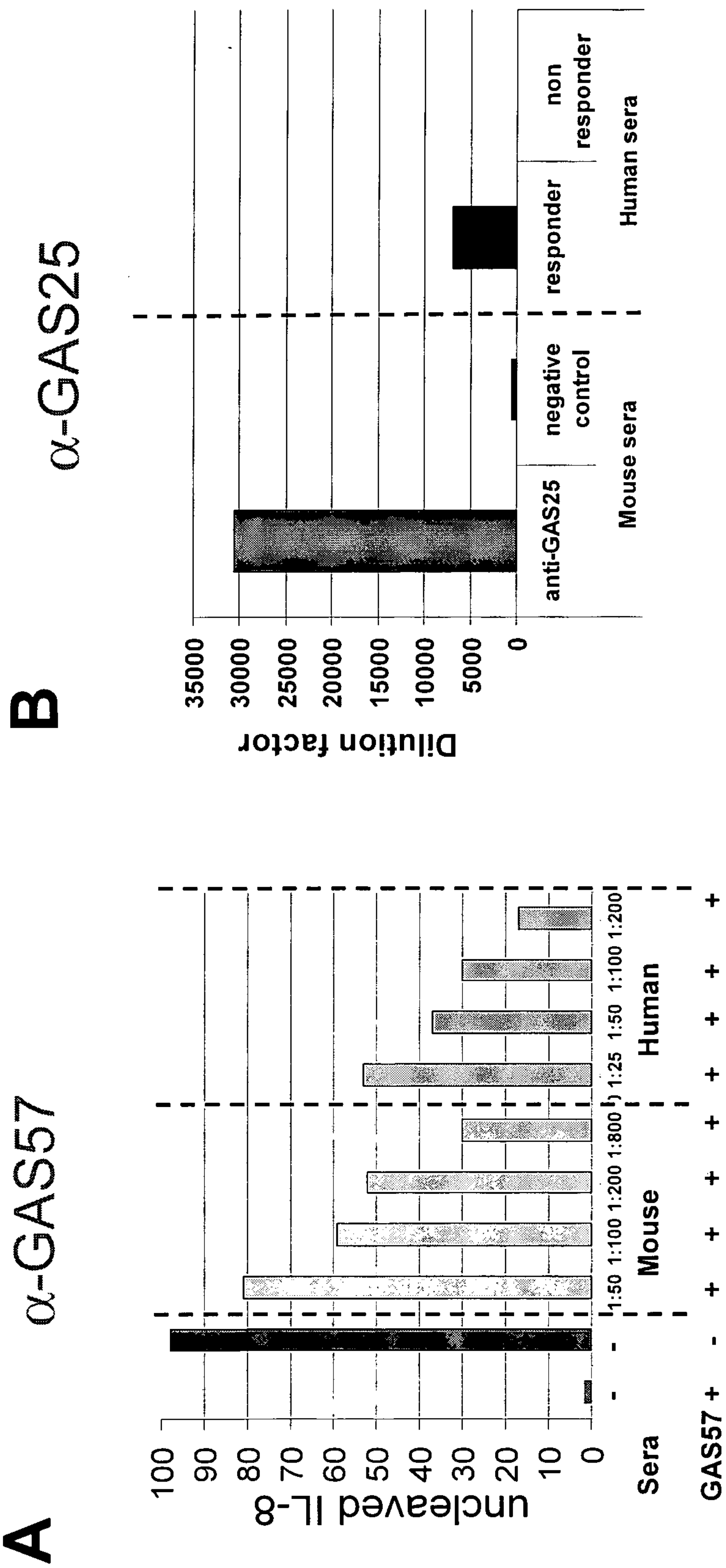
A
α-GAS57
uncleaved IL-8
100
90
80
70
60
50
40
30
20
10
0
Sera
GAS57 +
-
-
-
+
+
+
+
+
+
+
+
1:50 1:100 1:200 1:800
1:25 1:50 1:100 1:200
Mouse
Human
B
α-GAS25
Dilution factor
35000
30000
25000
20000
15000
10000
5000
0
anti-GAS25
negative control
Mouse sera
responder
non responder
Human sera

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006042027 A
- WO 2008108830 A
- WO 2005032582 A
- WO 0234771 A
- US 20060258849 A
- WO 05032582 A
- WO 0198340 A
- WO 9818931 A
- WO 9818930 A
- US 6699703 B
- US 6800744 B
- WO 9743303 A
- WO 9737026 A
- WO 02094851 A
- WO 0218595 A
- WO 9958562 A
- WO 03093306 A
- WO 04041157 A
- WO 2005002619 A
- WO 9924578 A
- WO 9936544 A
- WO 9957280 A
- WO 02079243 A
- WO 0037494 A
- WO 03049762 A
- WO 03068811 A
- WO 050026F19 A
- US 6756361 B
- WO 0202606 A
- US 5360897 A
- WO 0492360 A
- US 5378814 A
- US 6333164 B
- WO 0015255 A
- US 09581772 B
- WO 0022430 A
- WO 9629412 A
- WO 0152885 A
- GB 0016363 A
- WO IB0100166 A
- WO 9927105 A
- WO 0027994 A
- WO 9928475 A
- WO 93018150 A
- WO 9953310 A
- WO 9804702 A
- GB 0026333 A
- GB 0028727 A
- GB 0105640 A
- EP 0477508 A
- US 5306492 A
- WO 9842721 A
- EP 0372501 A
- EP 0378881 A
- EP 0427347 A
- WO 9317712 A
- WO 9858668 A
- EP 0471177 A
- WO 0056360 A
- WO 0067161 A
- WO 9403208 A
- WO 9101146 A
- WO 0061761 A
- WO 0172337 A
- WO 0023105 A
- WO 9014837 A
- US 6299884 B
- US 6451325 B
- US 5057540 A
- WO 9633739 A
- EP 0109942 A
- WO 9611711 A
- WO 0007621 A
- WO 03024480 A
- WO 03024481 A
- EP 0689454 A
- WO 0226757 A
- WO 9962923 A
- WO 9840100 A
- US 6207646 B
- US 6239116 B
- US 6429199 B
- WO 0195935 A
- WO 03035836 A
- WO 9517211 A
- WO 9842375 A
- WO 9927960 A
- US 6090406 A
- US 5916588 A
- EP 0626169 A
- WO 9952549 A
- WO 0121207 A
- WO 0121152 A
- US 4689338 A
- US 5389640 A
- US 5268376 A
- US 4929624 A

- US 5266575 A
- US 5352784 A
- US 5494916 A
- US 5482936 A
- US 5346905 A
- US 5395937 A
- US 5238944 A
- US 5525612 A
- WO 0460308 A
- WO 0464759 A
- WO 09911241 A
- WO 9400153 A
- WO 9857659 A
- EP 0835318 A
- EP 0735898 A
- EP 0761231 A
- WO 9927961 A
- WO 02074244 A
- WO 02064162 A
- WO 03028760 A
- US 61097551 B

**Non-patent literature cited in the description**


- **HENIKOFF ; HENIKOFF.** *Proc. Natl. Acad. Sci. USA,* 1992, vol. 89, 10915
- **KREIS et al.** *Int. J. Biol. Macromol.,* 1995, vol. 17, 117-30
- **CARUTHERS et al.** *Nucl. Acids Res. Symp. Ser.,* 1980, vol. 215, 223
- **HORN et al.** *Nucl. Acids Res. Symp. Ser.,* 1980, vol. 225, 232
- **HUNKAPILLER et al.** *Nature,* 1984, vol. 310, 105-11
- **GRANTHAM et al.** *Nucleic Acids Res.,* 1981, vol. 9, r43-r74
- **MERRIFIELD.** *J. Am. Chem. Soc.,* 1963, vol. 85, 2149-54
- **ROBERGE et al.** *Science,* 1995, vol. 269, 202-04
- **ROBINSON ; TORRES.** *Seminars in Immunology,* 1997, vol. 9, 271-283
- **DONNELLY et al.** *Ann. Rev Immunol,* 1997, vol. 15, 617-648
- **SCOTT-TAYLOR ; DALGLEISH.** *Expert Opin Investig Drugs,* 2000, vol. 9, 471-480
- **APOSTOLOPOULOS ; PLEBANSKI.** *Curr Opin Mol Ther,* 2000, vol. 2, 441-447
- **ILAN.** *Curr Opin Mol Ther,* 1999, vol. 1, 116-120
- **DUBENSKY et al.** *Mol Med,* 2000, vol. 6, 723-732
- **ROBINSON ; PERTMER.** *Adv Virus Res,* 2000, vol. 55, 1-74
- **DONNELLY et al.** *Am J Respir Crit Care Med,* 2000, vol. 162 (4), 190-193
- **DAVIS.** *Mt. Sinai J. Med.,* 1999, vol. 66, 84-90
- **DALE.** *Vaccine,* 1999, vol. 17, 193-200
- **DALE.** *Vaccine,* vol. 14 (10), 944-948
- *Infect Immun.,* May 2001, vol. 69 (5), 3510-3515
- **ZHU et al.** *Vaccine,* 2004, vol. 22, 660-669
- **PRICE et al.** *Infection and Immunity,* 2004, vol. 71 (1), 277-283
- **PLANTE et al.** *J. Infections Disease,* 2000, vol. 182, 848-55
- *Infect Immun.,* August 2002, vol. 70 (8), 4414
- *Infect Immun.,* January 2003, vol. 71 (1), 374-383
- *Infect Immun.,* October 1999, vol. 67 (10), 5395
- *Infect Immun.,* November 1997, vol. 65 (11), 4476-4482
- *Infect Immun.,* October 2004, vol. 72 (10), 6148
- *Proc Natl Acad Sci U S A.,* 24 August 2004, vol. 101 (34), 12652
- *Infect Immun.,* July 2004, vol. 72 (7), 3829
- *Biochim Biophys Acta,* 01 November 2004, vol. 1702 (2), 145
- *J Autoimmun.,* June 1989, vol. 2 (81
- *Infect Immun.,* October 2003, vol. 71 (10), 5498-504
- *Infect Immun.,* May 2001, vol. 69 (5), 3323-3334
- *J Clin Microbiol.,* December 1999, vol. 37 (12), 3997
- *Can J Biochem Cell Biol.,* May 1984, vol. 62 (5), 270-5
- *J Gen Virol.,* November 2004, vol. 85, 3229
- **HOUGHTON et al.** *Hepatology,* 1991, vol. 14, 381
- Vaccines. 2004
- **MURRAY et al.** Medical Microbiology. 2002
- Virology. 1988
- Fundamental Virology. 1991
- **TETTELIN et al.** *Science,* 2000, vol. 287, 1809-1815
- **PIZZA et al.** *Science,* 2000, vol. 287, 1816-1820
- **BJUNE et al.** *Lancet,* 1991, vol. 338, 8775
- **FUSKASAWA et al.** *Vaccine,* 1999, vol. 17, 2951-2958
- **ROSENQIST et al.** *Dev. Biol. Strand,* 1998, vol. 92, 323-333
- **CONSTANTINO et al.** *Vaccine,* 1992, vol. 10, 691-698
- **CONSTANTINO et al.** *Vaccine,* 1999, vol. 17, 1251-1263

- **WATSON.** *Pediatr Infect Dis J,* 2000, vol. 19, 331-332
- **RUBIN.** *Pediatr Clin North Am,* vol. 47, 269-285
- **JEDRZEJAS.** *Microbiol Mol Biol Rev,* 2001, vol. 65, 187-207
- **KALMAN et al.** *Nature Genetics,* 1999, vol. 21, 385-389
- **READ et al.** *Nucleic Acids Res,* 2000, vol. 28, 1397-406
- **SHIRAI et al.** *J. Infect. Dis,* 2000, vol. 181 (3), S524-S527
- **BELL.** *Pediatr Infect Dis J,* 2000, vol. 19, 1187-1188
- **IWARSON.** *APMIS,* 1995, vol. 103, 321-326
- **GERLICH et al.** *Vaccine,* 1990, vol. 8, 63-68, 79-80
- **HSU et al.** *Clin Liver Dis,* 1999, vol. 3, 901-915
- **GASTOFSSON et al.** *N. Engl. J. Med.,* 1996, vol. 334, 349-355
- **RAPPUOLI et al.** *TIBTECH,* 1991, vol. 9, 232-238
- Vaccines. 1988
- **DEL GUIDICE et al.** *Molecular Aspects of Medicine,* 1998, vol. 19, 1-70
- **ROSS et al.** *Vaccine,* 2001, vol. 19, 135-142
- **SUTTER et al.** *Pediatr Clin North Am,* 2000, vol. 47, 287-308
- **ZIMMERMAN ; SPANN.** *Am Fan Physician,* 1999, vol. 59, 113-118, 125-126
- **DREENSEN.** *Vaccine,* 1997, vol. 15, 2-6
- *MMWR Morb Mortal Wkly rep,* 16 January 1998, vol. 47 (1), 12, , 9
- **MCMICHAEL.** *Vaccine,* 2000, vol. 19 (1), 101-107
- **SCHUCHAT.** *Lancer,* 1999, vol. 353 (9146), 51-6
- **DALE.** *Infect Disclin North Am,* 1999, vol. 13, 227-43
- **FERRETTI et al.** *PNAS USA,* 2001, vol. 98, 4658-4663
- **KURODA et al.** *Lancet,* 2001, vol. 357 (9264), 1225-1240
- *LANCET,* 1218-1219
- **RAMSAY et al.** *Lancet,* 2001, vol. 357 (9251), 195-196
- **LINDBERG.** *Vaccine,* 1999, vol. 17 (2), 28-36
- **BUTTERY ; MOXON.** *J R Coil Physicians Long,* 2000, vol. 34, 163-168
- **AHMAD ; CHAPNICK.** *Infect Dis Clin North Am,* 1999, vol. 13, 113-133
- **GOLDBLATT.** *J. Med. Microbiol.,* 1998, vol. 47, 663-567
- Conjugate Vaccines. vol. 10, 48-114
- **HERMANSON.** *Bioconjugate Techniques,* 1996, ISBN 012323368
- **BUTTERY ; MOXON.** *J R Coll Physicians Lond,* 2000, vol. 34, 163-168
- **GOLDBLATT.** *J. Med. Microbiol.,* 1998, vol. 47, 563-567
- **HERMANSON.** *Bioconjugate Techniques,* 1996
- **GENNARO.** Remington: The Science and Practice of Pharmacy. 2000
- Vaccine Design. Plenum Press, 1995
- **PODDA.** *Vaccine,* 2001, vol. 19, 2673-2680
- **FREY et al.** *Vaccine,* 2003, vol. 21, 4234-4237
- **OTT et al.** Vaccine Design: The Subunit and Adjuvant Approach. Plenum Press, 1995, 277-296
- **BARR et al.** *Advanced Drug Delivery Reviews,* 1998, vol. 32, 247-271
- **SJOLANDER et al.** *Advanced Drug Delivery Reviews,* 1998, vol. 32, 321-338
- **NIIKURA et al.** *Virology,* 2002, vol. 293, 273-280
- **LENZ et al.** *Journal of Immunology,* 2001, 5246-5355
- **PINTO et al.** *Journal of Infectious Diseases,* 2003, vol. 188, 327-338
- **GERBER et al.** *Journal of Virology,* 2001, vol. 75 (10), 4752-4760
- **GLUCK et al.** *Vaccine,* 2002, vol. 20, B10-B16
- **MISCHLER ; METCALFE.** *Vaccine,* 2002, vol. 20 (5), 17-23
- **JOHNSON et al.** *Bioorg Med Chem Lett,* 1999, vol. 9, 2273-2278
- **MERALDI et al.** *Vaccine,* 2003, vol. 21, 2485-2491
- **PAJAK et al.** *Vaccine,* 2003, vol. 21, 836-842
- **KANDIMALLA et al.** *Nucleic Acids Research,* 2003, vol. 31 (9), 2393-2400
- **KRIEG.** *Nature Medicine,* 2003, vol. 9 (7), 831-835
- **MCCLUSKIE et al.** *FEMS Immunology and Medical Microbiology,* 2002, vol. 32, 179-185
- **KANDIMALLA et al.** *Biochemical Society Transactions,* 2003, vol. 31, 654-658
- **BLACKWELL et al.** *J. Immunol.,* 2003, vol. 170 (8), 4061-4068
- **KRIEG.** *TRENDS in Immunology,* 2002, vol. 23 (2), 64-65
- **KANDIMALLA et al.** *BBRC,* 2003, vol. 306, 948-953
- **KANDIMALLA et al.** *Biochemical Society Transactions,* 2003, vol. 31, 664-658
- **BHAGAT et al.** *BBRC,* 2003, vol. 300, 853-861
- **BEIGNON et al.** *Infection and Immunity,* 2002, vol. 70 (6), 3012-3019
- **PIZZA et al.** *Vaccine,* 2001, vol. 19, 2534-2541
- **PIZZA et al.** *Int. J. Med. Microbiol,* 2000, vol. 290 (4-5), 455-461

- **SCHARTON-KERSTEN et al.** *Infection and Immunity,* 2000, vol. 68 (9), 5306-5313
- **RYAN et al.** *Infection and Immunity,* 1999, vol. 67 (12), 6270-6280
- **PARTIDOS et al.** *Immunol. Lett.,* 1999, vol. 67 (3), 209-216
- **PEPPOLONI et al.** *Vaccines,* 2003, vol. 2 (2), 285-293
- **PINE et al.** *J. Control Release,* 2002, vol. 85 (1-3), 263-270
- **DOMENIGHINI et al.** *Mol. Microbiol,* 1995, vol. 15 (6), 1165-1167
- **SINGH et al.** *J. Cont. Rele.,* 2001, vol. 70, 267-276
- **ANDRIANOV et al.** Preparation of hydrogel microspheres by coacervation of aqueous polyphophazene solutions. *Biomaterials,* 1998, vol. 19 (1-3), 109-115
- **PAYNE et al.** Protein Release from Polyphosphazene Matrices. *Adv. Drug. Delivery Review,* 1998, vol. 31 (3), 185-196
- **STANLEY.** *Clin Exp Dermatol,* 2002, vol. 27 (7), 571-577
- **JONES.** *Curr Opin Investig Drugs,* 2003, vol. 4 (2), 214-218

SEQUENCE LISTING

<110> Novartis AG

<120> COMBINATION GAS VACCINES AND THERAPEUTICS

<130> P053514WO

<140>
<141>

<150> US 61/097,551
<151> 2008-09-17

<160> 198

<170> FastSEQ for Windows Version 4.0

<210> 1
<211> 1647
<212> PRT
<213> Streptococcus pyogenes

<400> 1

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Lys Ser Gln Asp Thr Ser Gln Ile Thr Leu Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
```

```
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Ile
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser His Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
```

```
            770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly His Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Val Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
```

Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Asp Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Ile Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Asp Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Ser Thr Thr Ser Ala Thr Ala Lys Ala Leu Pro Ser
1605 1610 1615
Thr Gly Glu Lys Met Gly Leu Lys Leu Arg Ile Val Gly Leu Val Leu
1620 1625 1630
Leu Gly Leu Thr Cys Val Phe Ser Arg Lys Lys Ser Thr Lys Asp
1635 1640 1645

<210> 2
<211> 1647
<212> PRT
<213> Streptococcus pyogenes

<400> 2

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
```

```
Ala Gln Gln Gln Ala Gln His Leu Thr Asn Thr Glu Leu Ser Ser Ala
    50                  55                  60
Glu Ser Lys Ser Gln Asp Thr Ser Gln Ile Thr Leu Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Ile
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
```

```
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser His Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly His Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
```

Gln Ala Thr Phe Asp Pro Glu Thr Asn Arg Phe Lys Pro Glu Pro Leu
1025 1030 1035 1040
Lys Asp Arg Gly Leu Ala Gly Val Arg Lys Asp Ser Val Phe Tyr Leu
1045 1050 1055
Glu Arg Lys Asp Asn Lys Pro Tyr Thr Val Thr Ile Asn Asp Ser Tyr
1060 1065 1070
Lys Tyr Val Ser Val Glu Asp Asn Lys Thr Phe Val Glu Arg Gln Ala
1075 1080 1085
Asp Gly Ser Phe Ile Leu Pro Leu Asp Lys Ala Lys Leu Gly Asp Phe
1090 1095 1100
Tyr Tyr Met Val Glu Asp Phe Ala Gly Asn Val Ala Ile Ala Lys Leu
1105 1110 1115 1120
Gly Asp His Leu Pro Gln Thr Leu Gly Lys Thr Pro Ile Lys Leu Lys
1125 1130 1135
Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Val Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Ile Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Asp Gln Leu Ile Pro

```
1505                1510                1515                1520
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Ser Thr Thr Ser Ala Thr Ala Lys Ala Leu Pro Ser
                1605                1610                1615
Thr Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 3
<211> 1647
<212> PRT
<213> Streptococcus pyogenes

<400> 3

Val Glu Lys Lys Gln Arg Phe Ser Leu Arg Lys Tyr Lys Ser Gly Thr
1               5                   10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Lys Ser Gln Asp Thr Ser Gln Ile Thr Leu Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile

```
    290                 295                 300
Ala Pro Glu Ala Gln Val Met Phe Met Arg Val Phe Ala Asn Asp Ile
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser His Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
```

Gln Met Pro Asn Gly Tyr Tyr Leu Glu Gly Phe Val Arg Phe Arg Asp
785 790 795 800
Ser Gln Asp Asp Gln Leu Asn Arg Val Asn Ile Pro Phe Val Gly Phe
805 810 815
Lys Gly Gln Phe Glu Asn Leu Ala Val Ala Glu Glu Ser Ile Tyr Arg
820 825 830
Leu Lys Ser Gln Gly Lys Thr Gly Phe Tyr Phe Asp Glu Ser Gly Pro
835 840 845
Lys Asp Asp Ile Tyr Val Gly Lys His Phe Thr Gly Leu Val Thr Leu
850 855 860
Gly Ser Glu Thr Asn Val Ser Thr Lys Thr Ile Ser Asp Asn Gly Leu
865 870 875 880
His Thr Leu Gly Thr Phe Lys Asn Ala Asp Gly Lys Phe Ile Leu Glu
885 890 895
Lys Asn Ala Gln Gly Asn Pro Val Leu Ala Ile Ser Pro Asn Gly Asp
900 905 910
Asn Asn Gln Asp Phe Ala Ala Phe Lys Gly Val Phe Leu Arg Lys Tyr
915 920 925
Gln Gly Leu Lys Ala Ser Val Tyr His Ala Ser Asp Lys Glu His Lys
930 935 940
Asn Pro Leu Trp Val Ser Pro Glu Ser Phe Lys Gly Asp Lys Asn Phe
945 950 955 960
Asn Ser Asp Ile Arg Phe Ala Lys Ser Thr Thr Leu Leu Gly Thr Ala
965 970 975
Phe Ser Gly Lys Ser Leu Thr Gly Ala Glu Leu Pro Asp Gly His Tyr
980 985 990
His Tyr Val Val Ser Tyr Tyr Pro Asp Val Val Gly Ala Lys Arg Gln
995 1000 1005
Glu Met Thr Phe Asp Met Ile Leu Asp Arg Gln Lys Pro Val Leu Ser
1010 1015 1020
Gln Ala Thr Phe Asp Pro Glu Thr Asn Arg Phe Lys Pro Glu Pro Leu
1025 1030 1035 1040
Lys Asp Arg Gly Leu Ala Gly Val Arg Lys Asp Ser Ala Phe Tyr Leu
1045 1050 1055
Glu Arg Lys Asp Asn Lys Pro Tyr Thr Val Thr Ile Asn Asp Ser Tyr
1060 1065 1070
Lys Tyr Val Ser Val Glu Asp Asn Lys Thr Phe Val Glu Arg Gln Ala
1075 1080 1085
Asp Gly Ser Phe Ile Leu Pro Leu Asp Lys Ala Lys Leu Gly Asp Phe
1090 1095 1100
Tyr Tyr Met Val Glu Asp Phe Ala Gly Asn Val Ala Ile Ala Lys Leu
1105 1110 1115 1120
Gly Asp His Leu Pro Gln Thr Leu Gly Lys Thr Pro Ile Lys Leu Lys
1125 1130 1135
Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Val Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr

1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Ile Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Asp Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Ser Thr Thr Ser Ala Thr Ala Lys Ala Leu Pro Ser
1605 1610 1615
Thr Gly Glu Lys Met Gly Leu Lys Leu Arg Ile Val Gly Leu Val Leu
1620 1625 1630
Leu Gly Leu Thr Cys Val Phe Ser Arg Lys Lys Ser Thr Lys Asp
1635 1640 1645

<210> 4
<211> 1647
<212> PRT
<213> Streptococcus pyogenes

<400> 4

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
```

```
    50                  55                  60
Glu Ser Lys Ser Gln Asp Thr Ser Gln Ile Thr Leu Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Ile
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
```

```
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser His Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly His Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
```

```
1025                1030                1035                1040
Lys Asp Arg Gly Leu Ala Gly Val Arg Lys Asp Ser Ala Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Val Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Ile Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Asp Gln Leu Ile Pro
1505                1510                1515                1520
```

```
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Ser Thr Thr Ser Ala Thr Ala Lys Ala Leu Pro Ser
                1605                1610                1615
Thr Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 5
<211> 1647
<212> PRT
<213> Streptococcus pyogenes

<400> 5

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Lys Ser Gln Asp Thr Ser Gln Ile Thr Leu Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
```

```
Ala Pro Glu Ala Gln Val Met Phe Met Arg Val Phe Ala Asn Asp Ile
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser His Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
```

```
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly His Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Ala Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Val Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
```

Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Ile Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Asp Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Ser Thr Thr Ser Ala Thr Ala Lys Ala Leu Pro Ser
1605 1610 1615
Thr Gly Glu Lys Met Gly Leu Lys Leu Arg Ile Val Gly Leu Val Leu
1620 1625 1630
Leu Gly Leu Thr Cys Val Phe Ser Arg Lys Lys Ser Thr Lys Asp
1635 1640 1645

<210> 6
<211> 1647
<212> PRT
<213> Streptococcus pyogenes

<400> 6

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Val Met Thr Thr Thr Val
            20                  25                  30
Ala Ala Asn Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
```

```
Glu Ser Lys Pro Gln Asp Thr Ser Gln Ile Thr Pro Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Pro Met Ala Asp Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Asp Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
```

```
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Lys Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
```

```
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
```

```
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Thr Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 7
<211> 1647
<212> PRT
<213> Streptococcus pyogenes

<400> 7

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Lys Ser Gln Asp Thr Ser Gln Ile Thr Pro Lys Thr Asn Arg
65                  70                  75                  80
Glu Lys Glu Gln Ser Gln Asp Leu Val Ser Glu Pro Thr Thr Thr Glu
                85                  90                  95
Leu Ala Asp Thr Asp Ala Ala Ser Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Asp Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
```

```
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Asp Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Val Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
```

```
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Ile Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
```

```
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Ile Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Ala Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 8
<211> 1646
<212> PRT
<213> Streptococcus pyogenes

<400> 8

```
Val Glu Lys Lys Gln Arg Phe Ser Leu Arg Lys Tyr Lys Ser Gly Thr
 1              5                  10                  15
Phe Ser Val Leu Ile Gly Ser Ala Phe Leu Met Met Thr Thr Thr Val
            20                  25                  30
Ala Ala Asp Glu Leu Ser Thr Met Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Ser Gln Asp Thr Ser Gln Ile Thr Pro Lys Thr Asn Arg
```

```
65                  70                  75                  80
Glu Lys Glu Gln Ser Gln Asp Leu Val Ser Glu Pro Thr Thr Glu Leu
                85                  90                  95
Ala Asp Thr Asp Ala Ala Pro Met Ala Asn Thr Gly Pro Asp Ala Thr
            100                 105                 110
Gln Lys Ser Ala Ser Leu Pro Pro Val Asn Thr Asp Val His Asp Trp
        115                 120                 125
Val Lys Thr Lys Gly Ala Trp Asp Lys Gly Tyr Lys Gly Gln Gly Lys
    130                 135                 140
Val Val Ala Val Ile Asp Thr Gly Ile Asp Pro Ala His Gln Ser Met
145                 150                 155                 160
Arg Ile Ser Asp Val Ser Thr Ala Lys Val Lys Ser Lys Glu Asp Met
                165                 170                 175
Leu Ala Arg Gln Lys Ala Ala Gly Ile Asn Tyr Gly Ser Trp Ile Asn
            180                 185                 190
Asp Lys Val Val Phe Ala His Asn Tyr Val Glu Asn Ser Asp Asn Ile
        195                 200                 205
Lys Glu Asn Gln Phe Glu Asp Phe Asp Glu Asp Trp Glu Asn Phe Glu
    210                 215                 220
Phe Asp Ala Asp Ala Glu Pro Lys Ala Ile Lys Lys His Lys Ile Tyr
225                 230                 235                 240
Arg Pro Gln Ser Thr Gln Ala Pro Lys Glu Thr Val Ile Lys Thr Glu
                245                 250                 255
Glu Thr Asp Gly Ser His Asp Ile Asp Trp Thr Gln Thr Asp Asp Asp
            260                 265                 270
Thr Lys Tyr Glu Ser His Gly Met His Val Thr Gly Ile Val Ala Gly
        275                 280                 285
Asn Ser Lys Glu Ala Ala Ala Thr Gly Glu Arg Phe Leu Gly Ile Ala
    290                 295                 300
Pro Glu Ala Gln Val Met Phe Met Arg Val Phe Ala Asn Asp Val Met
305                 310                 315                 320
Gly Ser Ala Glu Ser Leu Phe Ile Lys Ala Ile Glu Asp Ala Val Ala
                325                 330                 335
Leu Gly Ala Asp Val Ile Asn Leu Ser Leu Gly Thr Ala Asn Gly Ala
            340                 345                 350
Gln Leu Ser Gly Ser Lys Pro Leu Met Glu Ala Ile Glu Lys Ala Lys
        355                 360                 365
Lys Ala Gly Val Ser Val Val Val Ala Ala Gly Asn Glu Arg Val Tyr
    370                 375                 380
Gly Ser Asp His Asp Asp Pro Leu Ala Thr Asn Pro Asp Tyr Gly Leu
385                 390                 395                 400
Val Gly Ser Pro Ser Thr Gly Arg Thr Pro Thr Ser Val Ala Ala Ile
                405                 410                 415
Asn Ser Lys Trp Val Ile Gln Arg Leu Met Thr Val Lys Glu Leu Glu
            420                 425                 430
Asn Arg Ala Asp Leu Asn His Gly Lys Ala Ile Tyr Ser Glu Ser Val
        435                 440                 445
Asp Phe Lys Asn Ile Lys Asp Ser Leu Gly Tyr Asp Lys Ser His Gln
    450                 455                 460
Phe Ala Tyr Val Lys Glu Ser Thr Asp Ala Gly Tyr Lys Ala Gln Asp
465                 470                 475                 480
Val Lys Asp Lys Ile Ala Leu Ile Glu Arg Asp Pro Asn Lys Thr Tyr
                485                 490                 495
Asp Glu Met Ile Ala Leu Ala Lys Lys His Gly Ala Leu Gly Val Leu
            500                 505                 510
Ile Phe Asn Asn Lys Pro Gly Gln Ser Asn Arg Ser Met Arg Leu Thr
        515                 520                 525
Ala Asn Gly Met Gly Ile Pro Ser Ala Phe Ile Ser His Glu Phe Gly
    530                 535                 540
Lys Ala Met Ser Gln Leu Asn Gly Asn Gly Thr Gly Ser Leu Glu Phe
545                 550                 555                 560
```

Asp Ser Val Val Ser Lys Ala Pro Ser Gln Lys Gly Asn Glu Met Asn
565 570 575
His Phe Ser Asn Trp Gly Leu Thr Ser Asp Gly Tyr Leu Lys Pro Asp
580 585 590
Ile Thr Ala Pro Gly Gly Asp Ile Tyr Ser Thr Tyr Asn Asp Asn His
595 600 605
Tyr Gly Ser Gln Thr Gly Thr Ser Met Ala Ser Pro Gln Ile Ala Gly
610 615 620
Ala Ser Leu Leu Val Lys Gln Tyr Leu Glu Lys Thr Gln Pro Asn Leu
625 630 635 640
Pro Lys Glu Lys Ile Ala Asp Ile Val Lys Asn Leu Leu Met Ser Asn
645 650 655
Ala Gln Ile His Val Asn Pro Glu Thr Lys Thr Thr Thr Ser Pro Arg
660 665 670
Gln Gln Gly Ala Gly Leu Leu Asn Ile Asp Gly Ala Val Thr Ser Gly
675 680 685
Leu Tyr Val Thr Gly Lys Asp Asn Tyr Gly Ser Ile Ser Leu Gly Asn
690 695 700
Val Thr Asp Thr Met Thr Phe Asp Val Thr Val His Asn Leu Ser Asn
705 710 715 720
Lys Ala Lys Thr Leu Arg Tyr Asp Thr Glu Leu Leu Thr Asp His Val
725 730 735
Asp Pro Gln Lys Gly Arg Phe Thr Leu Thr Ser Arg Ser Leu Lys Thr
740 745 750
Tyr Gln Gly Gly Glu Val Thr Val Pro Ala Asn Gly Lys Val Thr Val
755 760 765
Arg Val Thr Met Asp Val Ser Gln Phe Thr Lys Glu Leu Thr Lys Gln
770 775 780
Met Pro Asn Gly Tyr Tyr Leu Glu Gly Phe Val Arg Phe Arg Asp Ser
785 790 795 800
Gln Asp Ala Gln Leu Asn Arg Val Asn Ile Pro Phe Val Gly Phe Lys
805 810 815
Gly Gln Phe Glu Asn Leu Ala Val Ala Glu Glu Ser Ile Tyr Arg Leu
820 825 830
Lys Ser Gln Gly Lys Thr Gly Phe Tyr Phe Asp Glu Ser Gly Pro Lys
835 840 845
Asp Asp Ile Tyr Val Gly Lys His Phe Thr Gly Leu Val Thr Leu Gly
850 855 860
Ser Glu Thr Asn Val Ser Thr Lys Thr Ile Ser Asp Asn Gly Leu His
865 870 875 880
Thr Leu Gly Thr Phe Lys Asn Ala Asp Gly Lys Phe Ile Leu Glu Lys
885 890 895
Asn Ala Gln Gly Asn Pro Val Leu Ala Ile Ser Pro Asn Gly Asp Asn
900 905 910
Asn Gln Asp Phe Ala Ala Phe Lys Gly Val Phe Leu Arg Lys Tyr Gln
915 920 925
Gly Leu Lys Ala Ser Val Tyr His Ala Ser Asp Lys Glu His Lys Asn
930 935 940
Pro Leu Trp Val Ser Pro Glu Ser Phe Lys Gly Asp Lys Asn Phe Asn
945 950 955 960
Ser Asp Ile Arg Phe Ala Lys Ser Thr Thr Leu Leu Gly Thr Ala Phe
965 970 975
Ser Gly Lys Ser Leu Thr Gly Ala Glu Leu Pro Asp Gly Tyr Tyr His
980 985 990
Tyr Val Val Ser Tyr Tyr Pro Asp Val Val Gly Ala Lys Arg Gln Glu
995 1000 1005
Met Thr Phe Asp Met Ile Leu Asp Arg Gln Lys Pro Val Leu Ser Gln
1010 1015 1020
Ala Thr Phe Asp Pro Glu Thr Asn Arg Phe Lys Pro Glu Pro Leu Lys
1025 1030 1035 1040
Asp Arg Gly Leu Ala Gly Val Arg Lys Asp Ser Val Phe Tyr Leu Glu

```
                1045                1050                1055
Arg Lys Asp Asn Lys Pro Tyr Thr Val Thr Ile Asn Asp Ser Tyr Lys
            1060                1065                1070
Tyr Val Ser Val Glu Asp Asn Lys Thr Phe Val Glu Arg Gln Ala Asp
        1075                1080                1085
Gly Ser Phe Ile Leu Pro Leu Asp Lys Ala Lys Leu Gly Asp Phe Tyr
    1090                1095                1100
Tyr Met Val Glu Asp Phe Ala Gly Asn Val Ala Ile Ala Lys Leu Gly
1105                1110                1115                1120
Asp His Leu Pro Gln Thr Leu Gly Lys Thr Pro Ile Lys Leu Lys Leu
                1125                1130                1135
Thr Asp Gly Asn Tyr Gln Thr Lys Glu Thr Leu Lys Asp Asn Leu Glu
            1140                1145                1150
Met Thr Gln Ser Asp Thr Gly Leu Val Thr Asn Gln Ala Gln Leu Ala
        1155                1160                1165
Val Val His Arg Asn Gln Pro Gln Ser Gln Leu Thr Lys Met Asn Gln
    1170                1175                1180
Asp Phe Phe Ile Ser Pro Asn Glu Asp Gly Asn Lys Asp Phe Val Ala
1185                1190                1195                1200
Phe Lys Gly Leu Lys Asn Asn Val Tyr Asn Asp Leu Thr Val Asn Val
                1205                1210                1215
Tyr Ala Lys Asp Asp His Gln Lys Gln Thr Pro Ile Trp Ser Ser Gln
            1220                1225                1230
Ala Gly Ala Ser Ala Ser Ala Ile Glu Ser Thr Ala Trp Tyr Gly Ile
        1235                1240                1245
Thr Ala Arg Gly Ser Lys Val Met Pro Gly Asp Tyr Gln Tyr Val Val
    1250                1255                1260
Thr Tyr Arg Asp Glu His Gly Lys Glu His Gln Lys Gln Tyr Thr Ile
1265                1270                1275                1280
Ser Val Asn Asp Lys Lys Pro Met Ile Thr Gln Gly Arg Phe Asp Thr
                1285                1290                1295
Ile Asn Gly Val Asp His Phe Thr Pro Asp Lys Thr Lys Ala Leu Gly
            1300                1305                1310
Ser Ser Gly Ile Val Arg Glu Glu Val Phe Tyr Leu Ala Lys Lys Asn
        1315                1320                1325
Gly Arg Lys Phe Asp Val Thr Glu Gly Lys Asp Gly Ile Thr Val Ser
    1330                1335                1340
Asp Asn Lys Val Tyr Ile Pro Lys Asn Pro Asp Gly Ser Tyr Thr Ile
1345                1350                1355                1360
Ser Lys Arg Asp Gly Val Thr Leu Ser Asp Tyr Tyr Tyr Leu Val Glu
                1365                1370                1375
Asp Arg Ala Gly Asn Val Ser Phe Ala Thr Leu Arg Asp Leu Lys Ala
            1380                1385                1390
Val Gly Lys Asp Lys Ala Val Val Asn Phe Gly Leu Asp Leu Pro Val
        1395                1400                1405
Pro Glu Asp Lys Gln Ile Val Asn Phe Thr Tyr Leu Val Arg Asp Ala
    1410                1415                1420
Asp Gly Lys Pro Ile Glu Asn Leu Glu Tyr Tyr Asn Asn Ser Gly Asn
1425                1430                1435                1440
Ser Leu Ile Leu Pro Tyr Gly Lys Tyr Thr Val Glu Leu Leu Thr Tyr
                1445                1450                1455
Asp Thr Asn Ala Ala Lys Leu Glu Ser Asp Lys Ile Val Ser Phe Thr
            1460                1465                1470
Leu Ser Ala Asp Asn Asn Phe Gln Gln Val Thr Phe Lys Met Thr Met
        1475                1480                1485
Leu Ala Thr Ser Gln Ile Thr Ala His Phe Asp His Leu Leu Pro Glu
    1490                1495                1500
Gly Ser Arg Val Ser Leu Lys Thr Ala Gln Gly Gln Leu Ile Pro Leu
1505                1510                1515                1520
Glu Gln Ser Leu Tyr Val Pro Lys Ala Tyr Gly Lys Thr Val Gln Glu
                1525                1530                1535
```

```
Gly Thr Tyr Glu Val Val Val Ser Leu Pro Lys Gly Tyr Arg Ile Glu
            1540                1545                1550
Gly Asn Thr Lys Val Asn Ala Leu Pro Asn Glu Val His Glu Leu Ser
        1555                1560                1565
Leu Arg Leu Val Lys Val Gly Asp Ala Ser Asp Ser Thr Gly Asp His
    1570                1575                1580
Lys Val Met Ser Lys Asn Asn Ser Gln Ala Leu Thr Ala Ser Ala Arg
1585                1590                1595                1600
Pro Thr Lys Thr Thr Thr Ser Ala Thr Ala Lys Ala Leu Pro Ser Ala
                1605                1610                1615
Gly Glu Lys Met Gly Leu Lys Leu Arg Ile Val Gly Leu Val Leu Leu
            1620                1625                1630
Gly Leu Thr Cys Val Phe Ser Arg Lys Lys Ser Thr Lys Glu
        1635                1640                1645
```

<210> 9
<211> 1645
<212> PRT
<213> Streptococcus pyogenes

<400> 9

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Val Met Thr Thr Thr Val
            20                  25                  30
Ala Ala Asp Glu Leu Ser Thr Met Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Lys Thr Gln Asp Thr Ser Gln Ile Thr Pro Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Ser Met Ala Asp Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys Asn Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
```

```
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asn Ile Lys Asp Ser Leu Gly Tyr Asp Lys Ser His Gln Phe
    450                 455                 460
Ala Tyr Val Lys Glu Ser Thr Asp Ala Gly Tyr Lys Ala Gln Asp Val
465                 470                 475                 480
Lys Asp Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
```

```
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly His Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Ala Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
```

```
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Thr Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 10
<211> 1646
<212> PRT
<213> Streptococcus pyogenes

<400> 10

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Ser Thr Met Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Ser Gln Asp Thr Ser Gln Ile Thr Pro Lys Thr Asn Arg
65                  70                  75                  80
```

```
Glu Lys Glu Gln Pro Gln Gly Leu Val Ser Glu Pro Thr Thr Glu Leu
                85                  90                  95
Ala Asp Thr Asp Ala Ala Pro Met Ala Asn Thr Gly Pro Asp Ala Thr
            100                 105                 110
Gln Lys Ser Ala Ser Leu Pro Pro Val Asn Thr Asp Val His Asp Trp
        115                 120                 125
Val Lys Thr Lys Gly Ala Trp Asp Lys Gly Tyr Lys Gly Gln Gly Lys
    130                 135                 140
Val Val Ala Val Ile Asp Thr Gly Ile Asp Pro Ala His Gln Ser Met
145                 150                 155                 160
Arg Ile Ser Asp Val Ser Thr Ala Lys Val Lys Ser Lys Glu Asp Met
                165                 170                 175
Leu Ala Arg Gln Lys Ala Ala Gly Ile Asn Tyr Gly Ser Trp Ile Asn
            180                 185                 190
Asp Lys Val Val Phe Ala His Asn Tyr Val Glu Asn Ser Asp Asn Ile
        195                 200                 205
Lys Glu Asn Gln Phe Gly Asp Phe Asp Glu Asp Trp Glu Asn Phe Glu
    210                 215                 220
Phe Asp Ala Glu Ala Glu Pro Lys Ala Ile Lys Lys His Lys Ile Tyr
225                 230                 235                 240
Arg Pro Gln Ser Thr Gln Ala Pro Lys Glu Thr Val Ile Lys Thr Glu
                245                 250                 255
Glu Thr Asp Gly Ser His Asp Ile Asp Trp Thr Gln Thr Asp Asp Asp
            260                 265                 270
Thr Lys Tyr Glu Ser His Gly Met His Val Thr Gly Ile Val Ala Gly
        275                 280                 285
Asn Ser Lys Glu Ala Ala Ala Thr Gly Glu Arg Phe Leu Gly Ile Ala
    290                 295                 300
Pro Glu Ala Gln Val Met Phe Met Arg Val Phe Ala Asn Asp Val Met
305                 310                 315                 320
Gly Ser Ala Glu Ser Leu Phe Ile Lys Ala Ile Glu Asp Ala Val Ala
                325                 330                 335
Leu Gly Ala Asp Val Ile Asn Leu Ser Leu Gly Thr Ala Asn Gly Ala
            340                 345                 350
Gln Leu Ser Gly Ser Lys Pro Leu Met Glu Ala Ile Glu Lys Ala Lys
        355                 360                 365
Lys Ala Gly Val Ser Val Val Val Ala Ala Gly Asn Glu Arg Val Tyr
    370                 375                 380
Gly Ser Asp His Asp Asp Pro Leu Ala Thr Asn Pro Asp Tyr Gly Leu
385                 390                 395                 400
Val Gly Ser Pro Ser Thr Gly Arg Thr Pro Thr Ser Val Ala Ala Ile
                405                 410                 415
Asn Ser Lys Trp Val Ile Gln Arg Leu Met Thr Val Lys Glu Leu Glu
            420                 425                 430
Asn Arg Ala Asp Leu Asn His Gly Lys Ala Ile Tyr Ser Glu Ser Val
        435                 440                 445
Asp Phe Lys Asn Ile Lys Asp Ser Leu Gly Tyr Asp Lys Ser His Gln
    450                 455                 460
Phe Ala Tyr Val Lys Glu Ser Thr Asp Ala Gly Tyr Lys Ala Gln Asp
465                 470                 475                 480
Val Lys Gly Lys Ile Ala Leu Ile Glu Arg Asp Pro Asn Lys Thr Tyr
                485                 490                 495
Asp Glu Met Ile Ala Leu Ala Lys Lys His Gly Ala Leu Gly Val Leu
            500                 505                 510
Ile Phe Asn Asn Lys Pro Gly Gln Ser Asn Arg Ser Met Arg Leu Thr
        515                 520                 525
Ala Asn Gly Met Gly Ile Pro Ser Ala Phe Ile Ser His Glu Phe Gly
    530                 535                 540
Lys Ala Met Ser Gln Leu Asn Gly Asn Gly Thr Gly Ser Leu Glu Phe
545                 550                 555                 560
Asp Ser Val Val Ser Lys Ala Pro Ser Gln Lys Gly Asn Glu Met Asn
```

```
                    565                     570                     575
His Phe Ser Asn Trp Gly Leu Thr Ser Asp Gly Tyr Leu Lys Pro Asp
                580                 585                 590
Ile Thr Ala Pro Gly Gly Asp Ile Tyr Ser Thr Tyr Asn Asp Asn His
            595                 600                 605
Tyr Gly Ser Gln Thr Gly Thr Ser Met Ala Ser Pro Gln Ile Ala Gly
    610                 615                 620
Ala Ser Leu Leu Val Lys Gln Tyr Leu Glu Lys Thr Gln Pro Asn Leu
625                 630                 635                 640
Pro Lys Glu Lys Ile Ala Asp Ile Val Lys Asn Leu Leu Met Ser Asn
                645                 650                 655
Ala Gln Ile His Val Asn Pro Glu Thr Lys Thr Thr Thr Ser Pro Arg
            660                 665                 670
Gln Gln Gly Ala Gly Leu Leu Asn Ile Asp Gly Ala Val Thr Ser Gly
        675                 680                 685
Leu Tyr Val Thr Gly Lys Asp Asn Tyr Gly Ser Ile Ser Leu Gly Asn
    690                 695                 700
Ile Thr Asp Thr Met Thr Phe Asp Val Thr Val His Asn Leu Ser Asn
705                 710                 715                 720
Lys Ala Lys Thr Leu Arg Tyr Asp Thr Glu Leu Leu Thr Asp His Val
                725                 730                 735
Asp Pro Gln Lys Gly Arg Phe Thr Leu Thr Ser Arg Ser Leu Lys Thr
            740                 745                 750
Tyr Gln Gly Gly Glu Val Thr Val Pro Ala Asn Gly Lys Val Thr Val
        755                 760                 765
Lys Val Thr Met Asp Val Ser Gln Phe Thr Lys Glu Leu Thr Lys Gln
    770                 775                 780
Met Pro Asn Gly Tyr Tyr Leu Glu Gly Phe Val Arg Phe Arg Asp Ser
785                 790                 795                 800
Gln Asp Ala Gln Leu Asn Arg Val Asn Ile Pro Phe Val Gly Phe Lys
                805                 810                 815
Gly Gln Phe Glu Asn Leu Ala Val Ala Glu Glu Ser Ile Tyr Arg Leu
            820                 825                 830
Lys Ser Gln Gly Lys Thr Gly Phe Tyr Phe Asp Glu Ser Gly Pro Lys
        835                 840                 845
Asp Asp Ile Tyr Val Gly Lys His Phe Thr Gly Leu Val Thr Leu Gly
    850                 855                 860
Ser Glu Thr Asn Val Ser Thr Lys Thr Ile Ser Asp Asn Gly Leu His
865                 870                 875                 880
Thr Leu Gly Thr Phe Lys Asn Ala Asp Gly Lys Phe Ile Leu Glu Lys
                885                 890                 895
Asn Ala Gln Gly Asn Pro Val Leu Ala Ile Ser Pro Asn Gly Asp Asn
            900                 905                 910
Asn Gln Asp Phe Ala Ala Phe Lys Gly Val Phe Leu Arg Lys Tyr Gln
        915                 920                 925
Gly Leu Lys Ala Ser Val Tyr His Ala Ser Asp Lys Glu His Lys Asn
    930                 935                 940
Pro Leu Trp Val Ser Pro Glu Ser Phe Lys Gly Asp Lys Asn Phe Asn
945                 950                 955                 960
Ser Asp Ile Arg Phe Ala Lys Ser Thr Thr Leu Leu Gly Thr Ala Phe
                965                 970                 975
Ser Gly Lys Ser Leu Thr Gly Ala Glu Leu Pro Asp Gly Tyr Tyr His
            980                 985                 990
Tyr Val Val Ser Tyr Tyr Pro Asp Val Val Gly Ala Lys Arg Gln Glu
        995                 1000                1005
Met Thr Phe Asp Met Ile Leu Asp Arg Gln Lys Pro Val Leu Ser Gln
    1010                1015                1020
Ala Thr Phe Asp Pro Glu Thr Asn Arg Phe Lys Pro Glu Pro Leu Lys
1025                1030                1035                1040
Asp Arg Gly Leu Ala Gly Val Arg Lys Asp Ser Val Phe Tyr Leu Glu
                1045                1050                1055
```

Arg Lys Asp Asn Lys Pro Tyr Thr Val Thr Ile Asn Asp Ser Tyr Lys
1060 1065 1070
Tyr Val Ser Val Ala Asp Asn Lys Thr Phe Val Glu Arg Gln Ala Asp
1075 1080 1085
Gly Ser Phe Ile Leu Pro Leu Asp Lys Ala Lys Leu Gly Asp Phe Tyr
1090 1095 1100
Tyr Met Val Glu Asp Phe Ala Gly Asn Val Ala Ile Ala Lys Leu Gly
1105 1110 1115 1120
Asp His Leu Pro Gln Thr Leu Gly Lys Thr Pro Ile Lys Leu Lys Leu
1125 1130 1135
Thr Asp Gly Asn Tyr Gln Thr Lys Glu Thr Leu Lys Asp Asn Leu Glu
1140 1145 1150
Met Thr Gln Ser Asp Thr Gly Leu Val Thr Asn Gln Ala Gln Leu Ala
1155 1160 1165
Val Val His Arg Asn Gln Pro Gln Ser Gln Leu Thr Lys Met Asn Gln
1170 1175 1180
Asp Phe Phe Ile Ser Pro Asn Glu Asp Gly Asn Lys Asp Phe Val Ala
1185 1190 1195 1200
Phe Lys Gly Leu Lys Asn Asn Val Tyr Asn Asp Leu Thr Val Asn Val
1205 1210 1215
Tyr Ala Lys Asp Asp His Gln Lys Gln Thr Pro Ile Trp Ser Ser Gln
1220 1225 1230
Ala Gly Ala Ser Ala Ser Ala Ile Glu Ser Thr Ala Trp Tyr Gly Ile
1235 1240 1245
Thr Ala Arg Gly Ser Lys Val Met Pro Gly Asp Tyr Gln Tyr Val Val
1250 1255 1260
Thr Tyr Arg Asp Glu His Gly Lys Glu His Gln Lys Gln Tyr Thr Ile
1265 1270 1275 1280
Ser Val Asn Asp Lys Lys Pro Met Ile Thr Gln Gly Arg Phe Asp Thr
1285 1290 1295
Ile Asn Gly Val Asp His Phe Thr Pro Asp Lys Thr Lys Ala Leu Gly
1300 1305 1310
Ser Ser Gly Ile Val Arg Glu Glu Val Phe Tyr Leu Ala Lys Lys Asn
1315 1320 1325
Gly Arg Lys Phe Asp Val Thr Glu Gly Lys Asp Gly Ile Thr Val Ser
1330 1335 1340
Asp Asn Lys Val Tyr Ile Pro Lys Asn Pro Asp Gly Ser Tyr Thr Ile
1345 1350 1355 1360
Ser Lys Arg Asp Gly Val Thr Leu Ser Asp Tyr Tyr Tyr Leu Val Glu
1365 1370 1375
Asp Arg Ala Gly Asn Val Ser Phe Ala Thr Leu Arg Asp Leu Lys Ala
1380 1385 1390
Val Gly Lys Asp Lys Ala Val Val Asn Phe Gly Leu Asp Leu Pro Val
1395 1400 1405
Pro Glu Asp Lys Gln Ile Val Asn Phe Thr Tyr Leu Val Arg Asp Ala
1410 1415 1420
Asp Gly Lys Pro Ile Glu Asn Leu Glu Tyr Tyr Asn Asn Ser Gly Asn
1425 1430 1435 1440
Ser Leu Ile Leu Pro Tyr Gly Lys Tyr Thr Val Glu Leu Leu Thr Tyr
1445 1450 1455
Asp Thr Asn Ala Ala Lys Leu Glu Ser Asp Lys Ile Val Ser Phe Thr
1460 1465 1470
Leu Ser Ala Asp Asn Asn Phe Gln Gln Val Thr Phe Lys Met Thr Met
1475 1480 1485
Leu Ala Thr Ser Gln Ile Thr Ala His Phe Asp His Leu Leu Pro Glu
1490 1495 1500
Gly Ser Arg Val Ser Leu Lys Thr Ala Gln Gly Gln Leu Ile Pro Leu
1505 1510 1515 1520
Glu Gln Ser Leu Tyr Val Pro Lys Ala Tyr Gly Lys Thr Val Gln Glu
1525 1530 1535
Gly Thr Tyr Glu Val Val Val Ser Leu Pro Lys Gly Tyr Arg Ile Glu

```
            1540                1545                1550
Gly Asn Thr Lys Val Asn Thr Leu Pro Asn Glu Val His Glu Leu Ser
        1555                1560                1565
Leu Arg Leu Val Lys Val Gly Asp Ala Ser Asp Ser Thr Gly Asp His
    1570                1575                1580
Lys Val Met Ser Lys Asn Asn Ser Gln Ala Leu Thr Ala Ser Ala Thr
1585                1590                1595                1600
Pro Thr Lys Thr Thr Thr Ser Ala Thr Ala Lys Ala Leu Pro Ser Ala
                1605                1610                1615
Gly Glu Lys Met Gly Leu Lys Leu Arg Ile Val Gly Leu Val Leu Leu
            1620                1625                1630
Gly Leu Thr Cys Val Phe Ser Arg Lys Lys Ser Thr Lys Asp
        1635                1640                1645
```

<210> 11
<211> 1647
<212> PRT
<213> Streptococcus pyogenes

<400> 11

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Gln Ser Pro Asp Thr Ser Gln Ile Thr Pro Lys Thr
65                  70                  75                  80
Asn Arg Glu Lys Glu Gln Pro Gln Gly Leu Val Ser Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Asp Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Gly Asp Phe Asp Glu Asp Trp Glu
    210                 215                 220
Asn Phe Glu Phe Asp Ala Glu Pro Lys Ala Ile Lys Lys Asn Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
```

```
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Ala Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Asp Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
```

```
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
```

```
                1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Asp Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Thr Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 12
<211> 1647
<212> PRT
<213> Streptococcus pyogenes

<400> 12

```
Val Glu Lys Lys Gln Arg Phe Ser Leu Arg Lys Tyr Lys Ser Gly Thr
 1              5                   10                  15
Phe Ser Val Leu Ile Gly Ser Val Phe Leu Met Met Met Thr Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Gln Ser Pro Asp Thr Ser Gln Ile Thr Pro Lys Thr
65                  70                  75                  80
Asn Arg Glu Lys Glu Gln Pro Gln Gly Leu Val Ser Glu Pro Thr Thr
```

```
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Asp Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Gly Asp Phe Asp Glu Asp Trp Glu
    210                 215                 220
Asn Phe Glu Phe Asp Ala Glu Pro Lys Ala Ile Lys Lys Asn Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Ala Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Asp Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
```

```
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
```

```
                1060                    1065                    1070
Lys Tyr Val Ser Val Glu Asp Asn Lys Thr Phe Val Glu Arg Gln Ala
            1075                    1080                    1085
Asp Gly Ser Phe Ile Leu Pro Leu Asp Lys Ala Lys Leu Gly Asp Phe
        1090                    1095                    1100
Tyr Tyr Met Val Glu Asp Phe Ala Gly Asn Val Ala Ile Ala Lys Leu
1105                    1110                    1115                    1120
Gly Asp His Leu Pro Gln Thr Leu Gly Lys Thr Pro Ile Lys Leu Lys
                1125                    1130                    1135
Leu Thr Asp Gly Asn Tyr Gln Thr Lys Glu Thr Leu Lys Asp Asn Leu
            1140                    1145                    1150
Glu Met Thr Gln Ser Asp Thr Gly Leu Val Thr Asn Gln Ala Gln Leu
        1155                    1160                    1165
Ala Val Val His Arg Asn Gln Pro Gln Ser Gln Leu Thr Lys Met Asn
    1170                    1175                    1180
Gln Asp Phe Phe Ile Ser Pro Asn Glu Asp Gly Asn Lys Asp Phe Val
1185                    1190                    1195                    1200
Ala Phe Lys Gly Leu Lys Asn Asn Val Tyr Asn Asp Leu Thr Val Asn
                1205                    1210                    1215
Val Tyr Ala Lys Asp Asp His Gln Lys Gln Thr Pro Ile Trp Ser Ser
            1220                    1225                    1230
Gln Ala Gly Ala Ser Ala Ser Ala Ile Glu Ser Thr Ala Trp Tyr Gly
        1235                    1240                    1245
Ile Thr Ala Arg Gly Ser Lys Val Met Pro Gly Asp Tyr Gln Tyr Val
    1250                    1255                    1260
Val Thr Tyr Arg Asp Glu His Gly Lys Glu His Gln Lys Gln Tyr Thr
1265                    1270                    1275                    1280
Ile Ser Val Asn Asp Lys Lys Pro Met Ile Thr Gln Gly Arg Phe Asp
                1285                    1290                    1295
Thr Ile Asn Gly Val Asp His Phe Thr Pro Asp Lys Thr Lys Ala Leu
            1300                    1305                    1310
Gly Ser Ser Gly Ile Val Arg Glu Glu Val Phe Tyr Leu Ala Lys Lys
        1315                    1320                    1325
Asn Gly Arg Lys Phe Asp Val Thr Glu Gly Lys Asp Gly Ile Thr Val
    1330                    1335                    1340
Ser Asp Asn Lys Val Tyr Ile Pro Lys Asn Pro Asp Gly Ser Tyr Thr
1345                    1350                    1355                    1360
Ile Ser Lys Arg Asp Gly Val Thr Leu Ser Asp Tyr Tyr Tyr Leu Val
                1365                    1370                    1375
Glu Asp Arg Ala Gly Asn Val Ser Phe Ala Thr Leu Arg Asp Leu Lys
            1380                    1385                    1390
Ala Val Gly Lys Asp Lys Ala Val Val Asn Phe Gly Leu Asp Leu Pro
        1395                    1400                    1405
Val Pro Glu Asp Lys Gln Ile Val Asn Phe Thr Tyr Leu Val Arg Asp
    1410                    1415                    1420
Ala Asp Gly Lys Pro Ile Glu Asn Leu Glu Tyr Tyr Asn Asn Ser Gly
1425                    1430                    1435                    1440
Asn Ser Leu Ile Leu Pro Tyr Gly Lys Tyr Thr Val Glu Leu Leu Thr
                1445                    1450                    1455
Tyr Asp Thr Asn Ala Ala Lys Leu Glu Ser Asp Lys Ile Val Ser Phe
            1460                    1465                    1470
Thr Leu Ser Ala Asp Asn Asn Phe Gln Gln Val Thr Phe Lys Met Thr
        1475                    1480                    1485
Met Leu Ala Thr Ser Gln Ile Thr Ala His Phe Asp His Leu Leu Pro
    1490                    1495                    1500
Glu Gly Ser Arg Val Ser Leu Lys Thr Ala Gln Gly Gln Leu Ile Pro
1505                    1510                    1515                    1520
Leu Glu Gln Ser Leu Tyr Val Pro Lys Ala Tyr Gly Lys Thr Val Gln
                1525                    1530                    1535
Glu Asp Thr Tyr Glu Val Val Val Ser Leu Pro Lys Gly Tyr Arg Ile
            1540                    1545                    1550
```

```
Glu Gly Asn Thr Lys Val Asn Ala Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Val Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 13
<211> 1647
<212> PRT
<213> Streptococcus pyogenes

<400> 13

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Met Thr Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Gln Ser Pro Asp Thr Ser Gln Ile Thr Pro Lys Thr
65                  70                  75                  80
Asn Arg Glu Lys Glu Gln Pro Gln Gly Leu Val Ser Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Asp Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Gly Asp Phe Asp Glu Asp Trp Glu
    210                 215                 220
Asn Phe Glu Phe Asp Ala Glu Pro Lys Ala Ile Lys Lys Asn Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
```

```
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Ala Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Asp Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
```

```
                820                 825                 830
Leu Lys Ser Gln Gly Lys Thr Gly Phe Tyr Phe Asp Glu Ser Gly Pro
            835                 840                 845
Lys Asp Asp Ile Tyr Val Gly Lys His Phe Thr Gly Leu Val Thr Leu
        850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
```

```
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Asp Thr Tyr Glu Val Val Val Ser Leu Pro Lys Gly Tyr Arg Ile
            1540                1545                1550
Glu Gly Asn Thr Lys Val Asn Ala Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Val Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 14
<211> 1647
<212> PRT
<213> Streptococcus pyogenes

<400> 14

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Gln Ser Pro Asp Thr Ser Gln Ile Thr Pro Lys Thr
65                  70                  75                  80
Asn Arg Glu Lys Glu Gln Pro Gln Gly Leu Val Phe Glu Pro Thr Thr
                85                  90                  95
```

```
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Asp Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Gly Asp Phe Asp Glu Asp Trp Glu
    210                 215                 220
Asn Phe Glu Phe Asp Ala Glu Pro Lys Ala Ile Lys Lys Asn Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Ala Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Lys Ala Gln
465                 470                 475                 480
Asp Val Lys Asp Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
```

```
                580                 585                 590
Asp Ile Thr Ala Pro Gly Gly Asp Ile Tyr Ser Thr Tyr Asn Asp Asn
            595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
```

Lys Tyr Val Ser Val Glu Asp Asn Lys Thr Phe Val Glu Arg Gln Ala
1075 1080 1085
Asp Gly Ser Phe Ile Leu Pro Leu Asp Lys Ala Lys Leu Gly Asp Phe
1090 1095 1100
Tyr Tyr Met Val Glu Asp Phe Ala Gly Asn Val Ala Ile Ala Lys Leu
1105 1110 1115 1120
Gly Asp His Leu Pro Gln Thr Leu Gly Lys Thr Pro Ile Lys Leu Lys
1125 1130 1135
Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Ala Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Met Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Gly Gln Leu Ile Pro
1505 1510 1515 1520
Leu Glu Gln Ser Leu Tyr Val Pro Lys Ala Tyr Gly Lys Thr Val Gln
1525 1530 1535
Glu Asp Thr Tyr Glu Val Val Val Ser Leu Pro Lys Gly Tyr Arg Ile
1540 1545 1550
Glu Gly Asn Thr Lys Val Asn Ala Leu Pro Asn Glu Val His Glu Leu

```
            1555                1560                1565
Ser Leu Arg Leu Val Lys Val Gly Asp Ala Ser Asp Ser Thr Gly Asp
        1570                1575                1580
His Lys Val Met Ser Lys Asn Asn Ser Gln Ala Leu Thr Val Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 15
<211> 1646
<212> PRT
<213> Streptococcus pyogenes

<400> 15

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Ala Gln Gln Gln Ala Gln His Leu Thr Asn Thr Glu Leu Ser Ser
    50                  55                  60
Ala Glu Ser Gln Ser Pro Asp Thr Ser Gln Ile Thr Pro Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Ser Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Pro Lys Ala Ile Lys Lys Asn Lys Ile Tyr
225                 230                 235                 240
Arg Pro Gln Ser Thr Gln Ala Pro Lys Glu Thr Val Ile Lys Thr Glu
                245                 250                 255
Glu Thr Asp Gly Ser His Asp Ile Asp Trp Thr Gln Thr Asp Asp Glu
            260                 265                 270
Thr Lys Tyr Glu Ser His Gly Met His Val Thr Gly Ile Val Ala Gly
        275                 280                 285
Asn Ser Lys Glu Ala Ala Ala Thr Gly Glu Arg Phe Leu Gly Ile Ala
    290                 295                 300
Pro Glu Ala Gln Val Met Phe Met Arg Val Phe Ala Asn Asp Val Met
305                 310                 315                 320
Gly Ser Ala Glu Ser Leu Phe Ile Lys Ala Ile Glu Asp Ala Val Ala
                325                 330                 335
Leu Gly Ala Asp Val Ile Asn Leu Ser Leu Gly Thr Ala Asn Gly Ala
```

```
            340                 345                 350
Gln Leu Ser Gly Ser Lys Pro Leu Met Glu Ala Ile Glu Lys Ala Lys
        355                 360                 365
Lys Ala Gly Val Ser Val Val Val Ala Ala Gly Asn Glu Arg Val Tyr
    370                 375                 380
Gly Ser Asp His Asp Asp Pro Leu Ala Thr Asn Pro Asp Tyr Gly Leu
385                 390                 395                 400
Val Gly Ser Pro Ser Thr Gly Arg Thr Pro Thr Ser Val Ala Ala Ile
                405                 410                 415
Asn Ser Lys Trp Val Ile Gln Arg Leu Met Thr Val Lys Glu Leu Glu
            420                 425                 430
Asn Arg Ala Asp Leu Asn His Gly Lys Ala Ile Tyr Ser Glu Ser Val
        435                 440                 445
Asp Phe Lys Asp Ile Lys Asp Ser Leu Gly Tyr Asp Lys Ser His Gln
    450                 455                 460
Phe Ala Tyr Val Lys Glu Ser Thr Asp Ala Gly Tyr Asn Ala Gln Asp
465                 470                 475                 480
Val Lys Gly Lys Ile Ala Leu Ile Glu Arg Asp Pro Asn Lys Thr Tyr
                485                 490                 495
Asp Glu Met Ile Ala Leu Ala Lys Lys His Gly Ala Leu Gly Val Leu
            500                 505                 510
Ile Phe Asn Asn Lys Pro Gly Gln Ser Asn Arg Ser Met Arg Leu Thr
        515                 520                 525
Ser Asn Gly Met Gly Ile Pro Ser Ala Phe Ile Ser His Glu Phe Gly
    530                 535                 540
Lys Ala Met Ser Gln Leu Asn Gly Asn Gly Thr Gly Ser Leu Glu Phe
545                 550                 555                 560
Asp Ser Val Val Ser Lys Ala Pro Ser Gln Lys Gly Asn Glu Met Asn
                565                 570                 575
His Phe Ser Asn Trp Gly Leu Thr Ser Asp Gly Tyr Leu Lys Pro Asp
            580                 585                 590
Ile Thr Ala Pro Gly Gly Asp Ile Tyr Ser Thr Tyr Asn Asp Asn His
        595                 600                 605
Tyr Gly Ser Gln Thr Gly Thr Ser Met Ala Ser Pro Gln Ile Ala Gly
    610                 615                 620
Ala Ser Leu Leu Val Lys Gln Tyr Leu Glu Lys Thr Gln Pro Asn Leu
625                 630                 635                 640
Pro Lys Glu Lys Ile Ala Asp Ile Val Lys Asn Leu Leu Met Ser Asn
                645                 650                 655
Ala Gln Ile His Val Asn Pro Glu Thr Lys Thr Thr Thr Ser Pro Arg
            660                 665                 670
Gln Gln Gly Ala Gly Leu Leu Asn Ile Asp Gly Ala Val Thr Ser Gly
        675                 680                 685
Leu Tyr Val Thr Gly Lys Asp Asn Tyr Gly Ser Ile Ser Leu Gly Asn
    690                 695                 700
Ile Thr Asp Thr Met Thr Phe Asp Val Thr Val His Asn Leu Ser Asn
705                 710                 715                 720
Lys Asp Lys Thr Leu Arg Tyr Asp Thr Glu Leu Leu Thr Asp His Val
                725                 730                 735
Asp Pro Gln Lys Gly Arg Phe Thr Leu Thr Ser Arg Ser Leu Lys Thr
            740                 745                 750
Tyr Gln Gly Gly Glu Val Thr Val Pro Ala Asn Gly Lys Val Thr Val
        755                 760                 765
Arg Val Thr Met Asp Val Ser Gln Phe Thr Lys Glu Leu Thr Lys Gln
    770                 775                 780
Met Pro Asn Gly Tyr Tyr Leu Glu Gly Phe Val Arg Phe Arg Asp Ser
785                 790                 795                 800
Gln Asp Asp Gln Leu Asn Arg Val Asn Ile Pro Phe Val Gly Phe Lys
                805                 810                 815
Gly Gln Phe Glu Asn Leu Ala Val Ala Glu Glu Ser Ile Tyr Arg Leu
            820                 825                 830
```

Lys Ser Gln Gly Lys Thr Gly Phe Tyr Phe Asp Glu Ser Gly Pro Lys
835 840 845
Asp Asp Ile Tyr Val Gly Lys His Phe Thr Gly Leu Val Thr Leu Gly
850 855 860
Ser Glu Thr Asn Val Ser Thr Lys Thr Ile Ser Asp Asn Gly Leu His
865 870 875 880
Thr Leu Gly Thr Phe Lys Asn Ala Asp Gly Lys Phe Ile Leu Glu Lys
885 890 895
Asn Ala Gln Gly Asn Pro Val Leu Ala Ile Ser Pro Asn Gly Asp Asn
900 905 910
Asn Gln Asp Phe Ala Ala Phe Lys Gly Val Phe Leu Arg Lys Tyr Gln
915 920 925
Gly Leu Lys Ala Ser Val Tyr His Ala Ser Asp Lys Glu His Lys Asn
930 935 940
Pro Leu Trp Val Ser Pro Glu Ser Phe Lys Gly Asp Lys Asn Phe Asn
945 950 955 960
Ser Asp Ile Arg Phe Ala Lys Ser Thr Thr Leu Leu Gly Thr Ala Phe
965 970 975
Ser Gly Lys Ser Leu Thr Gly Ala Glu Leu Pro Asp Gly Tyr Tyr His
980 985 990
Tyr Val Val Ser Tyr Tyr Pro Asp Val Val Gly Ala Lys Arg Gln Glu
995 1000 1005
Met Thr Phe Asp Met Ile Leu Asp Arg Gln Lys Pro Val Leu Ser Gln
1010 1015 1020
Ala Thr Phe Asp Pro Glu Thr Asn Arg Phe Lys Pro Glu Pro Leu Lys
1025 1030 1035 1040
Asp Arg Gly Leu Ala Gly Val Arg Lys Asp Ser Val Phe Tyr Leu Glu
1045 1050 1055
Arg Lys Asp Asn Lys Pro Tyr Thr Val Thr Ile Asn Asp Ser Tyr Lys
1060 1065 1070
Tyr Val Ser Val Glu Asp Asn Lys Thr Phe Val Glu Arg Gln Ala Asp
1075 1080 1085
Gly Ser Phe Ile Leu Pro Leu Asp Lys Ala Lys Leu Gly Asp Phe Tyr
1090 1095 1100
Tyr Met Val Glu Asp Phe Ala Gly Asn Val Ala Ile Ala Lys Leu Gly
1105 1110 1115 1120
Asp His Leu Pro Gln Thr Leu Gly Lys Thr Pro Ile Lys Leu Lys Leu
1125 1130 1135
Thr Asp Gly Asn Tyr Gln Thr Lys Glu Thr Leu Lys Asp Asn Leu Glu
1140 1145 1150
Met Thr Gln Ser Asp Thr Gly Leu Val Thr Asn Gln Ala Gln Leu Ala
1155 1160 1165
Val Val His Arg Asn Gln Pro Gln Ser Gln Leu Thr Lys Met Asn Gln
1170 1175 1180
Asp Phe Phe Ile Ser Pro Asn Glu Asp Gly Asn Lys Asp Phe Val Ala
1185 1190 1195 1200
Phe Lys Gly Leu Lys Asn Asn Val Tyr Asn Asp Leu Thr Val Asn Val
1205 1210 1215
Tyr Ala Lys Asp Asp His Gln Lys Gln Thr Pro Ile Trp Ser Ser Gln
1220 1225 1230
Ala Gly Ala Ser Ala Ser Ala Ile Glu Ser Thr Ala Trp Tyr Gly Ile
1235 1240 1245
Thr Ala Arg Gly Ser Lys Val Met Pro Gly Asp Tyr Gln Tyr Val Val
1250 1255 1260
Thr Tyr Arg Asp Glu His Gly Lys Val His Gln Lys Gln Tyr Thr Ile
1265 1270 1275 1280
Ser Val Asn Asp Lys Lys Pro Met Ile Thr Gln Gly Arg Phe Asp Thr
1285 1290 1295
Ile Asn Gly Val Asp His Phe Thr Pro Asp Lys Thr Lys Ala Leu Gly
1300 1305 1310
Ser Ser Gly Ile Val Arg Glu Glu Val Phe Tyr Leu Ala Lys Lys Asn

1315 1320 1325
Gly Arg Lys Phe Asp Val Thr Glu Gly Lys Asp Gly Ile Thr Val Ser
1330 1335 1340
Asp Asn Lys Val Tyr Ile Pro Lys Asn Pro Asp Gly Ser Tyr Thr Ile
1345 1350 1355 1360
Ser Lys Arg Asp Gly Val Thr Leu Ser Asp Tyr Tyr Tyr Leu Val Glu
1365 1370 1375
Asp Arg Ala Gly Asn Val Ser Phe Ala Thr Leu Arg Asp Leu Lys Ala
1380 1385 1390
Val Gly Lys Asp Lys Ala Val Val Asn Phe Gly Leu Asp Leu Pro Val
1395 1400 1405
Pro Glu Asp Lys Gln Ile Val Asn Phe Thr Tyr Leu Val Arg Asp Ala
1410 1415 1420
Asp Gly Lys Pro Ile Glu Asn Leu Glu Tyr Tyr Asn Asn Ser Gly Asn
1425 1430 1435 1440
Ser Leu Ile Leu Pro Tyr Gly Lys Tyr Thr Val Glu Leu Leu Thr Tyr
1445 1450 1455
Asp Thr Asn Ala Ala Lys Leu Glu Ser Asp Lys Ile Val Ser Phe Thr
1460 1465 1470
Leu Ser Ala Asp Asn Asn Phe Gln Gln Val Thr Phe Lys Met Thr Met
1475 1480 1485
Leu Ala Thr Ser Gln Ile Thr Ala His Phe Asp His Leu Leu Pro Glu
1490 1495 1500
Gly Ser Arg Val Ser Leu Lys Thr Ala Gln Gly Gln Leu Ile Pro Leu
1505 1510 1515 1520
Glu Gln Ser Leu Tyr Val Pro Lys Ala Tyr Gly Lys Thr Val Gln Glu
1525 1530 1535
Gly Thr Tyr Glu Val Val Val Ser Leu Pro Lys Gly Tyr Arg Ile Glu
1540 1545 1550
Gly Asn Thr Lys Val Asn Ala Leu Pro Asn Glu Val His Glu Leu Ser
1555 1560 1565
Leu Arg Leu Val Lys Val Gly Asp Ala Ser Asp Ser Thr Gly Asp His
1570 1575 1580
Lys Val Met Ser Lys Asn Asn Ser Gln Ala Leu Thr Ala Ser Ala Thr
1585 1590 1595 1600
Pro Thr Lys Thr Thr Thr Ser Ala Thr Ala Lys Ala Leu Pro Ser Thr
1605 1610 1615
Gly Glu Lys Met Gly Leu Lys Leu Arg Ile Val Gly Leu Val Leu Leu
1620 1625 1630
Gly Leu Thr Cys Val Phe Ser Arg Lys Lys Ser Thr Lys Asp
1635 1640 1645

<210> 16
<211> 1647
<212> PRT
<213> Streptococcus pyogenes

<400> 16

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Met Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Gln Ser Pro Asp Thr Ser Gln Ile Thr Pro Lys Thr
65                  70                  75                  80
Asn Arg Glu Lys Glu Gln Pro Gln Gly Leu Val Ser Glu Pro Thr Thr
                85                  90                  95
Thr Glu Leu Ala Asp Thr Asp Ala Ala Ser Met Ala Asn Thr Gly Pro
```

```
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Asp Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Glu Asp Phe Asp Glu Asp Trp Glu
    210                 215                 220
Asn Phe Glu Phe Asp Ala Glu Pro Lys Ala Ile Lys Lys Asn Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Glu Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ser Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
```

```
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
```

```
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Val His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Ala Leu Pro Asn Glu Val His Glu Leu
        1555                1560                1565
```

Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Thr Thr Thr Ser Ala Thr Ala Lys Ala Leu Pro Ser
1605 1610 1615
Thr Gly Glu Lys Met Gly Leu Lys Leu Arg Ile Val Gly Leu Val Leu
1620 1625 1630
Leu Gly Leu Thr Cys Val Phe Ser Arg Lys Lys Ser Thr Lys Asp
1635 1640 1645

<210> 17
<211> 1645
<212> PRT
<213> Streptococcus pyogenes

<400> 17

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Pro Pro Leu Thr Asn Thr Glu Leu Ser Ser Ala
    50                  55                  60
Glu Ser Gln Pro Gln Asp Thr Ser Gln Val Thr Pro Glu Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Ser Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys Asn Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
```

```
Leu Ser Gly Ser Lys Pro Leu Ile Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Lys Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser Tyr Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
```

```
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Ala Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
```

```
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Ala Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Thr Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 18
<211> 1645
<212> PRT
<213> Streptococcus pyogenes

<400> 18

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Pro Pro Leu Thr Asn Thr Glu Leu Ser Ser Ala
    50                  55                  60
Glu Ser Gln Pro Gln Asp Thr Ser Gln Val Thr Pro Glu Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Ser Asp Ala
            100                 105                 110
```

```
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys Asn Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Ile Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Lys Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser Tyr Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
```

595 600 605
Gly Ser Gln Thr Gly Thr Ser Met Ala Ser Pro Gln Ile Ala Gly Ala
610 615 620
Ser Leu Leu Val Lys Gln Tyr Leu Glu Lys Thr Gln Pro Asn Leu Pro
625 630 635 640
Lys Glu Lys Ile Ala Asp Ile Val Lys Asn Leu Leu Met Ser Asn Ala
645 650 655
Gln Ile His Val Asn Pro Glu Thr Lys Thr Thr Thr Ser Pro Arg Gln
660 665 670
Gln Gly Ala Gly Leu Leu Asn Ile Asp Gly Ala Val Thr Ser Gly Leu
675 680 685
Tyr Val Thr Gly Lys Asp Asn Tyr Gly Ser Ile Ser Leu Gly Asn Ile
690 695 700
Thr Asp Thr Met Thr Phe Asp Val Thr Val His Asn Leu Ser Asn Lys
705 710 715 720
Ala Lys Thr Leu Arg Tyr Asp Thr Glu Leu Leu Thr Asp His Val Asp
725 730 735
Pro Gln Lys Gly Arg Phe Thr Leu Thr Ser Arg Ser Leu Lys Thr Tyr
740 745 750
Gln Gly Gly Glu Val Thr Val Pro Ala Asn Gly Lys Val Thr Val Arg
755 760 765
Val Thr Met Asp Val Ser Gln Phe Thr Lys Glu Leu Thr Lys Gln Met
770 775 780
Pro Asn Gly Tyr Tyr Leu Glu Gly Phe Val Arg Phe Arg Asp Ser Gln
785 790 795 800
Asp Asp Gln Leu Asn Arg Val Asn Ile Pro Phe Val Gly Phe Lys Gly
805 810 815
Gln Phe Glu Asn Leu Ala Val Ala Glu Glu Ser Ile Tyr Arg Leu Lys
820 825 830
Ser Gln Gly Lys Thr Gly Phe Tyr Phe Asp Glu Ser Gly Pro Lys Asp
835 840 845
Asp Ile Tyr Val Gly Lys His Phe Thr Gly Leu Val Thr Leu Gly Ser
850 855 860
Glu Thr Asn Val Ser Thr Lys Thr Ile Ser Asp Asn Gly Leu His Thr
865 870 875 880
Leu Gly Thr Phe Lys Asn Ala Asp Gly Lys Phe Ile Leu Glu Lys Asn
885 890 895
Ala Gln Gly Asn Pro Val Leu Ala Ile Ser Pro Asn Gly Asp Asn Asn
900 905 910
Gln Asp Phe Ala Ala Phe Lys Gly Val Phe Leu Arg Lys Tyr Gln Gly
915 920 925
Leu Lys Ala Ser Val Tyr His Ala Ser Asp Lys Glu His Lys Asn Pro
930 935 940
Leu Trp Val Ser Pro Glu Ser Phe Lys Gly Asp Lys Asn Phe Asn Ser
945 950 955 960
Asp Ile Arg Phe Ala Lys Ser Thr Thr Leu Leu Gly Thr Ala Phe Ser
965 970 975
Gly Lys Ser Leu Thr Gly Ala Glu Leu Pro Asp Gly Tyr Tyr His Tyr
980 985 990
Val Val Ser Tyr Tyr Pro Asp Val Val Gly Ala Lys Arg Gln Glu Met
995 1000 1005
Thr Phe Asp Met Ile Leu Asp Arg Gln Lys Pro Val Leu Ser Gln Ala
1010 1015 1020
Thr Phe Asp Pro Glu Thr Asn Arg Phe Lys Pro Glu Pro Leu Lys Asp
1025 1030 1035 1040
Arg Gly Leu Ala Gly Val Arg Lys Asp Ser Val Phe Tyr Leu Glu Arg
1045 1050 1055
Lys Asp Asn Lys Pro Tyr Thr Val Thr Ile Asn Asp Ser Tyr Lys Tyr
1060 1065 1070
Val Ser Val Ala Asp Asn Lys Thr Phe Val Glu Arg Gln Ala Asp Gly
1075 1080 1085

Ser Phe Ile Leu Pro Leu Asp Lys Ala Lys Leu Gly Asp Phe Tyr Tyr
1090 1095 1100
Met Val Glu Asp Phe Ala Gly Asn Val Ala Ile Ala Lys Leu Gly Asp
1105 1110 1115 1120
His Leu Pro Gln Thr Leu Gly Lys Thr Pro Ile Lys Leu Lys Leu Thr
1125 1130 1135
Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp
1330 1335 1340
Asn Lys Val Tyr Ile Pro Lys Asn Pro Asp Gly Ser Tyr Thr Ile Ser
1345 1350 1355 1360
Lys Arg Asp Gly Val Thr Leu Ser Asp Tyr Tyr Tyr Leu Val Glu Asp
1365 1370 1375
Arg Ala Gly Asn Val Ser Phe Ala Thr Leu Arg Asp Leu Lys Ala Val
1380 1385 1390
Gly Lys Asp Lys Ala Val Val Asn Phe Gly Leu Asp Leu Pro Val Pro
1395 1400 1405
Glu Asp Lys Gln Ile Val Asn Phe Thr Tyr Leu Val Arg Asp Ala Asp
1410 1415 1420
Gly Lys Pro Ile Glu Asn Leu Glu Tyr Tyr Asn Asn Ser Gly Asn Ser
1425 1430 1435 1440
Leu Ile Leu Pro Tyr Gly Lys Tyr Thr Val Glu Leu Leu Thr Tyr Asp
1445 1450 1455
Thr Asn Ala Ala Lys Leu Glu Ser Asp Lys Ile Val Ser Phe Thr Leu
1460 1465 1470
Ser Ala Asp Asn Asn Phe Gln Gln Val Thr Phe Lys Met Thr Met Leu
1475 1480 1485
Ala Thr Ser Gln Ile Thr Ala His Phe Asp His Leu Leu Pro Glu Gly
1490 1495 1500
Ser Arg Val Ser Leu Lys Thr Ala Gln Gly Gln Leu Ile Pro Leu Glu
1505 1510 1515 1520
Gln Ser Leu Tyr Val Pro Lys Ala Tyr Gly Lys Thr Val Gln Glu Gly
1525 1530 1535
Thr Tyr Glu Val Val Val Ser Leu Pro Lys Gly Tyr Arg Ile Glu Gly
1540 1545 1550
Asn Thr Lys Val Asn Ala Leu Pro Asn Glu Val His Glu Leu Ser Leu
1555 1560 1565
Arg Leu Val Lys Val Gly Asp Ala Ser Asp Ser Thr Gly Asp His Lys

```
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Thr Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 19
<211> 1645
<212> PRT
<213> Streptococcus pyogenes

<400> 19

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro His Thr Ser Gln Val Thr Pro Glu Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met His Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
```

```
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Val Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Lys
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
```

Asp Ile Tyr Val Gly Lys His Phe Thr Gly Leu Val Thr Leu Gly Ser
850 855 860
Glu Thr Asn Val Ser Thr Lys Thr Ile Ser Asp Asn Gly Leu His Thr
865 870 875 880
Leu Gly Thr Phe Lys Asn Ala Asp Gly Lys Phe Ile Leu Glu Lys Asn
885 890 895
Ala Gln Gly Asn Pro Val Leu Ala Ile Ser Pro Asn Gly Asp Asn Asn
900 905 910
Gln Asp Phe Ala Ala Phe Lys Gly Val Phe Leu Arg Lys Tyr Gln Gly
915 920 925
Leu Lys Ala Ser Val Tyr His Ala Ser Asp Lys Glu His Lys Asn Pro
930 935 940
Leu Trp Val Ser Pro Glu Ser Phe Lys Gly Asp Lys Asn Phe Asn Ser
945 950 955 960
Asp Ile Arg Phe Ala Lys Ser Thr Thr Leu Leu Gly Thr Ala Phe Ser
965 970 975
Gly Lys Ser Leu Thr Gly Ala Glu Leu Pro Asp Gly Tyr Tyr His Tyr
980 985 990
Val Val Ser Tyr Tyr Pro Asp Val Val Gly Ala Lys Arg Gln Glu Met
995 1000 1005
Thr Phe Asp Met Ile Leu Asp Arg Gln Lys Pro Val Leu Ser Gln Ala
1010 1015 1020
Thr Phe Asp Pro Glu Thr Asn Arg Phe Lys Pro Glu Pro Leu Lys Asp
1025 1030 1035 1040
Arg Gly Leu Ala Gly Val Arg Lys Asp Ser Val Phe Tyr Leu Glu Arg
1045 1050 1055
Lys Asp Asn Lys Pro Tyr Thr Val Thr Ile Asn Asp Ser Tyr Lys Tyr
1060 1065 1070
Val Ser Val Glu Asp Asn Lys Thr Phe Val Glu Arg Gln Ala Asp Gly
1075 1080 1085
Ser Phe Ile Leu Pro Leu Asp Lys Ala Lys Leu Gly Asp Phe Tyr Tyr
1090 1095 1100
Met Val Glu Asp Phe Ala Gly Asn Val Ala Ile Ala Lys Leu Gly Asp
1105 1110 1115 1120
His Leu Pro Gln Thr Leu Gly Lys Thr Pro Ile Lys Leu Lys Leu Thr
1125 1130 1135
Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp

1330 1335 1340
Asn Lys Val Tyr Ile Pro Lys Asn Pro Asp Gly Ser Tyr Thr Ile Ser
1345 1350 1355 1360
Lys Arg Asp Gly Val Thr Leu Ser Asp Tyr Tyr Tyr Leu Val Glu Asp
1365 1370 1375
Arg Ala Gly Asn Val Ser Phe Ala Thr Leu Arg Asp Leu Lys Ala Val
1380 1385 1390
Gly Lys Asp Lys Ala Val Val Asn Phe Gly Leu Asp Leu Pro Val Pro
1395 1400 1405
Glu Asp Lys Gln Ile Val Asn Phe Thr Tyr Leu Val Arg Asp Ala Asp
1410 1415 1420
Gly Lys Pro Ile Glu Asn Leu Glu Tyr Tyr Asn Asn Ser Gly Asn Ser
1425 1430 1435 1440
Leu Ile Leu Pro Tyr Gly Lys Tyr Thr Val Glu Leu Leu Thr Tyr Asp
1445 1450 1455
Thr Asn Ala Ala Lys Leu Glu Ser Asp Lys Ile Val Ser Phe Thr Leu
1460 1465 1470
Ser Ala Asp Asn Asn Phe Gln Gln Val Thr Phe Lys Met Thr Met Leu
1475 1480 1485
Ala Thr Ser Gln Ile Thr Ala His Phe Asp His Leu Leu Pro Glu Gly
1490 1495 1500
Ser Arg Val Ser Leu Lys Thr Ala Gln Gly Gln Leu Ile Pro Leu Glu
1505 1510 1515 1520
Gln Ser Leu Tyr Val Pro Lys Ala Tyr Gly Lys Thr Val Gln Glu Gly
1525 1530 1535
Thr Tyr Glu Val Val Val Ser Leu Pro Lys Gly Tyr Arg Ile Glu Gly
1540 1545 1550
Asn Thr Lys Val Asn Ala Leu Pro Asn Glu Val His Glu Leu Ser Leu
1555 1560 1565
Arg Leu Val Lys Val Gly Asp Ala Ser Asp Leu Thr Gly Asp His Lys
1570 1575 1580
Val Met Ser Lys Asn Asn Ser Gln Ala Leu Thr Ala Ser Ala Thr Pro
1585 1590 1595 1600
Thr Lys Thr Thr Thr Ser Ala Thr Ala Lys Ala Leu Pro Ser Ala Gly
1605 1610 1615
Glu Lys Met Gly Leu Lys Leu Arg Ile Val Gly Leu Val Leu Leu Gly
1620 1625 1630
Leu Thr Cys Val Phe Ser Arg Lys Lys Ser Thr Lys Asp
1635 1640 1645

<210> 20
<211> 1645
<212> PRT
<213> Streptococcus pyogenes

<400> 20

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro His Thr Ser Gln Val Thr Pro Glu Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
```

```
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met His Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Val Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
```

```
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Lys
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
```

```
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Ala Leu Pro Asn Glu Val His Glu Leu Ser Leu
        1555                1560                1565
Arg Leu Val Lys Val Gly Asp Ala Ser Asp Leu Thr Gly Asp His Lys
    1570                1575                1580
```

```
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 21
<211> 1645
<212> PRT
<213> Streptococcus pyogenes

<400> 21

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro His Thr Ser Gln Val Thr Pro Glu Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met His Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
```

Ala Gly Val Ser Val Val Val Ala Ala Gly Asn Glu Arg Val Tyr Gly
370 375 380
Phe Asp His Asp Asp Pro Leu Ala Thr Asn Pro Asp Tyr Gly Leu Val
385 390 395 400
Gly Ser Pro Ser Thr Gly Arg Thr Pro Thr Ser Val Ala Ala Ile Asn
405 410 415
Ser Lys Trp Val Ile Gln Arg Leu Met Thr Val Lys Glu Leu Glu Asn
420 425 430
Arg Ala Asp Leu Asn His Gly Lys Ala Ile Tyr Ser Glu Ser Val Asp
435 440 445
Phe Lys Asp Ile Lys Asp Ser Leu Gly Tyr Asp Lys Ser His Gln Phe
450 455 460
Ala Tyr Val Lys Glu Ser Thr Asp Ala Gly Tyr Asn Ala Gln Asp Val
465 470 475 480
Lys Gly Lys Ile Ala Leu Ile Glu Arg Asp Pro Asn Lys Thr Tyr Asp
485 490 495
Glu Met Ile Ala Leu Ala Lys Lys His Gly Ala Leu Gly Val Leu Ile
500 505 510
Phe Asn Asn Lys Pro Gly Gln Ser Asn Arg Ser Met Arg Leu Thr Ala
515 520 525
Asn Gly Met Gly Ile Pro Ser Ala Phe Ile Ser His Glu Phe Gly Lys
530 535 540
Ala Met Ser Gln Leu Asn Gly Asn Gly Thr Gly Ser Leu Val Phe Asp
545 550 555 560
Ser Val Val Ser Lys Ala Pro Ser Gln Lys Gly Asn Glu Met Asn His
565 570 575
Phe Ser Asn Trp Gly Leu Thr Ser Asp Gly Tyr Leu Lys Pro Asp Ile
580 585 590
Thr Ala Pro Gly Gly Asp Ile Tyr Ser Thr Tyr Asn Asp Asn His Tyr
595 600 605
Gly Ser Gln Thr Gly Thr Ser Met Ala Ser Pro Gln Ile Ala Gly Ala
610 615 620
Ser Leu Leu Val Lys Gln Tyr Leu Glu Lys Thr Gln Pro Asn Leu Pro
625 630 635 640
Lys Glu Lys Ile Ala Asp Ile Val Lys Asn Leu Leu Met Ser Asn Ala
645 650 655
Gln Ile His Val Asn Pro Glu Thr Lys Thr Thr Thr Ser Pro Arg Gln
660 665 670
Gln Gly Ala Gly Leu Leu Asn Ile Asp Gly Ala Val Thr Ser Gly Leu
675 680 685
Tyr Val Thr Gly Lys Asp Asn Tyr Gly Ser Ile Ser Leu Gly Asn Ile
690 695 700
Thr Asp Thr Met Thr Phe Asp Val Thr Val His Asn Leu Ser Asn Lys
705 710 715 720
Ala Lys Thr Leu Arg Tyr Asp Thr Glu Leu Leu Thr Asp His Val Asp
725 730 735
Pro Gln Lys Gly Arg Phe Thr Leu Thr Ser Arg Ser Leu Lys Thr Tyr
740 745 750
Gln Gly Gly Glu Val Thr Val Pro Ala Asn Gly Lys Val Thr Val Lys
755 760 765
Val Thr Met Asp Val Ser Gln Phe Thr Lys Glu Leu Thr Lys Gln Met
770 775 780
Pro Asn Gly Tyr Tyr Leu Glu Gly Phe Val Arg Phe Arg Asp Ser Gln
785 790 795 800
Asp Asp Gln Leu Asn Arg Val Asn Ile Pro Phe Val Gly Phe Lys Gly
805 810 815
Gln Phe Glu Asn Leu Ala Val Ala Glu Glu Ser Ile Tyr Arg Leu Lys
820 825 830
Ser Gln Gly Lys Thr Gly Phe Tyr Phe Asp Glu Ser Gly Pro Lys Asp
835 840 845
Asp Ile Tyr Val Gly Lys His Phe Thr Gly Leu Val Thr Leu Gly Ser

```
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
```

```
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Ala Leu Pro Asn Glu Val His Glu Leu Ser Leu
        1555                1560                1565
Arg Leu Val Lys Val Gly Asp Ala Ser Asp Leu Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 22
<211> 1647
<212> PRT
<213> Streptococcus pyogenes

<400> 22

```
Val Glu Lys Lys Gln Arg Phe Ser Leu Arg Lys Tyr Lys Ser Gly Thr
 1               5                  10                  15
Phe Ser Val Leu Val Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro Asp Thr Ser Gln Val Thr Pro Glu Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
```

```
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Asp Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Glu Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asp Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asp Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
```

```
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Asp Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Ala Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
```

```
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Gly Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Ala Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
```

```
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 23
<211> 1647
<212> PRT
<213> Streptococcus pyogenes

<400> 23

```
Val Glu Lys Lys Gln Arg Phe Ser Leu Arg Lys Tyr Lys Ser Gly Thr
1               5                   10                  15
Phe Ser Val Leu Val Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro Asp Thr Ser Gln Val Thr Pro Glu Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Asp Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Glu Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
```

370 375 380
Tyr Gly Ser Asp His Asp Asp Pro Leu Ala Thr Asn Pro Asp Tyr Gly
385 390 395 400
Leu Val Gly Ser Pro Ser Thr Gly Arg Thr Pro Thr Ser Val Ala Ala
405 410 415
Ile Asn Ser Lys Trp Val Ile Gln Arg Leu Met Thr Val Lys Glu Leu
420 425 430
Glu Asn Arg Ala Asp Leu Asn His Gly Lys Ala Ile Tyr Ser Glu Ser
435 440 445
Val Asp Phe Lys Asp Ile Lys Asp Ser Leu Gly Tyr Asp Lys Ser His
450 455 460
Gln Phe Ala Tyr Val Lys Glu Ser Thr Asp Ala Gly Tyr Asn Ala Gln
465 470 475 480
Asp Val Lys Gly Lys Ile Ala Leu Ile Glu Arg Asp Pro Asn Lys Thr
485 490 495
Tyr Asp Glu Met Ile Ala Leu Ala Lys Lys His Gly Ala Leu Gly Val
500 505 510
Leu Ile Phe Asn Asn Lys Pro Gly Gln Ser Asn Arg Ser Met Arg Leu
515 520 525
Thr Ala Asn Gly Met Gly Ile Pro Ser Ala Phe Ile Ser His Glu Phe
530 535 540
Gly Lys Ala Met Ser Gln Leu Asn Gly Asn Gly Thr Gly Ser Leu Glu
545 550 555 560
Phe Asp Ser Val Val Ser Lys Ala Pro Ser Gln Lys Gly Asn Glu Met
565 570 575
Asn His Phe Ser Asn Trp Gly Leu Thr Ser Asp Gly Tyr Leu Lys Pro
580 585 590
Asp Ile Thr Ala Pro Gly Gly Asp Ile Tyr Ser Thr Tyr Asn Asp Asn
595 600 605
His Tyr Gly Ser Gln Thr Gly Thr Ser Met Ala Ser Pro Gln Ile Ala
610 615 620
Gly Ala Ser Leu Leu Val Lys Gln Tyr Leu Glu Lys Thr Gln Pro Asn
625 630 635 640
Leu Pro Lys Glu Lys Ile Ala Asp Ile Val Lys Asn Leu Leu Met Ser
645 650 655
Asn Ala Gln Ile His Val Asn Pro Glu Thr Lys Thr Thr Thr Ser Pro
660 665 670
Arg Gln Gln Gly Ala Gly Leu Leu Asn Ile Asp Gly Ala Val Thr Ser
675 680 685
Gly Leu Tyr Val Thr Gly Lys Asp Asn Tyr Gly Ser Ile Ser Leu Gly
690 695 700
Asn Ile Thr Asp Thr Met Thr Phe Asp Val Thr Val His Asn Leu Ser
705 710 715 720
Asn Lys Asp Lys Thr Leu Arg Tyr Asp Thr Glu Leu Leu Thr Asp His
725 730 735
Val Asp Pro Gln Lys Gly Arg Phe Thr Leu Thr Ser Arg Ser Leu Lys
740 745 750
Thr Tyr Gln Gly Gly Glu Val Thr Val Pro Ala Asn Gly Lys Val Thr
755 760 765
Val Arg Val Thr Met Asp Val Ser Gln Phe Thr Lys Glu Leu Thr Lys
770 775 780
Gln Met Pro Asn Gly Tyr Tyr Leu Glu Gly Phe Val Arg Phe Arg Asp
785 790 795 800
Ser Gln Asp Asp Gln Leu Asn Arg Val Asn Ile Pro Phe Val Gly Phe
805 810 815
Lys Gly Gln Phe Glu Asn Leu Ala Val Ala Glu Glu Ser Ile Tyr Arg
820 825 830
Leu Lys Ser Gln Gly Lys Thr Gly Phe Tyr Phe Asp Glu Ser Gly Pro
835 840 845
Lys Asp Asp Ile Tyr Val Gly Lys His Phe Thr Gly Leu Val Thr Leu
850 855 860

Gly Ser Glu Thr Asn Val Ser Thr Lys Thr Ile Ser Asp Asn Gly Leu
865 870 875 880
His Thr Leu Gly Thr Phe Lys Asn Ala Asp Gly Lys Phe Ile Leu Glu
885 890 895
Lys Asn Ala Gln Gly Asn Pro Val Leu Ala Ile Ser Pro Asn Gly Asp
900 905 910
Asn Asn Gln Asp Phe Ala Ala Phe Lys Gly Val Phe Leu Arg Lys Tyr
915 920 925
Gln Gly Leu Lys Ala Ser Val Tyr His Ala Ser Asp Lys Glu His Lys
930 935 940
Asn Pro Leu Trp Val Ser Pro Glu Ser Phe Lys Gly Asp Lys Asn Phe
945 950 955 960
Asn Ser Asp Ile Arg Phe Ala Lys Ser Thr Thr Leu Leu Gly Thr Ala
965 970 975
Phe Ser Gly Lys Ser Leu Thr Gly Ala Glu Leu Pro Asp Gly Tyr Tyr
980 985 990
His Tyr Val Val Ser Tyr Tyr Pro Asp Val Val Gly Ala Lys Arg Gln
995 1000 1005
Glu Met Thr Phe Asp Met Ile Leu Asp Arg Gln Lys Pro Val Leu Ser
1010 1015 1020
Gln Ala Thr Phe Asp Pro Glu Thr Asn Arg Phe Lys Pro Glu Pro Leu
1025 1030 1035 1040
Lys Asp Arg Gly Leu Ala Gly Val Arg Lys Asp Ser Val Phe Tyr Leu
1045 1050 1055
Glu Arg Lys Asp Asn Lys Pro Tyr Thr Val Thr Ile Asn Asp Ser Tyr
1060 1065 1070
Lys Tyr Val Ser Val Ala Asp Asn Lys Thr Phe Val Glu Arg Gln Ala
1075 1080 1085
Asp Gly Ser Phe Ile Leu Pro Leu Asp Lys Ala Lys Leu Gly Asp Phe
1090 1095 1100
Tyr Tyr Met Val Glu Asp Phe Ala Gly Asn Val Ala Ile Ala Lys Leu
1105 1110 1115 1120
Gly Asp His Leu Pro Gln Thr Leu Gly Lys Thr Pro Ile Lys Leu Lys
1125 1130 1135
Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Ala Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr

```
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Gly Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Ala Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 24
<211> 1645
<212> PRT
<213> Streptococcus pyogenes

<400> 24

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln Pro Leu Thr Asn Thr Glu Leu Ser Ser Ala
    50                  55                  60
Glu Ser Gln Ser Pro Asp Thr Ser Gln Ile Thr Pro Lys Thr Asn Arg
65                  70                  75                  80
Glu Lys Glu Gln Ser Gln Asp Leu Val Ser Lys Pro Thr Thr Thr Glu
                85                  90                  95
Leu Ala Asp Thr Asp Ser Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
```

```
        130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Gly Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Glu Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Ala Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Ser
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Lys Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Thr Asn Gly Thr Gly Ser Leu Val Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
```

Ser Leu Leu Val Lys Gln Tyr Leu Glu Lys Thr Gln Pro Asn Leu Pro
625 630 635 640
Lys Glu Lys Ile Ala Asp Ile Val Lys Asn Leu Leu Met Ser Asn Ala
645 650 655
Gln Ile His Val Asn Pro Glu Thr Lys Met Thr Thr Ser Pro Arg Gln
660 665 670
Gln Gly Ala Gly Leu Leu Asn Ile Asp Gly Ala Val Thr Ser Gly Leu
675 680 685
Tyr Val Thr Gly Lys Asp Asn Tyr Gly Ser Ile Ser Leu Gly Asn Ile
690 695 700
Thr Asp Thr Met Thr Phe Asp Val Thr Val His Asn Leu Ser Asn Lys
705 710 715 720
Ala Lys Thr Leu Arg Tyr Asp Thr Glu Leu Leu Thr Asp His Val Asp
725 730 735
Pro Gln Lys Gly His Phe Thr Leu Thr Ser Arg Ser Leu Lys Thr Tyr
740 745 750
Gln Gly Gly Glu Val Thr Val Pro Ala Asn Gly Lys Val Thr Val Arg
755 760 765
Val Thr Met Asp Val Ser Gln Phe Thr Lys Glu Leu Thr Lys Gln Met
770 775 780
Pro Asn Gly Tyr Tyr Leu Glu Gly Phe Val Arg Phe Arg Asp Ser Gln
785 790 795 800
Asp Asp Gln Leu Asn Arg Val Asn Ile Pro Phe Val Gly Phe Lys Gly
805 810 815
Gln Phe Glu Asn Leu Ala Val Ala Glu Glu Ser Ile Tyr Arg Leu Lys
820 825 830
Ser Gln Gly Lys Thr Gly Phe Tyr Phe Asp Glu Ser Gly Pro Lys Asp
835 840 845
Asp Ile Tyr Val Gly Lys His Phe Thr Gly Leu Val Thr Leu Gly Ser
850 855 860
Glu Thr Asn Val Ser Thr Lys Thr Ile Ser Asp Asn Gly Leu His Thr
865 870 875 880
Leu Gly Thr Phe Lys Asn Ala Asp Gly Lys Phe Ile Leu Glu Lys Asn
885 890 895
Ala Gln Gly Asn Pro Val Leu Ala Ile Ser Pro Asn Gly Asp Asn Asn
900 905 910
Gln Asp Phe Ala Ala Phe Lys Gly Val Phe Leu Arg Lys Tyr Gln Gly
915 920 925
Leu Lys Ala Ser Val Tyr His Ala Ser Asp Lys Glu His Lys Asn Pro
930 935 940
Leu Trp Val Ser Pro Glu Ser Phe Lys Gly Asp Lys Asn Phe Asn Ser
945 950 955 960
Asp Ile Arg Phe Ala Lys Ser Thr Thr Leu Leu Gly Thr Ala Phe Ser
965 970 975
Gly Lys Ser Leu Thr Gly Ala Glu Leu Pro Asp Gly Tyr Tyr His Tyr
980 985 990
Val Val Ser Tyr Tyr Pro Asp Val Val Gly Ala Lys Arg Gln Glu Met
995 1000 1005
Thr Phe Asp Met Ile Leu Asp Arg Gln Lys Pro Val Leu Ser Gln Ala
1010 1015 1020
Thr Phe Asp Pro Glu Thr Asn Arg Phe Lys Pro Glu Pro Leu Lys Asp
1025 1030 1035 1040
Arg Gly Leu Ala Gly Val Arg Lys Asp Ser Val Phe Tyr Leu Glu Arg
1045 1050 1055
Lys Asp Asn Lys Pro Tyr Thr Val Thr Ile Asn Asp Ser Tyr Lys Tyr
1060 1065 1070
Val Ser Val Glu Asp Asn Lys Thr Phe Val Glu Arg Gln Ala Asp Gly
1075 1080 1085
Ser Phe Ile Leu Pro Leu Asp Lys Ala Lys Leu Gly Asp Phe Tyr Tyr
1090 1095 1100
Met Val Glu Asp Phe Ala Gly Asn Val Ala Ile Ala Lys Leu Gly Asp

1105 1110 1115 1120
His Leu Pro Gln Thr Leu Gly Lys Thr Pro Ile Lys Leu Lys Leu Thr
1125 1130 1135
Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp
1330 1335 1340
Asn Lys Val Tyr Ile Pro Lys Asn Pro Asp Gly Ser Tyr Thr Ile Ser
1345 1350 1355 1360
Lys Arg Asp Gly Val Thr Leu Ser Asp Tyr Tyr Tyr Leu Val Glu Asp
1365 1370 1375
Arg Ala Gly Asn Val Ser Phe Ala Thr Leu Arg Asp Leu Lys Ala Val
1380 1385 1390
Gly Lys Asp Lys Ala Val Val Asn Phe Gly Leu Asp Leu Pro Val Pro
1395 1400 1405
Glu Asp Lys Gln Ile Val Asn Phe Thr Tyr Leu Val Arg Asp Ala Asp
1410 1415 1420
Gly Lys Pro Ile Glu Asn Leu Glu Tyr Tyr Asn Asn Ser Gly Asn Ser
1425 1430 1435 1440
Leu Ile Leu Pro Tyr Gly Lys Tyr Thr Val Glu Leu Leu Thr Tyr Asp
1445 1450 1455
Thr Asn Ala Ala Lys Leu Glu Ser Asp Lys Ile Val Ser Phe Thr Leu
1460 1465 1470
Ser Ala Asp Asn Asn Phe Gln Gln Val Thr Phe Lys Met Thr Met Leu
1475 1480 1485
Ala Thr Ser Gln Ile Thr Ala His Phe Asp His Leu Leu Pro Glu Gly
1490 1495 1500
Ser Arg Val Ser Leu Lys Thr Ala Gln Gly Gln Leu Ile Pro Leu Glu
1505 1510 1515 1520
Gln Ser Leu Tyr Val Pro Lys Ala Tyr Gly Lys Thr Val Gln Glu Gly
1525 1530 1535
Thr Tyr Glu Val Val Val Ser Leu Pro Lys Gly Tyr Arg Ile Glu Gly
1540 1545 1550
Asn Thr Lys Val Asn Thr Leu Pro Asn Glu Val His Glu Leu Ser Leu
1555 1560 1565
Arg Leu Val Lys Val Gly Asp Ala Ser Asp Ser Thr Gly Asp His Lys
1570 1575 1580
Val Met Ser Lys Asn Asn Ser Gln Ala Leu Thr Ala Ser Ala Thr Pro
1585 1590 1595 1600

```
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 25
<211> 1645
<212> PRT
<213> Streptococcus pyogenes

<400> 25

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Gln Ser Pro Asp Thr Ser Gln Ile Thr Pro Lys Ile Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Asp Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Thr Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
```

```
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
```

865 870 875 880
Leu Gly Thr Phe Lys Asn Ala Asp Gly Lys Phe Ile Leu Glu Lys Asn
885 890 895
Ala Gln Gly Asn Pro Val Leu Ala Ile Ser Pro Asn Gly Asp Asn Asn
900 905 910
Gln Asp Phe Ala Ala Phe Lys Gly Val Phe Leu Arg Lys Tyr Gln Gly
915 920 925
Leu Lys Ala Ser Val Tyr His Ala Ser Asp Lys Glu His Lys Asn Pro
930 935 940
Leu Trp Val Ser Pro Glu Ser Phe Lys Gly Asp Lys Asn Phe Asn Ser
945 950 955 960
Asp Ile Arg Phe Ala Lys Ser Thr Thr Leu Leu Gly Thr Ala Phe Ser
965 970 975
Gly Lys Ser Leu Thr Gly Ala Glu Leu Pro Asp Gly Tyr Tyr His Tyr
980 985 990
Val Val Ser Tyr Tyr Pro Asp Val Val Gly Ala Lys Arg Gln Glu Met
995 1000 1005
Thr Phe Asp Met Ile Leu Asp Arg Gln Lys Pro Val Leu Ser Gln Ala
1010 1015 1020
Thr Phe Asp Pro Glu Thr Asn Arg Phe Lys Pro Glu Pro Leu Lys Asp
1025 1030 1035 1040
Arg Gly Leu Ala Gly Val Arg Lys Asp Ser Val Phe Tyr Leu Glu Arg
1045 1050 1055
Lys Asp Asn Lys Pro Tyr Thr Val Thr Ile Asn Asp Ser Tyr Lys Tyr
1060 1065 1070
Val Ser Val Glu Asp Asn Lys Thr Phe Val Glu Arg Gln Ala Asp Gly
1075 1080 1085
Ser Phe Ile Leu Pro Leu Asp Lys Ala Lys Leu Gly Asp Phe Tyr Tyr
1090 1095 1100
Met Val Glu Asp Phe Ala Gly Asn Val Ala Ile Ala Lys Leu Gly Asp
1105 1110 1115 1120
His Leu Pro Gln Thr Leu Gly Lys Thr Pro Ile Lys Leu Lys Leu Thr
1125 1130 1135
Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp
1330 1335 1340
Asn Lys Val Tyr Ile Pro Lys Asn Pro Asp Gly Ser Tyr Thr Ile Ser
1345 1350 1355 1360

```
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Thr Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 26
<211> 1645
<212> PRT
<213> Streptococcus pyogenes

<400> 26

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Gln Ser Pro Asp Thr Ser Gln Ile Thr Pro Lys Ile Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
```

```
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Asp Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Thr Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
```

```
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
```

His Leu Pro Gln Thr Leu Gly Lys Thr Pro Ile Lys Leu Lys Leu Thr
1125 1130 1135
Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp
1330 1335 1340
Asn Lys Val Tyr Ile Pro Lys Asn Pro Asp Gly Ser Tyr Thr Ile Ser
1345 1350 1355 1360
Lys Arg Asp Gly Val Thr Leu Ser Asp Tyr Tyr Tyr Leu Val Glu Asp
1365 1370 1375
Arg Ala Gly Asn Val Ser Phe Ala Thr Leu Arg Asp Leu Lys Ala Val
1380 1385 1390
Gly Lys Asp Lys Ala Val Val Asn Phe Gly Leu Asp Leu Pro Val Pro
1395 1400 1405
Glu Asp Lys Gln Ile Val Asn Phe Thr Tyr Leu Val Arg Asp Ala Asp
1410 1415 1420
Gly Lys Pro Ile Glu Asn Leu Glu Tyr Tyr Asn Asn Ser Gly Asn Ser
1425 1430 1435 1440
Leu Ile Leu Pro Tyr Gly Lys Tyr Thr Val Glu Leu Leu Thr Tyr Asp
1445 1450 1455
Thr Asn Ala Ala Lys Leu Glu Ser Asp Lys Ile Val Ser Phe Thr Leu
1460 1465 1470
Ser Ala Asp Asn Asn Phe Gln Gln Val Thr Phe Lys Met Thr Met Leu
1475 1480 1485
Ala Thr Ser Gln Ile Thr Ala His Phe Asp His Leu Leu Pro Glu Gly
1490 1495 1500
Ser Arg Val Ser Leu Lys Thr Ala Gln Gly Gln Leu Ile Pro Leu Glu
1505 1510 1515 1520
Gln Ser Leu Tyr Val Pro Lys Ala Tyr Gly Lys Thr Val Gln Glu Gly
1525 1530 1535
Thr Tyr Glu Val Val Val Ser Leu Pro Lys Gly Tyr Arg Ile Glu Gly
1540 1545 1550
Asn Thr Lys Val Asn Thr Leu Pro Asn Glu Val His Glu Leu Ser Leu
1555 1560 1565
Arg Leu Val Lys Val Gly Asp Ala Ser Asp Ser Thr Gly Asp His Lys
1570 1575 1580
Val Met Ser Lys Asn Asn Ser Gln Ala Leu Thr Ala Ser Ala Thr Pro
1585 1590 1595 1600
Thr Lys Thr Thr Thr Ser Ala Thr Ala Lys Ala Leu Pro Ser Ala Gly

```
                    1605                     1610                     1615
Glu Lys Met Gly Leu Lys Leu Arg Ile Val Gly Leu Val Leu Leu Gly
               1620                     1625                     1630
Leu Thr Cys Val Phe Ser Arg Lys Lys Ser Thr Lys Asp
          1635                     1640                     1645
```

<210> 27
<211> 1645
<212> PRT
<213> Streptococcus pyogenes

<400> 27

Val Glu Lys Lys Gln Arg Phe Ser Leu Arg Lys Tyr Lys Ser Gly Thr
1 5 10 15
Phe Ser Val Leu Ile Gly Ser Val Phe Leu Met Met Thr Thr Thr Val
20 25 30
Ala Ala Asp Glu Leu Thr Thr Thr Ser Glu Pro Thr Ile Thr Asn His
35 40 45
Ala Gln Gln Gln Ala Gln His Leu Thr Asn Thr Glu Leu Ser Ser Ala
50 55 60
Glu Ser Gln Ser Pro Asp Thr Ser Gln Ile Thr Pro Lys Ile Asn Arg
65 70 75 80
Glu Lys Glu Gln Pro Gln Gly Leu Val Ser Glu Pro Thr Thr Thr Glu
85 90 95
Leu Ala Asp Thr Asp Ala Ala Pro Met Ala Asn Thr Gly Pro Asp Ala
100 105 110
Thr Gln Lys Ser Ala Ser Leu Pro Pro Val Asn Thr Asp Val His Asp
115 120 125
Trp Val Lys Thr Lys Gly Ala Trp Asp Lys Gly Tyr Lys Gly Gln Gly
130 135 140
Lys Val Val Ala Val Ile Asp Thr Gly Ile Asp Pro Ala His Gln Ser
145 150 155 160
Met Arg Ile Ser Asp Val Ser Thr Ala Lys Val Lys Ser Lys Glu Asp
165 170 175
Met Leu Ala Arg Gln Lys Ala Ala Gly Ile Asn Tyr Gly Ser Trp Ile
180 185 190
Asn Asp Lys Val Val Phe Ala His Asn Tyr Val Glu Asn Ser Asp Asn
195 200 205
Ile Lys Glu Asn Gln Phe Glu Asp Phe Asp Glu Asp Trp Glu Asn Phe
210 215 220
Glu Phe Asp Ala Glu Pro Lys Ala Ile Lys Lys His Lys Ile Tyr Arg
225 230 235 240
Pro Gln Ser Thr Gln Ala Pro Lys Glu Thr Val Ile Lys Thr Glu Glu
245 250 255
Thr Asp Gly Ser His Asp Ile Asp Trp Thr Gln Thr Asp Asp Asp Thr
260 265 270
Lys Tyr Glu Ser His Gly Met His Val Thr Gly Ile Val Ala Gly Asn
275 280 285
Ser Lys Glu Ala Ala Ala Thr Gly Glu Arg Phe Leu Gly Ile Ala Pro
290 295 300
Glu Thr Gln Val Met Phe Met Arg Val Phe Ala Asn Asp Val Met Gly
305 310 315 320
Ser Ala Glu Ser Leu Phe Ile Lys Ala Ile Glu Asp Ala Val Ala Leu
325 330 335
Gly Ala Asp Val Ile Asn Leu Ser Leu Gly Thr Ala Asn Gly Ala Gln
340 345 350
Leu Ser Gly Ser Lys Pro Leu Met Glu Ala Ile Glu Lys Ala Lys Lys
355 360 365
Ala Gly Val Ser Val Val Val Ala Ala Gly Asn Glu Arg Val Tyr Gly
370 375 380
Ser Asp His Asp Asp Pro Leu Ala Thr Asn Pro Asp Tyr Gly Leu Val

385 390 395 400
Gly Ser Pro Ser Thr Gly Arg Thr Pro Thr Ser Val Ala Ala Ile Asn
405 410 415
Ser Lys Trp Val Ile Gln Arg Leu Met Thr Val Lys Glu Leu Glu Asn
420 425 430
Arg Ala Asp Leu Asn His Gly Lys Ala Ile Tyr Ser Glu Ser Val Asp
435 440 445
Phe Lys Asp Ile Lys Asp Ser Leu Gly Tyr Asp Lys Ser His Gln Phe
450 455 460
Ala Tyr Val Lys Glu Ser Thr Asp Ala Gly Tyr Asn Ala Gln Asp Val
465 470 475 480
Lys Gly Lys Ile Ala Leu Ile Glu Arg Asp Pro Asn Lys Thr Tyr Asp
485 490 495
Glu Met Ile Ala Leu Ala Lys Lys His Gly Ala Leu Gly Val Leu Ile
500 505 510
Phe Asn Asn Lys Pro Gly Gln Ser Asn Arg Ser Met Arg Leu Thr Ala
515 520 525
Asn Gly Met Gly Ile Pro Ser Ala Phe Ile Ser His Glu Phe Gly Lys
530 535 540
Ala Met Ser Gln Leu Asn Gly Asn Gly Thr Gly Ser Leu Glu Phe Asp
545 550 555 560
Ser Val Val Ser Lys Ala Pro Ser Gln Lys Gly Asn Glu Met Asn His
565 570 575
Phe Ser Asn Trp Gly Leu Thr Ser Asp Gly Tyr Leu Lys Pro Asp Ile
580 585 590
Thr Ala Pro Gly Gly Asp Ile Tyr Ser Thr Tyr Asn Asp Asn His Tyr
595 600 605
Gly Ser Gln Thr Gly Thr Ser Met Ala Ser Pro Gln Ile Ala Gly Ala
610 615 620
Ser Leu Leu Val Lys Gln Tyr Leu Glu Lys Thr Gln Pro Asn Leu Pro
625 630 635 640
Lys Glu Lys Ile Ala Asp Ile Val Lys Asn Leu Leu Met Ser Asn Ala
645 650 655
Gln Ile His Val Asn Pro Glu Thr Lys Thr Thr Thr Ser Pro Arg Gln
660 665 670
Gln Gly Ala Gly Leu Leu Asn Ile Asp Gly Ala Val Thr Ser Gly Leu
675 680 685
Tyr Val Thr Gly Lys Asp Asn Tyr Gly Ser Ile Ser Leu Gly Asn Ile
690 695 700
Thr Asp Thr Met Thr Phe Asp Val Thr Val His Asn Leu Ser Asn Lys
705 710 715 720
Ala Lys Thr Leu Arg Tyr Asp Thr Glu Leu Leu Thr Asp His Val Asp
725 730 735
Pro Gln Lys Gly Arg Phe Thr Leu Thr Ser Arg Ser Leu Lys Thr Tyr
740 745 750
Gln Gly Gly Glu Val Thr Val Pro Ala Asn Gly Lys Val Thr Val Arg
755 760 765
Val Thr Met Asp Val Ser Gln Phe Thr Lys Glu Leu Thr Lys Gln Met
770 775 780
Pro Asn Gly Tyr Tyr Leu Glu Gly Phe Val Arg Phe Arg Asp Ser Gln
785 790 795 800
Asp Asp Gln Leu Asn Arg Val Asn Ile Pro Phe Val Gly Phe Lys Gly
805 810 815
Gln Phe Glu Asn Leu Ala Val Ala Glu Glu Ser Ile Tyr Arg Leu Lys
820 825 830
Ser Gln Gly Lys Thr Gly Phe Tyr Phe Asp Glu Ser Gly Pro Lys Asp
835 840 845
Asp Ile Tyr Val Gly Lys His Phe Thr Gly Leu Val Thr Leu Gly Ser
850 855 860
Glu Thr Asn Val Ser Thr Lys Thr Ile Ser Asp Asn Gly Leu His Thr
865 870 875 880

```
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
```

```
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Thr Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 28
<211> 1645
<212> PRT
<213> Streptococcus pyogenes

<400> 28

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Gln Ser Pro Asp Thr Ser Gln Ile Thr Pro Lys Ile Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
```

```
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Asp Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Thr Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
```

```
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
```

```
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Thr Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Ser Ala Thr Pro
1585                1590                1595                1600
Thr Lys Thr Thr Thr Ser Ala Thr Ala Lys Ala Leu Pro Ser Ala Gly
                1605                1610                1615
```

```
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 29
<211> 1645
<212> PRT
<213> Streptococcus pyogenes

<400> 29

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Gln Ser Pro Asp Thr Ser Gln Ile Thr Pro Lys Ile Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Asp Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Thr Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
```

```
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
```

```
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
Asp Gly Asn Tyr Gln Thr Lys Glu Thr Leu Lys Asp Asn Leu Glu Met
            1140                1145                1150
Thr Gln Ser Asp Thr Gly Leu Val Thr Asn Gln Ala Gln Leu Ala Val
        1155                1160                1165
Val His Arg Asn Gln Pro Gln Ser Gln Leu Thr Lys Met Asn Gln Asp
    1170                1175                1180
Phe Phe Ile Ser Pro Asn Glu Asp Gly Asn Lys Asp Phe Val Ala Phe
1185                1190                1195                1200
Lys Gly Leu Lys Asn Asn Val Tyr Asn Asp Leu Thr Val Asn Val Tyr
                1205                1210                1215
Ala Lys Asp Asp His Gln Lys Gln Thr Pro Ile Trp Ser Ser Gln Ala
            1220                1225                1230
Gly Ala Ser Ala Ser Ala Ile Glu Ser Thr Ala Trp Tyr Gly Ile Thr
        1235                1240                1245
Ala Arg Gly Ser Lys Val Met Pro Gly Asp Tyr Gln Tyr Val Val Thr
    1250                1255                1260
Tyr Arg Asp Glu His Gly Lys Glu His Gln Lys Gln Tyr Thr Ile Ser
1265                1270                1275                1280
Val Asn Asp Lys Lys Pro Met Ile Thr Gln Gly Arg Phe Asp Thr Ile
                1285                1290                1295
Asn Gly Val Asp His Phe Thr Pro Asp Lys Thr Lys Ala Leu Gly Ser
            1300                1305                1310
Ser Gly Ile Val Arg Glu Glu Val Phe Tyr Leu Ala Lys Lys Asn Gly
        1315                1320                1325
Arg Lys Phe Asp Val Thr Glu Gly Lys Asp Gly Ile Thr Val Ser Asp
    1330                1335                1340
Asn Lys Val Tyr Ile Pro Lys Asn Pro Asp Gly Ser Tyr Thr Ile Ser
1345                1350                1355                1360
Lys Arg Asp Gly Val Thr Leu Ser Asp Tyr Tyr Tyr Leu Val Glu Asp
                1365                1370                1375
```

```
Arg Ala Gly Asn Val Ser Phe Ala Thr Leu Arg Asp Leu Lys Ala Val
            1380                1385                1390
Gly Lys Asp Lys Ala Val Val Asn Phe Gly Leu Asp Leu Pro Val Pro
        1395                1400                1405
Glu Asp Lys Gln Ile Val Asn Phe Thr Tyr Leu Val Arg Asp Ala Asp
    1410                1415                1420
Gly Lys Pro Ile Glu Asn Leu Glu Tyr Tyr Asn Asn Ser Gly Asn Ser
1425                1430                1435                1440
Leu Ile Leu Pro Tyr Gly Lys Tyr Thr Val Glu Leu Leu Thr Tyr Asp
                1445                1450                1455
Thr Asn Ala Ala Lys Leu Glu Ser Asp Lys Ile Val Ser Phe Thr Leu
            1460                1465                1470
Ser Ala Asp Asn Asn Phe Gln Gln Val Thr Phe Lys Met Thr Met Leu
        1475                1480                1485
Ala Thr Ser Gln Ile Thr Ala His Phe Asp His Leu Leu Pro Glu Gly
    1490                1495                1500
Ser Arg Val Ser Leu Lys Thr Ala Gln Gly Gln Leu Ile Pro Leu Glu
1505                1510                1515                1520
Gln Ser Leu Tyr Val Pro Lys Ala Tyr Gly Lys Thr Val Gln Glu Gly
                1525                1530                1535
Thr Tyr Glu Val Val Val Ser Leu Pro Lys Gly Tyr Arg Ile Glu Gly
            1540                1545                1550
Asn Thr Lys Val Asn Thr Leu Pro Asn Glu Val His Glu Leu Ser Leu
        1555                1560                1565
Arg Leu Val Lys Val Gly Asp Ala Ser Asp Ser Thr Gly Asp His Lys
    1570                1575                1580
Val Met Ser Lys Asn Asn Ser Gln Ala Leu Thr Ala Phe Ala Thr Pro
1585                1590                1595                1600
Thr Lys Thr Thr Thr Ser Ala Thr Ala Lys Ala Leu Pro Ser Ala Gly
                1605                1610                1615
Glu Lys Met Gly Leu Lys Leu Arg Ile Val Gly Leu Val Leu Leu Gly
            1620                1625                1630
Leu Thr Cys Val Phe Ser Arg Lys Lys Ser Thr Lys Asp
        1635                1640                1645
```

<210> 30
<211> 1645
<212> PRT
<213> Streptococcus pyogenes

<400> 30

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Ala Gln Gln Gln Ala Gln His Leu Thr Asn Thr Glu Leu Ser Ser Ala
    50                  55                  60
Glu Ser Gln Ser Pro Asp Thr Ser Gln Ile Thr Pro Lys Ile Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
```

```
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Pro Lys Ala Ile Lys Lys His Lys Ile Tyr Arg
225                 230                 235                 240
Pro Gln Ser Thr Gln Ala Pro Lys Glu Thr Val Ile Lys Thr Glu Glu
                245                 250                 255
Thr Asp Gly Ser His Asp Ile Asp Trp Thr Gln Thr Asp Asp Asp Thr
            260                 265                 270
Lys Tyr Glu Ser His Gly Met His Val Thr Gly Ile Val Ala Gly Asn
        275                 280                 285
Ser Lys Glu Ala Ala Ala Thr Gly Glu Arg Phe Leu Gly Ile Ala Pro
    290                 295                 300
Glu Thr Gln Val Met Phe Met Arg Val Phe Ala Asn Asp Val Met Gly
305                 310                 315                 320
Ser Ala Glu Ser Leu Phe Ile Lys Ala Ile Glu Asp Ala Val Ala Leu
                325                 330                 335
Gly Ala Asp Val Ile Asn Leu Ser Leu Gly Thr Ala Asn Gly Ala Gln
            340                 345                 350
Leu Ser Gly Ser Lys Pro Leu Met Glu Ala Ile Glu Lys Ala Lys Lys
        355                 360                 365
Ala Gly Val Ser Val Val Val Ala Ala Gly Asn Glu Arg Val Tyr Gly
    370                 375                 380
Ser Asp His Asp Asp Pro Leu Ala Thr Asn Pro Asp Tyr Gly Leu Val
385                 390                 395                 400
Gly Ser Pro Ser Thr Gly Arg Thr Pro Thr Ser Val Ala Ala Ile Asn
                405                 410                 415
Ser Lys Trp Val Ile Gln Arg Leu Met Thr Val Lys Glu Leu Glu Asn
            420                 425                 430
Arg Ala Asp Leu Asn His Gly Lys Ala Ile Tyr Ser Glu Ser Val Asp
        435                 440                 445
Phe Lys Asp Ile Lys Asp Ser Leu Gly Tyr Asp Lys Ser His Gln Phe
    450                 455                 460
Ala Tyr Val Lys Glu Ser Thr Asp Ala Gly Tyr Asn Ala Gln Asp Val
465                 470                 475                 480
Lys Gly Lys Ile Ala Leu Ile Glu Arg Asp Pro Asn Lys Thr Tyr Asp
                485                 490                 495
Glu Met Ile Ala Leu Ala Lys Lys His Gly Ala Leu Gly Val Leu Ile
            500                 505                 510
Phe Asn Asn Lys Pro Gly Gln Ser Asn Arg Ser Met Arg Leu Thr Ala
        515                 520                 525
Asn Gly Met Gly Ile Pro Ser Ala Phe Ile Ser His Glu Phe Gly Lys
    530                 535                 540
Ala Met Ser Gln Leu Asn Gly Asn Gly Thr Gly Ser Leu Glu Phe Asp
545                 550                 555                 560
Ser Val Val Ser Lys Ala Pro Ser Gln Lys Gly Asn Glu Met Asn His
                565                 570                 575
Phe Ser Asn Trp Gly Leu Thr Ser Asp Gly Tyr Leu Lys Pro Asp Ile
            580                 585                 590
Thr Ala Pro Gly Gly Asp Ile Tyr Ser Thr Tyr Asn Asp Asn His Tyr
        595                 600                 605
Gly Ser Gln Thr Gly Thr Ser Met Ala Ser Pro Gln Ile Ala Gly Ala
    610                 615                 620
Ser Leu Leu Val Lys Gln Tyr Leu Glu Lys Thr Gln Pro Asn Leu Pro
625                 630                 635                 640
Lys Glu Lys Ile Ala Asp Ile Val Lys Asn Leu Leu Met Ser Asn Ala
```

```
                645                 650                 655
Gln Ile His Val Asn Pro Glu Thr Lys Thr Thr Thr Ser Pro Arg Gln
            660                 665                 670
Gln Gly Ala Gly Leu Leu Asn Ile Asp Gly Ala Val Thr Ser Gly Leu
        675                 680                 685
Tyr Val Thr Gly Lys Asp Asn Tyr Gly Ser Ile Ser Leu Gly Asn Ile
    690                 695                 700
Thr Asp Thr Met Thr Phe Asp Val Thr Val His Asn Leu Ser Asn Lys
705                 710                 715                 720
Ala Lys Thr Leu Arg Tyr Asp Thr Glu Leu Leu Thr Asp His Val Asp
                725                 730                 735
Pro Gln Lys Gly Arg Phe Thr Leu Thr Ser Arg Ser Leu Lys Thr Tyr
            740                 745                 750
Gln Gly Gly Glu Val Thr Val Pro Ala Asn Gly Lys Val Thr Val Arg
        755                 760                 765
Val Thr Met Asp Val Ser Gln Phe Thr Lys Glu Leu Thr Lys Gln Met
    770                 775                 780
Pro Asn Gly Tyr Tyr Leu Glu Gly Phe Val Arg Phe Arg Asp Ser Gln
785                 790                 795                 800
Asp Asp Gln Leu Asn Arg Val Asn Ile Pro Phe Val Gly Phe Lys Gly
                805                 810                 815
Gln Phe Glu Asn Leu Ala Val Ala Glu Glu Ser Ile Tyr Arg Leu Lys
            820                 825                 830
Ser Gln Gly Lys Thr Gly Phe Tyr Phe Asp Glu Ser Gly Pro Lys Asp
        835                 840                 845
Asp Ile Tyr Val Gly Lys His Phe Thr Gly Leu Val Thr Leu Gly Ser
    850                 855                 860
Glu Thr Asn Val Ser Thr Lys Thr Ile Ser Asp Asn Gly Leu His Thr
865                 870                 875                 880
Leu Gly Thr Phe Lys Asn Ala Asp Gly Lys Phe Ile Leu Glu Lys Asn
                885                 890                 895
Ala Gln Gly Asn Pro Val Leu Ala Ile Ser Pro Asn Gly Asp Asn Asn
            900                 905                 910
Gln Asp Phe Ala Ala Phe Lys Gly Val Phe Leu Arg Lys Tyr Gln Gly
        915                 920                 925
Leu Lys Ala Ser Val Tyr His Ala Ser Asp Lys Glu His Lys Asn Pro
    930                 935                 940
Leu Trp Val Ser Pro Glu Ser Phe Lys Gly Asp Lys Asn Phe Asn Ser
945                 950                 955                 960
Asp Ile Arg Phe Ala Lys Ser Thr Thr Leu Leu Gly Thr Ala Phe Ser
                965                 970                 975
Gly Lys Ser Leu Thr Gly Ala Glu Leu Pro Asp Gly Tyr Tyr His Tyr
            980                 985                 990
Val Val Ser Tyr Tyr Pro Asp Val Val Gly Ala Lys Arg Gln Glu Met
        995                 1000                1005
Thr Phe Asp Met Ile Leu Asp Arg Gln Lys Pro Val Leu Ser Gln Ala
    1010                1015                1020
Thr Phe Asp Pro Glu Thr Asn Arg Phe Lys Pro Glu Pro Leu Lys Asp
1025                1030                1035                1040
Arg Gly Leu Ala Gly Val Arg Lys Asp Ser Val Phe Tyr Leu Glu Arg
                1045                1050                1055
Lys Asp Asn Lys Pro Tyr Thr Val Thr Ile Asn Asp Ser Tyr Lys Tyr
            1060                1065                1070
Val Ser Val Glu Asp Asn Lys Thr Phe Val Glu Arg Gln Ala Asp Gly
        1075                1080                1085
Ser Phe Ile Leu Pro Leu Asp Lys Ala Lys Leu Gly Asp Phe Tyr Tyr
    1090                1095                1100
Met Val Glu Asp Phe Ala Gly Asn Val Ala Ile Ala Lys Leu Gly Asp
1105                1110                1115                1120
His Leu Pro Gln Thr Leu Gly Lys Thr Pro Ile Lys Leu Lys Leu Thr
                1125                1130                1135
```

Asp Gly Asn Tyr Gln Thr Lys Glu Thr Leu Lys Asp Asn Leu Glu Met
1140 1145 1150
Thr Gln Ser Asp Thr Gly Leu Val Thr Asn Gln Ala Gln Leu Ala Val
1155 1160 1165
Val His Arg Asn Gln Pro Gln Ser Gln Leu Thr Lys Met Asn Gln Asp
1170 1175 1180
Phe Phe Ile Ser Pro Asn Glu Asp Gly Asn Lys Asp Phe Val Ala Phe
1185 1190 1195 1200
Lys Gly Leu Lys Asn Asn Val Tyr Asn Asp Leu Thr Val Asn Val Tyr
1205 1210 1215
Ala Lys Asp Asp His Gln Lys Gln Thr Pro Ile Trp Ser Ser Gln Ala
1220 1225 1230
Gly Ala Ser Ala Ser Ala Ile Glu Ser Thr Ala Trp Tyr Gly Ile Thr
1235 1240 1245
Ala Arg Gly Ser Lys Val Met Pro Gly Asp Tyr Gln Tyr Val Val Thr
1250 1255 1260
Tyr Arg Asp Glu His Gly Lys Glu His Gln Lys Gln Tyr Thr Ile Ser
1265 1270 1275 1280
Val Asn Asp Lys Lys Pro Met Ile Thr Gln Gly Arg Phe Asp Thr Ile
1285 1290 1295
Asn Gly Val Asp His Phe Thr Pro Asp Lys Thr Lys Ala Leu Gly Ser
1300 1305 1310
Ser Gly Ile Val Arg Glu Glu Val Phe Tyr Leu Ala Lys Lys Asn Gly
1315 1320 1325
Arg Lys Phe Asp Val Thr Glu Gly Lys Asp Gly Ile Thr Val Ser Asp
1330 1335 1340
Asn Lys Val Tyr Ile Pro Lys Asn Pro Asp Gly Ser Tyr Thr Ile Ser
1345 1350 1355 1360
Lys Arg Asp Gly Val Thr Leu Ser Asp Tyr Tyr Tyr Leu Val Glu Asp
1365 1370 1375
Arg Ala Gly Asn Val Ser Phe Ala Thr Leu Arg Asp Leu Lys Ala Val
1380 1385 1390
Gly Lys Asp Lys Ala Val Val Asn Phe Gly Leu Asp Leu Pro Val Pro
1395 1400 1405
Glu Asp Lys Gln Ile Val Asn Phe Thr Tyr Leu Val Arg Asp Ala Asp
1410 1415 1420
Gly Lys Pro Ile Glu Asn Leu Glu Tyr Tyr Asn Asn Ser Gly Asn Ser
1425 1430 1435 1440
Leu Ile Leu Pro Tyr Gly Lys Tyr Thr Val Glu Leu Leu Thr Tyr Asp
1445 1450 1455
Thr Asn Ala Ala Lys Leu Glu Ser Asp Lys Ile Val Ser Phe Thr Leu
1460 1465 1470
Ser Ala Asp Asn Asn Phe Gln Gln Val Thr Phe Lys Met Thr Met Leu
1475 1480 1485
Ala Thr Ser Gln Ile Thr Ala His Phe Asp His Leu Leu Pro Glu Gly
1490 1495 1500
Ser Arg Val Ser Leu Lys Thr Ala Gln Gly Gln Leu Ile Pro Leu Glu
1505 1510 1515 1520
Gln Ser Leu Tyr Val Pro Lys Ala Tyr Gly Lys Thr Val Gln Glu Gly
1525 1530 1535
Thr Tyr Glu Val Val Val Ser Leu Pro Lys Gly Tyr Arg Ile Glu Gly
1540 1545 1550
Asn Thr Lys Val Asn Thr Leu Pro Asn Glu Val His Glu Leu Ser Leu
1555 1560 1565
Arg Leu Val Lys Val Gly Asp Ala Ser Asp Ser Thr Gly Asp His Lys
1570 1575 1580
Val Met Ser Lys Asn Asn Ser Gln Ala Leu Thr Ala Ser Ala Thr Pro
1585 1590 1595 1600
Thr Lys Thr Thr Thr Ser Ala Thr Ala Lys Ala Leu Pro Ser Ala Gly
1605 1610 1615
Glu Lys Met Gly Leu Lys Leu Arg Ile Val Gly Leu Val Leu Leu Gly

1620 1625 1630
Leu Thr Cys Val Phe Ser Arg Lys Lys Ser Thr Lys Asp
1635 1640 1645

<210> 31
<211> 1648
<212> PRT
<213> Streptococcus pyogenes

<400> 31

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Ala Gln Gln Gln Ala Gln His Leu Thr Asn Thr Glu Leu Ser Ser
    50                  55                  60
Ala Glu Ser Lys Pro Gln Asp Thr Ser Gln Ile Thr Pro Lys Thr Asn
65                  70                  75                  80
Arg Glu Lys Glu Gln Ser Gln Asp Leu Val Ser Glu Pro Thr Thr Thr
                85                  90                  95
Glu Leu Ala Asp Thr Asp Ala Ala Ser Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
```

```
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ser Asn Gly Met Gly Ile Pro Ser Ala Phe Ile Ser His Glu
    530                 535                 540
Phe Gly Lys Ala Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Thr Ile Ser Asp Asn Gly
865                 870                 875                 880
Leu His Thr Leu Gly Thr Phe Lys Asn Ala Asp Gly Lys Phe Ile Leu
                885                 890                 895
```

Glu Lys Asn Ala Gln Gly Asn Pro Val Leu Ala Ile Ser Pro Asn Gly
900 905 910
Asp Asn Asn Gln Asp Phe Ala Ala Phe Lys Gly Val Phe Leu Arg Lys
915 920 925
Tyr Gln Gly Leu Lys Ala Ser Val Tyr His Ala Ser Asp Lys Glu His
930 935 940
Lys Asn Pro Leu Trp Val Ser Pro Glu Ser Phe Lys Gly Asp Lys Asn
945 950 955 960
Phe Asn Ser Asp Ile Arg Phe Ala Lys Ser Thr Thr Leu Leu Gly Thr
965 970 975
Ala Phe Ser Gly Lys Ser Leu Thr Gly Ala Glu Leu Pro Asp Gly Tyr
980 985 990
Tyr His Tyr Val Val Ser Tyr Tyr Pro Asp Val Val Gly Ala Lys Arg
995 1000 1005
Gln Glu Met Thr Phe Asp Met Ile Leu Asp Arg Gln Lys Pro Val Leu
1010 1015 1020
Ser Gln Ala Thr Phe Asp Pro Glu Thr Asn Arg Phe Lys Pro Glu Pro
1025 1030 1035 1040
Leu Lys Asp Arg Gly Leu Ala Gly Val Arg Lys Asp Ser Val Phe Tyr
1045 1050 1055
Leu Glu Arg Lys Asp Asn Lys Pro Tyr Thr Val Thr Ile Asn Asp Ser
1060 1065 1070
Tyr Lys Tyr Val Ser Val Glu Asp Asn Lys Thr Phe Val Glu Arg Gln
1075 1080 1085
Ala Asp Gly Ser Phe Ile Leu Pro Leu Asp Lys Ala Lys Leu Gly Asp
1090 1095 1100
Phe Tyr Tyr Met Val Glu Asp Phe Ala Gly Asn Val Ala Ile Ala Lys
1105 1110 1115 1120
Leu Gly Asp His Leu Pro Gln Thr Leu Gly Lys Thr Pro Ile Lys Leu
1125 1130 1135
Lys Leu Thr Asp Gly Asn Tyr Gln Thr Lys Glu Thr Leu Lys Asp Asn
1140 1145 1150
Leu Glu Met Thr Gln Ser Asp Thr Gly Leu Val Thr Asn Gln Ala Gln
1155 1160 1165
Leu Ala Val Val His Arg Asn Gln Pro Gln Ser Gln Leu Thr Lys Met
1170 1175 1180
Asn Gln Asp Phe Phe Ile Ser Pro Asn Glu Asp Gly Asn Lys Asp Phe
1185 1190 1195 1200
Val Ala Phe Lys Gly Leu Lys Asn Asn Val Tyr Asn Asp Leu Thr Val
1205 1210 1215
Asn Val Tyr Ala Lys Asp Asp His Gln Lys Gln Thr Pro Ile Trp Ser
1220 1225 1230
Ser Gln Ala Gly Ala Ser Ala Ser Ala Ile Glu Ser Thr Ala Trp Tyr
1235 1240 1245
Gly Ile Thr Ala Arg Gly Ser Lys Val Met Pro Gly Asp Tyr Gln Tyr
1250 1255 1260
Val Val Thr Tyr Arg Asp Glu His Gly Lys Glu His Gln Lys Gln Tyr
1265 1270 1275 1280
Thr Ile Ser Val Asn Asp Lys Lys Pro Met Ile Thr Gln Gly Arg Phe
1285 1290 1295
Asp Thr Ile Asn Gly Val Asp His Phe Thr Pro Asp Lys Thr Lys Ala
1300 1305 1310
Leu Gly Ser Ser Gly Ile Val Arg Glu Glu Val Phe Tyr Leu Ala Lys
1315 1320 1325
Lys Asn Gly Arg Lys Phe Asp Val Thr Glu Gly Lys Asp Gly Ile Thr
1330 1335 1340
Val Ser Asp Asn Lys Val Tyr Ile Pro Lys Asn Pro Asp Gly Ser Tyr
1345 1350 1355 1360
Thr Ile Ser Lys Arg Asp Gly Val Thr Leu Ser Asp Tyr Tyr Tyr Leu
1365 1370 1375
Val Glu Asp Arg Ala Gly Asn Val Ser Phe Ala Thr Leu Arg Asp Leu

```
            1380                1385                1390
Lys Ala Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Ile Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asn Thr Lys Val Asn Thr Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
                1605                1610                1615
Ser Ala Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 32
<211> 1649
<212> PRT
<213> Streptococcus pyogenes

<400> 32

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Met Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Lys Pro Gln Asp Thr Ser Gln Ile Thr Pro Lys Thr
65                  70                  75                  80
Asn Arg Glu Lys Glu Gln Ser Gln Asp Leu Val Ser Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Gly Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
```

```
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Glu Asp Phe Asp Glu Asp Trp Glu
    210                 215                 220
Asn Phe Glu Phe Asp Ala Asp Ala Glu Pro Lys Ala Ile Lys Lys His
225                 230                 235                 240
Lys Ile Tyr Arg Pro Gln Ser Thr Gln Ala Pro Lys Glu Thr Val Ile
                245                 250                 255
Lys Thr Glu Glu Thr Asp Gly Ser His Asp Ile Asp Trp Thr Gln Thr
            260                 265                 270
Asp Asp Asp Thr Lys Tyr Glu Ser His Gly Met His Val Thr Gly Ile
        275                 280                 285
Val Ala Gly Asn Ser Lys Glu Ala Ala Ala Thr Gly Glu Arg Phe Leu
    290                 295                 300
Gly Ile Ala Pro Glu Ala Gln Val Met Phe Met Arg Val Phe Ala Asn
305                 310                 315                 320
Asp Val Met Gly Ser Ala Glu Ser Leu Phe Ile Lys Ala Ile Glu Asp
                325                 330                 335
Ala Val Ala Leu Gly Ala Asp Val Ile Asn Leu Ser Leu Gly Thr Ala
            340                 345                 350
Asn Gly Ala Gln Leu Ser Gly Ser Lys Pro Leu Met Glu Ala Ile Glu
        355                 360                 365
Lys Ala Lys Lys Ala Gly Val Ser Val Val Val Ala Ala Gly Asn Glu
    370                 375                 380
Arg Val Tyr Gly Ser Asp His Asp Asp Pro Leu Ala Thr Asn Pro Asp
385                 390                 395                 400
Tyr Gly Leu Val Gly Ser Pro Ser Thr Gly Arg Thr Pro Thr Ser Val
                405                 410                 415
Ala Ala Ile Asn Ser Lys Trp Val Ile Gln Arg Leu Met Thr Val Lys
            420                 425                 430
Glu Leu Glu Asn Arg Ala Asp Leu Asn His Gly Lys Ala Ile Tyr Ser
        435                 440                 445
Glu Ser Val Asp Phe Lys Asn Ile Lys Asp Ser Leu Gly Tyr Asp Lys
    450                 455                 460
Ser His Gln Phe Ala Tyr Val Lys Glu Ser Thr Asp Ala Gly Tyr Asn
465                 470                 475                 480
Ala Gln Asp Val Lys Gly Lys Ile Ala Leu Ile Glu Arg Asp Pro Asn
                485                 490                 495
Lys Thr Tyr Asp Glu Met Ile Ala Leu Ala Lys Lys His Gly Ala Leu
            500                 505                 510
Gly Val Leu Ile Phe Asn Asn Lys Pro Gly Gln Ser Asn Arg Ser Met
        515                 520                 525
Arg Leu Thr Ala Asn Gly Met Gly Ile Pro Ser Ala Phe Ile Ser His
    530                 535                 540
Glu Phe Gly Lys Ala Met Ser Gln Leu Asn Gly Asn Gly Thr Gly Ser
545                 550                 555                 560
Leu Glu Phe Asp Ser Val Val Ser Lys Ala Pro Ser Gln Lys Gly Asn
                565                 570                 575
Glu Met Asn His Phe Ser Asn Trp Gly Leu Thr Ser Asp Gly Tyr Leu
            580                 585                 590
Lys Pro Asp Ile Thr Ala Pro Gly Gly Asp Ile Tyr Ser Thr Tyr Asn
        595                 600                 605
Asp Asn His Tyr Gly Ser Gln Thr Gly Thr Ser Met Ala Ser Pro Gln
    610                 615                 620
Ile Ala Gly Ala Ser Leu Leu Val Lys Gln Tyr Leu Glu Lys Thr Gln
625                 630                 635                 640
Pro Asn Leu Pro Lys Glu Lys Ile Ala Asp Ile Val Lys Asn Leu Leu
                645                 650                 655
```

```
Met Ser Asn Ala Gln Ile His Val Asn Pro Glu Thr Lys Thr Thr Thr
            660                 665                 670
Ser Pro Arg Gln Gln Gly Ala Gly Leu Leu Asn Ile Asp Gly Ala Val
        675                 680                 685
Thr Ser Gly Leu Tyr Val Thr Gly Lys Asp Asn Tyr Gly Ser Ile Ser
    690                 695                 700
Leu Gly Asn Ile Thr Asp Thr Met Thr Phe Asp Val Thr Val His Asn
705                 710                 715                 720
Leu Ser Asn Lys Asp Lys Thr Leu Arg Tyr Asp Thr Glu Leu Leu Thr
                725                 730                 735
Asp His Val Asp Pro Gln Lys Gly Arg Phe Thr Leu Thr Ser Arg Ser
            740                 745                 750
Leu Lys Thr Tyr Gln Gly Gly Glu Val Thr Val Pro Ala Asn Gly Lys
        755                 760                 765
Val Thr Val Arg Val Thr Met Asp Val Ser Gln Phe Thr Lys Glu Leu
    770                 775                 780
Thr Lys Gln Met Pro Asn Gly Tyr Tyr Leu Glu Gly Phe Val Arg Phe
785                 790                 795                 800
Arg Asp Ser Gln Asp Asp Gln Leu Asn Arg Val Asn Ile Pro Phe Val
                805                 810                 815
Gly Phe Lys Gly Gln Phe Glu Asn Leu Ala Val Ala Glu Glu Ser Ile
            820                 825                 830
Tyr Arg Leu Lys Ser Gln Gly Lys Thr Gly Phe Tyr Phe Asp Glu Ser
        835                 840                 845
Gly Pro Lys Asp Asp Ile Tyr Val Gly Lys His Phe Thr Gly Leu Val
    850                 855                 860
Thr Leu Gly Ser Glu Thr Asn Val Ser Thr Lys Thr Ile Ser Asp Asn
865                 870                 875                 880
Gly Leu His Thr Leu Gly Thr Phe Lys Asn Ala Asp Gly Lys Phe Ile
                885                 890                 895
Leu Glu Lys Asn Ala Gln Gly Asn Pro Val Leu Ala Ile Ser Pro Asn
            900                 905                 910
Gly Asp Asn Asn Gln Asp Phe Ala Ala Phe Lys Gly Val Phe Leu Arg
        915                 920                 925
Lys Tyr Gln Gly Leu Lys Ala Ser Val Tyr His Ala Ser Asp Lys Glu
    930                 935                 940
His Lys Asn Pro Leu Trp Val Ser Pro Glu Ser Phe Lys Gly Asp Lys
945                 950                 955                 960
Asn Phe Asn Ser Asp Ile Arg Phe Ala Lys Ser Thr Thr Leu Leu Gly
                965                 970                 975
Thr Ala Phe Ser Gly Lys Ser Leu Thr Gly Ala Glu Leu Pro Asp Gly
            980                 985                 990
Tyr Tyr His Tyr Val Val Ser Tyr Tyr Pro Asp Val Val Gly Ala Lys
        995                 1000                1005
Arg Gln Glu Met Thr Phe Asp Met Ile Leu Asp Arg Gln Lys Pro Val
    1010                1015                1020
Leu Ser Gln Ala Thr Phe Asp Pro Glu Thr Asn Arg Phe Lys Pro Glu
1025                1030                1035                1040
Pro Leu Lys Asp Arg Gly Leu Ala Gly Val Arg Lys Asp Ser Val Phe
                1045                1050                1055
Tyr Leu Glu Arg Lys Asp Asn Lys Pro Tyr Thr Val Thr Ile Asn Asp
            1060                1065                1070
Ser Tyr Lys Tyr Val Ser Val Glu Asp Asn Lys Thr Phe Val Glu Arg
        1075                1080                1085
Gln Ala Asp Gly Ser Phe Ile Leu Pro Leu Asp Lys Ala Lys Leu Gly
    1090                1095                1100
Asp Phe Tyr Tyr Met Val Glu Asp Phe Ala Gly Asn Val Ala Ile Ala
1105                1110                1115                1120
Lys Leu Gly Asp His Leu Pro Gln Thr Leu Gly Lys Thr Pro Ile Lys
                1125                1130                1135
Leu Lys Leu Thr Asp Gly Asn Tyr Gln Thr Lys Glu Thr Leu Lys Asp
```

```
            1140                1145                1150
Asn Leu Glu Met Thr Gln Ser Asp Thr Gly Leu Val Thr Asn Gln Ala
        1155                1160                1165
Gln Leu Ala Val Val His Arg Asn Gln Pro Gln Ser Gln Leu Thr Lys
    1170                1175                1180
Met Asn Gln Asp Phe Phe Ile Ser Pro Asn Glu Asp Gly Asn Lys Asp
1185                1190                1195                1200
Phe Val Ala Phe Lys Gly Leu Lys Asn Asn Val Tyr Asn Asp Leu Thr
                1205                1210                1215
Val Asn Val Tyr Ala Lys Asp Asp His Gln Lys Gln Thr Pro Ile Trp
            1220                1225                1230
Ser Ser Gln Ala Gly Ala Ser Ala Ser Ala Ile Glu Ser Thr Ala Trp
        1235                1240                1245
Tyr Gly Ile Thr Ala Arg Gly Ser Lys Val Met Pro Gly Asp Tyr Gln
    1250                1255                1260
Tyr Val Val Thr Tyr Arg Asp Glu His Gly Lys Val His Gln Lys Gln
1265                1270                1275                1280
Tyr Thr Ile Ser Val Asn Asp Lys Lys Pro Met Ile Thr Gln Gly Arg
                1285                1290                1295
Phe Asp Thr Ile Asn Gly Val Asp His Phe Thr Pro Asp Lys Thr Lys
            1300                1305                1310
Ala Leu Gly Ser Ser Gly Ile Val Arg Glu Glu Val Phe Tyr Leu Ala
        1315                1320                1325
Lys Lys Asn Gly Arg Lys Phe Asp Val Thr Glu Gly Lys Asp Gly Ile
    1330                1335                1340
Thr Val Ser Asp Asn Lys Val Tyr Ile Pro Lys Asn Pro Asp Gly Ser
1345                1350                1355                1360
Tyr Thr Ile Ser Lys Arg Asp Gly Val Thr Leu Ser Asp Tyr Tyr Tyr
                1365                1370                1375
Leu Val Glu Asp Arg Ala Gly Asn Val Ser Phe Ala Thr Leu Arg Asp
            1380                1385                1390
Leu Lys Ala Val Gly Lys Asp Lys Ala Val Val Asn Phe Gly Leu Asp
        1395                1400                1405
Leu Pro Val Pro Glu Asp Lys Gln Ile Val Asn Phe Thr Tyr Leu Val
    1410                1415                1420
Arg Asp Ala Asp Gly Lys Pro Ile Glu Asn Leu Glu Tyr Tyr Asn Asn
1425                1430                1435                1440
Ser Gly Asn Ser Leu Ile Leu Pro Tyr Gly Lys Tyr Thr Val Glu Leu
                1445                1450                1455
Leu Thr Tyr Asp Thr Asn Ala Ala Lys Leu Glu Ser Asp Lys Ile Val
            1460                1465                1470
Ser Phe Thr Leu Ser Ala Asp Asn Asn Phe Gln Gln Val Thr Phe Lys
        1475                1480                1485
Met Thr Met Leu Ala Thr Ser Gln Ile Thr Ala His Phe Asp His Leu
    1490                1495                1500
Leu Pro Glu Gly Ser Arg Val Ser Leu Lys Thr Ala Gln Gly Gln Leu
1505                1510                1515                1520
Ile Pro Leu Glu Gln Ser Leu Tyr Val Pro Lys Ala Tyr Gly Lys Thr
                1525                1530                1535
Val Gln Glu Gly Thr Tyr Glu Val Val Val Ser Leu Pro Lys Gly Tyr
            1540                1545                1550
Arg Ile Glu Gly Asn Thr Lys Val Asn Thr Leu Pro Asn Glu Val His
        1555                1560                1565
Glu Leu Ser Leu Arg Leu Val Lys Val Gly Asp Ala Ser Asp Ser Thr
    1570                1575                1580
Gly Asp His Lys Val Met Ser Lys Asn Asn Ser Gln Ala Leu Thr Ala
1585                1590                1595                1600
Ser Ala Thr Pro Thr Lys Thr Thr Thr Ser Ala Thr Ala Lys Ala Leu
                1605                1610                1615
Pro Ser Thr Gly Glu Lys Met Gly Leu Lys Leu Arg Ile Val Gly Leu
            1620                1625                1630
```

```
Val Leu Leu Gly Leu Thr Cys Val Phe Ser Arg Lys Lys Ser Thr Lys
        1635                    1640                    1645
Asp
```

<210> 33
<211> 1649
<212> PRT
<213> Streptococcus pyogenes

<400> 33

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Met Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Lys Pro Gln Asp Thr Ser Gln Ile Thr Pro Lys Thr
65                  70                  75                  80
Asn Arg Glu Lys Glu Gln Ser Gln Asp Leu Val Ser Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Gly Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Glu Asp Phe Asp Glu Asp Trp Glu
    210                 215                 220
Asn Phe Glu Phe Asp Ala Asp Ala Glu Pro Lys Ala Ile Lys Lys His
225                 230                 235                 240
Lys Ile Tyr Arg Pro Gln Ser Thr Gln Ala Pro Lys Glu Thr Val Ile
                245                 250                 255
Lys Thr Glu Glu Thr Asp Gly Ser His Asp Ile Asp Trp Thr Gln Thr
            260                 265                 270
Asp Asp Asp Thr Lys Tyr Glu Ser His Gly Met His Val Thr Gly Ile
        275                 280                 285
Val Ala Gly Asn Ser Lys Glu Ala Ala Ala Thr Gly Glu Arg Phe Leu
    290                 295                 300
Gly Ile Ala Pro Glu Ala Gln Val Met Phe Met Arg Val Phe Ala Asn
305                 310                 315                 320
Asp Val Met Gly Ser Ala Glu Ser Leu Phe Ile Lys Ala Ile Glu Asp
                325                 330                 335
Ala Val Ala Leu Gly Ala Asp Val Ile Asn Leu Ser Leu Gly Thr Ala
            340                 345                 350
Asn Gly Ala Gln Leu Ser Gly Ser Lys Pro Leu Met Glu Ala Ile Glu
        355                 360                 365
Lys Ala Lys Lys Ala Gly Val Ser Val Val Val Ala Ala Gly Asn Glu
    370                 375                 380
Arg Val Tyr Gly Ser Asp His Asp Asp Pro Leu Ala Thr Asn Pro Asp
385                 390                 395                 400
```

```
Tyr Gly Leu Val Gly Ser Pro Ser Thr Gly Arg Thr Pro Thr Ser Val
                405                 410                 415
Ala Ala Ile Asn Ser Lys Trp Val Ile Gln Arg Leu Met Thr Val Lys
            420                 425                 430
Glu Leu Glu Asn Arg Ala Asp Leu Asn His Gly Lys Ala Ile Tyr Ser
        435                 440                 445
Glu Ser Val Asp Phe Lys Asn Ile Lys Asp Ser Leu Gly Tyr Asp Lys
    450                 455                 460
Ser His Gln Phe Ala Tyr Val Lys Glu Ser Thr Asp Ala Gly Tyr Asn
465                 470                 475                 480
Ala Gln Asp Val Lys Gly Lys Ile Ala Leu Ile Glu Arg Asp Pro Asn
                485                 490                 495
Lys Thr Tyr Asp Glu Met Ile Ala Leu Ala Lys Lys His Gly Ala Leu
            500                 505                 510
Gly Val Leu Ile Phe Asn Asn Lys Pro Gly Gln Ser Asn Arg Ser Met
        515                 520                 525
Arg Leu Thr Ala Asn Gly Met Gly Ile Pro Ser Ala Phe Ile Ser His
    530                 535                 540
Glu Phe Gly Lys Ala Met Ser Gln Leu Asn Gly Asn Gly Thr Gly Ser
545                 550                 555                 560
Leu Glu Phe Asp Ser Val Val Ser Lys Ala Pro Ser Gln Lys Gly Asn
                565                 570                 575
Glu Met Asn His Phe Ser Asn Trp Gly Leu Thr Ser Asp Gly Tyr Leu
            580                 585                 590
Lys Pro Asp Ile Thr Ala Pro Gly Gly Asp Ile Tyr Ser Thr Tyr Asn
        595                 600                 605
Asp Asn His Tyr Gly Ser Gln Thr Gly Thr Ser Met Ala Ser Pro Gln
    610                 615                 620
Ile Ala Gly Ala Ser Leu Leu Val Lys Gln Tyr Leu Glu Lys Thr Gln
625                 630                 635                 640
Pro Asn Leu Pro Lys Glu Lys Ile Ala Asp Ile Val Lys Asn Leu Leu
                645                 650                 655
Met Ser Asn Ala Gln Ile His Val Asn Pro Glu Thr Lys Thr Thr Thr
            660                 665                 670
Ser Pro Arg Gln Gln Gly Ala Gly Leu Leu Asn Ile Asp Gly Ala Val
        675                 680                 685
Thr Ser Gly Leu Tyr Val Thr Gly Lys Asp Asn Tyr Gly Ser Ile Ser
    690                 695                 700
Leu Gly Asn Ile Thr Asp Thr Met Thr Phe Asp Val Thr Val His Asn
705                 710                 715                 720
Leu Ser Asn Lys Asp Lys Thr Leu Arg Tyr Asp Thr Glu Leu Leu Thr
                725                 730                 735
Asp His Val Asp Pro Gln Lys Gly Arg Phe Thr Leu Thr Ser Arg Ser
            740                 745                 750
Leu Lys Thr Tyr Gln Gly Gly Glu Val Thr Val Pro Ala Asn Gly Lys
        755                 760                 765
Val Thr Val Arg Val Thr Met Asp Val Ser Gln Phe Thr Lys Glu Leu
    770                 775                 780
Thr Lys Gln Met Pro Asn Gly Tyr Tyr Leu Glu Gly Phe Val Arg Phe
785                 790                 795                 800
Arg Asp Ser Gln Asp Asp Gln Leu Asn Arg Val Asn Ile Pro Phe Val
                805                 810                 815
Gly Phe Lys Gly Gln Phe Glu Asn Leu Ala Val Ala Glu Glu Ser Ile
            820                 825                 830
Tyr Arg Leu Lys Ser Gln Gly Lys Thr Gly Phe Tyr Phe Asp Glu Ser
        835                 840                 845
Gly Pro Lys Asp Asp Ile Tyr Val Gly Lys His Phe Thr Gly Leu Val
    850                 855                 860
Thr Leu Gly Ser Glu Thr Asn Val Ser Thr Lys Thr Ile Ser Asp Asn
865                 870                 875                 880
Gly Leu His Thr Leu Gly Thr Phe Lys Asn Ala Asp Gly Lys Phe Ile
```

```
                    885                 890                 895
Leu Glu Lys Asn Ala Gln Gly Asn Pro Val Leu Ala Ile Ser Pro Asn
                900                 905                 910
Gly Asp Asn Asn Gln Asp Phe Ala Ala Phe Lys Gly Val Phe Leu Arg
            915                 920                 925
Lys Tyr Gln Gly Leu Lys Ala Ser Val Tyr His Ala Ser Asp Lys Glu
        930                 935                 940
His Lys Asn Pro Leu Trp Val Ser Pro Glu Ser Phe Lys Gly Asp Lys
945                 950                 955                 960
Asn Phe Asn Ser Asp Ile Arg Phe Ala Lys Ser Thr Thr Leu Leu Gly
                965                 970                 975
Thr Ala Phe Ser Gly Lys Ser Leu Thr Gly Ala Glu Leu Pro Asp Gly
            980                 985                 990
Tyr Tyr His Tyr Val Val Ser Tyr Tyr Pro Asp Val Val Gly Ala Lys
        995                 1000                1005
Arg Gln Glu Met Thr Phe Asp Met Ile Leu Asp Arg Gln Lys Pro Val
    1010                1015                1020
Leu Ser Gln Ala Thr Phe Asp Pro Glu Thr Asn Arg Phe Lys Pro Glu
1025                1030                1035                1040
Pro Leu Lys Asp Arg Gly Leu Ala Gly Val Arg Lys Asp Ser Val Phe
                1045                1050                1055
Tyr Leu Lys Arg Lys Asp Asn Lys Pro Tyr Thr Val Thr Ile Asn Asp
            1060                1065                1070
Ser Tyr Lys Tyr Val Ser Val Glu Asp Asn Lys Thr Phe Val Glu Arg
        1075                1080                1085
Gln Ala Asp Gly Ser Phe Ile Leu Pro Leu Asp Lys Ala Lys Leu Gly
    1090                1095                1100
Asp Phe Tyr Tyr Met Val Glu Asp Phe Ala Gly Asn Val Ala Ile Ala
1105                1110                1115                1120
Lys Leu Gly Asp His Leu Pro Gln Thr Leu Gly Lys Thr Pro Ile Lys
                1125                1130                1135
Leu Lys Leu Thr Asp Gly Asn Tyr Gln Thr Lys Glu Thr Leu Lys Asp
            1140                1145                1150
Asn Leu Glu Met Thr Gln Ser Asp Thr Gly Leu Val Thr Asn Gln Ala
        1155                1160                1165
Gln Leu Ala Val Val His Arg Asn Gln Pro Gln Ser Gln Leu Thr Lys
    1170                1175                1180
Met Asn Gln Asp Phe Phe Ile Ser Pro Asn Glu Asp Gly Asn Lys Asp
1185                1190                1195                1200
Phe Val Ala Phe Lys Gly Leu Lys Asn Asn Val Tyr Asn Asp Leu Thr
                1205                1210                1215
Val Asn Val Tyr Ala Lys Asp Asp His Gln Lys Gln Thr Pro Ile Trp
            1220                1225                1230
Ser Ser Gln Ala Gly Ala Ser Ala Ser Ala Ile Glu Ser Thr Ala Trp
        1235                1240                1245
Tyr Gly Ile Thr Ala Arg Gly Ser Lys Val Met Pro Gly Asp Tyr Gln
    1250                1255                1260
Tyr Val Val Thr Tyr Arg Asp Glu His Gly Lys Val His Gln Lys Gln
1265                1270                1275                1280
Tyr Thr Ile Ser Val Asn Asp Lys Lys Pro Met Ile Thr Gln Gly Arg
                1285                1290                1295
Phe Asp Thr Ile Asn Gly Val Asp His Phe Thr Pro Asp Lys Thr Lys
            1300                1305                1310
Ala Leu Gly Ser Ser Gly Ile Val Arg Glu Glu Val Phe Tyr Leu Ala
        1315                1320                1325
Lys Lys Asn Gly Arg Lys Phe Asp Val Thr Glu Gly Lys Asp Gly Ile
    1330                1335                1340
Thr Val Ser Asp Asn Lys Val Tyr Ile Pro Lys Asn Pro Asp Gly Ser
1345                1350                1355                1360
Tyr Thr Ile Ser Lys Arg Asp Gly Val Thr Leu Ser Asp Tyr Tyr Tyr
                1365                1370                1375
```

```
Leu Val Glu Asp Arg Ala Gly Asn Val Ser Phe Ala Thr Leu Arg Asp
            1380                1385                1390
Leu Lys Ala Val Gly Lys Asp Lys Ala Val Val Asn Phe Gly Leu Asp
        1395                1400                1405
Leu Pro Val Pro Glu Asp Lys Gln Ile Val Asn Phe Thr Tyr Leu Val
    1410                1415                1420
Arg Asp Ala Asp Gly Lys Pro Ile Glu Asn Leu Glu Tyr Tyr Asn Asn
1425                1430                1435                1440
Ser Gly Asn Ser Leu Ile Leu Pro Tyr Gly Lys Tyr Thr Val Glu Leu
                1445                1450                1455
Leu Thr Tyr Asp Thr Asn Ala Ala Lys Leu Glu Ser Asp Lys Ile Val
            1460                1465                1470
Ser Phe Thr Leu Ser Ala Asp Asn Asn Phe Gln Gln Val Thr Phe Lys
        1475                1480                1485
Met Thr Met Leu Ala Thr Ser Gln Ile Thr Ala His Phe Asp His Leu
    1490                1495                1500
Leu Pro Glu Gly Ser Arg Val Ser Leu Lys Thr Ala Gln Gly Gln Leu
1505                1510                1515                1520
Ile Pro Leu Glu Gln Ser Leu Tyr Val Pro Lys Ala Tyr Gly Lys Thr
                1525                1530                1535
Val Gln Glu Gly Thr Tyr Glu Val Val Val Ser Leu Pro Lys Gly Tyr
            1540                1545                1550
Arg Ile Glu Gly Asn Thr Lys Val Asn Thr Leu Pro Asn Glu Val His
        1555                1560                1565
Glu Leu Ser Leu Arg Leu Val Lys Val Gly Asp Ala Ser Asp Ser Thr
    1570                1575                1580
Gly Asp His Lys Val Met Ser Lys Asn Asn Ser Gln Ala Leu Thr Ala
1585                1590                1595                1600
Ser Ala Thr Pro Thr Lys Thr Thr Thr Ser Ala Thr Ala Lys Ala Leu
                1605                1610                1615
Pro Ser Thr Gly Glu Lys Met Gly Leu Lys Leu Arg Ile Val Gly Leu
            1620                1625                1630
Val Leu Leu Gly Leu Thr Cys Val Phe Ser Arg Lys Lys Ser Thr Lys
        1635                1640                1645
Asp
```

<210> 34
<211> 1649
<212> PRT
<213> Streptococcus pyogenes

<400> 34

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Met Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Lys Pro Gln Asp Thr Ser Gln Ile Thr Pro Lys Thr
65                  70                  75                  80
Asn Arg Glu Lys Glu Gln Ser Gln Asp Leu Val Phe Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Gly Lys Gly Tyr Lys Gly
    130                 135                 140
```

Gln Gly Lys Val Val Ala Val Ile Asp Thr Gly Ile Asp Pro Ala His
145 150 155 160
Gln Ser Met Arg Ile Ser Asp Val Ser Thr Ala Lys Val Lys Ser Lys
165 170 175
Glu Asp Met Leu Ala Arg Gln Lys Ala Ala Gly Ile Asn Tyr Gly Ser
180 185 190
Trp Ile Asn Asp Lys Val Val Phe Ala His Asn Tyr Val Glu Asn Ser
195 200 205
Asp Asn Ile Lys Glu Asn Gln Phe Glu Asp Phe Asp Glu Asp Trp Glu
210 215 220
Asn Phe Glu Phe Asp Ala Asp Ala Glu Pro Lys Ala Ile Lys Lys His
225 230 235 240
Lys Ile Tyr Arg Pro Gln Ser Thr Gln Ala Pro Lys Glu Thr Val Ile
245 250 255
Lys Thr Glu Glu Thr Asp Gly Ser His Asp Ile Asp Trp Thr Gln Thr
260 265 270
Asp Asp Asp Thr Lys Tyr Glu Ser His Gly Met His Val Thr Gly Ile
275 280 285
Val Ala Gly Asn Ser Lys Glu Ala Ala Ala Thr Gly Glu Arg Phe Leu
290 295 300
Gly Ile Ala Pro Glu Ala Gln Val Met Phe Met Arg Val Phe Ala Asn
305 310 315 320
Asp Val Met Gly Ser Ala Glu Ser Leu Phe Ile Lys Ala Ile Glu Asp
325 330 335
Ala Val Ala Leu Gly Ala Asp Val Ile Asn Leu Ser Leu Gly Thr Ala
340 345 350
Asn Gly Ala Gln Leu Ser Gly Ser Lys Pro Leu Met Glu Ala Ile Glu
355 360 365
Lys Ala Lys Lys Ala Gly Val Ser Val Val Val Ala Ala Gly Asn Glu
370 375 380
Arg Val Tyr Gly Ser Asp His Asp Asp Pro Leu Ala Thr Asn Pro Asp
385 390 395 400
Tyr Gly Leu Val Gly Ser Pro Ser Thr Gly Arg Thr Pro Thr Ser Val
405 410 415
Ala Ala Ile Asn Ser Lys Trp Val Ile Gln Arg Leu Met Thr Val Lys
420 425 430
Glu Leu Glu Asn Arg Ala Asp Leu Asn His Gly Lys Ala Ile Tyr Ser
435 440 445
Glu Ser Val Asp Phe Lys Asn Ile Lys Asp Ser Leu Gly Tyr Asp Lys
450 455 460
Ser His Gln Phe Ala Tyr Val Lys Glu Ser Thr Asp Ala Gly Tyr Asn
465 470 475 480
Ala Gln Asp Val Lys Gly Lys Ile Ala Leu Ile Glu Arg Asp Pro Asn
485 490 495
Lys Thr Tyr Asp Glu Met Ile Ala Leu Ala Lys Lys His Gly Ala Leu
500 505 510
Gly Val Leu Ile Phe Asn Asn Lys Pro Gly Gln Ser Asn Arg Ser Met
515 520 525
Arg Leu Thr Ala Asn Gly Met Gly Ile Pro Ser Ala Phe Ile Ser His
530 535 540
Glu Phe Gly Lys Ala Met Ser Gln Leu Asn Gly Asn Gly Thr Gly Ser
545 550 555 560
Leu Glu Phe Asp Ser Val Val Ser Lys Ala Pro Ser Gln Lys Gly Asn
565 570 575
Glu Met Asn His Phe Ser Asn Trp Gly Leu Thr Ser Asp Gly Tyr Leu
580 585 590
Lys Pro Asp Ile Thr Ala Pro Gly Gly Asp Ile Tyr Ser Thr Tyr Asn
595 600 605
Asp Asn His Tyr Gly Ser Gln Thr Gly Thr Ser Met Ala Ser Pro Gln
610 615 620
Ile Ala Gly Ala Ser Leu Leu Val Lys Gln Tyr Leu Glu Lys Thr Gln

```
625                 630                 635                 640
Pro Asn Leu Pro Lys Glu Lys Ile Ala Asp Ile Val Lys Asn Leu Leu
                645                 650                 655
Met Ser Asn Ala Gln Ile His Val Asn Pro Glu Thr Lys Thr Thr Thr
            660                 665                 670
Ser Pro Arg Gln Gln Gly Ala Gly Leu Leu Asn Ile Asp Gly Ala Val
        675                 680                 685
Thr Ser Gly Leu Tyr Val Thr Gly Lys Asp Asn Tyr Gly Ser Ile Ser
    690                 695                 700
Leu Gly Asn Ile Thr Asp Thr Met Thr Phe Asp Val Thr Val His Asn
705                 710                 715                 720
Leu Ser Asn Lys Asp Lys Thr Leu Arg Tyr Asp Thr Glu Leu Leu Thr
                725                 730                 735
Asp His Val Asp Pro Gln Lys Gly Arg Phe Thr Leu Thr Ser Arg Ser
            740                 745                 750
Leu Lys Thr Tyr Gln Gly Gly Glu Val Thr Val Pro Ala Asn Gly Lys
        755                 760                 765
Val Thr Val Arg Val Thr Met Asp Val Ser Gln Phe Thr Lys Glu Leu
    770                 775                 780
Thr Lys Gln Met Pro Asn Gly Tyr Tyr Leu Glu Gly Phe Val Arg Phe
785                 790                 795                 800
Arg Asp Ser Gln Asp Asp Gln Leu Asn Arg Val Asn Ile Pro Phe Val
                805                 810                 815
Gly Phe Lys Gly Gln Phe Glu Asn Leu Ala Val Ala Glu Glu Ser Ile
            820                 825                 830
Tyr Arg Leu Lys Ser Gln Gly Lys Thr Gly Phe Tyr Phe Asp Glu Ser
        835                 840                 845
Gly Pro Lys Asp Asp Ile Tyr Val Gly Lys His Phe Thr Gly Leu Val
    850                 855                 860
Thr Leu Gly Ser Glu Thr Asn Val Ser Thr Lys Thr Ile Ser Asp Asn
865                 870                 875                 880
Gly Leu His Thr Leu Gly Thr Phe Lys Asn Ala Asp Gly Lys Phe Ile
                885                 890                 895
Leu Glu Lys Asn Ala Gln Gly Asn Pro Val Leu Ala Ile Ser Pro Asn
            900                 905                 910
Gly Asp Asn Asn Gln Asp Phe Ala Ala Phe Lys Gly Val Phe Leu Arg
        915                 920                 925
Lys Tyr Gln Gly Leu Lys Ala Ser Val Tyr His Ala Ser Asp Lys Glu
    930                 935                 940
His Lys Asn Pro Leu Trp Val Ser Pro Glu Ser Phe Lys Gly Asp Lys
945                 950                 955                 960
Asn Phe Asn Ser Asp Ile Arg Phe Ala Lys Ser Thr Thr Leu Leu Gly
                965                 970                 975
Thr Ala Phe Ser Gly Lys Ser Leu Thr Gly Ala Glu Leu Pro Asp Gly
            980                 985                 990
Tyr Tyr His Tyr Val Val Ser Tyr Tyr Pro Asp Val Val Gly Ala Lys
        995                 1000                1005
Arg Gln Glu Met Thr Phe Asp Met Ile Leu Asp Arg Gln Lys Pro Val
    1010                1015                1020
Leu Ser Gln Ala Thr Phe Asp Pro Glu Thr Asn Arg Phe Lys Pro Glu
1025                1030                1035                1040
Pro Leu Lys Asp Arg Gly Leu Ala Gly Val Arg Lys Asp Ser Val Phe
                1045                1050                1055
Tyr Leu Glu Arg Lys Asp Asn Lys Pro Tyr Thr Val Thr Ile Asn Asp
            1060                1065                1070
Ser Tyr Lys Tyr Val Ser Val Glu Asp Asn Lys Thr Phe Val Glu Arg
        1075                1080                1085
Gln Ala Asp Gly Ser Phe Ile Leu Pro Leu Asp Lys Ala Lys Leu Gly
    1090                1095                1100
Asp Phe Tyr Tyr Met Val Glu Asp Phe Ala Gly Asn Val Ala Ile Ala
1105                1110                1115                1120
```

```
Lys Leu Gly Asp His Leu Pro Gln Thr Leu Gly Lys Thr Pro Ile Lys
                1125                1130                1135
Leu Lys Leu Thr Asp Gly Asn Tyr Gln Thr Lys Glu Thr Leu Lys Asp
            1140                1145                1150
Asn Leu Glu Met Thr Gln Ser Asp Thr Gly Leu Val Thr Asn Gln Ala
        1155                1160                1165
Gln Leu Ala Val Val His Arg Asn Gln Pro Gln Ser Gln Leu Thr Lys
    1170                1175                1180
Met Asn Gln Asp Phe Phe Ile Ser Pro Asn Glu Asp Gly Asn Lys Asp
1185                1190                1195                1200
Phe Val Ala Phe Lys Gly Leu Lys Asn Asn Val Tyr Asn Asp Leu Thr
                1205                1210                1215
Val Asn Val Tyr Ala Lys Asp Asp His Gln Lys Gln Thr Pro Ile Trp
            1220                1225                1230
Ser Ser Gln Ala Gly Ala Ser Ala Ser Ala Ile Glu Ser Thr Ala Trp
        1235                1240                1245
Tyr Gly Ile Thr Ala Arg Gly Ser Lys Val Met Pro Gly Asp Tyr Gln
    1250                1255                1260
Tyr Val Val Thr Tyr Arg Asp Glu His Gly Lys Val His Gln Lys Gln
1265                1270                1275                1280
Tyr Thr Ile Ser Val Asn Asp Lys Lys Pro Met Ile Thr Gln Gly Arg
                1285                1290                1295
Phe Asp Thr Ile Asn Gly Val Asp His Phe Thr Pro Asp Lys Thr Lys
            1300                1305                1310
Ala Leu Gly Ser Ser Gly Ile Val Arg Glu Glu Val Phe Tyr Leu Ala
        1315                1320                1325
Lys Lys Asn Gly Arg Lys Phe Asp Val Thr Glu Gly Lys Asp Gly Ile
    1330                1335                1340
Thr Val Ser Asp Asn Lys Val Tyr Ile Pro Lys Asn Pro Asp Gly Ser
1345                1350                1355                1360
Tyr Thr Ile Ser Lys Arg Asp Gly Val Thr Leu Ser Asp Tyr Tyr Tyr
                1365                1370                1375
Leu Val Glu Asp Arg Ala Gly Asn Val Ser Phe Ala Thr Leu Arg Asp
            1380                1385                1390
Leu Lys Ala Val Gly Lys Asp Lys Ala Val Val Asn Phe Gly Leu Asp
        1395                1400                1405
Leu Pro Val Pro Glu Asp Lys Gln Ile Val Asn Phe Thr Tyr Leu Val
    1410                1415                1420
Arg Asp Ala Asp Gly Lys Pro Ile Glu Asn Leu Glu Tyr Tyr Asn Asn
1425                1430                1435                1440
Ser Gly Asn Ser Leu Ile Leu Pro Tyr Gly Lys Tyr Thr Val Glu Leu
                1445                1450                1455
Leu Thr Tyr Asp Thr Asn Ala Ala Lys Leu Glu Ser Asp Lys Ile Val
            1460                1465                1470
Ser Phe Thr Leu Ser Ala Asp Asn Asn Phe Gln Gln Val Thr Phe Lys
        1475                1480                1485
Met Thr Met Leu Ala Thr Ser Gln Ile Thr Ala His Phe Asp His Leu
    1490                1495                1500
Leu Pro Glu Gly Ser Arg Val Ser Leu Lys Thr Ala Gln Gly Gln Leu
1505                1510                1515                1520
Ile Pro Leu Glu Gln Ser Leu Tyr Val Pro Lys Ala Tyr Gly Lys Thr
                1525                1530                1535
Val Gln Glu Gly Thr Tyr Glu Val Val Val Ser Leu Pro Lys Gly Tyr
            1540                1545                1550
Arg Ile Glu Gly Asn Thr Lys Val Asn Thr Leu Pro Asn Glu Val His
        1555                1560                1565
Glu Leu Ser Leu Arg Leu Val Lys Val Gly Asp Ala Ser Asp Ser Thr
    1570                1575                1580
Gly Asp His Lys Val Met Ser Lys Asn Asn Ser Gln Ala Leu Thr Ala
1585                1590                1595                1600
Ser Ala Thr Pro Thr Lys Thr Thr Thr Ser Ala Thr Ala Lys Ala Leu
```

```
                    1605                1610                1615
Pro Ser Thr Gly Glu Lys Met Gly Leu Lys Leu Arg Ile Val Gly Leu
                1620                1625                1630
Val Leu Leu Gly Leu Thr Cys Val Phe Ser Arg Lys Lys Ser Thr Lys
            1635                1640                1645
Asp
```

<210> 35
<211> 1649
<212> PRT
<213> Streptococcus pyogenes

<400> 35

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Met Thr Thr
            20                  25                  30
Thr Val Ala Ala Asp Glu Leu Thr Thr Thr Ser Glu Pro Thr Ile Thr
        35                  40                  45
Asn His Ala Gln Gln Gln Ala Gln His Leu Thr Asn Thr Glu Leu Ser
    50                  55                  60
Ser Ala Glu Ser Lys Pro Gln Asp Thr Ser Gln Ile Thr Pro Lys Thr
65                  70                  75                  80
Asn Arg Glu Lys Glu Gln Ser Gln Asp Leu Val Ser Glu Pro Thr Thr
                85                  90                  95
Thr Glu Leu Ala Asp Thr Asp Ala Ala Ser Met Ala Asn Thr Gly Pro
            100                 105                 110
Asp Ala Thr Gln Lys Ser Ala Ser Leu Pro Pro Val Asn Thr Asp Val
        115                 120                 125
His Asp Trp Val Lys Thr Lys Gly Ala Trp Gly Lys Gly Tyr Lys Gly
    130                 135                 140
Gln Gly Lys Val Val Ala Val Ile Asp Thr Gly Ile Asp Pro Ala His
145                 150                 155                 160
Gln Ser Met Arg Ile Ser Asp Val Ser Thr Ala Lys Val Lys Ser Lys
                165                 170                 175
Glu Asp Met Leu Ala Arg Gln Lys Ala Ala Gly Ile Asn Tyr Gly Ser
            180                 185                 190
Trp Ile Asn Asp Lys Val Val Phe Ala His Asn Tyr Val Glu Asn Ser
        195                 200                 205
Asp Asn Ile Lys Glu Asn Gln Phe Glu Asp Phe Asp Glu Asp Trp Glu
    210                 215                 220
Asn Phe Glu Phe Asp Ala Asp Ala Glu Pro Lys Ala Ile Lys Lys His
225                 230                 235                 240
Lys Ile Tyr Arg Pro Gln Ser Thr Gln Ala Pro Lys Glu Thr Val Ile
                245                 250                 255
Lys Thr Glu Glu Thr Asp Gly Ser His Asp Ile Asp Trp Thr Gln Thr
            260                 265                 270
Asp Asp Asp Thr Lys Tyr Glu Ser His Gly Met His Val Thr Gly Ile
        275                 280                 285
Val Ala Gly Asn Ser Lys Glu Ala Ala Ala Thr Gly Glu Arg Phe Leu
    290                 295                 300
Gly Ile Ala Pro Glu Ala Gln Val Met Phe Met Arg Val Phe Ala Asn
305                 310                 315                 320
Asp Val Met Gly Ser Ala Glu Ser Leu Phe Ile Lys Ala Ile Glu Asp
                325                 330                 335
Ala Val Ala Leu Gly Ala Asp Val Ile Asn Leu Ser Leu Gly Thr Ala
            340                 345                 350
Asn Gly Ala Gln Leu Ser Gly Ser Lys Pro Leu Met Glu Ala Ile Glu
        355                 360                 365
Lys Ala Lys Lys Ala Gly Val Ser Val Val Val Ala Ala Gly Asn Glu
```

```
    370                 375                 380
Arg Val Tyr Gly Ser Asp His Asp Asp Pro Leu Ala Thr Asn Pro Asp
385                 390                 395                 400
Tyr Gly Leu Val Gly Ser Pro Ser Thr Gly Arg Thr Pro Thr Ser Val
                405                 410                 415
Ala Ala Ile Asn Ser Lys Trp Val Ile Gln Arg Leu Met Thr Val Lys
            420                 425                 430
Glu Leu Glu Asn Arg Ala Asp Leu Asn His Gly Lys Ala Ile Tyr Ser
        435                 440                 445
Glu Ser Val Asp Phe Lys Asn Ile Lys Asp Ser Leu Gly Tyr Asp Lys
    450                 455                 460
Ser His Gln Phe Ala Tyr Val Lys Glu Ser Thr Asp Ala Gly Tyr Asn
465                 470                 475                 480
Ala Gln Asp Val Lys Gly Lys Ile Ala Leu Ile Glu Arg Asp Pro Asn
                485                 490                 495
Lys Thr Tyr Asp Glu Met Ile Ala Leu Ala Lys Lys His Gly Ala Leu
            500                 505                 510
Gly Val Leu Ile Phe Asn Asn Lys Pro Gly Gln Ser Asn Arg Ser Met
        515                 520                 525
Arg Leu Thr Ala Asn Gly Met Gly Ile Pro Ser Ala Phe Ile Ser His
    530                 535                 540
Glu Phe Gly Lys Ala Met Ser Gln Leu Asn Gly Asn Gly Thr Gly Ser
545                 550                 555                 560
Leu Glu Phe Asp Ser Val Val Ser Lys Ala Pro Ser Gln Lys Gly Asn
                565                 570                 575
Glu Met Asn His Phe Ser Asn Trp Gly Leu Thr Ser Asp Gly Tyr Leu
            580                 585                 590
Lys Pro Asp Ile Thr Ala Pro Gly Gly Asp Ile Tyr Ser Thr Tyr Asn
        595                 600                 605
Asp Asn His Tyr Gly Ser Gln Thr Gly Thr Ser Met Ala Ser Pro Gln
    610                 615                 620
Ile Ala Gly Ala Ser Leu Leu Val Lys Gln Tyr Leu Glu Lys Thr Gln
625                 630                 635                 640
Pro Asn Leu Pro Lys Glu Lys Ile Ala Asp Ile Val Lys Asn Leu Leu
                645                 650                 655
Met Ser Asn Ala Gln Ile His Val Asn Pro Glu Thr Lys Thr Thr Thr
            660                 665                 670
Ser Pro Arg Gln Gln Gly Ala Gly Leu Leu Asn Ile Asp Gly Ala Val
        675                 680                 685
Thr Ser Gly Leu Tyr Val Thr Gly Lys Asp Asn Tyr Gly Ser Ile Ser
    690                 695                 700
Leu Gly Asn Ile Thr Asp Thr Met Thr Phe Asp Val Thr Val His Asn
705                 710                 715                 720
Leu Ser Asn Lys Asp Lys Thr Leu Arg Tyr Asp Thr Glu Leu Leu Thr
                725                 730                 735
Asp His Val Asp Pro Gln Lys Gly Arg Phe Thr Leu Thr Ser Arg Ser
            740                 745                 750
Leu Lys Thr Tyr Gln Gly Gly Glu Val Thr Val Pro Ala Asn Gly Lys
        755                 760                 765
Val Thr Val Arg Val Thr Met Asp Val Ser Gln Phe Thr Lys Glu Leu
    770                 775                 780
Thr Lys Gln Met Pro Asn Gly Tyr Tyr Leu Glu Gly Phe Val Arg Phe
785                 790                 795                 800
Arg Asp Ser Gln Asp Asp Gln Leu Asn Arg Val Asn Ile Pro Phe Val
                805                 810                 815
Gly Phe Lys Gly Gln Phe Glu Asn Leu Ala Val Ala Glu Glu Ser Ile
            820                 825                 830
Tyr Arg Leu Lys Ser Gln Gly Lys Thr Gly Phe Tyr Phe Asp Glu Ser
        835                 840                 845
Gly Pro Lys Asp Asp Ile Tyr Val Gly Lys His Phe Thr Gly Leu Val
    850                 855                 860
```

Thr Leu Gly Ser Glu Thr Asn Val Ser Thr Lys Thr Ile Ser Asp Asn
865 870 875 880
Gly Leu His Thr Leu Gly Thr Phe Lys Asn Ala Asp Gly Lys Phe Ile
885 890 895
Leu Glu Lys Asn Ala Gln Gly Asn Pro Val Leu Ala Ile Ser Pro Asn
900 905 910
Gly Asp Asn Asn Gln Asp Phe Ala Ala Phe Lys Gly Val Phe Leu Arg
915 920 925
Lys Tyr Gln Gly Leu Lys Ala Ser Val Tyr His Ala Ser Asp Lys Glu
930 935 940
His Lys Asn Pro Leu Trp Val Ser Pro Glu Ser Phe Lys Gly Asp Lys
945 950 955 960
Asn Phe Asn Ser Asp Ile Arg Phe Ala Lys Ser Thr Thr Leu Leu Gly
965 970 975
Thr Ala Phe Ser Gly Lys Ser Leu Thr Gly Ala Glu Leu Pro Asp Gly
980 985 990
Tyr Tyr His Tyr Val Val Ser Tyr Tyr Pro Asp Val Val Gly Ala Lys
995 1000 1005
Arg Gln Glu Met Thr Phe Asp Met Ile Leu Asp Arg Gln Lys Pro Val
1010 1015 1020
Leu Ser Gln Ala Thr Phe Asp Pro Glu Thr Asn Arg Phe Lys Pro Glu
1025 1030 1035 1040
Pro Leu Lys Asp Arg Gly Leu Ala Gly Val Arg Lys Asp Ser Val Phe
1045 1050 1055
Tyr Leu Glu Arg Lys Asp Asn Lys Pro Tyr Thr Val Thr Ile Asn Asp
1060 1065 1070
Ser Tyr Lys Tyr Val Ser Val Glu Asp Asn Lys Thr Phe Val Glu Arg
1075 1080 1085
Gln Ala Asp Gly Ser Phe Ile Leu Pro Leu Asp Lys Ala Lys Leu Gly
1090 1095 1100
Asp Phe Tyr Tyr Met Val Glu Asp Phe Ala Gly Asn Val Ala Ile Ala
1105 1110 1115 1120
Lys Leu Gly Asp His Leu Pro Gln Thr Leu Gly Lys Thr Pro Ile Lys
1125 1130 1135
Leu Lys Leu Thr Asp Gly Asn Tyr Gln Thr Lys Glu Thr Leu Lys Asp
1140 1145 1150
Asn Leu Glu Met Thr Gln Ser Asp Thr Gly Leu Val Thr Asn Gln Ala
1155 1160 1165
Gln Leu Ala Val Val His Arg Asn Gln Pro Gln Ser Gln Leu Thr Lys
1170 1175 1180
Met Asn Gln Asp Phe Phe Ile Ser Pro Asn Glu Asp Gly Asn Lys Asp
1185 1190 1195 1200
Phe Val Ala Phe Lys Gly Leu Lys Asn Asn Val Tyr Asn Asp Leu Thr
1205 1210 1215
Val Asn Val Tyr Ala Lys Asp Asp His Gln Lys Gln Thr Pro Ile Trp
1220 1225 1230
Ser Ser Gln Ala Gly Ala Ser Ala Ser Ala Ile Glu Ser Thr Ala Trp
1235 1240 1245
Tyr Gly Ile Thr Ala Arg Gly Ser Lys Val Met Pro Gly Asp Tyr Gln
1250 1255 1260
Tyr Val Val Thr Tyr Arg Asp Glu His Gly Lys Val His Gln Lys Gln
1265 1270 1275 1280
Tyr Thr Ile Ser Val Asn Asp Lys Lys Pro Met Ile Thr Gln Gly Arg
1285 1290 1295
Phe Asp Thr Ile Asn Gly Val Asp His Phe Thr Pro Asp Lys Thr Lys
1300 1305 1310
Ala Leu Gly Ser Ser Gly Ile Val Arg Glu Glu Val Phe Tyr Leu Ala
1315 1320 1325
Lys Lys Asn Gly Arg Lys Phe Asp Val Thr Glu Gly Lys Asp Gly Ile
1330 1335 1340
Thr Val Ser Asp Asn Lys Val Tyr Ile Pro Lys Asn Pro Asp Gly Ser

```
1345                1350                1355                1360
Tyr Thr Ile Ser Lys Arg Asp Gly Val Thr Leu Ser Asp Tyr Tyr Tyr
                1365                1370                1375
Leu Val Glu Asp Arg Ala Gly Asn Val Ser Phe Ala Thr Leu Arg Asp
            1380                1385                1390
Leu Lys Ala Val Gly Lys Asp Lys Ala Val Val Asn Phe Gly Leu Asp
        1395                1400                1405
Leu Pro Val Pro Glu Asp Lys Gln Ile Val Asn Phe Thr Tyr Leu Val
    1410                1415                1420
Arg Asp Ala Asp Gly Lys Pro Ile Glu Asn Leu Glu Tyr Tyr Asn Asn
1425                1430                1435                1440
Ser Gly Asn Ser Leu Ile Leu Pro Tyr Gly Lys Tyr Thr Val Glu Leu
                1445                1450                1455
Leu Thr Tyr Asp Thr Asn Ala Ala Lys Leu Glu Ser Asp Lys Ile Val
            1460                1465                1470
Ser Phe Thr Leu Ser Ala Asp Asn Asn Phe Gln Gln Val Thr Phe Lys
        1475                1480                1485
Met Thr Met Leu Ala Thr Ser Gln Ile Thr Ala His Phe Asp His Leu
    1490                1495                1500
Leu Pro Glu Gly Ser Arg Val Ser Leu Lys Thr Ala Gln Gly Gln Leu
1505                1510                1515                1520
Ile Pro Leu Glu Gln Ser Leu Tyr Val Pro Lys Ala Tyr Gly Lys Thr
                1525                1530                1535
Val Gln Glu Gly Thr Tyr Glu Val Val Val Ser Leu Pro Lys Gly Tyr
            1540                1545                1550
Arg Ile Glu Gly Asn Thr Lys Val Asn Thr Leu Pro Asn Glu Val His
        1555                1560                1565
Glu Leu Ser Leu Arg Leu Val Lys Val Gly Asp Ala Ser Asp Ser Thr
    1570                1575                1580
Gly Asp His Lys Val Met Ser Lys Asn Asn Ser Gln Ala Leu Thr Ala
1585                1590                1595                1600
Ser Ala Thr Pro Thr Lys Thr Thr Thr Ser Ala Thr Ala Lys Ala Leu
                1605                1610                1615
Pro Ser Thr Gly Glu Lys Met Gly Leu Lys Leu Arg Ile Val Gly Leu
            1620                1625                1630
Val Leu Leu Gly Leu Thr Cys Val Phe Ser Arg Lys Lys Ser Thr Lys
        1635                1640                1645
Glu
```

<210> 36
<211> 1648
<212> PRT
<213> Streptococcus pyogenes

<400> 36

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
```

```
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Thr
    450                 455                 460
His Gln Phe Ala Tyr Val Lys Glu Ser Thr Asp Ala Gly Tyr Lys Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Ala Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
```

Asn His Tyr Gly Ser Gln Thr Gly Thr Ser Met Ala Ser Pro Gln Ile
610 615 620
Ala Gly Ala Ser Leu Leu Val Lys Gln Tyr Leu Glu Lys Thr Gln Pro
625 630 635 640
Asn Leu Pro Lys Glu Lys Ile Ala Asp Ile Val Lys Asn Leu Leu Met
645 650 655
Ser Asn Ala Gln Ile His Val Asn Pro Glu Thr Lys Thr Thr Thr Ser
660 665 670
Pro Arg Gln Gln Gly Ala Gly Leu Leu Asn Ile Asp Gly Ala Val Thr
675 680 685
Ser Gly Leu Tyr Val Thr Gly Lys Asp Asn Tyr Gly Ser Ile Ser Leu
690 695 700
Gly Asn Ile Thr Asp Thr Met Thr Phe Asp Val Thr Val His Asn Leu
705 710 715 720
Ser Asn Lys Asp Lys Thr Leu Arg Tyr Asp Thr Glu Leu Leu Thr Asp
725 730 735
His Val Asp Pro Gln Lys Gly Arg Phe Thr Leu Thr Ser Arg Ser Leu
740 745 750
Lys Thr Tyr Gln Gly Gly Glu Val Thr Val Pro Ala Asn Gly Lys Val
755 760 765
Thr Val Arg Val Thr Met Asp Val Ser Gln Phe Thr Lys Glu Leu Thr
770 775 780
Lys Gln Met Pro Asn Gly Tyr Tyr Leu Glu Gly Phe Val Arg Phe Arg
785 790 795 800
Asp Ser Gln Asp Ala Gln Leu Asn Arg Val Asn Ile Pro Phe Val Gly
805 810 815
Phe Lys Gly Gln Phe Glu Asn Leu Ala Val Ala Glu Glu Ser Ile Tyr
820 825 830
Arg Leu Lys Ser Gln Gly Lys Thr Gly Phe Tyr Phe Asp Glu Ser Gly
835 840 845
Pro Lys Asp Asp Ile Tyr Val Gly Lys His Phe Thr Gly Leu Val Thr
850 855 860
Leu Gly Ser Glu Thr Asn Val Ser Thr Lys Thr Ile Ser Asp Asn Gly
865 870 875 880
Leu His Thr Leu Gly Thr Phe Lys Asn Ala Asp Gly Lys Phe Ile Leu
885 890 895
Glu Lys Asn Ala Gln Gly Asn Pro Val Leu Ala Ile Ser Pro Asn Gly
900 905 910
Asp Asn Asn Gln Asp Phe Ala Ala Phe Lys Gly Val Phe Leu Arg Lys
915 920 925
Tyr Gln Gly Leu Lys Ala Ser Val Tyr His Ala Ser Asp Lys Glu His
930 935 940
Lys Asn Pro Leu Trp Val Ser Pro Glu Ser Phe Lys Gly Asp Lys Asn
945 950 955 960
Phe Asn Ser Asp Ile Arg Phe Ala Lys Ser Thr Thr Leu Leu Gly Thr
965 970 975
Ala Phe Ser Gly Lys Ser Leu Thr Gly Ala Glu Leu Pro Asp Gly His
980 985 990
Tyr His Tyr Val Val Ser Tyr Tyr Pro Asp Val Val Gly Ala Lys Arg
995 1000 1005
Gln Glu Met Thr Phe Asp Met Ile Leu Asp Arg Gln Lys Pro Val Leu
1010 1015 1020
Ser Gln Ala Thr Phe Asp Pro Glu Thr Asn Arg Phe Lys Pro Glu Pro
1025 1030 1035 1040
Leu Lys Asp Arg Gly Leu Ala Gly Val Arg Lys Asp Ser Val Phe Tyr
1045 1050 1055
Leu Glu Arg Lys Asp Asn Lys Pro Tyr Thr Val Thr Ile Asn Asp Ser
1060 1065 1070
Tyr Lys Tyr Val Ser Val Glu Asp Asn Lys Thr Phe Val Glu Arg Gln
1075 1080 1085
Ala Asp Gly Ser Phe Ile Leu Pro Leu Asp Lys Ala Lys Leu Gly Asp

1090 1095 1100
Phe Tyr Tyr Met Val Glu Asp Phe Ala Gly Asn Val Ala Ile Ala Lys
1105 1110 1115 1120
Leu Gly Asp His Leu Pro Gln Thr Leu Gly Lys Thr Pro Ile Lys Leu
1125 1130 1135
Lys Leu Thr Asp Gly Asn Tyr Gln Thr Lys Glu Thr Leu Lys Asp Asn
1140 1145 1150
Leu Glu Met Thr Gln Ser Asp Thr Gly Leu Val Thr Asn Gln Ala Gln
1155 1160 1165
Leu Ala Val Val His Arg Asn Gln Pro Gln Ser Gln Leu Thr Lys Met
1170 1175 1180
Asn Gln Asp Phe Phe Ile Ser Pro Asn Glu Asp Gly Asn Lys Asp Phe
1185 1190 1195 1200
Val Ala Phe Lys Gly Leu Lys Asn Asn Val Tyr Asn Asp Leu Thr Val
1205 1210 1215
Asn Val Tyr Ala Lys Asp Asp His Gln Lys Gln Thr Pro Ile Trp Ser
1220 1225 1230
Ser Gln Ala Gly Ala Ser Ala Ser Ala Ile Glu Ser Thr Ala Trp Tyr
1235 1240 1245
Gly Ile Thr Ala Arg Gly Ser Lys Val Met Pro Gly Asp Tyr Gln Tyr
1250 1255 1260
Val Val Thr Tyr Arg Asp Glu His Gly Lys Glu His Gln Lys Gln Tyr
1265 1270 1275 1280
Thr Ile Ser Val Asn Asp Lys Lys Pro Met Ile Thr Gln Gly Arg Phe
1285 1290 1295
Asp Thr Ile Asn Gly Val Asp His Phe Thr Pro Asp Lys Thr Lys Ala
1300 1305 1310
Leu Gly Ser Ser Gly Ile Val Arg Glu Glu Val Phe Tyr Leu Ala Lys
1315 1320 1325
Lys Asn Gly Arg Lys Phe Asp Val Thr Glu Gly Lys Asp Gly Ile Thr
1330 1335 1340
Val Ser Asp Asn Lys Val Tyr Ile Pro Lys Asn Pro Asp Gly Ser Tyr
1345 1350 1355 1360
Thr Ile Ser Lys Arg Asp Gly Val Thr Leu Ser Asp Tyr Tyr Tyr Leu
1365 1370 1375
Val Glu Asp Arg Ala Gly Asn Val Ser Phe Ala Thr Leu Arg Asp Leu
1380 1385 1390
Lys Ala Val Gly Lys Asp Lys Ala Val Val Asn Phe Gly Leu Asp Leu
1395 1400 1405
Pro Val Pro Glu Asp Lys Gln Ile Val Asn Phe Thr Tyr Leu Val Arg
1410 1415 1420
Asp Ala Asp Gly Lys Pro Ile Glu Asn Leu Glu Tyr Tyr Asn Asn Ser
1425 1430 1435 1440
Gly Asn Ser Leu Ile Leu Pro Tyr Gly Lys Tyr Thr Val Glu Leu Leu
1445 1450 1455
Thr Tyr Asp Thr Asn Ala Ala Lys Leu Glu Ser Asp Lys Ile Val Ser
1460 1465 1470
Phe Thr Leu Ser Ala Asp Asn Asn Phe Gln Gln Val Thr Phe Lys Met
1475 1480 1485
Thr Met Leu Ala Thr Ser Gln Ile Thr Ala His Phe Asp His Leu Leu
1490 1495 1500
Pro Glu Gly Ser Arg Val Ser Leu Lys Thr Ala Gln Gly Gln Leu Ile
1505 1510 1515 1520
Pro Leu Glu Gln Ser Leu Tyr Val Pro Lys Ala Tyr Gly Lys Thr Val
1525 1530 1535
Gln Glu Gly Thr Tyr Glu Val Val Val Ser Leu Pro Lys Gly Tyr Arg
1540 1545 1550
Ile Glu Gly Asn Thr Lys Val Asn Thr Leu Pro Asn Glu Val His Glu
1555 1560 1565
Leu Ser Leu Arg Leu Val Lys Val Gly Asp Ala Ser Asp Ser Thr Gly
1570 1575 1580

```
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
                1605                1610                1615
Ser Thr Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 37
<211> 1648
<212> PRT
<213> Streptococcus pyogenes

<400> 37

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
```

```
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Thr
    450                 455                 460
His Gln Phe Ala Tyr Val Lys Glu Ser Thr Asp Ala Gly Tyr Lys Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Ala Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Asp Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
```

```
    850                 855                 860
Leu Gly Ser Glu Thr Asn Val Ser Thr Lys Thr Ile Ser Asp Asn Gly
865                 870                 875                 880
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly His
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Asn Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
Phe Tyr Tyr Met Val Glu Asp Phe Ala Gly Asn Val Ala Ile Ala Lys
1105                1110                1115                1120
Leu Gly Asp His Leu Pro Gln Thr Leu Gly Lys Thr Pro Ile Lys Leu
                1125                1130                1135
Lys Leu Thr Asp Gly Asn Tyr Gln Thr Lys Glu Thr Leu Lys Asp Asn
            1140                1145                1150
Leu Glu Met Thr Gln Ser Asp Thr Gly Leu Val Thr Asn Gln Ala Gln
        1155                1160                1165
Leu Ala Val Val His Arg Asn Gln Pro Gln Ser Gln Leu Thr Lys Met
    1170                1175                1180
Asn Gln Asp Phe Phe Ile Ser Pro Asn Glu Asp Gly Asn Lys Asp Phe
1185                1190                1195                1200
Val Ala Phe Lys Gly Leu Lys Asn Asn Val Tyr Asn Asp Leu Thr Val
                1205                1210                1215
Asn Val Tyr Ala Lys Asp Asp His Gln Lys Gln Thr Pro Ile Trp Ser
            1220                1225                1230
Ser Gln Ala Gly Ala Ser Ala Ser Ala Ile Glu Ser Thr Ala Trp Tyr
        1235                1240                1245
Gly Ile Thr Ala Arg Gly Ser Lys Val Met Pro Gly Asp Tyr Gln Tyr
    1250                1255                1260
Val Val Thr Tyr Arg Asp Glu His Gly Lys Glu His Gln Lys Gln Tyr
1265                1270                1275                1280
Thr Ile Ser Val Asn Asp Lys Lys Pro Met Ile Thr Gln Gly Arg Phe
                1285                1290                1295
Asp Thr Ile Asn Gly Val Asp His Phe Thr Pro Asp Lys Thr Lys Ala
            1300                1305                1310
Leu Gly Ser Ser Gly Ile Val Arg Glu Glu Val Phe Tyr Leu Ala Lys
        1315                1320                1325
Lys Asn Gly Arg Lys Phe Asp Val Thr Glu Gly Lys Asp Gly Ile Thr
    1330                1335                1340
```

```
Val Ser Asp Asn Lys Val Tyr Ile Pro Lys Asn Pro Asp Gly Ser Tyr
1345                1350                1355                1360
Thr Ile Ser Lys Arg Asp Gly Val Thr Leu Ser Asp Tyr Tyr Tyr Leu
                1365                1370                1375
Val Glu Asp Arg Ala Gly Asn Val Ser Phe Ala Thr Leu Arg Asp Leu
            1380                1385                1390
Lys Ala Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Val Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asn Thr Lys Val Asn Thr Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
                1605                1610                1615
Ser Thr Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 38
<211> 1648
<212> PRT
<213> Streptococcus pyogenes

<400> 38

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
```

```
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Val Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
```

```
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Met Ile Ser Asp Asn Gly
865                 870                 875                 880
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly Tyr
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Ser Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
```

Phe Tyr Tyr Met Val Glu Asp Phe Ala Gly Asn Val Ala Ile Ala Lys
1105 1110 1115 1120
Leu Gly Asp His Leu Pro Gln Thr Leu Gly Lys Thr Pro Ile Lys Leu
1125 1130 1135
Lys Leu Thr Asp Gly Asn Tyr Gln Thr Lys Glu Thr Leu Lys Asp Asn
1140 1145 1150
Leu Glu Met Thr Gln Ser Asp Thr Gly Leu Val Thr Asn Gln Ala Gln
1155 1160 1165
Leu Ala Val Val His Arg Asn Gln Pro Gln Ser Gln Leu Thr Lys Met
1170 1175 1180
Asn Gln Asp Phe Phe Ile Ser Pro Asn Glu Asp Gly Asn Lys Asp Phe
1185 1190 1195 1200
Val Ala Phe Lys Gly Leu Lys Asn Asn Val Tyr Asn Asp Leu Thr Val
1205 1210 1215
Asn Val Tyr Ala Lys Asp Asp His Gln Lys Gln Thr Pro Ile Trp Ser
1220 1225 1230
Ser Gln Ala Gly Ala Ser Ala Ser Ala Ile Glu Ser Thr Ala Trp Tyr
1235 1240 1245
Gly Ile Thr Ala Arg Gly Ser Lys Val Met Pro Gly Asp Tyr Gln Tyr
1250 1255 1260
Val Val Thr Tyr Arg Asp Glu His Gly Lys Glu His Gln Lys Gln Tyr
1265 1270 1275 1280
Thr Ile Ser Val Asn Asp Lys Lys Pro Met Ile Thr Gln Gly Arg Phe
1285 1290 1295
Asp Thr Ile Asn Gly Val Asp His Phe Thr Pro Asp Lys Thr Lys Ala
1300 1305 1310
Leu Gly Ser Ser Gly Ile Val Arg Glu Glu Val Phe Tyr Leu Ala Lys
1315 1320 1325
Lys Asn Gly Arg Lys Phe Asp Val Thr Glu Gly Lys Asp Gly Ile Thr
1330 1335 1340
Val Ser Asp Asn Lys Val Tyr Ile Pro Lys Asn Pro Asp Gly Ser Tyr
1345 1350 1355 1360
Thr Ile Ser Lys Arg Asp Gly Val Thr Leu Ser Asp Tyr Tyr Tyr Leu
1365 1370 1375
Val Glu Asp Arg Ala Gly Asn Val Ser Phe Ala Thr Leu Arg Asp Leu
1380 1385 1390
Lys Val Val Gly Lys Asp Lys Ala Val Val Asn Phe Gly Leu Asp Leu
1395 1400 1405
Pro Val Pro Glu Asp Lys Gln Ile Val Asn Phe Thr Tyr Leu Val Arg
1410 1415 1420
Asp Ala Asp Gly Lys Pro Ile Glu Asn Leu Glu Tyr Tyr Asn Asn Ser
1425 1430 1435 1440
Gly Asn Ser Leu Ile Leu Pro Tyr Gly Lys Tyr Thr Val Glu Leu Leu
1445 1450 1455
Thr Tyr Asp Thr Asn Ala Ala Lys Leu Glu Ser Asp Lys Ile Val Ser
1460 1465 1470
Phe Thr Leu Ser Ala Asp Asn Asn Phe Gln Gln Val Thr Phe Lys Met
1475 1480 1485
Thr Met Leu Ala Thr Ser Gln Ile Thr Ala His Phe Asp His Leu Leu
1490 1495 1500
Pro Glu Gly Ser Arg Val Ser Leu Lys Thr Ala Gln Gly Gln Leu Ile
1505 1510 1515 1520
Pro Leu Glu Gln Ser Leu Tyr Val Pro Lys Ala Tyr Gly Lys Thr Val
1525 1530 1535
Gln Glu Gly Thr Tyr Glu Val Val Val Ser Leu Pro Lys Gly Tyr Arg
1540 1545 1550
Ile Glu Gly Asp Thr Lys Val Asn Ala Leu Pro Asn Glu Val His Glu
1555 1560 1565
Leu Ser Leu Arg Leu Val Lys Val Gly Asp Ala Ser Asp Ser Thr Gly
1570 1575 1580
Asp His Lys Val Met Ser Lys Asn Asn Ser Gln Ala Leu Thr Ala Ser

```
1585                1590                1595                1600
Ala Thr Pro Thr Lys Thr Thr Thr Ser Ala Thr Ala Lys Ala Leu Pro
                1605                1610                1615
Ser Ala Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 39
<211> 1648
<212> PRT
<213> Streptococcus pyogenes

<400> 39

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
```

```
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Val Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
```

Leu Gly Ser Glu Thr Asn Val Ser Thr Lys Met Ile Ser Asp Asn Gly
865 870 875 880
Leu His Thr Leu Gly Thr Phe Lys Asn Ala Asp Gly Lys Phe Ile Leu
885 890 895
Glu Lys Asn Ala Gln Gly Asn Pro Val Leu Ala Ile Ser Pro Asn Gly
900 905 910
Asp Asn Asn Gln Asp Phe Ala Ala Phe Lys Gly Val Phe Leu Arg Lys
915 920 925
Tyr Gln Gly Leu Lys Ala Ser Val Tyr His Ala Ser Asp Lys Glu His
930 935 940
Lys Asn Pro Leu Trp Val Ser Pro Glu Ser Phe Lys Gly Asp Lys Asn
945 950 955 960
Phe Asn Ser Asp Ile Arg Phe Ala Lys Ser Thr Thr Leu Leu Gly Thr
965 970 975
Ala Phe Ser Gly Lys Ser Leu Thr Gly Ala Glu Leu Pro Asp Gly Tyr
980 985 990
Tyr His Tyr Val Val Ser Tyr Tyr Pro Asp Val Val Gly Ala Lys Arg
995 1000 1005
Gln Glu Met Thr Phe Asp Met Ile Leu Asp Arg Gln Lys Pro Val Leu
1010 1015 1020
Ser Gln Ala Thr Phe Asp Pro Glu Thr Asn Arg Phe Lys Pro Glu Pro
1025 1030 1035 1040
Leu Lys Asp Arg Gly Leu Ala Gly Val Arg Lys Asp Ser Val Phe Tyr
1045 1050 1055
Leu Glu Arg Lys Asp Asn Lys Pro Tyr Thr Val Thr Ile Asn Asp Ser
1060 1065 1070
Tyr Lys Tyr Val Ser Val Glu Asp Ser Lys Thr Phe Val Glu Arg Gln
1075 1080 1085
Ala Asp Gly Ser Phe Ile Leu Pro Leu Asp Lys Ala Lys Leu Gly Asp
1090 1095 1100
Phe Tyr Tyr Met Val Glu Asp Phe Ala Gly Asn Val Ala Ile Ala Lys
1105 1110 1115 1120
Leu Gly Asp His Leu Pro Gln Thr Leu Gly Lys Thr Pro Ile Lys Leu
1125 1130 1135
Lys Leu Thr Asp Gly Asn Tyr Gln Thr Lys Glu Thr Leu Lys Asp Asn
1140 1145 1150
Leu Glu Met Thr Gln Ser Asp Thr Gly Leu Val Thr Asn Gln Ala Gln
1155 1160 1165
Leu Ala Val Val His Arg Asn Gln Pro Gln Ser Gln Leu Thr Lys Met
1170 1175 1180
Asn Gln Asp Phe Phe Ile Ser Pro Asn Glu Asp Gly Asn Lys Asp Phe
1185 1190 1195 1200
Val Ala Phe Lys Gly Leu Lys Asn Asn Val Tyr Asn Asp Leu Thr Val
1205 1210 1215
Asn Val Tyr Ala Lys Asp Asp His Gln Lys Gln Thr Pro Ile Trp Ser
1220 1225 1230
Ser Gln Ala Gly Ala Ser Ala Ser Ala Ile Glu Ser Thr Ala Trp Tyr
1235 1240 1245
Gly Ile Thr Ala Arg Gly Ser Lys Val Met Pro Gly Asp Tyr Gln Tyr
1250 1255 1260
Val Val Thr Tyr Arg Asp Glu His Gly Lys Glu His Gln Lys Gln Tyr
1265 1270 1275 1280
Thr Ile Ser Val Asn Asp Lys Lys Pro Met Ile Thr Gln Gly Arg Phe
1285 1290 1295
Asp Thr Ile Asn Gly Val Asp His Phe Thr Pro Asp Lys Thr Lys Ala
1300 1305 1310
Leu Gly Ser Ser Gly Ile Val Arg Glu Glu Val Phe Tyr Leu Ala Lys
1315 1320 1325
Lys Asn Gly Arg Lys Phe Asp Val Thr Glu Gly Lys Asp Gly Ile Thr
1330 1335 1340
Val Ser Asp Asn Lys Val Tyr Ile Pro Lys Asn Pro Asp Gly Ser Tyr

```
1345                1350                1355                1360
Thr Ile Ser Lys Arg Asp Gly Val Thr Leu Ser Asp Tyr Tyr Tyr Leu
                1365                1370                1375
Val Glu Asp Arg Ala Gly Asn Val Ser Phe Ala Thr Leu Arg Asp Leu
            1380                1385                1390
Lys Val Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Val Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asp Thr Lys Val Asn Ala Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
                1605                1610                1615
Ser Ala Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 40
<211> 1648
<212> PRT
<213> Streptococcus pyogenes

<400> 40

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
```

```
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Val Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
```

```
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Met Ile Ser Asp Asn Gly
865                 870                 875                 880
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly Tyr
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Ser Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
Phe Tyr Tyr Met Val Glu Asp Phe Ala Gly Asn Val Ala Ile Ala Lys
```

```
1105                1110                1115                1120
Leu Gly Asp His Leu Pro Gln Thr Leu Gly Lys Thr Pro Ile Lys Leu
                1125                1130                1135
Lys Leu Thr Asp Gly Asn Tyr Gln Thr Lys Glu Thr Leu Lys Asp Asn
            1140                1145                1150
Leu Glu Met Thr Gln Ser Asp Thr Gly Leu Val Thr Asn Gln Ala Gln
        1155                1160                1165
Leu Ala Val Val His Arg Asn Gln Pro Gln Ser Gln Leu Thr Lys Met
    1170                1175                1180
Asn Gln Asp Phe Phe Ile Ser Pro Asn Glu Asp Gly Asn Lys Asp Phe
1185                1190                1195                1200
Val Ala Phe Lys Gly Leu Lys Asn Asn Val Tyr Asn Asp Leu Thr Val
                1205                1210                1215
Asn Val Tyr Ala Lys Asp Asp His Gln Lys Gln Thr Pro Ile Trp Ser
            1220                1225                1230
Ser Gln Ala Gly Ala Ser Ala Ser Ala Ile Glu Ser Thr Ala Trp Tyr
        1235                1240                1245
Gly Ile Thr Ala Arg Gly Ser Lys Val Met Pro Gly Asp Tyr Gln Tyr
    1250                1255                1260
Val Val Thr Tyr Arg Asp Glu His Gly Lys Glu His Gln Lys Gln Tyr
1265                1270                1275                1280
Thr Ile Ser Val Asn Asp Lys Lys Pro Met Ile Thr Gln Gly Arg Phe
                1285                1290                1295
Asp Thr Ile Asn Gly Val Asp His Phe Thr Pro Asp Lys Thr Lys Ala
            1300                1305                1310
Leu Gly Ser Ser Gly Ile Val Arg Glu Glu Val Phe Tyr Leu Ala Lys
        1315                1320                1325
Lys Asn Gly Arg Lys Phe Asp Val Thr Glu Gly Lys Asp Gly Ile Thr
    1330                1335                1340
Val Ser Asp Asn Lys Val Tyr Ile Pro Lys Asn Pro Asp Gly Ser Tyr
1345                1350                1355                1360
Thr Ile Ser Lys Arg Asp Gly Val Thr Leu Ser Asp Tyr Tyr Tyr Leu
                1365                1370                1375
Val Glu Asp Arg Ala Gly Asn Val Ser Phe Ala Thr Leu Arg Asp Leu
            1380                1385                1390
Lys Val Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Val Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asp Thr Lys Val Asn Ala Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
```

```
Ala Thr Pro Thr Lys Thr Thr Thr Ser Ala Thr Ala Lys Ala Leu Pro
                1605                1610                1615
Ser Ala Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 41
<211> 1648
<212> PRT
<213> Streptococcus pyogenes

<400> 41

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
```

```
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Val Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Met Ile Ser Asp Asn Gly
```

```
865                 870                 875                 880
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly Tyr
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Ser Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
Phe Tyr Tyr Met Val Glu Asp Phe Ala Gly Asn Val Ala Ile Ala Lys
1105                1110                1115                1120
Leu Gly Asp His Leu Pro Gln Thr Leu Gly Lys Thr Pro Ile Lys Leu
                1125                1130                1135
Lys Leu Thr Asp Gly Asn Tyr Gln Thr Lys Glu Thr Leu Lys Asp Asn
            1140                1145                1150
Leu Glu Met Thr Gln Ser Asp Thr Gly Leu Val Thr Asn Gln Ala Gln
        1155                1160                1165
Leu Ala Val Val His Arg Asn Gln Pro Gln Ser Gln Leu Thr Lys Met
    1170                1175                1180
Asn Gln Asp Phe Phe Ile Ser Pro Asn Glu Asp Gly Asn Lys Asp Phe
1185                1190                1195                1200
Val Ala Phe Lys Gly Leu Lys Asn Asn Val Tyr Asn Asp Leu Thr Val
                1205                1210                1215
Asn Val Tyr Ala Lys Asp Asp His Gln Lys Gln Thr Pro Ile Trp Ser
            1220                1225                1230
Ser Gln Ala Gly Ala Ser Ala Ser Ala Ile Glu Ser Thr Ala Trp Tyr
        1235                1240                1245
Gly Ile Thr Ala Arg Gly Ser Lys Val Met Pro Gly Asp Tyr Gln Tyr
    1250                1255                1260
Val Val Thr Tyr Arg Asp Glu His Gly Lys Glu His Gln Lys Gln Tyr
1265                1270                1275                1280
Thr Ile Ser Val Asn Asp Lys Lys Pro Met Ile Thr Gln Gly Arg Phe
                1285                1290                1295
Asp Thr Ile Asn Gly Val Asp His Phe Thr Pro Asp Lys Thr Lys Ala
            1300                1305                1310
Leu Gly Ser Ser Gly Ile Val Arg Glu Glu Val Phe Tyr Leu Ala Lys
        1315                1320                1325
Lys Asn Gly Arg Lys Phe Asp Val Thr Glu Gly Lys Asp Gly Ile Thr
    1330                1335                1340
Val Ser Asp Asn Lys Val Tyr Ile Pro Lys Asn Pro Asp Gly Ser Tyr
1345                1350                1355                1360
```

Thr Ile Ser Lys Arg Asp Gly Val Thr Leu Ser Asp Tyr Tyr Tyr Leu
1365 1370 1375
Val Glu Asp Arg Ala Gly Asn Val Ser Phe Ala Thr Leu Arg Asp Leu
1380 1385 1390
Lys Val Val Gly Lys Asp Lys Ala Val Val Asn Phe Gly Leu Asp Leu
1395 1400 1405
Pro Val Pro Glu Asp Lys Gln Ile Val Asn Phe Thr Tyr Leu Val Arg
1410 1415 1420
Asp Ala Asp Gly Lys Pro Ile Glu Asn Leu Glu Tyr Tyr Asn Asn Ser
1425 1430 1435 1440
Gly Asn Ser Leu Ile Leu Pro Tyr Gly Lys Tyr Thr Val Glu Leu Leu
1445 1450 1455
Thr Tyr Asp Thr Asn Ala Ala Lys Leu Glu Ser Asp Lys Ile Val Ser
1460 1465 1470
Phe Thr Leu Ser Ala Asp Asn Asn Phe Gln Gln Val Thr Phe Lys Met
1475 1480 1485
Thr Met Leu Ala Thr Ser Gln Ile Thr Ala His Phe Asp His Leu Leu
1490 1495 1500
Pro Glu Gly Ser Arg Val Ser Leu Lys Thr Ala Gln Gly Gln Leu Ile
1505 1510 1515 1520
Pro Leu Glu Gln Ser Leu Tyr Val Pro Lys Ala Tyr Gly Lys Thr Val
1525 1530 1535
Gln Glu Gly Thr Tyr Glu Val Val Val Ser Leu Pro Lys Gly Tyr Arg
1540 1545 1550
Ile Glu Gly Asp Thr Lys Val Asn Ala Leu Pro Asn Glu Val His Glu
1555 1560 1565
Leu Ser Leu Arg Leu Val Lys Val Gly Asp Ala Ser Asp Ser Thr Gly
1570 1575 1580
Asp His Lys Val Met Ser Lys Asn Asn Ser Gln Ala Leu Thr Ala Ser
1585 1590 1595 1600
Ala Thr Pro Thr Lys Thr Thr Thr Ser Ala Thr Ala Lys Ala Leu Pro
1605 1610 1615
Ser Ala Gly Glu Lys Met Gly Leu Lys Leu Arg Ile Val Gly Leu Val
1620 1625 1630
Leu Leu Gly Leu Thr Cys Val Phe Ser Arg Lys Lys Ser Thr Lys Asp
1635 1640 1645

<210> 42
<211> 1648
<212> PRT
<213> Streptococcus pyogenes

<400> 42

```
Val Glu Lys Lys Gln Arg Phe Ser Leu Arg Lys Tyr Lys Ser Gly Thr
 1              5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
```

```
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Val Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
```

```
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Met Ile Ser Asp Asn Gly
865                 870                 875                 880
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly Tyr
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Ser Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
Phe Tyr Tyr Met Val Glu Asp Phe Ala Gly Asn Val Ala Ile Ala Lys
1105                1110                1115                1120
```

```
Leu Gly Asp His Leu Pro Gln Thr Leu Gly Lys Thr Pro Ile Lys Leu
                1125                1130                1135
Lys Leu Thr Asp Gly Asn Tyr Gln Thr Lys Glu Thr Leu Lys Asp Asn
            1140                1145                1150
Leu Glu Met Thr Gln Ser Asp Thr Gly Leu Val Thr Asn Gln Ala Gln
        1155                1160                1165
Leu Ala Val Val His Arg Asn Gln Pro Gln Ser Gln Leu Thr Lys Met
    1170                1175                1180
Asn Gln Asp Phe Phe Ile Ser Pro Asn Glu Asp Gly Asn Lys Asp Phe
1185                1190                1195                1200
Val Ala Phe Lys Gly Leu Lys Asn Asn Val Tyr Asn Asp Leu Thr Val
                1205                1210                1215
Asn Val Tyr Ala Lys Asp Asp His Gln Lys Gln Thr Pro Ile Trp Ser
            1220                1225                1230
Ser Gln Ala Gly Ala Ser Ala Ser Ala Ile Glu Ser Thr Ala Trp Tyr
        1235                1240                1245
Gly Ile Thr Ala Arg Gly Ser Lys Val Met Pro Gly Asp Tyr Gln Tyr
    1250                1255                1260
Val Val Thr Tyr Arg Asp Glu His Gly Lys Glu His Gln Lys Gln Tyr
1265                1270                1275                1280
Thr Ile Ser Val Asn Asp Lys Lys Pro Met Ile Thr Gln Gly Arg Phe
                1285                1290                1295
Asp Thr Ile Asn Gly Val Asp His Phe Thr Pro Asp Lys Thr Lys Ala
            1300                1305                1310
Leu Gly Ser Ser Gly Ile Val Arg Glu Glu Val Phe Tyr Leu Ala Lys
        1315                1320                1325
Lys Asn Gly Arg Lys Phe Asp Val Thr Glu Gly Lys Asp Gly Ile Thr
    1330                1335                1340
Val Ser Asp Asn Lys Val Tyr Ile Pro Lys Asn Pro Asp Gly Ser Tyr
1345                1350                1355                1360
Thr Ile Ser Lys Arg Asp Gly Val Thr Leu Ser Asp Tyr Tyr Tyr Leu
                1365                1370                1375
Val Glu Asp Arg Ala Gly Asn Val Ser Phe Ala Thr Leu Arg Asp Leu
            1380                1385                1390
Lys Val Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Val Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asp Thr Lys Val Asn Ala Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
```

```
                    1605                1610                1615
        Ser Ala Gly Glu Lys Met Gly Leu Lys Leu Arg Ile Val Gly Leu Val
                1620                1625                1630
        Leu Leu Gly Leu Thr Cys Val Phe Ser Arg Lys Lys Ser Thr Lys Asp
            1635                1640                1645
```

<210> 43
<211> 1648
<212> PRT
<213> Streptococcus pyogenes

<400> 43

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Met Thr Thr Thr
            20                  25                  30
Val Ala Ala Asp Glu Leu Thr Thr Thr Ser Glu Pro Thr Ile Thr Asn
        35                  40                  45
His Thr Gln Gln Gln Ala Gln His Leu Thr Asn Thr Glu Leu Ser Ser
    50                  55                  60
Ala Glu Ser Lys Pro Gln Asp Thr Ser Gln Ile Thr Leu Lys Thr Asn
65                  70                  75                  80
Arg Glu Lys Glu Gln Pro Gln Gly Leu Val Ser Glu Pro Thr Thr Thr
                85                  90                  95
Glu Leu Ala Asp Thr Asp Ala Ala Pro Met Ala Asn Thr Gly Pro Asp
            100                 105                 110
Ala Thr Gln Lys Ser Ala Ser Leu Pro Pro Val Asn Thr Asp Val His
        115                 120                 125
Asp Trp Val Lys Thr Lys Gly Ala Trp Asp Lys Gly Tyr Lys Gly Gln
    130                 135                 140
Gly Lys Val Val Ala Val Ile Asp Thr Gly Ile Asp Pro Ala His Gln
145                 150                 155                 160
Ser Met Arg Ile Ser Asp Val Ser Thr Ala Lys Val Lys Ser Lys Glu
                165                 170                 175
Asp Met Leu Ala Arg Gln Lys Ala Ala Gly Ile Asn Tyr Gly Ser Trp
            180                 185                 190
Ile Asn Asp Lys Val Val Phe Ala His Asn Tyr Val Glu Asn Ser Asp
        195                 200                 205
Asn Ile Lys Glu Asn Gln Phe Glu Asp Phe Asp Glu Asp Trp Glu Asn
    210                 215                 220
Phe Glu Phe Asp Ala Glu Ala Glu Pro Lys Ala Ile Lys Lys His Lys
225                 230                 235                 240
Ile Tyr Arg Pro Gln Ser Thr Gln Ala Pro Lys Glu Thr Val Ile Lys
                245                 250                 255
Thr Glu Glu Thr Asp Gly Ser His Asp Ile Asp Trp Thr Gln Thr Asp
            260                 265                 270
Asp Asp Thr Lys Tyr Glu Ser His Gly Met His Val Thr Gly Ile Val
        275                 280                 285
Ala Gly Asn Ser Lys Glu Ala Ala Ala Thr Gly Glu Arg Phe Leu Gly
    290                 295                 300
Ile Ala Pro Glu Ala Gln Val Met Phe Met Arg Val Phe Ala Asn Asp
305                 310                 315                 320
Val Met Gly Ser Ala Glu Ser Leu Phe Ile Lys Ala Ile Glu Asp Ala
                325                 330                 335
Val Ala Leu Gly Ala Asp Val Ile Asn Leu Ser Leu Gly Thr Ala Asn
            340                 345                 350
Gly Ala Gln Leu Ser Gly Ser Lys Pro Leu Met Glu Ala Ile Glu Lys
        355                 360                 365
Ala Lys Lys Ala Gly Val Ser Val Val Val Ala Ala Gly Asn Glu Arg
    370                 375                 380
Val Tyr Gly Ser Asp His Asp Asp Pro Leu Ala Thr Asn Pro Asp Tyr
```

```
385                 390                 395                 400
Gly Leu Val Gly Ser Pro Ser Thr Gly Arg Thr Pro Thr Ser Val Ala
                405                 410                 415
Ala Ile Asn Ser Lys Trp Val Ile Gln Arg Leu Met Thr Val Lys Glu
            420                 425                 430
Leu Glu Asn Arg Ala Asp Leu Asn His Gly Lys Ala Ile Tyr Ser Glu
        435                 440                 445
Ser Val Asp Phe Lys Asn Ile Lys Asp Ser Leu Gly Tyr Asp Lys Ser
    450                 455                 460
His Gln Phe Ala Tyr Val Lys Glu Ser Thr Asp Ala Gly Tyr Asn Ala
465                 470                 475                 480
Gln Asp Val Lys Gly Lys Ile Ala Leu Ile Glu Arg Asp Pro Asn Lys
                485                 490                 495
Thr Tyr Asp Glu Met Ile Ala Leu Ala Lys Lys His Gly Ala Leu Gly
            500                 505                 510
Val Leu Ile Phe Asn Asn Lys Pro Gly Gln Ser Asn Arg Ser Met Arg
        515                 520                 525
Leu Thr Ala Asn Gly Met Gly Ile Pro Ser Ala Phe Ile Ser His Glu
    530                 535                 540
Phe Gly Lys Ala Met Ser Gln Leu Asn Gly Asn Gly Thr Gly Ser Leu
545                 550                 555                 560
Glu Phe Asp Ser Val Val Ser Lys Ala Pro Ser Gln Lys Gly Asn Glu
                565                 570                 575
Met Asn His Phe Ser Asn Trp Gly Leu Thr Ser Asp Gly Tyr Leu Lys
            580                 585                 590
Pro Asp Ile Thr Ala Pro Gly Gly Asp Ile Tyr Ser Thr Tyr Asn Asp
        595                 600                 605
Asn His Tyr Gly Ser Gln Thr Gly Thr Ser Met Ala Ser Pro Gln Ile
    610                 615                 620
Ala Gly Ala Ser Leu Leu Val Lys Gln Tyr Leu Glu Lys Thr Gln Pro
625                 630                 635                 640
Asn Leu Pro Lys Glu Lys Ile Ala Asp Ile Val Lys Asn Leu Leu Met
                645                 650                 655
Ser Asn Ala Gln Ile His Val Asn Pro Glu Thr Lys Thr Thr Thr Ser
            660                 665                 670
Pro Arg Gln Gln Gly Ala Gly Leu Leu Asn Ile Asp Gly Ala Val Thr
        675                 680                 685
Ser Gly Leu Tyr Val Thr Gly Lys Asp Asn Tyr Gly Ser Ile Ser Leu
    690                 695                 700
Gly Asn Ile Thr Asp Thr Met Thr Phe Asp Val Thr Val His Asn Leu
705                 710                 715                 720
Ser Asn Lys Ala Lys Thr Leu Arg Tyr Asp Thr Glu Leu Leu Thr Asp
                725                 730                 735
His Val Asp Pro Gln Lys Gly Arg Phe Thr Leu Thr Ser Arg Ser Leu
            740                 745                 750
Lys Thr Tyr Gln Gly Gly Glu Val Thr Val Pro Ala Asn Gly Lys Val
        755                 760                 765
Thr Val Arg Val Thr Met Asp Val Ser Gln Phe Thr Lys Glu Leu Thr
    770                 775                 780
Lys Gln Met Pro Asn Gly Tyr Tyr Leu Glu Gly Phe Val Arg Phe Arg
785                 790                 795                 800
Asp Ser Gln Asp Asp Gln Leu Asn Arg Val Asn Ile Pro Phe Val Gly
                805                 810                 815
Phe Lys Gly Gln Phe Glu Asn Leu Ala Val Ala Glu Glu Ser Ile Tyr
            820                 825                 830
Arg Leu Lys Ser Gln Gly Lys Thr Gly Phe Tyr Phe Asp Glu Ser Gly
        835                 840                 845
Pro Lys Asp Asp Ile Tyr Val Gly Lys His Phe Thr Gly Leu Val Thr
    850                 855                 860
Leu Gly Ser Glu Thr Asn Val Ser Thr Lys Met Ile Ser Asp Asn Gly
865                 870                 875                 880
```

```
Leu His Thr Leu Gly Thr Phe Lys Asn Ala Asp Gly Lys Phe Ile Leu
                885                 890                 895
Glu Lys Asn Ala Gln Gly Asn Pro Val Leu Ala Ile Ser Pro Asn Gly
            900                 905                 910
Asp Asn Asn Gln Asp Phe Ala Ala Phe Lys Gly Val Phe Leu Arg Lys
        915                 920                 925
Tyr Gln Gly Leu Lys Ala Ser Val Tyr His Ala Ser Asp Lys Glu His
    930                 935                 940
Lys Asn Pro Leu Trp Val Ser Pro Glu Ser Phe Lys Gly Asp Lys Asn
945                 950                 955                 960
Phe Asn Ser Asp Ile Arg Phe Ala Lys Ser Thr Thr Leu Leu Gly Thr
                965                 970                 975
Ala Phe Ser Gly Lys Ser Leu Thr Gly Ala Glu Leu Pro Asp Gly Tyr
            980                 985                 990
Tyr His Tyr Val Val Ser Tyr Tyr Pro Asp Val Val Gly Ala Lys Arg
        995                 1000                1005
Gln Glu Met Thr Phe Asp Met Ile Leu Asp Arg Gln Lys Pro Val Leu
    1010                1015                1020
Ser Gln Ala Thr Phe Asp Pro Glu Thr Asn Arg Phe Lys Pro Glu Pro
1025                1030                1035                1040
Leu Lys Asp Arg Gly Leu Ala Gly Val Arg Lys Asp Ser Val Phe Tyr
                1045                1050                1055
Leu Glu Arg Lys Asp Asn Lys Pro Tyr Thr Val Thr Ile Asn Asp Ser
            1060                1065                1070
Tyr Lys Tyr Val Ser Val Glu Asp Ser Lys Thr Phe Val Glu Arg Gln
        1075                1080                1085
Ala Asp Gly Ser Phe Ile Leu Pro Leu Asp Lys Ala Lys Leu Gly Asp
    1090                1095                1100
Phe Tyr Tyr Met Val Glu Asp Phe Ala Gly Asn Val Ala Ile Ala Lys
1105                1110                1115                1120
Leu Gly Asp His Leu Pro Gln Thr Leu Gly Lys Thr Pro Ile Lys Leu
                1125                1130                1135
Lys Leu Thr Asp Gly Asn Tyr Gln Thr Lys Glu Thr Leu Lys Asp Asn
            1140                1145                1150
Leu Glu Met Thr Gln Ser Asp Thr Gly Leu Val Thr Asn Gln Ala Gln
        1155                1160                1165
Leu Ala Val Val His Arg Asn Gln Pro Gln Ser Gln Leu Thr Lys Met
    1170                1175                1180
Asn Gln Asp Phe Phe Ile Ser Pro Asn Glu Asp Gly Asn Lys Asp Phe
1185                1190                1195                1200
Val Ala Phe Lys Gly Leu Lys Asn Asn Val Tyr Asn Asp Leu Thr Val
                1205                1210                1215
Asn Val Tyr Ala Lys Asp Asp His Gln Lys Gln Thr Pro Ile Trp Ser
            1220                1225                1230
Ser Gln Ala Gly Ala Ser Ala Ser Ala Ile Glu Ser Thr Ala Trp Tyr
        1235                1240                1245
Gly Ile Thr Ala Arg Gly Ser Lys Val Met Pro Gly Asp Tyr Gln Tyr
    1250                1255                1260
Val Val Thr Tyr Arg Asp Glu His Gly Lys Glu His Gln Lys Gln Tyr
1265                1270                1275                1280
Thr Ile Ser Val Asn Asp Lys Lys Pro Met Ile Thr Gln Gly Arg Phe
                1285                1290                1295
Asp Thr Ile Asn Gly Val Asp His Phe Thr Pro Asp Lys Thr Lys Ala
            1300                1305                1310
Leu Gly Ser Ser Gly Ile Val Arg Glu Glu Val Phe Tyr Leu Ala Lys
        1315                1320                1325
Lys Asn Gly Arg Lys Phe Asp Val Thr Glu Gly Lys Asp Gly Ile Thr
    1330                1335                1340
Val Ser Asp Asn Lys Val Tyr Ile Pro Lys Asn Pro Asp Gly Ser Tyr
1345                1350                1355                1360
Thr Ile Ser Lys Arg Asp Gly Val Thr Leu Ser Asp Tyr Tyr Tyr Leu
```

```
                1365                1370                1375
Val Glu Asp Arg Ala Gly Asn Val Ser Phe Ala Thr Leu Arg Asp Leu
            1380                1385                1390
Lys Val Val Gly Lys Asp Lys Ala Val Val Asn Phe Gly Leu Asp Leu
        1395                1400                1405
Pro Val Pro Glu Asp Lys Gln Ile Val Asn Phe Thr Tyr Leu Val Arg
    1410                1415                1420
Asp Ala Asp Gly Lys Pro Ile Glu Asn Leu Glu Tyr Tyr Asn Asn Ser
1425                1430                1435                1440
Gly Asn Ser Leu Ile Leu Pro Tyr Gly Lys Tyr Thr Val Glu Leu Leu
                1445                1450                1455
Thr Tyr Asp Thr Asn Ala Ala Lys Leu Glu Ser Asp Lys Ile Val Ser
            1460                1465                1470
Phe Thr Leu Ser Ala Asp Asn Asn Phe Gln Gln Val Thr Phe Lys Met
        1475                1480                1485
Thr Met Leu Ala Thr Ser Gln Ile Thr Ala His Phe Asp His Leu Leu
    1490                1495                1500
Pro Glu Gly Ser Arg Val Ser Leu Lys Thr Ala Gln Gly Gln Leu Ile
1505                1510                1515                1520
Pro Leu Glu Gln Ser Leu Tyr Val Pro Lys Ala Tyr Gly Lys Thr Val
                1525                1530                1535
Gln Glu Gly Thr Tyr Glu Val Val Val Ser Leu Pro Lys Gly Tyr Arg
            1540                1545                1550
Ile Glu Gly Asp Thr Lys Val Asn Ala Leu Pro Asn Glu Val His Glu
        1555                1560                1565
Leu Ser Leu Arg Leu Val Lys Val Gly Asp Ala Ser Asp Ser Thr Gly
    1570                1575                1580
Asp His Lys Val Met Ser Lys Asn Asn Ser Gln Ala Leu Thr Ala Ser
1585                1590                1595                1600
Ala Thr Pro Thr Lys Thr Thr Thr Ser Ala Thr Ala Lys Ala Leu Pro
                1605                1610                1615
Ser Ala Gly Glu Lys Met Gly Leu Lys Leu Arg Ile Val Gly Leu Val
            1620                1625                1630
Leu Leu Gly Leu Thr Cys Val Phe Ser Arg Lys Lys Ser Thr Lys Asp
        1635                1640                1645
```

<210> 44
<211> 1647
<212> PRT
<213> Streptococcus pyogenes

<400> 44

```
Val Glu Lys Lys Gln Arg Phe Ser Leu Arg Lys Tyr Lys Ser Gly Thr
 1              5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
```

```
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asn Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
```

```
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Ala Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
```

1125 1130 1135
Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Ala Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Met Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Gly Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Thr Thr Thr Ser Ala Thr Ala Lys Ala Leu Pro Ser
1605 1610 1615

```
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 45
<211> 1647
<212> PRT
<213> Streptococcus pyogenes

<400> 45

```
Val Glu Lys Lys Gln Arg Phe Ser Leu Arg Lys Tyr Lys Ser Gly Thr
1               5                   10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
```

```
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asn Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Val Asp Gly Lys Phe Ile Leu Glu
```

```
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
```

Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Met Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Gly Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Thr Thr Thr Ser Ala Thr Ala Lys Ala Leu Pro Ser
1605 1610 1615
Ala Gly Glu Lys Met Gly Leu Lys Leu Arg Ile Val Gly Leu Val Leu
1620 1625 1630
Leu Gly Leu Thr Cys Val Phe Ser Arg Lys Lys Ser Thr Lys Asp
1635 1640 1645

<210> 46
<211> 1647
<212> PRT
<213> Streptococcus pyogenes

<400> 46

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
```

```
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asn Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
```

```
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Val Asp Gly Lys Phe Ile Leu Glu
                885                 890                 895
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
```

Leu Thr Asp Gly Asn Tyr Gln Thr Lys Glu Thr Leu Lys Asp Asn Leu
1140 1145 1150
Glu Met Thr Gln Ser Asp Thr Gly Leu Val Thr Asn Gln Ala Gln Leu
1155 1160 1165
Ala Val Val His Arg Asn Gln Pro Gln Ser Gln Leu Thr Lys Met Asn
1170 1175 1180
Gln Asp Phe Phe Ile Ser Pro Asn Glu Asp Gly Asn Lys Asp Phe Val
1185 1190 1195 1200
Ala Phe Lys Gly Leu Lys Asn Asn Val Tyr Asn Asp Leu Thr Val Asn
1205 1210 1215
Val Tyr Ala Lys Asp Asp His Gln Lys Gln Thr Pro Ile Trp Ser Ser
1220 1225 1230
Gln Ala Gly Ala Ser Ala Ser Ala Ile Glu Ser Thr Ala Trp Tyr Gly
1235 1240 1245
Ile Thr Ala Arg Gly Ser Lys Val Met Pro Gly Asp Tyr Gln Tyr Val
1250 1255 1260
Val Thr Tyr Arg Asp Glu His Gly Lys Glu His Gln Lys Gln Tyr Thr
1265 1270 1275 1280
Ile Ser Val Asn Asp Lys Lys Pro Met Ile Thr Gln Gly Arg Phe Asp
1285 1290 1295
Thr Ile Asn Gly Val Asp His Phe Thr Pro Asp Lys Thr Lys Ala Leu
1300 1305 1310
Gly Ser Ser Gly Ile Val Arg Glu Glu Val Phe Tyr Leu Ala Lys Lys
1315 1320 1325
Asn Gly Arg Lys Phe Asp Val Thr Glu Gly Lys Asp Gly Ile Thr Val
1330 1335 1340
Ser Asp Asn Lys Val Tyr Ile Pro Lys Asn Pro Asp Gly Ser Tyr Thr
1345 1350 1355 1360
Ile Ser Lys Arg Asp Gly Val Thr Leu Ser Asp Tyr Tyr Tyr Leu Val
1365 1370 1375
Glu Asp Arg Ala Gly Asn Val Ser Phe Ala Thr Leu Arg Asp Leu Lys
1380 1385 1390
Ala Val Gly Lys Asp Lys Ala Val Val Asn Phe Gly Leu Asp Leu Pro
1395 1400 1405
Val Pro Glu Asp Lys Gln Ile Val Asn Phe Thr Tyr Leu Val Arg Asp
1410 1415 1420
Ala Asp Gly Lys Pro Ile Glu Asn Leu Glu Tyr Tyr Asn Asn Ser Gly
1425 1430 1435 1440
Asn Ser Leu Ile Leu Pro Tyr Gly Lys Tyr Thr Val Glu Leu Leu Thr
1445 1450 1455
Tyr Asp Thr Asn Ala Ala Lys Leu Glu Ser Asp Lys Ile Val Ser Phe
1460 1465 1470
Thr Leu Ser Ala Asp Asn Asn Phe Gln Gln Val Thr Phe Lys Met Thr
1475 1480 1485
Met Leu Ala Thr Ser Gln Ile Thr Ala His Phe Asp His Leu Leu Pro
1490 1495 1500
Glu Gly Ser Arg Val Ser Leu Lys Thr Ala Gln Gly Gln Leu Ile Pro
1505 1510 1515 1520
Leu Glu Gln Ser Leu Tyr Val Pro Lys Ala Tyr Gly Lys Thr Val Gln
1525 1530 1535
Glu Gly Thr Tyr Glu Val Val Val Ser Leu Pro Lys Gly Tyr Arg Ile
1540 1545 1550
Glu Gly Asn Thr Lys Val Asn Thr Leu Pro Asn Glu Val His Glu Leu
1555 1560 1565
Ser Leu Arg Leu Val Lys Val Gly Asp Ala Ser Asp Ser Thr Gly Asp
1570 1575 1580
His Lys Val Met Ser Lys Asn Asn Ser Gln Ala Leu Thr Ala Ser Ala
1585 1590 1595 1600
Thr Pro Thr Lys Thr Thr Thr Ser Ala Thr Ala Lys Ala Leu Pro Ser
1605 1610 1615
Ala Gly Glu Lys Met Gly Leu Lys Leu Arg Ile Val Gly Leu Val Leu

1620 1625 1630
Leu Gly Leu Thr Cys Val Phe Ser Arg Lys Lys Ser Thr Lys Asp
1635 1640 1645

<210> 47
<211> 1647
<212> PRT
<213> Streptococcus pyogenes

<400> 47

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
```

```
                405                     410                     415
Ile Asn Ser Lys Trp Val Ile Gln Arg Leu Met Thr Val Lys Glu Leu
            420                     425                     430
Glu Asn Arg Ala Asp Leu Asn His Gly Lys Ala Ile Tyr Ser Glu Ser
        435                     440                     445
Val Asp Phe Lys Asn Ile Lys Asp Ser Leu Gly Tyr Asp Lys Ser His
    450                     455                     460
Gln Phe Ala Tyr Val Lys Glu Ser Thr Asp Ala Gly Tyr Asn Ala Gln
465                     470                     475                     480
Asn Val Lys Gly Lys Ile Ala Leu Ile Glu Arg Asp Pro Asn Lys Thr
                485                     490                     495
Tyr Asp Glu Met Ile Ala Leu Ala Lys Lys His Gly Ala Leu Gly Val
            500                     505                     510
Leu Ile Phe Asn Asn Lys Pro Gly Gln Ser Asn Arg Ser Met Arg Leu
        515                     520                     525
Thr Ala Asn Gly Met Gly Ile Pro Ser Ala Phe Ile Ser His Glu Phe
    530                     535                     540
Gly Lys Ala Met Ser Gln Leu Asn Gly Asn Gly Thr Gly Ser Leu Glu
545                     550                     555                     560
Phe Asp Ser Val Val Ser Lys Ala Pro Ser Gln Lys Gly Asn Glu Met
                565                     570                     575
Asn His Phe Ser Asn Trp Gly Leu Thr Ser Asp Gly Tyr Leu Lys Pro
            580                     585                     590
Asp Ile Thr Ala Pro Gly Gly Asp Ile Tyr Ser Thr Tyr Asn Asp Asn
        595                     600                     605
His Tyr Gly Ser Gln Thr Gly Thr Ser Met Ala Ser Pro Gln Ile Ala
    610                     615                     620
Gly Ala Ser Leu Leu Val Lys Gln Tyr Leu Glu Lys Thr Gln Pro Asn
625                     630                     635                     640
Leu Pro Lys Glu Lys Ile Ala Asp Ile Val Lys Asn Leu Leu Met Ser
                645                     650                     655
Asn Ala Gln Ile His Val Asn Pro Glu Thr Lys Thr Thr Thr Ser Pro
            660                     665                     670
Arg Gln Gln Gly Ala Gly Leu Leu Asn Ile Asp Gly Ala Val Thr Ser
        675                     680                     685
Gly Leu Tyr Val Thr Gly Lys Asp Asn Tyr Gly Ser Ile Ser Leu Gly
    690                     695                     700
Asn Ile Thr Asp Thr Met Thr Phe Asp Val Thr Val His Asn Leu Ser
705                     710                     715                     720
Asn Lys Ala Lys Thr Leu Arg Tyr Asp Thr Glu Leu Leu Thr Asp His
                725                     730                     735
Val Asp Pro Gln Lys Gly Arg Phe Thr Leu Thr Ser Arg Ser Leu Lys
            740                     745                     750
Thr Tyr Gln Gly Gly Glu Val Thr Val Pro Ala Asn Gly Lys Val Thr
        755                     760                     765
Val Arg Val Thr Met Asp Val Ser Gln Phe Thr Lys Glu Leu Thr Lys
    770                     775                     780
Gln Met Pro Asn Gly Tyr Tyr Leu Glu Gly Phe Val Arg Phe Arg Asp
785                     790                     795                     800
Ser Gln Asp Asp Gln Leu Asn Arg Val Asn Ile Pro Phe Val Gly Phe
                805                     810                     815
Lys Gly Gln Phe Glu Asn Leu Ala Val Ala Glu Glu Ser Ile Tyr Arg
            820                     825                     830
Leu Lys Ser Gln Gly Lys Thr Gly Phe Tyr Phe Asp Glu Ser Gly Pro
        835                     840                     845
Lys Asp Asp Ile Tyr Val Gly Lys His Phe Thr Gly Leu Val Thr Leu
    850                     855                     860
Gly Ser Glu Thr Asn Val Ser Thr Lys Thr Ile Ser Asp Asn Gly Leu
865                     870                     875                     880
His Thr Leu Gly Thr Phe Lys Asn Val Asp Gly Lys Phe Ile Leu Glu
                885                     890                     895
```

```
Lys Asn Ala Gln Gly Asn Pro Val Leu Ala Ile Ser Pro Asn Gly Asp
            900                 905                 910
Asn Asn Gln Asp Phe Ala Ala Phe Lys Gly Val Phe Leu Arg Lys Tyr
        915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
```

```
                1380                1385                1390
Ala Val Gly Lys Asp Lys Ala Val Val Asn Phe Gly Leu Asp Leu Pro
            1395                1400                1405
Val Pro Glu Asp Lys Gln Ile Val Asn Phe Thr Tyr Leu Val Arg Asp
        1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                    1445                1450                1455
Tyr Asp Thr Asn Ala Ala Lys Leu Glu Ser Asp Lys Ile Val Ser Phe
                1460                1465                1470
Thr Leu Ser Ala Asp Asn Asn Phe Gln Gln Val Thr Phe Lys Met Thr
            1475                1480                1485
Met Leu Ala Thr Ser Gln Ile Thr Ala His Phe Asp His Leu Leu Pro
        1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                    1525                1530                1535
Glu Gly Thr Tyr Glu Val Val Val Ser Leu Pro Lys Gly Tyr Arg Ile
                1540                1545                1550
Glu Gly Asn Thr Lys Val Asn Thr Leu Pro Asn Glu Val His Glu Leu
            1555                1560                1565
Ser Leu Arg Leu Val Lys Val Gly Asp Ala Ser Asp Ser Thr Gly Asp
        1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                    1605                1610                1615
Ala Gly Glu Lys Met Gly Leu Lys Leu Arg Ile Val Gly Leu Val Leu
                1620                1625                1630
Leu Gly Leu Thr Cys Val Phe Ser Arg Lys Lys Ser Thr Lys Asp
            1635                1640                1645
```

<210> 48
<211> 1647
<212> PRT
<213> Streptococcus pyogenes

<400> 48

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
```

```
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asn Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
```

Asn Ala Gln Ile His Val Asn Pro Glu Thr Lys Thr Thr Thr Ser Pro
660 665 670
Arg Gln Gln Gly Ala Gly Leu Leu Asn Ile Asp Gly Ala Val Thr Ser
675 680 685
Gly Leu Tyr Val Thr Gly Lys Asp Asn Tyr Gly Ser Ile Ser Leu Gly
690 695 700
Asn Ile Thr Asp Thr Met Thr Phe Asp Val Thr Val His Asn Leu Ser
705 710 715 720
Asn Lys Ala Lys Thr Leu Arg Tyr Asp Thr Glu Leu Leu Thr Asp His
725 730 735
Val Asp Pro Gln Lys Gly Arg Phe Thr Leu Thr Ser Arg Ser Leu Lys
740 745 750
Thr Tyr Gln Gly Gly Glu Val Thr Val Pro Ala Asn Gly Lys Val Thr
755 760 765
Val Arg Val Thr Met Asp Val Ser Gln Phe Thr Lys Glu Leu Thr Lys
770 775 780
Gln Met Pro Asn Gly Tyr Tyr Leu Glu Gly Phe Val Arg Phe Arg Asp
785 790 795 800
Ser Gln Asp Asp Gln Leu Asn Arg Val Asn Ile Pro Phe Val Gly Phe
805 810 815
Lys Gly Gln Phe Glu Asn Leu Ala Val Ala Glu Glu Ser Ile Tyr Arg
820 825 830
Leu Lys Ser Gln Gly Lys Thr Gly Phe Tyr Phe Asp Glu Ser Gly Pro
835 840 845
Lys Asp Asp Ile Tyr Val Gly Lys His Phe Thr Gly Leu Val Thr Leu
850 855 860
Gly Ser Glu Thr Asn Val Ser Thr Lys Thr Ile Ser Asp Asn Gly Leu
865 870 875 880
His Thr Leu Gly Thr Phe Lys Asn Val Asp Gly Lys Phe Ile Leu Glu
885 890 895
Lys Asn Ala Gln Gly Asn Pro Val Leu Ala Ile Ser Pro Asn Gly Asp
900 905 910
Asn Asn Gln Asp Phe Ala Ala Phe Lys Gly Val Phe Leu Arg Lys Tyr
915 920 925
Gln Gly Leu Lys Ala Ser Val Tyr His Ala Ser Asp Lys Glu His Lys
930 935 940
Asn Pro Leu Trp Val Ser Pro Glu Ser Phe Lys Gly Asp Lys Asn Phe
945 950 955 960
Asn Ser Asp Ile Arg Phe Ala Lys Ser Thr Thr Leu Leu Gly Thr Ala
965 970 975
Phe Ser Gly Lys Ser Leu Thr Gly Ala Glu Leu Pro Asp Gly Tyr Tyr
980 985 990
His Tyr Val Val Ser Tyr Tyr Pro Asp Val Val Gly Ala Lys Arg Gln
995 1000 1005
Glu Met Thr Phe Asp Met Ile Leu Asp Arg Gln Lys Pro Val Leu Ser
1010 1015 1020
Gln Ala Thr Phe Asp Pro Glu Thr Asn Arg Phe Lys Pro Glu Pro Leu
1025 1030 1035 1040
Lys Asp Arg Gly Leu Ala Gly Val Arg Lys Asp Ser Val Phe Tyr Leu
1045 1050 1055
Glu Arg Lys Asp Asn Lys Pro Tyr Thr Val Thr Ile Asn Asp Ser Tyr
1060 1065 1070
Lys Tyr Val Ser Val Glu Asp Asn Lys Thr Phe Val Glu Arg Gln Ala
1075 1080 1085
Asp Gly Ser Phe Ile Leu Pro Leu Asp Lys Ala Lys Leu Gly Asp Phe
1090 1095 1100
Tyr Tyr Met Val Glu Asp Phe Ala Gly Asn Val Ala Ile Ala Lys Leu
1105 1110 1115 1120
Gly Asp His Leu Pro Gln Thr Leu Gly Lys Thr Pro Ile Lys Leu Lys
1125 1130 1135
Leu Thr Asp Gly Asn Tyr Gln Thr Lys Glu Thr Leu Lys Asp Asn Leu

```
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
```

Leu Gly Leu Thr Cys Val Phe Ser Arg Lys Lys Ser Thr Lys Asp
1635 1640 1645

<210> 49
<211> 1647
<212> PRT
<213> Streptococcus pyogenes

<400> 49

```
Val Glu Lys Lys Gln Arg Phe Ser Leu Arg Lys Tyr Lys Ser Gly Thr
 1               5                  10                  15
Phe Ser Val Leu Ile Gly Ser Val Phe Leu Met Met Thr Thr Thr Val
            20                  25                  30
Ala Ala Asp Glu Leu Thr Thr Thr Ser Glu Pro Thr Ile Thr Asn His
        35                  40                  45
Thr Gln Gln Gln Ala Gln His Leu Thr Asn Thr Glu Leu Ser Ser Ala
    50                  55                  60
Glu Ser Lys Pro Gln Asp Thr Ser Gln Ile Thr Leu Lys Thr Asn Arg
65                  70                  75                  80
Glu Lys Glu Gln Pro Gln Gly Leu Val Ser Glu Pro Thr Thr Thr Glu
                85                  90                  95
Leu Ala Asp Thr Asp Ala Ala Pro Met Ala Asn Thr Gly Pro Asp Ala
            100                 105                 110
Thr Gln Lys Ser Ala Ser Leu Pro Pro Val Asn Thr Asp Val His Asp
        115                 120                 125
Trp Val Lys Thr Lys Gly Ala Trp Asp Lys Gly Tyr Lys Gly Gln Gly
    130                 135                 140
Lys Val Val Ala Val Ile Asp Thr Gly Ile Asp Pro Ala His Gln Ser
145                 150                 155                 160
Met Arg Ile Ser Asp Val Ser Thr Ala Lys Val Lys Ser Lys Glu Asp
                165                 170                 175
Met Leu Ala Arg Gln Lys Ala Ala Gly Ile Asn Tyr Gly Ser Trp Ile
            180                 185                 190
Asn Asp Lys Val Val Phe Ala His Asn Tyr Val Glu Asn Ser Asp Asn
        195                 200                 205
Ile Lys Glu Asn Gln Phe Glu Asp Phe Asp Glu Asp Trp Glu Asn Phe
    210                 215                 220
Glu Phe Asp Ala Glu Ala Glu Pro Lys Ala Ile Lys Lys His Lys Ile
225                 230                 235                 240
Tyr Arg Pro Gln Ser Thr Gln Ala Pro Lys Glu Thr Val Ile Lys Thr
                245                 250                 255
Glu Glu Thr Asp Gly Ser His Asp Ile Asp Trp Thr Gln Thr Asp Asp
            260                 265                 270
Asp Thr Lys Tyr Glu Ser His Gly Met His Val Thr Gly Ile Val Ala
        275                 280                 285
Gly Asn Ser Lys Glu Ala Ala Ala Thr Gly Glu Arg Phe Leu Gly Ile
    290                 295                 300
Ala Pro Glu Ala Gln Val Met Phe Met Arg Val Phe Ala Asn Asp Val
305                 310                 315                 320
Met Gly Ser Ala Glu Ser Leu Phe Ile Lys Ala Ile Glu Asp Ala Val
                325                 330                 335
Ala Leu Gly Ala Asp Val Ile Asn Leu Ser Leu Gly Thr Ala Asn Gly
            340                 345                 350
Ala Gln Leu Ser Gly Ser Lys Pro Leu Met Glu Ala Ile Glu Lys Ala
        355                 360                 365
Lys Lys Ala Gly Val Ser Val Val Val Ala Ala Gly Asn Glu Arg Val
    370                 375                 380
Tyr Gly Ser Asp His Asp Asp Pro Leu Ala Thr Asn Pro Asp Tyr Gly
385                 390                 395                 400
Leu Val Gly Ser Pro Ser Thr Gly Arg Thr Pro Thr Ser Val Ala Ala
                405                 410                 415
```

```
Ile Asn Ser Lys Trp Val Ile Gln Arg Leu Met Thr Val Lys Glu Leu
            420                 425                 430
Glu Asn Arg Ala Asp Leu Asn His Gly Lys Ala Ile Tyr Ser Glu Ser
        435                 440                 445
Val Asp Phe Lys Asn Ile Lys Asp Ser Leu Gly Tyr Asp Lys Ser His
    450                 455                 460
Gln Phe Ala Tyr Val Lys Glu Ser Thr Asp Ala Gly Tyr Asn Ala Gln
465                 470                 475                 480
Asn Val Lys Gly Lys Ile Ala Leu Ile Glu Arg Asp Pro Asn Lys Thr
                485                 490                 495
Tyr Asp Glu Met Ile Ala Leu Ala Lys Lys His Gly Ala Leu Gly Val
            500                 505                 510
Leu Ile Phe Asn Asn Lys Pro Gly Gln Ser Asn Arg Ser Met Arg Leu
        515                 520                 525
Thr Ala Asn Gly Met Gly Ile Pro Ser Ala Phe Ile Ser His Glu Phe
    530                 535                 540
Gly Lys Ala Met Ser Gln Leu Asn Gly Asn Gly Thr Gly Ser Leu Glu
545                 550                 555                 560
Phe Asp Ser Val Val Ser Lys Ala Pro Ser Gln Lys Gly Asn Glu Met
                565                 570                 575
Asn His Phe Ser Asn Trp Gly Leu Thr Ser Asp Gly Tyr Leu Lys Pro
            580                 585                 590
Asp Ile Thr Ala Pro Gly Gly Asp Ile Tyr Ser Thr Tyr Asn Asp Asn
        595                 600                 605
His Tyr Gly Ser Gln Thr Gly Thr Ser Met Ala Ser Pro Gln Ile Ala
    610                 615                 620
Gly Ala Ser Leu Leu Val Lys Gln Tyr Leu Glu Lys Thr Gln Pro Asn
625                 630                 635                 640
Leu Pro Lys Glu Lys Ile Ala Asp Ile Val Lys Asn Leu Leu Met Ser
                645                 650                 655
Asn Ala Gln Ile His Val Asn Pro Glu Thr Lys Thr Thr Thr Ser Pro
            660                 665                 670
Arg Gln Gln Gly Ala Gly Leu Leu Asn Ile Asp Gly Ala Val Thr Ser
        675                 680                 685
Gly Leu Tyr Val Thr Gly Lys Asp Asn Tyr Gly Ser Ile Ser Leu Gly
    690                 695                 700
Asn Ile Thr Asp Thr Met Thr Phe Asp Val Thr Val His Asn Leu Ser
705                 710                 715                 720
Asn Lys Ala Lys Thr Leu Arg Tyr Asp Thr Glu Leu Leu Thr Asp His
                725                 730                 735
Val Asp Pro Gln Lys Gly Arg Phe Thr Leu Thr Ser Arg Ser Leu Lys
            740                 745                 750
Thr Tyr Gln Gly Gly Glu Val Thr Val Pro Ala Asn Gly Lys Val Thr
        755                 760                 765
Val Arg Val Thr Met Asp Val Ser Gln Phe Thr Lys Glu Leu Thr Lys
    770                 775                 780
Gln Met Pro Asn Gly Tyr Tyr Leu Glu Gly Phe Val Arg Phe Arg Asp
785                 790                 795                 800
Ser Gln Asp Asp Gln Leu Asn Arg Val Asn Ile Pro Phe Val Gly Phe
                805                 810                 815
Lys Gly Gln Phe Glu Asn Leu Ala Val Ala Glu Glu Ser Ile Tyr Arg
            820                 825                 830
Leu Lys Ser Gln Gly Lys Thr Gly Phe Tyr Phe Asp Glu Ser Gly Pro
        835                 840                 845
Lys Asp Asp Ile Tyr Val Gly Lys His Phe Thr Gly Leu Val Thr Leu
    850                 855                 860
Gly Ser Glu Thr Asn Val Ser Thr Lys Thr Ile Ser Asp Asn Gly Leu
865                 870                 875                 880
His Thr Leu Gly Thr Phe Lys Asn Val Asp Gly Lys Phe Ile Leu Glu
                885                 890                 895
Lys Asn Ala Gln Gly Asn Pro Val Leu Ala Ile Ser Pro Asn Gly Asp
```

```
        900                 905                 910
Asn Asn Gln Asp Phe Ala Ala Phe Lys Gly Val Phe Leu Arg Lys Tyr
    915                 920                 925
Gln Gly Leu Lys Ala Ser Val Tyr His Ala Ser Asp Lys Glu His Lys
    930                 935                 940
Asn Pro Leu Trp Val Ser Pro Glu Ser Phe Lys Gly Asp Lys Asn Phe
945                 950                 955                 960
Asn Ser Asp Ile Arg Phe Ala Lys Ser Thr Thr Leu Leu Gly Thr Ala
                965                 970                 975
Phe Ser Gly Lys Ser Leu Thr Gly Ala Glu Leu Pro Asp Gly Tyr Tyr
            980                 985                 990
His Tyr Val Val Ser Tyr Tyr Pro Asp Val Val Gly Ala Lys Arg Gln
        995                 1000                1005
Glu Met Thr Phe Asp Met Ile Leu Asp Arg Gln Lys Pro Val Leu Ser
    1010                1015                1020
Gln Ala Thr Phe Asp Pro Glu Thr Asn Arg Phe Lys Pro Glu Pro Leu
1025                1030                1035                1040
Lys Asp Arg Gly Leu Ala Gly Val Arg Lys Asp Ser Val Phe Tyr Leu
                1045                1050                1055
Glu Arg Lys Asp Asn Lys Pro Tyr Thr Val Thr Ile Asn Asp Ser Tyr
            1060                1065                1070
Lys Tyr Val Ser Val Glu Asp Asn Lys Thr Phe Val Glu Arg Gln Ala
        1075                1080                1085
Asp Gly Ser Phe Ile Leu Pro Leu Asp Lys Ala Lys Leu Gly Asp Phe
    1090                1095                1100
Tyr Tyr Met Val Glu Asp Phe Ala Gly Asn Val Ala Ile Ala Lys Leu
1105                1110                1115                1120
Gly Asp His Leu Pro Gln Thr Leu Gly Lys Thr Pro Ile Lys Leu Lys
                1125                1130                1135
Leu Thr Asp Gly Asn Tyr Gln Thr Lys Glu Thr Leu Lys Asp Asn Leu
            1140                1145                1150
Glu Met Thr Gln Ser Asp Thr Gly Leu Val Thr Asn Gln Ala Gln Leu
        1155                1160                1165
Ala Val Val His Arg Asn Gln Pro Gln Ser Gln Leu Thr Lys Met Asn
    1170                1175                1180
Gln Asp Phe Phe Ile Ser Pro Asn Glu Asp Gly Asn Lys Asp Phe Val
1185                1190                1195                1200
Ala Phe Lys Gly Leu Lys Asn Asn Val Tyr Asn Asp Leu Thr Val Asn
                1205                1210                1215
Val Tyr Ala Lys Asp Asp His Gln Lys Gln Thr Pro Ile Trp Ser Ser
            1220                1225                1230
Gln Ala Gly Ala Ser Ala Ser Ala Ile Glu Ser Thr Ala Trp Tyr Gly
        1235                1240                1245
Ile Thr Ala Arg Gly Ser Lys Val Met Pro Gly Asp Tyr Gln Tyr Val
    1250                1255                1260
Val Thr Tyr Arg Asp Glu His Gly Lys Glu His Gln Lys Gln Tyr Thr
1265                1270                1275                1280
Ile Ser Val Asn Asp Lys Lys Pro Met Ile Thr Gln Gly Arg Phe Asp
                1285                1290                1295
Thr Ile Asn Gly Val Asp His Phe Thr Pro Asp Lys Thr Lys Ala Leu
            1300                1305                1310
Gly Ser Ser Gly Ile Val Arg Glu Glu Val Phe Tyr Leu Ala Lys Lys
        1315                1320                1325
Asn Gly Arg Lys Phe Asp Val Thr Glu Gly Lys Asp Gly Ile Thr Val
    1330                1335                1340
Ser Asp Asn Lys Val Tyr Ile Pro Lys Asn Pro Asp Gly Ser Tyr Thr
1345                1350                1355                1360
Ile Ser Lys Arg Asp Gly Val Thr Leu Ser Asp Tyr Tyr Tyr Leu Val
                1365                1370                1375
Glu Asp Arg Ala Gly Asn Val Ser Phe Ala Thr Leu Arg Asp Leu Lys
            1380                1385                1390
```

```
Ala Val Gly Lys Asp Lys Ala Val Val Asn Phe Gly Leu Asp Leu Pro
        1395                1400                1405
Val Pro Glu Asp Lys Gln Ile Val Asn Phe Thr Tyr Leu Val Arg Asp
    1410                1415                1420
Ala Asp Gly Lys Pro Ile Glu Asn Leu Glu Tyr Tyr Asn Asn Ser Gly
1425                1430                1435                1440
Asn Ser Leu Ile Leu Pro Tyr Gly Lys Tyr Thr Val Glu Leu Leu Thr
                1445                1450                1455
Tyr Asp Thr Asn Ala Ala Lys Leu Glu Ser Asp Lys Ile Val Ser Phe
            1460                1465                1470
Thr Leu Ser Ala Asp Asn Asn Phe Gln Gln Val Thr Phe Lys Met Thr
        1475                1480                1485
Met Leu Ala Thr Ser Gln Ile Thr Ala His Phe Asp His Leu Leu Pro
    1490                1495                1500
Glu Gly Ser Arg Val Ser Leu Lys Thr Ala Gln Gly Gln Leu Ile Pro
1505                1510                1515                1520
Leu Glu Gln Ser Leu Tyr Val Pro Lys Ala Tyr Gly Lys Thr Val Gln
                1525                1530                1535
Glu Gly Thr Tyr Glu Val Val Val Ser Leu Pro Lys Gly Tyr Arg Ile
            1540                1545                1550
Glu Gly Asn Thr Lys Val Asn Thr Leu Pro Asn Glu Val His Glu Leu
        1555                1560                1565
Ser Leu Arg Leu Val Lys Val Gly Asp Ala Ser Asp Ser Thr Gly Asp
    1570                1575                1580
His Lys Val Met Ser Lys Asn Asn Ser Gln Ala Leu Thr Ala Ser Ala
1585                1590                1595                1600
Thr Pro Thr Lys Thr Thr Thr Ser Ala Thr Ala Lys Ala Leu Pro Ser
                1605                1610                1615
Ala Gly Glu Lys Met Gly Leu Lys Leu Arg Ile Val Gly Leu Val Leu
            1620                1625                1630
Leu Gly Leu Thr Cys Val Phe Ser Arg Lys Lys Ser Thr Lys Asp
        1635                1640                1645
```

<210> 50
<211> 873
<212> PRT
<213> Streptococcus pyogenes

<400> 50

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
```

```
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
```

```
                660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 51
<211> 873
<212> PRT
<213> Streptococcus pyogenes

<400> 51

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
```

```
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Ser Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
```

```
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 52
<211> 873
<212> PRT
<213> Streptococcus pyogenes

<400> 52

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
```

```
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Ser Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
```

```
                740                     745                     750
Leu Thr Gly Val Lys Leu Leu Ser Tyr Ser Lys Val Glu Thr Thr Pro
            755                     760                     765
Leu Val Gln Glu Met Val Lys Glu Thr Lys Gln Leu Leu Glu Ala Ser
    770                     775                     780
Ala Arg Leu Ala Ala Glu Asn Thr Ser Leu Val Ala Glu Ala Leu Val
785                     790                     795                     800
Gly Gln Thr Ser Glu Met Val Ala Ser Asn Ala Ile Val Ser Lys Ile
                    805                     810                     815
Thr Ser Ser Ile Thr Gln Pro Ser Ser Lys Thr Ser Tyr Gly Ser Gly
                820                     825                     830
Ser Ser Thr Thr Ser Asn Leu Ile Ser Asp Val Asp Glu Ser Thr Gln
            835                     840                     845
Arg Ala Leu Lys Ala Gly Val Val Met Leu Ala Ala Val Gly Leu Thr
    850                     855                     860
Gly Phe Arg Phe Arg Lys Glu Ser Lys
865                     870
```

<210> 53
<211> 873
<212> PRT
<213> Streptococcus pyogenes

<400> 53

Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys

```
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Ser Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
```

```
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 54
<211> 873
<212> PRT
<213> Streptococcus pyogenes

<400> 54

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Ile His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
```

```
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Ser Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
```

```
                820                     825                     830
Ser Ser Thr Thr Ser Asn Leu Ile Ser Asp Val Asp Glu Ser Thr Gln
            835                     840                     845
Arg Ala Leu Lys Ala Gly Val Val Met Leu Ala Ala Val Gly Leu Thr
        850                     855                     860
Gly Phe Arg Phe Arg Lys Glu Ser Arg
865                 870
```

<210> 55
<211> 873
<212> PRT
<213> Streptococcus pyogenes

<400> 55

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
```

```
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Ser Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
```

Gly Phe Arg Phe Arg Lys Glu Ser Arg
865 870

<210> 56
<211> 873
<212> PRT
<213> Streptococcus pyogenes

<400> 56

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
```

```
Glu Asp Ser Ala Gly Ala Ser Gly Leu Ile Arg Asn Asp Asp Asn Met
        420                 425                 430
Tyr Glu Asn Ile Gly Ala Phe Asn Asp Val His Thr Val Asn Gly Ile
    435                 440                 445
Lys Arg Gly Ile Tyr Asp Ser Ile Lys Tyr Met Leu Phe Thr Asp His
450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 57

<211> 873
<212> PRT
<213> Streptococcus pyogenes

<400> 57

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
```

```
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 58
<211> 873
<212> PRT
<213> Streptococcus pyogenes

<400> 58

Met Asp Leu Glu Gln Thr Lys Pro Asn Gln Val Lys Gln Lys Ile Ala

```
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
```

```
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 59
<211> 873
<212> PRT
<213> Streptococcus pyogenes

<400> 59

```
Met Asp Leu Glu Gln Thr Lys Pro Asn Gln Val Lys Gln Lys Ile Ala
 1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
```

Thr His Asp Asp Ser Leu Pro Lys Pro Glu Thr Ile Gln Glu Ala Lys
50 55 60
Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Val Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys His Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala

```
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 60
<211> 873
<212> PRT
<213> Streptococcus pyogenes

<400> 60

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
```

```
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
```

```
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 61
<211> 873
<212> PRT
<213> Streptococcus pyogenes

<400> 61

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
```

```
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
```

```
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 62
<211> 873
<212> PRT
<213> Streptococcus pyogenes

<400> 62

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
```

```
                    165                                170                       175
Val Glu Gln Val Lys Thr Ser Glu Gln Asn Ile Ala Lys Leu Asn Ala
                180                 185                 190
Met Ile Ser Asn Pro Asp Ala Ile Thr Lys Ala Ala Gln Thr Ala Asn
            195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
```

```
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 63
<211> 873
<212> PRT
<213> Streptococcus pyogenes

<400> 63

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
```

```
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
```

690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Ser
740 745 750
Leu Thr Gly Val Lys Pro Leu Ser Tyr Ser Lys Val Glu Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 64
<211> 873
<212> PRT
<213> Streptococcus pyogenes

<400> 64

Met Asp Leu Glu Gln Thr Lys Pro Asn Gln Val Lys Gln Lys Ile Ala
1 5 10 15
Leu Thr Ser Thr Ile Ala Leu Leu Ser Ala Ser Val Gly Val Ser His
20 25 30
Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50 55 60
Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Ala Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg

```
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Lys Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
```

```
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 65
<211> 873
<212> PRT
<213> Streptococcus pyogenes

<400> 65

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
```

Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys His Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Lys Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Ser
740 745 750
Leu Thr Gly Val Lys Pro Leu Ser Tyr Ser Lys Val Glu Thr Thr Pro
755 760 765
Leu Val Gln Glu Met Val Lys Glu Thr Lys Gln Leu Leu Glu Ala Ser

```
    770                     775                     780
Ala Arg Leu Ala Ala Glu Asn Thr Ser Leu Val Ala Glu Ala Leu Val
785                 790                     795                 800
Gly Gln Thr Ser Glu Met Val Ala Ser Asn Ala Ile Val Ser Lys Ile
                805                     810                     815
Thr Ser Ser Ile Thr Gln Pro Ser Ser Lys Thr Ser Tyr Gly Ser Gly
            820                     825                     830
Ser Ser Thr Thr Ser Asn Leu Ile Ser Asp Val Asp Glu Ser Thr Gln
        835                     840                     845
Arg Ala Leu Lys Ala Gly Val Val Met Leu Ala Ala Val Gly Leu Thr
    850                     855                     860
Gly Phe Arg Phe Arg Lys Glu Ser Arg
865                 870
```

<210> 66
<211> 873
<212> PRT
<213> Streptococcus pyogenes

<400> 66

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
```

```
                325                     330                     335
Asp Asn Leu Thr Pro Glu Val Gln Asn Glu Leu Ala Gln Phe Ala Ala
            340                     345                     350
His Met Ile Asn Ser Val Arg Arg Gln Leu Gly Leu Pro Pro Val Thr
        355                     360                     365
Val Thr Ala Gly Ser Gln Glu Phe Ala Arg Leu Leu Ser Thr Ser Tyr
    370                     375                     380
Lys Lys Thr His Gly Asn Thr Arg Pro Ser Phe Val Tyr Gly Gln Pro
385                     390                     395                     400
Gly Val Ser Gly His Tyr Gly Val Gly Pro His Asp Lys Thr Ile Ile
                405                     410                     415
Glu Asp Ser Ala Gly Ala Ser Gly Leu Ile Arg Asn Asp Asp Asn Met
            420                     425                     430
Tyr Glu Asn Ile Gly Ala Phe Asn Asp Val His Thr Val Asn Gly Ile
        435                     440                     445
Lys Arg Gly Ile Tyr Asp Ser Ile Lys Tyr Met Leu Phe Thr Asp His
    450                     455                     460
Leu His Gly Asn Thr Tyr Gly His Ala Ile Asn Phe Leu Arg Val Asp
465                     470                     475                     480
Lys His Asn Pro Asn Ala Pro Val Tyr Leu Gly Phe Ser Thr Ser Asn
                485                     490                     495
Val Gly Ser Leu Asn Glu His Phe Val Met Phe Pro Glu Ser Asn Ile
            500                     505                     510
Ala Asn His Gln Arg Phe Asn Lys Thr Pro Ile Lys Ala Val Gly Ser
        515                     520                     525
Thr Lys Asp Tyr Ala Gln Arg Val Gly Thr Val Ser Asp Thr Ile Ala
    530                     535                     540
Ala Ile Lys Gly Lys Val Ser Ser Leu Glu Asn Arg Leu Ser Ala Ile
545                     550                     555                     560
His Gln Glu Ala Asp Ile Met Ala Ala Gln Ala Lys Val Ser Gln Leu
                565                     570                     575
Gln Gly Lys Leu Ala Ser Thr Leu Lys Gln Ser Asp Ser Leu Asn Leu
            580                     585                     590
Gln Val Arg Gln Leu Asn Asp Thr Lys Gly Ser Leu Arg Thr Glu Leu
        595                     600                     605
Leu Ala Ala Lys Ala Lys Gln Ala Gln Leu Glu Ala Thr Arg Asp Gln
    610                     615                     620
Ser Leu Ala Lys Leu Ala Ser Leu Lys Ala Ala Leu His Gln Thr Glu
625                     630                     635                     640
Ala Leu Ala Glu Gln Ala Ala Ala Arg Val Thr Ala Leu Val Ala Lys
                645                     650                     655
Lys Ala His Leu Gln Tyr Leu Lys Asp Phe Lys Leu Asn Pro Asn Arg
            660                     665                     670
Leu Gln Val Ile Arg Glu Arg Ile Asp Asn Thr Lys Gln Asp Leu Ala
        675                     680                     685
Lys Thr Thr Ser Ser Leu Leu Asn Ala Gln Glu Ala Leu Ala Ala Leu
    690                     695                     700
Gln Ala Lys Gln Ser Ser Leu Glu Ala Thr Ile Ala Thr Thr Glu His
705                     710                     715                     720
Gln Leu Thr Leu Leu Lys Thr Leu Ala Asn Glu Lys Glu Tyr Arg His
                725                     730                     735
Leu Asp Glu Asp Ile Ala Thr Val Pro Asp Leu Gln Val Ala Pro Ser
            740                     745                     750
Leu Thr Gly Val Lys Pro Leu Ser Tyr Ser Lys Val Glu Thr Thr Pro
        755                     760                     765
Leu Val Gln Glu Met Val Lys Glu Thr Lys Gln Leu Leu Glu Ala Ser
    770                     775                     780
Ala Arg Leu Ala Ala Glu Asn Thr Ser Leu Val Ala Glu Ala Leu Val
785                     790                     795                     800
Gly Gln Thr Ser Glu Met Val Ala Ser Asn Ala Ile Val Ser Lys Ile
                805                     810                     815
```

Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Arg
865 870

<210> 67
<211> 873
<212> PRT
<213> Streptococcus pyogenes

<400> 67

Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365

Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys His Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Ser
740 745 750
Leu Thr Gly Val Lys Pro Leu Ser Tyr Ser Lys Val Glu Thr Thr Pro
755 760 765
Leu Val Gln Glu Met Val Lys Glu Thr Lys His Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr

850 855 860
Gly Phe Arg Phe Arg Lys Glu Ser Lys
865 870

<210> 68
<211> 873
<212> PRT
<213> Streptococcus pyogenes

<400> 68

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
Asn Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
```

405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Ser
740 745 750
Leu Thr Gly Val Lys Pro Leu Ser Tyr Ser Lys Val Glu Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Arg
865 870

<210> 69
<211> 923
<212> PRT
<213> Streptococcus pyogenes

<400> 69

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
Asn Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
```

```
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Gly Gln Thr Ser Glu Met Val Ala Ser Asn Ala Ile Val Ser
    850                 855                 860
Lys Ile Thr Ser Ser Ile Thr Gln Pro Ser Ser Lys Thr Ser Tyr Gly
865                 870                 875                 880
Ser Gly Ser Ser Thr Thr Ser Asn Leu Ile Ser Asp Val Asp Glu Ser
                885                 890                 895
Thr Gln Arg Ala Leu Lys Ala Gly Val Val Met Leu Ala Ala Val Gly
            900                 905                 910
Leu Thr Gly Phe Arg Phe Arg Lys Glu Ser Arg
        915                 920
```

<210> 70
<211> 873
<212> PRT
<213> Streptococcus pyogenes

<400> 70

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
Asn Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
```

```
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 71
<211> 873
<212> PRT
<213> Streptococcus pyogenes

<400> 71

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
Asn Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
```

Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Ser
740 745 750
Leu Thr Gly Val Lys Pro Leu Ser Tyr Ser Lys Val Glu Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 72
<211> 873
<212> PRT
<213> Streptococcus pyogenes

<400> 72

```
Met Asp Leu Glu Gln Thr Lys Pro Asn Gln Val Lys Gln Lys Ile Ala
 1               5                   10                  15
Leu Thr Ser Thr Ile Ala Leu Leu Ser Ala Ser Val Gly Val Ser His
            20                  25                  30
```

Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50 55 60
Ala Thr Ile Asp Ala Val Glu Lys Thr Leu Ser Gln Gln Lys Ala Glu
65 70 75 80
Leu Thr Lys Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Ala Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser

```
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Asn Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Asp Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 73
<211> 873
<212> PRT
<213> Streptococcus pyogenes

<400> 73

```
Met Asp Leu Glu Gln Thr Lys Pro Asn Gln Val Lys Gln Lys Ile Ala
 1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
```

```
65                  70                  75                  80
Leu Thr Lys Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
```

His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 74
<211> 873
<212> PRT
<213> Streptococcus pyogenes

<400> 74

```
Met Asp Leu Glu Gln Thr Lys Pro Asn Gln Val Lys Gln Lys Ile Ala
 1              5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Lys Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
```

```
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
```

```
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Gly Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 75
<211> 873
<212> PRT
<213> Streptococcus pyogenes

<400> 75

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Lys Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
```

```
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
```

```
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Gly Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 76
<211> 873
<212> PRT
<213> Streptococcus pyogenes

<400> 76

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Lys Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
```

```
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Ile Lys Gln Asp Leu Ala
```

```
            675                 680                 685
Lys Thr Thr Ser Ser Leu Leu Asn Ala Gln Glu Thr Leu Ala Ala Leu
        690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 77
<211> 873
<212> PRT
<213> Streptococcus pyogenes

<400> 77

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Thr Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Asn Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
```

```
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Thr Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
```

```
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 78
<211> 873
<212> PRT
<213> Streptococcus pyogenes

<400> 78

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
```

```
Thr Met Lys Val Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Val Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Gly Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
```

```
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 79
<211> 873
<212> PRT
<213> Streptococcus pyogenes

<400> 79

Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Val Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu

```
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Val Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Gly Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
```

```
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 80
<211> 873
<212> PRT
<213> Streptococcus pyogenes

<400> 80

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Val Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
```

```
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Val Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Gly Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
```

```
            835                 840                 845
    Arg Ala Leu Lys Ala Gly Val Val Met Leu Ala Ala Val Gly Leu Thr
        850                 855                 860
    Gly Phe Arg Phe Arg Lys Glu Ser Lys
    865                 870
```

<210> 81
<211> 873
<212> PRT
<213> Streptococcus pyogenes

<400> 81

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Val Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
```

```
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Val Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Gly Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 82
<211> 873
<212> PRT
<213> Streptococcus pyogenes

<400> 82

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Val Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
```

```
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Val Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Gly Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 83
<211> 873
<212> PRT

<213> Streptococcus pyogenes

<400> 83

```
Met Asp Leu Glu Gln Thr Lys Pro Asn Gln Val Lys Gln Lys Ile Ala
 1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
Asn Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
```

```
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 84
<211> 873
<212> PRT
<213> Streptococcus pyogenes

<400> 84

Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His

```
                20                  25                  30
Gln Val Lys Ala Asp Asp Arg Ala Ser Gly Glu Thr Lys Ala Ser Asn
            35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
```

```
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 85
<211> 873
<212> PRT
<213> Streptococcus pyogenes

<400> 85

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
```

Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Val Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile

```
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Ile Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 86
<211> 873
<212> PRT
<213> Streptococcus pyogenes

<400> 86

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
```

```
                100                 105                 110
Gln Glu Ile Tyr Thr Asn Thr Leu Ala Ser Ser Glu Glu Thr Leu Leu
            115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
```

```
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 87
<211> 873
<212> PRT
<213> Streptococcus pyogenes

<400> 87

Met Asp Leu Glu Gln Thr Lys Pro Asn Gln Val Lys Gln Lys Ile Ala
1 5 10 15
Leu Thr Ser Thr Ile Ala Leu Leu Ser Ala Ser Val Gly Val Ser His
20 25 30
Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50. 55 60
Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140

Leu His Asn Ala Gln Val Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Glu Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu

625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 88
<211> 873
<212> PRT
<213> Streptococcus pyogenes

<400> 88

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
```

```
            180                     185                     190
Met Ile Ser Asn Pro Asp Ala Ile Thr Lys Ala Ala Gln Thr Ala Asn
        195                     200                     205
Asp Asn Thr Lys Ala Leu Ser Ser Glu Leu Glu Lys Ala Lys Ala Asp
    210                     215                     220
Leu Glu Asn Gln Lys Ala Lys Val Lys Lys Gln Leu Thr Glu Glu Leu
225                     230                     235                     240
Ala Ala Gln Lys Ala Ala Leu Ala Glu Lys Glu Ala Glu Leu Ser Arg
                245                     250                     255
Leu Lys Ser Ser Ala Pro Ser Thr Gln Asp Ser Ile Val Gly Asn Asn
            260                     265                     270
Thr Met Lys Ala Pro Gln Gly Tyr Pro Leu Glu Glu Leu Lys Lys Leu
        275                     280                     285
Glu Ala Ser Gly Tyr Ile Gly Ser Ala Ser Tyr Asn Asn Tyr Tyr Lys
    290                     295                     300
Glu His Ala Asp Gln Ile Ile Ala Lys Ala Ser Pro Gly Asn Gln Leu
305                     310                     315                     320
Asn Gln Tyr Gln Asp Ile Pro Ala Asp Arg Asn Arg Phe Val Asp Pro
                325                     330                     335
Asp Asn Leu Thr Pro Glu Val Gln Asn Glu Leu Ala Gln Phe Ala Ala
            340                     345                     350
His Met Ile Asn Ser Val Arg Arg Gln Leu Gly Leu Pro Pro Val Thr
        355                     360                     365
Val Thr Ala Gly Ser Gln Glu Phe Ala Arg Leu Leu Ser Thr Ser Tyr
    370                     375                     380
Lys Lys Thr His Gly Asn Thr Arg Pro Ser Phe Val Tyr Gly Gln Pro
385                     390                     395                     400
Gly Val Ser Gly His Tyr Gly Val Gly Pro His Asp Lys Thr Ile Ile
                405                     410                     415
Glu Asp Ser Ala Gly Ala Ser Gly Leu Ile Arg Asn Asp Asp Asn Met
            420                     425                     430
Tyr Glu Asn Ile Gly Ala Phe Asn Asp Val His Thr Val Asn Gly Ile
        435                     440                     445
Lys Arg Gly Ile Tyr Asp Ser Ile Lys Tyr Met Leu Phe Thr Asp His
    450                     455                     460
Leu His Gly Asn Thr Tyr Gly His Ala Ile Asn Phe Leu Arg Val Asp
465                     470                     475                     480
Lys Arg Asn Pro Asn Ala Pro Val Tyr Leu Gly Phe Ser Thr Ser Asn
                485                     490                     495
Val Gly Ser Leu Asn Glu His Phe Val Met Phe Pro Glu Ser Asn Ile
            500                     505                     510
Ala Asn His Gln Arg Phe Asn Lys Thr Pro Ile Lys Ala Val Gly Ser
        515                     520                     525
Thr Lys Asp Tyr Ala Gln Arg Val Gly Thr Val Ser Asp Thr Ile Ala
    530                     535                     540
Ala Ile Lys Gly Lys Val Ser Ser Leu Glu Asn Arg Leu Ser Ala Ile
545                     550                     555                     560
His Gln Glu Ala Asp Ile Met Ala Ala Gln Ala Lys Val Ser Gln Leu
                565                     570                     575
Glu Gly Lys Leu Ala Ser Thr Leu Lys Gln Ser Asp Ser Leu Asn Leu
            580                     585                     590
Gln Val Arg Gln Leu Asn Asp Thr Lys Gly Ser Leu Arg Thr Glu Leu
        595                     600                     605
Leu Ala Ala Lys Ala Lys Gln Ala Gln Leu Glu Ala Thr Arg Asp Gln
    610                     615                     620
Ser Leu Ala Lys Leu Ala Ser Leu Lys Ala Ala Leu His Gln Thr Glu
625                     630                     635                     640
Ala Leu Ala Glu Gln Ala Ala Ala Arg Val Thr Ala Leu Val Ala Lys
                645                     650                     655
Lys Ala His Leu Gln Tyr Leu Arg Asp Phe Lys Leu Asn Pro Asn Arg
            660                     665                     670
```

Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 89
<211> 873
<212> PRT
<213> Streptococcus pyogenes

<400> 89

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
```

```
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Val Leu
    690                 695                 700
Gln Ala Lys Gln Ser Ser Leu Glu Ala Thr Ile Ala Thr Thr Glu His
```

```
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 90
<211> 873
<212> PRT
<213> Streptococcus pyogenes

<400> 90

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
```

```
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Glu Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
```

```
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 91
<211> 873
<212> PRT
<213> Streptococcus pyogenes

<400> 91

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Thr Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Asn Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
```

```
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Thr Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
```

```
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 92
<211> 873
<212> PRT
<213> Streptococcus pyogenes

<400> 92

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Thr Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Asn Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
```

```
                340                 345                 350
His Met Ile Asn Ser Val Arg Arg Gln Leu Gly Leu Pro Pro Val Thr
            355                 360                 365
Val Thr Ala Gly Ser Gln Glu Phe Ala Arg Leu Leu Ser Thr Ser Tyr
        370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Thr Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
```

Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 93
<211> 873
<212> PRT
<213> Streptococcus pyogenes

<400> 93

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Thr Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Asn Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
```

```
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Thr Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
```

865 870

<210> 94
<211> 873
<212> PRT
<213> Streptococcus pyogenes

<400> 94

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
```

```
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Thr Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Ile Gly Leu Thr
    850                 855                 860
Gly Phe Arg Phe Arg Lys Glu Ser Lys
865                 870
```

<210> 95
<211> 873

<212> PRT
<213> Streptococcus pyogenes

<400> 95

Met Asp Leu Glu Gln Thr Lys Pro Asn Gln Val Lys Gln Lys Ile Ala
1 5 10 15
Leu Thr Ser Thr Ile Ala Leu Leu Ser Ala Ser Val Gly Val Ser His
20 25 30
Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50 55 60
Ala Thr Ile Glu Ala Val Glu Lys Thr Leu Ser Gln Gln Lys Ala Lys
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Ala Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460

Leu His Gly Asn Thr Tyr Gly His Ala Ile Asn Phe Leu Arg Val Asp
465 470 475 480
Lys His Asn Pro Lys Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Thr Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Thr Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Ile Gly Leu Thr
850 855 860
Gly Phe Arg Phe Arg Lys Glu Ser Lys
865 870

<210> 96
<211> 873
<212> PRT
<213> Streptococcus pyogenes

<400> 96

Met Asp Leu Glu Gln Thr Lys Pro Asn Gln Val Lys Gln Lys Ile Ala
1 5 10 15

Leu Thr Ser Thr Ile Ala Leu Leu Ser Ala Ser Val Gly Val Ser His
20 25 30
Gln Val Lys Ala Asp Asp Arg Ala Ser Glu Glu Thr Lys Ala Ser Asn
35 40 45
Thr His Asp Asp Ser Leu Pro Lys Pro Glu Thr Ile Gln Glu Ala Lys
50 55 60
Ala Thr Ile Glu Ala Val Glu Lys Thr Leu Ser Gln Gln Lys Thr Lys
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Ala Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile

```
                500                 505                 510
Ala Asn His Gln Arg Phe Asn Lys Thr Pro Ile Lys Ala Val Gly Ser
            515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 97
<211> 853
<212> PRT
<213> Streptococcus pyogenes

<400> 97

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Glu Glu Thr Lys Ala Ser Asn
        35                  40                  45
Thr His Asp Asp Ser Leu Pro Lys Pro Glu Thr Ile Gln Glu Ala Lys
```

```
    50                  55                  60
Ala Thr Ile Glu Ala Val Glu Lys Thr Leu Ser Gln Gln Lys Thr Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys Arg Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
```

Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala
850

<210> 98
<211> 873
<212> PRT
<213> Streptococcus pyogenes

<400> 98

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Glu Ala Val Glu Lys Thr Leu Ser Gln Gln Lys Ala Lys
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
```

Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Val Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys Arg Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu

```
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 99
<211> 873
<212> PRT
<213> Streptococcus pyogenes

<400> 99

```
Met Asp Leu Glu Gln Pro Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
His Val Lys Ala Asp Asp Leu Ala Pro Glu Gly Ala Lys Ala Ser Asn
        35                  40                  45
Thr Ser Glu Glu Ser Leu Pro Lys Thr Glu Thr Cys Glu Glu Ala Lys
    50                  55                  60
Ala Ala Val Glu Ala Val Glu Thr Asn Leu Asn Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu Tyr Gln Arg Glu Leu Thr Ala Thr Glu Thr Glu
    130                 135                 140
Leu His Asn Ala Gln Val Asp Gln His Ser Lys Glu Thr Ala Leu Ser
```

```
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Thr Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Thr Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Ala Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
```

```
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Thr Leu Ala Ala Leu
    690                 695                 700
Gln Ala Lys Lys Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Ile Asp Glu Ser Thr Gln
        835                 840                 845
Arg Ala Leu Lys Ala Gly Val Val Met Leu Ala Ala Val Gly Leu Thr
    850                 855                 860
Gly Val Lys Leu Arg Lys Asp Thr Lys
865                 870
```

<210> 100
<211> 873
<212> PRT
<213> Streptococcus pyogenes

<400> 100

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
```

```
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
```

```
        675                     680                     685
Lys Thr Thr Ser Ser Leu Leu Asn Ala Gln Glu Ala Leu Ala Ala Leu
    690                     695                     700
Gln Ala Lys Gln Ser Ser Leu Glu Ala Thr Ile Ala Thr Thr Glu His
705                     710                     715                     720
Gln Leu Thr Leu Leu Lys Thr Leu Ala Asn Glu Lys Glu Tyr Arg His
                725                     730                     735
Leu Asp Glu Asp Ile Ala Thr Val Pro Asp Leu Gln Val Ala Pro Pro
            740                     745                     750
Leu Thr Gly Val Lys Pro Leu Ser Tyr Ser Lys Ile Asp Thr Thr Pro
        755                     760                     765
Leu Val Gln Glu Met Val Lys Glu Thr Lys Gln Leu Leu Glu Ala Ser
    770                     775                     780
Ala Arg Leu Ala Ala Glu Asn Thr Ser Leu Val Ala Glu Ala Leu Val
785                     790                     795                     800
Gly Gln Thr Ser Glu Met Val Ala Ser Asn Ala Ile Val Ser Lys Ile
                805                     810                     815
Thr Ser Ser Ile Thr Gln Pro Ser Ser Lys Thr Ser Tyr Gly Ser Gly
            820                     825                     830
Ser Ser Thr Thr Ser Asn Leu Ile Ser Asp Val Asp Glu Ser Thr Gln
        835                     840                     845
Arg Ala Leu Lys Ala Gly Val Val Met Leu Ala Ala Val Gly Leu Thr
    850                     855                     860
Gly Phe Arg Phe Arg Lys Glu Ser Lys
865                     870
```

<210> 101
<211> 873
<212> PRT
<213> Streptococcus pyogenes

<400> 101

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
```

```
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
```

```
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 102
<211> 873
<212> PRT
<213> Streptococcus pyogenes

<400> 102

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
```

```
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
```

```
            755                 760                 765
Leu Val Gln Glu Met Val Lys Glu Thr Lys Gln Leu Leu Glu Ala Ser
        770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 103
<211> 873
<212> PRT
<213> Streptococcus pyogenes

<400> 103

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
```

```
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
```

```
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 104
<211> 873
<212> PRT
<213> Streptococcus pyogenes

<400> 104

```
Met Asp Leu Glu Gln Thr Lys Pro Asn Gln Val Lys Gln Lys Ile Ala
1               5                   10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
```

His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro
385 390 395 400
Gly Val Ser Gly His Tyr Gly Val Gly Pro His Asp Lys Thr Ile Ile
405 410 415
Glu Asp Ser Ala Gly Ala Ser Gly Leu Ile Arg Asn Asp Asp Asn Met
420 425 430
Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys His Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln

```
            835                 840                 845
Arg Ala Leu Lys Ala Gly Val Val Met Leu Ala Ala Val Gly Leu Thr
        850                 855                 860
Gly Phe Arg Phe Arg Lys Glu Ser Lys
865                 870
```

<210> 105
<211> 873
<212> PRT
<213> Streptococcus pyogenes

<400> 105

Met Asp Leu Glu Gln Thr Lys Pro Asn Gln Val Lys Gln Lys Ile Ala
1 5 10 15
Leu Thr Ser Thr Ile Ala Leu Leu Ser Ala Ser Val Gly Val Ser His
20 25 30
Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50 55 60
Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110
Gln Glu Ile Tyr Thr Asn Thr Leu Ala Ser Ser Glu Glu Thr Leu Leu
115 120 125
Ala Gln Gly Ala Glu His Gln Arg Glu Leu Thr Ala Thr Glu Thr Glu
130 135 140
Leu His Asn Ala Gln Ala Asp Gln His Ser Lys Glu Thr Ala Leu Ser
145 150 155 160
Glu Gln Lys Ala Ser Ile Ser Ala Glu Thr Thr Arg Ala Gln Asp Leu
165 170 175
Val Glu Gln Val Lys Thr Ser Glu Gln Asn Ile Ala Lys Leu Asn Ala
180 185 190
Met Ile Ser Asn Pro Asp Ala Ile Thr Lys Ala Ala Gln Thr Ala Asn
195 200 205
Asp Asn Thr Lys Ala Leu Ser Ser Glu Leu Glu Lys Ala Lys Ala Asp
210 215 220
Leu Glu Asn Gln Lys Ala Lys Val Lys Lys Gln Leu Thr Glu Glu Leu
225 230 235 240
Ala Ala Gln Lys Ala Ala Leu Ala Glu Lys Glu Ala Glu Leu Ser Arg
245 250 255
Leu Lys Ser Ser Ala Pro Ser Thr Gln Asp Ser Ile Val Gly Asn Asn
260 265 270
Thr Met Lys Ala Pro Gln Gly Tyr Pro Leu Glu Glu Leu Lys Lys Leu
275 280 285
Glu Ala Ser Gly Tyr Ile Gly Ser Ala Ser Tyr Asn Asn Tyr Tyr Lys
290 295 300
Glu His Ala Asp Gln Ile Ile Ala Lys Ala Ser Pro Gly Asn Gln Leu
305 310 315 320
Asn Gln Tyr Gln Asp Ile Pro Ala Asp Arg Asn Arg Phe Val Asp Pro
325 330 335
Asp Asn Leu Thr Pro Glu Val Gln Asn Glu Leu Ala Gln Phe Ala Ala
340 345 350
His Met Ile Asn Ser Val Arg Arg Gln Leu Gly Leu Pro Pro Val Thr
355 360 365
Val Thr Ala Gly Ser Gln Glu Phe Ala Arg Leu Leu Ser Thr Ser Tyr
370 375 380
Lys Lys Thr His Gly Asn Thr Arg Pro Ser Phe Val Tyr Gly Gln Pro

```
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Pro
            740                 745                 750
Leu Thr Gly Val Lys Pro Leu Ser Tyr Ser Lys Ile Asp Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Lys
865                 870
```

<210> 106
<211> 873
<212> PRT
<213> Streptococcus pyogenes

<400> 106

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
```

Tyr Glu Asn Ile Gly Ala Phe Asn Asp Val His Thr Val Asn Gly Ile
435 440 445
Lys Arg Gly Ile Tyr Asp Ser Ile Lys Tyr Met Leu Phe Thr Asp His
450 455 460
Leu His Gly Asn Thr Tyr Gly His Ala Ile Asn Phe Leu Arg Val Asp
465 470 475 480
Lys His Asn Pro Asn Ala Pro Val Tyr Leu Gly Phe Ser Thr Ser Asn
485 490 495
Val Gly Ser Leu Asn Glu His Phe Val Met Phe Pro Glu Ser Asn Ile
500 505 510
Ala Asn His Gln Arg Phe Asn Lys Thr Pro Ile Lys Ala Val Gly Ser
515 520 525
Thr Lys Asp Tyr Ala Gln Arg Val Gly Thr Val Ser Asp Thr Ile Ala
530 535 540
Ala Ile Lys Gly Lys Val Ser Ser Leu Glu Asn Arg Leu Ser Ala Ile
545 550 555 560
His Gln Glu Ala Asp Ile Met Ala Ala Gln Ala Lys Val Ser Gln Leu
565 570 575
Gln Gly Lys Leu Ala Ser Thr Leu Lys Gln Ser Asp Ser Leu Asn Leu
580 585 590
Gln Val Arg Gln Leu Asn Asp Thr Lys Gly Ser Leu Arg Thr Glu Leu
595 600 605
Leu Ala Ala Lys Ala Lys Gln Ala Gln Leu Glu Ala Thr Arg Asp Gln
610 615 620
Ser Leu Ala Lys Leu Ala Ser Leu Lys Ala Ala Leu His Gln Thr Glu
625 630 635 640
Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Lys
865 870

<210> 107
<211> 571
<212> PRT

<213> Streptococcus pyogenes

<400> 107

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
```

```
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 108
<211> 571
<212> PRT
<213> Streptococcus pyogenes

<400> 108

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
```

```
                    325                 330                 335
Leu Phe Val Ser Asn Val Ala Tyr Gly Arg Thr Val Phe Val Lys Leu
                340                 345                 350
Glu Thr Ser Ser Lys Ser Asn Asp Val Glu Ala Ala Phe Ser Ala Ala
            355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Ser Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 109
<211> 571
<212> PRT
<213> Streptococcus pyogenes

<400> 109

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
```

```
                180                     185                     190
Val Val Thr Lys Arg Asn Pro Gln Lys Ile His Ile Asp Leu Pro Gly
            195                     200                     205
Met Gly Asp Lys Ala Thr Val Glu Val Asn Asp Pro Thr Tyr Ala Asn
    210                     215                     220
Val Ser Thr Ala Ile Asp Asn Leu Val Asn Gln Trp His Asp Asn Tyr
225                     230                     235                     240
Ser Gly Gly Asn Thr Leu Pro Ala Arg Thr Gln Tyr Thr Glu Ser Met
                    245                     250                     255
Val Tyr Ser Lys Ser Gln Ile Glu Ala Ala Leu Asn Val Asn Ser Lys
                260                     265                     270
Ile Leu Asp Gly Thr Leu Gly Ile Asp Phe Lys Ser Ile Ser Lys Gly
            275                     280                     285
Glu Lys Lys Val Met Ile Ala Ala Tyr Lys Gln Ile Phe Tyr Thr Val
    290                     295                     300
Ser Ala Asn Leu Pro Asn Asn Pro Ala Asp Val Phe Asp Lys Ser Val
305                     310                     315                     320
Thr Phe Lys Glu Leu Gln Arg Lys Gly Val Ser Asn Glu Ala Pro Pro
                    325                     330                     335
Leu Phe Val Ser Asn Val Ala Tyr Gly Arg Thr Val Phe Val Lys Leu
                340                     345                     350
Glu Thr Ser Ser Lys Ser Asn Asp Val Glu Ala Ala Phe Ser Ala Ala
            355                     360                     365
Leu Lys Gly Thr Asp Val Lys Thr Asn Gly Lys Tyr Ser Asp Ile Leu
    370                     375                     380
Glu Asn Ser Ser Phe Thr Ala Val Val Leu Gly Gly Asp Ala Ala Glu
385                     390                     395                     400
His Asn Lys Val Val Thr Lys Asp Phe Asp Val Ile Arg Asn Val Ile
                    405                     410                     415
Lys Asp Asn Ala Thr Phe Ser Arg Lys Asn Pro Ala Tyr Pro Ile Ser
                420                     425                     430
Tyr Thr Ser Val Phe Leu Lys Asn Asn Lys Ile Ala Gly Val Asn Asn
            435                     440                     445
Arg Ser Glu Tyr Val Glu Thr Thr Ser Thr Glu Tyr Thr Ser Gly Lys
    450                     455                     460
Ile Asn Leu Ser His Gln Gly Ala Tyr Val Ala Gln Tyr Glu Ile Leu
465                     470                     475                     480
Trp Asp Glu Ile Asn Tyr Asp Asp Lys Gly Lys Glu Val Ile Thr Lys
                    485                     490                     495
Arg Arg Trp Asp Asn Asn Trp Tyr Ser Lys Thr Ser Pro Phe Ser Thr
                500                     505                     510
Val Ile Pro Leu Gly Ala Asn Ser Arg Asn Ile Arg Ile Met Ala Arg
            515                     520                     525
Glu Cys Thr Gly Leu Ala Trp Glu Trp Trp Arg Lys Val Ile Asp Glu
    530                     535                     540
Arg Asp Val Lys Leu Ser Lys Glu Ile Asn Val Asn Ile Ser Gly Ser
545                     550                     555                     560
Thr Leu Ser Pro Tyr Gly Ser Ile Thr Tyr Lys
                    565                     570
```

<210> 110
<211> 571
<212> PRT
<213> Streptococcus pyogenes

<400> 110

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
```

35 40 45
Gln Pro Lys Pro Glu Ser Ser Glu Leu Thr Thr Glu Lys Ala Gly Gln
50 55 60
Lys Met Asp Asp Met Leu Asn Ser Asn Asp Met Ile Lys Leu Ala Pro
65 70 75 80
Lys Glu Met Pro Leu Glu Ser Ala Glu Lys Glu Glu Lys Lys Ser Glu
85 90 95
Asp Lys Lys Lys Ser Glu Glu Asp His Thr Glu Glu Ile Asn Asp Lys
100 105 110
Ile Tyr Ser Leu Asn Tyr Asn Glu Leu Glu Val Leu Ala Lys Asn Gly
115 120 125
Glu Thr Ile Glu Asn Phe Val Pro Lys Glu Gly Val Lys Lys Ala Asp
130 135 140
Lys Phe Ile Val Ile Glu Arg Lys Lys Lys Asn Ile Asn Thr Thr Pro
145 150 155 160
Val Asp Ile Ser Ile Ile Asp Ser Val Thr Asp Arg Thr Tyr Pro Ala
165 170 175
Ala Leu Gln Leu Ala Asn Lys Gly Phe Thr Glu Asn Lys Pro Asp Ala
180 185 190
Val Val Thr Lys Arg Asn Pro Gln Lys Ile His Ile Asp Leu Pro Gly
195 200 205
Met Gly Asp Lys Ala Thr Val Glu Val Asn Asp Pro Thr Tyr Ala Asn
210 215 220
Val Ser Thr Ala Ile Asp Asn Leu Val Asn Gln Trp His Asp Asn Tyr
225 230 235 240
Ser Gly Gly Asn Thr Leu Pro Ala Arg Thr Gln Tyr Thr Glu Ser Met
245 250 255
Val Tyr Ser Lys Ser Gln Ile Glu Ala Ala Leu Asn Val Asn Ser Lys
260 265 270
Ile Leu Asp Gly Thr Leu Gly Ile Asp Phe Lys Ser Ile Ser Lys Gly
275 280 285
Glu Lys Lys Val Met Ile Ala Ala Tyr Lys Gln Ile Phe Tyr Thr Val
290 295 300
Ser Ala Asn Leu Pro Asn Asn Pro Ala Asp Val Phe Asp Lys Ser Val
305 310 315 320
Thr Phe Lys Glu Leu Gln Arg Lys Gly Val Ser Asn Glu Ala Pro Pro
325 330 335
Leu Phe Val Ser Asn Val Ala Tyr Gly Arg Thr Val Phe Val Lys Leu
340 345 350
Glu Thr Ser Ser Lys Ser Asn Asp Val Glu Ala Ala Phe Ser Ala Ala
355 360 365
Leu Lys Gly Thr Asp Val Lys Thr Asn Gly Lys Tyr Ser Asp Ile Leu
370 375 380
Glu Asn Ser Ser Phe Thr Ala Val Val Leu Gly Gly Asp Ala Ala Glu
385 390 395 400
His Asn Lys Val Val Thr Lys Asp Phe Asp Val Ile Arg Asn Val Ile
405 410 415
Lys Asp Asn Ala Thr Phe Ser Arg Lys Asn Pro Ala Tyr Pro Ile Ser
420 425 430
Tyr Thr Ser Val Phe Leu Lys Asn Asn Lys Ile Ala Gly Val Asn Asn
435 440 445
Arg Ser Glu Tyr Val Glu Thr Thr Ser Thr Glu Tyr Thr Ser Gly Lys
450 455 460
Ile Asn Leu Ser His Gln Gly Ala Tyr Val Ala Gln Tyr Glu Ile Leu
465 470 475 480
Trp Asp Glu Ile Asn Tyr Asp Asp Lys Gly Lys Glu Val Ile Thr Lys
485 490 495
Arg Arg Trp Asp Asn Asn Trp Tyr Ser Lys Thr Ser Pro Phe Ser Thr
500 505 510
Val Ile Pro Leu Gly Ala Asn Ser Arg Asn Ile Arg Ile Met Ala Arg
515 520 525

```
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 111
<211> 571
<212> PRT
<213> Streptococcus pyogenes

<400> 111

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
```

```
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Ser Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 112
<211> 521
<212> PRT
<213> Streptococcus pyogenes

<400> 112

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Thr Ser Thr Glu Thr Thr Thr Thr Asn Glu
        35                  40                  45
Gln Pro Lys Pro Glu Ser Ser Glu Leu Thr Ile Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Met Thr Tyr Pro Ala
                165                 170                 175
Ala Leu Gln Leu Ala Asp Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
```

```
Ser Gly Gly Asn Thr Leu Pro Ala Arg Thr Phe Tyr Thr Val Ser Ala
                245                 250                 255
Asn Leu Pro Asn Asn Pro Ala Asp Val Phe Asp Lys Ser Val Thr Phe
            260                 265                 270
Lys Glu Leu Gln Arg Lys Gly Val Ser Asn Glu Ala Pro Pro Leu Phe
        275                 280                 285
Val Ser Asn Val Ala Tyr Gly Arg Thr Val Phe Val Lys Leu Glu Thr
    290                 295                 300
Ser Ser Lys Ser Asn Asp Val Glu Ala Ala Phe Ser Ala Ala Leu Lys
305                 310                 315                 320
Gly Thr Asp Val Lys Thr Asn Gly Lys Tyr Ser Asp Ile Leu Glu Asn
                325                 330                 335
Ser Ser Phe Thr Ala Val Val Leu Gly Gly Asp Ala Ala Glu His Asn
            340                 345                 350
Lys Val Val Thr Lys Asp Phe Asp Val Ile Arg Asn Val Ile Lys Asp
        355                 360                 365
Asn Ala Thr Phe Ser Arg Lys Asn Pro Ala Tyr Pro Ile Ser Tyr Thr
    370                 375                 380
Ser Val Phe Leu Lys Asn Asn Lys Ile Ala Gly Val Asn Asn Arg Thr
385                 390                 395                 400
Glu Tyr Val Glu Thr Thr Ser Thr Glu Tyr Thr Ser Gly Lys Ile Asn
                405                 410                 415
Leu Ser His Arg Gly Ala Tyr Val Ala Gln Tyr Glu Ile Leu Trp Asp
            420                 425                 430
Glu Ile Asn Tyr Asp Asp Lys Gly Lys Glu Val Ile Thr Lys Arg Arg
        435                 440                 445
Trp Asp Asn Asn Trp Tyr Ser Lys Thr Ser Pro Phe Ser Thr Val Ile
    450                 455                 460
Pro Leu Gly Ala Asn Ser Arg Asn Ile Arg Ile Met Ala Arg Glu Cys
465                 470                 475                 480
Thr Gly Leu Ala Trp Glu Trp Trp Arg Lys Val Ile Asp Glu Arg Asp
                485                 490                 495
Val Lys Leu Ser Lys Glu Ile Asn Val Asn Ile Ser Gly Ser Thr Leu
            500                 505                 510
Ser Pro Tyr Gly Ser Ile Thr Tyr Lys
        515                 520
```

<210> 113
<211> 571
<212> PRT
<213> Streptococcus pyogenes

<400> 113

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Ile Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
```

Lys Phe Ile Val Ile Glu Arg Lys Lys Lys Asn Ile Asn Thr Thr Pro
145 150 155 160
Val Asp Ile Ser Ile Ile Asp Ser Val Thr Asp Met Thr Tyr Pro Ala
165 170 175
Ala Leu Gln Leu Ala Asp Lys Gly Phe Thr Glu Asn Lys Pro Asp Ala
180 185 190
Val Val Thr Lys Arg Asn Pro Gln Lys Ile His Ile Asp Leu Pro Gly
195 200 205
Met Gly Asp Lys Ala Thr Val Glu Val Asn Asp Pro Thr Tyr Ala Asn
210 215 220
Val Ser Thr Ala Ile Asp Asn Leu Val Asn Gln Trp His Asp Asn Tyr
225 230 235 240
Ser Gly Gly Asn Thr Leu Pro Ala Arg Thr Gln Tyr Thr Glu Ser Met
245 250 255
Val Tyr Ser Lys Ser Gln Ile Glu Ala Ala Leu Asn Val Asn Ser Lys
260 265 270
Ile Leu Asp Gly Thr Leu Gly Ile Asp Phe Lys Ser Ile Ser Lys Gly
275 280 285
Glu Lys Lys Val Met Ile Ala Ala Tyr Lys Gln Ile Phe Tyr Thr Val
290 295 300
Ser Ala Asn Leu Pro Asn Asn Pro Ala Asp Val Phe Asp Lys Ser Val
305 310 315 320
Thr Phe Lys Glu Leu Gln Arg Lys Gly Val Ser Asn Glu Ala Pro Pro
325 330 335
Leu Phe Val Ser Asn Val Ala Tyr Gly Arg Thr Val Phe Val Lys Leu
340 345 350
Glu Thr Ser Ser Lys Ser Asn Asp Val Glu Ala Ala Phe Ser Ala Ala
355 360 365
Leu Lys Gly Thr Asp Val Lys Thr Asn Gly Lys Tyr Ser Asp Ile Leu
370 375 380
Glu Asn Ser Ser Phe Thr Ala Val Val Leu Gly Gly Asp Ala Ala Glu
385 390 395 400
His Asn Lys Val Val Thr Lys Asp Phe Asp Val Ile Arg Asn Val Ile
405 410 415
Lys Asp Asn Ala Thr Phe Ser Arg Lys Asn Pro Ala Tyr Pro Ile Ser
420 425 430
Tyr Thr Ser Val Phe Leu Lys Asn Asn Lys Ile Ala Gly Val Asn Asn
435 440 445
Arg Thr Glu Tyr Val Glu Thr Thr Ser Thr Glu Tyr Thr Ser Gly Lys
450 455 460
Ile Asn Leu Ser His Arg Gly Ala Tyr Val Ala Gln Tyr Glu Ile Leu
465 470 475 480
Trp Asp Glu Ile Asn Tyr Asp Asp Lys Gly Lys Glu Val Ile Thr Lys
485 490 495
Arg Arg Trp Asp Asn Asn Trp Tyr Ser Lys Thr Ser Pro Phe Ser Thr
500 505 510
Val Ile Pro Leu Gly Ala Asn Ser Arg Asn Ile Arg Ile Met Ala Arg
515 520 525
Glu Cys Thr Gly Leu Ala Trp Glu Trp Trp Arg Lys Val Ile Asp Glu
530 535 540
Arg Asp Val Lys Leu Ser Lys Glu Ile Asn Val Asn Ile Ser Gly Ser
545 550 555 560
Thr Leu Ser Pro Tyr Gly Ser Ile Thr Tyr Lys
565 570

<210> 114
<211> 571
<212> PRT
<213> Streptococcus pyogenes

<400> 114

```
Met Ser Asn Lys Lys Thr Phe Lys Lys Tyr Ser Arg Val Ala Gly Leu
 1               5                   10                  15
Leu Thr Val Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Ile Glu Lys Ala Gly Gln
    50                  55                  60
Lys Met Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Asp Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
```

```
                        485                     490                     495
Arg Arg Trp Asp Asn Asn Trp Tyr Ser Lys Thr Ser Pro Phe Ser Thr
                    500                     505                     510
Val Ile Pro Leu Gly Ala Asn Ser Arg Asn Ile Arg Ile Met Ala Arg
            515                     520                     525
Glu Cys Thr Gly Leu Ala Trp Glu Trp Trp Arg Lys Val Ile Asp Glu
    530                     535                     540
Arg Asp Val Lys Leu Ser Lys Glu Ile Asn Val Asn Ile Ser Gly Ser
545                     550                     555                     560
Thr Leu Ser Pro Tyr Gly Ser Ile Thr Tyr Lys
                565                     570
```

<210> 115
<211> 571
<212> PRT
<213> Streptococcus pyogenes

<400> 115

Met Ser Asn Lys Lys Thr Phe Lys Lys Tyr Ser Arg Val Ala Gly Leu
1 5 10 15
Leu Thr Ala Ala Leu Ile Ile Gly Asn Leu Val Thr Ala Asn Ala Glu
20 25 30
Ser Asn Lys Gln Asn Thr Ala Ser Thr Glu Thr Thr Thr Thr Asn Glu
35 40 45
Gln Pro Lys Pro Glu Ser Ser Glu Leu Thr Thr Glu Lys Ala Gly Gln
50 55 60
Lys Thr Asp Asp Met Leu Asn Ser Asn Asp Met Ile Lys Leu Ala Pro
65 70 75 80
Lys Glu Met Pro Leu Glu Ser Ala Glu Lys Glu Glu Lys Lys Ser Glu
85 90 95
Asp Lys Lys Lys Ser Glu Glu Asp His Thr Glu Glu Ile Asn Asp Lys
100 105 110
Ile Tyr Ser Leu Asn Tyr Asn Glu Leu Glu Val Leu Ala Lys Asn Gly
115 120 125
Glu Thr Ile Glu Asn Phe Val Pro Lys Glu Gly Val Lys Lys Ala Asp
130 135 140
Lys Phe Ile Val Ile Glu Arg Lys Lys Lys Asn Ile Asn Thr Thr Pro
145 150 155 160
Val Asp Ile Ser Ile Ile Asp Ser Val Thr Asp Arg Thr Tyr Pro Ala
165 170 175
Ala Leu Gln Leu Ala Asn Lys Gly Phe Thr Glu Asn Lys Pro Asp Ala
180 185 190
Val Val Thr Lys Arg Asn Pro Gln Lys Ile His Ile Asp Leu Pro Gly
195 200 205
Met Gly Asp Lys Ala Thr Val Glu Val Asn Asp Pro Thr Tyr Ala Asn
210 215 220
Val Ser Thr Ala Ile Asp Asn Leu Val Asn Gln Trp His Asp Asn Tyr
225 230 235 240
Ser Gly Gly Asn Thr Leu Pro Ala Arg Thr Gln Tyr Thr Glu Ser Met
245 250 255
Val Tyr Ser Lys Ser Gln Ile Glu Ala Ala Leu Asn Val Asn Ser Lys
260 265 270
Ile Leu Asp Gly Thr Leu Gly Ile Asp Phe Lys Ser Ile Ser Lys Gly
275 280 285
Glu Lys Lys Val Met Ile Ala Ala Tyr Lys Gln Ile Phe Tyr Thr Val
290 295 300
Ser Ala Asn Leu Pro Asn Asn Pro Ala Asp Val Phe Asp Lys Ser Val
305 310 315 320
Thr Phe Lys Glu Leu Gln Arg Lys Gly Val Ser Asn Glu Ala Pro Pro
325 330 335
Leu Phe Val Ser Asn Val Ala Tyr Gly Arg Thr Val Phe Val Lys Leu

```
                340                     345                     350
Glu Thr Ser Ser Lys Ser Asn Asp Val Glu Ala Ala Phe Ser Ala Ala
            355                     360                     365
Leu Lys Gly Thr Asp Val Lys Thr Asn Gly Lys Tyr Ser Asp Ile Leu
    370                     375                     380
Glu Asn Ser Ser Phe Thr Ala Val Val Leu Gly Gly Asp Ala Ala Glu
385                     390                     395                     400
His Asn Lys Val Val Thr Lys Asp Phe Asp Val Ile Arg Asn Val Ile
                405                     410                     415
Lys Asp Asn Ala Thr Phe Ser Arg Lys Asn Pro Ala Tyr Pro Ile Ser
            420                     425                     430
Tyr Thr Ser Val Phe Leu Lys Asn Asn Lys Ile Ala Gly Val Asn Asn
        435                     440                     445
Arg Thr Glu Tyr Val Glu Thr Thr Ser Thr Glu Tyr Thr Ser Gly Lys
    450                     455                     460
Ile Asn Leu Ser His Arg Gly Ala Tyr Val Ala Gln Tyr Glu Ile Leu
465                     470                     475                     480
Trp Asp Glu Ile Asn Tyr Asp Asp Lys Gly Lys Glu Val Ile Thr Lys
                485                     490                     495
Arg Arg Trp Asp Asn Asn Trp Tyr Ser Lys Thr Ser Pro Phe Ser Thr
            500                     505                     510
Val Ile Pro Leu Gly Ala Asn Ser Arg Asn Ile Arg Ile Met Ala Arg
        515                     520                     525
Glu Cys Thr Gly Leu Ala Trp Glu Trp Trp Arg Lys Val Ile Asp Glu
    530                     535                     540
Arg Asp Val Lys Leu Ser Lys Glu Ile Asn Val Asn Ile Ser Gly Ser
545                     550                     555                     560
Thr Leu Ser Pro Tyr Gly Ser Ile Thr Tyr Lys
                565                     570
```

<210> 116
<211> 521
<212> PRT
<213> Streptococcus pyogenes

<400> 116

Met Ser Asn Lys Lys Thr Phe Lys Lys Tyr Ser Arg Val Ala Gly Leu
1 5 10 15
Leu Thr Ala Ala Leu Ile Ile Gly Asn Leu Val Thr Ala Asn Ala Glu
20 25 30
Ser Asn Lys Gln Asn Thr Ala Ser Thr Glu Thr Thr Thr Thr Asn Glu
35 40 45
Gln Pro Lys Pro Glu Ser Ser Glu Leu Thr Thr Glu Lys Ala Gly Gln
50 55 60
Lys Thr Asp Asp Met Leu Asn Ser Asn Asp Met Ile Lys Leu Ala Pro
65 70 75 80
Lys Glu Met Pro Leu Glu Ser Ala Glu Lys Glu Glu Lys Lys Ser Glu
85 90 95
Asp Lys Lys Lys Arg Lys Lys Lys Asn Ile Asn Thr Thr Pro Val Asp
100 105 110
Ile Ser Ile Ile Asp Ser Val Thr Asp Arg Thr Tyr Pro Ala Ala Leu
115 120 125
Gln Leu Ala Asn Lys Gly Phe Thr Glu Asn Lys Pro Asp Ala Val Val
130 135 140
Thr Lys Arg Asn Pro Gln Lys Ile His Ile Asp Leu Pro Gly Met Gly
145 150 155 160
Asp Lys Ala Thr Val Glu Val Asn Asp Pro Thr Tyr Ala Asn Val Ser
165 170 175
Thr Ala Ile Asp Asn Leu Val Asn Gln Trp His Asp Asn Tyr Ser Gly
180 185 190
Gly Asn Thr Leu Pro Ala Arg Thr Gln Tyr Thr Glu Ser Met Val Tyr

```
            195                 200                 205
Ser Lys Ser Gln Ile Glu Ala Ala Leu Asn Val Asn Ser Lys Ile Leu
        210                 215                 220
Asp Gly Thr Leu Gly Ile Asp Phe Lys Ser Ile Ser Lys Gly Glu Lys
225                 230                 235                 240
Lys Val Met Ile Ala Ala Tyr Lys Gln Ile Phe Tyr Thr Val Ser Ala
                245                 250                 255
Asn Leu Pro Asn Asn Pro Ala Asp Val Phe Asp Lys Ser Val Thr Phe
            260                 265                 270
Lys Asp Leu Gln Arg Lys Gly Val Ser Asn Glu Ala Pro Pro Leu Phe
        275                 280                 285
Val Ser Asn Val Ala Tyr Gly Arg Thr Val Phe Val Lys Leu Glu Thr
    290                 295                 300
Ser Ser Lys Ser Asn Asp Val Glu Ala Ala Phe Ser Ala Ala Leu Lys
305                 310                 315                 320
Gly Thr Asp Val Lys Thr Asn Gly Lys Tyr Ser Asp Ile Leu Glu Asn
                325                 330                 335
Ser Ser Phe Thr Ala Val Val Leu Gly Gly Asp Ala Ala Glu His Asn
            340                 345                 350
Lys Val Val Thr Lys Asp Phe Asp Val Ile Arg Asn Val Ile Lys Asp
        355                 360                 365
Asn Ala Thr Phe Ser Arg Lys Asn Pro Ala Tyr Pro Ile Ser Tyr Thr
    370                 375                 380
Ser Val Phe Leu Lys Asn Asn Lys Ile Ala Gly Val Asn Asn Arg Thr
385                 390                 395                 400
Glu Tyr Val Glu Thr Thr Ser Thr Glu Tyr Thr Ser Gly Lys Ile Asn
                405                 410                 415
Leu Ser His Gln Gly Ala Tyr Val Ala Gln Tyr Glu Ile Leu Trp Asp
            420                 425                 430
Glu Ile Asn Tyr Asp Asp Lys Gly Lys Glu Val Ile Thr Lys Arg Arg
        435                 440                 445
Trp Asp Asn Asn Trp Tyr Ser Lys Thr Ser Pro Phe Ser Thr Val Ile
    450                 455                 460
Pro Leu Gly Ala Asn Ser Arg Asn Ile Arg Ile Met Ala Arg Glu Cys
465                 470                 475                 480
Thr Gly Leu Ala Trp Glu Trp Trp Arg Lys Val Ile Asp Glu Arg Asp
                485                 490                 495
Val Lys Leu Ser Lys Glu Ile Asn Val Asn Ile Ser Gly Ser Thr Leu
            500                 505                 510
Ser Pro Tyr Gly Ser Ile Thr Tyr Lys
        515                 520
```

<210> 117
<211> 571
<212> PRT
<213> Streptococcus pyogenes

<400> 117

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
```

```
                100                 105                 110
Ile Tyr Ser Leu Asn Tyr Asn Glu Leu Glu Val Leu Ala Lys Asn Gly
            115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 118
<211> 571
<212> PRT
<213> Streptococcus pyogenes

<400> 118

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
```

```
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 119
<211> 571
<212> PRT
<213> Streptococcus pyogenes

<400> 119

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Ala Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Ile Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
```

```
Ser Ala Asn Leu Pro Asn Asn Pro Ala Asp Val Phe Ala Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 120
<211> 543
<212> PRT
<213> Streptococcus pyogenes

<400> 120

```
Met Ala Ser Glu Ser Asn Lys Gln Asn Thr Ala Ser Thr Glu Thr Thr
 1               5                  10                  15
Thr Thr Asn Glu Gln Pro Lys Pro Glu Ser Ser Glu Leu Thr Thr Glu
            20                  25                  30
Lys Ala Gly Gln Lys Thr Asp Asp Met Leu Asn Ser Asn Asp Met Ile
        35                  40                  45
Lys Leu Ala Pro Lys Glu Met Pro Leu Glu Ser Ala Glu Lys Glu Glu
    50                  55                  60
Lys Lys Ser Glu Asp Lys Lys Lys Ser Glu Glu Asp His Thr Glu Glu
65                  70                  75                  80
Ile Asn Asp Lys Ile Tyr Ser Leu Asn Tyr Asn Glu Leu Glu Val Leu
                85                  90                  95
Ala Lys Asn Gly Glu Thr Ile Glu Asn Phe Val Pro Lys Glu Gly Val
            100                 105                 110
Lys Lys Ala Asp Lys Phe Ile Val Ile Glu Arg Lys Lys Lys Asn Ile
        115                 120                 125
Asn Thr Thr Pro Val Asp Ile Ser Ile Ile Asp Ser Val Thr Asp Arg
    130                 135                 140
Thr Tyr Pro Ala Ala Leu Gln Leu Ala Asn Lys Gly Phe Thr Glu Asn
145                 150                 155                 160
```

```
Lys Pro Asp Ala Val Val Thr Lys Arg Asn Pro Gln Lys Ile His Ile
                165                 170                 175
Asp Leu Pro Gly Met Gly Asp Lys Ala Thr Val Glu Val Asn Asp Pro
            180                 185                 190
Thr Tyr Ala Asn Val Ser Thr Ala Ile Asp Asn Leu Val Asn Gln Trp
        195                 200                 205
His Asp Asn Tyr Ser Gly Gly Asn Thr Leu Pro Ala Arg Thr Gln Tyr
    210                 215                 220
Thr Glu Ser Met Val Tyr Ser Lys Ser Gln Ile Glu Ala Ala Leu Asn
225                 230                 235                 240
Val Asn Ser Lys Ile Leu Asp Gly Thr Leu Gly Ile Asp Phe Lys Ser
                245                 250                 255
Ile Ser Lys Gly Glu Lys Lys Val Met Ile Ala Ala Tyr Lys Gln Ile
            260                 265                 270
Phe Tyr Thr Val Ser Ala Asn Leu Pro Asn Asn Pro Ala Asp Val Phe
        275                 280                 285
Asp Lys Ser Val Thr Phe Lys Glu Leu Gln Arg Lys Gly Val Ser Asn
    290                 295                 300
Glu Ala Pro Pro Leu Phe Val Ser Asn Val Ala Tyr Gly Arg Thr Val
305                 310                 315                 320
Phe Val Lys Leu Glu Thr Ser Ser Lys Ser Asn Asp Val Glu Ala Ala
                325                 330                 335
Phe Ser Ala Ala Leu Lys Gly Thr Asp Val Lys Thr Asn Gly Lys Tyr
            340                 345                 350
Ser Asp Ile Leu Glu Asn Ser Ser Phe Thr Ala Val Val Leu Gly Gly
        355                 360                 365
Asp Ala Ala Glu His Asn Lys Val Val Thr Lys Asp Phe Asp Val Ile
    370                 375                 380
Arg Asn Val Ile Lys Asp Asn Ala Thr Phe Ser Arg Lys Asn Leu Ala
385                 390                 395                 400
Tyr Pro Ile Ser Tyr Thr Ser Val Phe Leu Lys Asn Asn Lys Ile Ala
                405                 410                 415
Gly Val Asn Asn Arg Thr Glu Tyr Val Glu Thr Thr Ser Thr Glu Tyr
            420                 425                 430
Thr Ser Gly Lys Ile Asn Leu Ser His Gln Gly Ala Tyr Val Ala Gln
        435                 440                 445
Tyr Glu Ile Leu Trp Asp Glu Ile Asn Tyr Asp Asp Lys Gly Lys Glu
    450                 455                 460
Val Ile Thr Lys Arg Arg Trp Asp Asn Asn Trp Tyr Ser Lys Thr Ser
465                 470                 475                 480
Pro Phe Ser Thr Val Ile Pro Leu Gly Ala Asn Ser Arg Asn Ile Arg
                485                 490                 495
Ile Met Ala Arg Glu Cys Thr Gly Leu Ala Trp Glu Trp Trp Arg Lys
            500                 505                 510
Val Ile Asp Glu Arg Asp Val Lys Leu Ser Lys Glu Ile Asn Val Asn
        515                 520                 525
Ile Ser Gly Ser Thr Leu Ser Pro Tyr Gly Ser Ile Thr Tyr Lys
    530                 535                 540
```

<210> 121
<211> 571
<212> PRT
<213> Streptococcus pyogenes

<400> 121

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
```

```
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Phe Glu Trp Trp Arg Lys Val Ile Asp Glu
```

```
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 122
<211> 571
<212> PRT
<213> Streptococcus pyogenes

<400> 122

```
Met Ser Asn Lys Lys Thr Phe Lys Lys Tyr Ser Arg Val Ala Gly Leu
1               5                   10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
```

```
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asp Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Gly Thr Gly Leu Ala Trp Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 123
<211> 570
<212> PRT
<213> Streptococcus pyogenes

<400> 123

```
Met Ser Asn Lys Lys Thr Phe Lys Lys Tyr Ser Arg Val Ala Gly Leu
1               5                   10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
            100                 105                 110
Ile Tyr Ser Leu Asn Tyr Asn Glu Leu Glu Val Leu Ala Lys Asn Gly
        115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Arg Thr Gln Tyr Thr Glu Ser Met Val
```

```
                        245                 250                 255
Tyr Ser Lys Ser Gln Ile Glu Ala Ala Leu Asn Val Asn Ser Lys Ile
                260                 265                 270
Leu Asp Gly Thr Leu Gly Ile Asp Phe Lys Ser Ile Ser Lys Gly Glu
            275                 280                 285
Lys Lys Val Met Ile Ala Ala Tyr Lys Gln Ile Phe Tyr Thr Val Ser
    290                 295                 300
Ala Asn Leu Pro Asn Asn Pro Ala Asp Val Phe Asp Lys Ser Val Thr
305                 310                 315                 320
Phe Lys Glu Leu Gln Arg Lys Gly Val Ser Asn Glu Ala Pro Pro Leu
                325                 330                 335
Phe Val Ser Asn Val Ala Tyr Gly Arg Thr Val Phe Val Lys Leu Glu
                340                 345                 350
Thr Ser Ser Lys Ser Asn Asp Val Glu Ala Ala Phe Ser Ala Ala Leu
            355                 360                 365
Lys Gly Thr Asp Val Lys Thr Asn Gly Lys Tyr Ser Asp Ile Leu Glu
    370                 375                 380
Asn Ser Ser Phe Thr Ala Val Val Leu Gly Gly Asp Ala Ala Glu His
385                 390                 395                 400
Asn Lys Val Val Thr Lys Asp Phe Asp Val Ile Arg Asn Val Ile Lys
                405                 410                 415
Asp Asn Ala Thr Phe Ser Arg Lys Asn Pro Ala Tyr Pro Ile Ser Tyr
            420                 425                 430
Thr Ser Val Phe Leu Lys Asn Asn Lys Ile Ala Gly Val Asn Asn Arg
            435                 440                 445
Thr Glu Tyr Val Glu Thr Thr Ser Thr Glu Tyr Thr Ser Gly Lys Ile
    450                 455                 460
Asn Leu Ser His Gln Gly Ala Tyr Val Ala Gln Tyr Glu Ile Leu Trp
465                 470                 475                 480
Asp Glu Ile Asn Tyr Asp Asp Lys Gly Lys Glu Val Ile Thr Lys Arg
                485                 490                 495
Arg Trp Asp Asn Asn Trp Tyr Ser Lys Thr Ser Pro Phe Ser Thr Val
            500                 505                 510
Ile Pro Leu Gly Ala Asn Ser Arg Asn Ile Arg Ile Met Ala Arg Glu
            515                 520                 525
Cys Thr Gly Leu Ala Trp Glu Trp Trp Arg Lys Val Ile Asp Glu Arg
    530                 535                 540
Asp Val Lys Leu Ser Lys Glu Ile Asn Val Asn Ile Ser Gly Ser Thr
545                 550                 555                 560
Leu Ser Pro Tyr Gly Ser Ile Thr Tyr Lys
                565                 570
```

<210> 124
<211> 571
<212> PRT
<213> Streptococcus pyogenes

<400> 124

```
Met Ser Asn Lys Lys Thr Phe Lys Lys Tyr Ser Arg Val Ala Gly Leu
 1               5                  10                  15
Leu Thr Ala Ala Leu Ile Ile Gly Asn Leu Val Thr Ala Asn Ala Glu
            20                  25                  30
Ser Asn Lys Gln Asn Thr Ala Ser Thr Glu Thr Thr Thr Thr Asn Glu
        35                  40                  45
Gln Pro Lys Pro Glu Ser Ser Glu Leu Thr Thr Glu Lys Ala Gly Gln
    50                  55                  60
Lys Thr Asp Asp Met Leu Asn Ser Asn Asp Met Ile Lys Leu Ala Pro
65                  70                  75                  80
Lys Glu Met Pro Leu Glu Ser Ala Glu Lys Glu Glu Lys Lys Ser Glu
                85                  90                  95
Asp Lys Lys Lys Ser Glu Glu Asp His Thr Glu Glu Ile Asn Asp Lys
```

```
                100                 105                 110
Ile Tyr Ser Leu Asn Tyr Asn Glu Leu Glu Val Leu Ala Lys Asn Gly
            115                 120                 125
Glu Thr Ile Glu Asn Phe Val Pro Lys Glu Gly Val Lys Lys Ala Asp
    130                 135                 140
Lys Phe Ile Val Ile Glu Arg Lys Lys Lys Asn Ile Asn Thr Thr Pro
145                 150                 155                 160
Val Asp Ile Ser Ile Ile Asp Ser Val Thr Asp Arg Thr Tyr Pro Ala
                165                 170                 175
Ala Leu Gln Leu Ala Asn Lys Gly Phe Thr Glu Asn Lys Pro Asp Ala
            180                 185                 190
Val Val Thr Lys Arg Asn Pro Gln Lys Ile His Ile Asp Leu Pro Gly
        195                 200                 205
Met Gly Asp Lys Ala Thr Val Glu Val Asn Asp Pro Thr Tyr Ala Asn
    210                 215                 220
Val Ser Thr Ala Ile Asp Asn Leu Val Asn Gln Trp His Asp Asn Tyr
225                 230                 235                 240
Ser Gly Gly Asn Thr Leu Pro Ala Arg Thr Gln Tyr Thr Glu Ser Met
                245                 250                 255
Val Tyr Ser Lys Ser Gln Ile Glu Ala Ala Leu Asn Val Asn Ser Lys
            260                 265                 270
Ile Leu Asp Gly Thr Leu Gly Ile Asp Phe Lys Ser Ile Ser Lys Gly
        275                 280                 285
Glu Lys Lys Val Met Ile Ala Ala Tyr Lys Gln Ile Phe Tyr Thr Val
    290                 295                 300
Ser Ala Asn Leu Pro Asn Asn Pro Ala Asp Val Phe Asp Lys Ser Val
305                 310                 315                 320
Thr Phe Lys Glu Leu Gln Arg Lys Gly Val Ser Asn Glu Ala Pro Pro
                325                 330                 335
Leu Phe Val Ser Asn Val Ala Tyr Gly Arg Thr Val Phe Val Lys Leu
            340                 345                 350
Glu Thr Ser Ser Lys Ser Asn Asp Val Glu Ala Ala Phe Ser Ala Ala
        355                 360                 365
Leu Lys Gly Thr Asp Val Lys Thr Asn Gly Lys Tyr Ser Asp Ile Leu
    370                 375                 380
Glu Asn Ser Ser Phe Thr Ala Val Val Leu Gly Gly Asp Ala Ala Glu
385                 390                 395                 400
His Asn Lys Val Val Thr Lys Asp Phe Asp Val Ile Arg Asn Val Ile
                405                 410                 415
Lys Asp Asn Ala Thr Phe Ser Arg Lys Asn Pro Ala Tyr Pro Ile Ser
            420                 425                 430
Tyr Thr Ser Val Phe Leu Lys Asn Asn Lys Ile Ala Gly Val Asn Asn
        435                 440                 445
Arg Thr Glu Tyr Val Glu Thr Thr Ser Thr Glu Tyr Thr Ser Gly Lys
    450                 455                 460
Ile Asn Leu Ser His Gln Gly Ala Tyr Val Ala Gln Tyr Glu Ile Leu
465                 470                 475                 480
Trp Asn Glu Ile Asn Tyr Asp Asp Lys Gly Lys Glu Val Ile Thr Lys
                485                 490                 495
Arg Arg Trp Asp Asn Asn Trp Tyr Ser Lys Thr Ser Pro Phe Ser Thr
            500                 505                 510
Val Ile Pro Leu Gly Ala Asn Ser Arg Asn Ile Arg Ile Met Ala Arg
        515                 520                 525
Glu Cys Thr Gly Leu Ala Phe Glu Trp Trp Arg Lys Val Ile Asp Glu
    530                 535                 540
Arg Asp Val Lys Leu Ser Lys Glu Ile Asn Val Asn Ile Ser Gly Ser
545                 550                 555                 560
Thr Leu Ser Pro Tyr Gly Ser Ile Thr Tyr Lys
                565                 570
```

<210> 125
<211> 543
<212> PRT
<213> Streptococcus pyogenes

<400> 125

```
Met Ala Ser Glu Ser Asn Lys Gln Asn Thr Ala Ser Thr Glu Thr Thr
1               5                   10                  15
Thr Thr Asn Glu Gln Pro Lys Pro Glu Ser Ser Glu Leu Thr Thr Glu
            20                  25                  30
Lys Ala Gly Gln Lys Thr Asp Asp Met Leu Asn Ser Asn Asp Met Ile
        35                  40                  45
Lys Leu Ala Pro Lys Glu Met Pro Leu Glu Ser Ala Glu Lys Glu Glu
    50                  55                  60
Lys Lys Ser Glu Asp Lys Lys Lys Ser Glu Glu Asp His Thr Glu Glu
65                  70                  75                  80
Ile Asn Asp Lys Ile Tyr Ser Leu Asn Tyr Asn Glu Leu Glu Val Leu
                85                  90                  95
Ala Lys Asn Gly Glu Thr Ile Glu Asn Phe Val Pro Lys Glu Gly Val
            100                 105                 110
Lys Lys Ala Asp Lys Phe Ile Val Ile Glu Arg Lys Lys Lys Asn Ile
        115                 120                 125
Asn Thr Thr Pro Val Asp Ile Ser Ile Ile Asp Ser Val Thr Asp Arg
    130                 135                 140
Thr Tyr Pro Ala Ala Leu Gln Leu Ala Asn Lys Gly Phe Thr Glu Asn
145                 150                 155                 160
Lys Pro Asp Ala Val Val Thr Lys Arg Asn Pro Gln Lys Ile His Ile
                165                 170                 175
Asp Leu Pro Gly Met Gly Asp Lys Ala Thr Val Glu Val Asn Asp Pro
            180                 185                 190
Thr Tyr Ala Asn Val Ser Thr Ala Ile Asp Asn Leu Val Asn Gln Trp
        195                 200                 205
His Asp Asn Tyr Ser Gly Gly Asn Thr Leu Pro Ala Arg Thr Gln Tyr
    210                 215                 220
Thr Glu Ser Met Val Tyr Ser Lys Ser Gln Ile Glu Ala Ala Leu Asn
225                 230                 235                 240
Val Asn Ser Lys Ile Leu Asp Gly Thr Leu Gly Ile Asp Phe Lys Ser
                245                 250                 255
Ile Ser Lys Gly Glu Lys Lys Val Met Ile Ala Ala Tyr Lys Gln Ile
            260                 265                 270
Phe Tyr Thr Val Ser Ala Asn Leu Pro Asn Asn Pro Ala Asp Val Phe
        275                 280                 285
Asp Lys Ser Val Thr Phe Lys Glu Leu Gln Arg Lys Gly Val Ser Asn
    290                 295                 300
Glu Ala Pro Pro Leu Phe Val Ser Asn Val Ala Tyr Gly Arg Thr Val
305                 310                 315                 320
Phe Val Lys Leu Glu Thr Ser Ser Lys Ser Asn Asp Val Glu Ala Ala
                325                 330                 335
Phe Ser Ala Ala Leu Lys Gly Thr Asp Val Lys Thr Asn Gly Lys Tyr
            340                 345                 350
Ser Asp Ile Leu Glu Asn Ser Ser Phe Thr Ala Val Val Leu Gly Gly
        355                 360                 365
Asp Ala Ala Glu His Asn Lys Val Val Thr Lys Asp Phe Asp Val Ile
    370                 375                 380
Arg Asn Val Ile Lys Asp Asn Ala Thr Phe Ser Arg Lys Asn Leu Ala
385                 390                 395                 400
Tyr Pro Ile Ser Tyr Thr Ser Val Phe Leu Lys Asn Asn Lys Ile Ala
                405                 410                 415
Gly Val Asn Asn Arg Thr Glu Tyr Val Glu Thr Thr Ser Thr Glu Tyr
            420                 425                 430
Thr Ser Gly Lys Ile Asn Leu Ser His Gln Gly Ala Tyr Val Ala Gln
        435                 440                 445
```

```
Tyr Glu Ile Leu Trp Asp Glu Ile Asn Tyr Asp Asp Lys Gly Lys Glu
    450                 455                 460
Val Ile Thr Lys Arg Arg Trp Asp Asn Asn Trp Tyr Ser Lys Thr Ser
465                 470                 475                 480
Pro Phe Ser Thr Val Ile Pro Leu Gly Ala Asn Ser Arg Asn Ile Arg
                485                 490                 495
Ile Met Ala Arg Glu Cys Thr Gly Leu Ala Phe Glu Trp Trp Arg Lys
            500                 505                 510
Val Ile Asp Glu Arg Asp Val Lys Leu Ser Lys Glu Ile Asn Val Asn
        515                 520                 525
Ile Ser Gly Ser Thr Leu Ser Pro Tyr Gly Ser Ile Thr Tyr Lys
    530                 535                 540
```

<210> 126
<211> 543
<212> PRT
<213> Streptococcus pyogenes

<400> 126

```
Met Ala Ser Glu Ser Asn Lys Gln Asn Thr Ala Ser Thr Glu Thr Thr
1               5                   10                  15
Thr Thr Asn Glu Gln Pro Lys Pro Glu Ser Ser Glu Leu Thr Thr Glu
            20                  25                  30
Lys Ala Gly Gln Lys Thr Asp Asp Met Leu Asn Ser Asn Asp Met Ile
        35                  40                  45
Lys Leu Ala Pro Lys Glu Met Pro Leu Glu Ser Ala Glu Lys Glu Glu
    50                  55                  60
Lys Lys Ser Glu Asp Lys Lys Lys Ser Glu Glu Asp His Thr Glu Glu
65                  70                  75                  80
Ile Asn Asp Lys Ile Tyr Ser Leu Asn Tyr Asn Glu Leu Glu Val Leu
                85                  90                  95
Ala Lys Asn Gly Glu Thr Ile Glu Asn Phe Val Pro Lys Glu Gly Val
            100                 105                 110
Lys Lys Ala Asp Lys Phe Ile Val Ile Glu Arg Lys Lys Lys Asn Ile
        115                 120                 125
Asn Thr Thr Pro Val Asp Ile Ser Ile Ile Asp Ser Val Thr Asp Arg
    130                 135                 140
Thr Tyr Pro Ala Ala Leu Gln Leu Ala Asn Lys Gly Phe Thr Glu Asn
145                 150                 155                 160
Lys Pro Asp Ala Val Val Thr Lys Arg Asn Pro Gln Lys Ile His Ile
                165                 170                 175
Asp Leu Pro Gly Met Gly Asp Lys Ala Thr Val Glu Val Asn Asp Pro
            180                 185                 190
Thr Tyr Ala Asn Val Ser Thr Ala Ile Asp Asn Leu Val Asn Gln Trp
        195                 200                 205
His Asp Asn Tyr Ser Gly Gly Asn Thr Leu Pro Ala Arg Thr Gln Tyr
    210                 215                 220
Thr Glu Ser Met Val Tyr Ser Lys Ser Gln Ile Glu Ala Ala Leu Asn
225                 230                 235                 240
Val Asn Ser Lys Ile Leu Asp Gly Thr Leu Gly Ile Asp Phe Lys Ser
                245                 250                 255
Ile Ser Lys Gly Glu Lys Lys Val Met Ile Ala Ala Tyr Lys Gln Ile
            260                 265                 270
Phe Tyr Thr Val Ser Ala Asn Leu Pro Asn Asn Pro Ala Asp Val Phe
        275                 280                 285
Asp Lys Ser Val Thr Phe Lys Glu Leu Gln Arg Lys Gly Val Ser Asn
    290                 295                 300
Glu Ala Pro Pro Leu Phe Val Ser Asn Val Ala Tyr Gly Arg Thr Val
305                 310                 315                 320
Phe Val Lys Leu Glu Thr Ser Ser Lys Ser Asn Asp Val Glu Ala Ala
                325                 330                 335
```

```
Phe Ser Ala Ala Leu Lys Gly Thr Asp Val Lys Thr Asn Gly Lys Tyr
            340                 345                 350
Ser Asp Ile Leu Glu Asn Ser Ser Phe Thr Ala Val Val Leu Gly Gly
        355                 360                 365
Asp Ala Ala Glu His Asn Lys Val Val Thr Lys Asp Phe Asp Val Ile
    370                 375                 380
Arg Asn Val Ile Lys Asp Asn Ala Thr Phe Ser Arg Lys Asn Leu Ala
385                 390                 395                 400
Tyr Pro Ile Ser Tyr Thr Ser Val Phe Leu Lys Asn Asn Lys Ile Ala
                405                 410                 415
Gly Val Asn Asn Arg Thr Glu Tyr Val Glu Thr Thr Ser Thr Glu Tyr
            420                 425                 430
Thr Ser Gly Lys Ile Asn Leu Ser His Gln Gly Ala Tyr Val Ala Gln
        435                 440                 445
Tyr Glu Ile Leu Trp Asp Glu Ile Asn Tyr Asp Asp Lys Gly Lys Glu
    450                 455                 460
Val Ile Thr Lys Arg Arg Trp Asp Asn Asn Trp Tyr Ser Lys Thr Ser
465                 470                 475                 480
Pro Phe Ser Thr Val Ile Pro Leu Gly Ala Asn Ser Arg Asn Ile Arg
                485                 490                 495
Ile Met Ala Arg Glu Gly Thr Gly Leu Ala Trp Glu Trp Trp Arg Lys
            500                 505                 510
Val Ile Asp Glu Arg Asp Val Lys Leu Ser Lys Glu Ile Asn Val Asn
        515                 520                 525
Ile Ser Gly Ser Thr Leu Ser Pro Tyr Gly Ser Ile Thr Tyr Lys
    530                 535                 540
```

<210> 127
<211> 543
<212> PRT
<213> Streptococcus pyogenes

<400> 127

```
Met Ala Ser Glu Ser Asn Lys Gln Asn Thr Ala Ser Thr Glu Thr Thr
 1               5                  10                  15
Thr Thr Asn Glu Gln Pro Lys Pro Glu Ser Ser Glu Leu Thr Thr Glu
            20                  25                  30
Lys Ala Gly Gln Lys Thr Asp Asp Met Leu Asn Ser Asn Asp Met Ile
        35                  40                  45
Lys Leu Ala Pro Lys Glu Met Pro Leu Glu Ser Ala Glu Lys Glu Glu
    50                  55                  60
Lys Lys Ser Glu Asp Lys Lys Lys Ser Glu Glu Asp His Thr Glu Glu
65                  70                  75                  80
Ile Asn Asp Lys Ile Tyr Ser Leu Asn Tyr Asn Glu Leu Glu Val Leu
                85                  90                  95
Ala Lys Asn Gly Glu Thr Ile Glu Asn Phe Val Pro Lys Glu Gly Val
            100                 105                 110
Lys Lys Ala Asp Lys Phe Ile Val Ile Glu Arg Lys Lys Lys Asn Ile
        115                 120                 125
Asn Thr Thr Pro Val Asp Ile Ser Ile Ile Asp Ser Val Thr Asp Arg
    130                 135                 140
Thr Tyr Pro Ala Ala Leu Gln Leu Ala Asn Lys Gly Phe Thr Glu Asn
145                 150                 155                 160
Lys Pro Asp Ala Val Val Thr Lys Arg Asn Pro Gln Lys Ile His Ile
                165                 170                 175
Asp Leu Pro Gly Met Gly Asp Lys Ala Thr Val Glu Val Asn Asp Pro
            180                 185                 190
Thr Tyr Ala Asn Val Ser Thr Ala Ile Asp Asn Leu Val Asn Gln Trp
        195                 200                 205
His Asp Asn Tyr Ser Gly Gly Asn Thr Leu Pro Ala Arg Thr Gln Tyr
    210                 215                 220
```

```
Thr Glu Ser Met Val Tyr Ser Lys Ser Gln Ile Glu Ala Ala Leu Asn
225                 230                 235                 240
Val Asn Ser Lys Ile Leu Asp Gly Thr Leu Gly Ile Asp Phe Lys Ser
                245                 250                 255
Ile Ser Lys Gly Glu Lys Lys Val Met Ile Ala Ala Tyr Lys Gln Ile
            260                 265                 270
Phe Tyr Thr Val Ser Ala Asn Leu Pro Asn Asn Pro Ala Asp Val Phe
        275                 280                 285
Asp Lys Ser Val Thr Phe Lys Glu Leu Gln Arg Lys Gly Val Ser Asn
    290                 295                 300
Glu Ala Pro Pro Leu Phe Val Ser Asn Val Ala Tyr Gly Arg Thr Val
305                 310                 315                 320
Phe Val Lys Leu Glu Thr Ser Ser Lys Ser Asn Asp Val Glu Ala Ala
                325                 330                 335
Phe Ser Ala Ala Leu Lys Gly Thr Asp Val Lys Thr Asn Gly Lys Tyr
            340                 345                 350
Ser Asp Ile Leu Glu Asn Ser Ser Phe Thr Ala Val Val Leu Gly Gly
        355                 360                 365
Asp Ala Ala Glu His Asn Lys Val Val Thr Lys Asp Phe Asp Val Ile
    370                 375                 380
Arg Asn Val Ile Lys Asp Asn Ala Thr Phe Ser Arg Lys Asn Leu Ala
385                 390                 395                 400
Tyr Pro Ile Ser Tyr Thr Ser Val Phe Leu Lys Asn Asn Lys Ile Ala
                405                 410                 415
Gly Val Asn Asn Arg Thr Glu Tyr Val Glu Thr Thr Ser Thr Glu Tyr
            420                 425                 430
Thr Ser Gly Lys Ile Asn Leu Ser His Gln Gly Ala Tyr Val Ala Gln
        435                 440                 445
Tyr Glu Ile Leu Trp Asp Glu Ile Asn Tyr Asp Asp Lys Gly Lys Glu
    450                 455                 460
Val Ile Thr Lys Arg Arg Trp Asp Asn Asn Trp Tyr Ser Lys Thr Ser
465                 470                 475                 480
Pro Phe Ser Thr Val Ile Pro Leu Gly Ala Asn Ser Arg Asn Ile Arg
                485                 490                 495
Ile Met Ala Arg Glu Gly Thr Gly Leu Ala Phe Glu Trp Trp Arg Lys
            500                 505                 510
Val Ile Asp Glu Arg Asp Val Lys Leu Ser Lys Glu Ile Asn Val Asn
        515                 520                 525
Ile Ser Gly Ser Thr Leu Ser Pro Tyr Gly Ser Ile Thr Tyr Lys
    530                 535                 540
```

<210> 128
<211> 873
<212> PRT
<213> Streptococcus pyogenes

<400> 128

Met Asp Leu Glu Gln Thr Lys Pro Asn Gln Val Lys Gln Lys Ile Ala
1 5 10 15
Leu Thr Ser Thr Ile Ala Leu Leu Ser Ala Ser Val Gly Val Ser His
20 25 30
Gln Val Lys Ala Asp Asp Arg Ala Ser Gly Glu Thr Lys Ala Ser Asn
35 40 45
Thr His Asp Asp Ser Leu Pro Lys Pro Glu Thr Ile Gln Glu Ala Lys
50 55 60
Ala Thr Ile Asp Ala Val Glu Lys Thr Leu Ser Gln Gln Lys Ala Glu
65 70 75 80
Leu Thr Glu Leu Ala Thr Ala Leu Thr Lys Thr Thr Ala Glu Ile Asn
85 90 95
His Leu Lys Glu Gln Gln Asp Asn Glu Gln Lys Ala Leu Thr Ser Ala
100 105 110

```
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
```

```
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 129
<211> 873
<212> PRT
<213> Streptococcus pyogenes

<400> 129

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Ala Asp Gln His Ser Lys Glu Thr Ala Leu Ser
```

```
145                 150                 155                 160
Glu Gln Lys Ala Ser Ile Ser Val Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Lys Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Ala Ala Lys Ala Lys Gln Ala Gln Leu Glu Ala Thr Arg Asp Gln
    610                 615                 620
Ser Leu Ala Lys Leu Ala Ser Leu Lys Ala Ala Leu His Gln Thr Glu
625                 630                 635                 640
```

Ala Leu Ala Glu Gln Ala Ala Ala Arg Val Thr Ala Leu Val Ala Lys
645 650 655
Lys Ala His Leu Gln Tyr Leu Arg Asp Phe Lys Leu Asn Pro Asn Arg
660 665 670
Leu Gln Val Ile Arg Glu Arg Ile Asp Asn Thr Lys Gln Asp Leu Ala
675 680 685
Lys Thr Thr Ser Ser Leu Leu Asn Ala Gln Glu Ala Leu Ala Ala Leu
690 695 700
Gln Ala Lys Gln Ser Ser Leu Glu Ala Thr Ile Ala Thr Thr Glu His
705 710 715 720
Gln Leu Thr Leu Leu Lys Thr Leu Ala Asn Glu Lys Glu Tyr Arg His
725 730 735
Leu Asp Glu Asp Ile Ala Thr Val Pro Asp Leu Gln Val Ala Pro Pro
740 745 750
Leu Thr Gly Val Lys Pro Leu Ser Tyr Ser Lys Ile Asp Thr Thr Pro
755 760 765
Leu Val Gln Glu Met Val Lys Glu Thr Lys Gln Leu Leu Glu Ala Ser
770 775 780
Ala Arg Leu Ala Ala Glu Asn Thr Ser Leu Val Ala Glu Ala Leu Val
785 790 795 800
Gly Gln Thr Ser Glu Met Val Ala Ser Asn Ala Ile Val Ser Lys Ile
805 810 815
Thr Ser Ser Ile Thr Gln Pro Ser Ser Lys Thr Ser Tyr Gly Ser Gly
820 825 830
Ser Ser Thr Thr Ser Asn Leu Ile Ser Asp Val Asp Glu Ser Thr Gln
835 840 845
Arg Ala Leu Lys Ala Gly Val Val Met Leu Ala Ala Val Gly Leu Thr
850 855 860
Gly Phe Arg Phe Arg Lys Glu Ser Arg
865 870

<210> 130
<211> 873
<212> PRT
<213> Streptococcus pyogenes

<400> 130

```
Met Asp Leu Glu Gln Thr Lys Pro Asn Gln Val Lys Gln Lys Ile Ala
 1               5                  10                  15
Leu Thr Ser Thr Ile Ala Leu Leu Ser Ala Ser Val Gly Val Ser His
            20                  25                  30
Gln Val Lys Ala Asp Asp Arg Ala Ser Gly Glu Thr Lys Ala Ser Asn
        35                  40                  45
Thr His Asp Asp Ser Leu Pro Lys Pro Glu Thr Ile Gln Glu Ala Lys
    50                  55                  60
Ala Thr Ile Asp Ala Val Glu Lys Thr Leu Ser Gln Gln Lys Ala Glu
65                  70                  75                  80
Leu Thr Glu Leu Ala Thr Ala Leu Thr Lys Thr Thr Ala Glu Ile Asn
                85                  90                  95
His Leu Lys Glu Gln Gln Asp Asn Glu Gln Lys Ala Leu Thr Ser Ala
            100                 105                 110
Gln Glu Ile Tyr Thr Asn Thr Leu Ala Ser Ser Glu Glu Thr Leu Leu
        115                 120                 125
Ala Gln Gly Ala Glu His Gln Arg Glu Leu Thr Ala Thr Glu Thr Glu
    130                 135                 140
Leu His Asn Ala Gln Val Asp Gln His Ser Lys Glu Thr Ala Leu Ser
145                 150                 155                 160
Glu Gln Lys Ala Ser Ile Ser Ala Glu Thr Thr Arg Ala Gln Asp Leu
                165                 170                 175
Val Glu Gln Val Lys Thr Ser Glu Gln Asn Ile Ala Lys Leu Asn Ala
            180                 185                 190
```

```
Met Ile Ser Asn Pro Asp Ala Ile Thr Lys Ala Ala Gln Thr Ala Asn
        195                 200                 205
Asp Asn Thr Lys Ala Leu Ser Ser Glu Leu Glu Lys Ala Lys Ala Asp
    210                 215                 220
Leu Glu Asn Gln Lys Ala Lys Val Lys Lys Gln Leu Thr Glu Glu Leu
225                 230                 235                 240
Ala Ala Gln Lys Ala Ala Leu Ala Glu Lys Glu Ala Glu Leu Ser Arg
                245                 250                 255
Leu Lys Ser Ser Ala Pro Ser Thr Gln Asp Ser Ile Val Gly Asn Asn
            260                 265                 270
Thr Met Lys Ala Pro Gln Gly Tyr Pro Leu Glu Glu Leu Lys Lys Leu
        275                 280                 285
Glu Ala Ser Gly Tyr Ile Gly Ser Ala Ser Tyr Asn Asn Tyr Tyr Lys
    290                 295                 300
Glu His Ala Asp Gln Ile Ile Ala Lys Ala Ser Pro Gly Asn Gln Leu
305                 310                 315                 320
Asn Gln Tyr Gln Asp Ile Pro Ala Asp Arg Asn Arg Phe Val Asp Pro
                325                 330                 335
Asp Asn Leu Thr Pro Glu Val Gln Asn Glu Leu Ala Gln Phe Ala Ala
            340                 345                 350
His Met Ile Asn Ser Val Arg Arg Gln Leu Gly Leu Pro Pro Val Thr
        355                 360                 365
Val Thr Ala Gly Ser Gln Glu Phe Ala Arg Leu Leu Ser Thr Ser Tyr
    370                 375                 380
Lys Lys Thr His Gly Asn Thr Arg Pro Ser Phe Val Tyr Gly Gln Pro
385                 390                 395                 400
Gly Val Ser Gly His Tyr Gly Val Gly Pro His Asp Lys Thr Ile Ile
                405                 410                 415
Glu Asp Ser Ala Gly Ala Ser Gly Leu Ile Arg Asn Asp Asp Asn Met
            420                 425                 430
Tyr Glu Asn Ile Gly Ala Phe Asn Asp Val His Thr Val Asn Gly Ile
        435                 440                 445
Lys Arg Gly Ile Tyr Asp Ser Ile Lys Tyr Met Leu Phe Thr Asp His
    450                 455                 460
Leu His Gly Asn Thr Tyr Gly His Ala Ile Asn Phe Leu Arg Val Asp
465                 470                 475                 480
Lys His Asn Pro Asn Ala Pro Val Tyr Leu Gly Phe Ser Thr Ser Asn
                485                 490                 495
Val Gly Ser Leu Asn Glu His Phe Val Met Phe Pro Glu Ser Asn Ile
            500                 505                 510
Ala Asn His Gln Arg Phe Asn Lys Thr Pro Ile Lys Ala Val Gly Ser
        515                 520                 525
Thr Lys Asp Tyr Ala Gln Arg Val Gly Thr Val Ser Asp Thr Ile Ala
    530                 535                 540
Ala Ile Lys Gly Lys Val Ser Ser Leu Glu Asn Arg Leu Ser Ala Ile
545                 550                 555                 560
His Gln Glu Ala Asp Ile Met Ala Ala Gln Ala Lys Val Ser Gln Leu
                565                 570                 575
Gln Gly Lys Leu Ala Ser Thr Leu Lys Gln Ser Asp Ser Leu Asn Leu
            580                 585                 590
Gln Val Arg Gln Leu Asn Asp Thr Lys Gly Ser Leu Arg Thr Glu Leu
        595                 600                 605
Leu Val Ala Lys Ala Lys Gln Ala Gln Leu Glu Ala Thr Arg Asp Gln
    610                 615                 620
Ser Leu Ala Lys Leu Ala Ser Leu Lys Ala Ala Met His Gln Thr Lys
625                 630                 635                 640
Ala Leu Ala Glu Gln Ala Ala Ala Arg Val Thr Ala Leu Val Ala Lys
                645                 650                 655
Lys Ala His Leu Gln Tyr Leu Arg Asp Phe Lys Leu Asn Pro Asn Arg
            660                 665                 670
Leu Gln Val Ile Arg Glu Arg Ile Asp Asn Thr Lys Gln Asp Leu Ala
```

```
        675                 680                 685
Lys Thr Thr Ser Ser Leu Leu Asn Ala Gln Glu Ala Leu Ala Ala Leu
    690                 695                 700
Gln Ala Lys Gln Ser Ser Leu Glu Ala Thr Ile Ala Thr Thr Glu His
705                 710                 715                 720
Gln Leu Thr Leu Leu Lys Thr Leu Ala Asn Glu Lys Glu Tyr Arg His
                725                 730                 735
Leu Asp Glu Asp Ile Ala Thr Val Pro Asp Leu Gln Val Ala Pro Ser
            740                 745                 750
Leu Thr Gly Val Lys Pro Leu Ser Tyr Ser Lys Val Glu Thr Thr Pro
        755                 760                 765
Leu Val Gln Glu Met Val Lys Glu Thr Lys Gln Leu Leu Glu Ala Ser
    770                 775                 780
Ala Arg Leu Ala Ala Glu Asn Thr Ser Leu Val Ala Glu Ala Leu Val
785                 790                 795                 800
Gly Gln Thr Ser Glu Met Val Ala Ser Asn Ala Ile Val Ser Lys Ile
                805                 810                 815
Thr Ser Ser Ile Thr Gln Pro Ser Ser Lys Thr Ser Tyr Gly Ser Gly
            820                 825                 830
Ser Ser Thr Thr Ser Asn Leu Ile Ser Asp Val Asp Glu Ser Thr Gln
        835                 840                 845
Arg Ala Leu Lys Ala Gly Val Val Met Leu Ala Ala Val Gly Leu Thr
    850                 855                 860
Gly Phe Arg Phe Arg Lys Glu Ser Arg
865                 870
```

<210> 131
<211> 107
<212> PRT
<213> Streptococcus pyogenes

<400> 131

```
Met Ala Arg Ala Ala Leu Ser Ala Ala Pro Ser Asn Pro Arg Leu Leu
1               5                   10                  15
Arg Val Ala Leu Leu Leu Leu Leu Leu Val Ala Ala Gly Arg Arg Ala
            20                  25                  30
Ala Gly Ala Ser Val Ala Thr Glu Leu Arg Cys Gln Cys Leu Gln Thr
        35                  40                  45
Leu Gln Gly Ile His Pro Lys Asn Ile Gln Ser Val Asn Val Lys Ser
    50                  55                  60
Pro Gly Pro His Cys Ala Gln Thr Glu Val Ile Ala Thr Leu Lys Asn
65                  70                  75                  80
Gly Arg Lys Ala Cys Leu Asn Pro Ala Ser Pro Ile Val Lys Lys Ile
                85                  90                  95
Ile Glu Lys Met Leu Asn Ser Asp Lys Ser Asn
            100                 105
```

<210> 132
<211> 107
<212> PRT
<213> Streptococcus pyogenes

<400> 132

```
Met Ala Arg Ala Thr Leu Ser Ala Ala Pro Ser Asn Pro Arg Leu Leu
 1              5                  10                  15
Arg Val Ala Leu Leu Leu Leu Leu Leu Val Ala Ala Ser Arg Arg Ala
            20                  25                  30
Ala Gly Ala Pro Leu Ala Thr Glu Leu Arg Cys Gln Cys Leu Gln Thr
        35                  40                  45
Leu Gln Gly Ile His Leu Lys Asn Ile Gln Ser Val Lys Val Lys Ser
    50                  55                  60

Pro Gly Pro His Cys Ala Gln Thr Glu Val Ile Ala Thr Leu Lys Asn
65                  70                  75                  80
Gly Gln Lys Ala Cys Leu Asn Pro Ala Ser Pro Met Val Lys Lys Ile
                85                  90                  95
Ile Glu Lys Met Leu Lys Asn Gly Lys Ser Asn
            100                 105
```

<210> 133
<211> 107
<212> PRT
<213> Streptococcus pyogenes

<400> 133

```
Met Ala His Ala Thr Leu Ser Ala Ala Pro Ser Asn Pro Arg Leu Leu
 1              5                  10                  15
Arg Val Ala Leu Leu Leu Leu Leu Leu Val Ala Ala Ser Arg Arg Ala
            20                  25                  30
Ala Gly Ala Ser Val Val Thr Glu Leu Arg Cys Gln Cys Leu Gln Thr
        35                  40                  45
Leu Gln Gly Ile His Leu Lys Asn Ile Gln Ser Val Asn Val Arg Ser
    50                  55                  60
Pro Gly Pro His Cys Ala Gln Thr Glu Val Ile Ala Thr Leu Lys Asn
65                  70                  75                  80
Gly Lys Lys Ala Cys Leu Asn Pro Ala Ser Pro Met Val Gln Lys Ile
                85                  90                  95
Ile Glu Lys Ile Leu Asn Lys Gly Ser Thr Asn
            100                 105
```

<210> 134
<211> 101
<212> PRT
<213> Streptococcus pyogenes

<220>
<221> VARIANT
<222> (1)...(105)
<223> Xaa = Any Amino Acid

<400> 134

Met Ser Ser Ala Ala Gly Phe Cys Ala Ser Arg Pro Gly Leu Leu Phe
1 5 10 15
Leu Gly Leu Leu Leu Leu Pro Leu Val Val Ala Phe Ala Ser Ala Glu
20 25 30
Ala Glu Glu Asp Gly Asp Leu Gln Cys Leu Cys Val Lys Thr Thr Ser
35 40 45
Gln Val Arg Pro Arg His Ile Thr Ser Leu Glu Val Ile Lys Ala Gly
50 55 60
Pro His Cys Pro Thr Ala Gln Leu Ile Ala Thr Leu Lys Asn Gly Arg
65 70 75 80
Lys Ile Cys Leu Asp Leu Gln Ala Pro Leu Tyr Lys Lys Ile Ile Lys
85 90 95
Lys Leu Leu Glu Ser
100

<210> 135
<211> 89
<212> PRT
<213> Streptococcus pyogenes

<400> 135

Met Asn Ala Lys Val Val Val Val Leu Val Leu Val Leu Thr Ala Leu
1 5 10 15
Cys Leu Ser Asp Gly Lys Pro Val Ser Leu Ser Tyr Arg Cys Pro Cys
20 25 30
Arg Phe Phe Glu Ser His Val Ala Arg Ala Asn Val Lys His Leu Lys
35 40 45
Ile Leu Asn Thr Pro Asn Cys Ala Leu Gln Ile Val Ala Arg Leu Lys
50 55 60
Asn Asn Asn Arg Gln Val Cys Ile Asp Pro Lys Leu Lys Trp Ile Gln
65 70 75 80
Glu Tyr Leu Glu Lys Ala Leu Asn Lys
85

<210> 136
<211> 93
<212> PRT
<213> Streptococcus pyogenes

<400> 136

Met Asn Ala Lys Val Val Val Val Leu Val Leu Val Leu Thr Ala Leu
1 5 10 15
Cys Leu Ser Asp Gly Lys Pro Val Ser Leu Ser Tyr Arg Cys Pro Cys
20 25 30
Arg Phe Phe Glu Ser His Val Ala Arg Ala Asn Val Lys His Leu Lys
35 40 45
Ile Leu Asn Thr Pro Asn Cys Ala Leu Gln Ile Val Ala Arg Leu Lys
50 55 60
Asn Asn Asn Arg Gln Val Cys Ile Asp Pro Lys Leu Lys Trp Ile Gln
65 70 75 80
Glu Tyr Leu Glu Lys Ala Leu Asn Lys Arg Phe Lys Met
85 90

<210> 137

<211> 119
<212> PRT
<213> Streptococcus pyogenes

<400> 137

```
Met Asn Ala Lys Val Val Val Val Leu Val Leu Val Leu Thr Ala Leu
 1               5                  10                  15
Cys Leu Ser Asp Gly Lys Pro Val Ser Leu Ser Tyr Arg Cys Pro Cys
            20                  25                  30
Arg Phe Phe Glu Ser His Val Ala Arg Ala Asn Val Lys His Leu Lys
        35                  40                  45
Ile Leu Asn Thr Pro Asn Cys Ala Leu Gln Ile Val Ala Arg Leu Lys
    50                  55                  60
Asn Asn Asn Arg Gln Val Cys Ile Asp Pro Lys Leu Lys Trp Ile Gln
65                  70                  75                  80
Glu Tyr Leu Glu Lys Ala Leu Asn Lys Gly Arg Arg Glu Glu Lys Val
                85                  90                  95
Gly Lys Lys Glu Lys Ile Gly Lys Lys Lys Arg Gln Lys Lys Arg Lys
            100                 105                 110
Ala Ala Gln Lys Arg Lys Asn
        115
```

<210> 138
<211> 114
<212> PRT
<213> Streptococcus pyogenes

<400> 138

```
Met Ser Leu Leu Ser Ser Arg Ala Ala Arg Val Pro Gly Pro Ser Ser


 1               5                  10                  15
Ser Leu Cys Ala Leu Leu Val Leu Leu Leu Leu Leu Thr Gln Pro Gly
            20                  25                  30
Pro Ile Ala Ser Ala Gly Pro Ala Ala Ala Val Leu Arg Glu Leu Arg
        35                  40                  45
Cys Val Cys Leu Gln Thr Thr Gln Gly Val His Pro Lys Met Ile Ser
    50                  55                  60
Asn Leu Gln Val Phe Ala Ile Gly Pro Gln Cys Ser Lys Val Glu Val
65                  70                  75                  80
Val Ala Ser Leu Lys Asn Gly Lys Glu Ile Cys Leu Asp Pro Glu Ala
                85                  90                  95
Pro Phe Leu Lys Lys Val Ile Gln Lys Ile Leu Asp Gly Gly Asn Lys
            100                 105                 110
Glu Asn
```

<210> 139
<211> 114
<212> PRT
<213> Streptococcus pyogenes

<400> 139

```
Met Ser Leu Pro Ser Ser Arg Ala Ala Arg Val Pro Gly Pro Ser Gly
 1               5                  10                  15
Ser Leu Cys Ala Leu Leu Ala Leu Leu Leu Leu Leu Thr Pro Pro Gly
            20                  25                  30
Pro Leu Ala Ser Ala Gly Pro Val Ser Ala Val Leu Thr Glu Leu Arg
        35                  40                  45
Cys Thr Cys Leu Arg Val Thr Leu Arg Val Asn Pro Lys Thr Ile Gly
    50                  55                  60
Lys Leu Gln Val Phe Pro Ala Gly Pro Gln Cys Ser Lys Val Glu Val
65                  70                  75                  80
Val Ala Ser Leu Lys Asn Gly Lys Gln Val Cys Leu Asp Pro Glu Ala
                85                  90                  95
Pro Phe Leu Lys Lys Val Ile Gln Lys Ile Leu Asp Ser Gly Asn Lys
            100                 105                 110
Lys Asn
```

<210> 140
<211> 128
<212> PRT
<213> Streptococcus pyogenes

<400> 140

```
Met Ser Leu Arg Leu Asp Thr Thr Pro Ser Cys Asn Ser Ala Arg Pro
 1               5                  10                  15
Leu His Ala Leu Gln Val Leu Leu Leu Leu Ser Leu Leu Leu Thr Ala
            20                  25                  30
Leu Ala Ser Ser Thr Lys Gly Gln Thr Lys Arg Asn Leu Ala Lys Gly
        35                  40                  45
Lys Glu Glu Ser Leu Asp Ser Asp Leu Tyr Ala Glu Leu Arg Cys Met
    50                  55                  60
Cys Ile Lys Thr Thr Ser Gly Ile His Pro Lys Asn Ile Gln Ser Leu
65                  70                  75                  80
Glu Val Ile Gly Lys Gly Thr His Cys Asn Gln Val Glu Val Ile Ala
                85                  90                  95
Thr Leu Lys Asp Gly Arg Lys Ile Cys Leu Asp Pro Asp Ala Pro Arg
            100                 105                 110
Ile Lys Lys Ile Val Gln Lys Lys Leu Ala Gly Asp Glu Ser Ala Asp
        115                 120                 125
```

<210> 141
<211> 125
<212> PRT
<213> Streptococcus pyogenes

<400> 141

```
Met Lys Lys Ser Gly Val Leu Phe Leu Leu Gly Ile Ile Leu Leu Val
 1               5                  10                  15
Leu Ile Gly Val Gln Gly Thr Pro Val Val Arg Lys Gly Arg Cys Ser
            20                  25                  30
Cys Ile Ser Thr Asn Gln Gly Thr Ile His Leu Gln Ser Leu Lys Asp
        35                  40                  45
Leu Lys Gln Phe Ala Pro Ser Pro Ser Cys Glu Lys Ile Glu Ile Ile
    50                  55                  60
Ala Thr Leu Lys Asn Gly Val Gln Thr Cys Leu Asn Pro Asp Ser Ala
65                  70                  75                  80
Asp Val Lys Glu Leu Ile Lys Lys Trp Glu Lys Gln Val Ser Gln Lys
                85                  90                  95
Lys Lys Gln Lys Asn Gly Lys Lys His Gln Lys Lys Lys Val Leu Lys
            100                 105                 110
Val Arg Lys Ser Gln Arg Ser Arg Gln Lys Lys Thr Thr
        115                 120                 125
```

<210> 142
<211> 98
<212> PRT
<213> Streptococcus pyogenes

<400> 142

```
Met Asn Gln Thr Ala Ile Leu Ile Cys Cys Leu Ile Phe Leu Thr Leu
 1               5                  10                  15
Ser Gly Ile Gln Gly Val Pro Leu Ser Arg Thr Val Arg Cys Thr Cys
            20                  25                  30
Ile Ser Ile Ser Asn Gln Pro Val Asn Pro Arg Ser Leu Glu Lys Leu
        35                  40                  45
Glu Ile Ile Pro Ala Ser Gln Phe Cys Pro Arg Val Glu Ile Ile Ala
    50                  55                  60
Thr Met Lys Lys Lys Gly Glu Lys Arg Cys Leu Asn Pro Glu Ser Lys
65                  70                  75                  80
Ala Ile Lys Asn Leu Leu Lys Ala Val Ser Lys Glu Arg Ser Lys Arg
                85                  90                  95
Ser Pro
```

<210> 143
<211> 94
<212> PRT
<213> Streptococcus pyogenes

<400> 143

```
Met Ser Val Lys Gly Met Ala Ile Ala Leu Ala Val Ile Leu Cys Ala
 1               5                  10                  15
Thr Val Val Gln Gly Phe Pro Met Phe Lys Arg Gly Arg Cys Leu Cys
            20                  25                  30
Ile Gly Pro Gly Val Lys Ala Val Lys Val Ala Asp Ile Glu Lys Ala
        35                  40                  45
Ser Ile Met Tyr Pro Ser Asn Asn Cys Asp Lys Ile Glu Val Ile Ile
    50                  55                  60
Thr Leu Lys Glu Asn Lys Gly Gln Arg Cys Leu Asn Pro Lys Ser Lys
65                  70                  75                  80
```

Gln Ala Arg Leu Ile Ile Lys Lys Val Glu Arg Lys Asn Phe
85 90

<210> 144
<211> 109
<212> PRT
<213> Streptococcus pyogenes

<400> 144

Met Lys Phe Ile Ser Thr Ser Leu Leu Leu Met Leu Leu Val Ser Ser
1 5 10 15
Leu Ser Pro Val Gln Gly Val Leu Glu Val Tyr Tyr Thr Ser Leu Arg
20 25 30
Cys Arg Cys Val Gln Glu Ser Ser Val Phe Ile Pro Arg Arg Phe Ile
35 40 45
Asp Arg Ile Gln Ile Leu Pro Arg Gly Asn Gly Cys Pro Arg Lys Glu
50 55 60
Ile Ile Val Trp Lys Lys Asn Lys Ser Ile Val Cys Val Asp Pro Gln
65 70 75 80
Ala Glu Trp Ile Gln Arg Met Met Glu Val Leu Arg Lys Arg Ser Ser
85 90 95
Ser Thr Leu Pro Val Pro Val Phe Lys Arg Lys Ile Pro
100 105

<210> 145
<211> 99
<212> PRT
<213> Streptococcus pyogenes

<400> 145

Met Arg Leu Leu Ala Ala Ala Leu Leu Leu Leu Leu Leu Ala Leu Tyr
1 5 10 15
Thr Ala Arg Val Asp Gly Ser Lys Cys Lys Cys Ser Arg Lys Gly Pro
20 25 30
Lys Ile Arg Tyr Ser Asp Val Lys Lys Leu Glu Met Lys Pro Lys Tyr
35 40 45
Pro His Cys Glu Glu Lys Met Val Ile Ile Thr Thr Lys Ser Val Ser
50 55 60
Arg Tyr Arg Gly Gln Glu His Cys Leu His Pro Lys Leu Gln Ser Thr
65 70 75 80
Lys Arg Phe Ile Lys Trp Tyr Asn Ala Trp Asn Glu Lys Arg Arg Val
85 90 95
Tyr Glu Glu

<210> 146
<211> 254
<212> PRT
<213> Streptococcus pyogenes

<400> 146

```
Met Gly Arg Asp Leu Arg Pro Gly Ser Arg Val Leu Leu Leu Leu Leu
1               5                   10                  15
Leu Leu Leu Leu Val Tyr Leu Thr Gln Pro Gly Asn Gly Asn Glu Gly
            20                  25                  30
Ser Val Thr Gly Ser Cys Tyr Cys Gly Lys Arg Ile Ser Ser Asp Ser
        35                  40                  45
Pro Pro Ser Val Gln Phe Met Asn Arg Leu Arg Lys His Leu Arg Ala
    50                  55                  60
Tyr His Arg Cys Leu Tyr Tyr Thr Arg Phe Gln Leu Leu Ser Trp Ser
65                  70                  75                  80

Val Cys Gly Gly Asn Lys Asp Pro Trp Val Gln Glu Leu Met Ser Cys
                85                  90                  95
Leu Asp Leu Lys Glu Cys Gly His Ala Tyr Ser Gly Ile Val Ala His
            100                 105                 110
Gln Lys His Leu Leu Pro Thr Ser Pro Pro Ile Ser Gln Ala Ser Glu
        115                 120                 125
Gly Ala Ser Ser Asp Ile His Thr Pro Ala Gln Met Leu Leu Ser Thr
    130                 135                 140
Leu Gln Ser Thr Gln Arg Pro Thr Leu Pro Val Gly Ser Leu Ser Ser
145                 150                 155                 160
Asp Lys Glu Leu Thr Arg Pro Asn Glu Thr Thr Ile His Thr Ala Gly
                165                 170                 175
His Ser Leu Ala Ala Gly Pro Glu Ala Gly Glu Asn Gln Lys Gln Pro
            180                 185                 190
Glu Lys Asn Ala Gly Pro Thr Ala Arg Thr Ser Ala Thr Val Pro Val
        195                 200                 205
Leu Cys Leu Leu Ala Ile Ile Phe Ile Leu Thr Ala Ala Leu Ser Tyr
    210                 215                 220
Val Leu Cys Lys Arg Arg Arg Gly Gln Ser Pro Gln Ser Ser Pro Asp
225                 230                 235                 240
Leu Pro Val His Tyr Ile Pro Val Ala Pro Asp Ser Asn Thr
                245                 250
```

<210> 147
<211> 1580
<212> PRT
<213> Streptococcus pyogenes

<400> 147

```
Ala Asp Glu Leu Ser Thr Met Ser Glu Pro Thr Ile Thr Asn His Ala
 1               5                  10                  15
Gln Gln Gln Ala Gln His Leu Thr Asn Thr Glu Leu Ser Ser Ala Glu
            20                  25                  30
Ser Lys Ser Gln Asp Thr Ser Gln Ile Thr Leu Lys Thr Asn Arg Glu
        35                  40                  45
Lys Glu Gln Ser Gln Asp Leu Val Ser Glu Pro Thr Thr Thr Glu Leu
    50                  55                  60
Ala Asp Thr Asp Ala Ala Ser Met Ala Asn Thr Gly Ser Asp Ala Thr
65                  70                  75                  80
Gln Lys Ser Ala Ser Leu Pro Pro Val Asn Thr Asp Val His Asp Trp
                85                  90                  95
Val Lys Thr Lys Gly Ala Trp Asp Lys Gly Tyr Lys Gly Gln Gly Lys
            100                 105                 110
Val Val Ala Val Ile Ala Thr Gly Ile Asp Pro Ala His Gln Ser Met
        115                 120                 125
Arg Ile Ser Asp Val Ser Thr Ala Lys Val Lys Ser Lys Glu Asp Met
    130                 135                 140
Leu Ala Arg Gln Lys Ala Ala Gly Ile Asn Tyr Gly Ser Trp Ile Asn
145                 150                 155                 160
Asp Lys Val Val Phe Ala His Asn Tyr Val Glu Asn Ser Asp Asn Ile
                165                 170                 175
Lys Glu Asn Gln Phe Glu Asp Phe Asp Glu Asp Trp Glu Asn Phe Glu
            180                 185                 190
Phe Asp Ala Glu Ala Glu Pro Lys Ala Ile Lys Lys His Lys Ile Tyr
        195                 200                 205
Arg Pro Gln Ser Thr Gln Ala Pro Lys Glu Thr Val Ile Lys Thr Glu
    210                 215                 220
Glu Thr Asp Gly Ser His Asp Ile Asp Trp Thr Gln Thr Asp Asp Asp
225                 230                 235                 240
Thr Lys Tyr Glu Ser His Gly Met His Val Thr Gly Ile Val Ala Gly
                245                 250                 255
```

```
Asn Ser Lys Glu Ala Ala Ala Thr Gly Glu Arg Phe Leu Gly Ile Ala
            260                 265                 270
Pro Glu Ala Gln Val Met Phe Met Arg Val Phe Ala Asn Asp Ile Met
        275                 280                 285
Gly Ser Ala Glu Ser Leu Phe Ile Lys Ala Ile Glu Asp Ala Val Ala
    290                 295                 300
Leu Gly Ala Asp Val Ile Asn Leu Ser Leu Gly Thr Ala Asn Gly Ala
305                 310                 315                 320
Gln Leu Ser Gly Ser Lys Pro Leu Met Glu Ala Ile Glu Lys Ala Lys
                325                 330                 335
Lys Ala Gly Val Ser Val Val Val Ala Ala Gly Asn Glu Arg Val Tyr
            340                 345                 350
Gly Ser Asp His Asp Asp Pro Leu Ala Thr Asn Pro Asp Tyr Gly Leu
        355                 360                 365
Val Gly Ser Pro Ser Thr Gly Arg Thr Pro Thr Ser Val Ala Ala Ile
    370                 375                 380
Asn Ser Lys Trp Val Ile Gln Arg Leu Met Thr Val Lys Glu Leu Glu
385                 390                 395                 400
Asn Arg Ala Asp Leu Asn His Gly Lys Ala Ile Tyr Ser Glu Ser Val
                405                 410                 415
Asp Phe Lys Asp Ile Lys Asp Ser Leu Gly Tyr Asp Lys Ser His Gln
            420                 425                 430
Phe Ala Tyr Val Lys Glu Ser Thr Asp Ala Gly Tyr Asn Ala Gln Asp
        435                 440                 445
Val Lys Gly Lys Ile Ala Leu Ile Glu Arg Asp Pro Asn Lys Thr Tyr
    450                 455                 460
Asp Glu Met Ile Ala Leu Ala Lys Lys His Gly Ala Leu Gly Val Leu
465                 470                 475                 480
Ile Phe Asn Asn Lys Pro Gly Gln Ser Asn Arg Ser Met Arg Leu Thr
                485                 490                 495
Ala Asn Gly Met Gly Ile Pro Ser Ala Phe Ile Ser His Glu Phe Gly
            500                 505                 510
Lys Ala Met Ser Gln Leu Asn Gly Asn Gly Thr Gly Ser Leu Glu Phe
        515                 520                 525
Asp Ser Val Val Ser Lys Ala Pro Ser Gln Lys Gly Asn Glu Met Asn
    530                 535                 540
His Phe Ser Asn Trp Gly Leu Thr Ser Asp Gly Tyr Leu Lys Pro Asp
545                 550                 555                 560
Ile Thr Ala Pro Gly Gly Asp Ile Tyr Ser Thr Tyr Asn Asp Asn His
                565                 570                 575
Tyr Gly Ser Gln Thr Gly Thr Ser Met Ala Ser Pro Gln Ile Ala Gly
            580                 585                 590
Ala Ser Leu Leu Val Lys Gln Tyr Leu Glu Lys Thr Gln Pro Asn Leu
        595                 600                 605
Pro Lys Glu Lys Ile Ala Asp Ile Val Lys Asn Leu Leu Met Ser Asn
    610                 615                 620
Ala Gln Ile His Val Asn Pro Glu Thr Lys Thr Thr Thr Ser Pro Arg
625                 630                 635                 640
Gln Gln Gly Ala Gly Leu Leu Asn Ile Asp Gly Ala Val Thr Ser Gly
                645                 650                 655
Leu Tyr Val Thr Gly Lys Asp Asn Tyr Gly Ser Ile Ser Leu Gly Asn
            660                 665                 670
Ile Thr Asp Thr Met Thr Phe Asp Val Thr Val His Asn Leu Ser Asn
        675                 680                 685
Lys Asp Lys Thr Leu Arg Tyr Asp Thr Glu Leu Leu Thr Asp His Val
    690                 695                 700
Asp Pro Gln Lys Gly Arg Phe Thr Leu Thr Ser His Ser Leu Lys Thr
705                 710                 715                 720
Tyr Gln Gly Gly Glu Val Thr Val Pro Ala Asn Gly Lys Val Thr Val
                725                 730                 735
Arg Val Thr Met Asp Val Ser Gln Phe Thr Lys Glu Leu Thr Lys Gln
```

```
                740                 745                 750
Met Pro Asn Gly Tyr Tyr Leu Glu Gly Phe Val Arg Phe Arg Asp Ser
        755                 760                 765
Gln Asp Asp Gln Leu Asn Arg Val Asn Ile Pro Phe Val Gly Phe Lys
    770                 775                 780
Gly Gln Phe Glu Asn Leu Ala Val Ala Glu Glu Ser Ile Tyr Arg Leu
785                 790                 795                 800
Lys Ser Gln Gly Lys Thr Gly Phe Tyr Phe Asp Glu Ser Gly Pro Lys
            805                 810                 815
Asp Asp Ile Tyr Val Gly Lys His Phe Thr Gly Leu Val Thr Leu Gly
                820                 825                 830
Ser Glu Thr Asn Val Ser Thr Lys Thr Ile Ser Asp Asn Gly Leu His
        835                 840                 845
Thr Leu Gly Thr Phe Lys Asn Ala Asp Gly Lys Phe Ile Leu Glu Lys
    850                 855                 860
Asn Ala Gln Gly Asn Pro Val Leu Ala Ile Ser Pro Asn Gly Asp Asn
865                 870                 875                 880
Asn Gln Asp Phe Ala Ala Phe Lys Gly Val Phe Leu Arg Lys Tyr Gln
            885                 890                 895
Gly Leu Lys Ala Ser Val Tyr His Ala Ser Asp Lys Glu His Lys Asn
                900                 905                 910
Pro Leu Trp Val Ser Pro Glu Ser Phe Lys Gly Asp Lys Asn Phe Asn
        915                 920                 925
Ser Asp Ile Arg Phe Ala Lys Ser Thr Thr Leu Leu Gly Thr Ala Phe
    930                 935                 940
Ser Gly Lys Ser Leu Thr Gly Ala Glu Leu Pro Asp Gly His Tyr His
945                 950                 955                 960
Tyr Val Val Ser Tyr Tyr Pro Asp Val Val Gly Ala Lys Arg Gln Glu
            965                 970                 975
Met Thr Phe Asp Met Ile Leu Asp Arg Gln Lys Pro Val Leu Ser Gln
                980                 985                 990
Ala Thr Phe Asp Pro Glu Thr Asn Arg Phe Lys Pro Glu Pro Leu Lys
        995                 1000                1005
Asp Arg Gly Leu Ala Gly Val Arg Lys Asp Ser Val Phe Tyr Leu Glu
    1010                1015                1020
Arg Lys Asp Asn Lys Pro Tyr Thr Val Thr Ile Asn Asp Ser Tyr Lys
1025                1030                1035                1040
Tyr Val Ser Val Glu Asp Asn Lys Thr Phe Val Glu Arg Gln Ala Asp
            1045                1050                1055
Gly Ser Phe Ile Leu Pro Leu Asp Lys Ala Lys Leu Gly Asp Phe Tyr
                1060                1065                1070
Tyr Met Val Glu Asp Phe Ala Gly Asn Val Ala Ile Ala Lys Leu Gly
        1075                1080                1085
Asp His Leu Pro Gln Thr Leu Gly Lys Thr Pro Ile Lys Leu Lys Leu
    1090                1095                1100
Thr Asp Gly Asn Tyr Gln Thr Lys Glu Thr Leu Lys Asp Asn Leu Glu
1105                1110                1115                1120
Met Thr Gln Ser Asp Thr Gly Leu Val Thr Asn Gln Ala Gln Leu Ala
            1125                1130                1135
Val Val His Arg Asn Gln Pro Gln Ser Gln Leu Thr Lys Met Asn Gln
                1140                1145                1150
Asp Phe Phe Ile Ser Pro Asn Glu Asp Gly Asn Lys Asp Phe Val Ala
        1155                1160                1165
Phe Lys Gly Leu Lys Asn Asn Val Tyr Asn Asp Leu Thr Val Asn Val
    1170                1175                1180
Tyr Ala Lys Asp Asp His Gln Lys Gln Thr Pro Ile Trp Ser Ser Gln
1185                1190                1195                1200
Ala Gly Ala Ser Val Ser Ala Ile Glu Ser Thr Ala Trp Tyr Gly Ile
            1205                1210                1215
Thr Ala Arg Gly Ser Lys Val Met Pro Gly Asp Tyr Gln Tyr Val Val
                1220                1225                1230
```

Thr Tyr Arg Asp Glu His Gly Lys Glu His Gln Lys Gln Tyr Thr Ile
1235 1240 1245
Ser Val Asn Asp Lys Lys Pro Met Ile Thr Gln Gly Arg Phe Asp Thr
1250 1255 1260
Ile Asn Gly Val Asp His Phe Thr Pro Asp Lys Thr Lys Ala Leu Asp
1265 1270 1275 1280
Ser Ser Gly Ile Val Arg Glu Glu Val Phe Tyr Leu Ala Lys Lys Asn
1285 1290 1295
Gly Arg Lys Phe Asp Val Thr Glu Gly Lys Asp Gly Ile Thr Val Ser
1300 1305 1310
Asp Asn Lys Val Tyr Ile Pro Lys Asn Pro Asp Gly Ser Tyr Thr Ile
1315 1320 1325
Ser Lys Arg Asp Gly Val Thr Leu Ser Asp Tyr Tyr Tyr Leu Val Glu
1330 1335 1340
Asp Arg Ala Gly Asn Val Ser Phe Ala Thr Leu Arg Asp Leu Lys Ala
1345 1350 1355 1360
Val Gly Lys Asp Lys Ala Val Val Asn Phe Gly Leu Asp Leu Pro Val
1365 1370 1375
Pro Glu Asp Lys Gln Ile Val Asn Phe Thr Tyr Leu Val Arg Asp Ala
1380 1385 1390
Asp Gly Lys Pro Ile Glu Asn Leu Glu Tyr Tyr Asn Asn Ser Gly Asn
1395 1400 1405
Ser Leu Ile Leu Pro Tyr Gly Lys Tyr Thr Val Glu Leu Leu Thr Tyr
1410 1415 1420
Asp Thr Asn Ala Ala Lys Leu Glu Ser Asp Lys Ile Val Ser Phe Thr
1425 1430 1435 1440
Leu Ser Ala Asp Asn Asn Phe Gln Gln Val Thr Phe Lys Ile Thr Met
1445 1450 1455
Leu Ala Thr Ser Gln Ile Thr Ala His Phe Asp His Leu Leu Pro Glu
1460 1465 1470
Gly Ser Arg Val Ser Leu Lys Thr Ala Gln Asp Gln Leu Ile Pro Leu
1475 1480 1485
Glu Gln Ser Leu Tyr Val Pro Lys Ala Tyr Gly Lys Thr Val Gln Glu
1490 1495 1500
Gly Thr Tyr Glu Val Val Val Ser Leu Pro Lys Gly Tyr Arg Ile Glu
1505 1510 1515 1520
Gly Asn Thr Lys Val Asn Thr Leu Pro Asn Glu Val His Glu Leu Ser
1525 1530 1535
Leu Arg Leu Val Lys Val Gly Asp Ala Ser Asp Ser Thr Gly Asp His
1540 1545 1550
Lys Val Met Ser Lys Asn Asn Ser Gln Ala Leu Thr Ala Ser Ala Thr
1555 1560 1565
Pro Thr Lys Ser Thr Thr Ser Ala Thr Ala Lys Ala
1570 1575 1580

<210> 148
<211> 1580
<212> PRT
<213> Streptococcus pyogenes

<400> 148

```
Ala Asp Glu Leu Ser Thr Met Ser Glu Pro Thr Ile Thr Asn His Ala
 1               5                  10                  15
Gln Gln Gln Ala Gln His Leu Thr Asn Thr Glu Leu Ser Ser Ala Glu
            20                  25                  30
Ser Lys Ser Gln Asp Thr Ser Gln Ile Thr Leu Lys Thr Asn Arg Glu
        35                  40                  45
Lys Glu Gln Ser Gln Asp Leu Val Ser Glu Pro Thr Thr Thr Glu Leu
    50                  55                  60
Ala Asp Thr Asp Ala Ala Ser Met Ala Asn Thr Gly Ser Asp Ala Thr
65                  70                  75                  80
```

Gln Lys Ser Ala Ser Leu Pro Pro Val Asn Thr Asp Val His Asp Trp
85 90 95
Val Lys Thr Lys Gly Ala Trp Asp Lys Gly Tyr Lys Gly Gln Gly Lys
100 105 110
Val Val Ala Val Ile Asp Thr Gly Ile Asp Pro Ala His Gln Ser Met
115 120 125
Arg Ile Ser Asp Val Ser Thr Ala Lys Val Lys Ser Lys Glu Asp Met
130 135 140
Leu Ala Arg Gln Lys Ala Ala Gly Ile Asn Tyr Gly Ser Trp Ile Asn
145 150 155 160
Asp Lys Val Val Phe Ala His Asn Tyr Val Glu Asn Ser Asp Asn Ile
165 170 175
Lys Glu Asn Gln Phe Glu Asp Phe Asp Glu Asp Trp Glu Asn Phe Glu
180 185 190
Phe Asp Ala Glu Ala Glu Pro Lys Ala Ile Lys Lys His Lys Ile Tyr
195 200 205
Arg Pro Gln Ser Thr Gln Ala Pro Lys Glu Thr Val Ile Lys Thr Glu
210 215 220
Glu Thr Asp Gly Ser His Asp Ile Asp Trp Thr Gln Thr Asp Asp Asp
225 230 235 240
Thr Lys Tyr Glu Ser His Gly Met His Val Thr Gly Ile Val Ala Gly
245 250 255
Asn Ser Lys Glu Ala Ala Ala Thr Gly Glu Arg Phe Leu Gly Ile Ala
260 265 270
Pro Glu Ala Gln Val Met Phe Met Arg Val Phe Ala Asn Asp Ile Met
275 280 285
Gly Ser Ala Glu Ser Leu Phe Ile Lys Ala Ile Glu Asp Ala Val Ala
290 295 300
Leu Gly Ala Asp Val Ile Asn Leu Ser Leu Gly Thr Ala Asn Gly Ala
305 310 315 320
Gln Leu Ser Gly Ser Lys Pro Leu Met Glu Ala Ile Glu Lys Ala Lys
325 330 335
Lys Ala Gly Val Ser Val Val Val Ala Ala Gly Asn Glu Arg Val Tyr
340 345 350
Gly Ser Asp His Asp Asp Pro Leu Ala Thr Asn Pro Asp Tyr Gly Leu
355 360 365
Val Gly Ser Pro Ser Thr Gly Arg Thr Pro Thr Ser Val Ala Ala Ile
370 375 380
Asn Ser Lys Trp Val Ile Gln Arg Leu Met Thr Val Lys Glu Leu Glu
385 390 395 400
Asn Arg Ala Asp Leu Asn His Gly Lys Ala Ile Tyr Ser Glu Ser Val
405 410 415
Asp Phe Lys Asp Ile Lys Asp Ser Leu Gly Tyr Asp Lys Ser His Gln
420 425 430
Phe Ala Tyr Val Lys Glu Ser Thr Asp Ala Gly Tyr Asn Ala Gln Asp
435 440 445
Val Lys Gly Lys Ile Ala Leu Ile Glu Arg Asp Pro Asn Lys Thr Tyr
450 455 460
Asp Glu Met Ile Ala Leu Ala Lys Lys His Gly Ala Leu Gly Val Leu
465 470 475 480
Ile Phe Asn Asn Lys Pro Gly Gln Ser Asn Arg Ser Met Arg Leu Thr
485 490 495
Ala Asn Gly Met Gly Ile Pro Ser Ala Phe Ile Ser His Glu Phe Gly
500 505 510
Lys Ala Met Ser Gln Leu Asn Gly Asn Gly Thr Gly Ser Leu Glu Phe
515 520 525
Asp Ser Val Val Ser Lys Ala Pro Ser Gln Lys Gly Asn Glu Met Asn
530 535 540
His Phe Ser Asn Trp Gly Leu Thr Ser Asp Gly Tyr Leu Lys Pro Asp
545 550 555 560
Ile Thr Ala Pro Gly Gly Asp Ile Tyr Ser Thr Tyr Asn Asp Asn His

```
                565                 570                 575
Tyr Gly Ser Gln Thr Gly Thr Ala Met Ala Ser Pro Gln Ile Ala Gly
            580                 585                 590
Ala Ser Leu Leu Val Lys Gln Tyr Leu Glu Lys Thr Gln Pro Asn Leu
        595                 600                 605
Pro Lys Glu Lys Ile Ala Asp Ile Val Lys Asn Leu Leu Met Ser Asn
    610                 615                 620
Ala Gln Ile His Val Asn Pro Glu Thr Lys Thr Thr Thr Ser Pro Arg
625                 630                 635                 640
Gln Gln Gly Ala Gly Leu Leu Asn Ile Asp Gly Ala Val Thr Ser Gly
                645                 650                 655
Leu Tyr Val Thr Gly Lys Asp Asn Tyr Gly Ser Ile Ser Leu Gly Asn
            660                 665                 670
Ile Thr Asp Thr Met Thr Phe Asp Val Thr Val His Asn Leu Ser Asn
        675                 680                 685
Lys Asp Lys Thr Leu Arg Tyr Asp Thr Glu Leu Leu Thr Asp His Val
    690                 695                 700
Asp Pro Gln Lys Gly Arg Phe Thr Leu Thr Ser His Ser Leu Lys Thr
705                 710                 715                 720
Tyr Gln Gly Gly Glu Val Thr Val Pro Ala Asn Gly Lys Val Thr Val
                725                 730                 735
Arg Val Thr Met Asp Val Ser Gln Phe Thr Lys Glu Leu Thr Lys Gln
            740                 745                 750
Met Pro Asn Gly Tyr Tyr Leu Glu Gly Phe Val Arg Phe Arg Asp Ser
        755                 760                 765
Gln Asp Asp Gln Leu Asn Arg Val Asn Ile Pro Phe Val Gly Phe Lys
    770                 775                 780
Gly Gln Phe Glu Asn Leu Ala Val Ala Glu Glu Ser Ile Tyr Arg Leu
785                 790                 795                 800
Lys Ser Gln Gly Lys Thr Gly Phe Tyr Phe Asp Glu Ser Gly Pro Lys
                805                 810                 815
Asp Asp Ile Tyr Val Gly Lys His Phe Thr Gly Leu Val Thr Leu Gly
            820                 825                 830
Ser Glu Thr Asn Val Ser Thr Lys Thr Ile Ser Asp Asn Gly Leu His
        835                 840                 845
Thr Leu Gly Thr Phe Lys Asn Ala Asp Gly Lys Phe Ile Leu Glu Lys
    850                 855                 860
Asn Ala Gln Gly Asn Pro Val Leu Ala Ile Ser Pro Asn Gly Asp Asn
865                 870                 875                 880
Asn Gln Asp Phe Ala Ala Phe Lys Gly Val Phe Leu Arg Lys Tyr Gln
                885                 890                 895
Gly Leu Lys Ala Ser Val Tyr His Ala Ser Asp Lys Glu His Lys Asn
            900                 905                 910
Pro Leu Trp Val Ser Pro Glu Ser Phe Lys Gly Asp Lys Asn Phe Asn
        915                 920                 925
Ser Asp Ile Arg Phe Ala Lys Ser Thr Thr Leu Leu Gly Thr Ala Phe
    930                 935                 940
Ser Gly Lys Ser Leu Thr Gly Ala Glu Leu Pro Asp Gly His Tyr His
945                 950                 955                 960
Tyr Val Val Ser Tyr Tyr Pro Asp Val Val Gly Ala Lys Arg Gln Glu
                965                 970                 975
Met Thr Phe Asp Met Ile Leu Asp Arg Gln Lys Pro Val Leu Ser Gln
            980                 985                 990
Ala Thr Phe Asp Pro Glu Thr Asn Arg Phe Lys Pro Glu Pro Leu Lys
        995                 1000                1005
Asp Arg Gly Leu Ala Gly Val Arg Lys Asp Ser Val Phe Tyr Leu Glu
    1010                1015                1020
Arg Lys Asp Asn Lys Pro Tyr Thr Val Thr Ile Asn Asp Ser Tyr Lys
1025                1030                1035                1040
Tyr Val Ser Val Glu Asp Asn Lys Thr Phe Val Glu Arg Gln Ala Asp
                1045                1050                1055
```

```
Gly Ser Phe Ile Leu Pro Leu Asp Lys Ala Lys Leu Gly Asp Phe Tyr
            1060                1065                1070
Tyr Met Val Glu Asp Phe Ala Gly Asn Val Ala Ile Ala Lys Leu Gly
        1075                1080                1085
Asp His Leu Pro Gln Thr Leu Gly Lys Thr Pro Ile Lys Leu Lys Leu
    1090                1095                1100
Thr Asp Gly Asn Tyr Gln Thr Lys Glu Thr Leu Lys Asp Asn Leu Glu
1105                1110                1115                1120
Met Thr Gln Ser Asp Thr Gly Leu Val Thr Asn Gln Ala Gln Leu Ala
                1125                1130                1135
Val Val His Arg Asn Gln Pro Gln Ser Gln Leu Thr Lys Met Asn Gln
            1140                1145                1150
Asp Phe Phe Ile Ser Pro Asn Glu Asp Gly Asn Lys Asp Phe Val Ala
        1155                1160                1165
Phe Lys Gly Leu Lys Asn Asn Val Tyr Asn Asp Leu Thr Val Asn Val
    1170                1175                1180
Tyr Ala Lys Asp Asp His Gln Lys Gln Thr Pro Ile Trp Ser Ser Gln
1185                1190                1195                1200
Ala Gly Ala Ser Val Ser Ala Ile Glu Ser Thr Ala Trp Tyr Gly Ile
                1205                1210                1215
Thr Ala Arg Gly Ser Lys Val Met Pro Gly Asp Tyr Gln Tyr Val Val
            1220                1225                1230
Thr Tyr Arg Asp Glu His Gly Lys Glu His Gln Lys Gln Tyr Thr Ile
        1235                1240                1245
Ser Val Asn Asp Lys Lys Pro Met Ile Thr Gln Gly Arg Phe Asp Thr
    1250                1255                1260
Ile Asn Gly Val Asp His Phe Thr Pro Asp Lys Thr Lys Ala Leu Asp
1265                1270                1275                1280
Ser Ser Gly Ile Val Arg Glu Glu Val Phe Tyr Leu Ala Lys Lys Asn
                1285                1290                1295
Gly Arg Lys Phe Asp Val Thr Glu Gly Lys Asp Gly Ile Thr Val Ser
            1300                1305                1310
Asp Asn Lys Val Tyr Ile Pro Lys Asn Pro Asp Gly Ser Tyr Thr Ile
        1315                1320                1325
Ser Lys Arg Asp Gly Val Thr Leu Ser Asp Tyr Tyr Tyr Leu Val Glu
    1330                1335                1340
Asp Arg Ala Gly Asn Val Ser Phe Ala Thr Leu Arg Asp Leu Lys Ala
1345                1350                1355                1360
Val Gly Lys Asp Lys Ala Val Val Asn Phe Gly Leu Asp Leu Pro Val
                1365                1370                1375
Pro Glu Asp Lys Gln Ile Val Asn Phe Thr Tyr Leu Val Arg Asp Ala
            1380                1385                1390
Asp Gly Lys Pro Ile Glu Asn Leu Glu Tyr Tyr Asn Asn Ser Gly Asn
        1395                1400                1405
Ser Leu Ile Leu Pro Tyr Gly Lys Tyr Thr Val Glu Leu Leu Thr Tyr
    1410                1415                1420
Asp Thr Asn Ala Ala Lys Leu Glu Ser Asp Lys Ile Val Ser Phe Thr
1425                1430                1435                1440
Leu Ser Ala Asp Asn Asn Phe Gln Gln Val Thr Phe Lys Ile Thr Met
                1445                1450                1455
Leu Ala Thr Ser Gln Ile Thr Ala His Phe Asp His Leu Leu Pro Glu
            1460                1465                1470
Gly Ser Arg Val Ser Leu Lys Thr Ala Gln Asp Gln Leu Ile Pro Leu
        1475                1480                1485
Glu Gln Ser Leu Tyr Val Pro Lys Ala Tyr Gly Lys Thr Val Gln Glu
    1490                1495                1500
Gly Thr Tyr Glu Val Val Val Ser Leu Pro Lys Gly Tyr Arg Ile Glu
1505                1510                1515                1520
Gly Asn Thr Lys Val Asn Thr Leu Pro Asn Glu Val His Glu Leu Ser
                1525                1530                1535
Leu Arg Leu Val Lys Val Gly Asp Ala Ser Asp Ser Thr Gly Asp His
```

1540 1545 1550
Lys Val Met Ser Lys Asn Asn Ser Gln Ala Leu Thr Ala Ser Ala Thr
1555 1560 1565
Pro Thr Lys Ser Thr Thr Ser Ala Thr Ala Lys Ala
1570 1575 1580

<210> 149
<211> 1580
<212> PRT
<213> Streptococcus pyogenes

<400> 149

```
Ala Asp Glu Leu Ser Thr Met Ser Glu Pro Thr Ile Thr Asn His Ala
1               5                   10                  15
Gln Gln Gln Ala Gln His Leu Thr Asn Thr Glu Leu Ser Ser Ala Glu
            20                  25                  30
Ser Lys Ser Gln Asp Thr Ser Gln Ile Thr Leu Lys Thr Asn Arg Glu
        35                  40                  45
Lys Glu Gln Ser Gln Asp Leu Val Ser Glu Pro Thr Thr Thr Glu Leu
    50                  55                  60
Ala Asp Thr Asp Ala Ala Ser Met Ala Asn Thr Gly Ser Asp Ala Thr
65                  70                  75                  80
Gln Lys Ser Ala Ser Leu Pro Pro Val Asn Thr Asp Val His Asp Trp
                85                  90                  95
Val Lys Thr Lys Gly Ala Trp Asp Lys Gly Tyr Lys Gly Gln Gly Lys
            100                 105                 110
Val Val Ala Val Ile Ala Thr Gly Ile Asp Pro Ala His Gln Ser Met
        115                 120                 125
Arg Ile Ser Asp Val Ser Thr Ala Lys Val Lys Ser Lys Glu Asp Met
    130                 135                 140
Leu Ala Arg Gln Lys Ala Ala Gly Ile Asn Tyr Gly Ser Trp Ile Asn
145                 150                 155                 160
Asp Lys Val Val Phe Ala His Asn Tyr Val Glu Asn Ser Asp Asn Ile
                165                 170                 175
Lys Glu Asn Gln Phe Glu Asp Phe Asp Glu Asp Trp Glu Asn Phe Glu
            180                 185                 190
Phe Asp Ala Glu Ala Glu Pro Lys Ala Ile Lys Lys His Lys Ile Tyr
        195                 200                 205
Arg Pro Gln Ser Thr Gln Ala Pro Lys Glu Thr Val Ile Lys Thr Glu
    210                 215                 220
Glu Thr Asp Gly Ser His Asp Ile Asp Trp Thr Gln Thr Asp Asp Asp
225                 230                 235                 240
Thr Lys Tyr Glu Ser His Gly Met His Val Thr Gly Ile Val Ala Gly
                245                 250                 255
Asn Ser Lys Glu Ala Ala Ala Thr Gly Glu Arg Phe Leu Gly Ile Ala
            260                 265                 270
Pro Glu Ala Gln Val Met Phe Met Arg Val Phe Ala Asn Asp Ile Met
        275                 280                 285
Gly Ser Ala Glu Ser Leu Phe Ile Lys Ala Ile Glu Asp Ala Val Ala
    290                 295                 300
Leu Gly Ala Asp Val Ile Asn Leu Ser Leu Gly Thr Ala Asn Gly Ala
305                 310                 315                 320
Gln Leu Ser Gly Ser Lys Pro Leu Met Glu Ala Ile Glu Lys Ala Lys
                325                 330                 335
Lys Ala Gly Val Ser Val Val Val Ala Ala Gly Asn Glu Arg Val Tyr
            340                 345                 350
Gly Ser Asp His Asp Asp Pro Leu Ala Thr Asn Pro Asp Tyr Gly Leu
        355                 360                 365
Val Gly Ser Pro Ser Thr Gly Arg Thr Pro Thr Ser Val Ala Ala Ile
    370                 375                 380
Asn Ser Lys Trp Val Ile Gln Arg Leu Met Thr Val Lys Glu Leu Glu
```

```
385                 390                 395                 400
Asn Arg Ala Asp Leu Asn His Gly Lys Ala Ile Tyr Ser Glu Ser Val
                405                 410                 415
Asp Phe Lys Asp Ile Lys Asp Ser Leu Gly Tyr Asp Lys Ser His Gln
            420                 425                 430
Phe Ala Tyr Val Lys Glu Ser Thr Asp Ala Gly Tyr Asn Ala Gln Asp
        435                 440                 445
Val Lys Gly Lys Ile Ala Leu Ile Glu Arg Asp Pro Asn Lys Thr Tyr
    450                 455                 460
Asp Glu Met Ile Ala Leu Ala Lys Lys His Gly Ala Leu Gly Val Leu
465                 470                 475                 480
Ile Phe Asn Asn Lys Pro Gly Gln Ser Asn Arg Ser Met Arg Leu Thr
                485                 490                 495
Ala Asn Gly Met Gly Ile Pro Ser Ala Phe Ile Ser His Glu Phe Gly
            500                 505                 510
Lys Ala Met Ser Gln Leu Asn Gly Asn Gly Thr Gly Ser Leu Glu Phe
        515                 520                 525
Asp Ser Val Val Ser Lys Ala Pro Ser Gln Lys Gly Asn Glu Met Asn
    530                 535                 540
His Phe Ser Asn Trp Gly Leu Thr Ser Asp Gly Tyr Leu Lys Pro Asp
545                 550                 555                 560
Ile Thr Ala Pro Gly Gly Asp Ile Tyr Ser Thr Tyr Asn Asp Asn His
                565                 570                 575
Tyr Gly Ser Gln Thr Gly Thr Ala Met Ala Ser Pro Gln Ile Ala Gly
            580                 585                 590
Ala Ser Leu Leu Val Lys Gln Tyr Leu Glu Lys Thr Gln Pro Asn Leu
        595                 600                 605
Pro Lys Glu Lys Ile Ala Asp Ile Val Lys Asn Leu Leu Met Ser Asn
    610                 615                 620
Ala Gln Ile His Val Asn Pro Glu Thr Lys Thr Thr Thr Ser Pro Arg
625                 630                 635                 640
Gln Gln Gly Ala Gly Leu Leu Asn Ile Asp Gly Ala Val Thr Ser Gly
                645                 650                 655
Leu Tyr Val Thr Gly Lys Asp Asn Tyr Gly Ser Ile Ser Leu Gly Asn
            660                 665                 670
Ile Thr Asp Thr Met Thr Phe Asp Val Thr Val His Asn Leu Ser Asn
        675                 680                 685
Lys Asp Lys Thr Leu Arg Tyr Asp Thr Glu Leu Leu Thr Asp His Val
    690                 695                 700
Asp Pro Gln Lys Gly Arg Phe Thr Leu Thr Ser His Ser Leu Lys Thr
705                 710                 715                 720
Tyr Gln Gly Gly Glu Val Thr Val Pro Ala Asn Gly Lys Val Thr Val
                725                 730                 735
Arg Val Thr Met Asp Val Ser Gln Phe Thr Lys Glu Leu Thr Lys Gln
            740                 745                 750
Met Pro Asn Gly Tyr Tyr Leu Glu Gly Phe Val Arg Phe Arg Asp Ser
        755                 760                 765
Gln Asp Asp Gln Leu Asn Arg Val Asn Ile Pro Phe Val Gly Phe Lys
    770                 775                 780
Gly Gln Phe Glu Asn Leu Ala Val Ala Glu Glu Ser Ile Tyr Arg Leu
785                 790                 795                 800
Lys Ser Gln Gly Lys Thr Gly Phe Tyr Phe Asp Glu Ser Gly Pro Lys
                805                 810                 815
Asp Asp Ile Tyr Val Gly Lys His Phe Thr Gly Leu Val Thr Leu Gly
            820                 825                 830
Ser Glu Thr Asn Val Ser Thr Lys Thr Ile Ser Asp Asn Gly Leu His
        835                 840                 845
Thr Leu Gly Thr Phe Lys Asn Ala Asp Gly Lys Phe Ile Leu Glu Lys
    850                 855                 860
Asn Ala Gln Gly Asn Pro Val Leu Ala Ile Ser Pro Asn Gly Asp Asn
865                 870                 875                 880
```

```
Asn Gln Asp Phe Ala Ala Phe Lys Gly Val Phe Leu Arg Lys Tyr Gln
                885                 890                 895
Gly Leu Lys Ala Ser Val Tyr His Ala Ser Asp Lys Glu His Lys Asn
            900                 905                 910
Pro Leu Trp Val Ser Pro Glu Ser Phe Lys Gly Asp Lys Asn Phe Asn
        915                 920                 925
Ser Asp Ile Arg Phe Ala Lys Ser Thr Thr Leu Leu Gly Thr Ala Phe
    930                 935                 940
Ser Gly Lys Ser Leu Thr Gly Ala Glu Leu Pro Asp Gly His Tyr His
945                 950                 955                 960
Tyr Val Val Ser Tyr Tyr Pro Asp Val Val Gly Ala Lys Arg Gln Glu
                965                 970                 975
Met Thr Phe Asp Met Ile Leu Asp Arg Gln Lys Pro Val Leu Ser Gln
            980                 985                 990
Ala Thr Phe Asp Pro Glu Thr Asn Arg Phe Lys Pro Glu Pro Leu Lys
        995                 1000                1005
Asp Arg Gly Leu Ala Gly Val Arg Lys Asp Ser Val Phe Tyr Leu Glu
    1010                1015                1020
Arg Lys Asp Asn Lys Pro Tyr Thr Val Thr Ile Asn Asp Ser Tyr Lys
1025                1030                1035                1040
Tyr Val Ser Val Glu Asp Asn Lys Thr Phe Val Glu Arg Gln Ala Asp
                1045                1050                1055
Gly Ser Phe Ile Leu Pro Leu Asp Lys Ala Lys Leu Gly Asp Phe Tyr
            1060                1065                1070
Tyr Met Val Glu Asp Phe Ala Gly Asn Val Ala Ile Ala Lys Leu Gly
        1075                1080                1085
Asp His Leu Pro Gln Thr Leu Gly Lys Thr Pro Ile Lys Leu Lys Leu
    1090                1095                1100
Thr Asp Gly Asn Tyr Gln Thr Lys Glu Thr Leu Lys Asp Asn Leu Glu
1105                1110                1115                1120
Met Thr Gln Ser Asp Thr Gly Leu Val Thr Asn Gln Ala Gln Leu Ala
                1125                1130                1135
Val Val His Arg Asn Gln Pro Gln Ser Gln Leu Thr Lys Met Asn Gln
            1140                1145                1150
Asp Phe Phe Ile Ser Pro Asn Glu Asp Gly Asn Lys Asp Phe Val Ala
        1155                1160                1165
Phe Lys Gly Leu Lys Asn Asn Val Tyr Asn Asp Leu Thr Val Asn Val
    1170                1175                1180
Tyr Ala Lys Asp Asp His Gln Lys Gln Thr Pro Ile Trp Ser Ser Gln
1185                1190                1195                1200
Ala Gly Ala Ser Val Ser Ala Ile Glu Ser Thr Ala Trp Tyr Gly Ile
                1205                1210                1215
Thr Ala Arg Gly Ser Lys Val Met Pro Gly Asp Tyr Gln Tyr Val Val
            1220                1225                1230
Thr Tyr Arg Asp Glu His Gly Lys Glu His Gln Lys Gln Tyr Thr Ile
        1235                1240                1245
Ser Val Asn Asp Lys Lys Pro Met Ile Thr Gln Gly Arg Phe Asp Thr
    1250                1255                1260
Ile Asn Gly Val Asp His Phe Thr Pro Asp Lys Thr Lys Ala Leu Asp
1265                1270                1275                1280
Ser Ser Gly Ile Val Arg Glu Glu Val Phe Tyr Leu Ala Lys Lys Asn
                1285                1290                1295
Gly Arg Lys Phe Asp Val Thr Glu Gly Lys Asp Gly Ile Thr Val Ser
            1300                1305                1310
Asp Asn Lys Val Tyr Ile Pro Lys Asn Pro Asp Gly Ser Tyr Thr Ile
        1315                1320                1325
Ser Lys Arg Asp Gly Val Thr Leu Ser Asp Tyr Tyr Tyr Leu Val Glu
    1330                1335                1340
Asp Arg Ala Gly Asn Val Ser Phe Ala Thr Leu Arg Asp Leu Lys Ala
1345                1350                1355                1360
Val Gly Lys Asp Lys Ala Val Val Asn Phe Gly Leu Asp Leu Pro Val
```

```
                1365                1370                1375
Pro Glu Asp Lys Gln Ile Val Asn Phe Thr Tyr Leu Val Arg Asp Ala
            1380                1385                1390
Asp Gly Lys Pro Ile Glu Asn Leu Glu Tyr Tyr Asn Asn Ser Gly Asn
        1395                1400                1405
Ser Leu Ile Leu Pro Tyr Gly Lys Tyr Thr Val Glu Leu Leu Thr Tyr
    1410                1415                1420
Asp Thr Asn Ala Ala Lys Leu Glu Ser Asp Lys Ile Val Ser Phe Thr
1425                1430                1435                1440
Leu Ser Ala Asp Asn Asn Phe Gln Gln Val Thr Phe Lys Ile Thr Met
                1445                1450                1455
Leu Ala Thr Ser Gln Ile Thr Ala His Phe Asp His Leu Leu Pro Glu
            1460                1465                1470
Gly Ser Arg Val Ser Leu Lys Thr Ala Gln Asp Gln Leu Ile Pro Leu
        1475                1480                1485
Glu Gln Ser Leu Tyr Val Pro Lys Ala Tyr Gly Lys Thr Val Gln Glu
    1490                1495                1500
Gly Thr Tyr Glu Val Val Val Ser Leu Pro Lys Gly Tyr Arg Ile Glu
1505                1510                1515                1520
Gly Asn Thr Lys Val Asn Thr Leu Pro Asn Glu Val His Glu Leu Ser
                1525                1530                1535
Leu Arg Leu Val Lys Val Gly Asp Ala Ser Asp Ser Thr Gly Asp His
            1540                1545                1550
Lys Val Met Ser Lys Asn Asn Ser Gln Ala Leu Thr Ala Ser Ala Thr
        1555                1560                1565
Pro Thr Lys Ser Thr Thr Ser Ala Thr Ala Lys Ala
    1570                1575                1580
```

<210> 150
<211> 28
<212> DNA
<213> Streptococcus pyogenes

<400> 150
gtgcgtcata tggcagatga gctaagca 28

<210> 151
<211> 21
<212> DNA
<213> Streptococcus pyogenes

<400> 151
ccctgtggca ataactgcga c 21

<210> 152
<211> 21
<212> DNA
<213> Streptococcus pyogenes

<400> 152
cgcagttatt gccacaggga t 21

<210> 153
<211> 30
<212> DNA
<213> Streptococcus pyogenes

<400> 153

ctgactgagt cgacagactc tgaatagatg 30

<210> 154
<211> 34
<212> DNA
<213> Streptococcus pyogenes

<400> 154
ctgactgagt cgactttaaa gacataaaag atag 34

<210> 155
<211> 21
<212> DNA
<213> Streptococcus pyogenes

<400> 155
gagaggccat agctgttcct g 21

<210> 156
<211> 21
<212> DNA
<213> Streptococcus pyogenes

<400> 156
ggaacagcta tggcctctcc t 21

<210> 157
<211> 35
<212> DNA
<213> Streptococcus pyogenes

<400> 157
gtgcgtgcta gcgaatcgaa caaacaaaac actgc 35

<210> 158
<211> 42
<212> DNA
<213> Streptococcus pyogenes

<400> 158
gcattcgatc ctcgagctac ttataagtaa tcgaaccata tg 42

<210> 159
<211> 45
<212> DNA
<213> Streptococcus pyogenes

<400> 159
gctaccttca gtagaaaaaa cctagcttat cctatttcat acacc 45

.<210> 160

<211> 45
<212> DNA
<213> Streptococcus pyogenes

<400> 160
ggtgtatgaa ataggataag ctaggttttt tctactgaag gtagc 45

<210> 161
<211> 41
<212> DNA
<213> Streptococcus pyogenes

<400> 161
gcattcgatc ctcgagctta taagtaatcg aaccatatgg g 41

<210> 162
<211> 42
<212> DNA
<213> Streptococcus pyogenes

<400> 162
gagtgcactg gcttagcttt cgaatggtgg cgaaaagtga tc 42

<210> 163
<211> 42
<212> DNA
<213> Streptococcus pyogenes

<400> 163
gatcactttt cgccaccatt cgaaagctaa gccagtgcac tc 42

<210> 164
<211> 55
<212> DNA
<213> Streptococcus pyogenes

<400> 164
gttgctcaat atgaaatcct ttggaatgaa atcaattatg atgacaaagg aaaag 55

<210> 165
<211> 55
<212> DNA
<213> Streptococcus pyogenes

<400> 165
cttttccttt gtcatcataa ttgatttcat tccaaaggat ttcatattga gcaac 55

<210> 166
<211> 42
<212> DNA
<213> Streptococcus pyogenes

<400> 166
ccgtatcatg gctagagagg gcactggctt agcttgggaa tg 42

<210> 167
<211> 42
<212> DNA
<213> Streptococcus pyogenes

<400> 167
cattcccaag ctaagccagt gccctctcta gccatgatac gg 42

<210> 168
<211> 30

<212> DNA
<213> Streptococcus pyogenes

<400> 168
gtgcgtgcta gcgaatcgaa caaacaaaac 30

<210> 169
<211> 34
<212> DNA
<213> Streptococcus pyogenes

<400> 169
gcgtctcgag tcacttataa gtaatcgaac cata 34

<210> 170
<211> 42
<212> DNA
<213> Streptococcus pyogenes

<400> 170
ccgtatcatg gctagagagg gcactggctt agctttcgaa tg 42

<210> 171
<211> 42
<212> DNA
<213> Streptococcus pyogenes

<400> 171
cattcgaaag ctaagccagt gccctctcta gccatgatac gg 42

<210> 172
<211> 45
<212> DNA
<213> Streptococcus pyogenes

<400> 172
ctggtggtaa tacgcttcct agaacacaat atactgaatc aatgg 45

<210> 173
<211> 45
<212> DNA
<213> Streptococcus pyogenes

<400> 173
ccattgattc agtatattgt gttctaggaa gcgtattacc accag 45

<210> 174
<211> 26
<212> PRT
<213> Streptococcus pyogenes

<400> 174

```
Gln Asn Thr Ala Ser Thr Glu Thr Thr Thr Thr Asn Glu Gln Pro Lys
 1               5                  10                  15
Pro Glu Ser Ser Glu Leu Thr Thr Glu Lys
            20                  25
```

<210> 175
<211> 18
<212> PRT
<213> Streptococcus pyogenes

<400> 175

Asn Ile Asn Thr Thr Pro Val Asp Ile Ser Ile Ile Asp Ser Val Thr
1               5                   10                  15
Asp Arg

<210> 176
<211> 11
<212> PRT
<213> Streptococcus pyogenes

<400> 176

Thr Glu Tyr Val Glu Thr Thr Ser Thr Glu Tyr
1               5                   10

<210> 177
<211> 824
<212> PRT
<213> S. pyogenes

<400> 177

```
Met Ser Val Gly Val Ser His Gln Val Lys Ala Asp Asp Arg Ala Ser
1               5                   10                  15
Gly Glu Thr Lys Ala Ser Asn Thr His Asp Asp Ser Leu Pro Lys Pro
            20                  25                  30
Glu Thr Ile Gln Glu Ala Lys Ala Thr Ile Asp Ala Val Glu Lys Thr
        35                  40                  45
Leu Ser Gln Gln Lys Ala Glu Leu Thr Glu Leu Ala Thr Ala Leu Thr
    50                  55                  60
Lys Thr Thr Ala Glu Ile Asn His Leu Lys Glu Gln Gln Asp Asn Glu
65                  70                  75                  80
Gln Lys Ala Leu Thr Ser Ala Gln Glu Ile Tyr Thr Asn Thr Leu Ala
                85                  90                  95
Ser Ser Glu Glu Thr Leu Leu Ala Gln Gly Ala Glu His Gln Arg Glu
            100                 105                 110
Leu Thr Ala Thr Glu Thr Glu Leu His Asn Ala Gln Ala Asp Gln His
        115                 120                 125
Ser Lys Glu Thr Ala Leu Ser Glu Gln Lys Ala Ser Ile Ser Ala Glu
    130                 135                 140
Thr Thr Arg Ala Gln Asp Leu Val Glu Gln Val Lys Thr Ser Glu Gln
145                 150                 155                 160
Asn Ile Ala Lys Leu Asn Ala Met Ile Ser Asn Pro Asp Ala Ile Thr
                165                 170                 175
Lys Ala Ala Gln Thr Ala Asn Asp Asn Thr Lys Ala Leu Ser Ser Glu
            180                 185                 190
Leu Glu Lys Ala Lys Ala Asp Leu Glu Asn Gln Lys Ala Lys Val Lys
        195                 200                 205
Lys Gln Leu Thr Glu Glu Leu Ala Ala Gln Lys Ala Ala Leu Ala Glu
    210                 215                 220
Lys Glu Ala Glu Leu Ser Arg Leu Lys Ser Ser Ala Pro Ser Thr Gln
225                 230                 235                 240
Asp Ser Ile Val Gly Asn Asn Thr Met Lys Ala Pro Gln Gly Tyr Pro
                245                 250                 255
Leu Glu Glu Leu Lys Lys Leu Glu Ala Ser Gly Tyr Ile Gly Ser Ala
            260                 265                 270
Ser Tyr Asn Asn Tyr Tyr Lys Glu His Ala Asp Gln Ile Ile Ala Lys
        275                 280                 285
Ala Ser Pro Gly Asn Gln Leu Asn Gln Tyr Gln Asp Ile Pro Ala Asp
    290                 295                 300
Arg Asn Arg Phe Val Asp Pro Asp Asn Leu Thr Pro Glu Val Gln Asn
305                 310                 315                 320
Glu Leu Ala Gln Phe Ala Ala His Met Ile Asn Ser Val Arg Arg Gln
                325                 330                 335
Leu Gly Leu Pro Pro Val Thr Val Thr Ala Gly Ser Gln Glu Phe Ala
            340                 345                 350
Arg Leu Leu Ser Thr Ser Tyr Lys Lys Thr His Gly Asn Thr Arg Pro
        355                 360                 365
Ser Phe Val Tyr Gly Gln Pro Gly Val Ser Gly His Tyr Gly Val Gly
    370                 375                 380
```

Pro His Asp Lys Thr Ile Ile Glu Asp Ser Ala Gly Ala Ser Gly Leu
385 390 395 400
Ile Arg Asn Asp Asp Asn Met Tyr Glu Asn Ile Gly Ala Phe Asn Asp
405 410 415
Val His Thr Val Asn Gly Ile Lys Arg Gly Ile Tyr Asp Ser Ile Lys
420 425 430
Tyr Met Leu Phe Thr Asp His Leu His Gly Asn Thr Tyr Gly His Ala
435 440 445
Ile Asn Phe Leu Arg Val Asp Lys His Asn Pro Asn Ala Pro Val Tyr
450 455 460
Leu Gly Phe Ser Thr Ser Asn Val Gly Ser Leu Asn Glu His Phe Val
465 470 475 480
Met Phe Pro Glu Ser Asn Ile Ala Asn His Gln Arg Phe Asn Lys Thr
485 490 495
Pro Ile Lys Ala Val Gly Ser Thr Lys Asp Tyr Ala Gln Arg Val Gly
500 505 510
Thr Val Ser Asp Thr Ile Ala Ala Ile Lys Gly Lys Val Ser Ser Leu
515 520 525
Glu Asn Arg Leu Ser Ala Ile His Gln Glu Ala Asp Ile Met Ala Ala
530 535 540
Gln Ala Lys Val Ser Gln Leu Gln Gly Lys Leu Ala Ser Thr Leu Lys
545 550 555 560
Gln Ser Asp Ser Leu Asn Leu Gln Val Arg Gln Leu Asn Asp Thr Lys
565 570 575
Gly Ser Leu Arg Thr Glu Leu Leu Ala Ala Lys Ala Lys Gln Ala Gln
580 585 590
Leu Glu Ala Thr Arg Asp Gln Ser Leu Ala Lys Leu Ala Ser Leu Lys
595 600 605
Ala Ala Leu His Gln Thr Glu Ala Leu Ala Glu Gln Ala Ala Ala Arg
610 615 620
Val Thr Ala Leu Val Ala Lys Lys Ala His Leu Gln Tyr Leu Arg Asp
625 630 635 640
Phe Lys Leu Asn Pro Asn Arg Leu Gln Val Ile Arg Glu Arg Ile Asp
645 650 655
Asn Thr Lys Gln Asp Leu Ala Lys Thr Thr Ser Ser Leu Leu Asn Ala
660 665 670
Gln Glu Ala Leu Ala Ala Leu Gln Ala Lys Gln Ser Ser Leu Glu Ala
675 680 685
Thr Ile Ala Thr Thr Glu His Gln Leu Thr Leu Leu Lys Thr Leu Ala
690 695 700
Asn Glu Lys Glu Tyr Arg His Leu Asp Glu Asp Ile Ala Thr Val Pro
705 710 715 720
Asp Leu Gln Val Ala Pro Pro Leu Thr Gly Val Lys Pro Leu Ser Tyr
725 730 735
Ser Lys Ile Asp Thr Thr Pro Leu Val Gln Glu Met Val Lys Glu Thr
740 745 750
Lys Gln Leu Leu Glu Ala Ser Ala Arg Leu Ala Ala Glu Asn Thr Ser
755 760 765
Leu Val Ala Glu Ala Leu Val Gly Gln Thr Ser Glu Met Val Ala Ser
770 775 780
Asn Ala Ile Val Ser Lys Ile Thr Ser Ser Ile Thr Gln Pro Ser Ser
785 790 795 800
Lys Thr Ser Tyr Gly Ser Gly Ser Ser Thr Thr Ser Asn Leu Ile Ser
805 810 815
Asp Val Asp Glu Ser Thr Gln Arg
820

<210> 178
<211> 398
<212> PRT

<213> S. pyogenes

<400> 178

```
Met Lys Lys Arg Ile Leu Ser Ala Val Leu Val Ser Gly Val Thr Leu
1               5                   10                  15
Gly Ala Ala Thr Thr Val Gly Ala Glu Asp Leu Ser Thr Lys Ile Ala
            20                  25                  30
Lys Gln Asp Ser Ile Ile Ser Asn Leu Thr Thr Glu Gln Lys Ala Ala
        35                  40                  45
Gln Asn Gln Val Ser Ala Leu Gln Ala Gln Val Ser Ser Leu Gln Ser
    50                  55                  60
Glu Gln Asp Lys Leu Thr Ala Arg Asn Thr Glu Leu Glu Ala Leu Ser
65                  70                  75                  80
Lys Arg Phe Glu Gln Glu Ile Lys Ala Leu Thr Ser Gln Ile Val Ala
                85                  90                  95
Arg Asn Glu Lys Leu Lys Asn Gln Ala Arg Ser Ala Tyr Lys Asn Asn
            100                 105                 110
Glu Thr Ser Gly Tyr Ile Asn Ala Leu Leu Asn Ser Lys Ser Ile Ser
        115                 120                 125
Asp Val Val Asn Arg Leu Val Ala Ile Asn Arg Ala Val Ser Ala Asn
    130                 135                 140
Ala Lys Leu Leu Glu Gln Gln Lys Ala Asp Lys Val Ser Leu Glu Glu
145                 150                 155                 160
Lys Gln Ala Ala Asn Gln Thr Ala Ile Asn Thr Ile Ala Ala Asn Met
                165                 170                 175
Ala Met Ala Glu Glu Asn Gln Asn Thr Leu Arg Thr Gln Gln Ala Asn
            180                 185                 190
Leu Val Ala Ala Thr Ala Asn Leu Ala Leu Gln Leu Ala Ser Ala Thr
        195                 200                 205
Glu Asp Lys Ala Asn Leu Val Ala Gln Lys Glu Ala Ala Glu Lys Ala
    210                 215                 220
Ala Ala Glu Ala Leu Ala Gln Glu Gln Ala Ala Lys Val Lys Ala Gln
225                 230                 235                 240
Glu Gln Ala Ala Gln Gln Ala Ala Ser Val Glu Ala Ala Lys Ser Ala
                245                 250                 255
Ile Thr Pro Ala Pro Gln Ala Thr Pro Ala Ala Gln Ser Ser Asn Ala
            260                 265                 270
Ile Glu Pro Ala Ala Leu Thr Ala Pro Ala Ala Pro Ser Ala Gly Pro
        275                 280                 285
Gln Thr Ser Tyr Asp Ser Ser Asn Thr Tyr Pro Val Gly Gln Cys Thr
    290                 295                 300
Trp Gly Ala Lys Ser Leu Ala Pro Trp Ala Gly Asn Asn Trp Gly Asn
305                 310                 315                 320
Gly Gly Gln Trp Ala Tyr Ser Ala Gln Ala Ala Gly Tyr Arg Thr Gly
                325                 330                 335
Ser Thr Pro Met Val Gly Ala Ile Ala Val Trp Asn Asp Gly Gly Tyr
            340                 345                 350
Gly His Val Ala Val Val Val Glu Val Gln Ser Ala Ser Ser Ile Arg
        355                 360                 365
Val Met Glu Ser Asn Tyr Ser Gly Arg Gln Tyr Ile Ala Asp His Arg
    370                 375                 380
Gly Trp Phe Asn Pro Thr Gly Val Thr Phe Ile Tyr Pro His
385                 390                 395
```

<210> 179
<211> 342
<212> PRT
<213> S. pyogenes

<400> 179

```
Met Ser Leu Gly Leu Val Ser Thr Ala Leu Phe Thr Leu Gly Gly Cys
1               5                   10                  15

Thr Asn Asn Ser Ala Lys Gln Thr Thr Asp Asn Ser Leu Lys Ile Ala
            20                  25                  30
Met Ile Thr Asn Gln Thr Gly Ile Asp Asp Lys Ser Phe Asn Gln Ser
        35                  40                  45
Ala Trp Glu Gly Leu Gln Ala Trp Gly Lys Glu Asn Lys Leu Glu Lys
    50                  55                  60
Gly Lys Gly Tyr Asp Tyr Phe Gln Ser Ala Asn Glu Ser Glu Phe Thr
65                  70                  75                  80
Thr Asn Leu Glu Ser Ala Val Thr Asn Gly Tyr Asn Leu Val Phe Gly
                85                  90                  95
Ile Gly Phe Pro Leu His Asp Ala Val Glu Lys Val Ala Ala Asn Asn
            100                 105                 110
Pro Asp Asn His Phe Ala Ile Val Asp Asp Val Ile Lys Gly Gln Lys
        115                 120                 125
Asn Val Ala Ser Ile Thr Phe Ser Asp His Glu Ala Ala Tyr Leu Ala
    130                 135                 140
Gly Val Ala Ala Ala Lys Thr Thr Lys Thr Lys Gln Val Gly Phe Val
145                 150                 155                 160
Gly Gly Met Glu Gly Asp Val Val Lys Arg Phe Glu Lys Gly Phe Glu
                165                 170                 175
Ala Gly Val Lys Ser Val Asp Asp Thr Ile Lys Val Arg Val Ala Tyr
            180                 185                 190
Ala Gly Ser Phe Ala Asp Ala Ala Lys Gly Lys Thr Ile Ala Ala Ala
        195                 200                 205
Gln Tyr Ala Glu Gly Ala Asp Val Ile Tyr His Ala Ala Gly Gly Thr
    210                 215                 220
Gly Ala Gly Val Phe Ser Glu Ala Lys Ser Ile Asn Glu Lys Arg Lys
225                 230                 235                 240
Glu Glu Asp Lys Val Trp Val Ile Gly Val Asp Arg Asp Gln Ser Glu
                245                 250                 255
Asp Gly Lys Tyr Thr Thr Lys Asp Gly Lys Ser Ala Asn Phe Val Leu
            260                 265                 270
Thr Ser Ser Ile Lys Glu Val Gly Lys Ala Leu Val Lys Val Ala Val
        275                 280                 285
Lys Thr Ser Glu Asp Gln Phe Pro Gly Gly Gln Ile Thr Thr Phe Gly
    290                 295                 300
Leu Lys Glu Gly Gly Val Ser Leu Thr Thr Asp Ala Leu Thr Gln Asp
305                 310                 315                 320
Thr Lys Lys Ala Ile Glu Ala Ala Lys Lys Ala Ile Ile Glu Gly Thr
                325                 330                 335
Ile Thr Val Pro Glu Asn
            340
```

<210> 180
<211> 310
<212> PRT
<213> S. pyogenes

<400> 180

```
Met Lys Lys Leu Thr Leu Leu Leu Thr Leu Cys Leu Thr Thr Ile Thr
1               5                   10                  15
Leu Ile Ala Cys Gly Asn Gln Ala Thr Asn His Ser Asn Thr Ala Ser
            20                  25                  30
Lys Ser Leu Ser Pro Met Pro Gln Ile Ala Gly Val Thr Tyr Tyr Gly
        35                  40                  45
Asp Ile Pro Lys Gln Pro Lys Arg Val Val Ser Leu Ala Ser Thr Tyr
    50                  55                  60
Thr Gly Tyr Leu Lys Lys Leu Asp Met Asn Leu Val Gly Val Thr Ser
65                  70                  75                  80
Tyr Asp Lys Lys Asn Pro Ile Leu Ala Lys Thr Val Lys Lys Ala Lys
                85                  90                  95

Gln Val Ala Ala Thr Asp Leu Glu Ala Val Thr Thr Leu Lys Pro Asp
            100                 105                 110
Leu Ile Val Val Gly Ser Thr Glu Glu Asn Ile Lys Gln Leu Ala Glu
        115                 120                 125
Ile Ala Pro Val Ile Ser Ile Glu Tyr Arg Lys Arg Asp Tyr Leu Gln
    130                 135                 140
Val Leu Ser Asp Phe Gly Arg Ile Phe Asn Lys Glu Asp Lys Ala Lys
145                 150                 155                 160
Lys Trp Leu Lys Asp Trp Lys Thr Lys Thr Ala Ala Tyr Glu Lys Glu
                165                 170                 175
Val Lys Ala Val Thr Gly Asp Lys Ala Thr Phe Thr Ile Met Gly Leu
            180                 185                 190
Tyr Glu Lys Asp Val Tyr Leu Phe Gly Lys Asp Trp Gly Arg Gly Gly
        195                 200                 205
Glu Ile Ile His Gln Ala Phe His Tyr Asp Ala Pro Glu Lys Val Lys
    210                 215                 220
Thr Glu Val Phe Lys Gln Gly Tyr Leu Ser Leu Ser Gln Glu Val Leu
225                 230                 235                 240
Pro Asp Tyr Ile Gly Asp Tyr Val Val Val Ala Ala Glu Asp Asp Lys
                245                 250                 255
Thr Gly Ser Ala Leu Tyr Glu Ser Lys Leu Trp Gln Ser Ile Pro Ala
            260                 265                 270
Val Lys Lys His His Val Ile Lys Val Asn Ala Asn Val Phe Tyr Phe
        275                 280                 285
Thr Asp Pro Leu Ser Leu Glu Tyr Gln Leu Glu Thr Leu Arg Glu Ala
    290                 295                 300
Ile Leu Ser Ser Glu Asn
305                 310
```

<210> 181
<211> 515
<212> PRT
<213> S. pyogenes

<400> 181

```
Met Lys Lys Lys Ile Leu Leu Met Met Ser Leu Ile Ser Val Phe Phe
 1               5                  10                  15
Ala Trp Gln Leu Thr Gln Ala Lys Gln Val Leu Ala Glu Gly Lys Val
            20                  25                  30
Lys Val Val Thr Thr Phe Tyr Pro Val Tyr Glu Phe Thr Lys Gly Val
        35                  40                  45
Ile Gly Asn Asp Gly Asp Val Phe Met Leu Met Lys Ala Gly Thr Glu
    50                  55                  60
Pro His Asp Phe Glu Pro Ser Thr Lys Asp Ile Lys Lys Ile Gln Asp
65                  70                  75                  80
Ala Asp Ala Phe Val Tyr Met Asp Asp Asn Met Glu Thr Trp Val Ser
                85                  90                  95
Asp Val Lys Lys Ser Leu Thr Ser Lys Lys Val Thr Ile Val Lys Gly
            100                 105                 110
Thr Gly Asn Met Leu Leu Val Ala Gly Ala Gly His Asp His Pro His
        115                 120                 125
Glu Asp Ala Asp Lys Lys His Glu His Asn Lys His Ser Glu Glu Gly
    130                 135                 140
His Asn His Ala Phe Asp Pro His Val Trp Leu Ser Pro Tyr Arg Ser
145                 150                 155                 160
Ile Thr Val Val Glu Asn Ile Arg Asp Ser Leu Ser Lys Ala Tyr Pro
                165                 170                 175
Glu Lys Ala Glu Asn Phe Lys Ala Asn Ala Ala Thr Tyr Ile Glu Lys
            180                 185                 190
Leu Lys Glu Leu Asp Lys Asp Tyr Thr Ala Ala Leu Ser Asp Ala Lys
        195                 200                 205
```

```
Gln Lys Ser Phe Val Thr Gln His Ala Ala Phe Gly Tyr Met Ala Leu
    210                 215                 220
Asp Tyr Gly Leu Asn Gln Ile Ser Ile Asn Gly Val Thr Pro Asp Ala
225                 230                 235                 240
Glu Pro Ser Ala Lys Arg Ile Ala Thr Leu Ser Lys Tyr Val Lys Lys
                245                 250                 255
Tyr Gly Ile Lys Tyr Ile Tyr Phe Glu Glu Asn Ala Ser Ser Lys Val
            260                 265                 270
Ala Lys Thr Leu Ala Lys Glu Ala Gly Val Lys Ala Ala Val Leu Ser
        275                 280                 285
Pro Leu Glu Gly Leu Thr Glu Lys Glu Met Lys Ala Gly Gln Asp Tyr
    290                 295                 300
Phe Thr Val Met Arg Lys Asn Leu Glu Thr Leu Arg Leu Thr Thr Asp
305                 310                 315                 320
Val Ala Gly Lys Glu Ile Leu Pro Glu Lys Asp Thr Thr Lys Thr Val
                325                 330                 335
Tyr Asn Gly Tyr Phe Lys Asp Lys Glu Val Lys Asp Arg Gln Leu Ser
            340                 345                 350
Asp Trp Ser Gly Ser Trp Gln Ser Val Tyr Pro Tyr Leu Gln Asp Gly
        355                 360                 365
Thr Leu Asp Gln Val Trp Asp Tyr Lys Ala Lys Lys Ser Lys Gly Lys
    370                 375                 380
Met Thr Ala Ala Glu Tyr Lys Asp Tyr Tyr Thr Thr Gly Tyr Lys Thr
385                 390                 395                 400
Asp Val Glu Gln Ile Lys Ile Asn Gly Lys Lys Lys Thr Met Thr Phe
                405                 410                 415
Val Arg Asn Gly Glu Lys Lys Thr Phe Thr Tyr Thr Tyr Ala Gly Lys
            420                 425                 430
Glu Ile Leu Thr Tyr Pro Lys Gly Asn Arg Gly Val Arg Phe Met Phe
        435                 440                 445
Glu Ala Lys Glu Ala Asp Ala Gly Glu Phe Lys Tyr Val Gln Phe Ser
    450                 455                 460
Asp His Ala Ile Ala Pro Glu Lys Ala Lys His Phe His Leu Tyr Trp
465                 470                 475                 480
Gly Gly Asp Ser Gln Glu Lys Leu His Lys Glu Leu Glu His Trp Pro
                485                 490                 495
Thr Tyr Tyr Gly Ser Asp Leu Ser Gly Arg Glu Ile Ala Gln Glu Ile
            500                 505                 510
Asn Ala His
        515
```

<210> 182
<211> 2045
<212> PRT
<213> S. pyogenes

<400> 182

```
Met Arg Lys Val Lys Lys Val Phe Val Ser Ser Cys Met Leu Leu Thr
 1               5                  10                  15
Val Gly Leu Gly Val Ala Val Pro Thr Gly Phe Ser Gln Ser Asn Gly
            20                  25                  30
Val Met Val Val Lys Ala Ala Glu Val Pro Ala Thr Asp Leu Ser Arg
        35                  40                  45
Gln Ala Ser Asp Ser Glu Arg Val Asp Glu Ser Ser Leu Leu Gln Lys
    50                  55                  60
Glu Asn Leu Ser Val Asp Ser Phe Lys Leu Glu Asn Leu Asn Gly Trp
65                  70                  75                  80
Glu Ala Glu Asn Asp Thr Ala Gly Asn Leu Gly Lys Phe Lys Asp Pro
                85                  90                  95
Asp Ser Ser Gly Tyr Gln Asn Ile Leu Thr Ser Ser Gly Lys Asn Ile
            100                 105                 110
```

```
Ser Val Ala Val Ala Pro Lys Gly Ser Gly Lys Met Asn Ile Lys Val
        115                 120                 125
Thr Lys Arg Ser Asn Phe Gln Gly Gly Tyr Tyr Val Gly Gly Leu Arg
    130                 135                 140
Thr Gln Thr Pro Val Leu Lys Leu Asn Asp Val Tyr Arg Tyr Ser Phe
145                 150                 155                 160
Thr Thr Lys Lys Leu Ser Gly Asn Ser Ser Glu Phe Lys Thr Arg Val
                165                 170                 175
Lys Pro Val Glu Ser Asn Asn Lys Leu Gly Lys Glu Leu Val Ile Arg
            180                 185                 190
Val Asp Asn Lys Asn Val Ser Thr Lys His Asp Trp Leu Pro Asp Ile
        195                 200                 205
Ser Asp Gly Thr His Thr Val Asp Phe Thr Gly Leu Asp Lys Lys Leu
    210                 215                 220
Ser Val Ala Phe Arg Phe Ser Pro Arg Gln Thr Ser Asn Val Val Tyr
225                 230                 235                 240
Glu Phe Ser Asn Ile Asn Ile Lys Asn Ile Ser Pro Ala Ser Val Pro
                245                 250                 255
Ala Ile Pro Ser Lys Val Leu Glu Gly Thr Ser Val Leu Ser Gly Thr
            260                 265                 270
Ala Ile Ser Ser Gly Asp Thr Leu Glu Lys Arg Lys Ser Phe Asp Gly
        275                 280                 285
Asp Ile Leu Arg Val Tyr Lys Asp Ser Lys Ile Ile Ala Arg Thr Val
    290                 295                 300
Ile Lys Gly Asn Lys Trp Asp Val Lys Leu Ser Lys Pro Leu Ile Ala
305                 310                 315                 320
Gly Glu Lys Leu Asp Phe Glu Ile Leu His Pro Arg Ser Gln Asn Val
                325                 330                 335
Ser Lys Lys Ile Ser Lys Gln Val Glu Ala Lys Pro Phe Asp Pro Ala
            340                 345                 350
Ser Tyr Lys Glu Lys Val Ile Ala Lys Leu Lys Pro Val Tyr Glu Ala
        355                 360                 365
Thr Ser Glu Lys Ile Thr Asn Asp Ala Trp Leu Asp Glu Asn Ala Lys
    370                 375                 380
Asp Leu Gln Lys Gln Lys Leu Glu Glu Gln Tyr Ile Ser Gly Lys Val
385                 390                 395                 400
Ala Ile Ser Glu Ala Gly Thr Lys Gln Glu Ala Ile Asp Ala Ala Tyr
                405                 410                 415
Asn Lys Tyr Ser Ser Gln Thr Asp Pro Asp Ser Leu Pro Ser Gln Tyr
            420                 425                 430
Lys Gln Gly Asn Lys Glu Asn Glu Gln Glu Lys Gly Arg Gln Asp Leu
        435                 440                 445
Ile Gln Thr Arg Asp Leu Thr Leu Lys Ala Ile Gln Glu Asp Lys Trp
    450                 455                 460
Leu Thr Glu Gln Glu Lys Thr Ile Gln Lys Glu Glu Ala Leu Lys Ala
465                 470                 475                 480
Phe Glu Thr Gly Ile Glu Ser Val Asn Gln Thr Val Ser Leu Glu Gln
                485                 490                 495
Leu Lys Gln Arg Leu Ile Val Tyr Lys Ala Ser Glu Lys Asp Ser Glu
            500                 505                 510
Lys Lys Glu Tyr Pro Glu Ser Ile Pro Asn Gln His Ile Pro Gly Lys
        515                 520                 525
Glu Lys Glu Val Lys Ala Ala Lys Gln Glu Glu Leu Lys Lys Leu His
    530                 535                 540
Asp Thr Thr Leu Glu Lys Ile Asn Gln Asp Lys Trp Leu Thr Pro Asp
545                 550                 555                 560
Gln Gln Ala Glu Gln Leu Lys Gln Ala Glu Val Thr Phe Lys Lys Gly
                565                 570                 575
Gln Glu Ala Ile Lys Ser Ala Gln Thr Leu Thr Gln Leu Glu Thr Asp
            580                 585                 590
Leu Ala Asp Tyr Val Ser Glu Asn Glu Gly Lys Gly Asn Ser Ile Pro
```

```
        595                 600                 605
Asp Lys Tyr Lys Ser Gly Asn Lys Asp Asp Leu Val Asn Lys Ala Glu
    610                 615                 620
Val Lys Leu Lys Glu Ala His Glu Ala Thr Lys Gln Ala Ile Glu Lys
625                 630                 635                 640
Asp Pro Trp Leu Ser Pro Glu Gln Lys Lys Ala Gln Lys Glu Lys Ala
                645                 650                 655
Lys Ala Arg Leu Asp Glu Gly Leu Lys Ala Leu Lys Ala Ala Asp Ser
            660                 665                 670
Leu Glu Ile Leu Lys Val Thr Glu Glu Ala Phe Val Asp Lys Glu Lys
        675                 680                 685
Asn Pro Asp Ser Ile Pro Asn Gln His Lys Ala Gly Thr Ala Asp Gln
    690                 695                 700
Ala Arg Lys Gln Ala Leu Asp Ser Leu Asp Lys Glu Val Gln Lys Glu
705                 710                 715                 720
Leu Glu Ser Ile Asp Asn Asp Asn Thr Leu Thr Thr Asp Glu Lys Ala
                725                 730                 735
Ala Ala Lys Lys Lys Val Asn Asp Ala Tyr Asp Val Ala Lys Gln Thr
            740                 745                 750
Ala Met Glu Ala Asn Ser Tyr Glu Asp Leu Thr Thr Ile Lys Asp Glu
        755                 760                 765
Phe Leu Ser Asn Leu Pro His Lys Gln Gly Thr Pro Leu Lys Asp Gln
    770                 775                 780
Gln Ser Asp Ala Ile Ala Glu Leu Glu Lys Lys Gln Gln Glu Ile Glu
785                 790                 795                 800
Lys Ala Ile Glu Gly Asp Lys Thr Leu Pro Arg Asp Glu Lys Glu Lys
                805                 810                 815
Gln Ile Ala Asp Ser Lys Glu Arg Leu Lys Ser Asp Thr Gln Lys Val
            820                 825                 830
Lys Asp Ala Lys Asn Ala Asp Ala Ile Lys Lys Ala Phe Glu Glu Gly
        835                 840                 845
Lys Val Asn Ile Pro Gln Ala His Ile Pro Gly Asp Leu Asn Lys Asp
    850                 855                 860
Lys Glu Lys Leu Leu Ala Glu Leu Lys Gln Lys Ala Asp Asp Thr Glu
865                 870                 875                 880
Lys Ala Ile Asp Val Asp Lys Thr Leu Thr Glu Asp Glu Lys Lys Glu
                885                 890                 895
Gln Lys Val Lys Thr Lys Ala Glu Leu Glu Lys Ala Lys Thr Asp Val
            900                 905                 910
Lys Asn Thr Gln Thr Arg Glu Glu Leu Asp Lys Lys Val Pro Glu Leu
        915                 920                 925
Lys Lys Ala Ile Glu Asp Thr His Val Lys Gly Asn Leu Glu Gly Val
    930                 935                 940
Lys Asn Lys Ala Ile Glu Asp Leu Lys Lys Ala His Thr Glu Thr Val
945                 950                 955                 960
Ala Lys Ile Asn Gly Asp Asp Thr Leu Asp Lys Ala Thr Lys Glu Ala
                965                 970                 975
Gln Val Lys Glu Ala Asp Lys Ala Leu Ala Ala Gly Lys Asp Ala Ile
            980                 985                 990
Thr Lys Ala Asp Asp Ala Asp Lys Val Ser Thr Ala Val Thr Glu His
        995                 1000                1005
Thr Pro Lys Ile Lys Ala Ala His Lys Thr Gly Asp Leu Lys Lys Ala
    1010                1015                1020
Gln Val Asp Ala Asn Thr Ala Leu Asp Lys Ala Ala Glu Lys Glu Arg
1025                1030                1035                1040
Gly Glu Ile Asn Lys Asp Ala Thr Leu Thr Thr Glu Asp Lys Ala Lys
                1045                1050                1055
Gln Leu Lys Glu Val Glu Thr Ala Leu Thr Lys Ala Lys Asp Asn Val
            1060                1065                1070
Lys Ala Ala Lys Thr Ala Asp Ala Ile Asn Asp Ala Arg Asp Lys Gly
        1075                1080                1085
```

```
Val Ala Thr Ile Asp Ala Val His Lys Ala Gly Gln Asp Leu Gly Ala
    1090                1095                1100
Arg Lys Ser Gly Gln Val Ala Lys Leu Glu Glu Ala Ala Lys Ala Thr
1105                1110                1115                1120
Lys Asp Lys Ile Ser Ala Asp Pro Thr Leu Thr Ser Lys Glu Lys Glu
                1125                1130                1135
Glu Gln Ser Lys Ala Val Asp Ala Glu Leu Lys Lys Ala Ile Glu Ala
            1140                1145                1150
Val Asn Ala Ala Asp Thr Ala Asp Lys Val Asp Asp Ala Leu Gly Glu
        1155                1160                1165
Gly Val Thr Asp Ile Lys Asn Gln His Lys Ser Gly Asp Ser Ile Asp
    1170                1175                1180
Ala Arg Arg Glu Ala His Gly Lys Glu Leu Asp Arg Val Ala Gln Glu
1185                1190                1195                1200
Thr Lys Gly Ala Ile Glu Lys Asp Pro Thr Leu Thr Thr Glu Glu Lys
                1205                1210                1215
Ala Lys Gln Val Lys Asp Val Asp Ala Ala Lys Glu Arg Gly Met Ala
            1220                1225                1230
Lys Leu Asn Glu Ala Lys Asp Ala Asp Ala Leu Asp Lys Ala Tyr Gly
        1235                1240                1245
Glu Gly Val Thr Asp Ile Lys Asn Gln His Lys Ser Gly Asp Pro Val
    1250                1255                1260
Asp Ala Arg Arg Gly Leu His Asn Lys Ser Ile Asp Glu Val Ala Gln
1265                1270                1275                1280
Ala Thr Lys Asp Ala Ile Thr Ala Asp Thr Thr Leu Thr Glu Ala Glu
                1285                1290                1295
Lys Glu Thr Gln Arg Gly Asn Val Asp Lys Glu Ala Thr Lys Ala Lys
            1300                1305                1310
Glu Glu Leu Ala Lys Ala Lys Asp Ala Asp Ala Leu Asp Lys Ala Tyr
        1315                1320                1325
Gly Asp Gly Val Thr Ser Ile Lys Asn Gln His Lys Ser Gly Lys Gly
    1330                1335                1340
Leu Asp Val Arg Lys Asp Glu His Lys Lys Ala Leu Glu Ala Val Ala
1345                1350                1355                1360
Lys Arg Val Thr Ala Glu Ile Glu Ala Asp Pro Thr Leu Thr Pro Glu
                1365                1370                1375
Val Arg Glu Gln Gln Lys Ala Glu Val Gln Lys Glu Leu Glu Leu Ala
            1380                1385                1390
Thr Asp Lys Ile Ala Glu Ala Lys Asp Ala Asp Glu Ala Asp Lys Ala
        1395                1400                1405
Tyr Gly Asp Gly Val Thr Ala Ile Glu Asn Ala His Val Ile Gly Lys
    1410                1415                1420
Gly Ile Glu Ala Arg Lys Asp Leu Ala Lys Lys Asp Leu Ala Glu Ala
1425                1430                1435                1440
Ala Ala Lys Thr Lys Ala Leu Ile Ile Glu Asp Lys Thr Leu Thr Asp
                1445                1450                1455
Asp Gln Arg Lys Glu Gln Leu Leu Gly Val Asp Thr Glu Tyr Ala Lys
            1460                1465                1470
Gly Ile Glu Asn Ile Asp Ala Ala Lys Asp Ala Ala Gly Val Asp Lys
        1475                1480                1485
Ala Tyr Ser Asp Gly Val Arg Asp Ile Leu Ala Gln Tyr Lys Glu Gly
    1490                1495                1500
Gln Asn Leu Asn Asp Arg Arg Asn Ala Ala Lys Glu Phe Leu Leu Lys
1505                1510                1515                1520
Glu Ala Asp Lys Val Thr Lys Leu Ile Asn Asp Asp Pro Thr Leu Thr
                1525                1530                1535
His Asp Gln Lys Val Asp Gln Ile Asn Lys Val Glu Gln Ala Lys Leu
            1540                1545                1550
Asp Ala Ile Lys Ser Val Asp Asp Ala Gln Thr Ala Asp Ala Ile Asn
        1555                1560                1565
Asp Ala Leu Gly Lys Gly Ile Glu Asn Ile Asn Asn Gln Tyr Gln His
```

1570 1575 1580
Gly Asp Gly Val Asp Val Arg Lys Ala Thr Ala Lys Gly Asp Leu Glu
1585 1590 1595 1600
Lys Glu Ala Ala Lys Val Lys Ala Leu Ile Ala Lys Asp Pro Thr Leu
1605 1610 1615
Thr Gln Ala Asp Lys Asp Lys Gln Thr Ala Ala Val Asp Ala Ala Lys
1620 1625 1630
Asn Thr Ala Ile Ala Ala Val Asp Lys Ala Thr Thr Thr Glu Gly Ile
1635 1640 1645
Asn Gln Glu Leu Gly Lys Gly Ile Thr Ala Ile Asn Lys Ala Tyr Arg
1650 1655 1660
Pro Gly Glu Gly Val Lys Ala Arg Lys Glu Ala Ala Lys Ala Asp Leu
1665 1670 1675 1680
Glu Lys Glu Ala Ala Lys Val Lys Ala Leu Ile Thr Asn Asp Pro Thr
1685 1690 1695
Leu Thr Lys Ala Asp Lys Ala Lys Gln Thr Glu Ala Val Ala Lys Ala
1700 1705 1710
Leu Lys Ala Ala Ile Ala Ala Val Asp Lys Ala Thr Thr Ala Glu Gly
1715 1720 1725
Ile Asn Gln Glu Leu Gly Lys Gly Ile Thr Ala Ile Asn Lys Ala Tyr
1730 1735 1740
Arg Pro Gly Glu Gly Val Lys Ala Arg Lys Glu Ala Ala Lys Ala Asp
1745 1750 1755 1760
Leu Glu Arg Glu Ala Ala Lys Val Arg Glu Ala Ile Ala Asn Asp Pro
1765 1770 1775
Thr Leu Thr Lys Ala Asp Lys Ala Lys Gln Thr Glu Ala Val Ala Lys
1780 1785 1790
Ala Leu Lys Ala Ala Ile Ala Ala Val Asp Lys Ala Thr Thr Ala Glu
1795 1800 1805
Gly Ile Asn Gln Glu Leu Gly Lys Gly Ile Thr Ala Ile Asn Lys Ala
1810 1815 1820
Tyr Arg Pro Gly Glu Gly Val Glu Ala His Lys Glu Ala Ala Lys Ala
1825 1830 1835 1840
Asn Leu Glu Lys Val Ala Lys Glu Thr Lys Ala Leu Ile Ser Gly Asp
1845 1850 1855
Arg Tyr Leu Ser Glu Thr Glu Lys Ala Val Gln Lys Gln Ala Val Glu
1860 1865 1870
Gln Ala Leu Ala Lys Ala Leu Gly Gln Val Glu Ala Ala Lys Thr Val
1875 1880 1885
Glu Ala Val Lys Leu Ala Glu Asn Leu Gly Thr Val Ala Ile Arg Ser
1890 1895 1900
Ala Tyr Val Ala Gly Leu Ala Lys Asp Thr Asp Gln Ala Thr Ala Ala
1905 1910 1915 1920
Leu Asn Glu Ala Lys Gln Ala Ala Ile Glu Ala Leu Lys Gln Ala Ala
1925 1930 1935
Ala Glu Thr Leu Ala Lys Ile Thr Thr Asp Ala Lys Leu Thr Glu Ala
1940 1945 1950
Gln Lys Ala Glu Gln Ser Glu Asn Val Ser Leu Ala Leu Lys Thr Ala
1955 1960 1965
Ile Ala Thr Val Arg Ser Ala Gln Ser Ile Ala Ser Val Lys Glu Ala
1970 1975 1980
Lys Asp Lys Gly Ile Thr Ala Ile Arg Ala Ala Tyr Val Pro Asn Lys
1985 1990 1995 2000
Ala Val Ala Lys Ser Ser Ser Ala Asn His Leu Pro Lys Ser Gly Asp
2005 2010 2015
Ala Asn Ser Ile Val Leu Val Gly Leu Gly Val Met Ser Leu Leu Leu
2020 2025 2030
Gly Met Val Leu Tyr Ser Lys Lys Lys Glu Ser Lys Asp
2035 2040 2045

<210> 183
<211> 278
<212> PRT
<213> S. pyogenes

<400> 183

```
Met Ile Ile Lys Lys Arg Thr Val Ala Ile Leu Ala Ile Ala Ser Ser
1               5                   10                  15
Phe Phe Leu Val Ala Cys Gln Ala Thr Lys Ser Leu Lys Ser Gly Asp
            20                  25                  30
Ala Trp Gly Val Tyr Gln Lys Gln Lys Ser Ile Thr Val Gly Phe Asp
        35                  40                  45
Asn Thr Phe Val Pro Met Gly Tyr Lys Asp Glu Ser Gly Arg Cys Lys
    50                  55                  60
Gly Phe Asp Ile Asp Leu Ala Lys Glu Val Phe His Gln Tyr Gly Leu
65                  70                  75                  80
Lys Val Asn Phe Gln Ala Ile Asn Trp Asp Met Lys Glu Ala Glu Leu
                85                  90                  95
Asn Asn Gly Lys Ile Asp Val Ile Trp Asn Gly Tyr Ser Ile Thr Lys
            100                 105                 110
Glu Arg Gln Asp Lys Val Ala Phe Thr Asp Ser Tyr Met Arg Asn Glu
        115                 120                 125
Gln Ile Ile Val Val Lys Lys Arg Ser Asp Ile Lys Thr Ile Ser Asp
    130                 135                 140
Met Lys His Lys Val Leu Gly Ala Gln Ser Ala Ser Ser Gly Tyr Asp
145                 150                 155                 160
Ser Leu Leu Arg Thr Pro Lys Leu Leu Lys Asp Phe Ile Lys Asn Lys
                165                 170                 175
Asp Ala Asn Gln Tyr Glu Thr Phe Thr Gln Ala Phe Ile Asp Leu Lys
            180                 185                 190
Ser Asp Arg Ile Asp Gly Ile Leu Ile Asp Lys Val Tyr Ala Asn Tyr
        195                 200                 205
Tyr Leu Ala Lys Glu Gly Gln Leu Glu Asn Tyr Arg Met Ile Pro Thr
    210                 215                 220
Thr Phe Glu Asn Glu Ala Phe Ser Val Gly Leu Arg Lys Glu Asp Lys
225                 230                 235                 240
Thr Leu Gln Ala Lys Ile Asn Arg Ala Phe Arg Val Leu Tyr Gln Asn
                245                 250                 255
Gly Lys Phe Gln Ala Ile Ser Glu Lys Trp Phe Gly Asp Asp Val Ala
            260                 265                 270
Thr Ala Asn Ile Lys Ser
        275
```

<210> 184
<211> 792
<212> PRT
<213> S. pyogenes

<400> 184

```
Met Lys Thr Lys Lys Val Ile Ile Leu Val Gly Leu Leu Leu Ser Ser
 1               5                  10                  15
Gln Leu Thr Leu Ile Ala Cys Gln Ser Arg Gly Asn Gly Thr Tyr Pro
            20                  25                  30
Ile Lys Thr Lys Gln Ser Arg Lys Gly Met Thr Ser Asn Lys Ile Lys
        35                  40                  45
Pro Ile Lys Lys Ser Lys Lys Thr Asn Lys Thr His Lys Gly Val Ala
    50                  55                  60
Gly Val Asp Phe Pro Thr Asp Asp Gly Phe Ile Leu Thr Lys Asp Ser
65                  70                  75                  80
Lys Ile Leu Ser Lys Thr Asp Gln Gly Ile Val Val Asp His Asp Gly
                85                  90                  95
His Ser His Phe Ile Phe Tyr Ala Asp Leu Lys Gly Ser Pro Phe Glu
```

```
            100                 105                 110
Tyr Leu Ile Pro Lys Gly Ala Ser Leu Ala Lys Pro Ala Val Ala Gln
        115                 120                 125
Arg Ala Ala Ser Gln Gly Thr Ser Lys Val Ala Asp Pro His His His
    130                 135                 140
Tyr Glu Phe Asn Pro Ala Asp Ile Val Ala Glu Asp Ala Leu Gly Tyr
145                 150                 155                 160
Thr Val Arg His Asp Asp His Phe His Tyr Ile Leu Lys Ser Ser Leu
                165                 170                 175
Ser Gly Gln Thr Gln Ala Gln Ala Lys Gln Val Ala Thr Arg Leu Pro
            180                 185                 190
Gln Thr Ser Ser Leu Val Ser Thr Ala Thr Ala Asn Gly Ile Pro Gly
        195                 200                 205
Leu His Phe Pro Thr Ser Asp Gly Phe Gln Phe Asn Gly Gln Gly Ile
    210                 215                 220
Val Gly Val Thr Lys Asp Ser Ile Leu Val Asp His Asp Gly His Leu
225                 230                 235                 240
His Pro Ile Ser Phe Ala Asp Leu Arg Gln Gly Gly Trp Ala His Val
                245                 250                 255
Ala Asp Gln Tyr Asp Pro Ala Lys Lys Ala Glu Lys Pro Ala Glu Thr
            260                 265                 270
His Gln Thr Pro Glu Leu Ser Glu Arg Glu Lys Glu Tyr Gln Glu Lys
        275                 280                 285
Leu Ala Tyr Leu Ala Glu Lys Leu Gly Ile Asp Pro Ser Thr Ile Lys
    290                 295                 300
Arg Val Glu Thr Gln Asp Gly Lys Leu Gly Leu Glu Tyr Pro His His
305                 310                 315                 320
Asp His Ala His Val Leu Met Leu Ser Asp Ile Glu Ile Gly Lys Asp
                325                 330                 335
Ile Pro Asp Pro His Ala Ile Glu His Ala Arg Glu Leu Glu Lys His
            340                 345                 350
Lys Val Gly Met Asp Thr Leu Arg Ala Leu Gly Phe Asp Glu Glu Val
        355                 360                 365
Ile Leu Asp Ile Val Arg Thr His Asp Ala Pro Thr Pro Phe Pro Ser
    370                 375                 380
Asn Glu Lys Asp Pro Asn Met Met Lys Glu Trp Leu Ala Thr Val Ile
385                 390                 395                 400
Lys Leu Asp Leu Gly Ser Arg Lys Asp Pro Leu Gln Arg Lys Gly Leu
                405                 410                 415
Ser Leu Leu Pro Asn Leu Glu Thr Leu Gly Ile Gly Phe Thr Pro Ile
            420                 425                 430
Lys Asp Ile Ser Pro Val Leu Gln Phe Lys Lys Leu Lys Gln Leu Leu
        435                 440                 445
Met Thr Lys Thr Gly Val Thr Asp Tyr Arg Phe Leu Asp Asn Met Pro
    450                 455                 460
Gln Leu Glu Gly Ile Asp Ile Ser Gln Asn Asn Leu Lys Asp Ile Ser
465                 470                 475                 480
Phe Leu Ser Lys Tyr Lys Asn Leu Thr Leu Val Ala Ala Ala Asp Asn
                485                 490                 495
Gly Ile Glu Asp Ile Arg Pro Leu Gly Gln Leu Pro Asn Leu Lys Phe
            500                 505                 510
Leu Val Leu Ser Asn Asn Lys Ile Ser Asp Leu Ser Pro Leu Ala Ser
        515                 520                 525
Leu His Gln Leu Gln Glu Leu His Ile Asp Asn Asn Gln Ile Thr Asp
    530                 535                 540
Leu Ser Pro Val Ser His Lys Glu Ser Leu Thr Val Val Asp Leu Ser
545                 550                 555                 560
Arg Asn Ala Asp Val Asp Leu Ala Thr Leu Gln Ala Pro Lys Leu Glu
                565                 570                 575
Thr Leu Met Val Asn Asp Thr Lys Val Ser His Leu Asp Phe Leu Lys
            580                 585                 590
```

```
Asn Asn Pro Asn Leu Ser Ser Leu Ser Ile Asn Arg Ala Gln Leu Gln
        595                 600                 605
Ser Leu Glu Gly Ile Glu Ala Ser Ser Val Ile Val Arg Val Glu Ala
    610                 615                 620
Glu Gly Asn Gln Ile Lys Ser Leu Val Leu Lys Asp Lys Gln Gly Ser
625                 630                 635                 640
Leu Thr Phe Leu Asp Val Thr Gly Asn Gln Leu Thr Ser Leu Glu Gly
                645                 650                 655
Val Asn Asn Phe Thr Ala Leu Asp Ile Leu Ser Val Ser Lys Asn Gln
            660                 665                 670
Leu Thr Asn Val Asn Leu Ser Lys Pro Asn Lys Thr Val Thr Asn Ile
        675                 680                 685
Asp Ile Ser His Asn Asn Ile Ser Leu Ala Asp Leu Lys Leu Asn Glu
    690                 695                 700
Gln His Ile Pro Glu Ala Ile Ala Lys Asn Phe Pro Ala Val Tyr Glu
705                 710                 715                 720
Gly Ser Met Val Gly Asn Gly Thr Ala Glu Glu Lys Ala Ala Met Ala
                725                 730                 735
Thr Lys Ala Lys Glu Ser Ala Gln Glu Ala Ser Glu Ser His Asp Tyr
            740                 745                 750
Asn His Asn His Thr Tyr Glu Asp Glu Glu Gly His Ala His Glu His
        755                 760                 765
Arg Asp Lys Asp Asp His Asp His Glu His Glu Asp Glu Asn Glu Ala
    770                 775                 780
Lys Asp Glu Gln Asn His Ala Asp
785                 790
```

<210> 185
<211> 351
<212> PRT
<213> S. pyogenes

<400> 185

```
Met Lys Asn Ser Asn Lys Leu Ile Ala Ser Val Val Thr Leu Ala Ser
 1               5                  10                  15
Val Met Ala Leu Ala Ala Cys Gln Ser Thr Asn Asp Asn Thr Lys Val
            20                  25                  30
Ile Ser Met Lys Gly Asp Thr Ile Ser Val Ser Asp Phe Tyr Asn Glu
        35                  40                  45
Thr Lys Asn Thr Glu Val Ser Gln Lys Ala Met Leu Asn Leu Val Ile
    50                  55                  60
Ser Arg Val Phe Glu Ala Gln Tyr Gly Asp Lys Val Ser Lys Lys Glu
65                  70                  75                  80
Val Glu Lys Ala Tyr His Lys Thr Ala Glu Gln Tyr Gly Ala Ser Phe
                85                  90                  95
Ser Ala Ala Leu Ala Gln Ser Ser Leu Thr Pro Glu Thr Phe Lys Arg
            100                 105                 110
Gln Ile Arg Ser Ser Lys Leu Val Glu Tyr Ala Val Lys Glu Ala Ala
        115                 120                 125
Lys Lys Glu Leu Thr Thr Gln Glu Tyr Lys Lys Ala Tyr Glu Ser Tyr
    130                 135                 140
Thr Pro Thr Met Ala Val Glu Met Ile Thr Leu Asp Asn Glu Glu Thr
145                 150                 155                 160
Ala Lys Ser Val Leu Glu Glu Leu Lys Ala Glu Gly Ala Asp Phe Thr
                165                 170                 175
Ala Ile Ala Lys Glu Lys Thr Thr Thr Pro Glu Lys Lys Val Thr Tyr
            180                 185                 190
Lys Phe Asp Ser Gly Ala Thr Asn Val Pro Thr Asp Val Val Lys Ala
        195                 200                 205
Ala Ser Ser Leu Asn Glu Gly Gly Ile Ser Asp Val Ile Ser Val Leu
    210                 215                 220

Asp Pro Thr Ser Tyr Gln Lys Lys Phe Tyr Ile Val Lys Val Thr Lys
225                 230                 235                 240
Lys Ala Glu Lys Lys Ser Asp Trp Gln Glu Tyr Lys Lys Arg Leu Lys
                245                 250                 255
Ala Ile Ile Ile Ala Glu Lys Ser Lys Asp Met Asn Phe Gln Asn Lys
            260                 265                 270
Val Ile Ala Asn Ala Leu Asp Lys Ala Asn Val Lys Ile Lys Asp Lys
        275                 280                 285
Ala Phe Ala Asn Ile Leu Ala Gln Tyr Ala Asn Leu Gly Gln Lys Thr
    290                 295                 300
Lys Ala Ala Ser Glu Ser Ser Thr Thr Ser Glu Ser Ser Lys Ala Ala
305                 310                 315                 320
Glu Glu Asn Pro Ser Glu Ser Glu Gln Thr Gln Thr Ser Ser Ala Glu
                325                 330                 335
Glu Pro Thr Glu Thr Glu Ala Gln Thr Gln Glu Pro Ala Ala Gln
            340                 345                 350
```

<210> 186
<211> 424
<212> PRT
<213> S. pyogenes

<400> 186

```
Met Lys Ile Gly Lys Lys Ile Val Leu Met Phe Thr Ala Ile Val Leu
1               5                   10                  15
Thr Thr Val Leu Ala Leu Gly Val Tyr Leu Thr Ser Ala Tyr Thr Phe
            20                  25                  30
Ser Thr Gly Glu Leu Ser Lys Thr Phe Lys Asp Phe Ser Thr Ser Ser
        35                  40                  45
Asn Lys Ser Asp Ala Ile Lys Gln Thr Arg Ala Phe Ser Ile Leu Leu
    50                  55                  60
Met Gly Val Asp Thr Gly Ser Ser Glu Arg Ala Ser Lys Trp Glu Gly
65                  70                  75                  80
Asn Ser Asp Ser Met Ile Leu Val Thr Val Asn Pro Lys Thr Lys Lys
                85                  90                  95
Thr Thr Met Thr Ser Leu Glu Arg Asp Thr Leu Thr Thr Leu Ser Gly
            100                 105                 110
Pro Lys Asn Asn Glu Met Asn Gly Val Glu Ala Lys Leu Asn Ala Ala
        115                 120                 125
Tyr Ala Ala Gly Gly Ala Gln Met Ala Ile Met Thr Val Gln Asp Leu
    130                 135                 140
Leu Asn Ile Thr Ile Asp Asn Tyr Val Gln Ile Asn Met Gln Gly Leu
145                 150                 155                 160
Ile Asp Leu Val Asn Ala Val Gly Gly Ile Thr Val Thr Asn Glu Phe
                165                 170                 175
Asp Phe Pro Ile Ser Ile Ala Glu Asn Glu Pro Glu Tyr Gln Ala Thr
            180                 185                 190
Val Ala Pro Gly Thr His Lys Ile Asn Gly Glu Gln Ala Leu Val Tyr
        195                 200                 205
Ala Arg Met Arg Tyr Asp Asp Pro Glu Gly Asp Tyr Gly Arg Gln Lys
    210                 215                 220
Arg Gln Arg Glu Val Ile Gln Lys Val Leu Lys Lys Ile Leu Ala Leu
225                 230                 235                 240
Asp Ser Ile Ser Ser Tyr Arg Lys Ile Leu Ser Ala Val Ser Ser Asn
                245                 250                 255
Met Gln Thr Asn Ile Glu Ile Ser Ser Arg Thr Ile Pro Ser Leu Leu
            260                 265                 270
Gly Tyr Arg Asp Ala Leu Arg Thr Ile Lys Thr Tyr Gln Leu Lys Gly
        275                 280                 285
Glu Asp Ala Thr Leu Ser Asp Gly Gly Ser Tyr Gln Ile Val Thr Ser
    290                 295                 300

Asn His Leu Leu Glu Ile Gln Asn Arg Ile Arg Thr Glu Leu Gly Leu
305                 310                 315                 320
His Lys Val Asn Gln Leu Lys Thr Asn Ala Thr Val Tyr Glu Asn Leu
                325                 330                 335
Tyr Gly Ser Thr Lys Ser Gln Thr Val Asn Asn Asn Tyr Asp Ser Ser
            340                 345                 350
Gly Gln Ala Pro Ser Tyr Ser Asp Ser His Ser Ser Tyr Ala Asn Tyr
        355                 360                 365
Ser Ser Gly Val Asp Thr Gly Gln Ser Ala Ser Thr Asp Gln Asp Ser
    370                 375                 380
Thr Ala Ser Ser His Arg Pro Ala Thr Pro Ser Ser Ser Ser Asp Ala
385                 390                 395                 400
Leu Ala Ala Asp Glu Ser Ser Ser Ser Gly Ser Gly Ser Leu Val Pro
                405                 410                 415
Pro Ala Asn Ile Asn Pro Gln Thr
            420
```

<210> 187
<211> 284
<212> PRT

<213> S. pyogenes

<400> 187

```
Met Lys Ser Lys Lys Val Val Ser Val Ile Ser Leu Thr Leu Ser Leu
 1               5                  10                  15
Phe Leu Val Thr Gly Cys Ala Lys Val Asp Asn Asn Lys Ser Val Asn
            20                  25                  30
Leu Lys Pro Ala Thr Lys Gln Thr Tyr Asn Ser Tyr Ser Asp Asp Gln
        35                  40                  45
Leu Arg Ser Arg Glu Asn Thr Met Ser Val Leu Trp Tyr Gln Arg Ala
    50                  55                  60
Ala Glu Thr Gln Ala Leu Tyr Leu Gln Gly Tyr Gln Leu Ala Thr Asp
65                  70                  75                  80
Arg Leu Lys Glu Gln Leu Asn Lys Pro Thr Asp Lys Pro Tyr Ser Ile
                85                  90                  95
Val Leu Asp Ile Asp Glu Thr Val Leu Asp Asn Ser Pro Tyr Gln Ala
            100                 105                 110
Lys Asn Val Leu Glu Gly Thr Gly Phe Thr Pro Glu Ser Trp Asp Tyr
        115                 120                 125
Trp Val Gln Lys Lys Glu Ala Lys Pro Val Ala Gly Ala Lys Asp Phe
    130                 135                 140
Leu Gln Phe Ala Asp Gln Asn Gly Val Gln Ile Tyr Tyr Ile Ser Asp
145                 150                 155                 160
Arg Ser Thr Thr Gln Val Asp Ala Thr Met Glu Asn Leu Gln Lys Glu
                165                 170                 175
Gly Ile Pro Val Gln Gly Arg Asp His Leu Leu Phe Leu Glu Lys Gly
            180                 185                 190
Val Lys Ser Lys Glu Ser Arg Arg Gln Lys Val Lys Glu Thr Thr Asn
        195                 200                 205
Val Thr Met Leu Phe Gly Asp Asn Leu Leu Asp Phe Ala Asp Phe Ser
    210                 215                 220
Lys Lys Ser Gln Glu Asp Arg Thr Ala Leu Leu Ser Asp Leu Gln Glu
225                 230                 235                 240
Glu Phe Gly Arg Arg Phe Ile Ile Phe Pro Asn Pro Met Tyr Gly Ser
                245                 250                 255
Trp Glu Gly Ala Ile Tyr Lys Gly Glu Lys Leu Asp Val Leu Lys Gln
            260                 265                 270
Leu Glu Glu Arg Arg Lys Ser Leu Lys Ser Phe Lys
        275                 280
```

<210> 188
<211> 440
<212> PRT
<213> S. pyogenes

<400> 188

```
Met Lys Asn Tyr Leu Ser Ile Gly Val Ile Ala Leu Leu Phe Ala Leu
 1               5                  10                  15
Thr Phe Gly Thr Val Lys Ser Val Gln Ala Ile Ala Gly Tyr Gly Trp
            20                  25                  30
Leu Pro Asp Arg Pro Pro Ile Asn Asn Ser Gln Leu Val Val Ser Met
        35                  40                  45
Ala Gly Ile Val Glu Gly Thr Asp Lys Lys Val Phe Ile Asn Phe Phe
    50                  55                  60
Glu Ile Asp Leu Thr Ser Gln Pro Ala His Gly Gly Lys Thr Glu Gln
65                  70                  75                  80
Gly Leu Ser Pro Lys Ser Lys Pro Phe Ala Thr Asp Asn Gly Ala Met
                85                  90                  95
Pro His Lys Leu Glu Lys Ala Asp Leu Leu Lys Ala Ile Gln Lys Gln
            100                 105                 110
Leu Ile Ala Asn Val His Ser Asn Asp Gly Tyr Phe Glu Val Ile Asp
        115                 120                 125
Phe Ala Ser Asp Ala Thr Ile Thr Asp Arg Asn Gly Lys Val Tyr Phe
    130                 135                 140
Ala Asp Lys Asp Gly Ser Val Thr Leu Pro Thr Gln Pro Val Gln Glu
145                 150                 155                 160
Phe Leu Leu Lys Gly His Val Arg Val Arg Pro Tyr Lys Glu Lys Pro
                165                 170                 175
Val Gln Asn Gln Ala Lys Ser Val Asp Val Glu Tyr Thr Val Gln Phe
            180                 185                 190
Thr Pro Leu Asn Pro Asp Asp Asp Phe Arg Pro Gly Leu Lys Asp Thr
        195                 200                 205
Lys Leu Leu Lys Thr Leu Ala Ile Gly Asp Thr Ile Thr Ser Gln Glu
    210                 215                 220
Leu Leu Ala Gln Ala Gln Ser Ile Leu Asn Lys Thr His Pro Gly Tyr
225                 230                 235                 240
Thr Ile Tyr Glu Arg Asp Ser Ser Ile Val Thr His Asp Asn Asp Ile
                245                 250                 255
Phe Arg Thr Ile Leu Pro Met Asp Gln Glu Phe Thr Tyr His Val Lys
            260                 265                 270
Asn Arg Glu Gln Ala Tyr Glu Ile Asn Pro Lys Thr Gly Ile Lys Glu
        275                 280                 285
Lys Thr Asn Asn Thr Asp Leu Val Ser Glu Lys Tyr Tyr Val Leu Lys
    290                 295                 300
Gln Gly Glu Lys Pro Tyr Asp Pro Phe Asp Arg Ser His Leu Lys Leu
305                 310                 315                 320
Phe Thr Ile Lys Tyr Val Asp Val Asn Thr Asn Glu Leu Leu Lys Ser
                325                 330                 335
Glu Gln Leu Leu Thr Ala Ser Glu Arg Asn Leu Asp Phe Arg Asp Leu
            340                 345                 350
Tyr Asp Pro Arg Asp Lys Ala Lys Leu Leu Tyr Asn Asn Leu Asp Ala
        355                 360                 365
Phe Asp Ile Met Asp Tyr Thr Leu Thr Gly Lys Val Glu Asp Asn His
    370                 375                 380
Asp Lys Asn Asn Arg Val Val Thr Val Tyr Met Gly Lys Arg Pro Lys
385                 390                 395                 400
Gly Ala Lys Gly Ser Tyr His Leu Ala Tyr Asp Lys Asp Leu Tyr Thr
                405                 410                 415
Glu Glu Glu Arg Lys Ala Tyr Ser Tyr Leu Arg Asp Thr Gly Thr Pro
            420                 425                 430
Ile Pro Asp Asn Pro Lys Asp Lys
        435                 440
```

<210> 189
<211> 542
<212> PRT

<213> S. pyogenes

<400> 189

```
Met Ser Lys Tyr Leu Lys Tyr Phe Ser Ile Ile Thr Leu Phe Leu Thr
 1               5                  10                  15
Gly Leu Ile Leu Val Ala Cys Gln Gln Gln Lys Pro Gln Thr Lys Glu
            20                  25                  30
Arg Gln Arg Lys Gln Arg Pro Lys Asp Glu Leu Val Val Ser Met Gly
        35                  40                  45
Ala Lys Leu Pro His Glu Phe Asp Pro Lys Asp Arg Tyr Gly Val His
    50                  55                  60
Asn Glu Gly Asn Ile Thr His Ser Thr Leu Leu Lys Arg Ser Pro Glu
65                  70                  75                  80
Leu Asp Ile Lys Gly Glu Leu Ala Lys Thr Tyr His Leu Ser Glu Asp
                85                  90                  95
Gly Leu Thr Trp Ser Phe Asp Leu His Asp Asp Phe Lys Phe Ser Asn
            100                 105                 110
Gly Glu Pro Val Thr Ala Asp Asp Val Lys Phe Thr Tyr Asp Met Leu
        115                 120                 125
Lys Ala Asp Gly Lys Ala Trp Asp Leu Thr Phe Ile Lys Asn Val Glu
    130                 135                 140
Val Val Gly Lys Asn Gln Val Asn Ile His Leu Thr Glu Ala His Ser
145                 150                 155                 160
Thr Phe Thr Ala Gln Leu Thr Glu Ile Pro Ile Val Pro Lys Lys His
                165                 170                 175
Tyr Asn Asp Lys Tyr Lys Ser Asn Pro Ile Gly Ser Gly Pro Tyr Met
            180                 185                 190
Val Lys Glu Tyr Lys Ala Gly Glu Gln Ala Ile Phe Val Arg Asn Pro
        195                 200                 205
Tyr Trp His Gly Lys Lys Pro Tyr Phe Lys Lys Trp Thr Trp Val Leu
    210                 215                 220
Leu Asp Glu Asn Thr Ala Leu Ala Ala Leu Glu Ser Gly Asp Val Asp
225                 230                 235                 240
Met Ile Tyr Ala Thr Pro Glu Leu Ala Asp Lys Lys Val Lys Gly Thr
                245                 250                 255
Arg Leu Leu Asp Ile Pro Ser Asn Asp Val Arg Gly Leu Ser Leu Pro
            260                 265                 270
Tyr Val Lys Lys Gly Val Ile Thr Asp Ser Pro Asp Gly Tyr Pro Val
        275                 280                 285
Gly Asn Asp Val Thr Ser Asp Pro Ala Ile Arg Lys Ala Leu Thr Ile
    290                 295                 300
Gly Leu Asn Arg Gln Lys Val Leu Asp Thr Val Leu Asn Gly Tyr Gly
305                 310                 315                 320
Lys Pro Ala Tyr Ser Ile Ile Asp Lys Thr Pro Phe Trp Asn Pro Lys
                325                 330                 335
Thr Ala Ile Lys Asp Asn Lys Val Ala Lys Ala Lys Gln Leu Leu Thr
            340                 345                 350
Lys Ala Gly Trp Lys Glu Gln Ala Asp Gly Ser Arg Lys Lys Gly Asp
        355                 360                 365
Leu Asp Ala Ala Phe Asp Leu Tyr Tyr Pro Thr Asn Asp Gln Leu Arg
    370                 375                 380
Ala Asn Leu Ala Val Glu Val Ala Glu Gln Ala Lys Ala Leu Gly Ile
385                 390                 395                 400
Thr Ile Lys Leu Lys Ala Ser Asn Trp Asp Glu Met Ala Thr Lys Ser
                405                 410                 415
His Asp Ser Ala Leu Leu Tyr Ala Gly Gly Arg His His Ala Gln Gln
            420                 425                 430
```

```
Phe Tyr Glu Ser His His Pro Ser Leu Ala Gly Lys Gly Trp Thr Asn
        435                 440                 445
Ile Thr Phe Tyr Asn Asn Pro Thr Val Thr Lys Tyr Leu Asp Lys Ala
    450                 455                 460
Met Thr Ser Ser Asp Leu Asp Lys Ala Asn Glu Tyr Trp Lys Leu Ala
465                 470                 475                 480
Gln Trp Asp Gly Lys Thr Gly Ala Ser Thr Leu Gly Asp Leu Pro Asn
                485                 490                 495
Val Trp Leu Val Ser Leu Asn His Thr Tyr Ile Gly Asp Lys Arg Ile
            500                 505                 510
Asn Val Gly Lys Gln Gly Val His Ser His Gly His Asp Trp Ser Leu
        515                 520                 525
Leu Thr Asn Ile Ala Glu Trp Thr Trp Asp Glu Ser Thr Lys
    530                 535                 540
```

<210> 190
<211> 308
<212> PRT
<213> S. pyogenes

<400> 190

```
Met Asn Ile Arg Asn Lys Ile Glu Asn Ser Lys Thr Leu Leu Phe Thr
 1               5                  10                  15
Ser Leu Val Ala Val Ala Leu Leu Gly Ala Thr Gln Pro Val Ser Ala
            20                  25                  30
Glu Thr Tyr Thr Ser Arg Asn Phe Asp Trp Ser Gly Asp Asp Trp Ser
        35                  40                  45
Gly Asp Asp Trp Pro Glu Asp Asp Trp Ser Gly Asp Gly Leu Ser Lys
    50                  55                  60
Tyr Asp Arg Ser Gly Val Gly Leu Ser Gln Tyr Gly Trp Ser Lys Tyr
65                  70                  75                  80
Gly Trp Ser Ser Asp Lys Glu Glu Trp Pro Glu Asp Trp Pro Glu Asp
                85                  90                  95
Asp Trp Ser Ser Asp Lys Lys Asp Glu Thr Glu Asp Lys Thr Arg Pro
            100                 105                 110
Pro Tyr Gly Glu Ala Leu Gly Thr Gly Tyr Glu Lys Arg Asp Asp Trp
        115                 120                 125
Gly Gly Pro Gly Thr Val Ala Thr Asp Pro Tyr Thr Pro Pro Tyr Gly
    130                 135                 140
Gly Ala Leu Gly Thr Gly Tyr Glu Lys Arg Asp Asp Trp Gly Gly Pro
145                 150                 155                 160
Gly Thr Val Ala Thr Asp Pro Tyr Thr Pro Pro Tyr Gly Gly Ala Leu
                165                 170                 175
Gly Thr Gly Tyr Glu Lys Arg Asp Asp Trp Arg Gly Pro Gly His Ile
            180                 185                 190
Pro Lys Pro Glu Asn Glu Gln Ser Pro Asn Pro Leu His Ile Pro Glu
        195                 200                 205
Pro Pro Gln Ile Glu Trp Pro Gln Trp Asn Gly Phe Asp Gly Leu Ser
    210                 215                 220
Phe Gly Pro Ser Asp Trp Gly Gln Ser Glu Asp Thr Pro Pro Ser Glu
225                 230                 235                 240
Pro Arg Val Pro Glu Lys Pro Gln His Thr Pro Gln Lys Asn Pro Gln
                245                 250                 255
Glu Ser Asp Phe Asp Arg Gly Phe Ser Ala Gly Leu Lys Ala Lys Asn
            260                 265                 270
Ser Gly Arg Gly Ile Asp Phe Glu Gly Phe Gln Tyr Gly Gly Trp Ser
        275                 280                 285
Asp Glu Tyr Lys Lys Gly Tyr Met Gln Ala Phe Gly Thr Pro Tyr Thr
    290                 295                 300
Pro Ser Ala Thr
305
```

<210> 191
<211> 428
<212> PRT
<213> S. pyogenes

<400> 191

```
Met Ser His Met Lys Lys Arg Pro Glu Val Leu Ser Pro Ala Gly Thr
1               5                   10                  15
Leu Glu Lys Leu Lys Val Ala Ile Asp Tyr Gly Ala Asp Ala Val Phe
            20                  25                  30
Val Gly Gly Gln Ala Tyr Gly Leu Arg Ser Arg Ala Gly Asn Phe Ser
        35                  40                  45
Met Glu Glu Leu Gln Glu Gly Ile Asp Tyr Ala His Ala Arg Gly Ala
    50                  55                  60
Lys Val Tyr Val Ala Ala Asn Met Val Thr His Glu Gly Asn Glu Ile
65                  70                  75                  80
Gly Ala Gly Glu Trp Phe Arg Gln Leu Arg Asp Met Gly Leu Asp Ala
                85                  90                  95
Val Ile Val Ser Asp Pro Ala Leu Ile Val Ile Cys Ser Thr Glu Ala
            100                 105                 110
Pro Gly Leu Glu Ile His Leu Ser Thr Gln Ala Ser Ser Thr Asn Tyr
        115                 120                 125
Glu Thr Phe Glu Phe Trp Lys Ala Met Gly Leu Thr Arg Val Val Leu
    130                 135                 140
Ala Arg Glu Val Asn Met Ala Glu Leu Ala Glu Ile Arg Lys Arg Thr
145                 150                 155                 160
Asp Val Glu Ile Glu Ala Phe Val His Gly Ala Met Cys Ile Ser Tyr
                165                 170                 175
Ser Gly Arg Cys Val Leu Ser Asn His Met Ser His Arg Asp Ala Asn
            180                 185                 190
Arg Gly Gly Cys Ser Gln Ser Cys Arg Trp Lys Tyr Asp Leu Tyr Asp
        195                 200                 205
Met Pro Phe Gly Gly Glu Arg Arg Ser Leu Lys Gly Glu Ile Pro Glu
    210                 215                 220
Asp Tyr Ser Met Ser Ser Val Asp Met Cys Met Ile Asp His Ile Pro
225                 230                 235                 240
Asp Leu Ile Glu Asn Gly Val Asp Ser Leu Lys Ile Glu Gly Arg Met
                245                 250                 255
Lys Ser Ile His Tyr Val Ser Thr Val Thr Asn Cys Tyr Lys Ala Ala
            260                 265                 270
Val Gly Ala Tyr Met Glu Ser Pro Glu Ala Phe Tyr Ala Ile Lys Glu
        275                 280                 285
Glu Leu Ile Asp Glu Leu Trp Lys Val Ala Gln Arg Glu Leu Ala Thr
    290                 295                 300
Gly Phe Tyr Tyr Gly Ile Pro Thr Glu Asn Glu Gln Leu Phe Gly Ala
305                 310                 315                 320
Arg Arg Lys Ile Pro Gln Tyr Lys Phe Val Gly Glu Val Val Ala Phe
                325                 330                 335
Asp Ser Ala Ser Met Thr Ala Thr Ile Arg Gln Arg Asn Val Ile Met
            340                 345                 350
Glu Gly Asp Arg Ile Glu Cys Tyr Gly Pro Gly Phe Arg His Phe Glu
        355                 360                 365
Thr Val Val Lys Asp Leu His Asp Ala Asp Gly Gln Lys Ile Asp Arg
    370                 375                 380
Ala Pro Asn Pro Met Glu Leu Leu Thr Ile Ser Leu Pro Arg Glu Val
385                 390                 395                 400
Lys Pro Gly Asp Met Ile Arg Ala Cys Lys Glu Gly Leu Val Asn Leu
                405                 410                 415
Tyr Gln Lys Asp Gly Thr Ser Lys Thr Val Arg Thr
            420                 425
```

<210> 192
<211> 357
<212> PRT
<213> S. pyogenes

<400> 192

```
Met Arg Lys Leu Tyr Ser Phe Leu Ala Gly Val Leu Gly Val Ile Val
 1               5                  10                  15
Ile Leu Thr Ser Leu Ser Phe Ile Leu Gln Lys Lys Ser Gly Ser Gly
            20                  25                  30
Ser Gln Ser Asp Lys Leu Val Ile Tyr Asn Trp Gly Asp Tyr Ile Asp
        35                  40                  45
Pro Ala Leu Leu Lys Lys Phe Thr Lys Glu Thr Gly Ile Glu Val Gln
    50                  55                  60
Tyr Glu Thr Phe Asp Ser Asn Glu Ala Met Tyr Thr Lys Ile Lys Gln
65                  70                  75                  80
Gly Gly Thr Thr Tyr Asp Ile Ala Val Pro Ser Asp Tyr Thr Ile Asp
                85                  90                  95
Lys Met Ile Lys Glu Asn Leu Leu Asn Lys Leu Asp Lys Ser Lys Leu
            100                 105                 110
Val Gly Met Asp Asn Ile Gly Lys Glu Phe Leu Gly Lys Ser Phe Asp
        115                 120                 125
Pro Gln Asn Asp Tyr Ser Leu Pro Tyr Phe Trp Gly Thr Val Gly Ile
    130                 135                 140
Val Tyr Asn Asp Gln Leu Val Asp Lys Ala Pro Met His Trp Glu Asp
145                 150                 155                 160
Leu Trp Arg Pro Glu Tyr Lys Asn Ser Ile Met Leu Ile Asp Gly Ala
                165                 170                 175
Arg Glu Met Leu Gly Val Gly Leu Thr Thr Phe Gly Tyr Ser Val Asn
            180                 185                 190
Ser Lys Asn Leu Glu Gln Leu Gln Ala Ala Glu Arg Lys Leu Gln Gln
        195                 200                 205
Leu Thr Pro Asn Val Lys Ala Ile Val Ala Asp Glu Met Lys Gly Tyr
    210                 215                 220
Met Ile Gln Gly Asp Ala Ala Ile Gly Ile Thr Phe Ser Gly Glu Ala
225                 230                 235                 240
Ser Glu Met Leu Asp Ser Asn Glu His Leu His Tyr Ile Val Pro Ser
                245                 250                 255
Glu Gly Ser Asn Leu Trp Phe Asp Asn Leu Val Leu Pro Lys Thr Met
            260                 265                 270
Lys His Glu Lys Glu Ala Tyr Ala Phe Leu Asn Phe Ile Asn Arg Pro
        275                 280                 285
Glu Asn Ala Ala Gln Asn Ala Ala Tyr Ile Gly Tyr Ala Thr Pro Asn
    290                 295                 300
Lys Lys Ala Lys Ala Leu Leu Pro Asp Glu Ile Lys Asn Asp Pro Ala
305                 310                 315                 320
Phe Tyr Pro Thr Asp Asp Ile Ile Lys Lys Leu Glu Val Tyr Asp Asn
                325                 330                 335
Leu Gly Ser Arg Trp Leu Gly Ile Tyr Asn Asp Leu Tyr Leu Gln Phe
            340                 345                 350
Lys Met Tyr Arg Lys
        355
```

<210> 193
<211> 328
<212> PRT
<213> S. pyogenes

<400> 193

```
Met Lys His Pro Ile Arg Lys Thr Leu Val Thr Leu Gly Leu Leu Leu
```

```
1               5                   10                  15
Thr Leu Cys Leu Pro Thr Pro Val Ala Ala Ser Ser Arg Ser Trp Lys
            20                  25                  30
Ser Trp Phe Ile Glu Gln Tyr Phe Trp Leu Lys Arg Asp Lys Ser Tyr
        35                  40                  45
Tyr Ser Lys Gln Asp Asp Pro Ser Phe Gln Arg Tyr Leu Asp Ala Cys
    50                  55                  60
Arg Glu Gln Ser Asp Lys Pro Tyr Gln Leu Asp Thr Asn Leu Val Asn
65                  70                  75                  80
Gly Pro Leu Val Gln Glu Asn Leu Tyr Gly Met Gln Val Tyr Ser Trp
                85                  90                  95
Asn Asp Asn Gly Lys Pro Asp Gln Lys Thr Ile Ile Tyr Leu Ala Gly
            100                 105                 110
Gly Ser Tyr Leu Asn Asn Pro Thr Thr Tyr His Ile Asn Met Leu Lys
        115                 120                 125
Thr Leu Ser Thr Ser Leu Asp Ala Lys Ile Val Leu Pro Ile Tyr Pro
    130                 135                 140
Lys Ala Pro Arg Tyr Thr Tyr Asn Tyr Thr Met Pro Lys Leu Val Asn
145                 150                 155                 160
Leu Tyr Gln His Tyr Tyr His Lys Asn Gln Asn Val Phe Leu Met Gly
                165                 170                 175
Asp Ser Ala Gly Gly Gly Leu Ala Leu Gly Leu Ala His Ala Leu His
            180                 185                 190
Asn Glu Ser Val Pro Gln Pro Lys Gln Leu Val Leu Leu Ser Pro Trp
        195                 200                 205
Leu Asp Val Thr Met Ser His Pro Glu Ile Pro Glu Tyr Glu Asp Ala
    210                 215                 220
Asp Pro Ile Leu Ser Ser Trp Gly Leu Lys Arg Val Gly Glu Leu Trp
225                 230                 235                 240
Ala Tyr Ser Ala Asp Asn Thr Asn His Ile Tyr Val Ser Pro Lys Asn
                245                 250                 255
Gly Pro Ile Thr Tyr Leu Pro Pro Ile Thr Leu Phe Thr Gly Thr Arg
            260                 265                 270
Glu Ile Phe Tyr Pro Asp Ile Arg Asp Tyr Ala Ala Lys Leu Lys Ala
        275                 280                 285
Ala Asn His Asn Ile Thr Phe Ile Thr Gln Glu Gly Met Asn His Val
    290                 295                 300
Tyr Pro Ile Tyr Pro Ile Glu Glu Ala Lys Thr Ala Gln Tyr Gln Ile
305                 310                 315                 320
Ile Asp Ala Ile Asn Lys Thr Pro
                325
```

<210> 194
<211> 541
<212> PRT
<213> S. pyogenes

<400> 194

```
Met Lys Lys Arg Lys Leu Leu Ala Val Thr Leu Leu Ser Thr Ile Leu
 1               5                  10                  15
Leu Asn Ser Ala Val Pro Leu Val Val Ala Asp Thr Ser Leu Arg Asn
            20                  25                  30
Ser Thr Ser Ser Thr Asp Gln Pro Thr Thr Ala Asp Thr Asp Thr Asp
        35                  40                  45
Asp Glu Ser Glu Thr Pro Lys Lys Asp Lys Lys Ser Lys Glu Thr Ala
    50                  55                  60
Ser Gln His Asp Thr Gln Lys Asp His Lys Pro Ser His Thr His Pro
65                  70                  75                  80
Thr Pro Pro Ser Asn Asp Thr Lys Gln Thr Asp Gln Ala Ser Ser Glu
                85                  90                  95
Ala Thr Asp Lys Pro Asn Lys Asp Lys Asn Asp Thr Lys Gln Pro Asp
```

```
                100                     105                     110
Ser Ser Asp Gln Ser Thr Pro Ser Pro Lys Asp Gln Ser Ser Gln Lys
            115                     120                     125
Glu Ser Gln Asn Lys Asp Gly Arg Pro Thr Pro Ser Pro Asp Gln Gln
    130                     135                     140
Lys Asp Gln Thr Pro Asp Lys Thr Pro Glu Lys Ser Ala Asp Lys Thr
145                     150                     155                     160
Pro Glu Lys Gly Pro Glu Lys Ala Thr Asp Lys Thr Pro Glu Pro Asn
                165                     170                     175
Arg Asp Ala Pro Lys Pro Ile Gln Pro Pro Leu Ala Ala Ala Pro Val
            180                     185                     190
Phe Ile Pro Trp Arg Glu Ser Asp Lys Asp Leu Ser Lys Leu Lys Pro
        195                     200                     205
Ser Ser Arg Ser Ser Ala Ala Tyr Val Arg His Trp Thr Gly Asp Ser
    210                     215                     220
Ala Tyr Thr His Asn Leu Leu Ser Arg Arg Tyr Gly Ile Thr Ala Glu
225                     230                     235                     240
Gln Leu Asp Gly Phe Leu Asn Ser Leu Gly Ile His Tyr Asp Lys Glu
                245                     250                     255
Arg Leu Asn Gly Lys Arg Leu Leu Glu Trp Glu Lys Leu Thr Gly Leu
            260                     265                     270
Asp Val Arg Ala Ile Val Ala Ile Ala Met Ala Glu Ser Ser Leu Gly
        275                     280                     285
Thr Gln Gly Val Ala Lys Glu Lys Gly Ala Asn Met Phe Gly Tyr Gly
    290                     295                     300
Ala Phe Asp Phe Asn Pro Asn Asn Ala Lys Lys Tyr Ser Asp Glu Val
305                     310                     315                     320
Ala Ile Arg His Met Val Glu Asp Thr Ile Ile Ala Asn Lys Asn Gln
                325                     330                     335
Thr Phe Glu Arg Gln Asp Leu Lys Ala Lys Lys Trp Ser Leu Gly Gln
            340                     345                     350
Leu Asp Thr Leu Ile Asp Gly Gly Val Tyr Phe Thr Asp Thr Ser Gly
        355                     360                     365
Ser Gly Gln Arg Arg Ala Asp Ile Met Thr Lys Leu Asp Gln Trp Ile
    370                     375                     380
Asp Asp His Gly Ser Thr Pro Glu Ile Pro Glu His Leu Lys Ile Thr
385                     390                     395                     400
Ser Gly Thr Gln Phe Ser Glu Val Pro Val Gly Tyr Lys Arg Ser Gln
                405                     410                     415
Pro Gln Asn Val Leu Thr Tyr Lys Ser Glu Thr Tyr Ser Phe Gly Gln
            420                     425                     430
Cys Thr Trp Tyr Ala Tyr Asn Arg Val Lys Glu Leu Gly Tyr Gln Val
        435                     440                     445
Asp Arg Tyr Met Gly Asn Gly Gly Asp Trp Gln Arg Lys Pro Gly Phe
    450                     455                     460
Val Thr Thr His Lys Pro Lys Val Gly Tyr Val Val Ser Phe Ala Pro
465                     470                     475                     480
Gly Gln Ala Gly Ala Asp Ala Thr Tyr Gly His Val Ala Val Val Glu
                485                     490                     495
Gln Ile Lys Glu Asp Gly Ser Ile Leu Ile Ser Glu Ser Asn Val Met
            500                     505                     510
Gly Leu Gly Thr Ile Ser Tyr Arg Thr Phe Thr Ala Glu Gln Ala Ser
        515                     520                     525
Leu Leu Thr Tyr Val Val Gly Asp Lys Leu Pro Arg Pro
    530                     535                     540
```

<210> 195
<211> 271
<212> PRT
<213> S. pyogenes

<400> 195

```
Met Asn Leu Leu Gly Ser Arg Arg Val Phe Ser Lys Lys Cys Arg Leu
 1               5                  10                  15
Val Lys Phe Ser Met Val Ala Leu Val Ser Ala Thr Met Ala Val Thr
            20                  25                  30
Thr Val Thr Leu Glu Asn Thr Ala Leu Ala Arg Gln Thr Gln Val Ser
        35                  40                  45
Asn Asp Val Val Leu Asn Asp Gly Ala Ser Lys Tyr Leu Asn Glu Ala
    50                  55                  60
Leu Ala Trp Thr Phe Asn Asp Ser Pro Asn Tyr Tyr Lys Thr Leu Gly
65                  70                  75                  80
Thr Ser Gln Ile Thr Pro Ala Leu Phe Pro Lys Ala Gly Asp Ile Leu
                85                  90                  95
Tyr Ser Lys Leu Asp Glu Leu Gly Arg Thr Arg Thr Ala Arg Gly Thr
            100                 105                 110
Leu Thr Tyr Ala Asn Val Glu Gly Ser Tyr Gly Val Arg Gln Ser Phe
        115                 120                 125
Gly Lys Asn Gln Asn Pro Ala Gly Trp Thr Gly Asn Pro Asn His Val
    130                 135                 140
Lys Tyr Lys Ile Glu Trp Leu Asn Gly Leu Ser Tyr Val Gly Asp Phe
145                 150                 155                 160
Trp Asn Arg Ser His Leu Ile Ala Asp Ser Leu Gly Gly Asp Ala Leu
                165                 170                 175
Arg Val Asn Ala Val Thr Gly Thr Arg Thr Gln Asn Val Gly Gly Arg
            180                 185                 190
Asp Gln Lys Gly Gly Met Arg Tyr Thr Glu Gln Arg Ala Gln Glu Trp
        195                 200                 205
Leu Glu Ala Asn Arg Asp Gly Tyr Leu Tyr Tyr Glu Ala Ala Pro Ile
    210                 215                 220
Tyr Asn Ala Asp Glu Leu Ile Pro Arg Ala Val Val Val Ser Met Gln
225                 230                 235                 240
Ser Ser Asp Asn Thr Ile Asn Glu Lys Val Leu Val Tyr Asn Thr Ala
                245                 250                 255
Asn Gly Tyr Thr Ile Asn Tyr His Asn Gly Thr Pro Thr Gln Lys
            260                 265                 270
```

<210> 196
<211> 265
<212> PRT
<213> S. pyogenes

<400> 196

```
Met Thr Thr Met Gln Lys Thr Ile Ser Leu Leu Ser Leu Ala Leu Leu
 1               5                  10                  15
Ile Gly Leu Leu Gly Thr Ser Gly Lys Ala Ile Ser Val Tyr Ala Gln
            20                  25                  30
Asp Gln His Thr Asp Asn Val Ile Ala Glu Ser Thr Ile Ser Gln Val
        35                  40                  45
Ser Val Glu Ala Ser Met Arg Gly Thr Glu Pro Tyr Ile Asp Ala Thr
    50                  55                  60
Val Thr Thr Asp Gln Pro Val Arg Gln Pro Thr Gln Ala Thr Ile Thr
65                  70                  75                  80
Leu Lys Asp Ala Ser Asp Asn Thr Ile Asn Ser Trp Val Tyr Thr Met
                85                  90                  95
Ala Ala Gln Gln Arg Arg Phe Thr Ala Trp Phe Asp Leu Thr Gly Gln
            100                 105                 110
Lys Ser Gly Asp Tyr His Val Thr Val Thr Val His Thr Gln Glu Lys
        115                 120                 125
Ala Val Thr Gly Gln Ser Gly Thr Val His Phe Asp Gln Asn Lys Ala
    130                 135                 140
Arg Lys Thr Pro Thr Asn Met Gln Gln Lys Asp Thr Ser Lys Ala Met

145                 150                 155                 160
Thr Asn Ser Val Asp Val Asp Thr Lys Ala Gln Thr Asn Gln Ser Ala
                165                 170                 175
Asn Gln Glu Ile Asp Ser Thr Ser Asn Pro Phe Arg Ser Ala Thr Asn
            180                 185                 190
His Arg Ser Thr Ser Leu Lys Arg Ser Thr Lys Asn Glu Lys Leu Thr
        195                 200                 205
Pro Thr Ala Ser Asn Ser Gln Lys Asn Gly Ser Asn Lys Thr Lys Met
    210                 215                 220
Leu Val Asp Lys Glu Glu Val Lys Pro Thr Ser Lys Arg Gly Phe Pro
225                 230                 235                 240
Trp Val Leu Leu Gly Leu Val Val Ser Leu Ala Ala Gly Leu Phe Ile
                245                 250                 255
Ala Ile Gln Lys Val Ser Arg Arg Lys
            260                 265
```

<210> 197
<211> 632
<212> PRT
<213> S. pyogenes

<400> 197

```
Met Ile Gln Ile Gly Lys Leu Phe Ala Gly Arg Tyr Arg Ile Leu Lys
 1               5                  10                  15
Ser Ile Gly Arg Gly Gly Met Ala Asp Val Tyr Leu Ala Asn Asp Leu
            20                  25                  30
Ile Leu Asp Asn Glu Asp Val Ala Ile Lys Val Leu Arg Thr Asn Tyr
        35                  40                  45
Gln Thr Asp Gln Val Ala Val Ala Arg Phe Gln Arg Glu Ala Arg Ala
    50                  55                  60
Met Ala Glu Leu Asn His Pro Asn Ile Val Ala Ile Arg Asp Ile Gly
65                  70                  75                  80
Glu Glu Asp Gly Gln Gln Phe Leu Val Met Glu Tyr Val Asp Gly Ala
                85                  90                  95
Asp Leu Lys Arg Tyr Ile Gln Asn His Ala Pro Leu Ser Asn Asn Glu
            100                 105                 110
Val Val Arg Ile Met Glu Glu Val Leu Ser Ala Met Thr Leu Ala His
        115                 120                 125
Gln Lys Gly Ile Val His Arg Asp Leu Lys Pro Gln Asn Ile Leu Leu
    130                 135                 140
Thr Lys Glu Gly Val Val Lys Val Thr Asp Phe Gly Ile Ala Val Ala
145                 150                 155                 160
Phe Ala Glu Thr Ser Leu Thr Gln Thr Asn Ser Met Leu Gly Ser Val
                165                 170                 175
His Tyr Leu Ser Pro Glu Gln Ala Arg Gly Ser Lys Ala Thr Ile Gln
            180                 185                 190
Ser Asp Ile Tyr Ala Met Gly Ile Met Leu Phe Glu Met Leu Thr Gly
        195                 200                 205
His Ile Pro Tyr Asp Gly Asp Ser Ala Val Thr Ile Ala Leu Gln His
    210                 215                 220
Phe Gln Lys Pro Leu Pro Ser Ile Ile Glu Glu Asn His Asn Val Pro
225                 230                 235                 240
Gln Ala Leu Glu Asn Val Val Ile Arg Ala Thr Ala Lys Lys Leu Ser
                245                 250                 255
Asp Arg Tyr Gly Ser Thr Phe Glu Met Ser Arg Asp Leu Met Thr Ala
            260                 265                 270
Leu Ser Tyr Asn Arg Ser Arg Glu Arg Lys Ile Ile Phe Glu Asn Val
        275                 280                 285
Glu Ser Thr Lys Pro Leu Pro Lys Val Ala Ser Gly Pro Thr Ala Ser
    290                 295                 300
Val Lys Leu Ser Pro Pro Thr Pro Thr Val Leu Thr Gln Glu Ser Arg
```

```
305                 310                 315                 320
Leu Asp Gln Thr Asn Gln Thr Asp Ala Leu Gln Pro Pro Thr Lys Lys
                325                 330                 335
Lys Lys Ser Gly Arg Phe Leu Gly Thr Leu Phe Lys Ile Leu Phe Ser
            340                 345                 350
Phe Phe Ile Val Gly Val Ala Leu Phe Thr Tyr Leu Ile Leu Thr Lys
        355                 360                 365
Pro Thr Ser Val Lys Val Pro Asn Val Ala Gly Thr Ser Leu Lys Val
    370                 375                 380
Ala Lys Gln Glu Leu Tyr Asp Val Gly Leu Lys Val Gly Lys Ile Arg
385                 390                 395                 400
Gln Ile Glu Ser Asp Thr Val Ala Glu Gly Asn Val Val Arg Thr Asp
                405                 410                 415
Pro Lys Ala Gly Thr Ala Lys Arg Gln Gly Ser Ser Ile Thr Leu Tyr
            420                 425                 430
Val Ser Ile Gly Asn Lys Gly Phe Asp Met Glu Asn Tyr Lys Gly Leu
        435                 440                 445
Asp Tyr Gln Glu Ala Met Asn Ser Leu Ile Glu Thr Tyr Gly Val Pro
    450                 455                 460
Lys Ser Lys Ile Lys Ile Glu Arg Ile Val Thr Asn Glu Tyr Pro Glu
465                 470                 475                 480
Asn Thr Val Ile Ser Gln Ser Pro Ser Ala Gly Asp Lys Phe Asn Pro
                485                 490                 495
Asn Gly Lys Ser Lys Ile Thr Leu Ser Val Ala Val Ser Asp Thr Ile
            500                 505                 510
Thr Met Pro Met Val Thr Glu Tyr Ser Tyr Ala Asp Ala Val Asn Thr
        515                 520                 525
Leu Thr Ala Leu Gly Ile Asp Ala Ser Arg Ile Lys Ala Tyr Val Pro
    530                 535                 540
Ser Ser Ser Ser Ala Thr Gly Phe Val Pro Ile His Ser Pro Ser Ser
545                 550                 555                 560
Lys Ala Ile Val Ser Gly Gln Ser Pro Tyr Tyr Gly Thr Ser Leu Ser
                565                 570                 575
Leu Ser Asp Lys Gly Glu Ile Ser Leu Tyr Leu Tyr Pro Glu Glu Thr
            580                 585                 590
His Ser Ser Ser Ser Ser Ser Ser Ser Thr Ser Ser Ser Asn Ser Ser
        595                 600                 605
Ser Ile Asn Asp Ser Thr Ala Pro Gly Ser Asn Thr Glu Leu Ser Pro
    610                 615                 620
Ser Glu Thr Thr Ser Gln Thr Pro
625                 630
```

<210> 198
<211> 1581
<212> PRT
<213> S. pyogenes

<400> 198

```
Met Ala Asp Glu Leu Ser Thr Met Ser Glu Pro Thr Ile Thr Asn His
 1               5                  10                  15
Ala Gln Gln Gln Ala Gln His Leu Thr Asn Thr Glu Leu Ser Ser Ala
            20                  25                  30
Glu Ser Lys Ser Gln Asp Thr Ser Gln Ile Thr Leu Lys Thr Asn Arg
        35                  40                  45
Glu Lys Glu Gln Ser Gln Asp Leu Val Ser Glu Pro Thr Thr Thr Glu
    50                  55                  60
Leu Ala Asp Thr Asp Ala Ala Ser Met Ala Asn Thr Gly Ser Asp Ala
65                  70                  75                  80
Thr Gln Lys Ser Ala Ser Leu Pro Pro Val Asn Thr Asp Val His Asp
                85                  90                  95
Trp Val Lys Thr Lys Gly Ala Trp Asp Lys Gly Tyr Lys Gly Gln Gly
```

```
                100                 105                 110
Lys Val Val Ala Val Ile Ala Thr Gly Ile Asp Pro Ala His Gln Ser
            115                 120                 125
Met Arg Ile Ser Asp Val Ser Thr Ala Lys Val Lys Ser Lys Glu Asp
    130                 135                 140
Met Leu Ala Arg Gln Lys Ala Ala Gly Ile Asn Tyr Gly Ser Trp Ile
145                 150                 155                 160
Asn Asp Lys Val Val Phe Ala His Asn Tyr Val Glu Asn Ser Asp Asn
                165                 170                 175
Ile Lys Glu Asn Gln Phe Glu Asp Phe Asp Glu Asp Trp Glu Asn Phe
            180                 185                 190
Glu Phe Asp Ala Glu Ala Glu Pro Lys Ala Ile Lys Lys His Lys Ile
        195                 200                 205
Tyr Arg Pro Gln Ser Thr Gln Ala Pro Lys Glu Thr Val Ile Lys Thr
    210                 215                 220
Glu Glu Thr Asp Gly Ser His Asp Ile Asp Trp Thr Gln Thr Asp Asp
225                 230                 235                 240
Asp Thr Lys Tyr Glu Ser His Gly Met His Val Thr Gly Ile Val Ala
                245                 250                 255
Gly Asn Ser Lys Glu Ala Ala Ala Thr Gly Glu Arg Phe Leu Gly Ile
            260                 265                 270
Ala Pro Glu Ala Gln Val Met Phe Met Arg Val Phe Ala Asn Asp Ile
        275                 280                 285
Met Gly Ser Ala Glu Ser Leu Phe Ile Lys Ala Ile Glu Asp Ala Val
    290                 295                 300
Ala Leu Gly Ala Asp Val Ile Asn Leu Ser Leu Gly Thr Ala Asn Gly
305                 310                 315                 320
Ala Gln Leu Ser Gly Ser Lys Pro Leu Met Glu Ala Ile Glu Lys Ala
                325                 330                 335
Lys Lys Ala Gly Val Ser Val Val Val Ala Ala Gly Asn Glu Arg Val
            340                 345                 350
Tyr Gly Ser Asp His Asp Asp Pro Leu Ala Thr Asn Pro Asp Tyr Gly
        355                 360                 365
Leu Val Gly Ser Pro Ser Thr Gly Arg Thr Pro Thr Ser Val Ala Ala
    370                 375                 380
Ile Asn Ser Lys Trp Val Ile Gln Arg Leu Met Thr Val Lys Glu Leu
385                 390                 395                 400
Glu Asn Arg Ala Asp Leu Asn His Gly Lys Ala Ile Tyr Ser Glu Ser
                405                 410                 415
Val Asp Phe Lys Asp Ile Lys Asp Ser Leu Gly Tyr Asp Lys Ser His
            420                 425                 430
Gln Phe Ala Tyr Val Lys Glu Ser Thr Asp Ala Gly Tyr Asn Ala Gln
        435                 440                 445
Asp Val Lys Gly Lys Ile Ala Leu Ile Glu Arg Asp Pro Asn Lys Thr
    450                 455                 460
Tyr Asp Glu Met Ile Ala Leu Ala Lys Lys His Gly Ala Leu Gly Val
465                 470                 475                 480
Leu Ile Phe Asn Asn Lys Pro Gly Gln Ser Asn Arg Ser Met Arg Leu
                485                 490                 495
Thr Ala Asn Gly Met Gly Ile Pro Ser Ala Phe Ile Ser His Glu Phe
            500                 505                 510
Gly Lys Ala Met Ser Gln Leu Asn Gly Asn Gly Thr Gly Ser Leu Glu
        515                 520                 525
Phe Asp Ser Val Val Ser Lys Ala Pro Ser Gln Lys Gly Asn Glu Met
    530                 535                 540
Asn His Phe Ser Asn Trp Gly Leu Thr Ser Asp Gly Tyr Leu Lys Pro
545                 550                 555                 560
Asp Ile Thr Ala Pro Gly Gly Asp Ile Tyr Ser Thr Tyr Asn Asp Asn
                565                 570                 575
His Tyr Gly Ser Gln Thr Gly Thr Ala Met Ala Ser Pro Gln Ile Ala
            580                 585                 590
```

```
Gly Ala Ser Leu Leu Val Lys Gln Tyr Leu Glu Lys Thr Gln Pro Asn
        595                 600                 605
Leu Pro Lys Glu Lys Ile Ala Asp Ile Val Lys Asn Leu Leu Met Ser
    610                 615                 620
Asn Ala Gln Ile His Val Asn Pro Glu Thr Lys Thr Thr Thr Ser Pro
625                 630                 635                 640
Arg Gln Gln Gly Ala Gly Leu Leu Asn Ile Asp Gly Ala Val Thr Ser
                645                 650                 655
Gly Leu Tyr Val Thr Gly Lys Asp Asn Tyr Gly Ser Ile Ser Leu Gly
            660                 665                 670
Asn Ile Thr Asp Thr Met Thr Phe Asp Val Thr Val His Asn Leu Ser
        675                 680                 685
Asn Lys Asp Lys Thr Leu Arg Tyr Asp Thr Glu Leu Leu Thr Asp His
    690                 695                 700
Val Asp Pro Gln Lys Gly Arg Phe Thr Leu Thr Ser His Ser Leu Lys
705                 710                 715                 720
Thr Tyr Gln Gly Gly Glu Val Thr Val Pro Ala Asn Gly Lys Val Thr
                725                 730                 735
Val Arg Val Thr Met Asp Val Ser Gln Phe Thr Lys Glu Leu Thr Lys
            740                 745                 750
Gln Met Pro Asn Gly Tyr Tyr Leu Glu Gly Phe Val Arg Phe Arg Asp
        755                 760                 765
Ser Gln Asp Asp Gln Leu Asn Arg Val Asn Ile Pro Phe Val Gly Phe
    770                 775                 780
Lys Gly Gln Phe Glu Asn Leu Ala Val Ala Glu Glu Ser Ile Tyr Arg
785                 790                 795                 800
Leu Lys Ser Gln Gly Lys Thr Gly Phe Tyr Phe Asp Glu Ser Gly Pro
                805                 810                 815
Lys Asp Asp Ile Tyr Val Gly Lys His Phe Thr Gly Leu Val Thr Leu
            820                 825                 830
Gly Ser Glu Thr Asn Val Ser Thr Lys Thr Ile Ser Asp Asn Gly Leu
        835                 840                 845
His Thr Leu Gly Thr Phe Lys Asn Ala Asp Gly Lys Phe Ile Leu Glu
    850                 855                 860
Lys Asn Ala Gln Gly Asn Pro Val Leu Ala Ile Ser Pro Asn Gly Asp
865                 870                 875                 880
Asn Asn Gln Asp Phe Ala Ala Phe Lys Gly Val Phe Leu Arg Lys Tyr
                885                 890                 895
Gln Gly Leu Lys Ala Ser Val Tyr His Ala Ser Asp Lys Glu His Lys
            900                 905                 910
Asn Pro Leu Trp Val Ser Pro Glu Ser Phe Lys Gly Asp Lys Asn Phe
        915                 920                 925
Asn Ser Asp Ile Arg Phe Ala Lys Ser Thr Thr Leu Leu Gly Thr Ala
    930                 935                 940
Phe Ser Gly Lys Ser Leu Thr Gly Ala Glu Leu Pro Asp Gly His Tyr
945                 950                 955                 960
His Tyr Val Val Ser Tyr Tyr Pro Asp Val Val Gly Ala Lys Arg Gln
                965                 970                 975
Glu Met Thr Phe Asp Met Ile Leu Asp Arg Gln Lys Pro Val Leu Ser
            980                 985                 990
Gln Ala Thr Phe Asp Pro Glu Thr Asn Arg Phe Lys Pro Glu Pro Leu
        995                 1000                1005
Lys Asp Arg Gly Leu Ala Gly Val Arg Lys Asp Ser Val Phe Tyr Leu
    1010                1015                1020
Glu Arg Lys Asp Asn Lys Pro Tyr Thr Val Thr Ile Asn Asp Ser Tyr
1025                1030                1035                1040
Lys Tyr Val Ser Val Glu Asp Asn Lys Thr Phe Val Glu Arg Gln Ala
                1045                1050                1055
Asp Gly Ser Phe Ile Leu Pro Leu Asp Lys Ala Lys Leu Gly Asp Phe
            1060                1065                1070
Tyr Tyr Met Val Glu Asp Phe Ala Gly Asn Val Ala Ile Ala Lys Leu
```

```
        1075                1080                1085
Gly Asp His Leu Pro Gln Thr Leu Gly Lys Thr Pro Ile Lys Leu Lys
    1090                1095                1100
Leu Thr Asp Gly Asn Tyr Gln Thr Lys Glu Thr Leu Lys Asp Asn Leu
1105                1110                1115                1120
Glu Met Thr Gln Ser Asp Thr Gly Leu Val Thr Asn Gln Ala Gln Leu
                1125                1130                1135
Ala Val Val His Arg Asn Gln Pro Gln Ser Gln Leu Thr Lys Met Asn
            1140                1145                1150
Gln Asp Phe Phe Ile Ser Pro Asn Glu Asp Gly Asn Lys Asp Phe Val
        1155                1160                1165
Ala Phe Lys Gly Leu Lys Asn Asn Val Tyr Asn Asp Leu Thr Val Asn
    1170                1175                1180
Val Tyr Ala Lys Asp Asp His Gln Lys Gln Thr Pro Ile Trp Ser Ser
1185                1190                1195                1200
Gln Ala Gly Ala Ser Val Ser Ala Ile Glu Ser Thr Ala Trp Tyr Gly
                1205                1210                1215
Ile Thr Ala Arg Gly Ser Lys Val Met Pro Gly Asp Tyr Gln Tyr Val
            1220                1225                1230
Val Thr Tyr Arg Asp Glu His Gly Lys Glu His Gln Lys Gln Tyr Thr
        1235                1240                1245
Ile Ser Val Asn Asp Lys Lys Pro Met Ile Thr Gln Gly Arg Phe Asp
    1250                1255                1260
Thr Ile Asn Gly Val Asp His Phe Thr Pro Asp Lys Thr Lys Ala Leu
1265                1270                1275                1280
Asp Ser Ser Gly Ile Val Arg Glu Glu Val Phe Tyr Leu Ala Lys Lys
                1285                1290                1295
Asn Gly Arg Lys Phe Asp Val Thr Glu Gly Lys Asp Gly Ile Thr Val
            1300                1305                1310
Ser Asp Asn Lys Val Tyr Ile Pro Lys Asn Pro Asp Gly Ser Tyr Thr
        1315                1320                1325
Ile Ser Lys Arg Asp Gly Val Thr Leu Ser Asp Tyr Tyr Tyr Leu Val
    1330                1335                1340
Glu Asp Arg Ala Gly Asn Val Ser Phe Ala Thr Leu Arg Asp Leu Lys
1345                1350                1355                1360
Ala Val Gly Lys Asp Lys Ala Val Val Asn Phe Gly Leu Asp Leu Pro
                1365                1370                1375
Val Pro Glu Asp Lys Gln Ile Val Asn Phe Thr Tyr Leu Val Arg Asp
            1380                1385                1390
Ala Asp Gly Lys Pro Ile Glu Asn Leu Glu Tyr Tyr Asn Asn Ser Gly
        1395                1400                1405
Asn Ser Leu Ile Leu Pro Tyr Gly Lys Tyr Thr Val Glu Leu Leu Thr
    1410                1415                1420
Tyr Asp Thr Asn Ala Ala Lys Leu Glu Ser Asp Lys Ile Val Ser Phe
1425                1430                1435                1440
Thr Leu Ser Ala Asp Asn Asn Phe Gln Gln Val Thr Phe Lys Ile Thr
                1445                1450                1455
Met Leu Ala Thr Ser Gln Ile Thr Ala His Phe Asp His Leu Leu Pro
            1460                1465                1470
Glu Gly Ser Arg Val Ser Leu Lys Thr Ala Gln Asp Gln Leu Ile Pro
        1475                1480                1485
Leu Glu Gln Ser Leu Tyr Val Pro Lys Ala Tyr Gly Lys Thr Val Gln
    1490                1495                1500
Glu Gly Thr Tyr Glu Val Val Val Ser Leu Pro Lys Gly Tyr Arg Ile
1505                1510                1515                1520
Glu Gly Asn Thr Lys Val Asn Thr Leu Pro Asn Glu Val His Glu Leu
                1525                1530                1535
Ser Leu Arg Leu Val Lys Val Gly Asp Ala Ser Asp Ser Thr Gly Asp
            1540                1545                1550
His Lys Val Met Ser Lys Asn Asn Ser Gln Ala Leu Thr Ala Ser Ala
        1555                1560                1565
```

```
Thr Pro Thr Lys Ser Thr Thr Ser Ala Thr Ala Lys Ala
    1570                1575                1580
```